# EUROPEAN PATENT APPLICATION

(11) **EP 4 484 420 A1**
(43) Date of publication of application: **01.01.2025**
(21) Application number: 23755927.3
(22) Date of filing: 21.02.2023
(51) Int. Cl.: C07D 405/14, C07D 307/81, C07D 401/14, C07D 403/14, C07D 407/14, C07D 413/14, C07D 417/14, C07D 491/14, A61P 35/00, A61K 31/343, A61K 31/443

(54) **INHIBITOR OF INTERACTION BETWEEN YAP/TAZ AND TEAD, PREPARATION THEREOF, PHARMACEUTICAL COMPOSITION THEREOF AND USE THEREOF**

(30) Priority: 21.02.2022 CN 202210158641; 31.03.2022 CN 202210344511; 05.07.2022 CN 202210794926; 01.12.2022 CN 202211544166; 06.12.2022 CN 202211559809
(71) Applicant: Etern Biopharma (Shanghai) Co., Ltd., Shanghai 201203 (CN)
(72) Inventor: ZHENG, Qiangang, Shanghai 201203 (CN); JIANG, Xiangqing, Shanghai 201203 (CN); LU, Jiting, Shanghai 201203 (CN); ZHUGE, Hao, Shanghai 201203 (CN); YAO, Jiaqi, Shanghai 201203 (CN); YANG, Kun, Shanghai 201203 (CN); LIU, Jing, Shanghai 201203 (CN); XU, Ming, Shanghai 201203 (CN); LI, Jing, Shanghai 201203 (CN); ZENG, Qinglong, Shanghai 201203 (CN); ZHONG, Xiaofeng, Shanghai 201203 (CN); ZHU, Jidong, Shanghai 201203 (CN)
(74) Representative: Santoro, Sofia
(86) International application number: PCT/CN2023/077504
(87) International publication number: WO 2023/155927

(57) **Abstract**

The present disclosure provides an inhibitor, represented by formula I, of interaction between YAP/TAZ and TEAD, preparation thereof, a pharmaceutical composition thereof and use thereof. The compound provided in the present disclosure can be used as an inhibitor of interaction between YAP/TAZ and TEAD, and is used for treating or preventing diseases mediated by interaction between YAP/TAZ and TEAD.

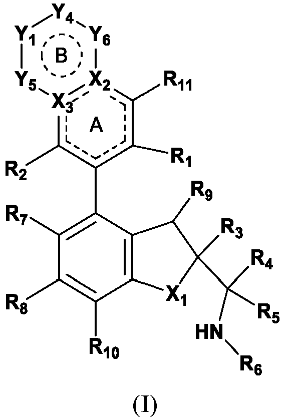

## Description

### CROSS-REFERENCE

The present application claims the benefit of Chinese patent application No. 202210158641.4 filed on February 21, 2022, Chinese patent application No. 202210344511.X filed on March 31, 2022, Chinese patent application No. 202210794926.7 filed on July 5, 2022, Chinese patent application No. 202211544166.0 filed on December 1, 2022 and Chinese patent application No. 202211559809.9 filed on December 6, 2022. The applications are incorporated herein by reference in their entireties.

### TECHNICAL FIELD

The present disclosure relates to an inhibitor of interaction between YAP/TAZ and TEAD, a preparation thereof, a pharmaceutical composition thereof and a use thereof.

### BACKGROUND

The Hippo pathway is involved in regulating cell growth, proliferation and apoptosis, and plays an important role in regulating organ size, cancer occurrence, tissue regeneration, and the renewal and differentiation of stem cells and progenitor cells, etc. Studies have found that in mammals, this pathway has a tumor-suppressing function, and the abnormal activation of the main effector molecules in the pathway is closely related to the occurrence and development of various tumors. In addition, the Hippo pathway interacts with other pathways such as Wnt, Notch, Hedgehog and MAPK/ERK to jointly regulate cell fate. The dysregulation of this pathway also has a significant impact on diseases other than tumors.

The Hippo signaling pathway is highly conserved in the process of evolution. The core part of the Hippo signaling pathway in mammalian cells includes a kinase chain composed of MST1/2 (member of Ste20-like kinase, the homologous gene in Drosophila is Hippo) and LATS1/2 (large tumor suppressor 1/2, the homologous gene in Drosophila is Warts) protein kinases and their adaptor proteins SAV1 and Mob1 (Mps one binder kinase activator-like 1A and 1B, the homologous gene in Drosophila is Mats). This kinase chain can phosphorylate the transcription co-activators YAP (Yes-Associated Protein) and TAZ (Transcription co-activator with PDZ binding motif, also known as WWTR1) (corresponding to Yorkie in Drosophila).

The core components of the mammalian Hippo pathway, LATS1/2, belong to the Nuclear Dbf2-related (NDR) family of kinases, which are activated by binding to the scaffold protein Mob1A/B. LATS1/2 can also be directly activated after phosphorylation by MST1/2. LATS1/2 kinase can phosphorylate multiple sites on the downstream effector YAP, among which the phosphorylation at Ser127 plays a key role in inhibiting YAP. YAP phosphorylated at Ser127 binds to the 14-3-3 protein in the cytoplasm, is retained in the cytoplasm, and cannot enter the nucleus to perform transcription functions, thereby inhibiting YAP's pro-proliferation and anti-apoptotic activity. Similarly, LATS1/2 kinase can phosphorylate multiple sites on the transcription factor TAZ, among which the phosphorylation at Ser89 plays a key role in inhibiting TAZ. Phosphorylated TAZ will be retained or isolated in the cytoplasm. At the same time, phosphorylated YAP or TAZ can be further recognized and degraded by ubiquitin ligase SCF β-TRCP. Therefore, if the Hippo pathway is "on", YAP and/or TAZ will be phosphorylated and inactivated, and retained in the cytoplasm. Conversely, if the Hippo pathway is "off", YAP and/or TAZ will be dephosphorylated and activated, and are often found to be located in the cell nucleus.

YAP, as a transcription factor, does not contain a DNA binding region itself. The activated YAP must bind to the transcription factor after entering the cell nucleus to jointly perform transcription functions. The transcription factor that YAP binds most closely after entering the cell nucleus is TEAD. Human TEAD family proteins include TEAD 1/TEAD2/TEAD3/TEAD4. YAP, together with TEAD (or other transcription factors, such as Smad1, RUNX, ErbB4 and p73), can initiate a series of downstream genes, including the transcription of CTGF (connective tissue growth factor), Gli2, Birc5, Birc2, FGF1 (fibroblast growth factor 1) and AREG (amphiregulin). Like YAP, non-phosphorylated TAZ will enter the cell nucleus, where it binds to a variety of DNA-binding transcription factors, such as PPAPγ (peroxisome proliferation-activated receptor γ), TTF-1 (thyroid transcription factor-1), Pax3, TBX5, RUNX, TEAD1 and Smad2/3/4. Most of the genes whose expression is activated by YAP or TAZ transcription factor complexes are related to cell growth and proliferation.

Based on the above, the Hippo-YAP pathway regulates the size and normal physiological functions of organs by regulating cell proliferation and apoptosis, and is strictly regulated under normal physiological conditions. The inactivation of the protein kinase or the activation of YAP in the Hippo pathway can promote tumor occurrence. In fact, the abnormal activation of the Hippo pathway is a major event in the occurrence and development of various malignant tumors. In tumors including non-small cell lung cancer, breast cancer, head and neck cancer, esophageal cancer, ovarian cancer, liver cancer, prostate cancer, mesothelioma and skin cancer, etc., increased expression levels and nuclear localization of YAP or TAZ have been found.

Malignant pleural mesothelioma (MPM) is a rare malignant tumor of the chest. Its clinical manifestations are usually non-specific and hidden, and many patients are in the late stage of the disease at the time of diagnosis. The treatment options for surgically unresectable MPM are extremely limited, and the current first-line pemetrexed/platinum treatment is unsatisfactory, only achieving a median overall survival of about one year, leaving a large unmet clinical need. Abnormal activation of the Hippo-YAP pathway is present in about 70% of MPM patients and is considered an important cancer driver gene. Lowering the activity of the Hippo-YAP pathway through biological means and small chemical molecules has shown good tumor growth inhibitory activity, indicating that Hippo-YAP is a potential therapeutic target for MPM.

Lung cancer is currently one of the cancers with the highest mortality rate in the world. At present, there are various treatment options for lung cancer in clinical practice, including targeted therapy and immunotherapy, but they all face problems of low response rate or recurrence. In recent years, many studies have shown that the YAP signaling pathway can mediate tumor cell dormancy, resist apoptosis, and other mechanisms to produce drug resistance to lung cancer drugs such as EGFR inhibitors. Inhibiting the Hippo-YAP signaling pathway can increase the sensitivity of tumor cells to EGFR-targeted drugs, suggesting that a combination strategy can be used in clinical practice to improve treatment effects.

Liver cancer is a high-incidence cancer in China, and the current clinical treatment breakthroughs are very limited, leaving a large unmet clinical need. YAP is an important gene regulating the occurrence and development of liver cancer. Multiple *in vivo* experiments have shown that overexpression of YAP alone or knockout of upstream regulatory factors MST1-2 in mouse liver, without introducing other oncogenes, will induce the occurrence of hepatocellular carcinoma; at the same time, in the established liver cancer mouse model, knocking down YAP expression can significantly inhibit tumors and promote tumor cells to differentiate into functional hepatic parenchymal-like cells, accompanied by the recovery of liver function, suggesting that YAP is a potential therapeutic target for liver cancer.

In pancreatic ductal adenocarcinoma (PDAC), KRas mutations are widespread, and the means of targeting KRas are considered to have broad clinical application prospects in PDAC. In the PDAC mouse model, targeting KRas can inhibit tumor growth, but also faces tumor recurrence. Studies have shown that YAP plays an important role in this by regulating Fos and inducing EMT (epithelial-mesenchymal transition); knocking down YAP expression in recurrent tumors can re-inhibit tumor growth, indicating that targeting YAP also has potential clinical application prospects in pancreatic ductal adenocarcinoma.

Inhibitors targeting BRAF and MEK have a wide range of clinical applications in various tumors, including melanoma, colon cancer, thyroid cancer, etc., but they also face the problem of resistance and recurrence after administration. Studies have shown that Hippo-YAP, as a pathway promoting tumor cell growth, is overactivated in multiple drug-resistant tumor models, and inhibiting its activity can significantly increase the sensitivity to BRAF/MEK inhibitors, suggesting that it has the potential for drug combination in clinical practice.

*In vitro,* overexpression of YAP or TAZ in mammalian epithelial cells can lead to cell transformation. Enhanced YAP/TAZ transcriptional activity can induce EMT (epithelial-mesenchymal transition) and endow stem cells with the characteristics of breast cancer cells.

In summary, treatment strategies targeting the Hippo-Yap pathway are very likely to provide new ideas for the treatment of various tumors. The development of specific small molecules to disrupt the interaction between YAP/TAZ and TEAD, weaken YAP's transcriptional activity, and thereby inhibit the occurrence of tumors with abnormal Hippo pathway is expected to become a new strategy for tumor treatment, with a relatively broad clinical application prospect.

### CONTENT OF THE PRESENT INVENTION

The present disclosure provides an inhibitor of interaction between YAP/TAZ and TEAD. The present disclosure also provides a preparation method for the inhibitor, a pharmaceutical composition containing the same and a use thereof for treating or preventing diseases mediated by the interaction between YAP/TAZ and TEAD.

In one aspect, the present disclosure provides a compound of the following formula I or a pharmaceutically acceptable salt, enantiomer, diastereomer, tautomer, solvate, isotopic substitute, polymorph, prodrug or metabolite thereof: 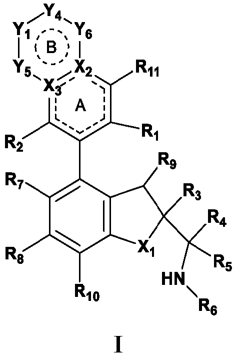 in the formula:
X₁ is selected from: O and NRₐ;
X₂ is selected from: C and N;
X₃ is selected from: C and N;
Y₅ and Y₆ are independently selected from: a bond, O, S, N, -S(O)-, -S(O)₂-, C, CH(R₁₃), C(R₁₃)₂, C(R₁₃), -OCH₂-, -N(R₁₂)CH₂-, -CH₂CH₂- and N(R₁₂), and Y₅ and Y₆ are not simultaneously a bond;
Y₁ and Y₄ are independently selected from: a bond, O, N, C, S, -S(O)-, -C(O)-, -S(O)₂-, CH, CH₂, NH, C(R₁₃), CH(R₁₃), C(R₁₃)₂ and N(R₁₂), provided that Y₁ and Y₄, Y₁ and Y₅, Y₄ and Y₆ are not simultaneously a bond, O, S, -S(O)- or -S(O)₂- and satisfy the valence bond theory;
alternatively, when Y₁, Y₄ and Y₆ are arbitrarily selected from C(R₁₃)₂, two R₁₃ on the same carbon atom, together with the C atom to which they are attached, can form a substituted or unsubstituted 3- to 8-membered cyclohydrocarbyl or substituted or unsubstituted 4- to 9-membered heterocyclyl ring, thereby forming a spiro ring structure with ring B;
alternatively, the ring atom in Y₁ or Y₆ and the ring atom in Y₄, together with the substituent attached to the ring atom, form a substituted or unsubstituted 3- to 8-membered cyclohydrocarbyl, substituted or unsubstituted 4- to 9-membered heterocyclyl, substituted or unsubstituted 6- to 10-membered aryl or substituted or unsubstituted 5- to 10-membered heteroaryl ring, thereby forming a fused ring structure with ring B;
alternatively, X₂, together with the ring atom in Y₆ and the ring atom attached to R₁₁ and the substituent attached to the ring atom, forms a substituted or unsubstituted 5- or 6-membered cyclohydrocarbyl, substituted or unsubstituted 5- or 6-membered heterocyclyl, substituted or unsubstituted phenyl or substituted or unsubstituted 5- or 6-membered heteroaryl ring, thereby forming a three-fused ring structure with rings A and B;
ring B is a 5-, 6- or 7-membered heterocyclyl ring, a benzene ring or a 5-, 6- or 7-membered heteroaryl ring; ring B can be optionally substituted by one or more R₁₃ and/or R₁₂ that do not participate in ring formation, and can be further optionally substituted by 1, 2 or 3 substituents selected from hydroxyl, amino, halogen, substituted or unsubstituted alkyl, -NR'R" and substituted or unsubstituted alkoxy;
R₁ is selected from: cyano and -C(O)NR'R";
R₂ is selected from: H, halogen, substituted or unsubstituted alkyl and substituted or unsubstituted alkoxy;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₇ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl;
R₁₁ is selected from: H, hydroxyl, amino, halogen, substituted or unsubstituted alkyl, - NR'R" and substituted or unsubstituted alkoxy;
each R₁₂ that does not participate in ring formation is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 6- to 14-membered aryl-S(O)₂-, substituted or unsubstituted 4- to 9-membered heterocyclyl, NR'R"-C(O)-, substituted or unsubstituted 3- to 8-membered cyclohydrocarbyl-C(O)- and substituted or unsubstituted C1-C4 alkyl;
each R₁₃ that does not participate in ring formation is independently selected from: H, halogen, substituted or unsubstituted C1-C4 acyl, hydroxyl, cyano, -NR'R", substituted or unsubstituted C1-C4 alkoxy, substituted or unsubstituted C1-6 alkyl, substituted or unsubstituted C1-C4 alkyl-S(O)₂-, substituted or unsubstituted C6-14 aryl-S(O)₂-, substituted or unsubstituted 3- to 6-membered cyclohydrocarbyl-C(O)- and NR'R"-C(O)-, or two R₁₃ on the same carbon atom form an oxo (=O) or thio (=S) group;
after R₁₃ or R₁₂ participates in ring formation, its ring atom can be optionally substituted by 1 to 3 heteroatoms selected from O, N and S;
R' and R" are each independently selected from H, C1-C4 alkyl, C3-C6 cycloalkyl, C1-C4 alkyl-S(O)₂- and C1-C4 haloalkyl;
Rₐ is selected from H and C1-C4 alkyl;
wherein the dashed line indicates the presence or absence of a double bond.

In another aspect, the present disclosure provides a pharmaceutical composition comprising a therapeutically or preventively effective amount of the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIII a, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, and the pharmaceutical composition can further comprise a pharmaceutically acceptable carrier or excipient.

In another aspect, the present disclosure provides a use of the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition of the present disclosure in the manufacture of a medicament for treating or preventing diseases mediated by the interaction between YAP/TAZ and TEAD.

In another aspect, the present disclosure provides the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition of the present disclosure for use in treating or preventing diseases. In some embodiments, the present disclosure also provides the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition of the present disclosure for use in treating or preventing diseases mediated by the interaction between YAP/TAZ and TEAD.

In yet another aspect, the present disclosure provides a method for treating or preventing diseases mediated by the interaction between YAP/TAZ and TEAD, and the method comprises administering to a subject in need thereof a therapeutically effective amount of the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, or the pharmaceutical composition of the present disclosure.

The more detailed technical solutions and descriptions of the present disclosure are as follows.

### DETAILED DESCRIPTION OF THE PREFERRED EMBODIMENT

The following specific embodiments are provided to enable those skilled in the art to more clearly understand the contents of the present disclosure. It should be noted that these embodiments are described for the purpose of illustration only and are not intended to limit the scope of protection of the present disclosure.

### I. Term

In the following description, certain specific details are set forth in order to provide a thorough understanding of various embodiments. However, those skilled in the art should understand that the technical solutions of the present disclosure may be implemented without these details. In other instances, well-known structures are not shown or described in detail to avoid unnecessarily obscuring the description of the embodiments. Unless the context requires otherwise, throughout this specification and the following claims, the word "include" and variations thereof, such as "comprise" and "contain" are to be interpreted in an open, inclusive sense, that is, as "include but not limited to". In addition, the headings provided herein are for convenience only and do not interpret the scope or meaning of the claimed disclosure.

Reference in this specification to "an embodiment" means that a particular feature, structure or characteristic described with respect to the embodiment is included in at least one embodiment. Accordingly, the appearances of the phrase "in an embodiment" in various places in this specification are not necessarily all referring to the same embodiment. In addition, the particular features, structures or characteristics can be combined in any suitable manner in one or more embodiments. Additionally, as used in this specification and the claims, the singular forms "a", "an" and "the" include plural referents unless the context clearly dictates otherwise. It should also be noted that the term "or" is generally used in its sense including "and/or" unless the context clearly dictates otherwise.

Definitions of standard chemical terms can be found in references (including Carey and Sundberg "ADVANCED ORGANIC CHEMISTRY 4TH ED." Vols. A (2000) and B (2001), Plenum Press, New York). Unless otherwise stated, conventional methods within the skill in the art, such as mass spectrometry, NMR, IR and UV/VIS spectroscopy and pharmacological methods, are used. Unless specific definitions are given, the terms used herein in the relevant descriptions of analytical chemistry, organic synthetic chemistry, and pharmaceutical and medicinal chemistry are those known in the art. Standard techniques can be used in chemical synthesis, chemical analysis, drug preparation, formulation and delivery, and in the treatment of patients. For example, the reaction and purification can be carried out using the manufacturer's instructions for the use of the kit, or in a manner known in the art or as described in the present disclosure. The above techniques and methods can generally be implemented in accordance with conventional methods well known in the art, as described in the various general and more specific documents cited and discussed in this specification. In this specification, groups and substituents thereof can be selected by those skilled in the art to provide stable structural moieties and compounds.

When a substituent is described by a conventional chemical formula written from left to right, the substituent also includes the chemically equivalent substituent obtained when the structural formula is written from right to left. For example, -CH₂O- is equivalent to -OCH₂-.

Certain chemical groups defined herein are preceded by simplified symbols to indicate the total number of carbon atoms present in the group. For example, C1-C6 alkyl refers to an alkyl group as defined below having a total of 1 to 6 carbon atoms. The total number of carbon atoms in the simplified symbols does not include carbon that may be present in the substituent of the group.

When a variable is mentioned in the present disclosure as "selected from: ...", it means that the variable is selected from any one of the options listed after the colon, or, where possible, it means that the variable is selected from one or more of the options listed after the colon.

All numerical ranges referred to in the present disclosure are expressed to include both endpoints of the range, all integers within the range, and subranges formed from those integers.

In addition to the foregoing, when used in the specification and claims of the present disclosure, the following terms have the meanings shown below unless otherwise specifically stated.

The term "halogen" refers to fluorine, chlorine, bromine or iodine.

"Hydroxyl" refers to -OH group.

"Hydroxyalkyl" refers to an alkyl group as defined below substituted by hydroxyl (-OH).

"Carbonyl" refers to -C(=O)- group.

"Oxo" refers to =O group.

"Thio" refers to =S group.

"Nitro" refers to -NO₂.

"Cyano" refers to -CN.

"Amino" refers to -NH₂.

"Substituted amino" refers to an amino group substituted by one or two of the alkyl, alkylcarbonyl, aralkyl, aryl, heteroaryl, heterocyclyl, heteroaralkyl as defined below, for example, monoalkylamino, dialkylamino, alkylcarbonylamino, aralkylamino, heteroaralkylamino, heterocyclylamino, heteroarylamino and arylamino. In some embodiments herein, "substituted amino" is represented as -NR'R", wherein R' and R" are each independently selected from H, C1-C4 alkyl, C3-C6 cycloalkyl, C1-C4 alkyl-S(O)₂- and C1-C4 haloalkyl (provided that at least one of R' and R" is not H).

"Carboxyl" refers to -COOH.

-C(O) refers to -C(=O).

In the present disclosure, the term "Ci-Cj" represents a range of carbon atom numbers, where i and j are integers, and where the range of carbon atom numbers includes the endpoints (i.e., i and j) as well as each integer point between the endpoints, where j is greater than i. For example, C1-C6 represents a range of 1 to 6 carbon atoms, including 1 carbon atom, 2 carbon atoms, 3 carbon atoms, 4 carbon atoms, 5 carbon atoms and 6 carbon atoms. For another example, the term "C1-C12" represents 1 to 12, especially 1 to 10, especially 1 to 8, especially 1 to 6, especially 1 to 5, especially 1 to 4, especially 1 to 3 or especially 1 to 2 carbon atoms.

In the present disclosure, as a group or a part of other groups (e.g., used in a group such as an alkyl group substituted by halogen (e.g., fluorine, chlorine, bromine or iodine)), the term "alkyl" refers to a fully saturated straight or branched hydrocarbon chain group consisting only of carbon and hydrogen atoms, having, for example, 1 to 12 (preferably 1 to 8, more preferably 1 to 6) carbon atoms, and is linked to the rest of the molecule by a single bond. In some embodiments, the alkyl contains 1 to 11 carbon atoms. In some embodiments, the alkyl contains 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms or 1 to 2 carbon atoms. Non-limiting examples of the alkyl include, but are not limited to, methyl, ethyl, *n*-propyl, isopropyl, *n*-butyl, isobutyl, *sec*-butyl, *tert-butyl, n*-pentyl, *tert*-pentyl, 2-methylbutyl, 2,2-dimethylpropyl, *n*-hexyl, *n*-heptyl, 2-methylhexyl, 3-methylhexyl, *n*-octyl, *n*-nonyl, *n*-decyl, *n*-undecyl, *n*-dodecyl, etc. Preferably, each alkyl of embodiments of the present disclosure is C1-C4 alkyl. In some embodiments, the carbon chain length of the alkyl may range from 8 to 22 carbon atoms, and is preferably an aliphatic chain. Unless otherwise specifically stated in this specification, the alkyl can be optionally substituted.

In the present disclosure, as a group or a part of other groups, the term "alkenyl" refers to a straight or branched hydrocarbon chain group consisting only of carbon and hydrogen atoms with one or more carbon-carbon double bonds (-C=C-), and is linked to the rest of the molecule by a single bond. In some embodiments, the alkenyl contains 2 to 12 carbon atoms. In some embodiments, the alkenyl contains 2 to 11 carbon atoms. In some embodiments, the alkenyl contains 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, 2 to 3 carbon atoms, and in some embodiments, the alkenyl contains 2 carbon atoms. Non-limiting examples of the alkenyl include vinyl, 1-propenyl, 2-propenyl (allyl), isopropenyl, 2-methyl-1-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, 1-undecenyl, 2-undecenyl, 3-undecenyl, 4-undecenyl, 5-undecenyl, 6-undecenyl, 7-undecenyl, 8-undecenyl, 9-undecenyl, 10-undecenyl, 1-dodecenyl, 2-dodecenyl, 3-dodecenyl, 4-dodecenyl, 5-dodecenyl, 6-dodecenyl, 7-dodecenyl, 8-dodecenyl, 9-dodecenyl, 10-dodecenyl and 11-dodecenyl. Unless otherwise specifically stated in this specification, the alkenyl can be optionally substituted.

In the present disclosure, as a group or a part of other groups, the term "alkynyl" refers to a straight or branched hydrocarbon chain group consisting only of carbon and hydrogen atoms with one or more carbon-carbon triple bonds (-C=C-), and is linked to the rest of the molecule by a single bond. In some embodiments, the alkynyl contains 2 to 12 carbon atoms. In some embodiments, the alkynyl contains 2 to 11 carbon atoms. In some embodiments, the alkynyl contains 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, 2 to 3 carbon atoms, and in some embodiments, the alkynyl contains 2 carbon atoms. Non-limiting examples of the alkynyl include ethynyl, propynyl, butynyl, pentynyl, etc. Unless otherwise specifically stated in this specification, the alkynyl can be optionally substituted.

In the present disclosure, as a group or a part of other groups, the term "alkoxy" refers to "-O-alkyl" group, wherein the alkyl has the meaning described above.

In the present disclosure, as a group or a part of other groups, the term "haloalkyl" refers to an alkyl group substituted by one or more halogens, wherein the alkyl has the meaning described above and the number of halogens may be as many as the number of substitutable hydrogens on the alkyl.

In the present disclosure, as a group or a part of other groups, the term "acyl" refers to the monovalent group remaining after removing the hydroxyl from an aliphatic carboxylic acid, which can be represented by the general formula "G-C(=O)-", wherein G represents H or the alkyl, alkenyl or alkynyl as described above. For example, specific examples of C1-C4 acyl include, but are not limited to, formyl, acetyl, propionyl, butyryl, etc.

In the present disclosure, as a group or a part of other groups, the term "cyclohydrocarbyl" refers to a saturated or partially unsaturated non-aromatic monocyclic or polycyclic hydrocarbyl or moiety (representing a structural fragment of an organic molecule) consisting only of carbon and hydrogen atoms and optionally having one or more carbon-carbon double bonds (-C=C-) or carbon-carbon triple bonds (-C=C-), which can be linked to the rest of the molecule by any suitable carbon atom, and includes "cycloalkyl", "cycloalkenyl" and "cycloalkynyl". In some embodiments, the cyclohydrocarbyl contains 3, 4, 5, 6, 7, 8, 9, 10, 11 or 12 ring carbon atoms. Non-limiting examples of the cyclohydrocarbyl include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl and cyclooctynyl. Unless otherwise specifically stated in this specification, the carbon atoms in the cyclohydrocarbyl can be optionally oxidized (thus forming an oxo (=O) group), and the cyclohydrocarbyl can be optionally substituted.

In the present disclosure, as a group or a part of other groups, the term "cycloalkyl" refers to a saturated non-aromatic monocyclic or polycyclic hydrocarbyl or moiety consisting only of carbon and hydrogen atoms, which can be linked to the rest of the molecule by any suitable carbon atom. In some embodiments, the cycloalkyl contains 3 to 12 ring carbon atoms, 3 to 10 ring carbon atoms, 3 to 9 ring carbon atoms, 3 to 8 ring carbon atoms, 3 to 7 ring carbon atoms, 3 to 6 ring carbon atoms, 3 to 5 ring carbon atoms, 4 to 12 ring carbon atoms, 4 to 10 ring carbon atoms, 4 to 9 ring carbon atoms, 4 to 8 ring carbon atoms, 4 to 7 ring carbon atoms, 4 to 6 ring carbon atoms, 4 to 5 ring carbon atoms. Non-limiting examples of the cycloalkyl include cyclopropyl, cyclobutyl, cyclopentyl, cyclohexyl, cycloheptyl, cyclooctyl, etc. Unless otherwise specifically stated in this specification, the carbon atoms in the cycloalkyl can be optionally oxidized (thus forming an oxo (=O) group), and the cycloalkyl can be optionally substituted.

In the present disclosure, as a group or a part of other groups, the term "cycloalkenyl" refers to a partially unsaturated non-aromatic monocyclic or polycyclic hydrocarbyl or moiety consisting only of carbon and hydrogen atoms and having one or more carbon-carbon double bonds (-C=C-), which can be linked to the rest of the molecule by any suitable carbon atom. In some embodiments, the cycloalkenyl contains 5 to 12 ring carbon atoms, 5 to 10 ring carbon atoms, 5 to 9 ring carbon atoms, 5 to 8 ring carbon atoms, 5 to 7 ring carbon atoms, 5 to 6 ring carbon atoms. Non-limiting examples of the cycloalkenyl include cyclopentenyl, cyclohexenyl, cycloheptenyl, cyclooctenyl, etc. Unless otherwise specifically stated in this specification, the carbon atoms in the cycloalkenyl can be optionally oxidized (thus forming an oxo (=O) group), and the cycloalkenyl can be optionally substituted.

In the present disclosure, as a group or a part of other groups, the term "cycloalkynyl" refers to a partially unsaturated non-aromatic monocyclic or polycyclic hydrocarbyl or moiety consisting only of carbon and hydrogen atoms and having one or more carbon-carbon triple bonds (-C=C-), which can be linked to the rest of the molecule by any suitable carbon atom. In some embodiments, the cycloalkynyl contains 8 to 12 ring carbon atoms, 8 to 11 ring carbon atoms, 8 to 10 ring carbon atoms, 8 to 9 ring carbon atoms. Non-limiting examples of the cycloalkynyl include cyclooctynyl, etc. Unless otherwise specifically stated in this specification, the carbon atoms in the cycloalkynyl can be optionally oxidized (thus forming an oxo (=O) group), and the cycloalkynyl can be optionally substituted.

In the present disclosure, as a group or a part of other groups, the term "spiro cyclyl" or "spiro ring" refers to a ring system of a cyclohydrocarbyl consisting of a plurality of rings (e.g., two, three or more), wherein at least two rings are bonded to each other by sharing a carbon atom. In some embodiments, the spiro cyclyl contains 6 to 12 ring carbon atoms, 6 to 11 ring carbon atoms, 6 to 10 ring carbon atoms, 6 to 9 ring carbon atoms, 6 to 8 ring carbon atoms, 6 to 7 ring carbon atoms. Non-limiting examples of the spiro cyclyl include, but are not limited to, spiro[5.5]undecyl, spiro-pentadienyl, spiro[3.6]decyl, etc. Unless otherwise specifically stated in this specification, the spiro cyclyl can be optionally substituted.

In the present disclosure, as a group or a part of other groups, the term "fused cyclyl" or "fused ring" (also referred to as "condensed ring") refers to a ring system of a cyclohydrocarbyl consisting of a plurality of rings (e.g., two, three or more), wherein at least two rings are bonded to each other by sharing two adjacent carbon atoms (i.e., the at least two rings share a covalent bond, so that the bridgehead atoms are directly connected). In some embodiments, the fused cyclyl contains 5 to 12 ring carbon atoms, 5 to 11 ring carbon atoms, 5 to 10 ring carbon atoms, 5 to 9 ring carbon atoms, 5 to 8 ring carbon atoms, 5 to 7 ring carbon atoms, 5 to 6 ring carbon atoms. Non-limiting examples of the fused cyclyl include, but are not limited to, 2,3-indanyl, 1,2,3,4-tetrahydro-naphthyl, 5,6,7,8-tetrahydro-naphthyl, 8,9-dihydro-7*H*-benzocyclohepten-6-yl, 6,7,8,9-tetrahydro-5*H*-benzocycloheptenyl, 5,6,7,8,9,10-hexahydro-benzocyclooctenyl, etc. Unless otherwise specifically stated in this specification, the fused cyclyl can be optionally substituted.

In the present disclosure, as a group or a part of other groups, the term "bridged cyclyl" or "bridged ring" refers to a ring system of a cyclohydrocarbyl consisting of a plurality of rings (e.g., two, three or more), wherein at least two rings are bonded to each other by sharing three or more carbon atoms (the at least two rings are separated by two bridgehead atoms by a bridge containing at least one atom). In some embodiments, the bridged cyclyl contains 8 to 12 ring carbon atoms, 8 to 11 ring carbon atoms, 8 to 10 ring carbon atoms, 8 to 9 ring carbon atoms. Non-limiting examples of the bridged cyclyl include, but are not limited to, bicyclo[1.1.1]pentenyl, bicyclo[2.2.1]heptenyl, bicyclo[2.2.1]heptyl, bicyclo[3.1.1]heptyl, bicyclo[2.2.2]octyl, bicyclo[3.2.1]octyl, bicyclo[3.2.1]octenyl, bicyclo[3.3.1]nonyl, bicyclo[3.3.3]undecyl, adamantyl, etc. Unless otherwise specifically stated in this specification, the bridged cyclyl can be optionally substituted.

In the present disclosure, as a group or a part of other groups, the term "heterocyclyl" or "heterocycle" refers to a stable saturated or partially unsaturated non-aromatic cyclic group or moiety consisting of carbon atoms (e.g., 2, 3, 4, 5, 6, 7, 8, 9, 10, 11, 12, 13 or 14 carbon atoms), as well as heteroatoms (e.g., 1 to 6 heteroatoms, more preferably 1, 2 or 3 heteroatoms) selected from nitrogen, phosphorus, oxygen and sulfur (preferably nitrogen, oxygen or sulfur). In some embodiments, the heterocyclyl can contain 3, 4, 5, 6, 7, 8, 9, 10, 11, 12, 13, 14, 15, 16, 17, 18, 19 or 20 ring-forming atoms, e.g., 3 to 20 ring-forming atoms, 3 to 19 ring-forming atoms, 3 to 18 ring-forming atoms, 3 to 17 ring-forming atoms, 3 to 16 ring-forming atoms, 3 to 15 ring-forming atoms, 4 to 12 ring-forming atoms, 4 to 10 ring-forming atoms, 4 to 9 ring-forming atoms, 4 to 8 ring-forming atoms, 4 to 7 ring-forming atoms, 4 to 6 ring-forming atoms, 4 to 5 ring-forming atoms. Unless otherwise specifically stated in this specification, the heterocyclyl can be monocyclic, bicyclic, tricyclic or more cyclic systems, which can include a condensed/fused ring system (i.e., fused heterocyclyl, such as 4- to 9-membered fused heterocyclyl), bridged ring system (i.e., bridged heterocyclyl, such as 6- to 12-membered bridged heterocyclyl) or spiro ring system (i.e., spiroheterocyclyl, such as 6- to 12-membered spiroheterocyclyl). A fused heterocyclyl group refers to a ring system of a heterocyclyl group consisting of a plurality of rings (e.g., two, three or more), wherein at least two rings are bonded to each other by sharing two adjacent atoms (i.e., at least two rings share a covalent bond, so that the bridgehead atoms are directly connected), preferably bicyclic fused heterocyclyl. A bridged heterocyclyl group refers to a ring system of a heterocyclyl group consisting of a plurality of rings (e.g., two, three or more), wherein at least two rings are bonded to each other by sharing three or more atoms (at least two rings are separated by two bridgehead atoms by a bridge containing at least one atom). A spiroheterocyclyl group refers to a ring system of a heterocyclyl group consisting of a plurality of rings (e.g., two, three or more), wherein at least two rings are bonded to each other by sharing a carbon atom. In the heterocyclyl, the nitrogen, carbon or sulfur atoms can be optionally oxidized, and the nitrogen atoms can be optionally quaternized. The heterocyclyl can be linked to the rest of the molecule via a carbon atom or a heteroatom and through a single bond. In some cases, the heterocyclyl can be carbon-linked, nitrogen-linked or sulfur-linked. In some embodiments, the heterocyclyl is carbon-linked. In some embodiments, the heterocyclyl is nitrogen-linked. In some embodiments, the heterocyclyl is sulfur-linked. Unless otherwise specifically stated in this specification, the heterocyclyl can be optionally substituted.

The heterocyclyl also includes groups in which the heterocyclyl group is fused to a saturated, partially unsaturated or fully unsaturated (i.e., aromatic) cyclohydrocarbyl, aryl, heterocyclyl or heteroaryl group. In the heterocyclyl containing fused rings, one or more rings can be the aryl or heteroaryl as defined below. Examples of the fused heterocyclyl include, but are not limited to, phenyl-fused heterocyclyl or pyridyl-fused heterocyclyl, as well as quinolinyl, isoquinolinyl, quinoxalinyl, quinozinyl, quinazolinyl, azaindolizinyl, pteridinyl, chromenyl, isochromenyl, indolyl, isoindolyl, indolizinyl, indazolyl, purinyl, benzofuranyl, isobenzofuranyl, benzimidazolyl, benzothienyl, benzothiazolyl, carbazolyl, phenazinyl, phenothiazinyl, phenanthridinyl, imidazo[1,2-*a*]pyridinyl, [1,2,4]triazolo[4,3-*a*]pyridyl, [1,2,3]triazolo[4,3-*a*]pyridyl-fused heterocyclyl, etc.

In some embodiments, the heterocyclyl is a stable 4- to 12-membered, 5- to 12-membered, 6- to 10-membered, 4- to 10-membered or 4- to 9-membered nonaromatic monocyclic, bicyclic, tricyclic or more cyclic group (including a fused, bridged or spiro ring group) containing 1 to 3 heteroatoms selected from nitrogen, oxygen and sulfur, for example, a stable 5- to 10-membered (e.g., 4- to 9-membered) nonaromatic monocyclic, bicyclic, tricyclic or more cyclic group (including a fused, bridged or spiro ring group) containing 1 to 3 heteroatoms selected from nitrogen, oxygen and sulfur. Examples of the heterocyclyl include, but are not limited to: pyrrolidinyl, morpholinyl, piperazinyl, homopiperazinyl, piperidinyl, thiomorpholinyl, 2,7-diaza-spiro[3.5]nonan-7-yl, 2-oxa-6-aza-spiro[3.3]heptan-6-yl, 2-oxa-6-aza-spiro[3.4]octan-7-yl, 8-oxa-2-aza-spiro[4.5]decan-6-yl, 2,5-diaza-bicyclo[2.2.1]heptan-2-yl, azetidinyl, oxetanyl, thietanyl, thiolanyl, pyranyl, tetrahydropyranyl, thiopyranyl, tetrahydrofuranyl, oxazinyl, dioxolanyl, tetrahydroisoquinolinyl, decahydroisoquinolinyl, imidazolinyl, imidazolidinyl, quinozinyl, thiazolidinyl, isothiazolidinyl, isoxazolidinyl, indolinyl, octahydroindolyl, octahydroisoindolyl, pyrazolidinyl, phthalimido, dioxothiomorpholinyl, dioxothiolanyl, dioxothietanyl, thianyl, dioxothianyl, thiomorpholinyl, 1,4-oxathianyl, etc.

In the present disclosure, as a group or a part of other groups, "aryl" or "aromatic ring" refers to a conjugated hydrocarbon ring system group or moiety having 6 to 18 carbon atoms (e.g., 6 to 14 carbon atoms or 6 to 10 carbon atoms, e.g., 6, 7, 8, 9 or 10 carbon atoms). The aryl can be monocyclic, bicyclic, tricyclic or more cyclic ring systems, and can also be fused with the cyclohydrocarbyl or heterocyclyl as defined above. In the case of polycyclic systems, only one ring needs to be aromatic, although all rings can be aromatic. Examples of the aryl include, but are not limited to, phenyl, naphthyl, anthracenyl, phenanthrenyl, fluorenyl, 2,3-dihydro-1*H*-isoindolyl, 2-benzoxazolinone, 2*H*-1,4-benzoxazin-3(4*H*)-one-7-yl, etc. Unless otherwise specifically stated in this specification, the aryl can be optionally substituted.

In the present disclosure, as a group or a part of other groups, the term "heteroaryl" or "heteroaromatic ring" refers to a conjugated ring system group or moiety having carbon atoms (e.g., 1 to 15 carbon atoms, 1 to 14 carbon atoms, 1 to 13 carbon atoms, 1 to 12 carbon atoms, 1 to 11 carbon atoms, 1 to 10 carbon atoms, e.g., 1, 2, 3, 4, 5, 6, 7, 8, 9 or 10 carbon atoms) and heteroatoms selected from nitrogen, oxygen and sulfur (e.g., 1 to 6 heteroatoms, more preferably 1, 2 or 3 heteroatoms) within the ring. In some embodiments, the heteroaryl can contain 5, 6, 7, 8, 9, 10, 11, 12, 13, 14, 15, 16, 17, 18, 19 or 20 ring-forming atoms, e.g., 5 to 20 ring-forming atoms, 5 to 19 ring-forming atoms, 5 to 18 ring-forming atoms, 5 to 17 ring-forming atoms, 5 to 16 ring-forming atoms, 5 to 15 ring-forming atoms, 5 to 14 ring-forming atoms, 5 to 13 ring-forming atoms, 5 to 12 ring-forming atoms, 5 to 10 ring-forming carbon atoms, 5 to 9 ring-forming atoms, 5 to 8 ring-forming atoms, 5 to 7 ring-forming atoms or 5 to 6 ring-forming atoms. Unless otherwise specifically stated in this specification, the heteroaryl can be monocyclic, bicyclic, tricyclic or more cyclic ring systems, and can also be fused with the cyclohydrocarbyl, aryl or heterocyclyl as defined above. In the heteroaryl, the nitrogen, carbon or sulfur atoms can be optionally oxidized, and the nitrogen atoms can be optionally quaternized. For the purposes of the present disclosure, the heteroaryl is preferably a stabilized 5- to 12-membered aromatic group comprising 1 to 5 heteroatoms selected from nitrogen, oxygen and sulfur, more preferably a stabilized 5- to 10-membered aromatic group comprising 1 to 4 heteroatoms selected from nitrogen, oxygen and sulfur or a 5- to 6-membered aromatic group comprising 1, 2 or 3 heteroatoms selected from nitrogen, oxygen and sulfur. Unless otherwise specifically stated in this specification, the heteroaryl can be optionally substituted.

Examples of the heteroaryl include, but are not limited to, thienyl, imidazolyl, pyrazolyl, thiazolyl, isothiazolyl, oxazolyl, oxadiazolyl, isoxazolyl, pyridyl, pyrimidinyl, pyrazinyl, pyridazinyl, benzimidazolyl, benzomorpholinyl, benzoisodiazolyl, benzotriazolyl, benzopyrazolyl, imidazopyridyl, pyridomorpholinyl, pyrazolopyridyl, indolyl, furyl, pyrrolyl, triazolyl, tetrazolyl, triazinyl, pyridinonyl, pyrimidinonyl, pyridazinonyl, indolizinyl, isoindolyl, indazolyl, isoindazolyl, purinyl, quinolinyl, isoquinolinyl, phthalazinyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, indolizinyl, *o-*phenanthrolinyl, phenoxazinyl, phenothiazinyl, 4,5,6,7-tetrahydrobenzo[*b*]thienyl, naphthopyridyl, [1,2,4]triazolo[4,3-*b*]pyridazinyl, [1,2,4]triazolo[4,3-*a*]pyrazinyl, [1,2,4]triazolo[4,3-*c*]pyrimidinyl, [1,2,4]triazolo[4,3-*a*]pyridyl, imidazo[1,2*-a*]pyridyl, imidazo[1,2-*b*]pyridazinyl, imidazo[1,2-*a*]pyrazinyl, etc.

In the present disclosure, all cyclic groups can exist as substituents on the mother nucleus, or they can exist as part of a chain-like (including straight and branched) structure, mother nucleus or part of the mother nucleus.

In the present disclosure, the term "heteroarylalkyl" refers to the alkyl as defined above substituted by the heteroaryl as defined above.

In the present disclosure, "optional" or "optionally" means that the subsequently described event or condition may or may not occur, and that the description includes both instances in which the event or condition does and does not occur.

In the present disclosure, the term "substituted", whether or not the term "optionally" is used before (i.e., equivalent to substituted or unsubstituted), means that one or more hydrogens of a specified group or moiety are substituted by a "suitable substituent". Herein, the number of the substituent can be one or more, i.e., 1, 2, 3, 4, 5 or 6 or more, depending on the group being substituted and the nature of the substituent. For example, when the substituent of the ethyl is halogen, the group can be substituted by 1, 2, 3, 4 or 5 substituents according to the structure of the substituted group, such as trifluoromethyl, pentafluoroethyl, etc. In some embodiments, the number of the substituent is 1, 2 or 3. In some embodiments, the number of the substituent is 1 or 2. In some embodiments, the number of the substituent is 1. It will be understood that "substituted" or "substituted by ..." includes the implicit condition that such substitution is carried out according to the allowable valence of the substitution atom, and the substitution results in a stable or chemically viable compound, such as a compound that does not spontaneously transform, for example, by rearrangement, cyclization and elimination. Unless otherwise stated, an "optionally substituted" group can have a suitable substituent at each substitutable position of the group, and where more than one position in any given structure can be substituted by more than one substituent selected from the specified group, the substituent can be the same or different at each position. Those skilled in the art will understand that the substituents themselves can be substituted, if appropriate. Unless specifically indicated as "unsubstituted", references to chemical moieties herein are to be understood as including substituted variants. For example, reference to an "aryl" group or moiety implicitly includes both unsubstituted aryl groups and substituted variations.

For the entirety of this application, the above "suitable substituents" should be understood to include, but are not limited to, alkyl, alkenyl, alkynyl, halogen, haloalkyl, haloalkenyl, haloalkynyl, alkoxy, cyano, hydroxyl, amino, monoalkylamino, dialkylamino, nitro, aryl, heteroaryl, cyclohydrocarbyl (e.g., cycloalkyl and cycloalkenyl) and heterocyclyl as described herein; these groups as substituents, including alkyl, alkenyl, alkynyl, alkyl in haloalkyl, alkenyl in haloalkenyl, alkynyl in haloalkynyl, alkoxy, alkyl in monoalkylamino, alkyl in dialkylamino, aryl, heteroaryl, cyclohydrocarbyl and heterocyclyl, are also optionally substituted, for example, they can also be optionally substituted by one or more groups selected from alkyl, halogen, haloalkyl, alkoxy, hydroxyl, amino, monoalkylamino, dialkylamino, nitro, aryl, heteroaryl, cyclohydrocarbyl and heterocyclyl.

In some embodiments, the "suitable substituents" are selected from: hydroxyl, halogen, C1-C4 alkoxy optionally substituted by halogen, C1-C4 alkoxycarbonyl, cyano, -S(O)₂-C1-C4 alkyl optionally substituted by halogen, C1-C4 alkyl optionally substituted by halogen, -NR'R", -S(O)₂-NR'R", -S(O)₂-NH₂, -C(O)-C1-C4 alkyl, -NR'-C(O)-C1-C4 alkyl, C3-C8 cyclohydrocarbyloxy and 6- to 14-membered aryl, 5- to 10-membered heteroaryl, C3-C8 cyclohydrocarbyl and 4- to 9-membered heterocyclyl, wherein the 6- to 14-membered aryl, 5- to 10-membered heteroaryl, C3-C8 cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, C3-C8 cyclohydrocarbyl and 4- to 9-membered heterocyclyl; wherein R' and R" are each independently selected from H and C1-C4 alkyl. In some embodiments, the "suitable substituents" are hydroxyl, amino, halogen, substituted or unsubstituted alkyl, -NR'R" and substituted or unsubstituted alkoxy, wherein R' and R" are each independently selected from H, C1-C4 alkyl, C3-C6 cycloalkyl, C1-C4 alkyl-S(O)₂- and C1-C4 haloalkyl. In some embodiments, the "suitable substituents" are selected from deuterium, hydroxyl, cyano, C1-C4 alkyl, halogen and C1-C4 alkoxy. In some embodiments, the "suitable substituents" are deuterium, hydroxyl, methyl, F and methoxy. In some embodiments, the "suitable substituents" are selected from hydroxyl, halogen, C1-C4 alkoxy, cyano and -S(O)₂-C1-C4 alkyl. In some embodiments, the "suitable substituents" are hydroxyhalogen, hydroxyl, cyano, -S(O)₂-C1-C4 alkyl, C1-C4 alkyl and C1-C4 alkoxy. In some embodiments, the "suitable substituents" are hydroxy-C1-C4 alkyl, C1-C4 alkoxy and cyano. In some embodiments, the "suitable substituents" are F, hydroxyl and cyano.

As used herein, the terms "moiety", "structural moiety", "chemical moiety", "group", "chemical group" refer to a specific fragment or functional group in a molecule. Chemical moieties are generally considered as chemical entities embedded in or attached to a molecule.

It should also be understood by those skilled in the art that in the method described below, the functional group of the intermediate compound may need to be protected by an appropriate protecting group. Such functional groups include hydroxyl, amino, amidino, guanidino, sulfhydryl and carboxyl. Suitable hydroxyl protecting groups include trialkylsilyl or diarylalkylsilyl (e.g., *tert*-butyldimethylsilyl, *tert*-butyldiphenylsilyl or trimethylsilyl), tetrahydropyranyl, benzyl, etc. Suitable protecting groups for amino, amidino and guanidino include *tert*-butoxycarbonyl, benzyloxycarbonyl, etc. Suitable sulfhydryl protecting groups include -C(O)-R^{X} (wherein R^{X} is alkyl, aryl or aralkyl), *p*-methoxybenzyl, triphenylmethyl, etc. Suitable carboxyl protecting groups include alkyl esters, aryl esters or aralkyl esters.

Protecting groups can be introduced and removed according to standard techniques known to those skilled in the art and as described herein. The use of protecting groups is detailed in Greene, T. W. and P. G. M. Wuts, Protective Groups in Organi Synthesis, (1999), 4th Ed., Wiley. The protecting group can also be a polymer resin.

As used herein, "subjects" are mammals such as primates, rats, mice, cows, horses, pigs, sheep, goats, dogs and cats, especially primates, more specifically humans. In some embodiments, "mammals" are selected from: humans; domesticated animals, such as laboratory animals, household pets or farm animals, such as cats, dogs, pigs, cows, sheep, goats, horses, rabbits; and non-domesticated animals, such as wild animals. The subject may be suspected of or at risk of suffering from cancers such as prostate cancer, breast cancer, ovarian cancer, bladder cancer, glioblastoma, melanoma, renal cell carcinoma, mantle cell lymphoma, pancreatic cancer, hepatocellular carcinoma, endometrial cancer, salivary gland cancer, or suspected of or at risk of suffering from alopecia, acne, hirsutism, ovarian cysts, polycystic ovary disease, precocious puberty, spinal and bulbar muscular atrophy, age-related macular degeneration. Those skilled in the art know the diagnosis methods for a variety of cancers such as prostate cancer, breast cancer, ovarian cancer, bladder cancer, glioblastoma, melanoma, renal cell carcinoma, mantle cell lymphoma, pancreatic cancer, hepatocellular carcinoma, endometrial cancer, salivary gland cancer, and diagnostic methods for alopecia, acne, hirsutism, ovarian cysts, polycystic ovary disease, precocious puberty, spinal and bulbar muscular atrophy or age-related macular degeneration, as well as clinical characteristics for cancers such as prostate cancer, breast cancer, ovarian cancer, bladder cancer, glioblastoma, melanoma, renal cell carcinoma, mantle cell lymphoma, pancreatic cancer, hepatocellular carcinoma, endometrial cancer, salivary gland cancer, and clinical characteristics for alopecia, acne, hirsutism, ovarian cysts, polycystic ovary disease, precocious puberty, spinal and bulbar muscular atrophy or age-related macular degeneration. In some embodiments, the subject is confirmed or diagnosed with the disease described above.

As used herein, the term "pharmaceutically acceptable" refers to a substance (e.g., a carrier or diluent) that does not affect the biological activity or properties of the active pharmaceutical ingredient (i.e., the compound or the pharmaceutically acceptable salt, enantiomer, diastereomer, tautomer, solvate, isotopic substitute, polymorph, prodrug or metabolite thereof of the present disclosure) and is relatively non-toxic, i.e., the substance can be administered to an individual without causing adverse biological reactions or interacting adversely with any component of the pharmaceutical composition. Pharmaceutically acceptable carriers or excipients include, but are not limited to, any adjuvants, carriers, excipients, flow enhancers, sweeteners, diluents, preservatives, dyes/colorants, flavor enhancers, surfactants, wetting agents, dispersants, suspending agents, stabilizers, isotonic agents, solvents or emulsifying agents that have been approved for use in humans or domesticated animals by regulatory agencies such as the Food and Drug Administration (FDA) of the United States.

The term "pharmaceutical composition" refers to a formulation containing the compound or the pharmaceutically acceptable salt, enantiomer, diastereomer, tautomer, solvate, isotopic substitute, polymorph, prodrug or metabolite thereof of the present disclosure, together with a medium generally accepted in the art for the delivery of a biologically (pharmacologically) active compound to a mammal (e.g., a human). The medium includes a pharmaceutically acceptable carrier or excipient. The purpose of the pharmaceutical composition is to facilitate the administration to living organisms, facilitate the absorption of active ingredients, and thereby exert biological (pharmacological) activity. Generally, the pharmaceutical composition of the present disclosure contains 0.1% to 99.5% by weight of the active pharmaceutical ingredient (i.e., the compound or the pharmaceutically acceptable salt, enantiomer, diastereomer, tautomer, solvate, isotopic substitute, polymorph, prodrug or metabolite thereof of the present disclosure). In some embodiments, the pharmaceutical composition of the present disclosure contains 0.5% to 90% by weight of the active pharmaceutical ingredient, such as 1%, 1.5%, 2%, 5%, 10%, 15%, 20%, 25%, 30% or 50% by weight.

The compounds of the present disclosure can exist in a number of different forms or derivatives, all of which are within the scope of the present disclosure. These forms or derivatives include, for example, tautomers, stereoisomers (such as enantiomers, diastereomers), racemic mixtures, geometric isomers, salts, prodrugs, solvates, isotopic substitutes, different crystal forms or polymorphs and metabolites (herein specifically referred to as active metabolites).

In the present disclosure, the term "pharmaceutically acceptable salts" includes pharmaceutically acceptable acid addition salts and pharmaceutically acceptable base addition salts.

"Pharmaceutically acceptable acid addition salts" refer to salts formed with inorganic or organic acids that retain the biological effectiveness of the free base without other side effects. Inorganic acid salts include, but are not limited to, hydrochloride, hydrobromide, sulfate, nitrate, phosphate, etc.; organic acid salts include, but are not limited to, formate, acetate, 2,2-dichloroacetate, trifluoroacetate, propionate, caproate, caprylate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalene disulfonate, etc. These salts can be prepared by methods known in the art.

"Pharmaceutically acceptable base addition salts" refer to salts formed with inorganic or organic bases that retain the biological effectiveness of the free acid without other side effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts. Salts derived from organic bases include, but are not limited to, the following salts: primary, secondary and tertiary amines, substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resin, etc. Preferred organic bases include isopropylamine, diethylamine, ethanolamine, trimethylamine, dicyclohexylamine, choline and caffeine. These salts can be prepared by methods known in the art.

As used herein, the term "prodrug" refers to those compounds that will be metabolized (i.e., converted *in vivo*) into compounds of the present disclosure with pharmacological activity after administration. When the compound of the present disclosure itself is difficult to absorb from the gastrointestinal tract, its bioavailability can be improved by making it into a prodrug. Examples of prodrugs of the compounds of the present disclosure can include simple esters of carboxyl-containing compounds (e.g., esters obtained by condensation with C₁₋₄ alcohols according to methods known in the art); esters of hydroxyl-containing compounds (e.g., esters obtained by condensation with C₁₋₄ monocarboxylic acids, C₃₋₆ dicarboxylic acids or anhydrides thereof such as succinic anhydride or fumaric anhydride according to methods known in the art); imines of amino-containing compounds (e.g., imines obtained by condensation with C₁₋₄ aldehydes or ketones according to methods known in the art); carbamates of amino-containing compounds, such as those described by Leu et al. (J. Med. Chem., 42:3623-3628(1999)) and Greenwald et al. (J. Med. Chem., 42:3657-3667(1999)); aldehyde acetals or ketone acetals of hydroxyl-containing compounds (e.g., those acetals obtained by condensation with chloromethyl methyl ether or chloromethyl ethyl ether according to methods known in the art).

As used herein, the term "solvate" refers to an aggregate comprising one or more molecules of the compound of the present disclosure and one or more solvent molecules. The solvent can be water, in which case the solvate can be a hydrate. Alternatively, the solvent can be an organic solvent. Accordingly, the compounds of the present disclosure can exist as hydrates, including monohydrate, dihydrate, hemihydrate, sesquihydrate, trihydrate, tetrahydrate, etc., as well as the corresponding solvated forms. The compounds of the present disclosure can be true solvates, while in other cases, the compounds of the present disclosure can remain merely amorphous water or a mixture of water plus some indefinite solvent.

Herein, "stereoisomer" refers to a compound composed of the same atoms, bonded by the same bonds, but having different three-dimensional structures. The present disclosure will cover various stereoisomers and mixtures thereof.

When the compounds of the present disclosure contain an alkene double bond, the compounds of the present disclosure are intended to contain E- and Z- geometric isomers unless otherwise indicated.

"Tautomer" refers to an isomer formed by the transfer of a proton from one atom of a molecule to another atom of the same molecule. All tautomeric forms of the compounds of the present disclosure are also intended to be included within the scope of the present disclosure.

The compounds or pharmaceutically acceptable salts thereof of the present disclosure may contain one or more chiral carbon atoms, and thus may produce enantiomeric, diastereomeric and other stereoisomeric forms. Each chiral carbon atom can be defined as (R)- or (S)- based on stereochemistry. The present disclosure is intended to include all possible isomers, as well as their racemic and optically pure forms. The compounds of the present disclosure can be prepared by selecting racemates, diastereomers or enantiomers as raw materials or intermediates. Optically active isomers can be prepared using chiral synthons or chiral reagents, or resolved using conventional techniques such as crystallization and chiral chromatography.

Conventional techniques for the preparation/separation of individual isomers include chiral synthesis from suitable optically pure precursors or the resolution of racemates (or racemates of salts or derivatives) using methods like chiral high-performance liquid chromatography, see, for example, GeRald Gübitz and Martin G. Schmid (Eds.), ChiRal SepaRations, Methods and Protocols, Methods in Molecular Biology, Vol. 243, 2004; A.M. Stalcup, ChiRal SepaRations, Annu. Rev. Anal. Chem. 3:341-63, 2010; Fumiss et al.(eds.), VOGEL'S ENCYCLOPEDIA OF PRaCTICAL ORGANIC CHEMISTRY.sup.TH ED., Longman Scientific and Technical Ltd., Essex, 1991, 809-816; Heller, Acc. Chem. Res. 1990, 23, 128, and the disclosure of which is incorporated herein by reference in its entirety.

The present disclosure also includes all suitable isotopic substitutes (or isotopic variants) of the compounds or pharmaceutically acceptable salts thereof of the present disclosure. Isotopic variants of the compounds or pharmaceutically acceptable salts thereof of the present disclosure are defined as those in which at least one atom has been replaced by an atom having the same atomic number but an atomic mass different from that typically found in nature. Isotopes that can be incorporated into the compounds and pharmaceutically acceptable salts thereof of the present disclosure include, but are not limited to, isotopes of H, C, N and O, such as ²H, ³H, ¹¹C, ¹³C, ¹⁴C, ¹⁵N, ¹⁷O, ¹⁸O, ³⁵S, ¹⁸F, ³⁶Cl and ¹²⁵I. Isotopic variants of the compounds or pharmaceutically acceptable salts thereof described in the present disclosure can be prepared by conventional techniques using appropriate isotopic variants of suitable reagents.

Herein, unless otherwise specified, the wavy lines on each structural formula or group generally indicate the position where the structural formula or group is connected to other parts of the compound.

As used herein, the terms "crystalline form", "crystal form" and "polymorph" are used interchangeably and refer to crystal structures in which compounds (or salts, solvates or other derivatives thereof such as prodrugs or metabolites) can crystallize in different crystal stacking arrangements (all with the same elemental composition). Different crystal forms typically have different X-ray diffraction patterns, infrared spectra, melting points, density hardness, crystal shape, optical and electrical properties, stability and solubility. Recrystallization solvent, crystallization rate, storage temperature or other factors may lead to the dominance of one of these crystalline forms. Polymorphs of compounds can be prepared by crystallization under different conditions.

As used herein, the terms "metabolite" and "active metabolite" can be used interchangeably, and refer to derivatives with the same pharmacological activity produced by the metabolic process of the active ingredient of the parent drug in a subject. For example, such metabolites can be produced by oxidation, reduction, hydrolysis, amidation, deamidation, esterification, deesterification, enzymatic cleavage, etc. of administered compounds or salts or prodrugs.

As used herein, "prevention" refers to preventing the occurrence of the disease or condition in a mammal (preferably a human), in particular when the mammal is susceptible to the condition but has not yet been diagnosed with the condition, or preventing the recurrence of a cured disease or condition in a mammal (preferably a human).

As used herein, "treatment" encompasses the treatment of a disease or condition of interest in a mammal (preferably a human) suffering from the disease or condition of interest, including one or more of the following aspects: (i) inhibiting the disease or condition, i.e., curbing its development; (ii) alleviating the disease or condition, i.e., causing the disease or condition to subside; and (iii) alleviating the symptoms caused by the disease or condition, i.e., alleviating pain without addressing the underlying disease or condition.

"Effective amount" refers to a therapeutically effective amount or a prophylactically effective amount. "Therapeutically effective amount" refers to the amount that effectively achieves the required therapeutic result (alleviating one or more symptoms of the treated disease or condition, such as reducing tumor size, increasing lifespan or enhancing life expectancy to some extent) at the necessary dosage and for the necessary time period. The therapeutically effective amount of a compound may vary according to the following factors: the disease state, age, sex and weight of the subject, and the ability of the compound to elicit the desired response in the subject. The dosage regimen can be adjusted to provide the optimal therapeutic response. A therapeutically effective amount is also an amount in which any toxic or detrimental effects of the compound are outweighed by therapeutically beneficial effects. "Prophylactically effective amount" refers to the amount that effectively achieves the required preventive results (such as preventing the occurrence of diseases in subjects at risk of suffering from diseases and the recurrence of diseases in cured sick subjects) at the necessary dosage and for the necessary time period. Typically, a prophylactic dose is administered to a subject before or during the early stages of the disease such that the prophylactically effective amount can be less than the therapeutically effective amount. Techniques such as dose-escalation tests can be used to determine the effective amount appropriate in any individual case.

As used herein, the terms "taking", "administering", "administrating" and the like refer to methods capable of delivering a compound or pharmaceutical composition to a desired site for biological action. Any method of administration well known in the art can be used in the present disclosure. These methods include, but are not limited to, oral administration, transduodenal administration, parenteral administration (including intrapulmonary, intranasal administration; intrathecal, intravenous, subcutaneous, intraperitoneal, intramuscular, intraarterial injection or infusion), topical administration and transrectal administration. Those skilled in the art are familiar with administration techniques that can be used for the compounds and methods described herein, such as those discussed in Goodman and Gilman, The Pharmacological Basis of Therapeutics, current ed.; Pergamon; and Remington's, Pharmaceutical Sciences (current edition), Mack Publishing Co., Easton, Pa. In preferred embodiments, the compounds, the pharmaceutically acceptable salts, enantiomers, diastereomers, tautomers, solvates, isotopic substitutes, polymorphs, prodrugs or metabolites thereof, or the pharmaceutical compositions thereof of the present disclosure are administered by oral administration.

Herein, "combination", "drug combination", "combination administration" or "combination therapy" refers to drug treatment obtained by mixing or combining more than one active ingredient, which includes fixed and unfixed combinations of active ingredients, and also includes the combination of two or more different treatment methods. The term "fixed combination" refers to the simultaneous administration to a patient of at least one compound described herein and at least one other active ingredient in the form of a single entity or a single dosage form. The term "unfixed combination" refers to the simultaneous administration, combined administration or sequential administration at variable intervals of at least one compound described herein and at least one other active ingredient to a patient in the form of a single entity. These are also applied to cocktail therapy, such as the administration of three or more active ingredients.

For the avoidance of doubt, in the present disclosure, compounds drawn with wedge bonds (e.g., compound 67) are compounds of a single configuration determined by the absolute stereochemical structure.

### II. Compound

In one aspect, the present disclosure provides a compound of the following formula I or a pharmaceutically acceptable salt, enantiomer, diastereomer, tautomer, solvate, isotopic substitute, polymorph, prodrug or metabolite thereof: 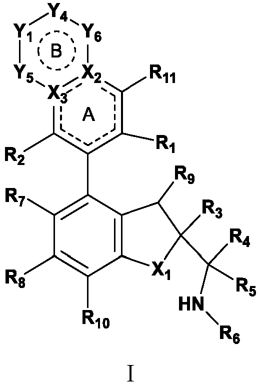 in the formula:
X₁ is selected from: O and NRₐ;
X₂ is selected from: C and N;
X₃ is selected from: C and N;
Y₅ and Y₆ are independently selected from: a bond, O, S, N, -S(O)-, -S(O)₂-, C, CH(R₁₃), C(R₁₃)₂, C(R₁₃), -OCH₂-, -N(R₁₂)CH₂-, -CH₂CH₂- and N(R₁₂), and Y₅ and Y₆ are not simultaneously a bond;
Y₁ and Y₄ are independently selected from: a bond, O, N, C, S, -S(O)-, -C(O)-, -S(O)₂-, CH, CH₂, NH, C(R₁₃), CH(R₁₃), C(R₁₃)₂ and N(R₁₂), provided that Y₁ and Y₄, Y₁ and Y₅, Y₄ and Y₆ are not simultaneously a bond, O, S, -S(O)- or -S(O)₂- and satisfy the valence bond theory;
alternatively, when Y₁, Y₄ and Y₆ are arbitrarily selected from C(R₁₃)₂, two R₁₃ on the same carbon atom, together with the C atom to which they are attached, can form a substituted or unsubstituted 3- to 8-membered cyclohydrocarbyl or substituted or unsubstituted 4- to 9-membered heterocyclyl ring, thereby forming a spiro ring structure with ring B;
alternatively, the ring atom in Y₁ or Y₆ and the ring atom in Y₄, together with the substituent attached to the ring atom, form a substituted or unsubstituted 3- to 8-membered cyclohydrocarbyl, substituted or unsubstituted 4- to 9-membered heterocyclyl, substituted or unsubstituted 6- to 10-membered aryl or substituted or unsubstituted 5- to 10-membered heteroaryl ring, thereby forming a fused ring structure with ring B;
alternatively, X₂, together with the ring atom in Y₆ and the ring atom attached to R₁₁ and the substituent attached to the ring atom, forms a substituted or unsubstituted 5- or 6-membered cyclohydrocarbyl, substituted or unsubstituted 5- or 6-membered heterocyclyl, substituted or unsubstituted phenyl or substituted or unsubstituted 5- or 6-membered heteroaryl ring, thereby forming a three-fused ring structure with rings A and B;
ring B is a 5-, 6- or 7-membered heterocyclyl ring, a benzene ring or a 5-, 6- or 7-membered heteroaryl ring; ring B can be optionally substituted by one or more R₁₃ and/or R₁₂ that do not participate in ring formation, and can be further optionally substituted by 1, 2 or 3 substituents selected from hydroxyl, amino, halogen, substituted or unsubstituted alkyl, -NR'R" and substituted or unsubstituted alkoxy;
R₁ is selected from: cyano and -C(O)NR'R";
R₂ is selected from: H, halogen, substituted or unsubstituted alkyl and substituted or unsubstituted alkoxy;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₇ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl;
R₁₁ is selected from: H, hydroxyl, amino, halogen, substituted or unsubstituted alkyl, - NR'R" and substituted or unsubstituted alkoxy;
each R₁₂ that does not participate in ring formation is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 6- to 14-membered aryl-S(O)₂-, substituted or unsubstituted 4- to 9-membered heterocyclyl, NR'R"-C(O)-, substituted or unsubstituted 3- to 8-membered cyclohydrocarbyl-C(O)- and substituted or unsubstituted C1-C4 alkyl;
each R₁₃ that does not participate in ring formation is independently selected from: H, halogen, substituted or unsubstituted C1-C4 acyl, hydroxyl, cyano, -NR'R", substituted or unsubstituted C1-C4 alkoxy, substituted or unsubstituted C1-6 alkyl, substituted or unsubstituted C1-C4 alkyl-S(O)₂-, substituted or unsubstituted C6-14 aryl-S(O)₂-, substituted or unsubstituted 3- to 6-membered cyclohydrocarbyl-C(O)- and NR'R"-C(O)-, or two R₁₃ on the same carbon atom form an oxo (=O) or thio (=S) group;
after R₁₃ or R₁₂ participates in ring formation, its ring atom can be optionally substituted by 1 to 3 heteroatoms selected from O, N and S;
R' and R" are each independently selected from H, C1-C4 alkyl, C3-C6 cycloalkyl, C1-C4 alkyl-S(O)₂- and C1-C4 haloalkyl;
Rₐ is selected from H and C1-C4 alkyl;
wherein the dashed line indicates the presence or absence of a double bond.

In some embodiments, R' and R" are each independently selected from H, C1-C4 alkyl, C1-C4 alkyl-S(O)₂- and C1-C4 haloalkyl.

In some embodiments, the compound of formula I has a structure shown in the following formula Ia:

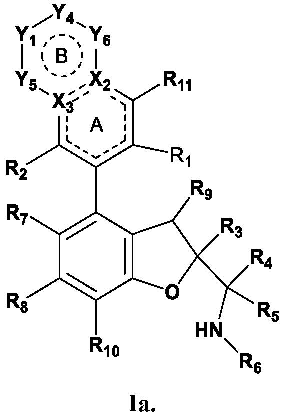

In some embodiments, the compound of formula I has a structure shown in the following formula Ib : 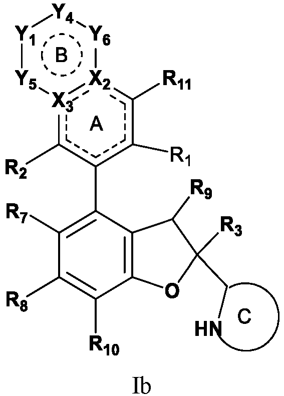 in the formula,
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, ring A is a benzene ring or a 6-membered heteroaryl ring.

In some embodiments, ring A is a benzene ring or a pyridine ring.

In some embodiments, the compound of formula I has a structure shown in the following formula IIa: 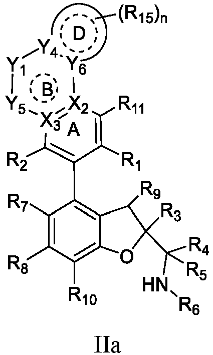 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

In some embodiments, X₂ is selected from: C and N;
X₃ is selected from: C and N;
Y₁ is selected from: a bond, O, NH, -N(CH₃)-, CH₂ and -CH(CH₃)-;
Y₅ is selected from: O, S, N, CH₂, CH, NH, -N(CH₃)-, -OCH₂-, -NHCH₂-, -N(CH₃)CH₂- and -CH₂CH₂-;
Y₄ is selected from: C, CH or N;
Y₆ is selected from: C, CH or N;
R₁₅ is selected from: H, fluorine, chlorine, acetyl, hydroxyl, C1-C4 alkoxy, cyano, - NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl;
n is 0 or 1;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.

In some embodiments, R' and R" are each independently selected from H, C1-C4 alkyl and C3-C6 cycloalkyl.

In some embodiments, R₁ is selected from: cyano, -C(O)NH₂ and -C(O)NHCH₃; R₂ is selected from: H, fluorine and methoxy;
R₃ is phenyl;
R₄ and R₅ are each independently selected from: Hand C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 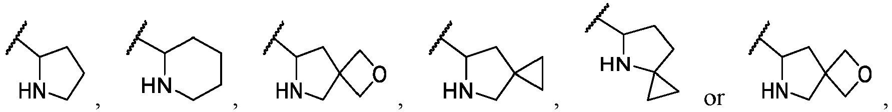 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, R₁ is selected from: cyano, -C(O)NH₂, -C(O)NHCH₃ and

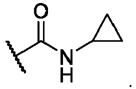

In some embodiments, the compound of formula I has a structure shown in the following formula lib: 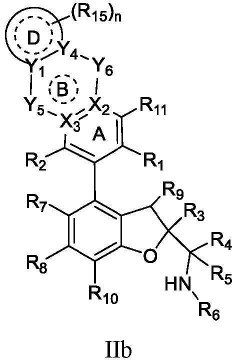 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

In some embodiments, X₂ and X₃ are C;
Y₁ is selected from: C, CH and N;
Y₄ is selected from: C, CH and N;
Y₅ is selected from: a bond, O, S, -S(O)-, -S(O)₂-, -C(O)-, N, NH, -N(CH₃)-, CH₂, - CH(CH₃)-, -OCH₂-, -NHCH₂-, -N(CH₃)CH₂- and -CH₂CH₂-;
Y₆ is selected from: a bond, O, S, -S(O)-, -S(O)₂-, -C(O)-, N, CH₂, CH, NH, -N(CH₃)-, - OCH₂-, -NHCH₂-, -N(CH₃)CH₂- and -CH₂CH₂-; and Y₅ and Y₆ are not simultaneously a bond;
R₁₅ is selected from: H, fluorine, chlorine, acetyl, hydroxyl, C1-C4 alkoxy, cyano, - NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl;
n is 0 or 1;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.

In some embodiments, R₁ is selected from: cyano, -C(O)NH₂ and -C(O)NHCH₃;
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 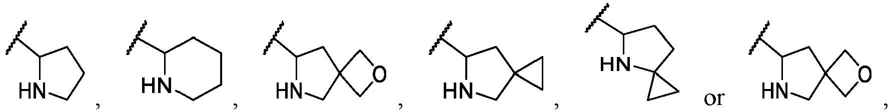 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, ring D is a 3- to 6-membered cyclohydrocarbyl, 4- to 6-membered heterocyclyl, 6- to 10-membered aryl or 5- to 8-membered heteroaryl ring, wherein the 4- to 6-membered heterocyclyl and 5- to 8-membered heteroaryl contain 1, 2 or 3 heteroatoms selected from O, N and S; R₁₅ is selected from: H, halogen, substituted or unsubstituted C1-C4 acyl, hydroxyl, substituted or unsubstituted C1-C4 alkoxy, cyano, -NR'R", substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; R' and R" are each independently selected from H and C1-C4 alkyl; n is 0, 1 or 2.

In some embodiments, R₁ is selected from: cyano, -C(O)NH₂, -C(O)NHCH₃ and 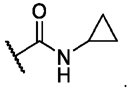 In some embodiments, R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl; n is 0 or 1.

In some embodiments, R' and R" are each independently selected from H, C1-C4 alkyl and C3-C6 cycloalkyl.

In some embodiments, the compound of formula I has a structure shown in the following formula IIc: 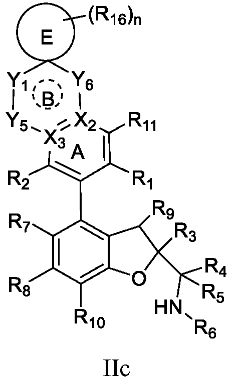 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

In some embodiments, X₂ and X₃ are C;
Y₁ is selected from: O or CH₂;
Y₅ is selected from: a bond, O, NH, -N(CH₃)-, CH₂ and -CH(CH₃)-;
Y₆ is selected from: a bond, O, S, N, CH₂, CH, NH and -N(CH₃)-;
R₁₆ is selected from halogen, C1-C4 alkyl, hydroxyl, C1-C4 acyl and C1-C4 alkoxy;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.

In some embodiments, R₁ is selected from: cyano, -C(O)NH₂ and -C(O)NHCH₃;
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 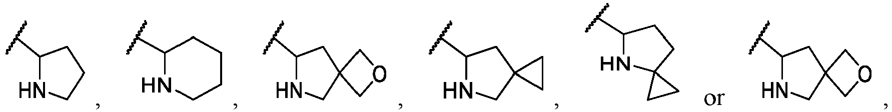 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, R' and R" are each independently selected from H, C1-C4 alkyl and C3-C6 cycloalkyl.

In some embodiments, R₁ is selected from: cyano, -C(O)NH₂, -C(O)NHCH₃ and

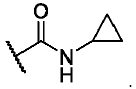

In some embodiments, the compound of formula I has a structure shown in the following formula IId: 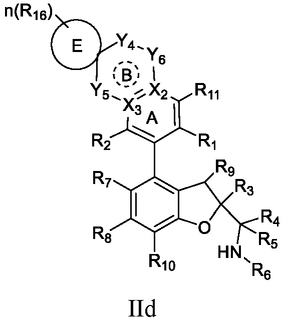 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

In some embodiments, X₂ and X₃ are C;
Y₄ is selected from: O or CH₂;
Y₅ is selected from: a bond, O, NH, -N(CH₃)-, CH₂ and -CH(CH₃)-;
Y₆ is selected from: a bond, O, S, N, CH₂, CH, NH and -N(CH₃)-;
R₁₆ is selected from halogen, C1-C4 alkyl, hydroxyl, C1-C4 acyl and C1-C4 alkoxy;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.

In some embodiments, R₁ is selected from: cyano, -C(O)NH₂ and -C(O)NHCH₃;
R₂ is selected from: H, fluorine and methoxy;
R₃ is phenyl;
R₄ and R₅ are each independently selected from: Hand C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 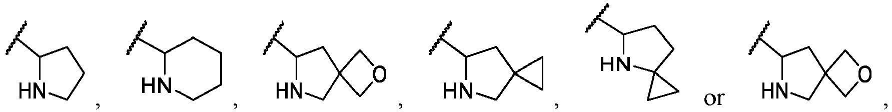 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, ring E is a 3- to 6-membered cyclohydrocarbyl or 4- to 6-membered heterocyclyl ring, wherein the 4- to 6-membered heterocyclyl contains 1, 2 or 3 heteroatoms selected from O, N and S; R₁₆ is selected from H, halogen, C1-C4 alkyl, hydroxyl and acetyl; n is 0 or 1.

In some embodiments, ring E is a cyclopropyl, cyclobutyl, cyclopentyl, cyclohexyl, oxetanyl, oxolanyl, tetrahydropyranyl, azetidinyl, azacyclopentyl or piperidinyl ring; R₁₆ is selected from H, fluorine, methyl, ethyl, hydroxyl and acetyl; n is 0 or 1.

In some embodiments, the compound of formula I has a structure shown in the following formula IIe: 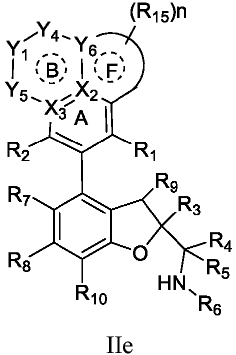 in the formula,
X₂ is C;
Y₆ is selected from: C, CH and N;
ring F is a 5- or 6-membered cyclohydrocarbyl, 5- or 6-membered heterocyclyl, phenyl or 5- or 6-membered heteroaryl ring, wherein the 5- or 6-membered heterocyclyl or 5- or 6-membered heteroaryl contains 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; and
n is 0, 1, 2 or 3.

In some embodiments, ring F is a phenyl or 5- or 6-membered heteroaryl ring, wherein the 5- or 6-membered heteroaryl contains 1, 2 or 3 heteroatoms selected from O, N and S; and/or R₁₅ is selected from: H, halogen, substituted or unsubstituted C1-C4 acyl, hydroxyl, substituted or unsubstituted C1-C4 alkoxy, cyano, -NR'R", substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl. In some embodiments, the C1-C4 acyl, C1-C4 alkoxy, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; wherein R' and R" are each independently selected from H and C1-C4 alkyl, and n is 0, 1 or 2.

In some embodiments, ring F is a 5-membered heteroaryl ring, wherein the 5-membered heteroaryl contains 1 or 2 heteroatoms selected from O and N; and/or R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; wherein R' and R" are each independently selected from H and C1-C4 alkyl. In some embodiments, n is 0 or 1.

In some embodiments, ring F is a pyrrolyl, furyl, pyrazolyl, imidazolyl, oxazolyl or isoxazolyl ring.

In some embodiments, Y₁, Y₄ and Y₅ are independently selected from: O, CH, CH₂, - CH(CH₃)-, N, NH and -N(CH₃)-;
ring F is a phenyl or 5- or 6-membered heteroaryl ring, wherein the 5- or 6-membered heteroaryl contains 1, 2 or 3 heteroatoms selected from O, N and S; R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl; n is 0 or 1;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.
In some embodiments, R₁ is selected from: cyano, -C(O)NH₂ and -C(O)NHCH₃;
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 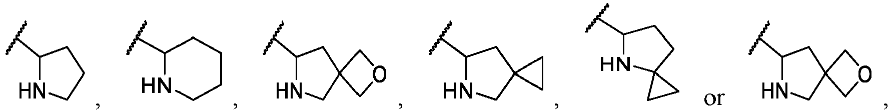 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, R' and R" are each independently selected from H, C1-C4 alkyl and C3-C6 cycloalkyl.

In some embodiments, R₁ is selected from: cyano, -C(O)NH₂, -C(O)NHCH₃ and

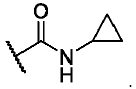

In some embodiments, the compound of formula IIa has a structure shown in the following formula IIIa: 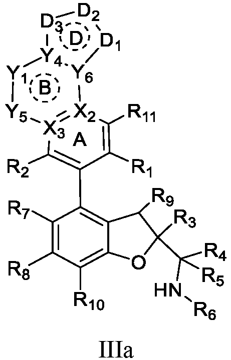 in the formula,
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRe, N-NRₑ, CR_{f}-NRₑ, 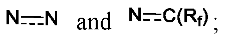 C(R_{f}).
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group; and
each p is independently 1, 2, 3 or 4, preferably 1 or 2.

In some embodiments, X₂ is selected from: C and N;
X₃ is selected from: C and N;
Y₁ is selected from: a bond, O, NH, -N(CH₃)-, CH₂ and -CH(CH₃)-;
Y₅ is selected from: O, S, N, CH₂, CH, NH, -N(CH₃)-, -OCH₂-, -NHCH₂-, -N(CH₃)CH₂- and -CH₂CH₂-;
Y₄ is selected from: C, CH or N;
Y₆ is selected from: C, CH or N;
D₁ is selected from: O, S, N, NRₑ, CR_{f} and CR_{f}R_{g};
D₂ is selected from: O, S, N, NRₑ, (CR_{f})ₚ and (CR_{f}R_{g})ₚ;
D₃ is selected from: O, S, N, NRₑ, (CR_{f})ₚ and (CR_{f}R_{g})ₚ;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.
In some embodiments, R₁ is selected from: cyano, -C(O)NH₂ and -C(O)NHCH₃;
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 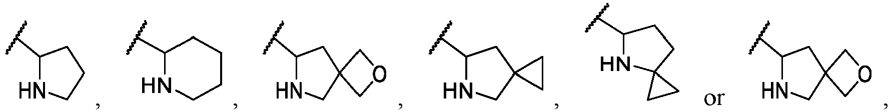 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, R₁ is selected from: cyano, -C(O)NH₂, -C(O)NHCH₃ and

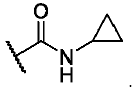

In some embodiments, R' and R" are each independently selected from H, C1-C4 alkyl and C3-C6 cycloalkyl.

In some embodiments, the compound of formula IIb has a structure shown in the following formula IIIb: 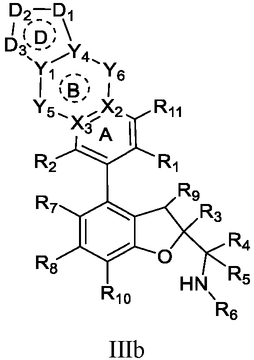 in the formula,
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRe, N-NRₑ, CR_{f}-NRₑ, 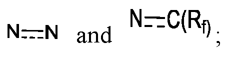
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group; and
each p is independently 1, 2, 3 or 4, preferably 1 or 2.

In some embodiments, X₂ and X₃ are C;
Y₁ is selected from: C, CH and N;
Y₄ is selected from: C, CH and N;
Y₅ is selected from: a bond, O, S, -S(O)-, -S(O)₂-, -C(O)-, N, NH, -N(CH₃)-, CH, CH₂, - CH(CH₃)-, -OCH₂-, -NHCH₂-, -N(CH₃)CH₂- and -CH₂CH₂-;
Y₆ is selected from: a bond, O, S, -S(O)-, -S(O)₂-, -C(O)-, N, CH₂, CH, -CH(CH₃)-, NH, -N(CH₃)-, -OCH₂-, -NHCH₂-, -N(CH₃)CH₂- and -CH₂CH₂-; and Y₅ and Y₆ are not simultaneously a bond;
D₁ is selected from: O, S, N, NRₑ, CR_{f} and CR_{f}R_{g};
D₂ is selected from: O, S, N, NRₑ, (CR_{f})ₚ, (CR_{f}R_{g})ₚ, C(O)-NRₑ, N-NRₑ, CR_{f}-NRₑ, 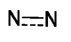 and 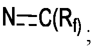
D₃ is selected from: O, S, N, NRₑ, (CR_{f})ₚ and (CR_{f}R_{g})ₚ;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.

In some embodiments, R₁ is selected from: cyano, -C(O)NH₂ and -C(O)NHCH₃;
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 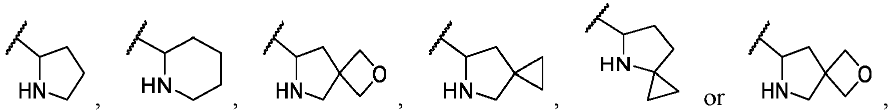 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and - NH(CH₃).

In some embodiments, R₁ is selected from: cyano, -C(O)NH₂, -C(O)NHCH₃ and

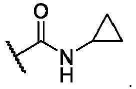

In some embodiments, R' and R" are each independently selected from H, C1-C4 alkyl and C3-C6 cycloalkyl.

In some embodiments, the compound of formula IIIa has a structure shown in the following formula IVa: 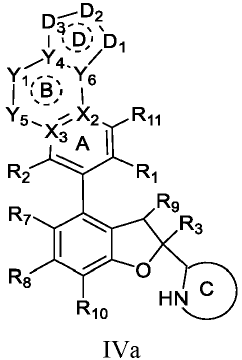 in the formula,
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, the compound of formula IIIb has a structure shown in the following formula IVb: 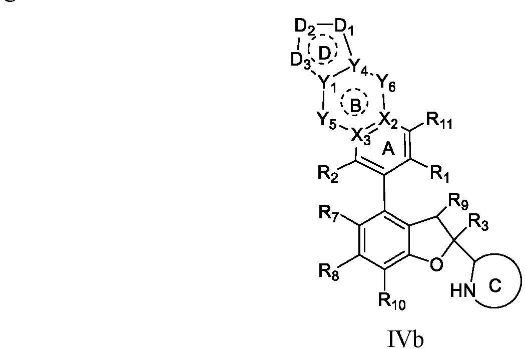 in the formula,
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, the compound of formula IIe has a structure shown in the following formula IVc: 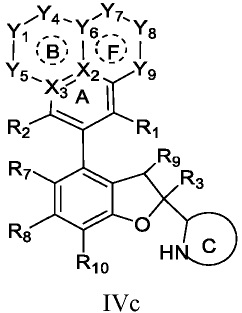 in the formula,
Y₇, Y₈ and Y₉ are independently selected from: a bond, O, S, S(O), S(O)₂, N, NRₑ, CR_{f} and CR_{f}R_{g}, provided that at most one of Y₇, Y₈ and Y₉ is a bond, Y₇ and Y₈, Y₈ and Y₉ are not simultaneously O, S, -S(O)- or -S(O)₂- and satisfy the valence bond theory,
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, ring C is a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring, and the 4- to 8-membered nitrogen-containing monocyclicheterocyclyl, 6- to 12-membered nitrogen-containing spiro heterocyclyl and 6- to 12-membered nitrogen-containing fused heterocyclyl each optionally further contain 1 or 2 heteroatoms selected from N and O.

In some embodiments, ring C is a 4- to 9-membered monocyclic or fused heterocyclyl ring optionally substituted by 1, 2 or 3 substituents selected from H, deuterium, hydroxyl, cyano, C1-C4 alkyl, halogen and C1-C4 alkoxy.

In some embodiments, ring C is the following groups, which are optionally substituted by 1, 2 or 3 substituents selected from H, deuterium, hydroxyl, methyl, F and methoxy; 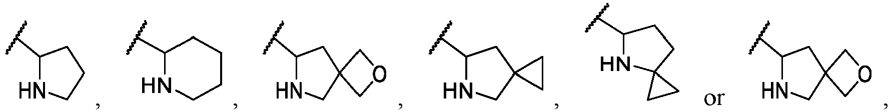 wherein the wavy line indicates the bond connection position.

In some embodiments, ring C is 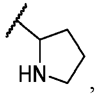 which is optionally substituted by 1, 2 or 3 substituents selected from H, deuterium, hydroxyl, methyl, F and methoxy.

In some embodiments, R₁ is selected from: cyano and -C(=O)NR'R"; the R' and R" are each independently selected from H, C1-C4 alkyl, C1-C4 alkyl-S(O)₂- and C1-C4 haloalkyl.

In some embodiments, R₁ is selected from: cyano and -C(=O)NR'R"; the R' and R" are each independently selected from H, C1-C4 alkyl, C3-C6 cycloalkyl, C1-C4 alkyl-S(O)₂- and C1-C4 haloalkyl.

In some embodiments, R₁ is selected from: cyano, -C(=O)NH₂ and -C(=O)NHCH₃.

In some embodiments, R₁ is selected from: cyano, -C(=O)NH₂, -C(=O)NHCH₃ and

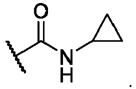

In some embodiments, R₁ is -C(O)NR'R".

In some embodiments, R₁ is -C(O)NHR'; the R' is selected from H and C1-C4 alkyl.

In some embodiments, R₁ is -C(O)NHR'; the R' is selected from H, C1-C4 alkyl and C3-C6 cycloalkyl.

In some embodiments, R₁ is -C(O)NH₂.

In some embodiments, R₁ is -C(O)NHCH₃.

In some embodiments, R₂ is selected from: H, halogen, substituted or unsubstituted C1-C4 alkyl and substituted or unsubstituted C1-C4 alkoxy.

In some embodiments, R₂ is selected from H, halogen, C1-C4 alkyl and C1-C4 alkoxy.

In some embodiments, R₂ is selected from H, halogen, C1-C3 alkyl and C1-C3 alkoxy.

In some embodiments, R₂ is selected from H, halogen and methoxy.

In some embodiments, R₂ is halogen.

In some embodiments, R₂ is selected from fluorine and chlorine.

In some embodiments, R₂ is fluorine.

In some embodiments, R₃ is selected from phenyl or heteroaryl, and the phenyl and heteroaryl are unsubstituted or substituted by 1 to 3 substituents selected from hydroxyl, halogen, C1-C4 alkyl, C1-C4 haloalkyl and C1-C4 alkoxy.

In some embodiments, R₃ is selected from substituted or unsubstituted aryl and substituted or unsubstituted heteroaryl.

In some embodiments, R₃ is selected from substituted or unsubstituted phenyl and substituted or unsubstituted pyridyl.

In some embodiments, R₃ is phenyl, fluorophenyl or pyridyl.

In some embodiments, R₃ is phenyl.

In some embodiments, R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S.

In some embodiments, R₄ and R₅ are each independently selected from: H and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S.

In some embodiments, R₄ and R₅ are each independently selected from: H, substituted or unsubstituted C1-C3 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S.

In some embodiments, R₄ and R₅ are each independently selected from: H and substituted or unsubstituted C1-C4 alkyl.

In some embodiments, R₄ and R₅ are each independently selected from: H and substituted or unsubstituted C1-C3 alkyl.

In some embodiments, R₄ and R₅ are each independently selected from: H and C1-C3 alkyl.

In some embodiments, R₄ and R₅ are each independently selected from: H and methyl.

In some embodiments, R₄ and R₅ are both H.

In some embodiments, R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl.

In some embodiments, R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or

R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or

R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl.

In some embodiments, R₆ is selected from: H; C1-C4 alkyl, which is optionally substituted by 1, 2 or 3 substituents selected from hydroxyl, halogen, C1-C4 alkoxy, cyano and C1-C4 alkyl-S(O)₂-; 3- to 8-membered cyclohydrocarbyl, which is optionally substituted by 1, 2 or 3 substituents selected from halogen, hydroxyl, cyano, C1-C4 alkyl-S(O)₂-, C1-C4 alkyl and C1-C4 alkoxy; 4- to 9-membered heterocyclyl, which is optionally substituted by 1, 2 or 3 substituents selected from halogen, hydroxyl, cyano, C1-C4 alkyl-S(O)₂-, C1-C4 alkyl and C1-C4 alkoxy; and 6- to 12-membered bridged cyclyl, 6- to 12-membered spiro cyclyl, 6- to 12-membered bridged heterocyclyl or 6- to 12-membered spiro heterocyclyl, each of which is optionally substituted by 1, 2 or 3 substituents selected from C1-C4 alkyl, C1-C4 alkoxy and cyano.

In some embodiments, R₆ is -L-W-Z, wherein L is a bond or substituted or unsubstituted C1-C4 alkyl; W is a bond, substituted or unsubstituted C1-C4 alkyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl; Z is hydrogen, halogen, C1-C4 alkyl, C1-C4 alkoxy, hydroxyl, -S(O)₂-C1-C4 alkyl, -S(O)₂-NR'R", -S(O)₂-NH₂, cyano, -C(O)-C1-C4 alkyl, -O-3- to 8-membered cyclohydrocarbyl or -NR'-C(O)-C 1-C4 alkyl, wherein R' and R" are each independently H or C1-C4 alkyl.

In some embodiments, the -L-W-Z is selected from: 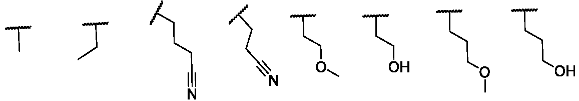 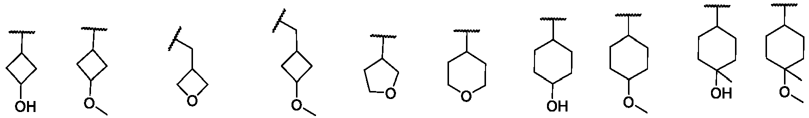 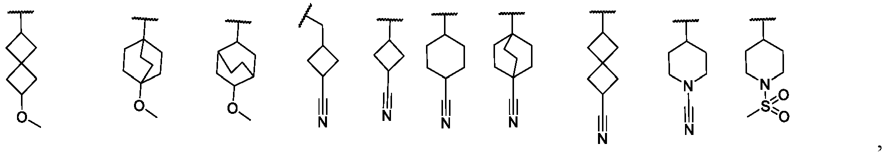 wherein the wavy line indicates the bond connection position.

In some embodiments, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy.

In some embodiments, R₄ or R₅ and R₆, together with the atom to which they are attached, form the following group optionally substituted by 1, 2 or 3 substituents selected from H, deuterium, hydroxyl, methyl, F and methoxy: 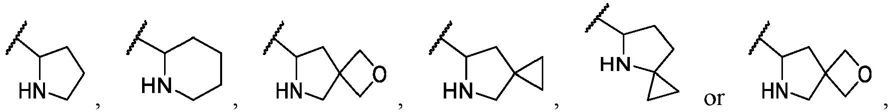 wherein the wavy line indicates the bond connection position.

In some embodiments, R₄ or R₅ and R₆, together with the atom to which they are attached, form 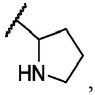 which is optionally substituted by 1, 2 or 3 substituents selected from H, deuterium, hydroxyl, methyl, F and methoxy.

In some embodiments, R₇ and R₈ are each independently selected from: H, halogen, cyano, substituted or unsubstituted C1-C4 alkyl, substituted or unsubstituted C1-C4 haloalkyl, substituted or unsubstituted C1-C4 alkoxy, substituted or unsubstituted C2-C4 alkenyl and substituted or unsubstituted C2-C4 alkynyl.

In some embodiments, R₇ and R₈ are each independently selected from: H, halogen, cyano, substituted or unsubstituted C1-C3 alkyl, substituted or unsubstituted C1-C3 haloalkyl, substituted or unsubstituted C1-C3 alkoxy, substituted or unsubstituted C2-C4 alkenyl and substituted or unsubstituted C2-C4 alkynyl.

In some embodiments, R₇ and R₈ are each independently selected from: H, halogen, cyano, methyl and methoxy.

In some embodiments, R₇ and R₈ are each independently selected from: fluorine and chlorine.

In some embodiments, R₇ is chlorine, R₈ is fluorine.

In some embodiments, R₉ is selected from H, C1-C4 alkyl, C1-C4 alkoxy, hydroxyl and halogen.

In some embodiments, R₉ is selected from H, C1-C3 alkyl, C1-C3 alkoxy, hydroxyl and halogen.

In some embodiments, R₉ is selected from H, methyl, methoxy, hydroxyl and F.

In some embodiments, R₉ is H.

In some embodiments, R₁₀ is selected from: H, halogen and C1-C4 alkyl.

In some embodiments, R₁₀ is selected from: H, halogen and C1-C3 alkyl.

In some embodiments, R₁₀ is selected from: H, F and methyl.

In some embodiments, R₁₀ is H.

In some embodiments, Rn is selected from: H, hydroxyl, amino, halogen, C1-C3 alkyl, - NR'R" and C1-C3 alkoxy, wherein R' and R" are independently selected from C1-C4 alkyl.

In some embodiments, Rn is selected from: H, hydroxyl, amino, fluorine, chlorine, methyl, methoxy and -NH(CH₃).

In some embodiments, Rn is H.

In some embodiments, when R₁₂, which does not participate in ring formation, is present, each R₁₂ is independently selected from: H, substituted or unsubstituted C1-C4 acyl, substituted or unsubstituted C1-C4 alkoxy, substituted or unsubstituted C1-C4 alkyl-S(O)₂-, substituted or unsubstituted 4- to 9-membered heterocyclyl, NR'R"-C(O)-, substituted or unsubstituted 3- to 8-membered cyclohydrocarbyl-C(O)- and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 8-membered cyclohydrocarbyl-C(O)- and C1-C4 alkyl are optionally substituted by 1, 2 or 3 substituents selected from F, hydroxyl and cyano.

In some embodiments, when R₁₂, which does not participate in ring formation, is present, each R₁₂ is independently selected from: H, acetyl, propionyl, C1-C4 alkoxy, C1-C4 alkyl-S(O)₂-, 4- to 6-membered heterocyclyl, 3- to 5-membered cyclohydrocarbyl-C(O)- and substituted or unsubstituted C1-C4 alkyl; preferably, the C1-C4 alkyl is optionally substituted by 1, 2 or 3 substituents selected from F, hydroxyl and cyano.

In some embodiments, when R₁₃, which does not participate in ring formation, is present, each R₁₃ is independently selected from: H, halogen, substituted or unsubstituted C1-C4 acyl, hydroxyl, cyano, -NR'R", substituted or unsubstituted C1-C4 alkoxy, substituted or unsubstituted C1-C6 alkyl, substituted or unsubstituted C1-C4 alkyl-S(O)₂-, substituted or unsubstituted 3- to 6-membered cyclohydrocarbyl-C(O)- and NR'R"-C(O)-, or two R₁₃ on the same carbon atom form an oxo (=O) or thio (=S) group; wherein R' and R" are each independently selected from H and C1-C4 alkyl; preferably, the C1-C4 acyl, C1-C4 alkoxy, C1-C6 alkyl, C1-C4 alkyl-S(O)₂- and 3-to 6-membered cyclohydrocarbyl-C(O)- are optionally substituted by 1, 2 or 3 substituents selected from F, hydroxyl and cyano.

In some embodiments, when R₁₃, which does not participate in ring formation, is present, each R₁₃ is independently selected from: H, fluorine, chlorine, C1-C4 acyl, hydroxyl, cyano, C1-C4 alkoxy, C1-C6 alkyl, C1-C4 alkyl-S(O)₂- and 3- to 4-membered cyclohydrocarbyl-C(O)-.

In some embodiments, R' and R" are each independently selected from H and C1-C4 alkyl.

In some embodiments, R' and R" are each independently selected from H, methyl and ethyl.

In some embodiments, Rₐ is selected from H, methyl and ethyl.

In some embodiments, R₁₄ is H, halogen or C1-C4 alkyl.

In some embodiments, R₁₄ is H or methyl.

In some embodiments, R₁₄ is H.

In some embodiments, R₁₅ is selected from: H, halogen, substituted or unsubstituted C1-C4 acyl, hydroxyl, substituted or unsubstituted C1-C4 alkoxy, cyano, -NR'R", substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; R' and R" are each independently selected from H and C1-C4 alkyl.

In some embodiments, R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl.

In some embodiments, R₁₆ is selected from halogen, C1-C4 alkyl, hydroxyl, C1-C4 acyl and C1-C4 alkoxy.

In some embodiments, R₁₆ is selected from H, halogen, C1-C4 alkyl, hydroxyl and acetyl.

In some embodiments, R₁₆ is selected from H, hydroxyl, halogen and C1-C4 alkyl.

In some embodiments, R₁₆ is selected from H, fluorine, methyl, ethyl, hydroxyl and acetyl.

In some embodiments, R₁₆ is H, hydroxyl or methyl.

In some embodiments, R₁₆ is H.

In some embodiments, n is 0, 1, 2, 3 or 4.

In some embodiments, n is 0, 1 or 2.

In some embodiments, n is 0 or 1.

In some embodiments, X₁ is O.

In some embodiments, X₁ is NH.

In some embodiments, X₁ is N(CH₃).

In some embodiments, X₂ is C.

In some embodiments, X₂ is N.

In some embodiments, X₃ is C.

In some embodiments, X₃ is N.

In some embodiments, Y₅ is selected from: O, S, N, CH(R₁₃), C(R₁₃), -OCH₂-, - N(R₁₂)CH₂-, -CH₂CH₂- or N(R₁₂).

In some embodiments, Y₅ is selected from: a bond, O, S, -S(O)-, N, CH₂, CH, -C(O)-, - CH(OH)-, NH, -N(CH₃)-, -OCH₂-, -NHCH₂-, -N(CH₃)CH₂- and -CH₂CH₂-.

In some embodiments, Y₅ is selected from: O, S, N, CH₂, CH, NH and -N(CH₃)-.

In some embodiments, Y₅ is selected from: a bond, O, S, N, CH₂, CH, NH and -N(CH₃)-.

In some embodiments, Y₅ is selected from: a bond, O, CH₂, NH, -N(CH₃)- and -CH(CH₃)-.

In some embodiments, Y₅ is O.

In some embodiments, Y₅ is CH or CH₂.

In some embodiments, Y₅ is NH or N.

In some embodiments, Y₆ is selected from: O, N, -S(O)-, -SO₂-, S, CH(R₁₃), C(R₁₃), - OCH₂-, -N(R₁₂)CH₂-, -CH₂CH₂- and N(R₁₂).

In some embodiments, Y₆ is selected from: a bond, O, S, -S(O)-, -S(O)₂-, N, CH, -C(O)-, CH₂, NH, -N(CH₃)-, -N(C₂H₄OH)-, -N(C(O)CH₃)-, -N(C(O)C₂H₅)-, -OCH₂-, -NHCH₂-, - N(CH₃)CH₂- and -CH₂CH₂-.

In some embodiments, Y₆ is selected from: O, S, N, CH₂, CH, NH and -N(CH₃)-.

In some embodiments, Y₆ is selected from: a bond, O, S, N, CH, CH₂, NH and -N(CH₃)-.

In some embodiments, Y₁ and Y₄ are independently selected from: a bond, O, N, NH, - N(CH₃)-, CH, CH₂ and -CH(CH₃)-, and Y₁ and Y₄ are not simultaneously a bond or O.

In some embodiments, Y₁ is selected from: a bond, O, NH, -N(CH₃)-, CH₂ and -CH(CH₃)-.

In some embodiments, Y₁ is selected from: O or CH₂.

In some embodiments,
Y₁ is selected from: a bond, O, N, CH, CH₂, NH, CH(R₁₃), C(R₁₃)₂ and N(R₁₂);
Y₄ is selected from: a bond, O, N, CH, CH₂, NH, CH(R₁₃), C(R₁₃)₂ and N(R₁₂);
and Y₁ and Y₄ are not simultaneously a bond or O.

In some embodiments,
Y₁ is selected from: a bond, O, NH, -N(CH₃)-, CH₂ and -CH(CH₃)-;
Y₄ is selected from: a bond, O, NH, -N(CH₃)-, CH₂ and -CH(CH₃)-;
and Y₁ and Y₄ are not simultaneously a bond or O.

In some embodiments,
Y₅ is selected from: O, S, N, CH₂, CH, NH and -N(CH₃)-;
Y₆ is selected from: O, S, N, CH₂, CH, NH and -N(CH₃)-;
Y₁ is selected from: a bond, O, NH, -N(CH₃)-, CH₂ and -CH(CH₃)-;
Y₄ is selected from: a bond, O, NH, -N(CH₃)-, CH₂ and -CH(CH₃)-;
and Y₁ and Y₄ are not simultaneously a bond or O.

In some embodiments,
Y₁ is selected from: a bond, O, NH, -N(CH₃)-, CH₂ and -CH(CH₃)-;
Y₅ is selected from: O, S, N, CH₂, CH, NH and -N(CH₃)-;
and Y₁ and Y₅ are not simultaneously O.

In some embodiments, ring B can be optionally substituted by one or more R₁₃ and/or R₁₂ that do not participate in ring formation, and can be further optionally substituted by 1, 2 or 3 substituents selected from hydroxyl, amino, halogen, substituted or unsubstituted C1-C4 alkyl, - NR'R" and substituted or unsubstituted C1-C4 alkoxy.

In some embodiments, ring B can be optionally substituted by one or more R₁₃ and/or R₁₂ that do not participate in ring formation, and can be further optionally substituted by 1, 2 or 3 substituents selected from hydroxyl, amino, halogen, substituted or unsubstituted C1-C3 alkyl, - NR'R" and substituted or unsubstituted C1-C3 alkoxy.

In some embodiments, ring A and ring B, together with their substituents, form any one of the following groups: 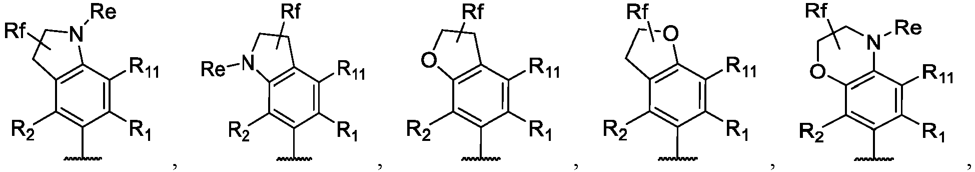 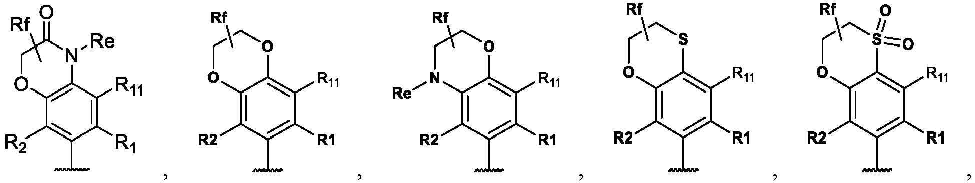 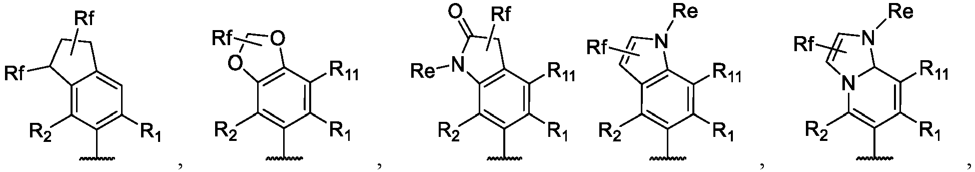 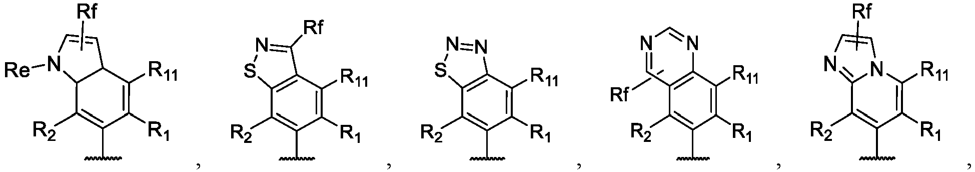 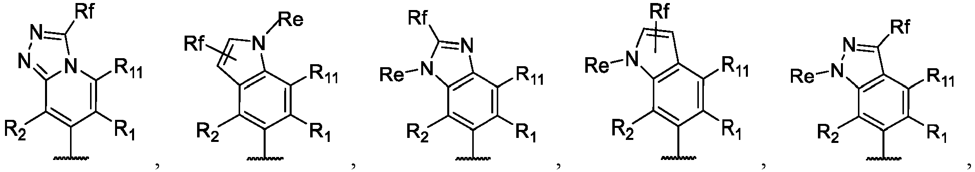 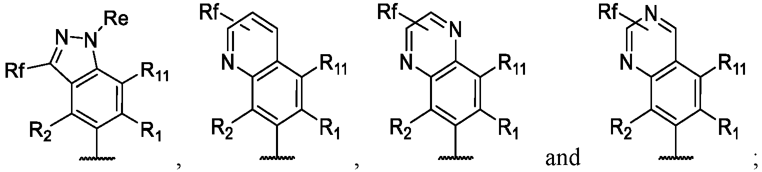 wherein R₁ is cyano or -C(O)NR'R"; R₂ is H or halogen; Rn is H, hydroxyl, -NR'R", halogen or C1-C4 alkyl; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3- to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H and C1-C4 alkyl; the wavy line indicates the bond connection position.

In some embodiments, R₁ is cyano, -C(O)NH₂, -CONHCH₃ and 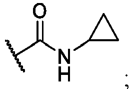 R₂ is H or F; Rn is H; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, acetyl, propionyl and cyclopropionyl; R_{f} is selected from H, F, Cl, hydroxyl, cyano, -NH₂, methoxy, -CH₂OH, -(CH₂)₂OH, -(CH₂)₂OCH₃, methyl, ethyl, cyclopropyl and cyclobutyl.

In some embodiments, R₁ is cyano, -C(O)NH₂ or -C(O)NHCH₃; R₂ is H or F; Rn is H; Rₑ is selected from H, methyl, ethyl, cyclopropyl, cyclobutyl, -CH₂CH₂OH, acetyl, propionyl and cyclopropionyl; R_{f} is selected from H, F, Cl, hydroxyl, cyano, -NH₂, methoxy, methyl, ethyl, cyclopropyl and cyclobutyl.

In some embodiments, R₁ is -C(O)NH₂ or -C(O)NHCH₃; R₂ is F; Rn is H; Rₑ is selected from H, methyl, cyclopropyl and acetyl; R_{f} is selected from H, F, Cl, hydroxyl, cyano, -NH₂, - CH₂OH, methoxy and methyl.

In some embodiments, R₁ is -C(O)NH₂ or -C(O)NHCH₃; R₂ is F; Rn is H; Rₑ is selected from H, methyl, cyclopropyl and acetyl; R_{f} is selected from H, F, Cl, hydroxyl, cyano, -NH₂, methoxy and methyl.

In some embodiments, ring A and ring B, together with their substituents, form any one of the following groups: 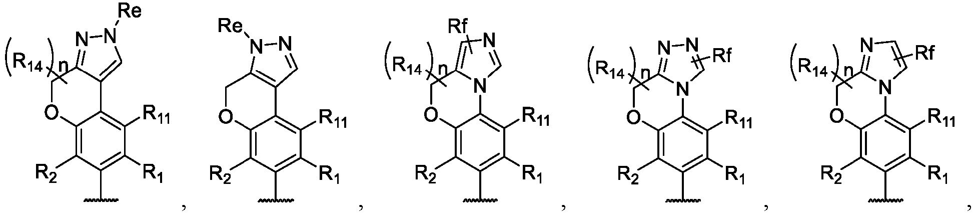 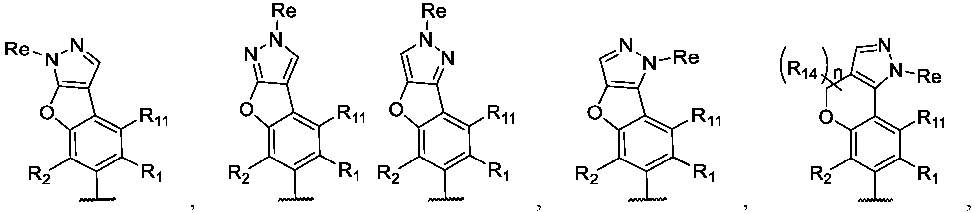 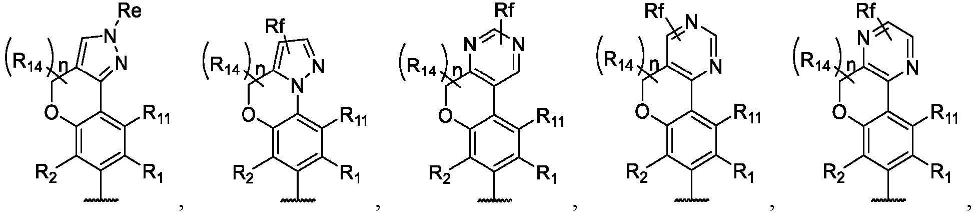 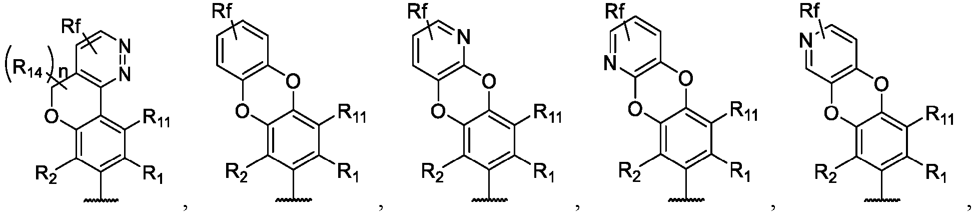 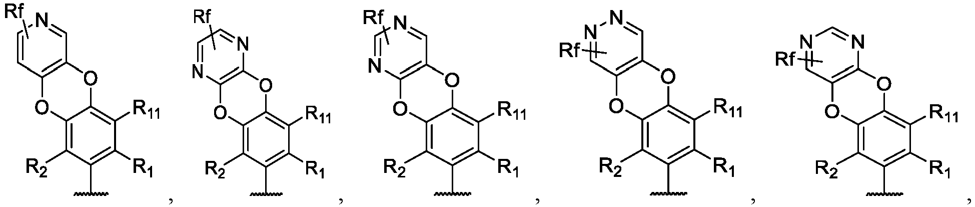 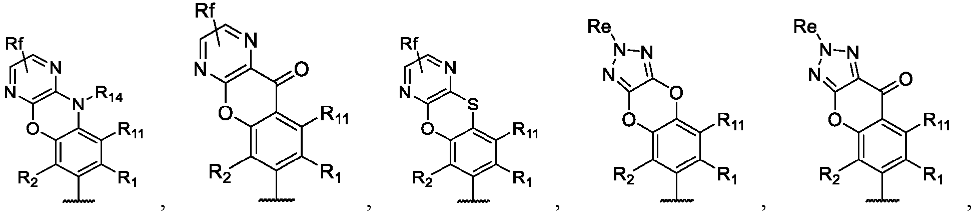 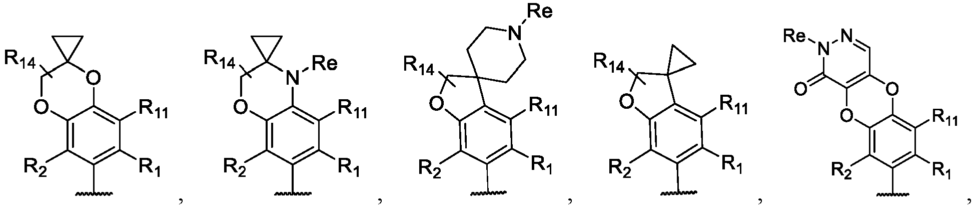 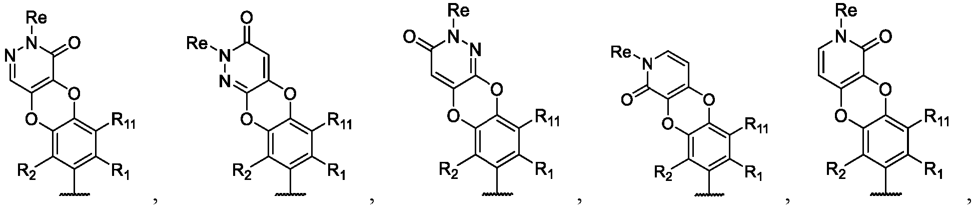 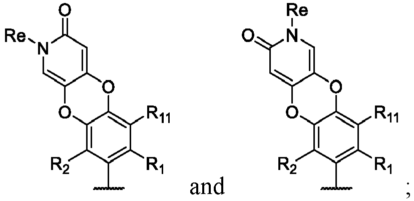 wherein R₁ is cyano or -C(O)NR'R"; R₂ is H or halogen; Rn is H, hydroxyl, -NR'R", halogen or C1-C4 alkyl; R₁₄ is H, halogen or C1-C4 alkyl; n is 0, 1 or 2; R₁₆ is selected from H, hydroxyl, halogen and C1-C4 alkyl; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3- to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H and C1-C4 alkyl; the wavy line indicates the bond connection position.

In some embodiments, R' and R" are each independently selected from H, C1-C4 alkyl and C3-C6 cycloalkyl.

In some embodiments, R₁ is cyano, -C(O)NH₂, -C(O)NHCH₃ and 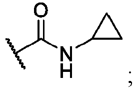 R₂ is H or F; Rn is H; R₁₄ is H or methyl; n is 0, 1 or 2; R₁₆ is H, hydroxyl or methyl; Rₑ is selected from H, methyl, deuteromethyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, acetyl, propionyl and cyclopropionyl; R_{f} is selected from H, F, Cl, hydroxyl, cyano, -NH₂, methoxy, methyl, ethyl, cyclopropyl and cyclobutyl.

In some embodiments, R₁ is cyano, -C(O)NH₂ or -C(O)NHCH₃; R₂ is H or F; Rn is H; R₁₄ is H or methyl; n is 0, 1 or 2; R₁₆ is H, hydroxyl or methyl; Rₑ is selected from H, methyl, ethyl, cyclopropyl, cyclobutyl, -CH₂CH₂OH, acetyl, propionyl and cyclopropionyl; R_{f} is selected from H, F, Cl, hydroxyl, cyano, -NH₂, methoxy, methyl, ethyl, cyclopropyl and cyclobutyl.

In some embodiments, R₁ is -C(O)NH₂, -C(O)NHCH₃ and 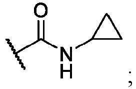 R₂ is F; R₁₁ is H; R₁₄ is H; R₁₆ is H; Rₑ is selected from H, methyl, deuteromethyl, difluoromethyl, trifluoromethyl, ethyl, fluoroethyl, difluoroethyl, trifluoroethyl, isopropyl, trifluoropropyl, oxocyclobutyl, 1,1-difluorocyclobutyl, cyclopropyl and acetyl; R_{f} is selected from H, F, Cl, hydroxyl, cyano, -NH₂, -CH₂OH, methoxy and methyl.

In some embodiments, R₁ is -C(O)NH₂ or -C(O)NHCH₃; R₂ is F; Rn is H; R₁₄ is H; R₁₆ is H; Rₑ is selected from H, methyl, ethyl, cyclopropyl and acetyl; R_{f} is selected from H, F, Cl, hydroxyl, cyano, -NH₂, methoxy and methyl.

In some embodiments, ring A and ring B, together with their substituents, form any one of the following groups: 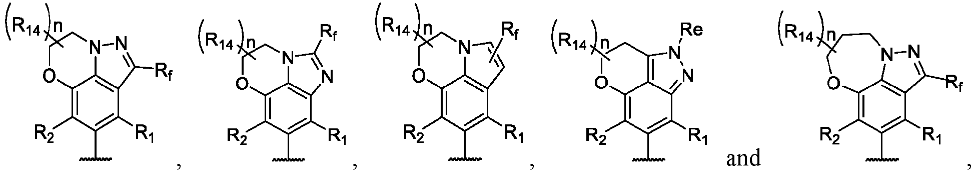 wherein R₁ is cyano or -C(O)NR'R"; R₂ is H or halogen; R₁₄ is H, halogen or C1-C4 alkyl; n is 0, 1 or 2; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3-to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H and C1-C4 alkyl; the wavy line indicates the bond connection position.

In some embodiments, R₁ is cyano, -C(O)NH₂, -C(O)NHCH₃ and 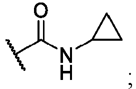 R₂ is H or F; R₁₄ is H or methyl; n is 0, 1 or 2; Rₑ is selected from H, methyl, deuteromethyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, acetyl, propionyl and cyclopropionyl; R_{f} is selected from H, F, Cl, hydroxyl, cyano, -NH₂, methoxy, methyl, ethyl, - CH₂OH, cyclopropyl and cyclobutyl.

In some embodiments, R₁ is cyano, -C(O)NH₂ or -C(O)NHCH₃; R₂ is H or F; R₁₄ is H or methyl; n is 0, 1 or 2; Rₑ is selected from H, methyl, cyclopropyl, cyclobutyl, -CH₂CH₂OH, acetyl, propionyl and cyclopropionyl; R_{f} is selected from H, F, Cl, hydroxyl, cyano, -NH₂, methoxy, methyl, ethyl, cyclopropyl and cyclobutyl.

In some embodiments, X₂ is selected from: C and N;
X₃ is selected from: C and N;
Y₅ and Y₆ are independently selected from: O, S, N, -S(O)-, -S(O)₂-, CH(R₁₃), C(R₁₃)₂, C(R₁₃), -OCH₂-, -N(R₁₂)CH₂-, -CH₂CH₂- and N(R₁₂); and Y₅ and Y₆ are not simultaneously a bond;
Y₁ is selected from: a bond, O, N, CH, CH₂, NH, CH(R₁₃), C(R₁₃)₂ and N(R₁₂);
Y₄ is selected from: a bond, O, N, CH, CH₂, NH, CH(R₁₃), C(R₁₃)₂ and N(R₁₂);
ring B is optionally substituted by one or more R₁₃ and/or R₁₂ that do not participate in ring formation, and further optionally substituted by 1, 2 or 3 substituents selected from hydroxyl, amino, halogen, substituted or unsubstituted C1-C4 alkyl, -NR'R" and substituted or unsubstituted C1-C4 alkoxy;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
each R₁₂ that does not participate in ring formation is independently selected from H, substituted or unsubstituted C1-C4 acyl, substituted or unsubstituted C1-C4 alkoxy, substituted or unsubstituted C1-C4 alkyl-S(O)₂-, substituted or unsubstituted 4- to 9-membered heterocyclyl, NR'R"-C(O)-, 3- to 8-membered cyclohydrocarbyl-C(O)- and substituted or unsubstituted C1-C4 alkyl; the C1-C4 acyl, C1-C4 alkoxy, C1-C4 alkyl-S(O)₂-, 4- to 9-membered heterocyclyl and C1-C4 alkyl are optionally substituted by 1, 2 or 3 substituents selected from F, hydroxyl and cyano;
each R13 that does not participate in ring formation is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted C1-C4 alkyl-S(O)₂-, substituted or unsubstituted 3- to 6-membered cyclohydrocarbyl-C(O)- and NR'R"-C(O)-; the C1-C4 acyl, C1-C4 alkoxy, C1-C4 alkyl, C1-C4 alkyl-S(O)₂- and 3- to 6-membered cyclohydrocarbyl-C(O)- are optionally substituted by 1, 2 or 3 substituents selected from F, hydroxyl and cyano, or two R₁₃ on the same carbon atom form an oxo (=O) or thio (=S) group;
R' and R" are each independently selected from H and C1-C4 alkyl;
wherein the dashed line indicates the presence or absence of a double bond.

In some embodiments, R' and R" can also be each independently selected from C3-C6 cycloalkyl.

In some embodiments, R₁ is

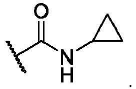

In some embodiments, X₂ is selected from: C and N;
X₃ is selected from: C and N;
Y₅ is selected from: O, S, -S(O)-, -S(O)₂-, N, CH₂, CH, -C(O)-, NH and -N(CH₃)-;
Y₆ is selected from: O, S, -S(O)-, -S(O)₂-, N, CH₂, CH, -C(O)-, NH and -N(CH₃)-;
Y₁ is selected from: a bond, O, NH, -N(CH₃)-, CH₂ and -CH(CH₃)-;
Y₄ is selected from: a bond, O, NH, -N(CH₃)-, CH₂ and -CH(CH₃)-;
H on ring B is further optionally substituted by 1, 2 or 3 substituents selected from hydroxyl, amino, halogen, substituted or unsubstituted C1-C4 alkyl, -NR'R" and substituted or unsubstituted C1-C4 alkoxy;
R₁ is selected from: cyano, -C(O)NH₂ and -C(O)NHCH₃;
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, - NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 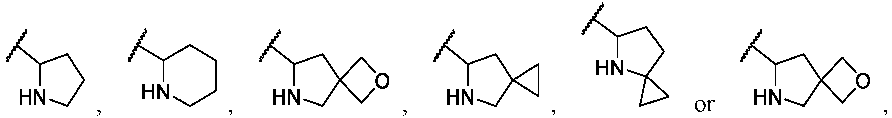 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl; Rn is selected from: H, hydroxyl, amino, fluorine, chlorine, methyl, methoxy and -NH(CH₃).

In some embodiments, ring A and ring B, together with their ring substituents, form any one of the following groups: 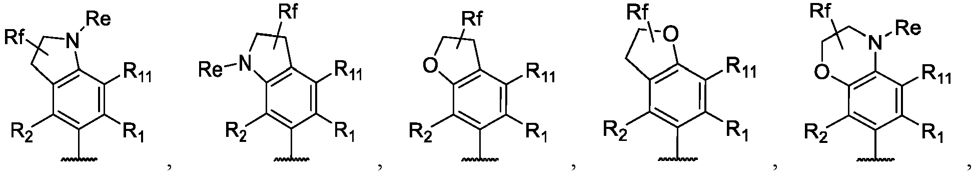 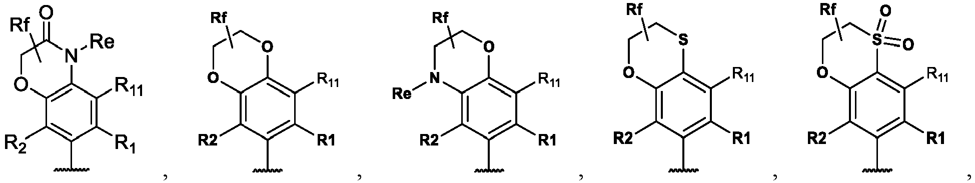 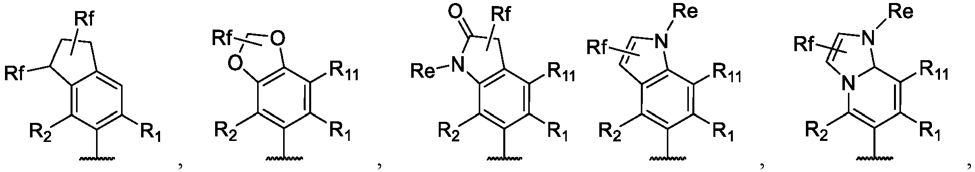 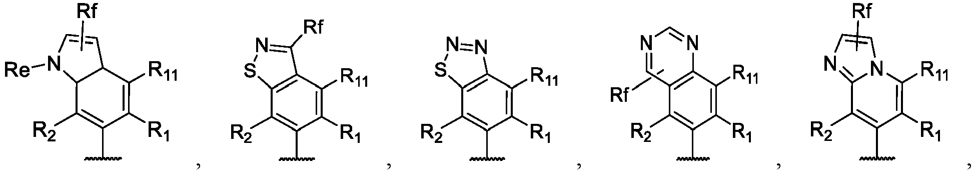 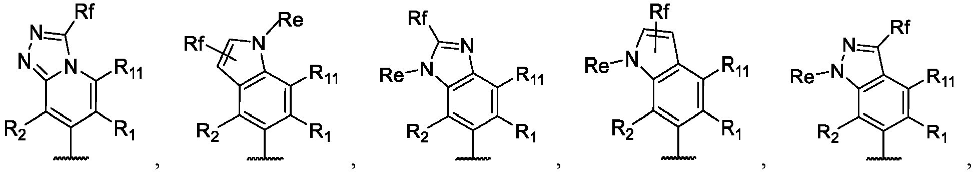 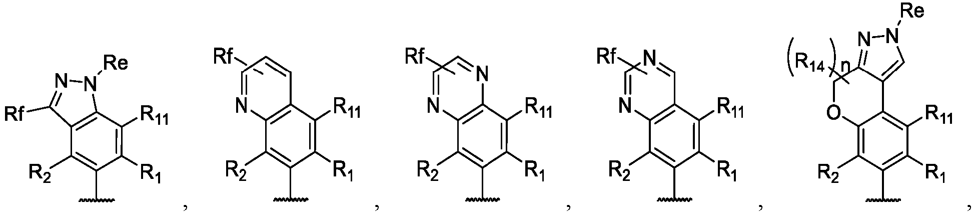 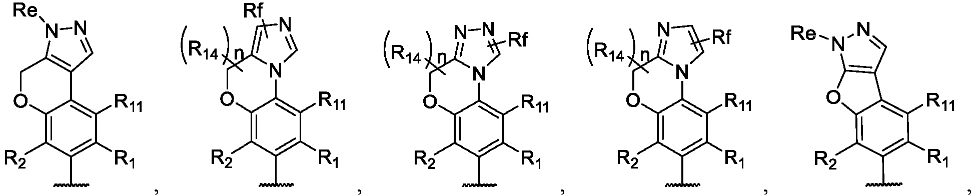 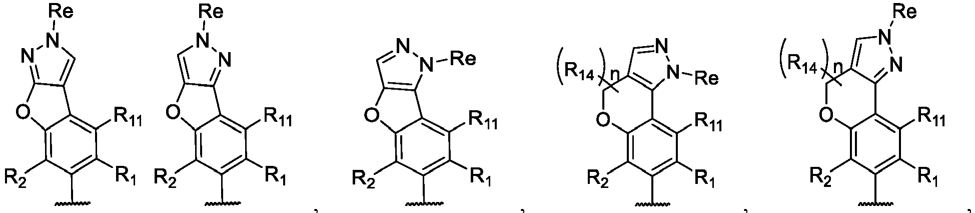 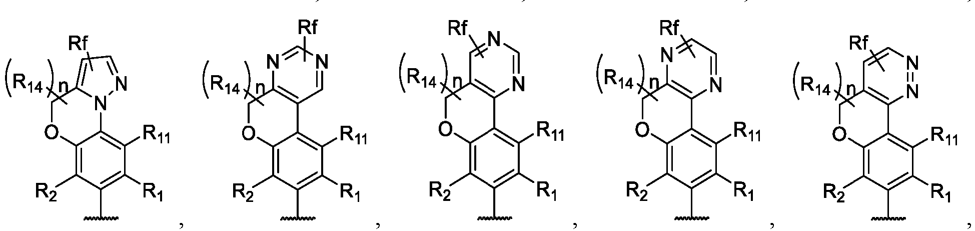 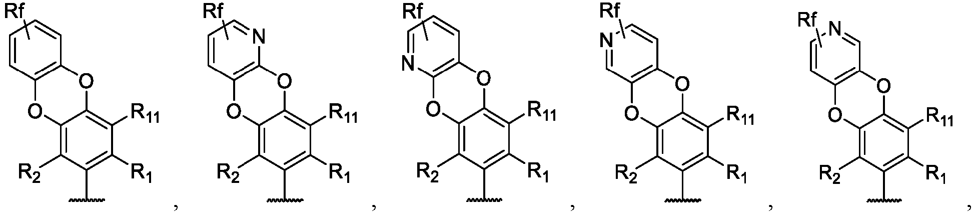 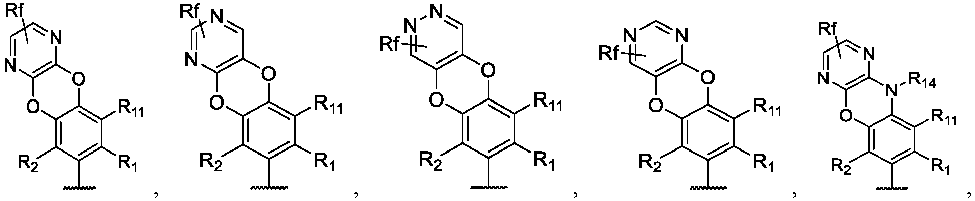 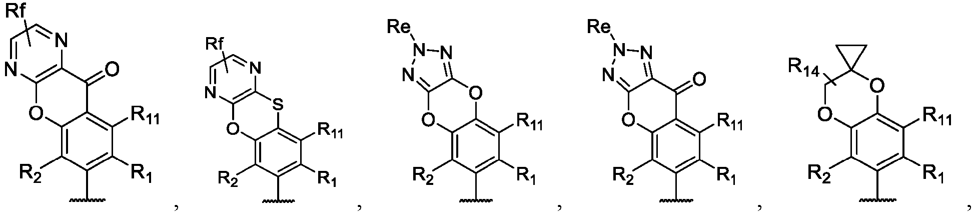 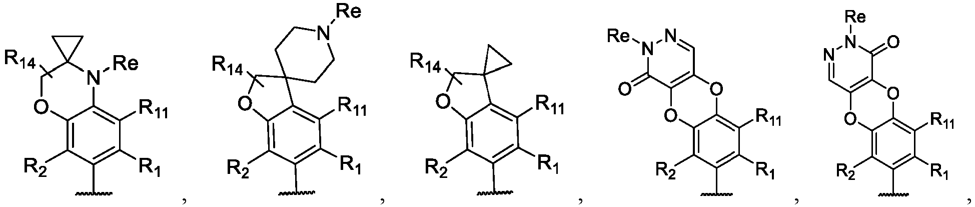 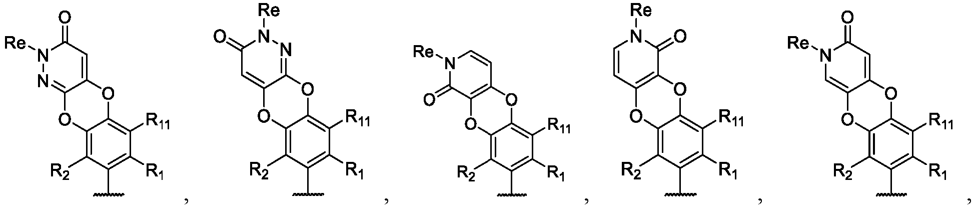 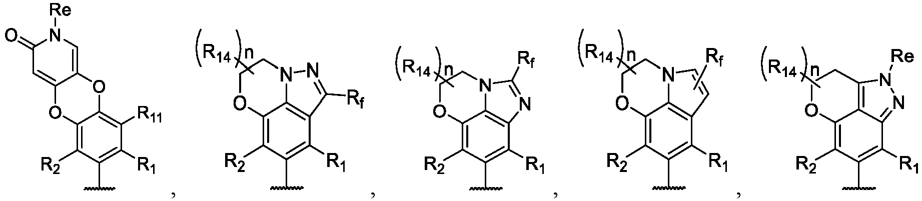 and 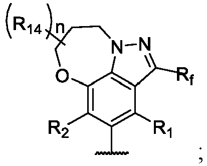 wherein R₁ is cyano or -C(O)NR'R"; R₂ is H or halogen; Rn is H, hydroxyl, -NR'R", halogen or C1-C4 alkyl; R₁₄ is H, halogen or C1-C4 alkyl; n is 0, 1 or 2; R₁₆ is selected from H, hydroxyl, halogen and C1-C4 alkyl; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3- to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H and C1-C4 alkyl; the wavy line indicates the bond connection position;
X₁ is O;
R₃ is selected from unsubstituted phenyl or phenyl substituted by 1, 2 or 3 substituents selected from hydroxyl, halogen, C1-C4 alkyl, C1-C4 haloalkyl and C1-C4 alkoxy;
R₄ is selected from H and C1-C4 alkyl;
R₅ is selected from H and C1-C4 alkyl;
R₆ is selected from: H; C1-C4 alkyl, which is optionally substituted by 1, 2 or 3 substituents selected from hydroxyl, halogen, C1-C4 alkoxy, cyano and -S(O)₂-C1-C4 alkyl; 3- to 8-membered cyclohydrocarbyl, which is optionally substituted by 1, 2 or 3 substituents selected from halogen, hydroxyl, cyano, -S(O)₂-C1-C4 alkyl, C1-C4 alkyl and C1-C4 alkoxy; 4- to 9-membered monocyclic or fused heterocyclyl, which is optionally substituted by 1, 2 or 3 substituents selected from halogen, hydroxyl, cyano, -S(O)₂-C1-C4 alkyl, C1-C4 alkyl and C1-C4 alkoxy; and 6- to 12-membered bridged cyclyl, 6- to 12-membered spiro cyclyl, 6- to 12-membered bridged heterocyclyl or 6- to 12-membered spiro heterocyclyl, which are each optionally substituted by 1, 2 or 3 substituents selected from C1-C4 alkyl, C1-C4 alkoxy and cyano;
alternatively, R₄ or R₅ and R₆, together with the atom to which they are attached, form the following group optionally substituted by 1, 2 or 3 substituents selected from H, deuterium, hydroxyl, methyl, F and methoxy: 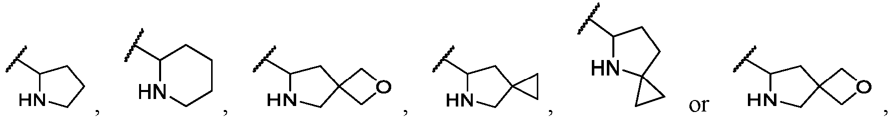 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl.

In some embodiments, R' and R" are each independently selected from H, C1-C4 alkyl and C3-C6 cycloalkyl.

In some embodiments, ring A and ring B, together with their ring substituents, form any one of the following groups: 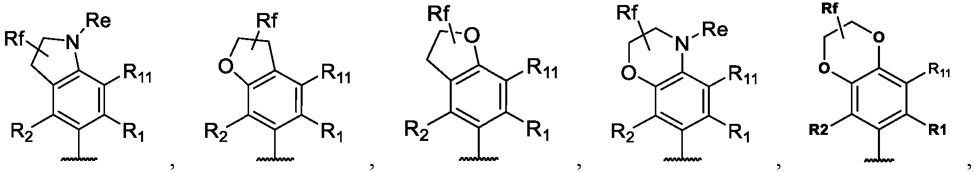 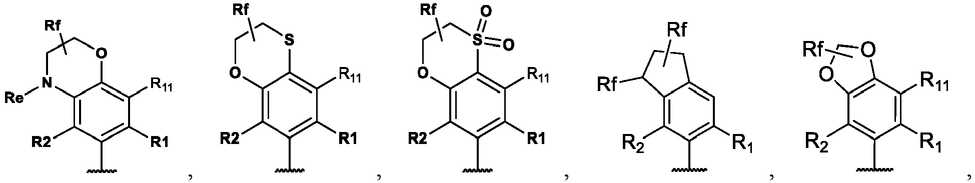 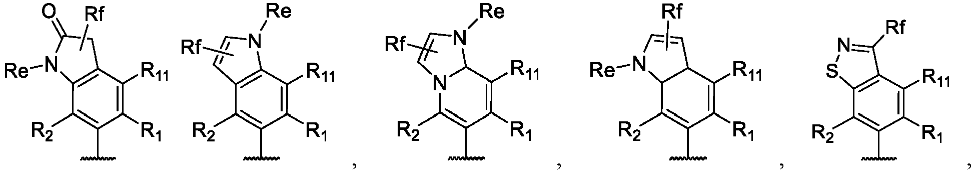 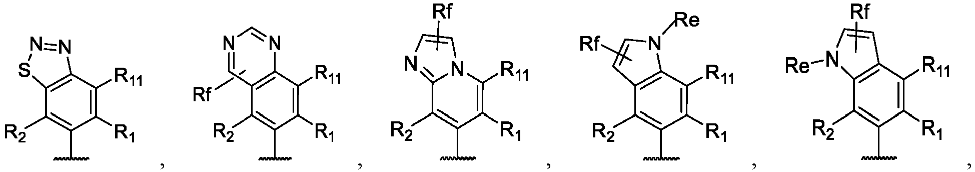 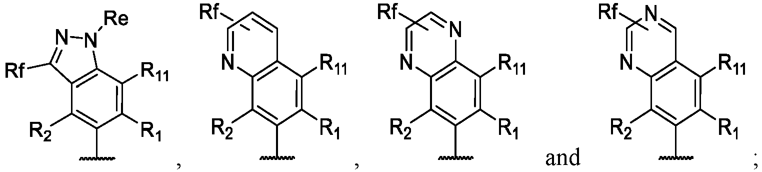 wherein R₁ is -C(O)NR'R"; R₂ is F; Rn is H, F or methyl; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, - CH₂CH₂(CH₃)OH, -CH₂CH₂OCH₃ and acetyl; R_{f} is selected from H, F, Cl, hydroxyl, cyano, - NR'R", methoxy, ethoxy, -CH₂OH, -CH₂CH₂OH, -CH₂CH₂OCH₃, C1-C6 alkyl and 3- to 6-membered cyclohydrocarbyl, wherein R' and R" are each independently selected from H and methyl; the wavy line indicates the bond connection position. In some embodiments, R' and R" are each independently cyclopropyl.

In some embodiments, R₁ is -C(O)NR'R"; R₂ is F; Rn is H, F or methyl; Rₑ is selected from H, methyl, ethyl, cyclopropyl, cyclobutyl, -CH₂CH₂OH, -CH₂CH₂OCH₃ and acetyl; R_{f} is selected from H, F, Cl, hydroxyl, cyano, -NR'R", methoxy, ethoxy, C1-C6 alkyl and 3- to 6-membered cyclohydrocarbyl, wherein R' and R" are each independently selected from H and methyl.

In some embodiments, ring A and ring B, together with their ring substituents, form any one of the following groups: 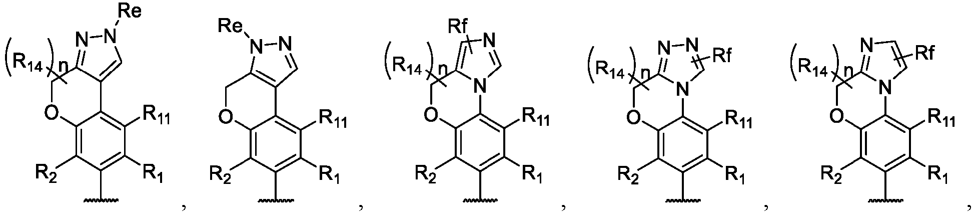 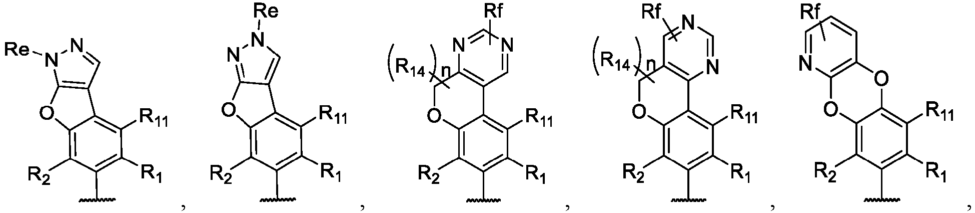 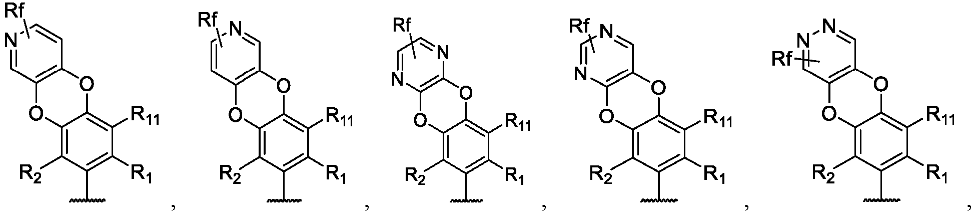 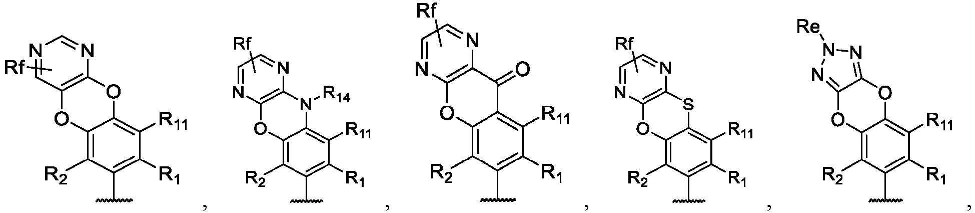 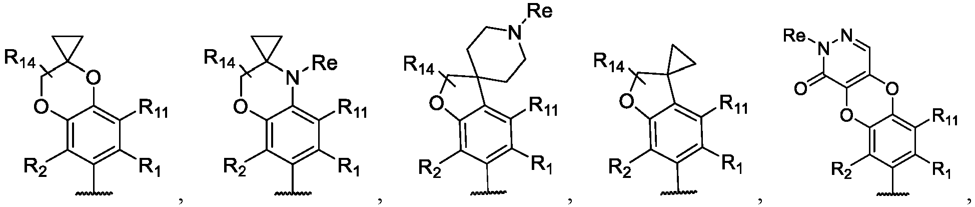 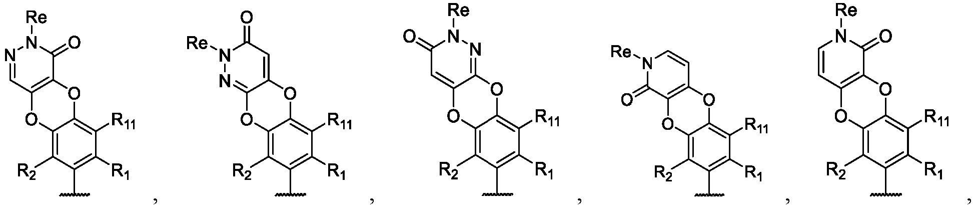 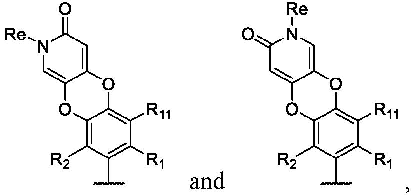 wherein R₁ is -C(O)NR'R"; R₂ is F; Rn is H, F or methyl; R₁₄ is H, F or methyl; n is 0, 1 or 2; R₁₆ is selected from H, hydroxyl and methyl; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂OCH₃ and acetyl; R_{f} is selected from H, F, Cl, hydroxyl, cyano, -NR'R", methoxy, ethoxy, -CH₂OH, C1-C6 alkyl and 3- to 6-membered cyclohydrocarbyl, wherein R' and R" are each independently selected from H and methyl; the wavy line indicates the bond connection position. In some embodiments, R' and R" are each independently cyclopropyl.

In some embodiments, R₁ is -C(O)NR'R"; R₂ is F; Rn is H, F or methyl; R₁₄ is H, F or methyl; n is 0, 1 or 2; R₁₆ is selected from H, hydroxyl and methyl; Rₑ is selected from H, methyl, ethyl, cyclopropyl, cyclobutyl, -CH₂CH₂OH, -CH₂CH₂OCH₃ and acetyl; R_{f} is selected from H, F, Cl, hydroxyl, cyano, -NR'R", methoxy, ethoxy, C1-C6 alkyl and 3- to 6-membered cyclohydrocarbyl, wherein R' and R" are each independently selected from H and methyl.

In some embodiments, the compound is selected from the following compounds:

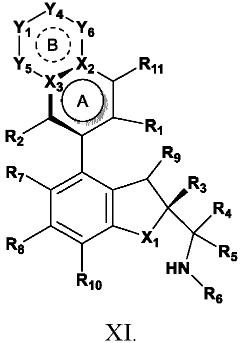

In some embodiments, the compound is selected from the following compounds: 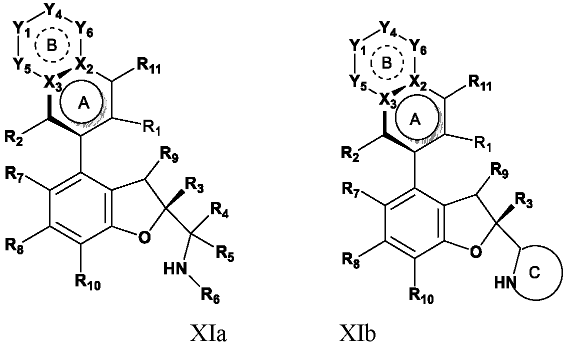 wherein ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, the compound is selected from the following compounds: 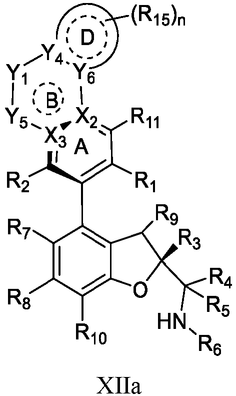 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

In some embodiments, the compound is selected from the following compounds: 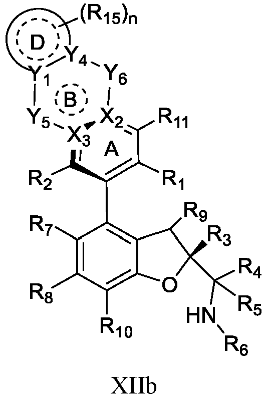 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

In some embodiments, the compound is selected from the following compounds: 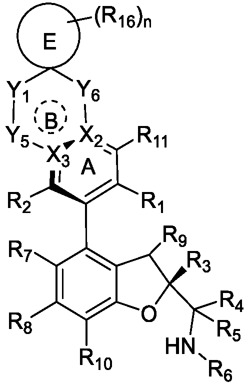 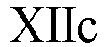 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

In some embodiments, the compound is selected from the following compounds: 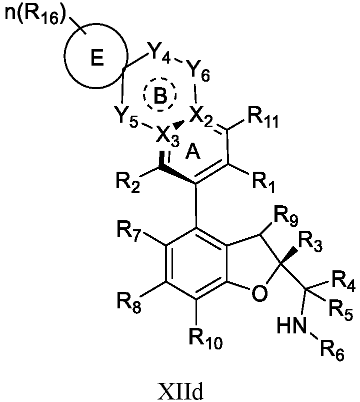 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

In some embodiments, the compound is selected from the following compounds: 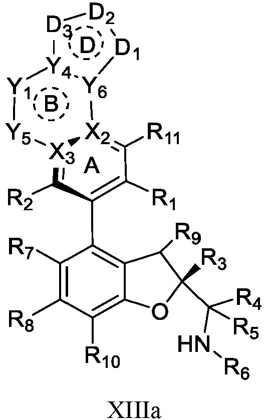 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRe, N-NRₑ, CR_{f}-NRe, N-̅ -̅ -̅N and N-̅ -̅C(R_{f});
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2.

In some embodiments, D₂ is selected from: O, S, S(O), S(O)₂, N, NRₑ, (CR_{f})ₚ, (CR_{f}R_{g})ₚ, N-̅ -̅ -̅N and N-̅ -̅C(R_{f}).

In some embodiments, the compound is selected from the following compounds: 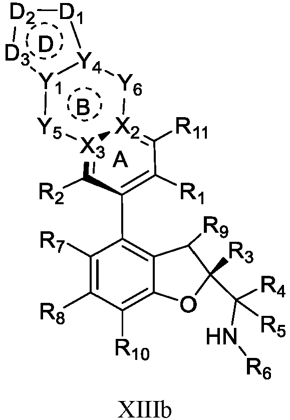 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRe, N-NRₑ, CR_{f}-NRₑ, N-̅ -̅ -̅N and
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2.

In some embodiments, D₂ is selected from: O, S, S(O), S(O)₂, N, NRₑ, (CR_{f})ₚ, (CR_{f}R_{g})ₚ, N-̅ -̅ -̅N and N-̅ -̅C(R_{f)}.

In some embodiments, the compound is selected from the following compounds: 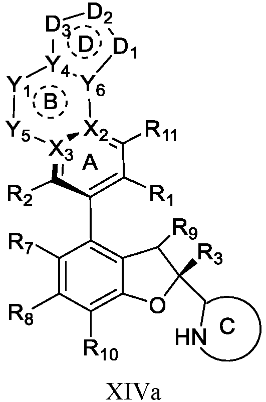 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRe, N-NRₑ, CR_{f}-NRe, N-̅ -̅ -̅N and N-̅ -̅C(R_{f});
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, in formula XIVa, D₂ is selected from: O, S, S(O), S(O)₂, N, NRₑ, (CR_{f})ₚ, (CR_{f}R_{g})ₚ, N-̅ -̅ -̅N and N-̅ -̅C(R_{f}).

In some embodiments, the compound is selected from the following compounds: 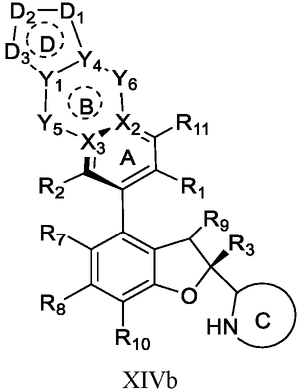 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRe, N-NRₑ, CR_{f}-NRₑ, N-̅ -̅ -̅N and N-̅ -̅C(R_{f)};
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, in formula XIVb, D₂ is selected from: O, S, S(O), S(O)₂, N, NRₑ, (CR_{f})ₚ, (CR_{f}R_{g})ₚ, N-̅ -̅ -̅N and N-̅ -̅C(R_{f)}.

In some embodiments, the compound is selected from the following compounds: 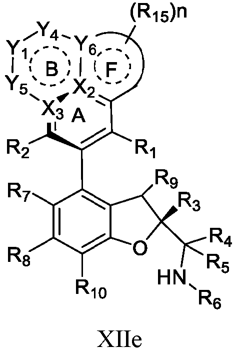 in the formula,
X₂ is C;
Y₆ is selected from: C, CH and N;
ring F is a 5- or 6-membered cyclohydrocarbyl, 5- or 6-membered heterocyclyl, phenyl or 5- or 6-membered heteroaryl ring, wherein the 5- or 6-membered heterocyclyl or 5- or 6-membered heteroaryl contains 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2 or 3.

In some embodiments, the compound is selected from the following compounds: 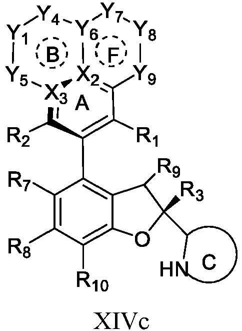 in the formula,
X₂ is C;
Y₆ is selected from: C, CH and N;
ring F is a 5- or 6-membered cyclohydrocarbyl, 5- or 6-membered heterocyclyl, phenyl or 5- or 6-membered heteroaryl ring, wherein the 5- or 6-membered heterocyclyl or 5- or 6-membered heteroaryl contains 1, 2 or 3 heteroatoms selected from O, N and S;
Y₇, Ys and Y₉ are independently selected from: a bond, O, S, S(O), S(O)₂, N, NRₑ, CRf and CR_{f}R_{g}, provided that at most one of Y₇, Y₈ and Y₉ is a bond, Y₇ and Y₈, Y₈ and Y₉ are not simultaneously O, S, -S(O)- or -S(O)₂- and satisfy the valence bond theory,
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

In some embodiments, the compound is selected from the following compounds: 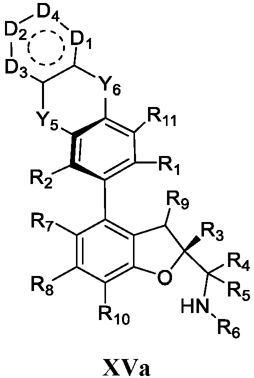 in the formula,
D₁ is selected from: O, S, S(O), S(O)₂, N, NRₑ, CR_{f}R_{g}, CR_{f} and C(=O);
D₂ is selected from: O, S, S(O), S(O)₂, N, NRₑ, CR_{f}R_{g}, CR_{f} and C(=O);
D₃ is selected from: O, S, S(O), S(O)₂, N, NRₑ, CR_{f}R_{g}, CR_{f} and C(=O);
D₄ is selected from: O, S, S(O), S(O)₂, N, NRₑ, CR_{f}R_{g}, CR_{f} and C(=O); and wherein the ring in which D1, D2, D3 and D₄ are located satisfies valence bond theory and contains at most two C(=O) and three N atoms;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl; each R_{f} at each occurrence is independently selected from: H, halogen, hydroxyl, cyano, oxo, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₁ is selected from: cyano and -C(O)NR'R";
R₂ is selected from: H, halogen, substituted or unsubstituted alkyl and substituted or unsubstituted alkoxy;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted C1-C4 alkyl;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 6-membered heterocyclyl ring; the 4- to 6-membered heterocyclyl contains 1 or 2 heteroatoms selected from N and O;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
each p is independently 1, 2, 3 or 4.

In some embodiments, Rₑ is selected from H, methyl, difluoromethyl, trifluoromethyl, deuteromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂OCH₃ and acetyl.

In some embodiments, in XVa, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano.

In some embodiments, the compound is selected from the following compounds: 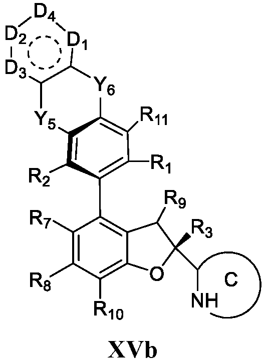 in the formula,
in the formula,
D₁ is selected from: O, S, S(O), S(O)₂, N, NRₑ, CR_{f}R_{g}, CR_{f} and C(=O);
D₂ is selected from: O, S, S(O), S(O)₂, N, NRₑ, CR_{f}R_{g}, CR_{f} and C(=O);
D₃ is selected from: O, S, S(O), S(O)₂, N, NRₑ, CR_{f}R_{g}, CR_{f} and C(=O);
D₄ is selected from: O, S, S(O), S(O)₂, N, NRₑ, CR_{f}R_{g}, CR_{f} and C(=O); and wherein the ring in which D1, D2, D3 and D₄ are located satisfies valence bond theory and contains at most two C(=O) and three N atoms;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₂ is selected from: H, halogen, substituted or unsubstituted alkyl and substituted or unsubstituted alkoxy;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring;
each p is 0, 1, 2, 3 or 4.

In some embodiments, in XVb, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano.

In some embodiments, Rₑ is selected from H, methyl, difluoromethyl, trifluoromethyl, deuteromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂OCH₃ and acetyl.

In some embodiments, the compound is selected from the following compounds: 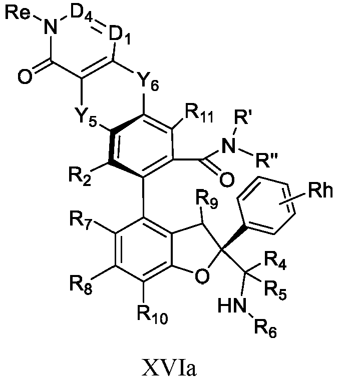 in the formula,
D₁ is selected from: N and CR_{f};
D₄ is selected from: N and CR_{f}, and D1 and D₄ are not simultaneously N;
Rₑ is selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl; each R_{f} at each occurrence is independently selected from: H, halogen, hydroxyl, cyano, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₂ is selected from: H, halogen, substituted or unsubstituted alkyl and substituted or unsubstituted alkoxy;
R₄ and R₅ are each independently selected from: H, halogen and substituted or unsubstituted C1-C4 alkyl;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 6-membered heterocyclyl ring; the 4- to 6-membered heterocyclyl contains 1 or 2 heteroatoms selected from N and O;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
Rₕ is selected from: H, halogen, substituted or unsubstituted C1-C4 alkyl and substituted or unsubstituted C1-C4 alkoxy.

In some embodiments, Rₑ is selected from H, methyl, difluoromethyl, trifluoromethyl, deuteromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂OCH₃ and acetyl.

In some embodiments, the compound is selected from the following compounds: 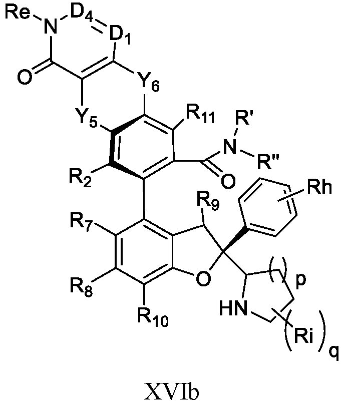 in the formula,
D₁ is selected from: N and CR_{f};
D₄ is selected from: N and CR_{f}, and D1 and D₄ are not simultaneously N;
Rₑ is selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl; each R_{f} at each occurrence is independently selected from: H, halogen, hydroxyl, cyano, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₂ is selected from: H, halogen, substituted or unsubstituted alkyl and substituted or unsubstituted alkoxy;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
Rₕ is selected from: H, halogen, substituted or unsubstituted C1-C4 alkyl and substituted or unsubstituted C1-C4 alkoxy;
Rᵢ is selected from: H, halogen, substituted or unsubstituted C1-C4 alkyl and substituted or unsubstituted C1-C4 alkoxy;
p is 1, 2 or 3;
q is 0, 1, 2, 3 or 4.

In some embodiments, Rₑ is selected from H, methyl, difluoromethyl, trifluoromethyl, deuteromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂OCH₃ and acetyl.

In some embodiments, the compound is selected from the following compounds:

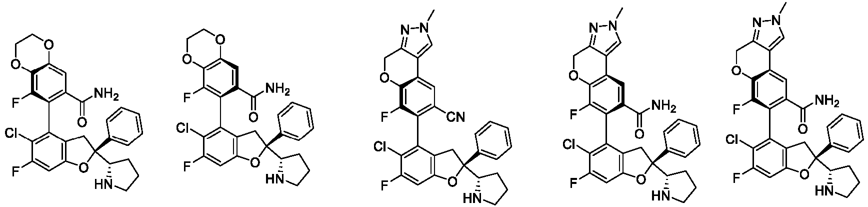

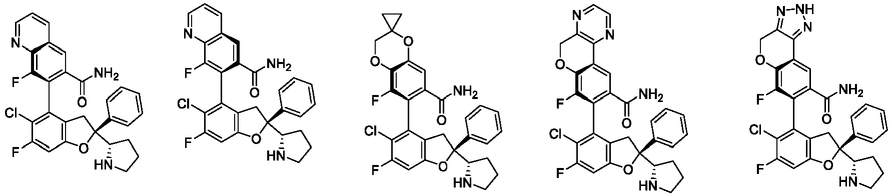

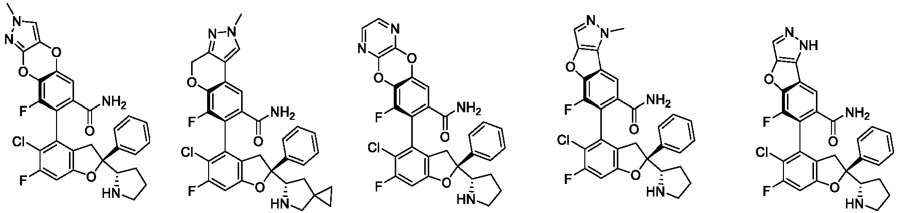

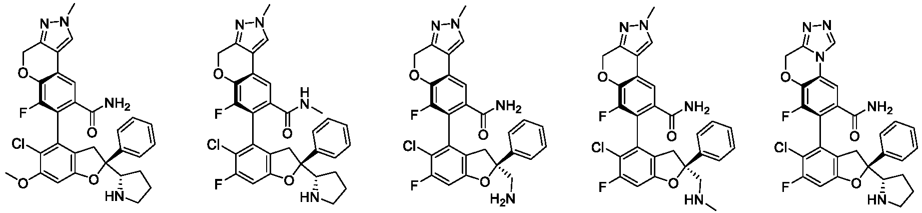

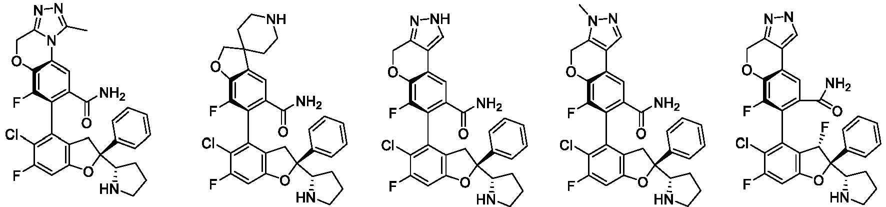

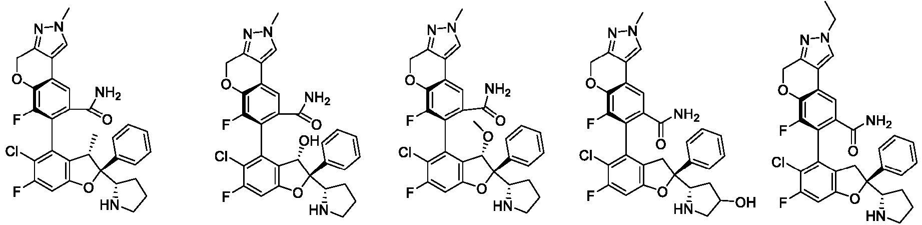

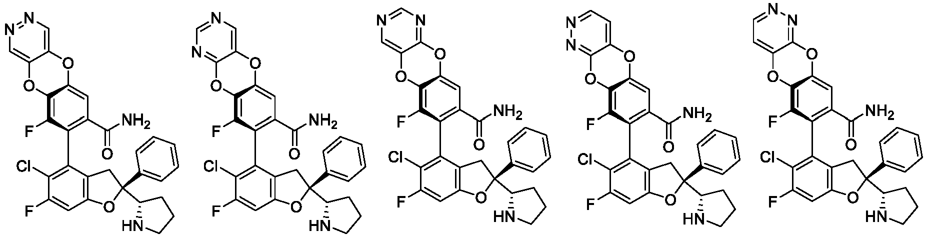

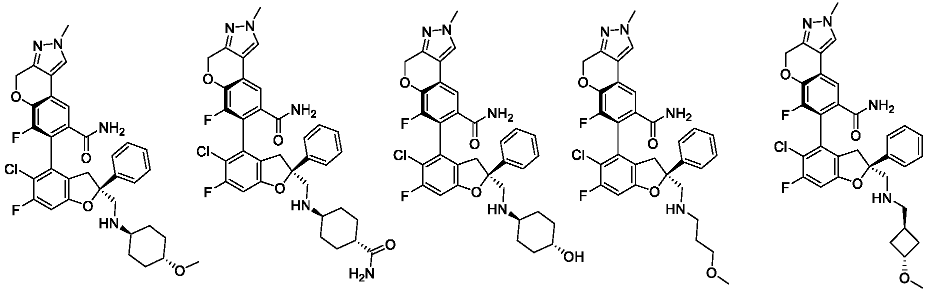

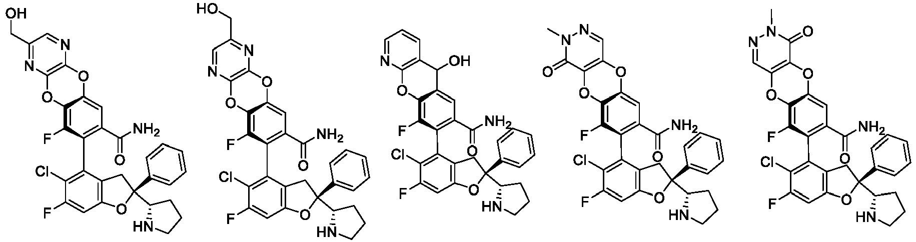

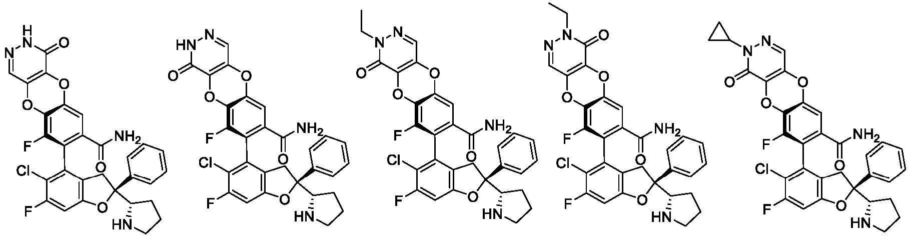

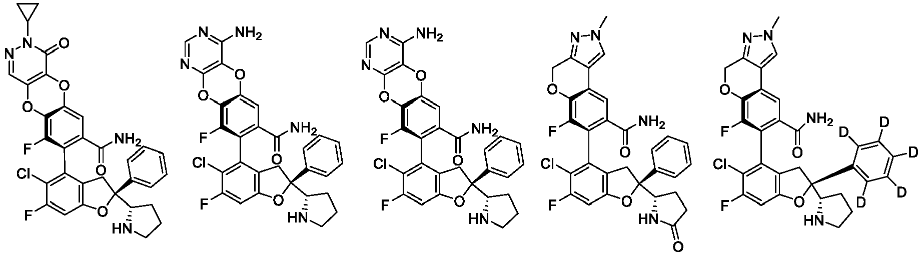

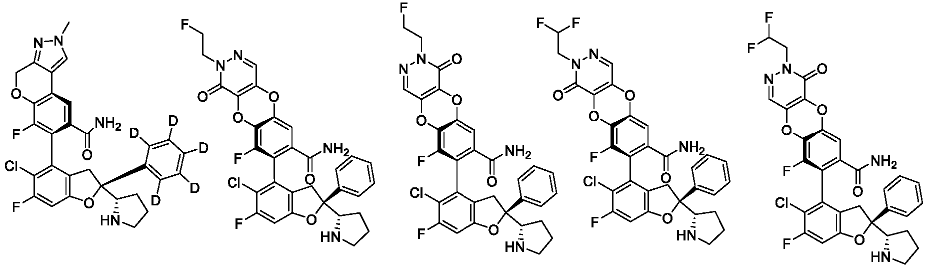

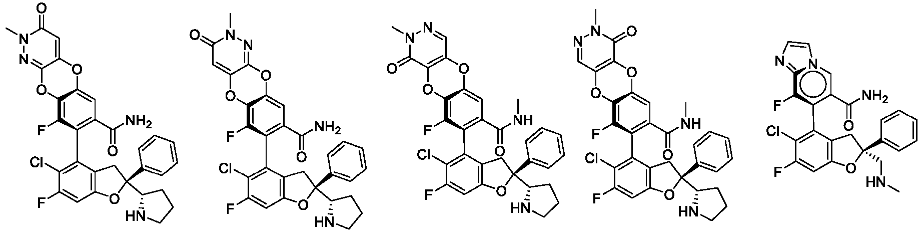

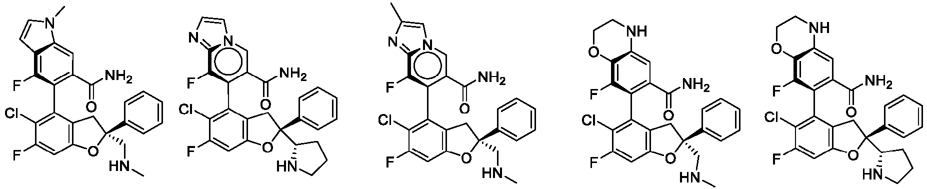

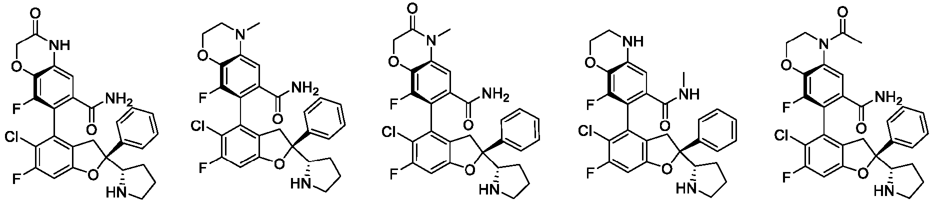

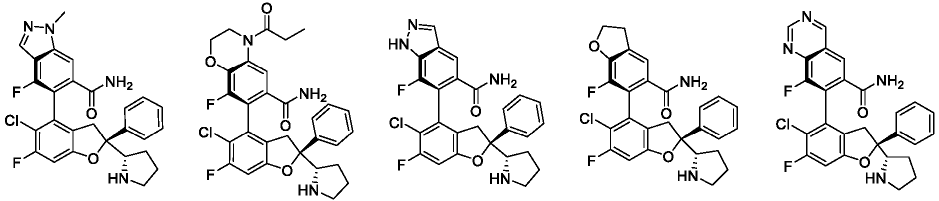

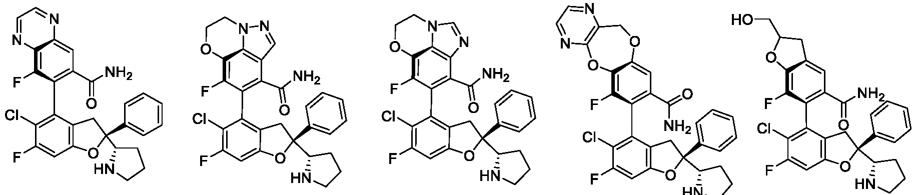

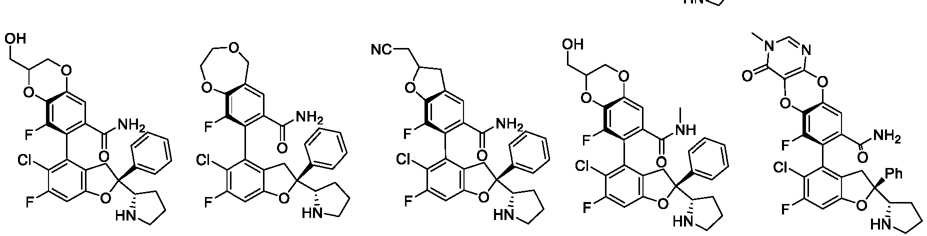

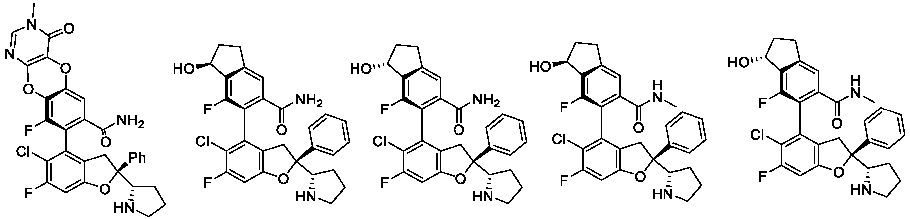

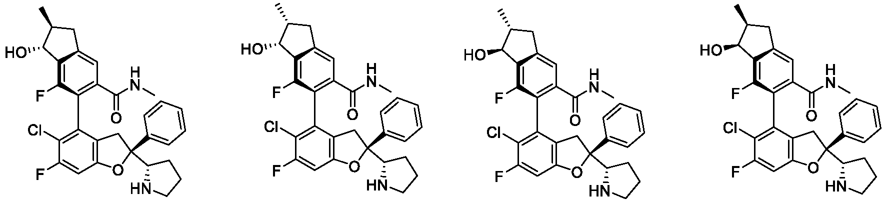

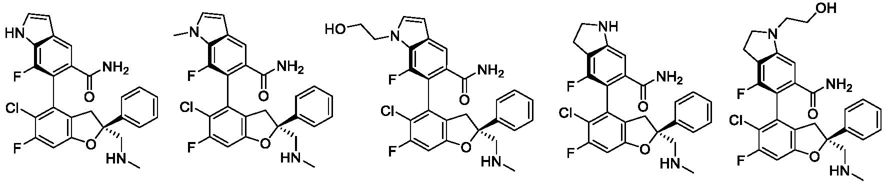

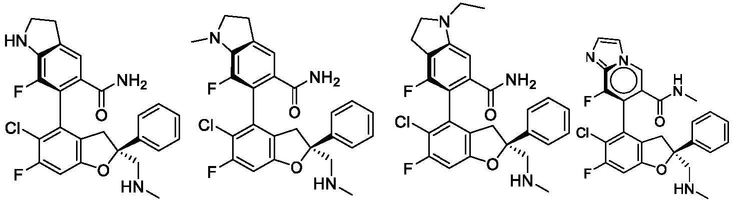

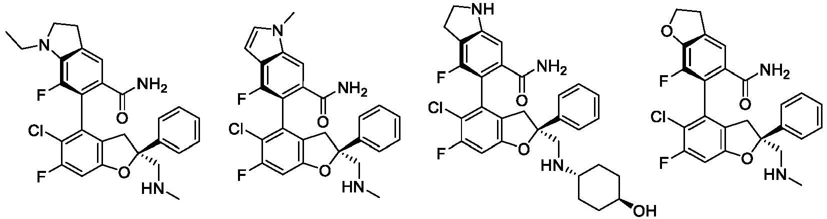

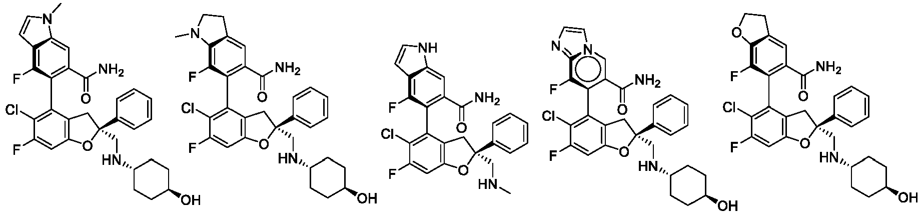

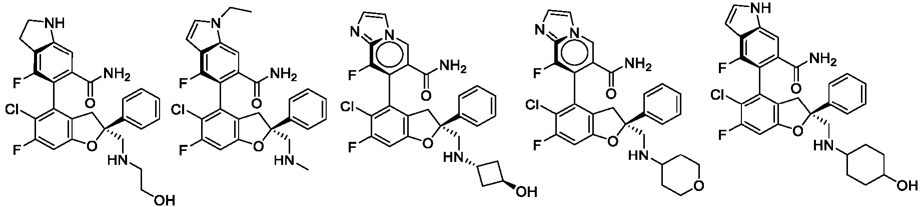

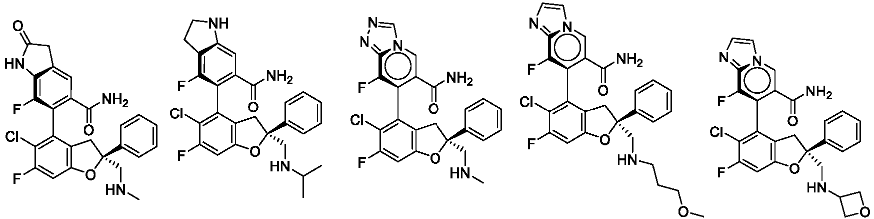

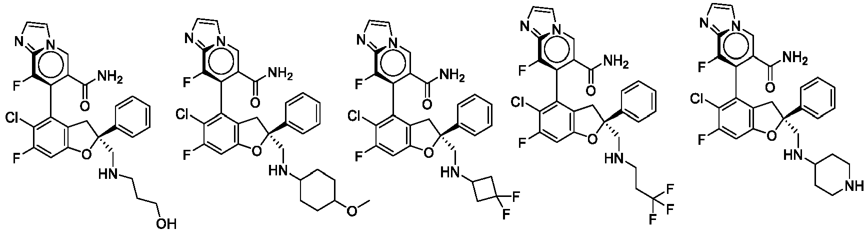

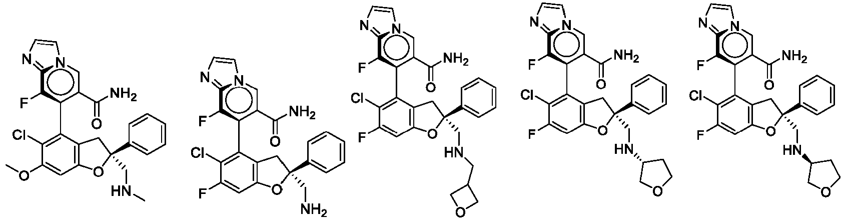

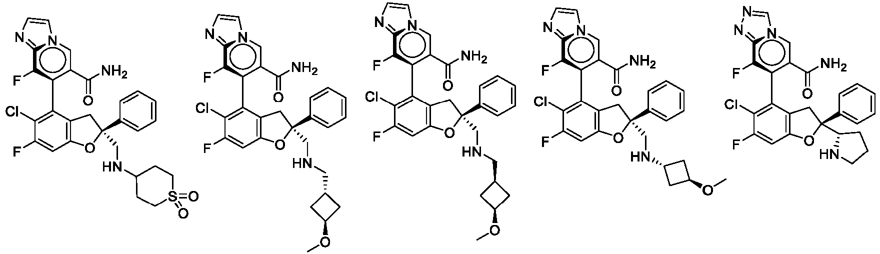

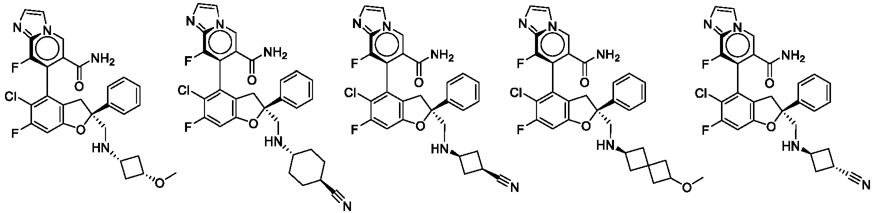

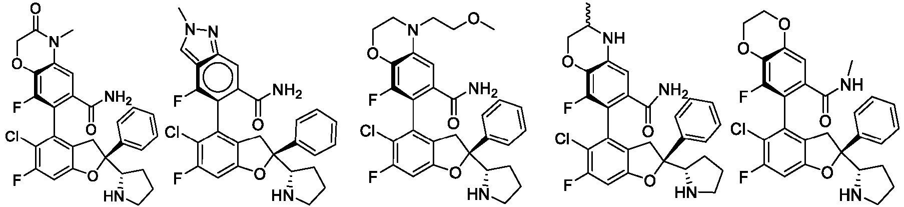

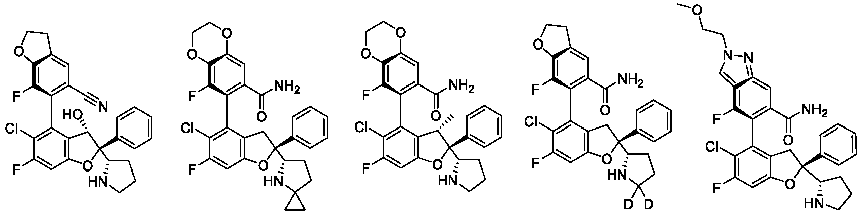

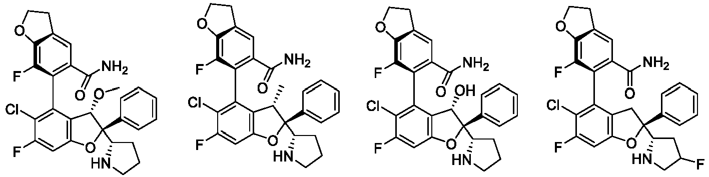

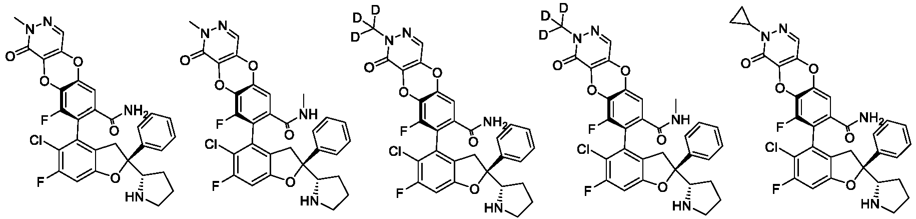

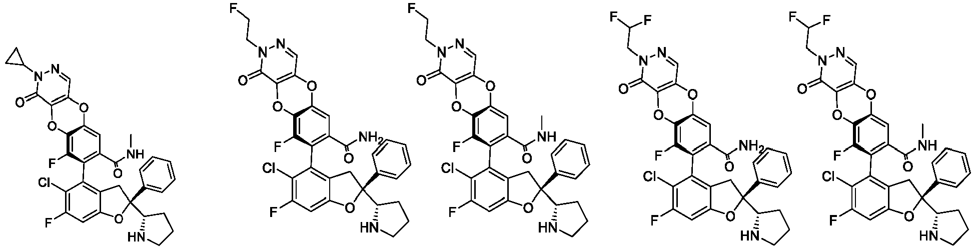

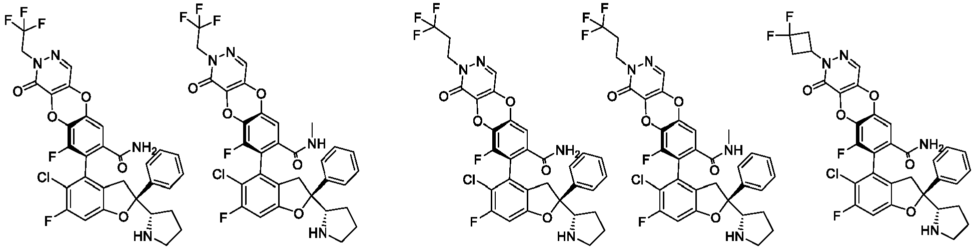

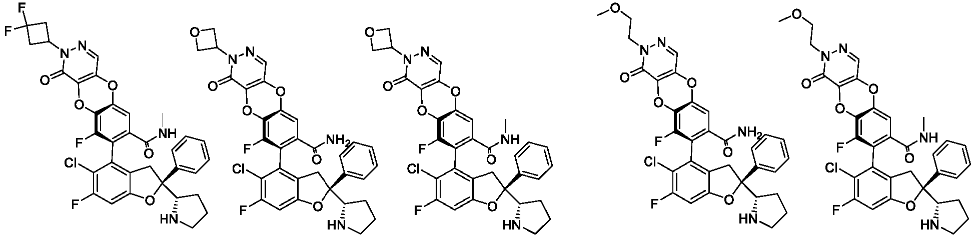

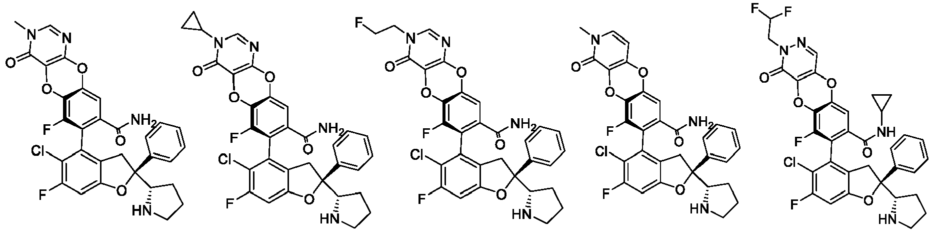

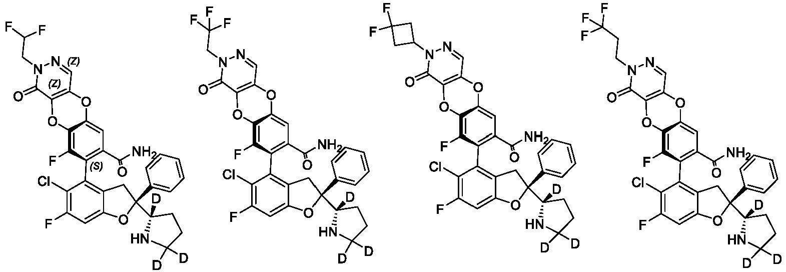

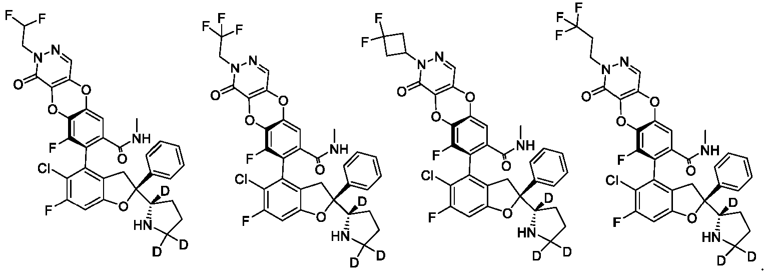

In another aspect, the present disclosure provides a compound of the following formula Ya, 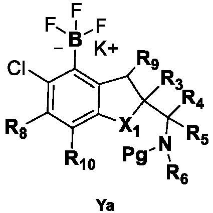 in the formula,
X₁ is selected from: O and NRₐ;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl.

In yet another aspect, the present disclosure provides a preparation method for a compound of formula Ya, comprising the following steps: 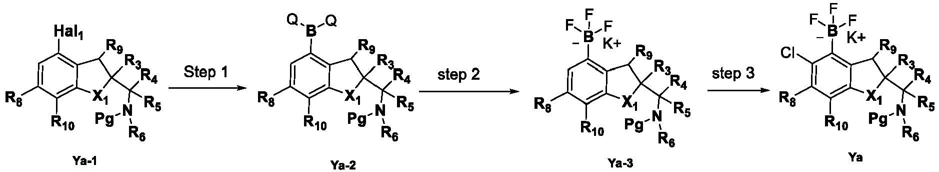
(i) coupling a borate ester under alkaline conditions with palladium catalysis, converting halogen Hal₁ in formula Ya-1 into Q-B-Q to obtain Ya-2;
(ii) converting Ya-2 into the potassium trifluoroborate salt of Ya-3 in an organic solvent at room temperature;
(iii) using a chlorinating reagent to convert Ya-3 into Ya under acid catalysis in an organic solvent;
in the formula,
X₁ is selected from: O and NRₐ; wherein Rₐ is selected from H and C1-C4 alkyl;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl;
Hal₁ is halogen;
Q-B-Q is boric acid or borate ester;
Pg is a nitrogen protecting group.

In some embodiments, the organic solvent in step (ii) is selected from polar organic solvents. In some embodiments, the organic solvent in step (ii) is selected from methanol, ethanol, acetone and acetonitrile. In some embodiments, the organic solvent in step (ii) is acetonitrile.

In some embodiments, the organic solvent in step (iii) is selected from polar organic solvents. In some embodiments, the organic solvent in step (iii) is selected from acetonitrile, acetone, 1,2-dichloroethane, dioxane and cyclopentyl methyl ether.

In yet another aspect, the present disclosure provides a compound of formula Za, 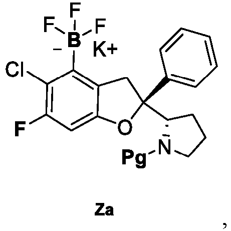 in the formula, Pg is a nitrogen protecting group.

In some embodiments, Pg is Boc.

In a further aspect, the present disclosure provides a preparation method for the compound of formula Za, comprising the following steps: 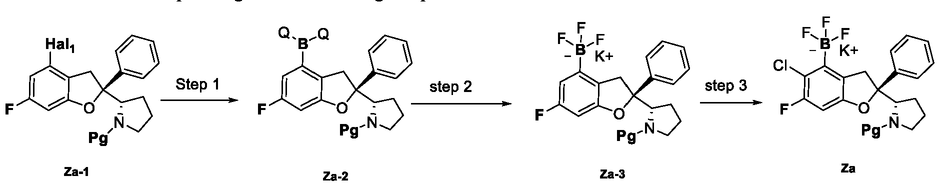
(i) coupling a borate ester under alkaline conditions with palladium catalysis, converting halogen Hal₁ in formula Za-1 into Q-B-Q to obtain Za-2;
(ii) adding potassium hydrogen fluoride aqueous solution to convert Q-B-Q into potassium trifluoroborate salt in an organic solvent at room temperature to obtain Za-3;
(iii) using a chlorinating reagent to convert Za-3 into Za under acid catalysis in an organic solvent;
in the formula, Hal₁ is halogen; Q-B-Q is boric acid or borate ester; Pg is a nitrogen protecting group.

In some embodiments, the organic solvent in step (ii) is selected from polar organic solvents. In some embodiments, the organic solvent in step (ii) is selected from methanol, ethanol, acetone and acetonitrile. In some embodiments, the organic solvent in step (ii) is acetonitrile.

In some embodiments, the organic solvent in step (iii) is selected from polar organic solvents. In some embodiments, the organic solvent in step (iii) is selected from acetonitrile, acetone, 1,2-dichloroethane, dioxane and cyclopentyl methyl ether.

The present disclosure also includes pharmaceutically acceptable salts, enantiomers, diastereomers, tautomers, solvates, isotopic substitutes, polymorphs, prodrugs or metabolites of the above compounds of each formula.

Herein, "stereoisomer" refers to a compound composed of the same atoms, bonded by the same bonds, but having different three-dimensional structures. The present disclosure will cover various stereoisomers and mixtures thereof.

When the compounds of the present disclosure contain an alkene double bond, the compounds of the present disclosure are intended to contain E- and Z- geometric isomers unless otherwise indicated.

"Tautomer" refers to an isomer formed by the transfer of a proton from one atom of a molecule to another atom of the same molecule. All tautomeric forms of the compounds of the present disclosure are also intended to be included within the scope of the present disclosure.

The compounds or pharmaceutically acceptable salts thereof of the present disclosure may contain one or more chiral carbon atoms, and thus may produce enantiomeric, diastereomeric and other stereoisomeric forms. Each chiral carbon atom can be defined as (R)- or (S)- based on stereochemistry. The present disclosure is intended to include all possible isomers, as well as their racemic and optically pure forms. The compounds of the present disclosure can be prepared by selecting racemates, diastereomers or enantiomers as raw materials or intermediates. Optically active isomers can be prepared using chiral synthons or chiral reagents, or resolved using conventional techniques such as crystallization and chiral chromatography.

Conventional techniques for the preparation/separation of individual isomers include chiral synthesis from suitable optically pure precursors or the resolution of racemates (or racemates of salts or derivatives) using methods like chiral high-performance liquid chromatography, see, for example, GeRald Gübitz and Martin G. Schmid(Eds.), ChiRal SepaRations, Methods and Protocols, Methods in Molecular Biology, Vol. 243, 2004; A.M. Stalcup, ChiRal SepaRations, Annu. Rev. Anal. Chem. 3:341-63, 2010; Fumiss et al.(eds.), VOGEL'S ENCYCLOPEDIA OF PRaCTICAL ORGANIC CHEMISTRY.sup.TH ED., Longman Scientific and Technical Ltd., Essex, 1991, 809-816; Heller, Acc. Chem. Res. 1990, 23, 128, and the disclosure of which is incorporated herein by reference in its entirety.

The present disclosure also includes all suitable isotopic substitutes (or isotopic variants) of the compounds or pharmaceutically acceptable salts thereof of the present disclosure. Isotopic variants of the compounds or pharmaceutically acceptable salts thereof of the present disclosure are defined as those in which at least one atom has been replaced by an atom having the same atomic number but an atomic mass different from that typically found in nature. Isotopes that can be incorporated into the compounds and pharmaceutically acceptable salts thereof of the present disclosure include, but are not limited to, isotopes of H, C, N and O, such as ²H, ³H, ¹¹C, ¹³C, ¹⁴C, ¹⁵N, ¹⁷O, ¹⁸O, ³⁵S, ¹⁸F, ³⁶Cl and ¹²⁵I. Isotopic variants of the compounds or pharmaceutically acceptable salts thereof described in the present disclosure can be prepared by conventional techniques using appropriate isotopic variants of suitable reagents.

In the present disclosure, the term "pharmaceutically acceptable salts" include pharmaceutically acceptable acid addition salts and pharmaceutically acceptable base addition salts.

"Pharmaceutically acceptable acid addition salts" refer to salts formed with inorganic or organic acids that retain the biological effectiveness of the free base without other side effects. Inorganic acid salts include, but are not limited to, hydrochloride, hydrobromide, sulfate, nitrate, phosphate, etc.; organic acid salts include, but are not limited to, formate, acetate, 2,2-dichloroacetate, trifluoroacetate, propionate, caproate, caprylate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalene disulfonate, etc. These salts can be prepared by methods known in the art.

"Pharmaceutically acceptable base addition salts" refer to salts formed with inorganic or organic bases that retain the biological effectiveness of the free acid without other side effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts and magnesium salts. Salts derived from organic bases include, but are not limited to, the following salts: primary, secondary and tertiary amines, substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, *N-*ethylpiperidine, polyamine resin, etc. Preferred organic bases include isopropylamine, diethylamine, ethanolamine, trimethylamine, dicyclohexylamine, choline and caffeine. These salts can be prepared by methods known in the art.

As used herein, the term "prodrug" refers to those compounds that will be metabolized (i.e., converted *in vivo*) into compounds of the present disclosure with pharmacological activity after administration. When the compound of the present disclosure itself is difficult to absorb from the gastrointestinal tract, its bioavailability can be improved by making it into a prodrug. Examples of prodrugs of the compounds of the present disclosure can include simple esters of carboxyl-containing compounds (e.g., esters obtained by condensation with C1-C4 alcohols according to methods known in the art); esters of hydroxyl-containing compounds (e.g., esters obtained by condensation with C1-C4 monocarboxylic acids, C3-C6 dicarboxylic acids or anhydrides thereof such as succinic anhydride or fumaric anhydride according to methods known in the art); imines of amino-containing compounds (e.g., imines obtained by condensation with C1-C4 aldehydes or ketones according to methods known in the art); carbamates of amino-containing compounds, such as those described by Leu et al. (J. Med. Chem., 42:3623-3628(1999)) and Greenwald et al. (J. Med. Chem., 42:3657-3667(1999)); aldehyde acetals or ketone acetals of hydroxyl-containing compounds (e.g., those acetals obtained by condensation with chloromethyl methyl ether or chloromethyl ethyl ether according to methods known in the art).

### III. Compound preparation

Herein, the compound of formula I can be prepared by the following general synthetic route:

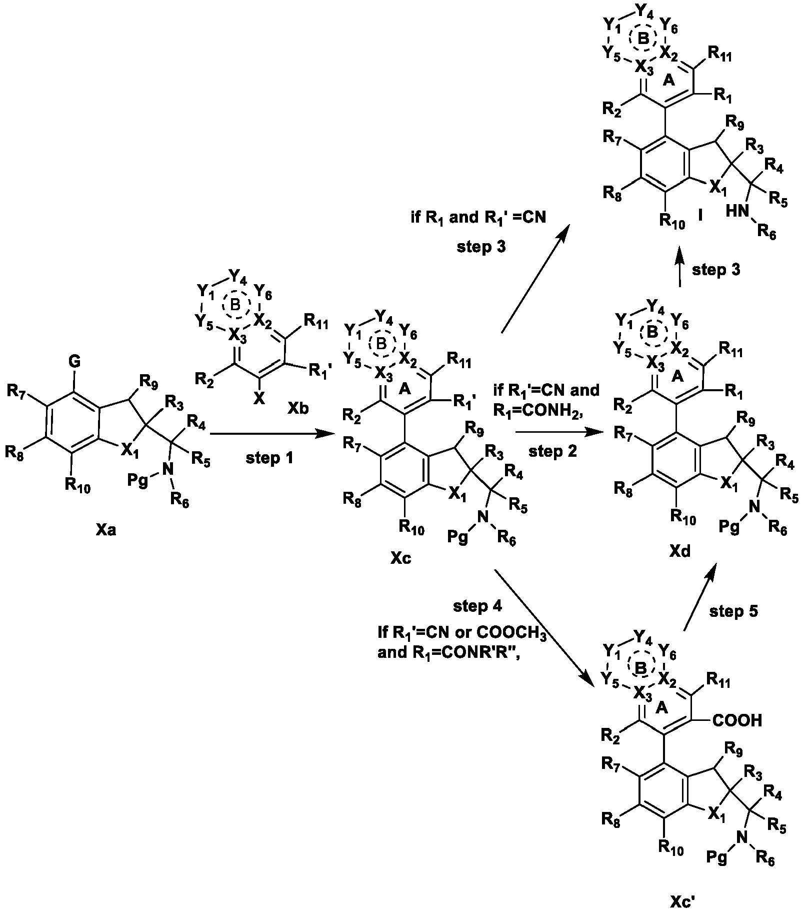

R₁-R₁₀, R₁₁, X₁-X₃ and Y₁-Y₆ in each structural formula are as defined in any one of the previous embodiments; G is boric acid, potassium trifluoroborate salt or borate ester, etc.; X is halogen, preferably Br or I; R₁' is a conversion functional group of R₁, preferably cyano or ester group, which can be converted into R₁ under suitable conditions; Pg is a nitrogen protecting group, preferably *tert*-butoxycarbonyl (tBuOC(O)-).

### The above method includes:

When R₁ and R₁' are both cyano, the compound of formula I can be obtained through steps 1 and 3. In step 1, formulas Xa and Xb are coupled via a palladium catalyst, ligand and base to obtain formula Xc. When R₁' and/or R₂ are not H, formula Xc usually contains a pair of axial chiral isomers. In step 3, Pg is removed from formula Xc under suitable conditions to obtain the compound of formula I.

When R₁ is CONH₂ and R₁' is cyano, the compound of formula I can be obtained through step 1, step 2 and step 3. In step 1, formulas Xa and Xb are coupled via a palladium catalyst, ligand and base to obtain formula Xc. When R₁' and/or R₂ are not H, formula Xc usually contains a pair of axial chiral isomers. In step 2, formula Xc is hydrolyzed to amide via a copper catalyst to obtain formula Xd, and the copper catalyst is preferably copper acetate. In step 3, Pg is removed from formula Xd under suitable conditions to obtain the compound of formula I.

When R₁ is CONR'R", R' and R" are not simultaneously H, and R₁' is cyano or methyl formate group (-COOCH₃), then the compound of formula I can be obtained by the reaction sequence of step 1, step 4, step 5 and step 3. In step 1, formulas Xa and Xb are coupled via a palladium catalyst, ligand and base to obtain formula Xc. When R₁' and/or R₂ are not H, formula Xc usually contains a pair of axial chiral isomers. In step 4, formula Xc is hydrolyzed by an inorganic base to obtain formula Xc', and the inorganic base is preferably sodium hydroxide, lithium hydroxide, potassium hydroxide, etc. In step 5, formula Xc' and R'R"NH are condensed by a condensing agent to obtain formula Xd. In step 3, Pg is removed from formula Xd under suitable conditions to obtain the compound of formula I.

Other compounds of the present disclosure can be prepared by referring to the above reaction flow chart.

When G in formula Xa is BF₃K and R₇ is chlorine, then formula Ya can be synthesized by the following route 2:

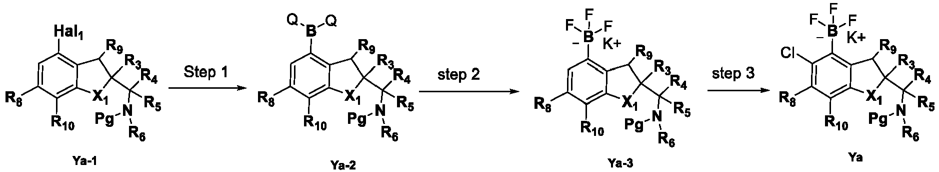

### Synthesis route for formula Ya

R₃-R₆, R₈-R₁₀ and X₁ in each structural formula are as defined in any one of the previous embodiments; preferably, R₈ is F, R₉ and R₁₀ are H, R₃ is phenyl, and R₄ or R₅ and R₆, together with the C and N to which they are attached, form 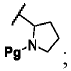 formula Za can be synthesized by the following route 3:

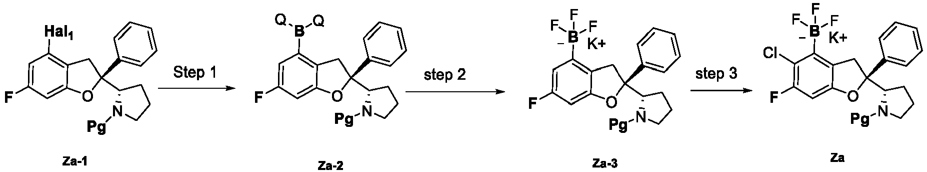

### Synthesis route for formula Za

In the synthesis routes 2 and 3, Hal₁ is halogen, preferably Br or I; Q-B-Q is boric acid or borate ester, preferably boric acid (B(OH)₂), pinacol borate 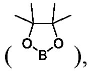 diisopropyl borate or 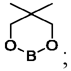 Pg is a nitrogen protecting group, preferably *tert*-butoxycarbonyl (tBuOC(O)-). In synthetic routes 2 and 3, step 1: the halogen (Hal₁) in formula Ya-1 or formula Za-1 is converted into boric acid or borate ester (QBQ), which can be obtained by palladium-catalyzed coupling of the borate ester under alkaline conditions; for the palladium-catalyzed coupling, the palladium catalyst is preferably 1,1'-bis(diphenylphosphino)ferrocene-palladium(II)dichloride dichloromethane complex, the borate ester is preferably pinacol borate, the base used is preferably potassium acetate, the reaction solvent is preferably toluene, and the reaction temperature is preferably 75 to 105°C. Step 2: Boric acid or borate ester (QBQ) is converted into potassium trifluoroborate salt Ya-3 in an organic solvent (preferably an alcohol solvent) by the addition of potassium hydrogen fluoride aqueous solution at room temperature. Step 3: Ya-3 is chlorinated using a chlorinated reagent in an organic solvent under acid catalysis to obtain Ya; the organic solvent is preferably acetonitrile, the chlorinated reagent is preferably *N*-chlorosuccinimide, the acid is preferably p-toluenesulfonic acid, and the reaction temperature is preferably -10 to 40°C.

### IV. Pharmaceutical compositions, methods, uses and other embodiments

In another aspect, the present disclosure provides a pharmaceutical composition, and the pharmaceutical composition comprises the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIII a, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure and a pharmaceutically acceptable carrier or excipient.

In another aspect, the present disclosure provides a use of the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition of the present disclosure in the manufacture of a medicament for treating or preventing diseases mediated by the interaction between YAP/TAZ and TEAD.

In another aspect, the present disclosure provides the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition of the present disclosure for use in treating or preventing diseases. In some embodiments, the present disclosure also provides the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition of the present disclosure for use in treating or preventing diseases mediated by the interaction between YAP/TAZ and TEAD.

In yet another aspect, the present disclosure provides a method for treating or preventing diseases mediated by the interaction between YAP/TAZ and TEAD, and the method comprises administering to a subject in need thereof a therapeutically effective amount of the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, or the pharmaceutical composition of the present disclosure.

The compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure is an inhibitor of the interaction between YAP/TAZ and TEAD, more specifically, an inhibitor of the interaction between YAP and TEAD. Accordingly, the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure can be used to treat or prevent diseases mediated by the interaction between YAP/TAZ and TEAD.

Herein, "diseases mediated by the interaction between YAP/TAZ and TEAD" refer to diseases in which the interaction between YAP/TAZ and TEAD is involved in the occurrence and/or development of the disease, and the purpose of alleviation, treatment and/or prevention can be achieved by inhibiting the expression and/or activity of YAP and TEAD or by inhibiting or blocking the interaction of YAP-TEAD protein.

In some embodiments, the diseases mediated by the interaction between YAP/TAZ and TEAD are tumors, liver fibrosis and renal fibrosis.

In some embodiments, the diseases mediated by the interaction between YAP/TAZ and TEAD are benign tumors.

In some embodiments, the diseases mediated by the interaction between YAP/TAZ and TEAD are malignant tumors, such as cancers or sarcomas.

In some embodiments, the diseases mediated by the interaction between YAP/TAZ and TEAD are solid tumors or hematological tumors.

In some embodiments, the diseases mediated by the interaction between YAP/TAZ and TEAD are: mesothelioma (e.g., pleural mesothelioma (such as malignant pleural mesothelioma), peritoneal mesothelioma, pericardial mesothelioma or tunica vaginalis mesothelioma), cervical squamous cell carcinoma, endometrial carcinoma, bladder urothelial carcinoma, skin squamous cell carcinoma, poroma (e.g., benign poroma), porocarcinoma, epithelioid hemangioendothelioma, breast cancer (e.g., triple-negative breast cancer), lung cancer (e.g., non-small cell lung cancer), ovarian cancer, colorectal cancer, melanoma, pancreatic cancer, prostate cancer, gastric cancer, esophageal cancer (e.g., esophageal squamous cell carcinoma or esophageal adenocarcinoma), liver cancer (e.g., hepatocellular carcinoma or hepatoblastoma), cholangiocarcinoma, neurilemmoma, renal cancer, sarcoma (e.g., rhabdomyosarcoma, embryonal rhabdomyosarcoma, osteosarcoma, undifferentiated pleomorphic sarcoma, Kaposi's sarcoma and soft tissue sarcoma (such as rare soft tissue sarcoma)), bone cancer, brain cancer (e.g., ependymoma (e.g., supratentorial ependymoma, such as pediatric supratentorial ependymoma), neuroblastoma, medulloblastoma, glioma or meningioma) or head and neck cancer (e.g., head and neck squamous cell carcinoma).

In some embodiments, the disease mediated by the interaction between YAP/TAZ and TEAD is mesothelioma (e.g., pleural mesothelioma (such as malignant pleural mesothelioma), peritoneal mesothelioma, pericardial mesothelioma or tunica vaginalis mesothelioma). In some embodiments, the disease mediated by the interaction between YAP/TAZ and TEAD is malignant pleural mesothelioma (MPM), which is a rare malignant tumor of the chest. Abnormal activation of the Hippo-YAP pathway is present in about 70% of MPM patients and is considered an important cancer driver gene. Reducing the activity of the Hippo-YAP pathway by biological means and by chemical small molecules has shown good tumor growth inhibitory activity.

In some embodiments, the disease mediated by the interaction between YAP/TAZ and TEAD is breast cancer (e.g., triple negative breast cancer).

In some embodiments, the disease mediated by the interaction between YAP/TAZ and TEAD is lung cancer (e.g., non-small cell lung cancer).

In some embodiments, the disease mediated by the interaction between YAP/TAZ and TEAD is colorectal cancer.

In some embodiments, the disease mediated by the interaction between YAP/TAZ and TEAD is pancreatic cancer (e.g., pancreatic ductal adenocarcinoma (PDAC)).

In some embodiments, the disease mediated by the interaction between YAP/TAZ and TEAD is gastric cancer.

In some embodiments, the disease mediated by the interaction between YAP/TAZ and TEAD is liver cancer (e.g., hepatocellular carcinoma or hepatoblastoma).

In some embodiments, the disease mediated by the interaction between YAP/TAZ and TEAD is brain cancer (e.g., ependymoma (e.g., supratentorial ependymoma, such as pediatric supratentorial ependymoma), neuroblastoma, medulloblastoma, glioma or meningioma).

In some embodiments, the disease mediated by the interaction between YAP/TAZ and TEAD is head and neck cancer (e.g., head and neck squamous cell carcinoma).

The YAP signaling pathway can produce drug resistance to a variety of anticancer targeted drugs by mechanisms such as mediating tumor cell dormancy and resistance to apoptosis, so inhibiting the Hippo-YAP signaling pathway can increase the sensitivity of tumor cells to targeted drugs. In addition, Hippo-YAP, as a pathway that promotes tumor cell growth, is overactivated in multiple drug-resistant tumor models, and inhibiting its activity can significantly increase the sensitivity of treated tumor cells to related inhibitors. Accordingly, in some embodiments, the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure can be used to improve the sensitivity of treated tumor cells by targeted drugs (e.g., EGFR inhibitors, BRAF-targeting inhibitors, MEK-targeting inhibitors), thereby improving the therapeutic effect of these targeted drugs.

As used herein, the terms "taking", "administering", "administrating" and the like refer to methods capable of delivering a compound or pharmaceutical composition to a desired site for biological action. Any method of administration well known in the art can be used in the present disclosure. These methods include, but are not limited to, oral administration, transduodenal administration, parenteral administration (including intrapulmonary, intranasal administration; intrathecal, intravenous, subcutaneous, intraperitoneal, intramuscular, intraarterial injection or infusion), topical administration and transrectal administration. Those skilled in the art are familiar with administration techniques that can be used for the compounds and methods described herein, such as those discussed in Goodman and Gilman, The Pharmacological Basis of Therapeutics, current ed.; Pergamon; and Remington's, Pharmaceutical Sciences (current edition), Mack Publishing Co., Easton, Pa. In preferred embodiments, the compounds, the pharmaceutically acceptable salts, enantiomers, diastereomers, tautomers, solvates, isotopic substitutes, polymorphs, prodrugs or metabolites thereof, or the pharmaceutical compositions thereof of the present disclosure are administered by oral administration.

In some embodiments, the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof, or the pharmaceutical composition thereof of the present disclosure is used in combination with other compounds with pharmacological activity, for example, for the treatment of cancer. For example, in some embodiments, the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, or the pharmaceutical composition of the present disclosure is administered simultaneously, sequentially or separately in combination with one or more compounds with pharmacological activity selected from: chemotherapeutic agents, such as mitotic inhibitors, such as taxane compounds (e.g., paclitaxel or docetaxel), vinca alkaloids (e.g., vincristine, vinblastine, vinorelbine or vinflunine), other anticancer agents such as metal platinum complexes (e.g., cisplatin, carboplatin or oxaliplatin), antimetabolites (e.g., pyrimidine antagonists, such as 5-fluorouracil, 5-fluoro-2-4(1*H*,3*H*)-pyrimidinedione (5FU) or gemcitabine), hormonal antitumor drugs (e.g., flutamide), etc. In some embodiments, the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, or the pharmaceutical composition of the present disclosure is used in combination with tumor immunotherapy drugs (such as anti-PD1 antibodies) and the like well known in the art for the treatment of cancer. In some embodiments, the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, or the pharmaceutical composition of the present disclosure is used in combination with conventional radiotherapy.

Herein, "combination", "drug combination", "combination administration" or "combination therapy" refers to drug treatment obtained by mixing or combining more than one active ingredient, which includes fixed and unfixed combinations of active ingredients, and also includes the combination of two or more different treatment methods. The term "fixed combination" refers to the simultaneous administration to a patient of at least one compound described herein and at least one other active ingredient in the form of a single entity or a single dosage form. The term "unfixed combination" refers to the simultaneous administration, combined administration or sequential administration at variable intervals of at least one compound described herein and at least one other active ingredient to a patient in the form of a single entity. These are also applied to cocktail therapy, such as the administration of three or more active ingredients.

In the present disclosure, a pharmaceutical composition contains the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure and a pharmaceutically acceptable carrier or excipient (which is a generally accepted medium in the art for delivering bioactive compounds to mammals (such as humans)). The purpose of preparing active compounds into pharmaceutical compositions is to facilitate the administration to living organisms, facilitate the absorption of active ingredients, and thereby exert biological activity. Generally, the pharmaceutical composition of the present disclosure contains 0.1% to 99.5% by weight of the active pharmaceutical ingredient. In some embodiments, the pharmaceutical composition of the present disclosure contains 0.5% to 90% by weight of the active pharmaceutical ingredient, such as 1%, 1.5%, 2%, 5%, 10%, 15%, 20%, 25%, 30% or 50% by weight.

In some embodiments, the pharmaceutical composition of the present disclosure contains, in addition to the compound of formula I or any subformula thereof (including but not limited to Ia, Ib, IIa, IIb, IIc, IId, IIe, IIIa, IIIb, IVa, IVb, IVc, XI, XIa, XIb, XIIa, XIIb, XIIc, XIId, XIIe, XIIIa, XIIIb, XIVa, XIVb and XIVc) or the pharmaceutically acceptable salt, enantiomer, diastereomer, tautomer, solvate, isotopic substitute, polymorph, prodrug or metabolite thereof of the present disclosure, other known anticancer agents, including but not limited to chemotherapeutic agents, such as mitotic inhibitors, such as taxane compounds (e.g., paclitaxel or docetaxel), vinca alkaloids (e.g., vincristine, vinblastine, vinorelbine or vinflunine), other anticancer agents such as metal platinum complexes (e.g., cisplatin, carboplatin or oxaliplatin), antimetabolites (e.g., pyrimidine antagonists, such as 5-fluorouracil, 5-fluoro-2-4(1*H*,3*H*)-pyrimidinedione (5FU), gemcitabine), hormonal antitumor drugs (e.g., flutamide), tumor immunotherapy drugs (such as anti-PD1 antibody), etc.

In another aspect, the present disclosure also provides the following embodiments:
1. A compound of the following formula I or a pharmaceutically acceptable salt, enantiomer, diastereomer, tautomer, solvate, isotopic substitute, polymorph, prodrug or metabolite thereof: 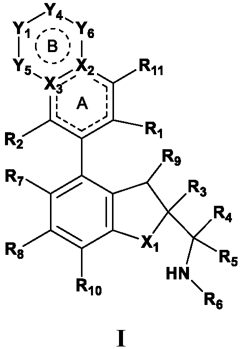 in the formula:
   X₁ is selected from: O and NRₐ;
   X₂ is selected from: C or N;
   X₃ is selected from: C or N;
   Y₅ is selected from: a bond, O, S, N, S(=O), SO₂, CHR₁₃, C(R₁₃)₂, CR₁₃, -OCH₂-, -NCH₂-, - CH₂CH₂- or NR₁₂;
   Y₁, Y₄ and Y₆ are independently selected from: a bond, O, N, C, S, -S(O)-, -SO₂-, CH, CH₂, NH, CHR₁₃, CHR₁₃, C(R₁₃)₂ or NR₁₂; alternatively, when Y₁, Y₄ and Y₆ are arbitrarily selected from C(R₁₃)₂, two R₁₃, together with the C atom to which they are attached, can form a substituted or unsubstituted 3- to 8-membered cyclohydrocarbyl or substituted or unsubstituted 4- to 9-membered heterocyclyl ring, thereby forming a spiro ring structure with ring B; alternatively, the ring atom in Y₁ or Y₆ and the ring atom in Y₄, together with the substituent attached to the ring atom, form a substituted or unsubstituted 3- to 8-membered cyclohydrocarbyl, substituted or unsubstituted 4- to 9-membered heterocyclyl, substituted or unsubstituted 6- to 10-membered aryl or substituted or unsubstituted 5- to 10-membered heteroaryl ring, thereby forming a fused ring structure with ring B;
   ring B is a 5- or 6-membered heterocyclyl, cyclohydrocarbyl or heteroaryl ring; ring B can be optionally substituted by one or more R₁₃ and/or R₁₂ that do not participate in ring formation, and can be further optionally substituted by 1 to 3 substituents selected from hydroxyl, amino, halogen, substituted or unsubstituted alkyl, -NR'R" and substituted or unsubstituted alkoxy;
   R₁ is selected from: cyano and -C(=O)NR'R";
   R₂ is selected from: H, halogen, substituted or unsubstituted alkyl and substituted or unsubstituted alkoxy;
   R₃ is selected from: substituted or unsubstituted aryl, substituted or unsubstituted heteroaryl and substituted or unsubstituted heterocyclyl;
   R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1 to 3 heteroatoms selected from O, N and S;
   R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl or substituted or unsubstituted heterocyclyl;
   alternatively, R₄ or R₅ and R₆, together with the C and N to which they are attached, form a substituted or unsubstituted 4- to 12-membered monocyclic or fused heterocyclyl, 6- to 12-membered bridged heterocyclyl or 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from O or N;
   R₇ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
   R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
   R₉ is selected from: H, substituted or unsubstituted alkyl, substituted or unsubstituted alkoxy, hydroxyl and halogen;
   R₁₀ is selected from: H, halogen and substituted or unsubstituted alkyl;
   R₁₁ is selected from: H, hydroxyl, amino, halogen, substituted or unsubstituted alkyl, -NR'R" and substituted or unsubstituted alkoxy;
   each R₁₂ is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 6- to 14-membered aryl-S(O)₂-, 4- to 9-membered heterocyclyl, NR'R"-C(O)-, C3-C8 cyclohydrocarbyl-C(O)- and substituted or unsubstituted C1-C4 alkyl;
   each R₁₃ is independently selected from: H, halogen, oxo (=O), substituted or unsubstituted C1-C4 acyl, -OH, cyano, -NR'R", substituted or unsubstituted C1-C4 alkoxy, substituted or unsubstituted C1-6 alkyl, substituted or unsubstituted C1-C4 alkyl-S(O)₂-, substituted or unsubstituted C6-14 aryl-S(O)₂-, C3-C6 cyclohydrocarbyl-C(O)- and -C(O)-NR'R";
   and after R₁₃ or R₁₂ participates in ring formation, its ring atom can be optionally substituted by 1 to 3 heteroatoms selected from O, N and S;
   R' and R" are each independently selected from H, C1-C4 alkyl, C1-C4 alkyl-S(O)₂-, C1-C4 haloalkyl;
   Ra is selected from H or C1-C4 alkyl;
   wherein the dashed line indicates the presence or absence of a double bond.
2. The compound or the pharmaceutically acceptable salt, enantiomer, diastereomer, tautomer, solvate, isotopic substitute, polymorph, prodrug or metabolite thereof according to embodiment 1, characterized in that the compound of formula I has a structure shown in the following formula Ia: 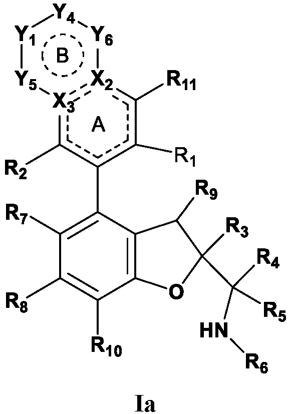
   in the formula, Y₁ and Y₄ are independently selected from: a bond, O, N, CH, CH₂, CHR₁₃, CR₁₃, C(R₁₃)₂ or NR₁₂; and Y₁ and Y₄ are not simultaneously a bond or O;
   Y₅ and Y₆ are independently selected from: O, N, S, -S(O)-, -SO₂-, CH, CH₂, NH, CHR₁₃, CR₁₃, C(R₁₃)₂ or NR₁₂;
   ring B, R₁ to R₁₃, X₂ and X₃ are as defined in embodiment 1;
   wherein the dashed line indicates the presence or absence of a double bond.
3. The compound or the pharmaceutically acceptable salt, enantiomer, diastereomer, tautomer, solvate, isotopic substitute, polymorph, prodrug or metabolite thereof according to embodiment 1 or 2, characterized in that the compound of formula I has a structure shown in the following formula Ib: 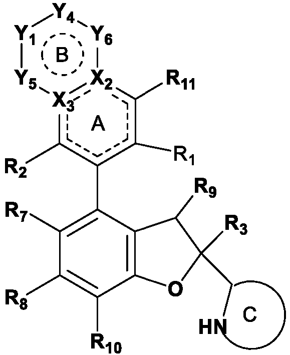 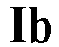
   in the formula, Y₁, Y₄, Y₅ and Y₆ are as defined in embodiment 2; R₁ to R₃, R₇ to R₁₃, X₂ and X₃ are as defined in embodiment 1;
   ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring; the 4- to 12-membered monocyclic or fused heterocyclyl, substituted or unsubstituted 6- to 12-membered bridged heterocyclyl or substituted or unsubstituted 6- to 12-membered spiro heterocyclyl can optionally further contain 1, 2 or 3 heteroatoms selected from N and O;
   wherein the dashed line indicates the presence or absence of a double bond.
4. The compound or the pharmaceutically acceptable salt, enantiomer, diastereomer, tautomer, solvate, isotopic substitute, polymorph, prodrug or metabolite thereof according to any one of embodiments 1 to 3, characterized in that when Y₁ or Y₄ or Y₅ or Y₆ is NR₁₂, R₁₂ is selected from: H, substituted or unsubstituted C1-C4 acyl, substituted or unsubstituted C1-C4 alkoxy, substituted or unsubstituted C1-C4 alkyl-S(O)₂-, substituted or unsubstituted 4- to 9-membered heterocyclyl, NR'R"-C(O)-, C3-C8 cyclohydrocarbyl-C(O)- and substituted or unsubstituted C1-C4 alkyl; R' and R" are each independently selected from H and C1-C4 alkyl; preferably, the C1-C4 acyl, C1-C4 alkoxy, C1-C4 alkyl-S(O)₂-, 4- to 9-membered heterocyclyl and C1-C4 alkyl are optionally substituted by 1 to 3 substituents selected from F, hydroxyl and cyano.
5. The compound or the pharmaceutically acceptable salt, enantiomer, diastereomer, tautomer, solvate, isotopic substitute, polymorph, prodrug or metabolite thereof according to any one of embodiments 1 to 4, characterized in that when Y₁ or Y₄ or Y₅ or Y₆ is NR₁₂, R₁₂ is selected from: H, acetyl, propionyl, C1-C3 alkoxy, C1-C3 alkyl-S(O)₂-, 4- to 6-membered heterocyclyl, C3-C5 cyclohydrocarbyl-C(O)- and substituted or unsubstituted C1-C4 alkyl; preferably, the C1-C4 alkyl is optionally substituted by 1 to 3 substituents selected from F, hydroxyl and cyano.
6. The compound or the pharmaceutically acceptable salt, enantiomer, diastereomer, tautomer, solvate, isotopic substitute, polymorph, prodrug or metabolite thereof according to any one of embodiments 1 to 5, characterized in that when Y₁ or Y₄ or Y₅ or Y₆ is arbitrarily selected from CHR₁₃, CR₁₃ or C(R₁₃)₂, R₁₃ is selected from: H, halogen, oxo (=O), substituted or unsubstituted C1-C4 acyl, -OH, cyano, -NR'R", substituted or unsubstituted C1-C4 alkoxy, substituted or unsubstituted C1-C6 alkyl, substituted or unsubstituted C1-C4 alkyl-S(O)₂-, C3-C6 cyclohydrocarbyl-C(O)- and -C(O)-NR'R"; R' and R" are each independently selected from H, C1-C4 alkyl; preferably, the C1-C4 acyl, C1-C4 alkoxy and C1-C6 alkyl are optionally substituted by 1 to 3 substituents selected from F, hydroxyl and cyano.
7. The compound or the pharmaceutically acceptable salt, enantiomer, diastereomer, tautomer, solvate, isotopic substitute, polymorph, prodrug or metabolite thereof according to any one of embodiments 1 to 6, characterized in that when Y₁ or Y₄ or Y₅ or Y₆ is arbitrarily selected from CHR₁₃, CR₁₃ or C(R₁₃)₂, R₁₃ is selected from: H, fluorine, chlorine, oxo (=O), C1-C4 acyl, -OH, cyano, C1-C4 alkoxy, C1-C6 alkyl, C1-C4 alkyl-S(O)₂- and C3-C4 cyclohydrocarbyl-C(O)-.
8. The compound or the pharmaceutically acceptable salt, enantiomer, diastereomer, tautomer, solvate, isotopic substitute, polymorph, prodrug or metabolite thereof according to embodiment 1, characterized in that the compound of formula I has a structure shown in the following formula IIa: 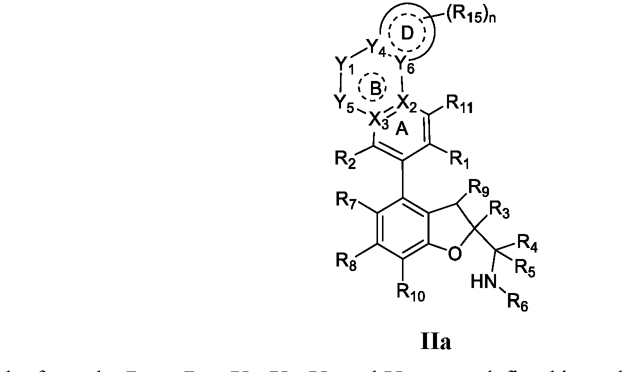
   in the formula, R₁ to R₁₃, X₂, X₃, Y₁ and Y₅ are as defined in embodiment 1;
   Y₄ is selected from: C, CH or N;
   Y₆ is selected from: C, CH or N;
   ring D is a C3-C8 cyclohydrocarbyl, C4-C9 heterocyclyl, C5-C10 heteroaryl or C6-C10 aryl ring; the C4-C9 heterocyclyl and C5-C10 heteroaryl can optionally contain 1 to 3 heteroatoms selected from O, N and S;
   R₁₅ is selected from: H, halogen, oxo (=O), substituted or unsubstituted C1-C4 acyl, hydroxyl, substituted or unsubstituted C1-C4 alkoxy, cyano, -NR'R", -C(O)-NR'R", substituted or unsubstituted C1-C4 alkyl-S(O)₂-, substituted or unsubstituted C6-C14 aryl-S(O)₂-, 4- to 9-membered heterocyclyl, NR'R"-C(O)-, C3-C8 cyclohydrocarbyl, C3-C8 cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 haloalkyl and C1-C4 alkyl are optionally substituted by 1 to 3 substituents selected from F, hydroxyl, and cyano;
   each n is independently 0, 1, 2, 3 or 4;
   wherein the dashed line indicates the presence or absence of a double bond.
9. The compound or the pharmaceutically acceptable salt, enantiomer, diastereomer, tautomer, solvate, isotopic substitute, polymorph, prodrug or metabolite thereof according to embodiment 1, characterized in that the compound of formula I has a structure shown in the following formula lib: 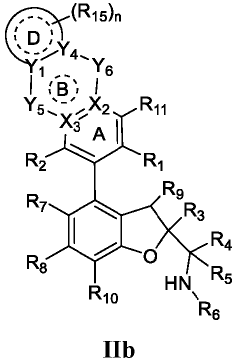
   in the formula, R₁ to R₁₃, X₂, X₃, Y₅ and Y₆ are as defined in embodiment 1;
   Y₁ is selected from: C, CH or N;
   Y₄ is selected from: C, CH or N;
   ring D, R₁₅ and n are as defined in embodiment 8;
   wherein the dashed line indicates the presence or absence of a double bond.
10. The compound or the pharmaceutically acceptable salt, enantiomer, diastereomer, tautomer, solvate, isotopic substitute, polymorph, prodrug or metabolite thereof according to embodiment 8 or 9, characterized in that ring D is a C3-C6 cyclohydrocarbyl, C4-C6 heterocyclyl, C5-C8 heteroaryl or C6-C10 aryl ring; the C4-C6 heterocyclyl and C5-C8 heteroaryl can optionally contain 1 to 3 heteroatoms selected from O, N and S; R₁₅ is selected from: H, halogen, substituted or unsubstituted C1-C4 acyl, hydroxyl, substituted or unsubstituted C1-C4 alkoxy, cyano, -NR'R", -C(O)-NR'R", 4- to 9-membered heterocyclyl, NR'R"-C(O)-, C3-C8 cyclohydrocarbyl, C3-C8 cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 haloalkyl and C1-C4 alkyl are optionally substituted by 1 to 3 substituents selected from F, hydroxyl and cyano; R' and R" are each independently selected from H, C1-C4 alkyl; n is 0, 1 or 2.
11. The compound or the pharmaceutically acceptable salt, enantiomer, diastereomer, tautomer, solvate, isotopic substitute, polymorph, prodrug or metabolite thereof according to any one of embodiments 8 to 10, characterized in that R₁₅ is selected from: H, fluorine, chlorine, acetyl, hydroxyl, C1-C4 alkoxy, cyano, -NR'R", -C(O)-NR'R", NR'R"-C(O)-, C3-C8 cyclohydrocarbyl-C(O)-, C1-C4 haloalkyl and C1-C4 alkyl; R' and R" are each independently selected from H, C1-C4 alkyl; n is 0 or 1.
12. The compound or the pharmaceutically acceptable salt, enantiomer, diastereomer, tautomer, solvate, isotopic substitute, polymorph, prodrug or metabolite thereof according to embodiment 1, characterized in that the compound of formula I has a structure shown in the following formula IIc: 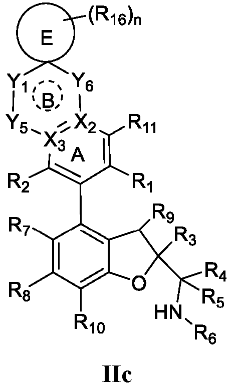
   in the formula, R₁ to R₁₃, X₂, X₃, Y₁, Y₅ and Y₆ are as defined in embodiment 1;
   ring E is a C3-C8 cyclohydrocarbyl or C4-C9 heterocyclyl ring; the C4-C9 heterocyclyl can optionally contain 1 to 3 heteroatoms selected from O, N and S;
   R₁₆ is selected from halogen, substituted or unsubstituted C1-C4 alkyl, hydroxyl, substituted or unsubstituted C1-C4 acyl and substituted or unsubstituted C1-C4 alkoxy; preferably, the C1-C4 acyl, C1-C4 alkoxy and C1-C4 alkyl are optionally substituted by 1 to 3 substituents selected from F, hydroxyl and cyano;
   n is 0, 1, 2, 3 or 4;
   wherein the dashed line indicates the presence or absence of a double bond.
13. The compound or the pharmaceutically acceptable salt, enantiomer, diastereomer, tautomer, solvate, isotopic substitute, polymorph, prodrug or metabolite thereof according to embodiment 1, characterized in that the compound of formula I has a structure shown in the following formula IIc: 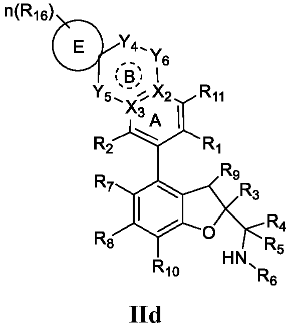
   in the formula, R₁ to R₁₃, X₂, X₃, Y₄, Y₅ and Y₆ are as defined in embodiment 1;
   ring E, R₁₆ and n are as defined in embodiment 12;
   wherein the dashed line indicates the presence or absence of a double bond.
14. The compound or the pharmaceutically acceptable salt, enantiomer, diastereomer, tautomer, solvate, isotopic substitute, polymorph, prodrug or metabolite thereof according to embodiment 12 or 13, characterized in that ring E is a C3-C6 cyclohydrocarbyl or C4-C6 heterocyclyl ring; the C4-C6 heterocyclyl can optionally contain 1 to 3 heteroatoms selected from O, N and S; R₁₆ is selected from H, halogen, C1-C4 alkyl and hydroxyl; n is 0 or 1.
15. The compound or the pharmaceutically acceptable salt, enantiomer, diastereomer, tautomer, solvate, isotopic substitute, polymorph, prodrug or metabolite thereof according to any one of embodiments 12 to 15, wherein ring E is a cyclopropyl, cyclobutyl, cyclopentyl, cyclohexyl, oxetanyl, oxolanyl, azetidinyl, azacyclopentyl ring; R₁₆ is selected from H, fluorine, methyl, ethyl, hydroxyl.
16. The compound or the pharmaceutically acceptable salt, enantiomer, diastereomer, tautomer, solvate, isotopic substitute, polymorph, prodrug or metabolite thereof according to embodiment 8, characterized in that the compound of formula IIa has a structure shown in the following formula IIIa: 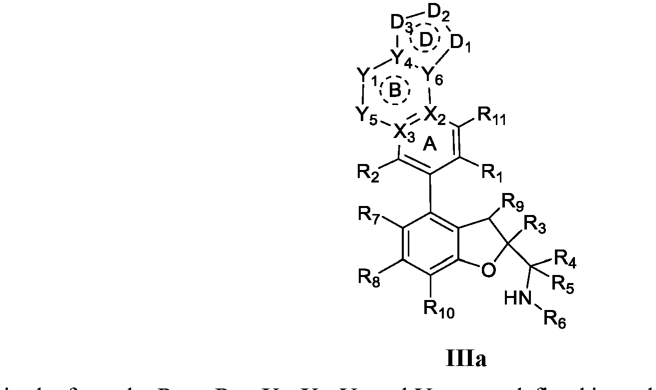
   in the formula, R₁ to R₁₃, X₂, X₃, Y₁ and Y₅ are as defined in embodiment 1;
   Y₄ and Y₆ are as defined in embodiment 8;
   D₁ is selected from: O, S, S(O)₂, N, NRₑ, CR_{f} and CR_{f}R_{g};
   D₂ is selected from: O, S, S(O)₂, N, NRₑ, (CR_{f})ₚ and (CR_{f}R_{g})ₚ;
   D₃ is selected from: O, S, S(O)₂, N, NRₑ, (CR_{f})ₚ and (CR_{f}R_{g})ₚ;
   each Rₑ is independently selected from: H, substituted or unsubstituted C1-C4 acyl, substituted or unsubstituted C1-C4 alkoxy, cyano, substituted or unsubstituted C1-C4 alkyl-S(O)₂-, 4- to 9-membered heteroaryl, 4- to 9-membered heterocyclyl, NR'R"-C(O)-, C3-C6 cyclohydrocarbyl, C3-C6 cyclohydrocarbyl-C(O)-, substituted or unsubstituted C1-C4 haloalkyl and substituted or unsubstituted C1-C4 alkyl; wherein R' and R" are each independently selected from H and C1-C4 alkyl; preferably, the C1-C4 acyl, C1-C4 alkoxy and C1-C4 alkyl are optionally substituted by 1 to 3 substituents selected from F, hydroxyl and cyano;
   each Rf is independently selected from: H, halogen, oxo (=O), substituted or unsubstituted C1-C4 acyl, -OH, cyano, -NR'R", substituted or unsubstituted C1-C4 alkoxy, substituted or unsubstituted C1-C4 alkyl, C3-C6 cyclohydrocarbyl, substituted or unsubstituted C1-C4 alkylS(O)₂-, C3-C6 cyclohydrocarbyl-C(O)- and -C(O)-NR'R"; wherein R' and R" are each independently selected from H and C1-C4 alkyl; preferably, the C1-C4 acyl, C1-C4 alkoxy and C1-C4 alkyl are optionally substituted by 1 to 3 substituents selected from F, hydroxyl and cyano;
   each R_{g} is independently selected from: H, halogen, substituted or unsubstituted C1-C4 acyl, -OH, cyano, -NR'R", substituted or unsubstituted C1-C4 alkoxy, substituted or unsubstituted C1-C4 alkyl, C3-C6 cyclohydrocarbyl, substituted or unsubstituted C1-C4 alkyl-S(O)₂-, C3-C6 cyclohydrocarbyl-C(O)- and -C(O)-NR'R"; wherein R' and R" are each independently selected from H and C1-C4 alkyl; preferably, the C1-C4 acyl, C1-C4 alkoxy and C1-C4 alkyl are optionally substituted by 1 to 3 substituents selected from F, hydroxyl and cyano; each p is independently an integer from 1 to 4, preferably 1 or 2;
   wherein the dashed line indicates the presence or absence of a double bond.
17. The compound or the pharmaceutically acceptable salt, enantiomer, diastereomer, tautomer, solvate, isotopic substitute, polymorph, prodrug or metabolite thereof according to embodiment 9, characterized in that the compound of formula IIb has a structure shown in the following formula IIIb: 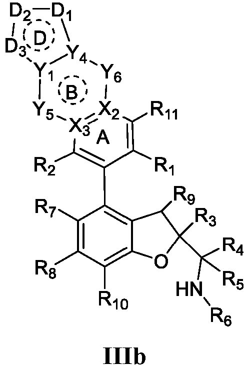
   in the formula, R₁ to R₁₃, X₂, X₃, Y₅ and Y₆ are as defined in embodiment 1;
   Y₁ and Y₄ are as defined in embodiment 9;
   D₁, D₂ and D₃ are as defined in embodiment 16;
   wherein the dashed line indicates the presence or absence of a double bond.
18. The compound or the pharmaceutically acceptable salt, enantiomer, diastereomer, tautomer, solvate, isotopic substitute, polymorph, prodrug or metabolite thereof according to embodiment 16, characterized in that the compound of formula IIIa has a structure shown in the following formula IVa: 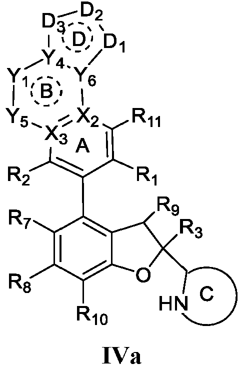
   in the formula, R₁ to R₁₃, X₂, X₃, Y₁ and Y₅ are as defined in embodiment 1;
   Y₄ and Y₆ are as defined in embodiment 8;
   D₁, D₂ and D₃ are as defined in embodiment 16;
   ring C is as defined in embodiment 3;
   wherein the dashed line indicates the presence or absence of a double bond.
19. The compound or the pharmaceutically acceptable salt, enantiomer, diastereomer, tautomer, solvate, isotopic substitute, polymorph, prodrug or metabolite thereof according to embodiment 17, characterized in that the compound of formula IIIb has a structure shown in the following formula IVb: 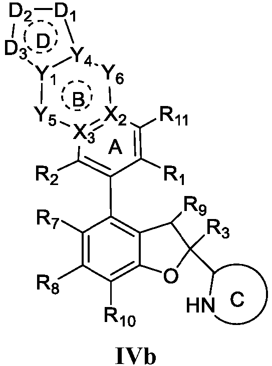
   in the formula, R₁ to R₁₃, X₂, X₃, Y₅ and Y₆ are as defined in embodiment 1;
   Y₁ and Y₄ are as defined in embodiment 9;
   D₁, D₂ and D₃ are as defined in embodiment 16;
   ring C is as defined in embodiment 3;
   wherein the dashed line indicates the presence or absence of a double bond.
20. The compound or the pharmaceutically acceptable salt, enantiomer, diastereomer, tautomer, solvate, isotopic substitute, polymorph, prodrug or metabolite thereof according to any one of embodiments 1 to 19, characterized in that R₁ is -C(=O)NR'R"; R' and R" are each independently selected from H, C1-C4 alkyl, C1-C4 alkyl-S(O)₂-, C1-C4 haloalkyl.
21. The compound or the pharmaceutically acceptable salt, enantiomer, diastereomer, tautomer, solvate, isotopic substitute, polymorph, prodrug or metabolite thereof according to any one of embodiments 1 to 20, characterized in that R₁ is -C(=O)NHR'; the R' is selected from H and C1-C4 alkyl.
22. The compound or the pharmaceutically acceptable salt, enantiomer, diastereomer, tautomer, solvate, isotopic substitute, polymorph, prodrug or metabolite thereof according to any one of embodiments 1 to 21, characterized in that R₁ is -C(=O)NH₂.
23. The compound or the pharmaceutically acceptable salt, enantiomer, diastereomer, tautomer, solvate, isotopic substitute, polymorph, prodrug or metabolite thereof according to any one of embodiments 1 to 21, characterized in that R₁ is -C(=O)NHCH₃.
24. The compound or the pharmaceutically acceptable salt, enantiomer, diastereomer, tautomer, solvate, isotopic substitute, polymorph, prodrug or metabolite thereof according to any one of embodiments 1 to 23, characterized in that R₂ is selected from H, halogen, C1-C4 alkyl and C1-C4 alkoxy.
25. The compound or the pharmaceutically acceptable salt, enantiomer, diastereomer, tautomer, solvate, isotopic substitute, polymorph, prodrug or metabolite thereof according to any one of embodiments 1 to 24, characterized in that R₂ is selected from H, halogen and methoxy.
26. The compound or the pharmaceutically acceptable salt, enantiomer, diastereomer, tautomer, solvate, isotopic substitute, polymorph, prodrug or metabolite thereof according to any one of embodiments 1 to 25, characterized in that R₂ is selected from halogen.
27. The compound or the pharmaceutically acceptable salt, enantiomer, diastereomer, tautomer, solvate, isotopic substitute, polymorph, prodrug or metabolite thereof according to any one of embodiments 1 to 26, characterized in that R₂ is selected from fluorine and chlorine.
28. The compound or the pharmaceutically acceptable salt, enantiomer, diastereomer, tautomer, solvate, isotopic substitute, polymorph, prodrug or metabolite thereof according to any one of embodiments 1 to 27, characterized in that R₂ is selected from fluorine.
29. The compound or the pharmaceutically acceptable salt, enantiomer, diastereomer, tautomer, solvate, isotopic substitute, polymorph, prodrug or metabolite thereof according to any one of embodiments 1 to 28, characterized in that R₃ is selected from substituted or unsubstituted aryl.
30. The compound or the pharmaceutically acceptable salt, enantiomer, diastereomer, tautomer, solvate, isotopic substitute, polymorph, prodrug or metabolite thereof according to any one of embodiments 1 to 29, characterized in that R₃ is selected from substituted or unsubstituted phenyl.
31. The compound or the pharmaceutically acceptable salt, enantiomer, diastereomer, tautomer, solvate, isotopic substitute, polymorph, prodrug or metabolite thereof according to any one of embodiments 1 to 30, characterized in that R₃ is selected from phenyl.
32. The compound or the pharmaceutically acceptable salt, enantiomer, diastereomer, tautomer, solvate, isotopic substitute, polymorph, prodrug or metabolite thereof according to any one of embodiments 1 to 31, characterized in that R₄ and R₅ are each independently selected from: H, substituted or unsubstituted alkyl; alternatively, R₄ and R₅, together with the carbon atom to which they are attached, form a 3- to 6-membered substituted or unsubstituted ring, and the ring optionally contains 1 to 3 heteroatoms selected from O, N and S.
33. The compound or the pharmaceutically acceptable salt, enantiomer, diastereomer, tautomer, solvate, isotopic substitute, polymorph, prodrug or metabolite thereof according to any one of embodiments 1 to 32, characterized in that R₄ and R₅ are each independently selected from: H, substituted or unsubstituted C1-C4 alkyl.
34. The compound or the pharmaceutically acceptable salt, enantiomer, diastereomer, tautomer, solvate, isotopic substitute, polymorph, prodrug or metabolite thereof according to any one of embodiments 1 to 33, characterized in that R₄ and R₅ are each independently selected from: H, C1-C3 alkyl.
35. The compound or the pharmaceutically acceptable salt, enantiomer, diastereomer, tautomer, solvate, isotopic substitute, polymorph, prodrug or metabolite thereof according to any one of embodiments 1 to 34, characterized in that R₄ and R₅ are each independently selected from: H, methyl.
36. The compound or the pharmaceutically acceptable salt, enantiomer, diastereomer, tautomer, solvate, isotopic substitute, polymorph, prodrug or metabolite thereof according to any one of embodiments 1 to 35, characterized in that R₄ and R₅ are both H.
37. The compound or the pharmaceutically acceptable salt, enantiomer, diastereomer, tautomer, solvate, isotopic substitute, polymorph, prodrug or metabolite thereof according to any one of embodiments 1 to 36, characterized in that R₇ and R₈ are each independently selected from: H, halogen, cyano, substituted or unsubstituted C1-C4 alkyl, substituted or unsubstituted C1-C4 haloalkyl, substituted or unsubstituted C1-C4 alkoxy, substituted or unsubstituted alkenyl and substituted or unsubstituted alkynyl.
38. The compound or the pharmaceutically acceptable salt, enantiomer, diastereomer, tautomer, solvate, isotopic substitute, polymorph, prodrug or metabolite thereof according to any one of embodiments 1 to 37, characterized in that R₇ and R₈ are each independently selected from: H, halogen, cyano, methyl and methoxy.
39. The compound or the pharmaceutically acceptable salt, enantiomer, diastereomer, tautomer, solvate, isotopic substitute, polymorph, prodrug or metabolite thereof according to any one of embodiments 1 to 38, characterized in that R₇ and R₈ are each independently selected from: fluorine and chlorine.
40. The compound or the pharmaceutically acceptable salt, enantiomer, diastereomer, tautomer, solvate, isotopic substitute, polymorph, prodrug or metabolite thereof according to any one of embodiments 1 to 39, characterized in that R₇ is chlorine, R₈ is fluorine.
41. The compound or the pharmaceutically acceptable salt, enantiomer, diastereomer, tautomer, solvate, isotopic substitute, polymorph, prodrug or metabolite thereof according to any one of embodiments 1 to 40, characterized in that R₉ is selected from H, C1-C4 alkyl, C1-C4 alkoxy, hydroxyl and halogen.
42. The compound or the pharmaceutically acceptable salt, enantiomer, diastereomer, tautomer, solvate, isotopic substitute, polymorph, prodrug or metabolite thereof according to any one of embodiments 1 to 41, characterized in that R₉ is selected from H, methyl, methoxy, hydroxyl and F.
43. The compound or the pharmaceutically acceptable salt, enantiomer, diastereomer, tautomer, solvate, isotopic substitute, polymorph, prodrug or metabolite thereof according to any one of embodiments 1 to 42, characterized in that R₉ is selected from H.
44. The compound or the pharmaceutically acceptable salt, enantiomer, diastereomer, tautomer, solvate, isotopic substitute, polymorph, prodrug or metabolite thereof according to any one of embodiments 1 to 43, characterized in that R₁₀ is selected: from H, halogen and C1-C4 alkyl.
45. The compound or the pharmaceutically acceptable salt, enantiomer, diastereomer, tautomer, solvate, isotopic substitute, polymorph, prodrug or metabolite thereof according to any one of embodiments 1 to 44, characterized in that R₁₀ is selected from: H, F and methyl.
46. The compound or the pharmaceutically acceptable salt, enantiomer, diastereomer, tautomer, solvate, isotopic substitute, polymorph, prodrug or metabolite thereof according to any one of embodiments 1 to 45, characterized in that R₁₀ is H.
47. The compound or the pharmaceutically acceptable salt, enantiomer, diastereomer, tautomer, solvate, isotopic substitute, polymorph, prodrug or metabolite thereof according to any one of embodiments 1 to 46, characterized in that R₁₁ is selected from: H, hydroxyl, amino, halogen, C1-C4 alkyl, -NR'R" and C1-C4 alkoxy, wherein R' and R" are independently selected from C1-C4 alkyl.
48. The compound or the pharmaceutically acceptable salt, enantiomer, diastereomer, tautomer, solvate, isotopic substitute, polymorph, prodrug or metabolite thereof according to any one of embodiments 1 to 47, characterized in that R₁₁ is selected from: H, hydroxyl, amino, fluorine, chlorine, methyl, methoxy and -NCH₃.
49. The compound or the pharmaceutically acceptable salt, enantiomer, diastereomer, tautomer, solvate, isotopic substitute, polymorph, prodrug or metabolite thereof according to any one of embodiments 1 to 48, characterized in that R₁₁ is H.
50. The compound or the pharmaceutically acceptable salt, enantiomer, diastereomer, tautomer, solvate, isotopic substitute, polymorph, prodrug or metabolite thereof according to any one of embodiments 1 to 49, characterized in that X₂ is C.
51. The compound or the pharmaceutically acceptable salt, enantiomer, diastereomer, tautomer, solvate, isotopic substitute, polymorph, prodrug or metabolite thereof according to any one of embodiments 1 to 49, characterized in that X₂ is N.
52. The compound or the pharmaceutically acceptable salt, enantiomer, diastereomer, tautomer, solvate, isotopic substitute, polymorph, prodrug or metabolite thereof according to any one of embodiments 1 to 51, characterized in that X₃ is C.
53. The compound or the pharmaceutically acceptable salt, enantiomer, diastereomer, tautomer, solvate, isotopic substitute, polymorph, prodrug or metabolite thereof according to any one of embodiments 1 to 50, characterized in that X₃ is N.
54. The compound or the pharmaceutically acceptable salt, enantiomer, diastereomer, tautomer, solvate, isotopic substitute, polymorph, prodrug or metabolite thereof according to any one of embodiments 1 to 53, characterized in that Y₅ is selected from: a bond, O, S, N, CH₂, CH, NH and NCH₃.
55. The compound or the pharmaceutically acceptable salt, enantiomer, diastereomer, tautomer, solvate, isotopic substitute, polymorph, prodrug or metabolite thereof according to any one of embodiments 1 to 54, characterized in that Y₅ is O.
56. The compound or the pharmaceutically acceptable salt, enantiomer, diastereomer, tautomer, solvate, isotopic substitute, polymorph, prodrug or metabolite thereof according to any one of embodiments 1 to 54, characterized in that Y₅ is CH or CH₂.
57. The compound or the pharmaceutically acceptable salt, enantiomer, diastereomer, tautomer, solvate, isotopic substitute, polymorph, prodrug or metabolite thereof according to any one of embodiments 1 to 54, characterized in that Y₅ is NH or N.
58. The compound or the pharmaceutically acceptable salt, enantiomer, diastereomer, tautomer, solvate, isotopic substitute, polymorph, prodrug or metabolite thereof according to any one of embodiments 2 to 7, 9, 12 to 13, 17 and 19 to 56, characterized in that Y₆ is selected from: a bond, O, S, N, CH, CH₂, NH or NCH₃.
59. The compound or the pharmaceutically acceptable salt, enantiomer, diastereomer, tautomer, solvate, isotopic substitute, polymorph, prodrug or metabolite thereof according to any one of embodiments 2 to 7, 8, 12, 16 and 18 to 53, characterized in that Y₁ is selected from: a bond, O, NH, -NCH₃, CH, CH₂ and -CH(CH₃)-.
60. The compound or the pharmaceutically acceptable salt, enantiomer, diastereomer, tautomer, solvate, isotopic substitute, polymorph, prodrug or metabolite thereof according to any one of embodiments 1 to 59, characterized in that R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted C3-C8 cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl.
61. The compound or the pharmaceutically acceptable salt, enantiomer, diastereomer, tautomer, solvate, isotopic substitute, polymorph, prodrug or metabolite thereof according to any one of embodiments 1 to 60, characterized in that R₆ is H, substituted C1-C6 alkyl, substituted C1-C6 alkoxy, substituted C2-C6 alkenyl or substituted C2-C6 alkynyl, and their substituents are 1 to 3 substituents selected from: hydroxyl, halogen, C1-C4 alkoxy optionally substituted by halogen, cyano, C1-C4 alkyl-S(O)₂- optionally substituted by halogen, sulfonyl, C1-C4 alkyl optionally substituted by halogen, -S(O)₂-NR'R", sulfanilamido, -C(O)-C1-C4 alkyl, -NR'-C(O)-C1-C4 alkyl and 6- to 14-membered aryl, 5- to 10-membered heteroaryl, C3-C8 cyclohydrocarbyl and 4-to 9-membered heterocyclyl, wherein the 6- to 14-membered aryl, 5- to 10-membered heteroaryl, C3-C8 cyclohydrocarbyl and 4- to 9-membered heterocyclyl are optionally substituted by substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, NR'R", 6- to 14-membered aryl, 5- to 10-membered heteroaryl, C3-C8 cyclohydrocarbyl and 4- to 9-membered heterocyclyl; wherein R' and R" are each independently selected from H and C1-C4 alkyl; or
   R₆ is substituted C3-C8 cyclohydrocarbyl or substituted 4- to 9-membered monocyclic or fused heterocyclyl, and their substituents are 1 to 3 substituents selected from: halogen, hydroxyl, cyano, -NR'R", C1-C4 alkoxy, C1-C4 haloalkoxy, -C(O)-C1-C4 alkyl and C1-C4 alkoxycarbonyl, and the C1-C4 alkyl and C1-C4 alkoxy are optionally substituted by 1 to 3 groups selected from halogen, hydroxyl and -NR'R", wherein the R' and R" are each independently selected from H and C1-C4 alkyl; or
   R₆ is substituted 6- to 12-membered bridged cyclyl, substituted 6- to 12-membered bridged heterocyclyl, substituted 6- to 12-membered spiro cyclyl or substituted 6- to 12-membered spiro heterocyclyl, and their substituents are 1 to 3 substituents selected from: C1-C4 alkyl, C1-C4 haloalkyl, C1-C4 alkoxy, C1-C4 haloalkoxy, cyano, C1-C4 alkyl-S(O)₂-, hydroxyl, halogen and NR'R", wherein the R' and R" are each independently selected from H and C1-C4 alkyl.
62. The compound or the pharmaceutically acceptable salt, enantiomer, diastereomer, tautomer, solvate, isotopic substitute, polymorph, prodrug or metabolite thereof according to any one of embodiments 1 to 59, characterized in that R₆ is -L-W-Z, wherein L is a bond or substituted or unsubstituted C1-C4 alkyl; W is a bond, substituted or unsubstituted C1-C4 alkyl, substituted or unsubstituted C3-C8 cyclohydrocarbyl, substituted or unsubstituted 6- to 14-membered aryl, substituted or unsubstituted 5- to 10-membered heteroaryl, substituted or unsubstituted 4- to 9-membered heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl; Z is hydrogen, halogen, C1-C4 alkyl, -O-C1-C4 alkyl, hydroxyl, sulfonyl, -S(O)₂-C1-C4 alkyl, -S(O)₂-NR'R", sulfanilamido, cyano, -C(O)-C1-C4 alkyl, C3-C8 cyclohydrocarbyl-O- or -NR'-C(O)-C1-C4 alkyl, wherein R' and R" are each independently H or C1-C4 alkyl.
63. The compound or the pharmaceutically acceptable salt, enantiomer, diastereomer, tautomer, solvate, isotopic substitute, polymorph, prodrug or metabolite thereof according to embodiment 62, characterized in that the -L-W-Z is selected from: (the wavy line indicates the bond connection position) 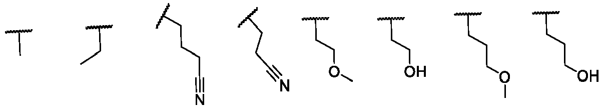 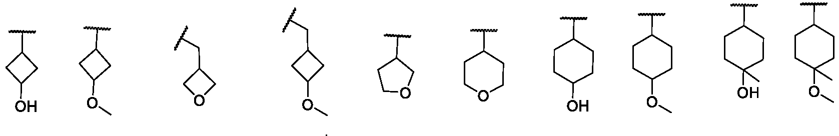 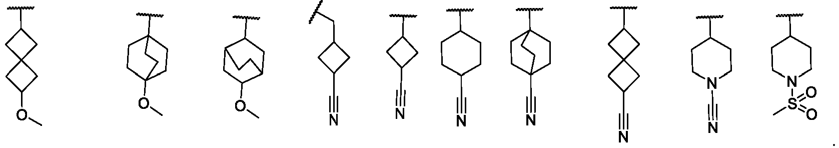
64. The compound or the pharmaceutically acceptable salt, enantiomer, diastereomer, tautomer, solvate, isotopic substitute, polymorph, prodrug or metabolite thereof according to any one of embodiments 1 to 2, 4 to 17 and 20 to 62, characterized in that R₄ or R₅ and R₆, together with the C and N to which they are attached, form a 4- to 9-membered heterocyclyl ring, and the 4- to 9-membered heterocyclyl can be substituted by 1 to 3 substituents selected from H, deuterium, hydroxyl, cyano, C1-C4 alkyl, halogen and C1-C4 alkoxy.
65. The compound or the pharmaceutically acceptable salt, enantiomer, diastereomer, tautomer, solvate, isotopic substitute, polymorph, prodrug or metabolite thereof according to any one of embodiments 1 to 2, 4 to 17, 20 to 62 and 64, characterized in that R₄ or R₅ and R₆, together with the C and N to which they are attached, form the following groups substituted by 1 to 3 substituents selected from H, deuterium, hydroxyl, methyl, F and methoxy: 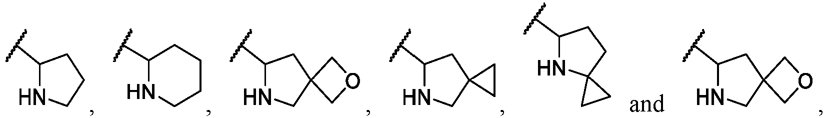 the wavy line indicates the bond connection position.
66. The compound or the pharmaceutically acceptable salt, enantiomer, diastereomer, tautomer, solvate, isotopic substitute, polymorph, prodrug or metabolite thereof according to any one of embodiments 1 to 2, 4 to 17, 20 to 62 and 64 to 65, characterized in that R₄ or R₅ and R₆, together with the C and N to which they are attached, form 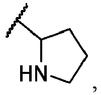 which can be substituted by 1 to 3 substituents selected from H, deuterium, hydroxyl, methyl, F and methoxy.
67. The compound or the pharmaceutically acceptable salt, enantiomer, diastereomer, tautomer, solvate, isotopic substitute, polymorph, prodrug or metabolite thereof according to any one of embodiments 3 and 18 to 19, characterized in that ring C is a 4- to 9-membered heterocyclyl ring, which can be substituted by 1 to 3 substituents selected from H, deuterium, hydroxyl, cyano, C1-C4 alkyl, halogen and C1-C4 alkoxy.
68. The compound or the pharmaceutically acceptable salt, enantiomer, diastereomer, tautomer, solvate, isotopic substitute, polymorph, prodrug or metabolite thereof according to any one of embodiments 3, 18 to 19 and 67, characterized in that ring C is the following groups substituted by 1 to 3 substituents selected from H, deuterium, hydroxyl, methyl, F and methoxy: 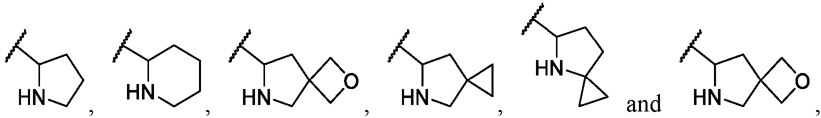 the wavy line indicates the bond connection position.
69. The compound or the pharmaceutically acceptable salt, enantiomer, diastereomer, tautomer, solvate, isotopic substitute, polymorph, prodrug or metabolite thereof according to any one of embodiments 3, 18 to 19 and 67 to 68, characterized in that ring C is 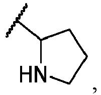 which can be substituted by 1 to 3 substituents selected from H, deuterium, hydroxyl, methyl, F and methoxy.
70. The compound or the pharmaceutically acceptable salt, enantiomer, diastereomer, tautomer, solvate, isotopic substitute, polymorph, prodrug or metabolite thereof according to any one of embodiments 1 to 7, 29 to 46 and 50 to 69, characterized in that the ring A and ring B of formulas I, Ia and Ib, together with their substituents, form any one of the following groups: 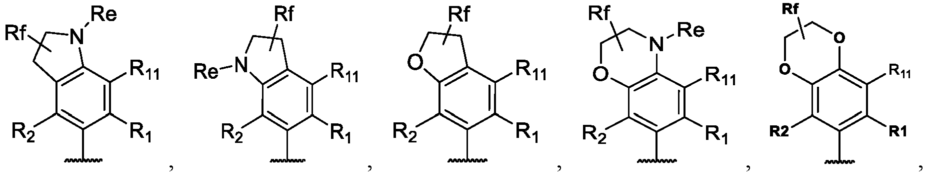 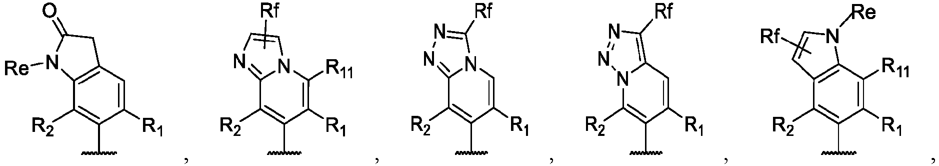 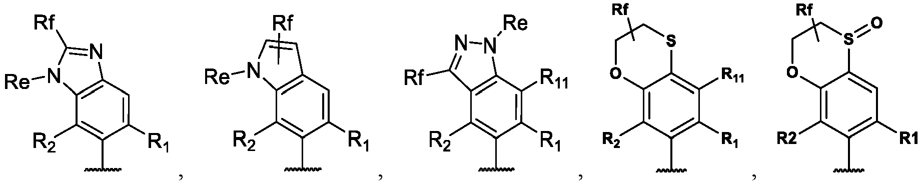 and 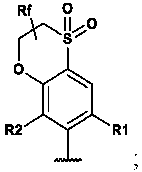 among the above groups, R₁ is cyano or -C(=O)NR'R"; R₂ is H or halogen; R₁₁ is H, OH, - NR'R", halogen or C1-C4 alkyl; Rₑ is selected from H, substituted or unsubstituted C1-C4 alkyl, substituted or unsubstituted C1-C4 alkoxy, C3-C6 cyclohydrocarbyl, substituted unsubstituted C1-C4 acyl; R_{f} is selected from H, halogen, -OH, cyano, -NR'R", substituted or unsubstituted C1-C4 alkoxy, substituted or unsubstituted C1-C4 alkyl, C3-C6 cyclohydrocarbyl; the substituted or unsubstituted C1-C4 acyl, substituted or unsubstituted C1-C4 alkoxy and substituted or unsubstituted C1-C4 alkyl can be optionally substituted by 1 to 3 substituents selected from F, hydroxyl and cyano; wherein R' and R" are each independently selected from H and C1-C4 alkyl; the wavy line indicates the bond connection position.
71. The compound or the pharmaceutically acceptable salt, enantiomer, diastereomer, tautomer, solvate, isotopic substitute, polymorph, prodrug or metabolite thereof according to embodiment 70, characterized in that R₁ is cyano, -C(=O)NH₂ or -C(=O)NHCH₃; R₂ is H or F; R₁₁ is H; Rₑ is selected from H, methyl, ethyl, cyclopropyl, cyclobutyl, ethylhydroxyl, acetyl, propionyl and cyclopropionyl; R_{f} is selected from H, F, Cl, -OH, cyano, -NH₂, methoxy, methyl, ethyl, cyclopropyl and cyclobutyl.
72. The compound or the pharmaceutically acceptable salt, enantiomer, diastereomer, tautomer, solvate, isotopic substitute, polymorph, prodrug or metabolite thereof according to embodiment 71, characterized in that R₁ is -C(=O)NH₂ or -C(=O)NHCH₃; R₂ is F; R₁₁ is H; Rₑ is selected from H, methyl, cyclopropyl and acetyl; R_{f} is selected from H, F, Cl, -OH, cyano, -NH₂, methoxy and methyl.
73. The compound or the pharmaceutically acceptable salt, enantiomer, diastereomer, tautomer, solvate, isotopic substitute, polymorph, prodrug or metabolite thereof according to any one of embodiments 1, 8 to 19, 29 to 46 and 50 to 69, characterized in that ring A and ring B of formula I together with their substituents, or ring A, ring B and ring D of formula IIa, IIb, IIIa, IIIb, IVa and IVb together with their substituents, or ring A, ring B and ring E of formula IIc and IId together with their substituents form any one of the following groups: 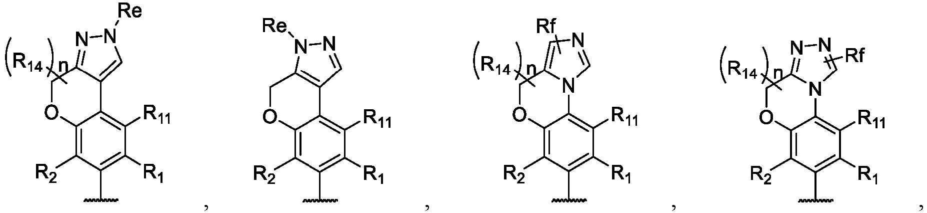 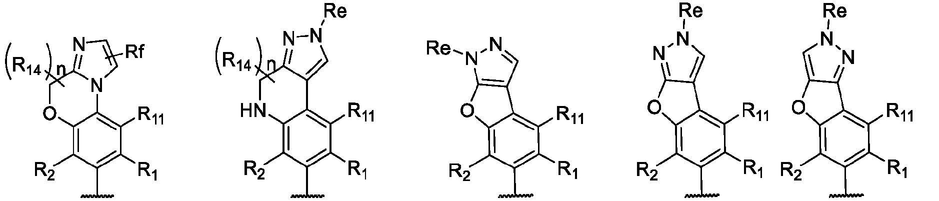 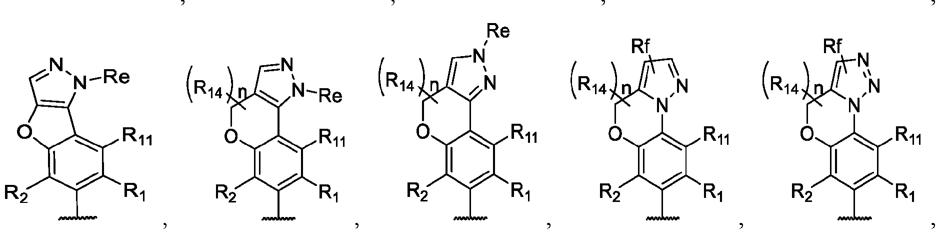 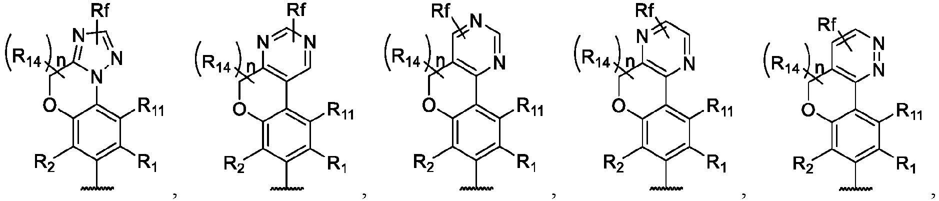 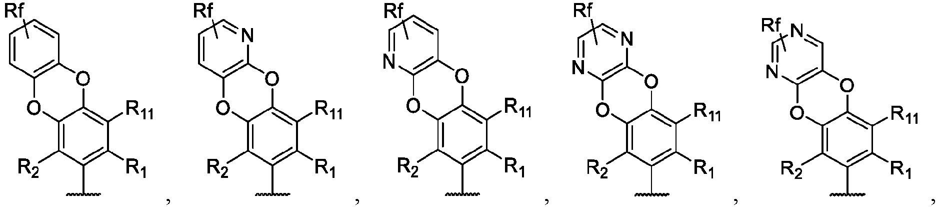 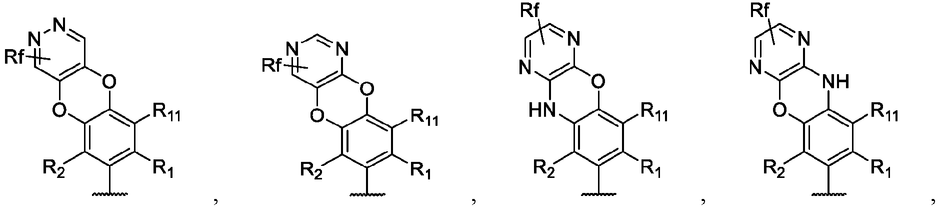 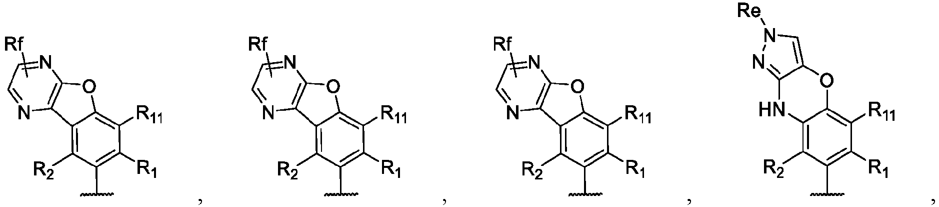 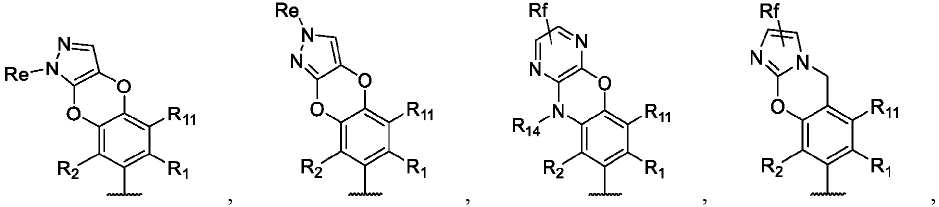 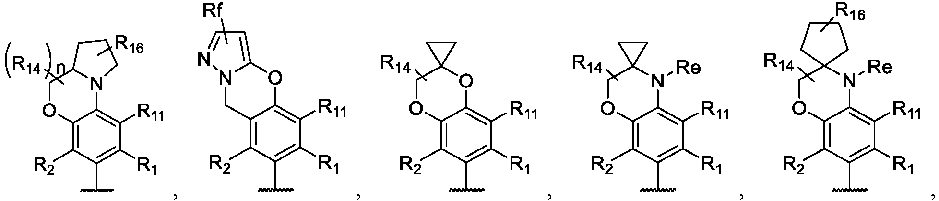 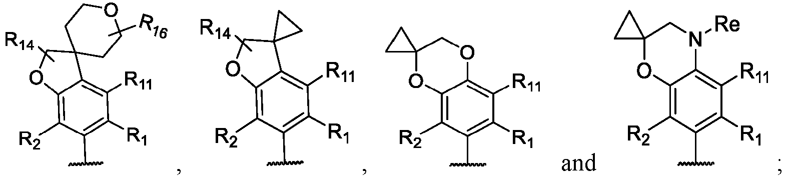 among the above groups, R₁ is cyano or -C(=O)NR'R"; R₂ is H or halogen; R₁₁ is H, OH, - NR'R", halogen or C1-C4 alkyl; R₁₄ is H, halogen or C1-C4 alkyl; n is 0, 1 or 2; R₁₆ is selected from H, hydroxyl, halogen and C1-C4 alkyl; Rₑ is selected from H, substituted or unsubstituted C1-C4 alkyl, substituted or unsubstituted C1-C4 alkoxy, C3-C6 cyclohydrocarbyl, substituted unsubstituted C1-C4 acyl; R_{f} is selected from H, halogen, -OH, cyano, -NR'R", substituted or Unsubstituted C1-C4 alkoxy, substituted or unsubstituted C1-C4 alkyl, C3-C6 cyclohydrocarbyl; preferably, the C1-C4 acyl, C1-C4 alkoxy and C1-C4 alkyl are optionally substituted by 1 to 3 substituents selected from F, hydroxyl and cyano; wherein R' and R" are each independently selected from H and C1-C4 alkyl; the wavy line indicates the bond connection position.
74. The compound or the pharmaceutically acceptable salt, enantiomer, diastereomer, tautomer, solvate, isotopic substitute, polymorph, prodrug or metabolite thereof according to embodiment 73, characterized in that R₁ is cyano, -C(=O)NH₂ or -C(=O)NHCH₃; R₂ is H or F; R₁₁ is H; R₁₄ is H or methyl; n is 0, 1 or 2; R₁₆ is H, hydroxyl or methyl; Rₑ is selected from H, methyl, ethyl, cyclopropyl, cyclobutyl, ethylhydroxyl, acetyl, propionyl and cyclopropionyl; R_{f} is selected from H, F, Cl, -OH, cyano, -NH₂, methoxy, methyl, ethyl, cyclopropyl and cyclobutyl.
75. The compound or the pharmaceutically acceptable salt, enantiomer, diastereomer, tautomer, solvate, isotopic substitute, polymorph, prodrug or metabolite thereof according to embodiment 74, characterized in that R₁ is -C(=O)NH₂ or -C(=O)NHCH₃; R₂ is F; R₁₁ is H; R₁₄ is H; R₁₆ is H; Rₑ is selected from H, methyl, cyclopropyl and acetyl; R_{f} is selected from H, F, Cl, - OH, cyano, -NH₂, methoxy and methyl.
76. The compound or the pharmaceutically acceptable salt, enantiomer, diastereomer, tautomer, solvate, isotopic substitute, polymorph, prodrug or metabolite thereof according to any one of embodiments 1 to 3, characterized in that
   X₂ is selected from: C or N;
   X₃ is selected from: C or N;
   Y₅ is selected from: O, S, N, CHR₁₃, CR₁₃, -OCH₂-, -NCH₂-, -CH₂CH₂- or NR₁₂;
   Y₆ is selected from: O, N, -S(O)-, -SO₂-, S, CHR₁₃, CR₁₃, -OCH₂-, -NCH₂-, -CH₂CH₂- or NR₁₂;
   Y₁ is selected from: a bond, O, N, CH, CH₂, NH, CHR₁₃, C(R₁₃)₂ or NR₁₂;
   Y₄ is selected from: a bond, O, N, CH, CH₂, NH, CHR₁₃, C(R₁₃)₂ or NR₁₂;
   and Y₁ and Y₄ are not simultaneously a bond or O;
   ring B can be optionally substituted by R₁₃ and/or R₁₂ that do not participate in ring formation, and can be further optionally substituted by 1 to 3 substituents selected from hydroxyl, amino, halogen, substituted or unsubstituted C1-C4 alkyl, -NR'R" and substituted or unsubstituted C1-C4 alkoxy;
   R₁ is selected from: cyano and -C(=O)NR'R";
   R₂ is selected from: H, halogen, substituted or unsubstituted C1-C4 alkyl and substituted or unsubstituted C1-C4 alkoxy;
   R₃ is selected from: substituted or unsubstituted aryl;
   R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1 to 3 heteroatoms selected from O, N and S;
   R₆ is selected from H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted C3-C8 cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6-to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
   alternatively, R₄ or R₅ and R₆, together with the C and N to which they are attached, form a 4- to 9-membered heterocyclyl ring, and the 4- to 9-membered heterocyclyl can be substituted by 1 to 3 substituents selected from H, deuterium, hydroxyl, cyano, C1-C4 alkyl, halogen and C1-C4 alkoxy;
   R₇ and R₈ are each independently selected from: H, halogen, cyano, substituted or unsubstituted C1-C4 alkyl, substituted or unsubstituted C1-C4 haloalkyl or substituted or unsubstituted C1-C4 alkoxy;
   R₉ is selected from: H, C1-C4 alkyl, C1-C4 alkoxy, hydroxyl and halogen;
   R₁₀ is selected from: H, halogen or C1-C4 alkyl;
   R₁₁ is selected from: H, hydroxyl, amino, halogen, C1-C4 alkyl, -NR'R" and C1-C4 alkoxy;
   each R₁₂ is independently selected from H, substituted or unsubstituted C1-C4 acyl, substituted or unsubstituted C1-C4 alkoxy, substituted or unsubstituted C1-C4 alkyl-S(O)₂-, 4- to 9-membered heterocyclyl, NR'R"-C(O)-, C3-C8 cyclohydrocarbyl-C(O)- and substituted or unsubstituted C1-C4 alkyl; the substituted or unsubstituted C1-C4 acyl, substituted or unsubstituted C1-C4 alkoxy and substituted or unsubstituted C1-C4 alkyl can be optionally substituted by 1 to 3 substituents selected from F, hydroxy, and cyano;
   each R₁₃ is independently selected from: H, halogen, oxo (=O), substituted or unsubstituted C1-C4 acyl, -OH, cyano, -NR'R", substituted or unsubstituted C1-C4 alkoxy, substituted or unsubstituted C1-C4 alkyl, substituted or unsubstituted C1-C4 alkyl-S(O)₂-, C3-C6 cyclohydrocarbyl-C(O)- and -C(O)-NR'R"; the substituted or unsubstituted C1-C4 acyl, substituted or unsubstituted C1-C4 alkoxy and substituted or unsubstituted C1-C4 alkyl can be optionally substituted by 1 to 3 substituents selected from F, hydroxyl and cyano;
   R' and R" are each independently selected from H and C1-C4 alkyl;
   wherein the dashed line indicates the presence or absence of a double bond.
77. The compound or the pharmaceutically acceptable salt, enantiomer, diastereomer, tautomer, solvate, isotopic substitute, polymorph, prodrug or metabolite thereof according to any one of embodiments 1 to 3 and 76, characterized in that
   X₂ is selected from: C or N;
   X₃ is selected from: C or N;
   Y₅ is selected from: O, S, N, CH₂, CH, NH and NCH₃;
   Y₆ is selected from: O, S, N, CH₂, CH, NH and NCH₃;
   Y₁ is selected from: a bond, O, NH, -NCH₃, CH₂ and -CH(CH₃)-;
   Y₄ is selected from: a bond, O, NH, -NCH₃, CH₂ and -CH(CH₃)-;
   and Y₁ and Y₄ are not simultaneously a bond or O;
   ring B can be further optionally substituted by 1 to 3 substituents selected from hydroxyl, amino, halogen, substituted or unsubstituted C1-C4 alkyl, -NR'R" and substituted or unsubstituted C1-C4 alkoxy;
   R₁ is selected from: cyano, -C(=O)NH₂ and -C(=O)NH₂CH₃;
   R₂ is selected from: H, fluorine and methoxy;
   R₃ is benzene ring;
   R₄ and R₅ are each independently selected from: H and C1-C4 alkyl;
   R₆ is H, substituted C1-C6 alkyl, substituted C1-C6 alkoxy, substituted C2-C6 alkenyl or substituted C2-C6 alkynyl, and their substituents are 1 to 3 substituents selected from: hydroxyl, halogen, C1-C4 alkoxy optionally substituted by halogen, cyano, C1-C4 alkyl-S(O)₂- optionally substituted by halogen, sulfonyl, C1-C4 alkyl optionally substituted by halogen, -S(O)₂-NR'R", sulfanilamido, -C(O)-C1-C4 alkyl, -NR'-C(O)-C1-C4 alkyl and 6- to 14-membered aryl, 5- to 10-membered heteroaryl, C3-C8 cyclohydrocarbyl and 4- to 9-membered heterocyclyl, wherein the 6- to 14-membered aryl, 5- to 10-membered heteroaryl, C3-C8 cyclohydrocarbyl and 4- to 9-membered heterocyclyl are optionally substituted by substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, NR'R", 6- to 14-membered aryl, 5- to 10-membered heteroaryl, C3-C8 cyclohydrocarbyl and 4- to 9-membered heterocyclyl; wherein R' and R" are each independently selected from H and C1-C4 alkyl; or
   R₆ is substituted C3-C8 cyclohydrocarbyl or substituted 4- to 9-membered monocyclic or fused heterocyclyl, and their substituents are 1 to 3 substituents selected from: halogen, hydroxyl, cyano, -NR'R", C1-C4 alkoxy, C1-C4 haloalkoxy, -C(O)-C1-C4 alkyl and C1-C4 alkoxycarbonyl, and the C1-C4 alkyl and C1-C4 alkoxy are optionally substituted by 1 to 3 groups selected from halogen, hydroxyl and -NR'R", wherein the R' and R" are each independently selected from H and C1-C4 alkyl; or
   R₆ is substituted 6- to 12-membered bridged cyclyl, substituted 6- to 12-membered bridged heterocyclyl, substituted 6- to 12-membered spiro cyclyl or substituted 6- to 12-membered spiro heterocyclyl, and their substituents are 1 to 3 substituents selected from: C1-C4 alkyl, C1-C4 haloalkyl, C1-C4 alkoxy, C1-C4 haloalkoxy, cyano, C1-C4 alkyl-S(O)₂-, hydroxyl, halogen and NR'R", wherein the R' and R" are each independently selected from H and C1-C4 alkyl;
   alternatively, R₄ or R₅ and R₆, together with the C and N to which they are attached, form the following groups substituted by 1 to 3 substituents selected from H, deuterium, hydroxyl, methyl, F and methoxy: 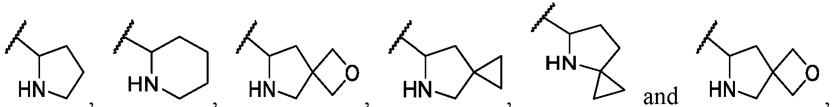
   R₇ and R₈ are each independently selected from: H, halogen, cyano, methyl and methoxy;
   R₉ is selected from: H, methyl, methoxy, hydroxyl and F;
   R₁₀ is selected from: H, methyl and F;
   R₁₁ is selected from: H, hydroxyl, amino, fluorine, chlorine, methyl, methoxy and -NCH₃;
   each R₁₂ is independently selected from H, acetyl, propionyl, C1-C4 alkoxy, C1-C4 alkylS(O)₂-, 4- to 6-membered heterocyclyl, C3-C5 cyclohydrocarbyl-C(O)- and substituted or unsubstituted C1-C4 alkyl;
   each R₁₃ is independently selected from H, fluorine, chlorine, oxo (=O), C1-C4 acyl, -OH, cyano, C1-C4 alkoxy, C1-6 alkyl, C1-C4 alkyl-S(O)₂- and C3-C4 cyclohydrocarbyl-C(O)-,
   the wavy line indicates the bond connection position.
78. The compound or the pharmaceutically acceptable salt, enantiomer, diastereomer, tautomer, solvate, isotopic substitute, polymorph, prodrug or metabolite thereof according to embodiment 8, characterized in that
   X₂ is selected from: C or N;
   X₃ is selected from: C or N;
   Y₁ is selected from: a bond, O, NH, -NCH₃, CH₂ and -CH(CH₃)-;
   Y₅ is selected from: O, S, N, CH₂, CH, NH and NCH₃; and Y₁ and Y₅ are not simultaneously O;
   Y₄ is selected from: C, CH or N;
   Y₆ is selected from: C, CH or N;
   ring D is a C3-C8 cyclohydrocarbyl, C4-C9 heterocyclyl, C5-C10 heteroaryl or C6-C10 aryl ring; the C4-C9 heterocyclyl and C5-C10 heteroaryl can optionally contain 1 to 3 heteroatoms selected from O, N and S;
   R₁₅ is selected from: H, halogen, oxo (=O), substituted or unsubstituted C1-C4 acyl, hydroxyl, substituted or unsubstituted C1-C4 alkoxy, cyano, -NR'R", -C(O)-NR'R", substituted or unsubstituted C1-C4 alkyl-S(O)₂-, substituted or unsubstituted C6-C14 aryl-S(O)₂-, 4- to 9-membered heterocyclyl, NR'R"-C(O)-, C3-C8 cyclohydrocarbyl-C(O)-, substituted or unsubstituted C1-C4 haloalkyl and substituted or unsubstituted C1-C4 alkyl;
   each n is independently 0, 1, 2, 3 or 4;
   R₁ to R₁₁ are as defined in embodiment 76.
79. The compound or the pharmaceutically acceptable salt, enantiomer, diastereomer, tautomer, solvate, isotopic substitute, polymorph, prodrug or metabolite thereof according to embodiment 78, characterized in that R₁ to R₁₁ are as defined in embodiment 77.
80. The compound or the pharmaceutically acceptable salt, enantiomer, diastereomer, tautomer, solvate, isotopic substitute, polymorph, prodrug or metabolite thereof according to embodiment 9, characterized in that
   X₂ and X₃ are C;
   Y₁ is selected from: C, CH or N;
   Y₄ is selected from: C, CH or N;
   Y₅ is selected from: a bond, O, NH, -NCH₃, CH₂ and -CH(CH₃)-;
   Y₆ is selected from: a bond, O, S, N, CH₂, CH, NH and NCH₃; and Y₅ and Y₆ are not simultaneously a bond;
   ring D is a C3-C8 cyclohydrocarbyl, C4-C9 heterocyclyl, C5-C10 heteroaryl or C6-C10 aryl ring; the C4-C9 heterocyclyl and C5-C10 heteroaryl can optionally contain 1 to 3 heteroatoms selected from O, N and S;
   R₁₅ is selected from: H, halogen, oxo (=O), substituted or unsubstituted C1-C4 acyl, hydroxyl, substituted or unsubstituted C1-C4 alkoxy, cyano, -NR'R", -C(O)-NR'R", substituted or unsubstituted C1-C4 alkyl-S(O)₂-, substituted or unsubstituted C6-C14 aryl-S(O)₂-, 4- to 9-membered heterocyclyl, NR'R"-C(O)-, C3-C8 cyclohydrocarbyl-C(O)-, substituted or unsubstituted C1-C4 haloalkyl and substituted or unsubstituted C1-C4 alkyl; preferably, the C1-C4 acyl, C1-C4 alkoxy and C1-C4 alkyl are optionally substituted by 1 to 3 substituents selected from F, hydroxyl, and cyano;
   each n is independently 0, 1, 2, 3 or 4;
   R₁ to R₁₁ are as defined in embodiment 76.
81. The compound or the pharmaceutically acceptable salt, enantiomer, diastereomer, tautomer, solvate, isotopic substitute, polymorph, prodrug or metabolite thereof according to embodiment 80, characterized in that R₁ to R₁₁ are as defined in embodiment 77.
82. The compound or the pharmaceutically acceptable salt, enantiomer, diastereomer, tautomer, solvate, isotopic substitute, polymorph, prodrug or metabolite thereof according to embodiment 12, characterized in that
   X₂ and X₃ are C;
   Y₁ is selected from: O or CH₂;
   Y₅ is selected from: a bond, O, NH, -NCH₃, CH₂ and -CH(CH₃)-;
   Y₆ is selected from: a bond, O, S, N, CH₂, CH, NH and NCH₃; and Y₅ and Y₆ are not simultaneously a bond;
   ring E is a C3-C8 cyclohydrocarbyl or C4-C9 heterocyclyl ring; the C4-C9 heterocyclyl can optionally contain 1 to 3 heteroatoms selected from O, N and S;
   R₁₆ is selected from halogen, C1-C4 alkyl, hydroxyl, C1-C4 acyl and C1-C4 alkoxy;
   n is 0, 1, 2, 3 or 4;
   R₁ to R₁₁ are as defined in embodiment 76.
83. The compound or the pharmaceutically acceptable salt, enantiomer, diastereomer, tautomer, solvate, isotopic substitute, polymorph, prodrug or metabolite thereof according to embodiment 82, characterized in that R₁ to R₁₁ are as defined in embodiment 77.
84. The compound or the pharmaceutically acceptable salt, enantiomer, diastereomer, tautomer, solvate, isotopic substitute, polymorph, prodrug or metabolite thereof according to embodiment 16, characterized in that
   X₂, X₃, Y₁, Y₅, Y₄ and Y₆ are as defined in embodiment 78;
   D₁ is selected from: O, S, N, NRₑ, CR_{f} and CR_{f}R_{g};
   D₂ is selected from: O, S, N, NRₑ, (CR_{f})ₚ and (CR_{f}R_{g})ₚ;
   D₃ is selected from: O, S, N, NRₑ, (CR_{f})ₚ and (CR_{f}R_{g})ₚ;
   each Rₑ is independently selected from: H, substituted or unsubstituted C1-C4 acyl, substituted or unsubstituted C1-C4 alkoxy, cyano, substituted or unsubstituted C1-C4 alkyl-S(O)₂-, 4- to 9-membered heterocyclyl, NR'R"-C(O)-, C3-C6 cyclohydrocarbyl, C3-C6 cyclohydrocarbyl-C(O)-, substituted or unsubstituted C1-C4 haloalkyl and substituted or unsubstituted C1-C4 alkyl; preferably, the C1-C4 acyl, C1-C4 alkoxy and C1-C4 alkyl are optionally substituted by 1 to 3 substituents selected from F, hydroxyl and cyano;
   wherein R' and R" are each independently selected from H and C1-C4 alkyl;
   each R_{f} is independently selected from: H, halogen, oxo (=O), substituted or unsubstituted C1-C4 acyl, -OH, cyano, -NR'R", substituted or unsubstituted C1-C4 alkoxy, substituted or unsubstituted C1-C4 alkyl, C3-C6 cyclohydrocarbyl, substituted or unsubstituted C1-C4 alkylS(O)₂-, C3-C6 cyclohydrocarbyl-C(O)- and -C(O)-NR'R"; preferably, the C1-C4 acyl, C1-C4 alkoxy and C1-C4 alkyl are optionally substituted by 1 to 3 substituents selected from F, hydroxyl and cyano; wherein R' and R" are each independently selected from H and C1-C4 alkyl;
   each R_{g} is independently selected from: H, halogen, substituted or unsubstituted C1-C4 acyl, -OH, cyano, -NR'R", substituted or unsubstituted C1-C4 alkoxy, substituted or unsubstituted C1-C4 alkyl, C3-C6 cyclohydrocarbyl, substituted or unsubstituted C1-C4 alkyl-S(O)₂-, C3-C6 cyclohydrocarbyl-C(O)- and -C(O)-NR'R"; preferably, the C1-C4 acyl, C1-C4 alkoxy and C1-C4 alkyl are optionally substituted by 1 to 3 substituents selected from F, hydroxyl and cyano;
   wherein R' and R" are each independently selected from H and C1-C4 alkyl;
   each p is independently an integer from 1 to 4, preferably 1 or 2;
   R₁ to R₁₁ are as defined in embodiment 76.
85. The compound or the pharmaceutically acceptable salt, enantiomer, diastereomer, tautomer, solvate, isotopic substitute, polymorph, prodrug or metabolite thereof according to embodiment 84, characterized in that R₁ to R₁₁ are as defined in embodiment 77.
86. The compound or the pharmaceutically acceptable salt, enantiomer, diastereomer, tautomer, solvate, isotopic substitute, polymorph, prodrug or metabolite thereof according to embodiment 17, characterized in that
   X₂, X₃, Y₁, Y₅, Y₄, Y₆ and n are as defined in embodiment 80;
   D₁, D₂ and D₃ are as defined in embodiment 84;
   R₁ to R₁₁ are as defined in embodiment 76.
87. The compound or the pharmaceutically acceptable salt, enantiomer, diastereomer, tautomer, solvate, isotopic substitute, polymorph, prodrug or metabolite thereof according to embodiment 86, characterized in that R₁ to R₁₁ are as defined in embodiment 77.
88. The compound or the pharmaceutically acceptable salt, enantiomer, diastereomer, tautomer, solvate, isotopic substitute, polymorph, prodrug or metabolite thereof according to embodiment 1, characterized in that
   ring A and ring B, together with their ring substituents, form: 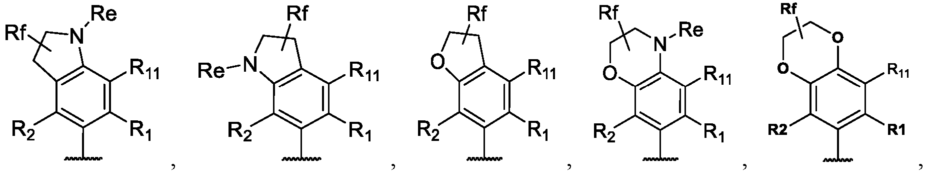 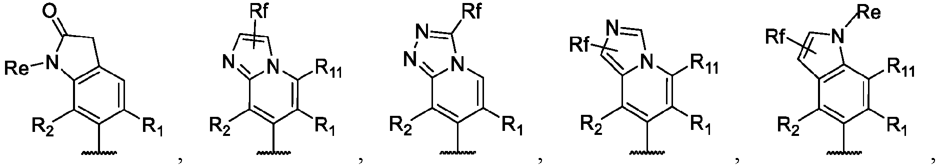 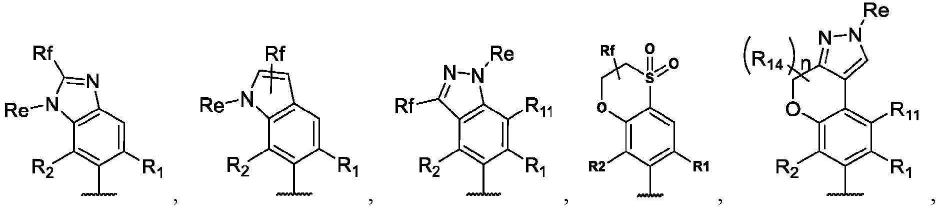 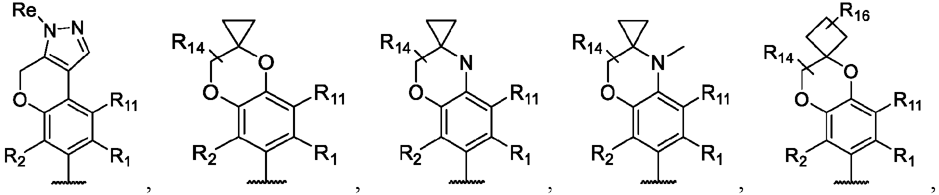 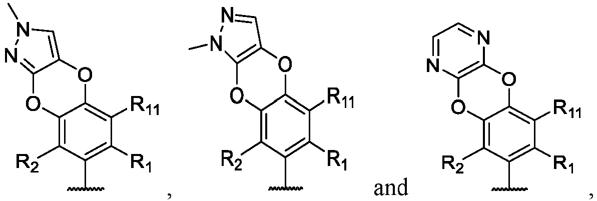
   wherein R₁ is cyano or -C(=O)NR'R"; R₂ is H or halogen; R₁₁ is H, OH, -NR'R", halogen or C1-C4 alkyl; R₁₄ is H, halogen or C1-C4 alkyl; n is 0, 1 or 2; R₁₆ is selected from H, hydroxyl, halogen and C1-C4 alkyl; Rₑ is selected from H, substituted or unsubstituted C1-C4 alkyl, substituted or unsubstituted C1-C4 alkoxy, C3-C6 cyclohydrocarbyl, substituted unsubstituted C1-C4 acyl; R_{f} is selected from H, halogen, -OH, cyano, -NR'R", substituted or Unsubstituted C1-C4 alkoxy, substituted or unsubstituted C1-C4 alkyl, C3-C6 cyclohydrocarbyl; preferably, the C1-C4 acyl, C1-C4 alkoxy and C1-C4 alkyl are optionally substituted by 1 to 3 substituents selected from F, hydroxyl and cyano; wherein R' and R" are each independently selected from H and C1-C4 alkyl; the wavy line indicates the bond connection position;
   X₁ is O;
   R₃ is selected from unsubstituted phenyl or phenyl substituted by 1 to 3 substituents selected from hydroxyl, halogen, C1-C4 alkyl, C1-C4 haloalkyl and C1-C4 alkoxy;
   R₄ is selected from H or C1-C4 alkyl;
   R₅ is selected from H or C1-C4 alkyl;
   R₆ is selected from: H; C1-C4 alkyl, which is optionally substituted by 1 to 3 substituents selected from hydroxyl, halogen, C1-C4 alkoxy, cyano, C1-C4 alkyl-S(O)₂- and sulfonyl; 3- to 8-membered cyclohydrocarbyl, which is optionally substituted by 1 to 3 substituents selected from halogen, hydroxyl, cyano, C1-C4 alkyl-S(O)₂-, C1-C4 alkyl and C1-C4 alkoxy; 4- to 9-membered heterocyclyl, which is optionally substituted by 1 to 3 substituents selected from halogen, hydroxyl, cyano, C1-C4 alkyl-S(O)₂-, C1-C4 alkyl and C1-C4 alkoxy; and 6- to 12-membered bridged cyclyl, 6- to 12-membered spiro cyclyl, 6- to 12-membered bridged heterocyclyl or 6- to 12-membered spiro heterocyclyl, each of which is optionally substituted by 1 to 3 substituents selected from C1-C4 alkyl, C1-C4 alkoxy and cyano;
   alternatively, R₄ or R₅ and R₆, together with the C and N to which they are attached, form the following groups substituted by 1 to 3 substituents selected from H, deuterium, hydroxyl, methyl, F and methoxy: 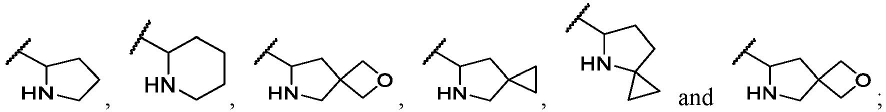
   R₇ and R₈ are each independently selected from: H, halogen, cyano, methyl and methoxy;
   R₉ is selected from: H, methyl, methoxy, hydroxyl and F;
   R₁₀ is selected from: H, methyl and F,
   the wavy line indicates the bond connection position.
89. The compound or the pharmaceutically acceptable salt, enantiomer, diastereomer, tautomer, solvate, isotopic substitute, polymorph, prodrug or metabolite thereof according to embodiment 88, characterized in that
   ring A and ring B, together with their ring substituents, form: 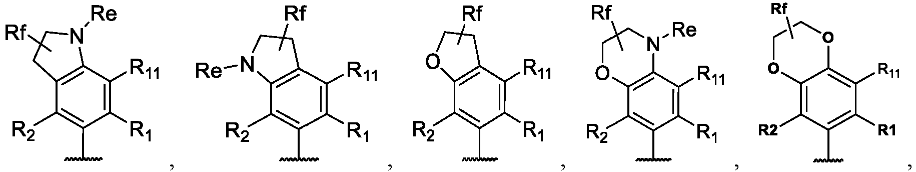 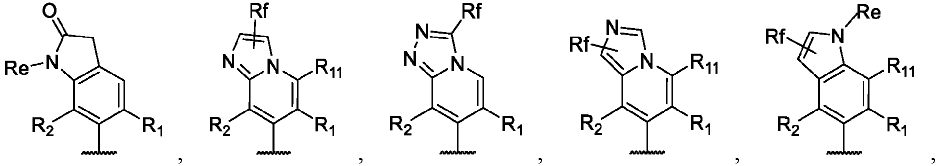 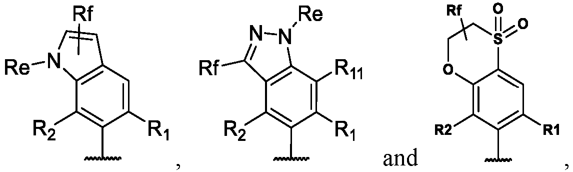 wherein R₁ is -C(O)NR'R"; R₂ is F; R₁₁ is H, F or methyl; Rₑ is selected from H, methyl, ethyl, cyclopropyl, cyclobutyl, -CH₂CH₂OH, -CH₂CH₂OCH₃ and acetyl; R_{f} is selected from H, F, Cl, -OH, cyano, -NR'R", methoxy, ethoxy, C1-C6 alkyl and C3-C6 cyclohydrocarbyl; wherein R' and R" are each independently selected from H and methyl; the wavy line indicates the bond connection position.
90. The compound or the pharmaceutically acceptable salt, enantiomer, diastereomer, tautomer, solvate, isotopic substitute, polymorph, prodrug or metabolite thereof according to embodiment 88, characterized in that
   ring A and ring B, together with their ring substituents, form: 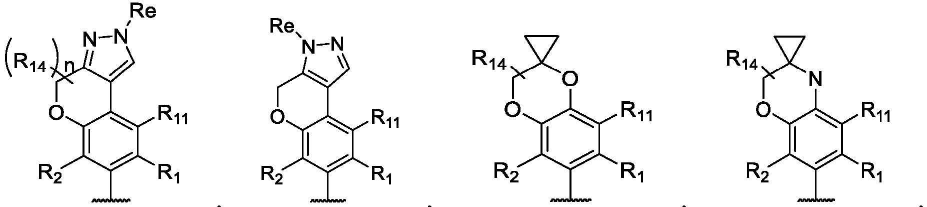 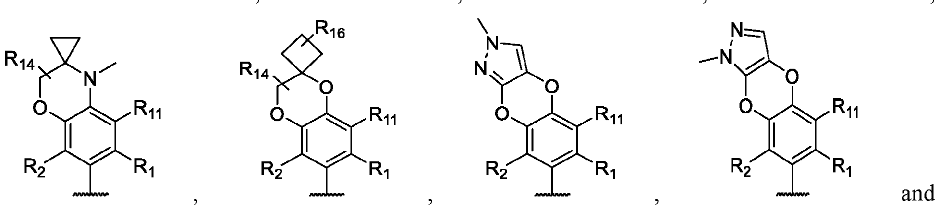 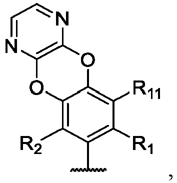 wherein R₁ is -C(=O)NR'R"; R₂ is F; R₁₁ is H, F or methyl; R₁₄ is H, F or methyl; n is 0, 1 or 2; R₁₆ is selected from H, hydroxyl and methyl; Rₑ is selected from H, methyl, ethyl, cyclopropyl, cyclobutyl, -CH₂CH₂OH, -CH₂CH₂OCH₃ and acetyl; R_{f} is selected from H, F, Cl, -OH, cyano, - NR'R", methoxy, ethoxy, C1-C6 alkyl and C3-C6 cyclohydrocarbyl; wherein R' and R" are each independently selected from H and methyl; the wavy line indicates the bond connection position.
91. The compound or the pharmaceutically acceptable salt, enantiomer, diastereomer, tautomer, solvate, isotopic substitute, polymorph, prodrug or metabolite thereof according to embodiment 1, characterized in that the compound is selected from the following compounds: 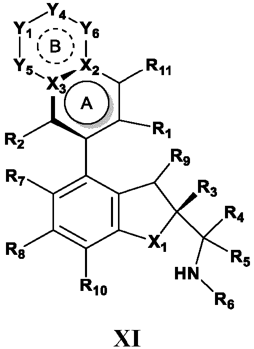 in the formula,
   R₁ to R₁₁, X₁ to X₃, Y₁, Y₄, Y₅, Y₆ and ring B are as defined in embodiment 1.
92. The compound or the pharmaceutically acceptable salt, enantiomer, diastereomer, tautomer, solvate, isotopic substitute, polymorph, prodrug or metabolite thereof according to embodiment 1, characterized in that the compound is selected from the following compounds: 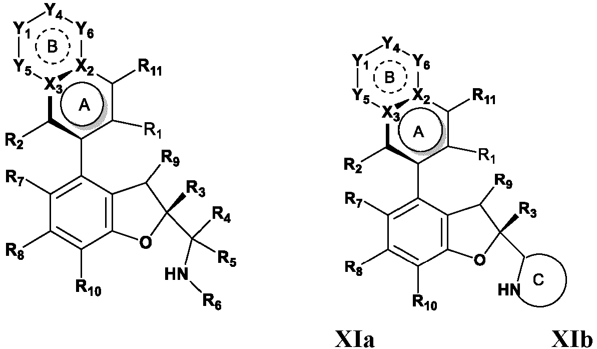
   in formula XIa, R₁ to R₁₁, X₂ to X₃, Y₁, Y₄, Y₅, Y₆ and ring B are as defined in embodiment 2;
   in formula XIb, R₁ to R₃, R₇ to R₁₃, X₂, X₃ and ring B are as defined in embodiment 1; Y₁, Y₄, Y₅, Y₆ and ring C are as defined in embodiment 2.
93. The compound or the pharmaceutically acceptable salt, enantiomer, diastereomer, tautomer, solvate, isotopic substitute, polymorph, prodrug or metabolite thereof according to embodiment 1, characterized in that the compound is selected from the following compounds: 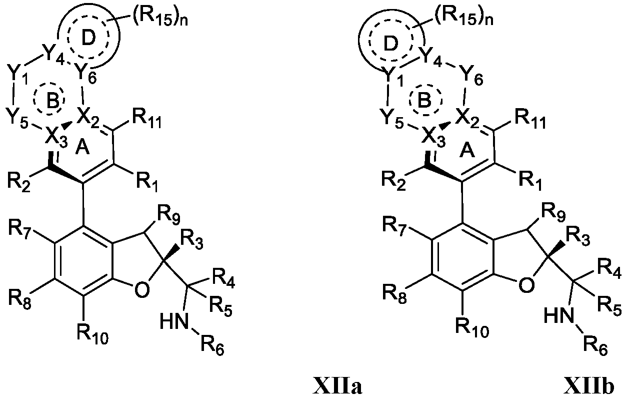 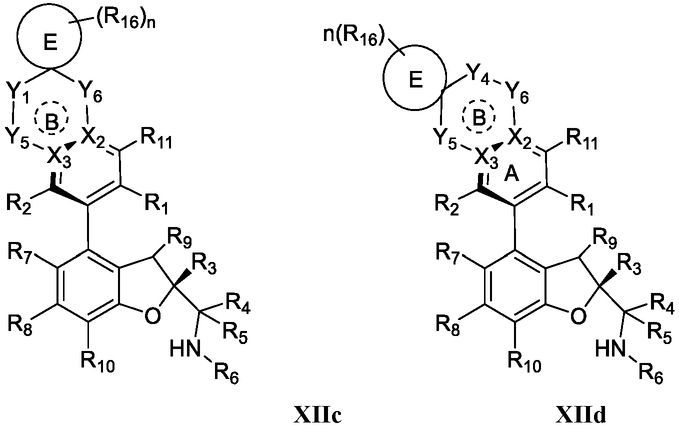
   in formula XIIa, R₁ to R₁₃, X₂ to X₃, Y₁ and Y₅ are as defined in embodiment 1; Y₄, Y₆, R₁₅, ring D and n are as defined in embodiment 8;
   in formula XIIb, R₁ to R₁₃, X₂ to X₃, Y₁ and Y₅ are as defined in embodiment 1; Y₄, Y₆, R₁₅, ring D and n are as defined in embodiment 9;
   in formula XIIc, R₁ to R₁₃, X₂ to X₃, Y₁, Y₅ and Y₆ are as defined in embodiment 1; ring E, R₁₆ and n are as defined in embodiment 12;
   in formula XIId, R₁ to R₁₃, X₂, X₃, Y₄, Y₅ and Y₆ are as defined in embodiment 1; ring E, R₁₆ and n are as defined in embodiment 12;
   in each formula, the dashed line indicates the presence or absence of a double bond.
94. The compound or the pharmaceutically acceptable salt, enantiomer, diastereomer, tautomer, solvate, isotopic substitute, polymorph, prodrug or metabolite thereof according to embodiment 1, characterized in that the compound is selected from the following compounds: 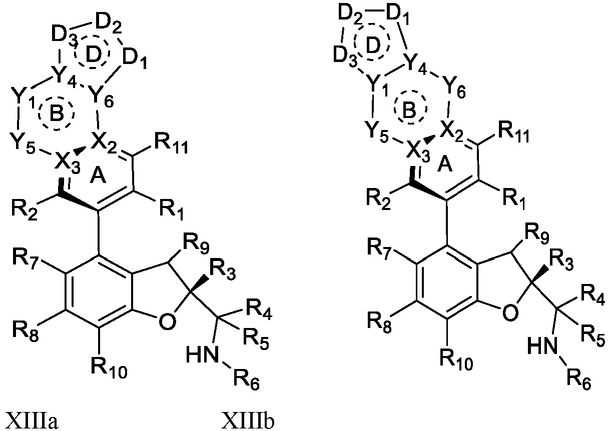 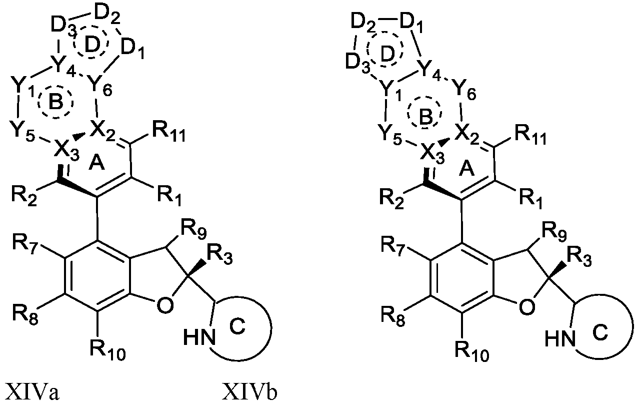
   in formula XIIIa, R₁ to R₁₃, X₂ to X₃, Y₁ and Y₅ are as defined in embodiment 1; Y₄ and Y₆ are as defined in embodiment 8; D₁, D₂ and D₃ are as defined in embodiment 16;
   in formula XIIIb, R₁ to R₁₃, X₂ to X₃, Y₅ and Y₆ are as defined in embodiment 1; Y₁ and Y₄ are as defined in embodiment 9; D₁, D₂ and D₃ are as defined in embodiment 16;
   in formula XIVa, R₁ to R₁₃, X₂ to X₃, Y₁ and Y₅ are as defined in embodiment 1; Y₄ and Y₆ are as defined in embodiment 8; D₁, D₂ and D₃ are as defined in embodiment 16; ring C is as defined in embodiment 3;
   in formula XIVb, R₁ to R₁₃, X₂, X₃, Y₅ and Y₆ are as defined in embodiment 1; Y₁ and Y₄ are as defined in embodiment 9; D₁, D₂ and D₃ are as defined in embodiment 16; ring C is as defined in embodiment 3;
   in each formula, the dashed line indicates the presence or absence of a double bond.
95. The compound or the pharmaceutically acceptable salt, enantiomer, diastereomer, tautomer, solvate, isotopic substitute, polymorph, prodrug or metabolite thereof according to embodiment 1, characterized in that the compound is selected from the following compounds: 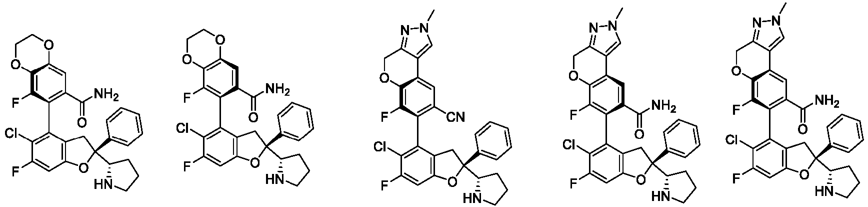 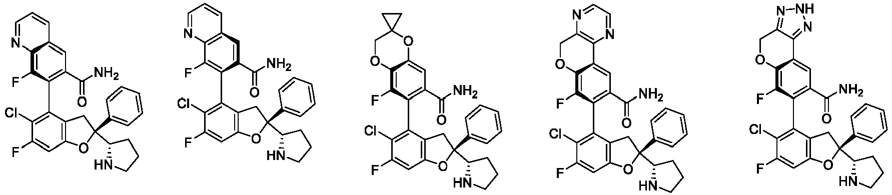 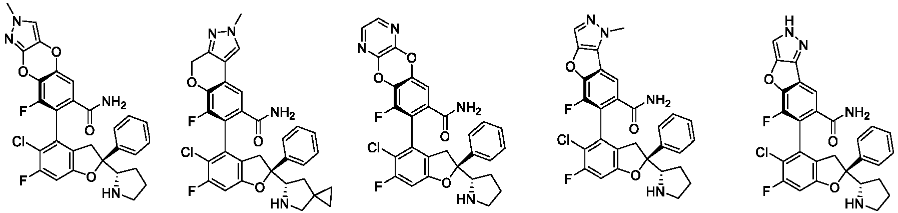 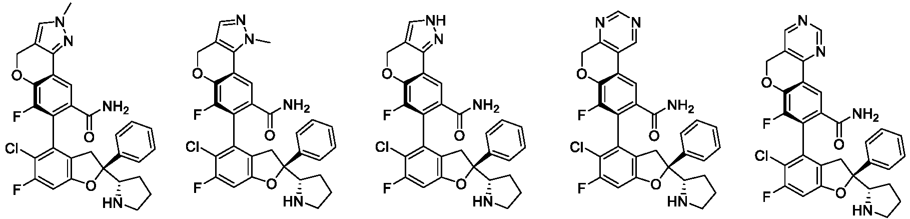 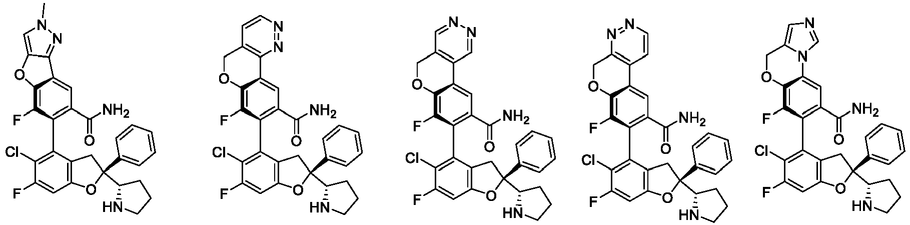 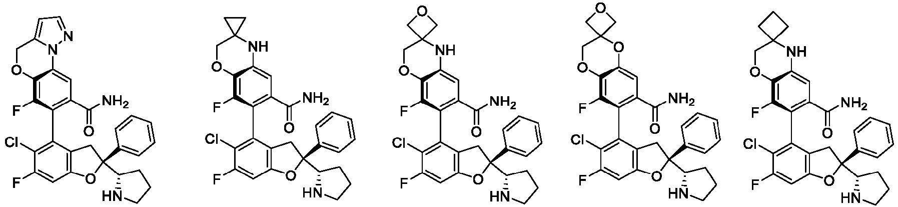 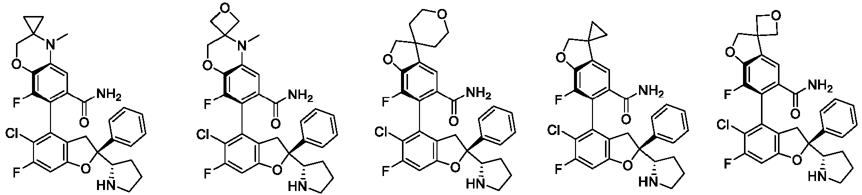 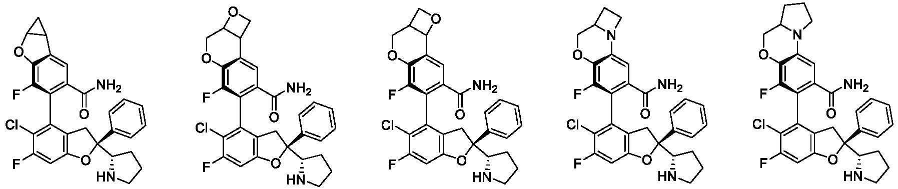 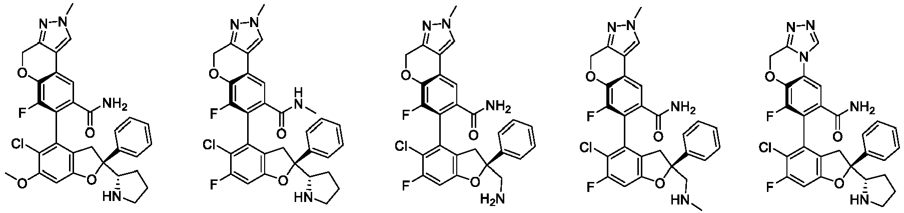 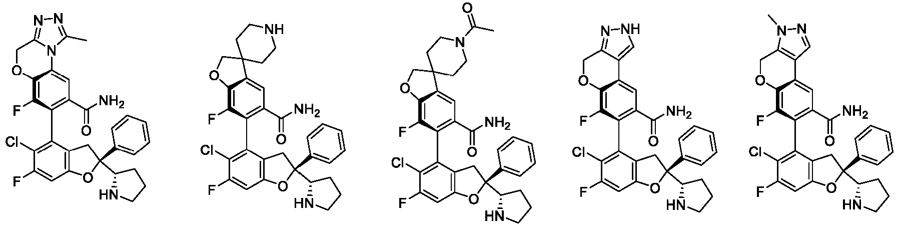 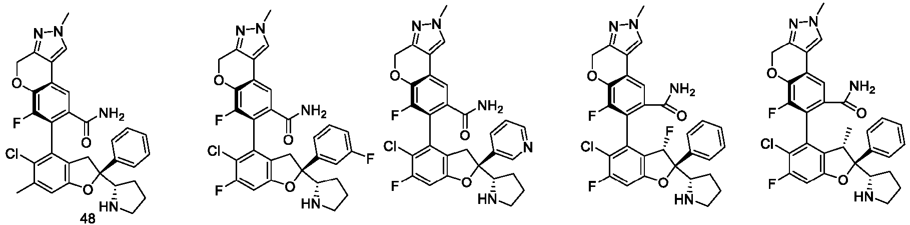 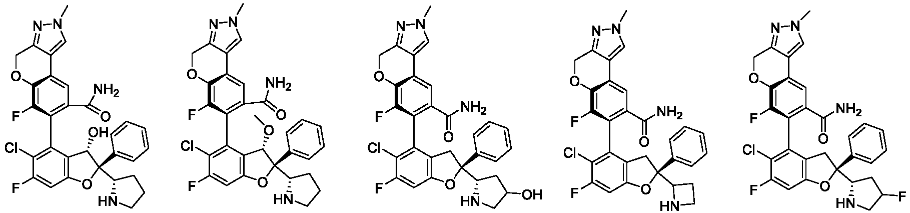 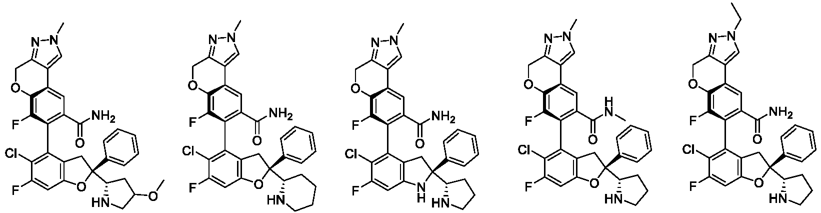 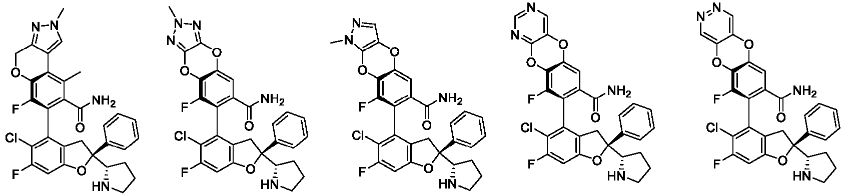 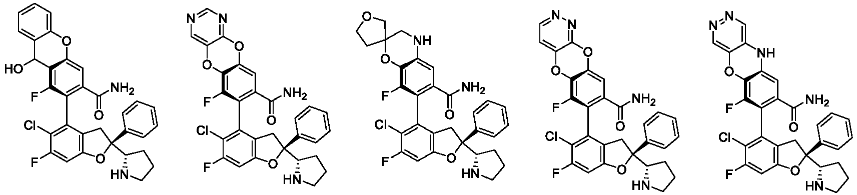 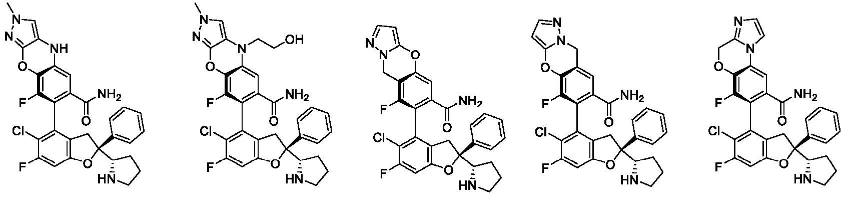 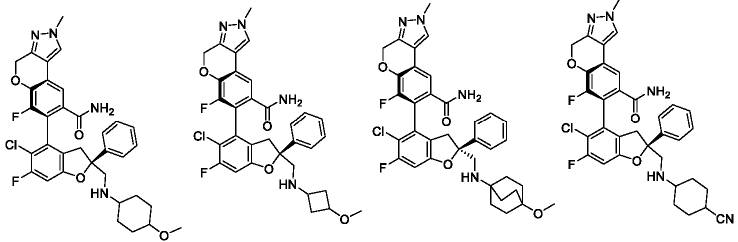 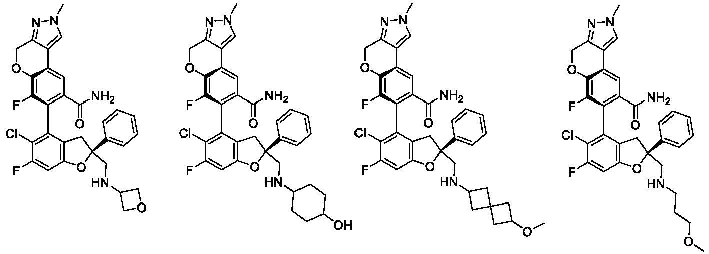 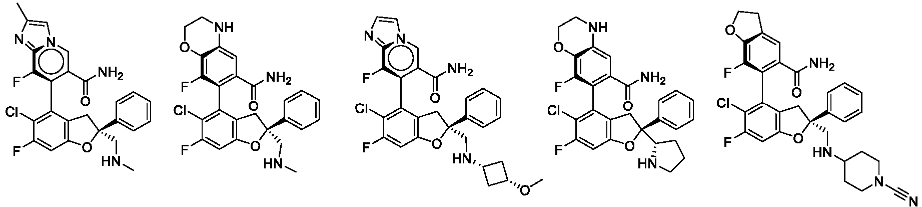 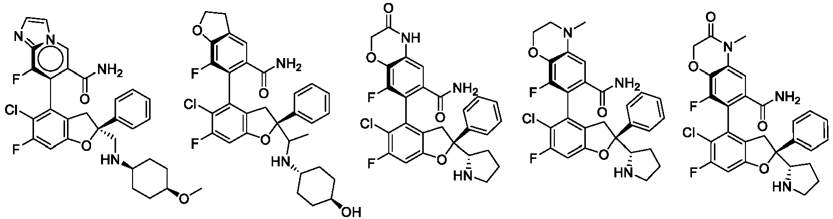 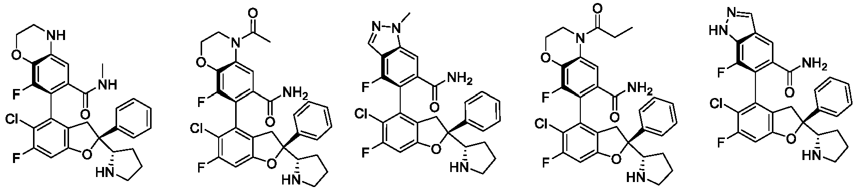 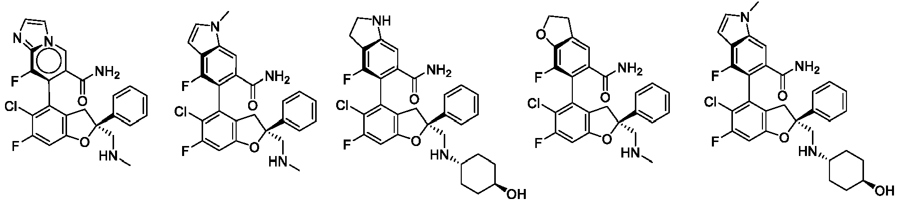 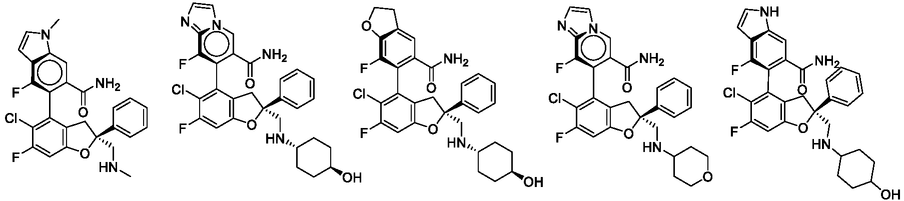 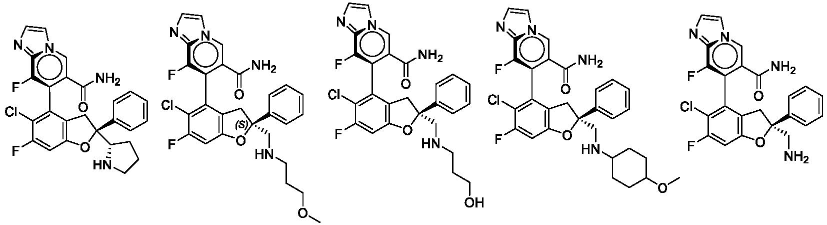 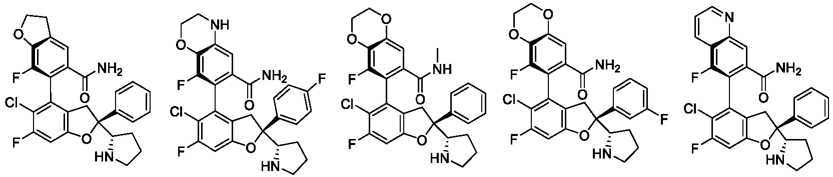 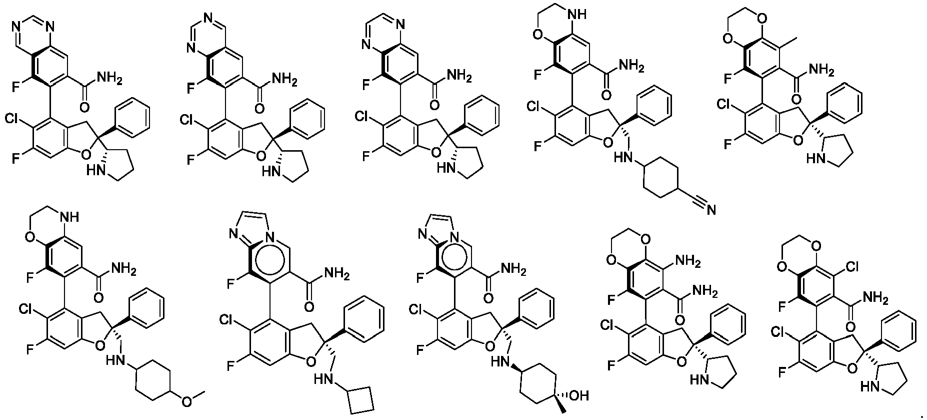
95. A pharmaceutical composition, characterized in that the pharmaceutical composition comprises the compound or the pharmaceutically acceptable salt, enantiomer, diastereomer, tautomer, solvate, isotopic substitute, polymorph, prodrug or metabolite thereof according to any one of embodiments 1 to 94, and a pharmaceutically acceptable carrier or excipient.
96. A use of the compound or the pharmaceutically acceptable salt, enantiomer, diastereomer, tautomer, solvate, isotopic substitute, polymorph, prodrug or metabolite thereof according to any one of embodiments 1 to 95 in the manufacture of a medicament for treating or preventing diseases mediated by interaction between YAP/TAZ and TEAD; preferably, the diseases mediated by the interaction between YAP/TAZ and TEAD are tumors or cancers, including solid tumors or hematological tumors; more preferably, the diseases mediated by the interaction between YAP/TAZ and TEAD are selected from mesothelioma (including pleural mesothelioma, malignant pleural mesothelioma, peritoneal mesothelioma, pericardial mesothelioma and tunica vaginalis mesothelioma), cervical squamous cell carcinoma, endometrial carcinoma, esophageal squamous cell carcinoma, esophageal adenocarcinoma, bladder urothelial carcinoma, skin squamous cell carcinoma, poroma (benign poroma), porocarcinoma (including malignant porocarcinoma), supratentorial ependymoma (including pediatric supratentorial ependymoma), epithelioid hemangioendothelioma, ependymoma, breast cancer (including triple-negative breast cancer), lung cancer (including non-small cell lung cancer), ovarian cancer, colorectal cancer, melanoma, pancreatic cancer, prostate cancer, gastric cancer, esophageal cancer, liver cancer (including hepatocellular carcinoma, cholangiocarcinoma and hepatoblastoma), neuroblastoma, neurilemmoma, renal cancer, sarcoma (including rhabdomyosarcoma, embryonal rhabdomyosarcoma, osteosarcoma, undifferentiated pleomorphic sarcoma, Kaposi's sarcoma, soft tissue sarcoma and rare soft tissue sarcoma), bone cancer, brain cancer, medulloblastoma, glioma, meningioma and head and neck cancer (including head and neck squamous cell carcinoma).

### V. Examples

The present disclosure will be further described below in conjunction with specific examples. It should be understood that these examples are only used to illustrate the present disclosure and are not intended to limit the scope of the present disclosure. Experimental methods for which specific conditions are not indicated in the following examples are usually in accordance with conventional conditions, or in accordance with conditions recommended by the manufacturer. Unless otherwise stated, percentages and parts are calculated by weight.

The starting materials used in the following examples can be purchased from chemical vendors such as Aldrich, TCI, Alfa Aesar, Bide, Energy, etc., or synthesized using known methods.

In the following examples, if not otherwise indicated, the ice bath is carried out at a temperature of -5°C to 0°C, room temperature refers to 10°C to 30°C, the reflux temperature refers to the temperature at which the solvent is refluxed under normal pressure, and reaction overnight refers to a reaction duration of 8 to 15 hours. In the following examples, when the specific operating temperature is not limited, all operations are carried out at room temperature.

In the following examples, if not otherwise indicated, the separation and purification of the intermediates and the final product are carried out by normal or reversed phase chromatographic column separation or other suitable methods. Normal phase flash chromatographic columns use ethyl acetate and n-hexane or methanol and dichloromethane as mobile phases. Reversed phase preparative high-pressure liquid chromatography (HPLC) is carried out by a C18 column and detected at UV 214 nm and 254 nm. The mobile phases are mobile phases A (0.1% formic acid aqueous solution) and B (acetonitrile) or mobile phases A (0.1% ammonium bicarbonate aqueous solution) and B (acetonitrile).

The analytical conditions used in each example are as follows:
LCMS instrument: Pump Agilent 1260;
UV detector: Agilent 1260 DAD Mass Spectrometer API 3000;
chromatographic column: Waters sunfire C18, 4.6 × 50 mm, 5 µm;
mobile phase: A-H₂O (containing 0.1% HCOOH); B-acetonitrile
NMR: Bruker Ascend 400M (¹H NMR: 400 MHz; ¹³C NMR: 100 MHz).

The corresponding meanings of the English abbreviations appearing in the chemical equations and texts of the present disclosure are as shown in the following table:

| | | | |
|---|---|---|---|
| Ti(OEt)₄ | Tetraethyl titanate | DMF | *N,N-*Dimethylformamide |
| LiBH₄ | Lithium borohydride | Na₂CO₃ | Sodium carbonate |
| TFA | Trifluoroacetic acid | EtOH | Ethanol |
| LDA | Lithium diisopropylamide | CBr₄ | Carbon tetrabromide |
| Pd(AmPhos)₂ Cl₂ | Bis(di-*tert*-butyl(4-dimethylaminophenyl)phosphine)dichloropalladium(II) | Ph₃P | Triphenylphosphine |
| Cs₂CO₃ | Cesium carbonate | NBS | *N*-bromosuccinimide |
| DMAc or DMA | *N*,*N*-Dimethylacetatnide | BPO | Benzoyl peroxide |
| THF | Tetrahydrofuran | TMP | 2,2,6,6-Tetramethylpiperidine |
| DCM | Dichloromethane | PBr₃ | Phosphorus tribromide |
| MszO | Methanesulfonic anhydride | CCl₄ | Carbon tetrachloride |
| n-BuLi | *n*-Butyllithium | N₂H₄ | Hydrazine |
| Dibal-H | Diisobutylaluminium hydride | H₂SO₄ | Sulfuric acid |
| Dioxane | 1,4-Dioxane | POCl₃ | Phosphorus oxychloride |
| PPA | Polyphosphoric acid | Pd(OAc)₂ | Palladium acetate |
| NaBH₄ | Sodium borohydride | EtONa | Sodium ethoxide |
| MeOH | Methanol | Pd₂(dba)₃ | Tris(dibenzylideneacetone)dip alladium |
| Et₃N or TEA | Triethylamine | XantPhos | 4,5-Bis(diphenylphosphino)-9,9-dimethylxanthene |
| DIPEA | *N,N*-Diisopropylethylamine | CH₃CN | Acetonitrile |
| HCl | Hydrogen chloride | Boc₂O | Di-tert-butyl dicarbonate |
| PMB-Br | 4-Methoxybenzyl bromide | K₂CO₃ | Potassium carbonate |
| SEMCl | 2-(Trimethylsilyl)ethoxymethyl chloride | DBAD | Diisopropyl azodicarboxylate |
| t-BuXphos-Pd-G3 | Methanesulfonato(2-di-*t-*butylphosphino-2',4',6'-tri-*i*-propyl-1,1'-biphenyl)(2'-amino-1,1'-biphenyl-2-yl)palladium(II) | Ruphos-Pd-G3 | Methanesulfonato(2-dicyclohexylphosphino-2',6'-di-*i*-propoxy-1,1'-biphenyl)(2'-amino-1,1'-biphenyl-2-yl)palladium(II) |
| Cyclohexane | Cyclohexane | Toluene | Toluene |
| i-PrMgBr | Isopropylmagnesium bromide | LiCl | Lithium chloride |
| NaH | Sodium hydride | CH₃ONa | Sodium methoxide |
| NCS | *N-*Chlorosuccinimide | LiOH | Lithium hydroxide |
| NaBH(OAc)₃ | Sodium triacetoxyborohydride | Pddppf(Cl)₂ | [1,1'-Bis(diphenylphosphino)ferroc ene]dichloropalladium(II) |
| CuI | Cuprous iodide | MeI | Iodomethane |
| LiHMDS | Lithium bis(trimethylsilyl)amide | Bromo(phe nyl) magnesium | Phenylmagnesium bromide |
| *n*-Heptane | *n*-Heptane | *t*-BuOK | Potassium *tert*-butoxide |
| B₂(Pin)₂ | Pinacol borate | KOPiv | Potassium pivalate |
| Xylene | Xylene | Ti(iPrO)₄ | Tetraisopropyl titanate |
| HTMP | 2,2,6,6-Tetramethylpiperidine | TBSOTf | *tert*-Butyldimethylsilyl trifluoromethanesulfonate |
| DtBuAD | Di-*tert*-butyl azodicarboxylate | KOAc | Potassium acetate |
| DMSO | Dimethyl sulfoxide | DMAP | 4-Dimethylaminopyridine |
| *tert-BuOH* | *tert*-Butanol | Acetone | Acetone |
| 1-Butanol/*n-*BuOH | *n*-Butanol | NMP | *N-*Methylpyrrolidone |
| Ac₂O | Acetic anhydride | AcOH | Acetic acid |
| Lawesson's reagent | Lawesson's reagent | NIS | *N*-Iodosuccinimide |
| BnBr | Benzyl bromide | *N*-Xantphos | 4,6-Bis(diphenylphosphino)pheno xazine |
| Ruphos | 2-Dicyclohexylphosphino-2',6'-di-*i-*propoxy-1,1'-biphenyl | TsOH | *p*-Toluenesulfonic acid |
| Cu(OAc)₂ | Copper acetate | Microwave | Microwave |
| RT | Room temperature | | |

### Synthesis of intermediate A1: tert-Butyl (S)-2-((S)-5-chloro-6-fluoro-2-phenyl-4-(4,4,5,5-tetramethyl-1,3,2-dioxaborolan-2-yl)-2,3-dihydrobenzofuran-2-yl)pyrrolidine-1-carboxylate

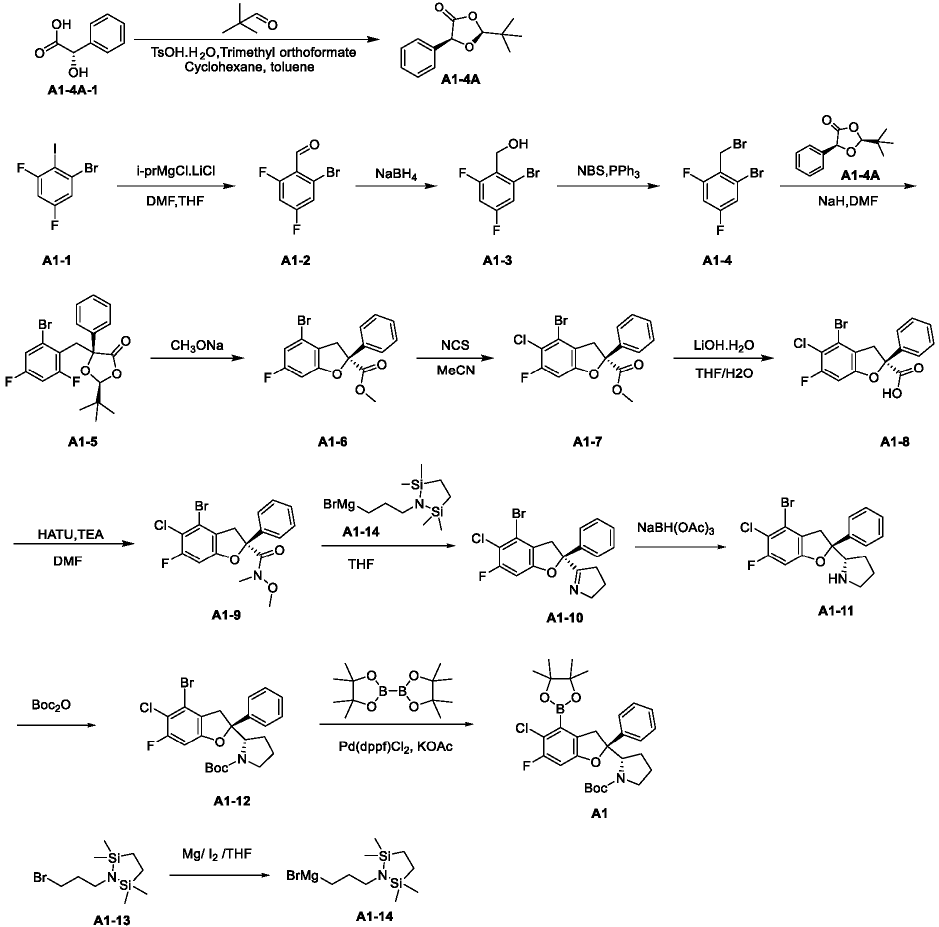

Step 1: Tetrahydrofuran (THF) (150 mL) and **A1-1** (15 g, 47.04 mmol) were sequentially added to a dry three-neck flask under nitrogen atmosphere, and *i*-PrMgCl.LiCl (39.8 mL, 51.74 mmol, a solution dissolved in THF, concentration of 1.3 M) was added dropwise after cooling to -70°C, and stirred at this temperature for 1 hour. Then DMF (5.16 g, 70.56 mmol) was added dropwise, and stirred for another 1 hour. TLC (thin-layer chromatography) showed that the reaction was completed. The reaction mixture was quenched by pouring into saturated NH₄Cl aqueous solution at 0°C and extracted with ethyl acetate, then the combined organic phases were washed with saturated brine, dried over anhydrous sodium sulfate, filtered, and concentrated under reduced pressure to obtain yellow oily **A1-2** (5.8 g, crude), which was used directly in the next step.

Step 2: THF (60 mL), MeOH (10 mL), **A1-2** (5.8 g, crude) and NaBH₄ (596 mg, 15.75 mmol) were sequentially added to a dry 250 mL single-neck flask. The reaction mixture was stirred at 20°C for 0.5 hours. TLC showed that the reaction was completed. The reaction mixture was quenched by pouring into saturated NH₄Cl aqueous solution at 0°C and extracted with ethyl acetate, then the combined organic phases were washed with saturated brine, dried over anhydrous sodium sulfate, filtered, and concentrated under reduced pressure to obtain yellow oily **A1-3** (4.2 g, crude), which was used directly in the next step.

Step 3: DCM (40 mL), **A1-3** (4.2 g, 18.83 mmol) and PPh₃ (5.93 g, 22.6 mmol) were sequentially added to a dry 100 mL single-neck flask, and stirred to dissolve. Then NBS (4.02 g, 22.6 mmol) was slowly added thereto. The resulting yellow reaction mixture was stirred at 20°C for 0.5 hours. TLC showed that the reaction was completed. The reaction mixture was loaded onto the column using the wet method, then subjected to silica gel column chromatography (5 to 10% dichloromethane/petroleum ether) to obtain **A1-4** as a colorless oil (4.1 g, yield: 76%).

¹H NMR (400 MHz, Chloroform-d) δ 7.19 (dt, *J* = 7.8, 2.1 Hz, 1H), 6.85 (ddd, *J* = 9.4, 8.4, 2.5 Hz, 1H), 4.60 (d, *J* = 2.0 Hz, 2H).

Step 4: Cyclohexane (150 mL), (*S*)-(+)-mandelic acid (25 g, 164 mmol, 1.0 eq) and trimethyl orthoformate (27 mL, 246 mmol, 1.5 eq) were sequentially added to a reaction flask and stirred at 100°C for 2 hours, and then concentrated under reduced pressure. The residue was dissolved in toluene (100 mL), then pivalaldehyde (23.1 mL, 181 mmol, 1.1 eq, purity of 85%) and *p*-toluenesulfonic acid (936 mg, 4.92 mmol, 0.03 eq) were added thereto, stirred at 25°C for 12 hours and concentrated under reduced pressure. The residue was slurried with a mixed solvent of cyclohexane/toluene (100 mL, 3/1) and filtered. The filter cake was vacuum dried to obtain **A1-4A** as a white solid (23 g, yield: 63.5%).

¹H NMR (400 MHz, DMSO-*d*₆) δ 7.50 - 7.35 (m, 5H), 5.57 (d, *J=* 1.2 Hz, 1H), 5.48 (d, *J=* 1.2 Hz, 1H), 1.02 (s, 9H).

DMF (65 mL), **A1-4** (6.5 g, 22.73 mmol) and **A1-4A** (5.01 g, 22.73 mmol) were sequentially added to a dry 250 mL three-neck flask. The mixture was stirred to dissolve and slowly added with NaH (1.27 g, 31.83 mmol, dispersed in mineral oil, concentration of 60%). The resulting yellow reaction mixture was stirred at 25°C for 2 hours. TLC showed that the reaction was completed. The reaction mixture was quenched by pouring into saturated NH₄Cl aqueous solution and extracted with ethyl acetate, then the combined organic phases were washed with saturated brine, dried over anhydrous sodium sulfate, filtered, and concentrated under reduced pressure, and the resulting residue was subjected to silica gel column chromatography (0 to 5% ethyl acetate/petroleum ether) to obtain **A1-5** as a white solid (8.3 g, yield: 86%).

¹H NMR (400 MHz, Chloroform-*d*) δ 7.67 - 7.62 (m, 2H), 7.39 - 7.31 (m, 3H), 7.12 (ddd, *J* = 8.0, 2.6, 1.8 Hz, 1H), 6.73 (ddd, *J* = 9.6, 8.4, 2.6 Hz, 1H), 5.12 (s, 1H), 3.62 (dd, *J=* 14.2, 2.0 Hz, 1H), 3.47 (dd, *J* = 14.2, 2.3 Hz, 1H), 0.90 (s, 9H).

Step 5: **A1-5** (8.3 g, 19.52 mmol) and MeONa (8.3 mL, 44.89 mmol, methanol solution, concentration of 5.4 M) were sequentially added to a dry 250 mL single-neck flask, and the resulting yellow suspension was stirred at 50°C for 2 hours. After the reaction was completed, the reaction mixture was quenched by pouring into 1 M hydrochloric acid, diluted with water and extracted with ethyl acetate. The combined organic phases were washed with saturated brine, dried over anhydrous sodium sulfate, filtered, and concentrated under reduced pressure, and the resulting residue was subjected to silica gel column chromatography (0 to 5% ethyl acetate/petroleum ether) to obtain **A1-6** as a yellow oil (6 g, yield: 87%).

¹H NMR (400 MHz, Chloroform-*d*) δ 7.59 - 7.53 (m, 2H), 7.43 - 7.32 (m, 3H), 6.81 (dd, *J* = 8.8, 2.1 Hz, 1H), 6.70 (dd, *J=* 8.9, 2.1 Hz, 1H), 4.13 (dd, *J=* 16.2, 1.4 Hz, 1H), 3.77 (s, 3H), 3.50 (dd, *J* = 16.2, 1.7 Hz, 1H).

Step 6: Acetonitrile (45 mL), **A1-6** (4.4 g, 12.53 mmol) and N-chlorosuccinimide (NCS) (1.84 g, 13.7 mmol) were sequentially added to a dry single-neck flask. The resulting reaction mixture was stirred at 80°C for 2 hours. After TLC showed that the reaction was completed, the reaction mixture was concentrated under reduced pressure, and the resulting residue was purified by silica gel chromatography (0 to 4% ethyl acetate/petroleum ether) to obtain **A1-7** as a colorless oil (4.3 g, yield: 89%).

¹H NMR (400 MHz, Chloroform-*d*) δ 7.58 - 7.52 (m, 2H), 7.44 - 7.34 (m, 3H), 6.83 (d, *J* = 8.8 Hz, 1H), 4.16 - 4.08 (m, 1H), 3.77 (s, 3H), 3.54 (dd, *J* = 16.4, 1.9 Hz, 1H).

Step 7: THF (15 mL), water (7.5 mL), **A1-7** (1.5 g, 3.89 mmol) and lithium hydroxide monohydrate (653 mg, 2.07 mmol) were sequentially added to a clean 100 mL single-neck flask. The resulting yellow reaction mixture was stirred at 20°C for 1.5 hours. TLC showed that the reaction was completed. The pH of the reaction mixture was adjusted to about 5 with 1 M hydrochloric acid, and the mixture was extracted with ethyl acetate. The combined organic phases were washed with saturated brine, dried over anhydrous sodium sulfate, filtered, and concentrated under reduced pressure to obtain **A1-8** as a colorless oil (1.5 g, crude), which was used directly in the next step.

¹H NMR (400 MHz, DMSO-*d*₆) δ 13.72 (s, 1H), 7.55 (d, *J* = 7.5 Hz, 2H), 7.49 - 7.34 (m, 3H), 7.26 (d, *J=* 9.4 Hz, 1H), 4.05 (d, *J* = 15.2 Hz, 1H), 3.56 (d, *J* = 16.4 Hz, 1H).

Step 8: DMF (50 mL), **A1-8** (1.2 g, 3.2 mmol), *N,O*-dimethylhydroxylamine hydrochloride (0.46 g, 1.5 mmol) and HATU (1.85 g, 1.5 mmol) were sequentially added to a dry 100 mL single-neck flask. Then triethylamine (2.5 mL, 16.5 mmol) was slowly added dropwise, and the resulting yellow suspension was stirred at 25°C for 1 hour. TLC showed that the reaction was completed. The reaction mixture was diluted with water and extracted with ethyl acetate, then the combined organic phases were washed with saturated brine, dried over anhydrous sodium sulfate, filtered, and concentrated under reduced pressure, and the resulting residue was purified by silica gel chromatography (0 to 20% ethyl acetate/petroleum ether) to obtain yellow oily **A1-9** (0.91 g, yield: 67%).

Step 9: Metal magnesium chips (150 mg, 6.25 mmol), iodine (50 mg, 0.20 mmol) and anhydrous tetrahydrofuran (10 mL) were added to a 50 mL three-neck flask. The reaction mixture was heated and stirred at 60°C under nitrogen atmosphere until the color disappeared. A tetrahydrofuran solution (9 mL) of **A1-13** (1.0 g, 3.58 mmol) was added dropwise to the reaction mixture. After the addition was completed, the reaction mixture continued to reflux for 2 hours. The reaction mixture was cooled to room temperature to obtain a tetrahydrofuran solution of **A1-14** (0.32 M), which was used directly in the next step.

Step 10: **A1-9** (1.0 g, 2.5 mmol) and anhydrous tetrahydrofuran solution (20 mL) were added to a 100 mL eggplant-shaped flask, and then **A1-14** (0.32 M, 15.6 mL, 4.99 mmol) was added thereto. The reaction mixture was stirred at room temperature overnight, and then quenched by adding saturated ammonium chloride. The mixture was extracted with ethyl acetate, dried, and concentrated to obtain crude **A1-10** (1.1 g).

¹H NMR (400 MHz, Chloroform-*d*) δ ppm 7.49 - 7.19 (m, 5 H), 6.71 (d, 1 H), 4.42 (d, 1 H), 3.90 - 3.70 (m, 2 H), 3.27 (d, 1 H), 2.70 - 2.55 (m, 1 H), 2.33 - 2.20 (m, 1 H), 1.80 - 1.70 (m, 2H).

Step 11: **A1-10** (600 mg, 1.5 mmol), anhydrous DCM (80 mL) and NaBH(OAc)₃ (485 mg, 2.3 mmol) were added to a dry eggplant-shaped flask, and then 0.2 mL of acetic acid was added thereto. The reaction mixture was stirred at room temperature overnight and concentrated. The resulting residue was added with saturated sodium bicarbonate solution and extracted with ethyl acetate. The organic phase was dried and then concentrated under reduced pressure to obtain crude **A1-11** (600 mg).

Step 12: **A1-11** (600 mg, 1.52 mmol), DCM (50 mL), Boc₂O (397mg, 1.82 mmol) and triethylamine (460 mg, 4.55 mmol) were added to an eggplant-shaped flask. The reaction mixture was stirred at room temperature overnight. After the reaction was completed, the reaction mixture was concentrated, and the resulting residue was purified by silica gel chromatography (petroleum ether/ethyl acetate = 10:1) to obtain intermediate **A1-12** (350 mg).

LC-MS: m/z 496.8 [M+H]⁺.

Step 13: Intermediate **A1-12** (89 mg, 0.179 mmol), bis(pinacolato)diboron (63 mg, 0.25 mmol), xylene (2 mL), Pd(dppf)Cl₂·CH₂Cl₂ (15 mg, 0.018 mmol) and potassium acetate (52 mg, 0.537 mmol) were sequentially added to a dry three-neck flask. The reaction mixture was stirred at 100°C for 12 hours under nitrogen atmosphere. After the reaction was completed, the reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 10:1) to obtain crude intermediate **A1.**

### Synthesis of intermediate A2: tert-Butyl (S)-6-((S)-6-fluoro-2-phenyl-4-(4,4,5,5-tetramethyl-1,3,2-dioxaborolan-2-yl)-2,3-dihydrobenzofuran-2-yl)-5-azaspiro[2.4]heptane-5-carboxylate

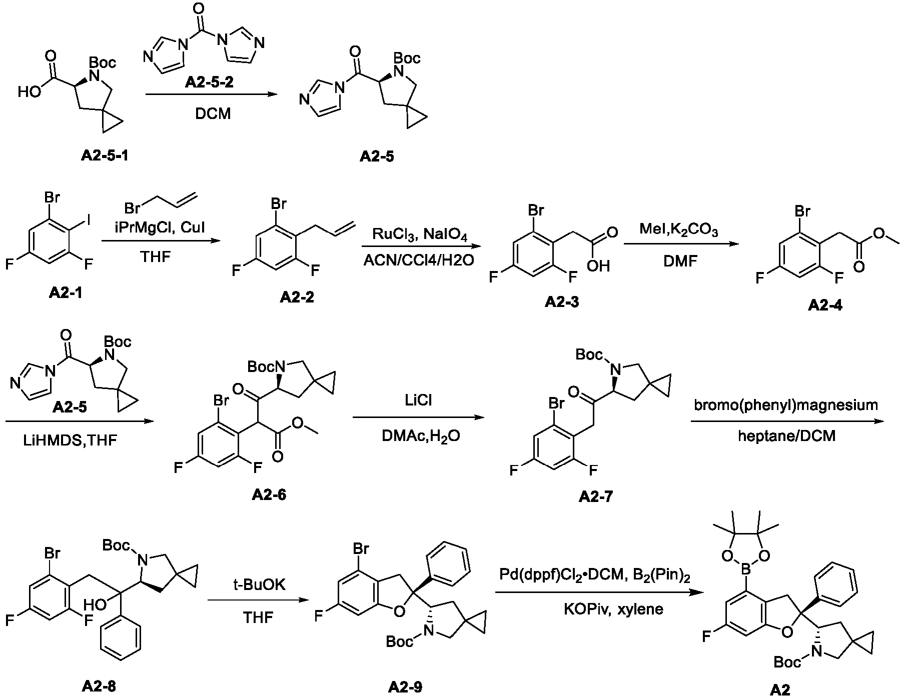

Step 1: DCM (100 mL), **A2-5-1** (9.3 g, 38.543 mmol) and **A2-5-2** (7.5 g, 46.251 mmol) were sequentially added to a dry 250 mL single-neck flask at 0°C, restored to room temperature, and stirred for 12 hours. TLC showed that the reaction was completed. The reaction mixture was washed separately with water, saturated sodium bicarbonate solution and saturated brine, each once. Then the organic phase was dried over anhydrous sodium sulfate, filtered, and concentrated under reduced pressure to obtain **A2-5** as a white solid (11.2 g, crude).

¹H NMR (400 MHz, CDCl₃) δ 8.22 (d, *J* = 5.8 Hz, 1H), 7.51 (d, *J=* 13.5 Hz, 1H), 7.17 - 7.07 (m, 1H), 5.03 (ddd, *J* = 13.9, 8.6, 4.7 Hz, 1H), 3.50 (dt, *J* = 19.4, 10.2 Hz, 2H), 2.40 (ddd, *J* = 44.0, 12.8, 8.6 Hz, 1H), 1.94 (ddd, *J=* 16.7, 12.7, 4.6 Hz, 1H), 1.48 - 1.28 (m, 9H), 0.81 - 0.50 (m, 4H).

Step 2: *i*-PrMgCl.LiCl (20.383 mL, 40.766 mmol, a solution dissolved in THF, concentration of 1.3 M) was added to a THF (100 mL) solution of **A2-1** (10 g, 31.359 mmol) at - 40°C under nitrogen atmosphere. The mixture was then slowly warmed to 0°C and stirred for 1 hour. CuI (1.49 g, 7.840 mmol) was added to the mixture and stirred at 0°C (gray solution) for 10 minutes. A THF (10 mL) solution of 3-bromoprop-1-ene (4.094 mL, 47.038 mmol) was added dropwise to the mixture (temperature < 10°C). The mixture was warmed to 17°C overnight. TLC showed that the reaction was completed. The mixture was quenched with saturated ammonium chloride solution and extracted with DCM. The organic phases were mixed, washed with saturated brine, dried over anhydrous sodium sulfate, filtered, and concentrated under reduced pressure. The resulting residue was subjected to silica gel column chromatography (100% petroleum ether) to obtain **A2-2** as a colorless oil (7 g, yield: 95.76%).

¹HNMR (400MHz, CDCl₃) δ 7.14 (m, 1H), 6.80 (m, 1H), 5.88 (ddt,*J* = 16.4, 10.2, 6.1Hz, 1H), 5.05 (ddd,*J* = 20.4, 10.7, 1. 1Hz, 2H), 3.51 (m, 2H).

Step 3: A solution of sodium periodate (917.75 mg, 4.291 mmol) dissolved in water (8 mL) was added to a solution of **A2-2** (200.00 mg, 0.858 mmol) dissolved in CCl₄ (4 mL). After stirring at room temperature for 5 minutes, a solution of ruthenium chloride trihydrate (22.44 mg, 0.086 mmol) dissolved in ACN (4 mL) was added dropwise to the above two-phase solution. The reaction was stirred at 13°C for 18 hours. TLC showed that the reaction was completed. 10% H₂SO₄ (20 mL) was added to the mixture and extracted with DCM (2 * 15 mL). The resulting organic layers were combined, dried over anhydrous Na₂SO₄, filtered and concentrated under reduced pressure. The resulting residue was subjected to silica gel column chromatography (0 to 30% ethyl acetate/petroleum ether) to obtain **A2-3** as a white solid (140 mg, yield: 63.64%).

¹HNMR (400MHz, DMSO-*d*₆) δ 12.74 (s, 1H), 7.54 (m, 1H), 7.39 (m, 1H), 3.72 (d, *J* = 2.0Hz, 2H).

Step 4: Iodomethane (0.274 mL, 4.402 mmol) and potassium carbonate (935.90 mg, 6.772 mmol) were added to a DMF (10 mL) solution of **A2-3** (850 mg, 3.386 mmol), and the mixture was stirred at 13°C for 18 hours. TLC showed that the reaction was completed. The mixture was diluted with water (200 mL) and extracted with ethyl acetate (EA) (80 mL × 2). The combined organic layers were washed with saturated saline, dried over Na₂SO₄, filtered and concentrated under reduced pressure. The resulting **A2-4** as a colorless oil (890 mg, crude) was used directly for the next step.

¹H NMR (400 MHz, CDCl₃) δ 7.21 - 7.15 (m, 1H), 6.89 - 6.80 (m, 1H), 3.82 (d, *J=* 2.0 Hz, 2H), 3.73 (s, 3H).

Step 5: **A2-4** (890 mg, 3.358 mmol), **A2-5** (978.31 mg, 3.358 mmol) and THF (10 mL) were sequentially added to a dry 50 mL three-neck flask under nitrogen atmosphere at -65°C. LiHMDS (6.7 mL, 6.7 mmol, a solution dissolved in THF, concentration of 1 M) was added dropwise to the mixture, and the resulting yellow suspension was slowly warmed to room temperature and stirred for 3 hours. After the reaction was completed, the reaction mixture was quenched by pouring into ice-cold saturated ammonium chloride solution (20 mL), and then the pH was adjusted to 4 with 3 M hydrochloric acid, and the mixture was diluted with water and then extracted with ethyl acetate. The combined organic phases were washed with saturated brine, dried over anhydrous sodium sulfate, filtered, and concentrated under reduced pressure, and the resulting residue was subjected to silica gel column chromatography (0 to 6% ethyl acetate/petroleum ether) to obtain **A2-6** as a yellow oil (860 mg, yield: 52.44%).

¹H NMR (400 MHz, CDCl₃) δ 7.26 - 7.16 (m, 1H), 6.86 (ddd, *J* = 12.9, 11.1, 5.4 Hz, 1H), 4.30 (dd, *J* = 14.3, 8.2 Hz, 1H), 3.84 - 3.75 (m, 1H), 3.74 - 3.62 (m, 3H), 3.49 - 3.15 (m, 2H), 1.97 - 1.60 (m, 2H), 1.55 - 1.26 (m, 9H), 0.70 - 0.32 (m, 4H).

Step 6: Dimethylacetamide (DMAc) (17 mL), water (1.7 mL), **A2-6** (860 mg, 1.761 mmol) and LiCl (149.31 mg, 3.522 mmol) were sequentially added to a dry single-neck flask. The resulting reaction mixture was stirred at 100°C for 12 hours. TLC showed that the reaction was completed, and then the mixture was diluted with water (200 mL) and extracted with EA (80 mL * 2). The combined organic layers were washed with saturated brine, dried over Na₂SO₄, filtered and concentrated under reduced pressure. The resulting residue was purified by silica gel chromatography (0 to 20% ethyl acetate/petroleum ether) to obtain **A2-7** as a colorless oil (750 g, yield: 98.97%).

¹H NMR (400 MHz, CDCl₃) δ 7.17 (t, *J* = 7.9 Hz, 1H), 6.88 - 6.77 (m, 1H), 4.52 (ddd, *J* = 13.1, 9.0, 3.7 Hz, 1H), 4.12 (dd, *J =* 16.9, 10.1 Hz, 2H), 3.55 (dd, *J =* 27.8, 10.4 Hz, 1H), 3.33 (dd, *J =* 41.6, 10.4 Hz, 1H), 2.37 - 2.23 (m, 1H), 1.94 - 1.83 (m, 1H), 1.49 (d, *J =* 3.8 Hz, 9H), 0.72 - 0.55 (m, 4H).

Step 7: DCM (5 mL), *n*-heptane (5 mL) and **A2-7** (950 mg, 2.208 mmol) were sequentially added to a dry 50 mL three-neck flask under nitrogen atmosphere at 0°C, and phenylmagnesium bromide (9.9 mL, 9.9 mmol, a solution dissolved in THF, concentration of 1 M) was added dropwise to the mixture. After the reaction was completed, the reaction mixture was quenched by pouring into ice-cold saturated ammonium chloride solution (20 mL), diluted with water and extracted with ethyl acetate. The combined organic phases were washed with saturated brine, dried over anhydrous sodium sulfate, filtered, and concentrated under reduced pressure, and the resulting residue was subjected to silica gel column chromatography (0 to 20% ethyl acetate/petroleum ether) to obtain **A2-8** as a yellow oil (300 mg, yield: 26.73%).

¹H NMR (400 MHz, CDCl₃) δ 7.67 (d, *J =* 7.2 Hz, 2H), 7.29 (t, *J =* 7.4 Hz, 2H), 7.25 - 7.20 (m, 1H), 7.12 - 7.05 (m, 1H), 6.57 (ddd, *J =* 10.0, 8.7, 2.6 Hz, 1H), 6.22 (s, 1H), 4.28 (d, *J =* 5.1 Hz, 1H), 3.69 (d, *J =* 14.0 Hz, 1H), 3.63 - 3.55 (m, 2H), 3.41 (s, 1H), 1.96 (dd, *J =* 13.3, 4.5 Hz, 1H), 1.74 (s, 1H), 1.51 (s, 9H), 0.66 - 0.42 (m, 4H).

Step 8: THF (5 mL), **A2-8** (300 mg, 0.598 mmol) and potassium *tert*-butoxide (0.708 mL, 0.708 mmol, a solution dissolved in THF, concentration of 1 M) were sequentially added to a dry 50 mL single-neck flask. The resulting yellow solution was stirred at 0°C for 10 minutes. TLC showed that the reaction was completed. The reaction mixture was quenched by adding saturated citric acid aqueous solution (5 mL) and extracted with MTBE (5 mL). The combined organic phases were washed with saturated brine, dried over anhydrous sodium sulfate, filtered, and concentrated under reduced pressure, and the resulting residue was purified by silica gel chromatography (0 to 10% ethyl acetate/petroleum ether) to obtain **A2-9** as a white solid (288 mg, yield: 99.93%).

LC-MS: m/z 434 [M-55]⁺.

Step 9: Compound **A2-9** (288 mg, 0.59 mmol), bis(pinacolato)diboron (299.5 mg, 1.179 mmol), xylene (6 mL), Pd(dppf)Cl₂·CH₂Cl₂ (48.16 mg, 0.059 mmol) and potassium pivalate (248.06 mg, 1.769 mmol) were sequentially added to a dry three-neck flask. The reaction mixture was stirred at 100°C for 12 hours under nitrogen atmosphere. After the reaction was completed, the reaction mixture was diluted with water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 10:1) to obtain crude intermediate **A2** (289 mg).

LC-MS: m/z 436 [M+H-100]⁺.

¹H NMR (400 MHz, CDCl₃) δ 7.55 - 7.49 (m, 2H), 7.32 (dd, *J =* 10.3, 4.7 Hz, 2H), 7.23 (d, *J* = 7.3 Hz, 1H), 6.91 (dd, *J =* 9.5, 2.2 Hz, 1H), 6.63 (dd, *J =* 9.3, 2.3 Hz, 1H), 4.63 (t, *J =* 11.0 Hz, 1H), 4.10 - 4.02 (m, 1H), 3.61 - 3.34 (m, 2H), 3.02 - 2.75 (m, 1H), 2.18 (dd, *J =* 25.7, 12.5 Hz, 1H), 1.77 (dd, *J =* 22.4, 14.7 Hz, 1H), 1.47 - 1.36 (m, 9H), 1.31 (t, *J =* 5.8 Hz, 12H), 0.48 - 0.32 (m, 2H), 0.23 - 0.01 (m, 2H).

### Synthesis of intermediate A11: tert-Butyl (S)-((5-chloro-6-fluoro-2-phenyl-4-(4,4,5,5-tetramethyl-1,3,2-dioxaborolan-2-yl)-2,3-dihydrobenzofuran-2-yl)methyl)(methyl)carbamate

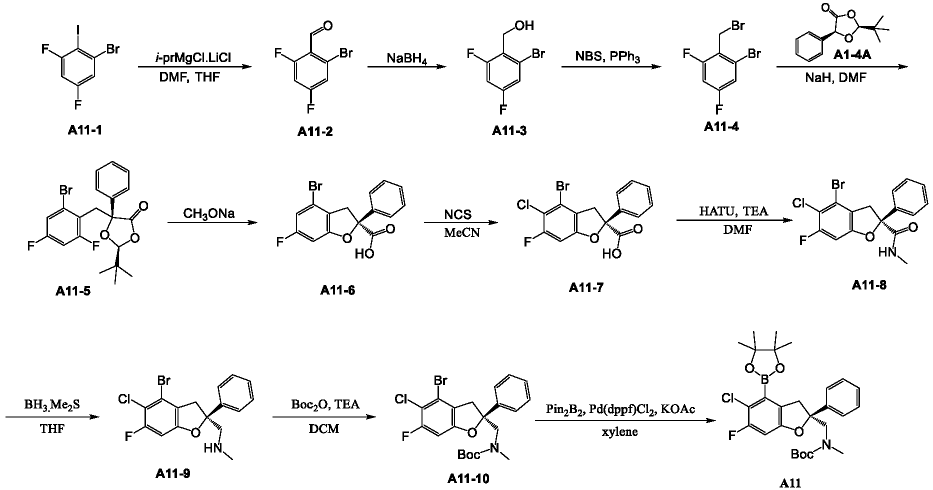

Step 1: THF (150 mL) and **A11-1** (15 g, 47.04 mmol) were sequentially added to a dry three-neck flask under nitrogen atmosphere, and *i*-PrMgCl.LiCl (39.8 mL, 51.74 mmol, a solution dissolved in THF, concentration of 1.3 M) was added dropwise after cooling to -70°C, and stirred at this temperature for 1 hour. Then DMF (5.16 g, 70.56 mmol) was added dropwise, and stirred for another 1 hour. TLC showed that the reaction was completed. The reaction mixture was quenched by pouring into saturated NH₄Cl aqueous solution at 0°C and extracted with ethyl acetate, then the combined organic phases were washed with saturated brine, dried over anhydrous sodium sulfate, filtered, and concentrated under reduced pressure to obtain yellow oily **A11-2** (5.8 g, crude), which was used directly in the next step.

1H NMR (400 MHz, Chloroform-d) δ 10.28 (s, 1H), 7.31 - 7.23 (m, 1H), 6.92 (ddd, J = 10.7, 8.4, 2.5 Hz, 1H).

Step 2: THF (60 mL), MeOH (10 mL), **A11-2** (5.8 g, crude) and NaBH₄ (596 mg, 15.75 mmol) were sequentially added to a dry 250 mL single-neck flask. The reaction mixture was stirred at 20°C for 0.5 hours. TLC showed that the reaction was completed. The reaction mixture was quenched by pouring into saturated NH₄Cl aqueous solution at 0°C and extracted with ethyl acetate, then the combined organic phases were washed with saturated brine, dried over anhydrous sodium sulfate, filtered, and concentrated under reduced pressure to obtain yellow oily **A11-3** (4.2 g, crude), which was used directly in the next step.

Step 3: DCM (40 mL), **A11-3** (4.2 g, 18.83 mmol) and PPh₃ (5.93 g, 22.6 mmol) were sequentially added to a dry 100 mL single-neck flask, and stirred to dissolve. Then NBS (4.02 g, 22.6 mmol) was slowly added thereto. The resulting yellow reaction mixture was stirred at 20°C for 0.5 hours. TLC showed that the reaction was completed. The reaction mixture was loaded onto the column using the wet method, then subjected to silica gel column chromatography (5 to 10% dichloromethane/petroleum ether) to obtain **A11-4** as a colorless oil (4.1 g, yield: 76%).

¹H NMR (400 MHz, Chloroform-d) δ 7.19 (dt, J = 7.8, 2.1 Hz, 1H), 6.85 (ddd, J = 9.4, 8.4, 2.5 Hz, 1H), 4.60 (d, J = 2.0 Hz, 2H).

Step 4: DMF (65 mL), **A11-4** (6.5 g, 22.73 mmol) and **A1-4A** (5.01 g, 22.73 mmol) were sequentially added to a dry 250 mL three-neck flask. The mixture was stirred to dissolve and slowly added with NaH (1.27 g, 31.83 mmol, dispersed in mineral oil, concentration of 60%). The resulting yellow reaction mixture was stirred at 25°C for 2 hours. TLC showed that the reaction was completed. The reaction mixture was quenched by pouring into saturated NH₄Cl aqueous solution and extracted with ethyl acetate, then the combined organic phases were washed with saturated brine, dried over anhydrous sodium sulfate, filtered, and concentrated under reduced pressure, and the resulting residue was subjected to silica gel column chromatography (0 to 5% ethyl acetate/petroleum ether) to obtain **A11-5** as a white solid (8.3 g, yield: 86%).

Step 5: **A11-5** (8.3 g, 19.52 mmol) and MeONa (8.3 mL, 44.89 mmol, a solution dissolved in MeOH, concentration of 5.4 M) were sequentially added to a dry 250 mL single-neck flask, and the resulting yellow suspension was stirred at 50°C for 2 hours. After the reaction was completed, the reaction mixture was quenched by pouring into 1 M hydrochloric acid, diluted with water and extracted with ethyl acetate. The combined organic phases were washed with saturated brine, dried over anhydrous sodium sulfate, filtered, and concentrated under reduced pressure, and the resulting residue was subjected to silica gel column chromatography (0 to 5% ethyl acetate/petroleum ether) to obtain **A11-6** as a yellow oil (6 g, yield: 87%).

¹H NMR (400 MHz, DMSO-*d*₆) δ 13.64 (s, 1H), 7.59 - 7.52 (m, 2H), 7.48 - 7.34 (m, 3H), 7.07 (dd, J = 9.1, 2.2 Hz, 1H), 7.02 (dd, J = 9.4, 2.2 Hz, 1H), 3.98 (dd, J = 16.2, 1.4 Hz, 1H), 3.50 (dd, J = 16.2, 1.8 Hz, 1H).

LCMS: m/z 337 [M+H]⁺.

Step 6: Acetonitrile (45 mL), **A11-6** (4.4 g, 12.53 mmol) and NCS (1.84 g, 13.7 mmol) were sequentially added to a dry single-neck flask. The resulting reaction mixture was stirred at 80°C for 2 hours. After TLC showed that the reaction was completed, the reaction mixture was concentrated under reduced pressure, and the resulting residue was purified by silica gel chromatography (0 to 4% ethyl acetate/petroleum ether) to obtain **A11-7** as a colorless oil (4.3 g, yield: 89%).

Step 7: DMF (120 mL), **A11-7** (10 g, 26.91 mmol), methylamine hydrochloride (3.63 g, 53.82 mmol) and HATU (15.35 g, 40.37 mmol) were sequentially added to a dry 250 mL single-neck flask. Then TEA (8.17 g, 80.73 mmol) was slowly added dropwise, and the resulting yellow suspension was stirred at 25°C for 1 hour. TLC showed that the reaction was completed. The reaction mixture was diluted with water and extracted with ethyl acetate, then the combined organic phases were washed with saturated brine, dried over anhydrous sodium sulfate, filtered, and concentrated under reduced pressure, and the resulting residue was purified by silica gel chromatography (0 to 20% ethyl acetate/petroleum ether) to obtain yellow oily **A11-8** (6.8 g, yield: 65.7%).

¹H NMR (400 MHz, Chloroform-d) δ 7.64 - 7.56 (m, 2H), 7.41 - 7.31 (m, 3H), 6.78 (d, J = 8.6 Hz, 1H), 6.62 (s, 1H), 4.22 (dd, J = 16.5, 1.7 Hz, 1H), 3.46 (dd, J = 16.6, 2.0 Hz, 1H), 2.82 (d, J = 5.0 Hz, 3H).

LC-MS: m/z 385.9 [M+H]⁺.

Step 8: THF (80 mL) and **A11-8** (8.0 g, 20.8 mmol) were sequentially added to a dry 300 mL sealed tube, and then BH₃.Me₂S (62.4 mL, 2 M, 124.8 mmol) was slowly added thereto at room temperature, and the resulting colorless solution was heated to 80°C and stirred for 12 hours. TLC showed that the reaction was completed. The reaction mixture was quenched by slowly adding MeOH dropwise until there was no obvious bubbling, and then subjected to rotary evaporation to dryness. The residue was then quenched with a 4 M dioxane solution of hydrochloric acid until there was no bubbling. Then the pH was adjusted to about 9 with saturated NaHCO₃ solution. The mixture was extracted with ethyl acetate, then the combined organic phases were washed with saturated brine, dried over anhydrous sodium sulfate, filtered, and concentrated under reduced pressure to obtain yellow oily **A11-9** (7.5 g, crude), which was used directly in the next step.

Step 9: DCM (75 mL), **A11-9** (7.5 g, 20.23 mmol), TEA (2.46 g, 24.28 mmol) and Boc₂O (5.3 g, 24.28 mmol) were sequentially added to a dry 250 mL single-neck flask. The resulting yellow reaction mixture was stirred at 20°C for 1 hour. TLC showed that the reaction was completed. The reaction mixture was subjected to rotary evaporation to dryness under reduced pressure, and subjected to silica gel column chromatography (0 to 10% dichloromethane/petroleum ether) to obtain **A11-10** as a colorless oil (8.0 g, purity: 84%).

Step 10: Anhydrous xylene (89 mL), **A11-10** (8.9 g, 18.91 mmol), bis(pinacolato)diboron (6.7 g, 26.47 mmol, 1.4 eq), Pd(dppf)Cl₂.CH₂Cl₂ (1.24 g, 1.51 mmol) and KOAc (5.57 g, 56.72 mmol) were sequentially added to a 250 mL dry single-neck flask, and the reaction mixture was replaced with nitrogen three times and then heated to 120°C and stirred for 11 hours. TLC showed that the reaction was completed. The reaction mixture was filtered, subjected to rotary evaporation to dryness under reduced pressure, and subjected to silica gel column chromatography (0 to 9% ethyl acetate/petroleum ether) to obtain **A11** as a white solid (6.6 g, purity: 74%).

### Synthesis of intermediate A20: tert-Butyl (2S)-2-((S)-5-chloro-6-fluoro-2-phenyl-4-(4,4,5,5-tetramethyl-1,3,2-dioxaborolan-2-yl)-2,3-dihydrobenzofuran-2-yl)-4-hydroxypyrrolidine-1 -carboxylate

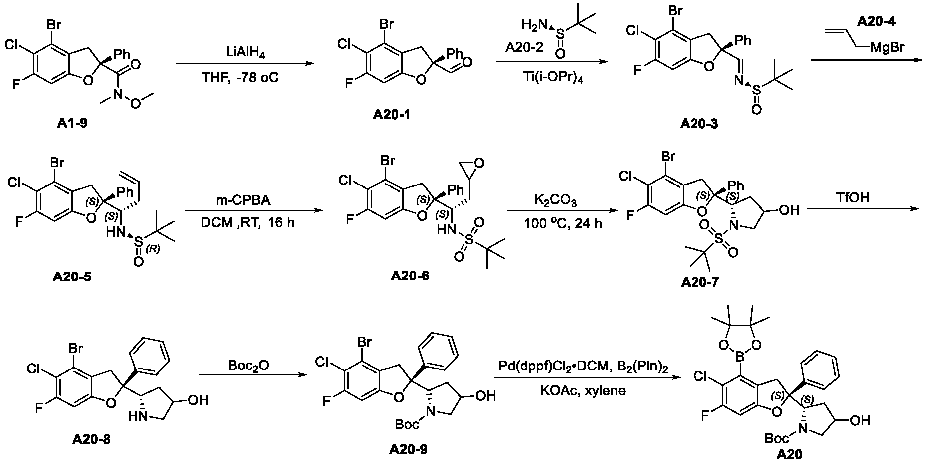

Step 1: Lithium aluminum hydride (7.36 g, 217.05 mmol) was added to anhydrous tetrahydrofuran (300 mL), cooled to -30°C, and a tetrahydrofuran solution (300 mL) of **A1-9** (60 g, 144.70 mmol) was slowly added dropwise, and then the reaction mixture was stirred and reacted for another 1 hour at 0°C. TLC showed that the reaction was completed. Water (7.4 mL), 15% sodium hydroxide solution (7.4 mL) and water (22 mL) were sequentially added to the reaction mixture, and diatomite was added thereto, stirred for 10 minutes and then subjected to suction filtration. The filtrate was concentrated to obtain **A20-1** (60.00 g, crude) as a brown oil.

Step 2: **A20-1** (60 g, 168.74 mmol) and **A20-2** (20.45 g, 168.74 mmol) were dissolved in dichloroethane (600 mL), and tetraisopropyl titanate (143.87 g, 506.21 mmol) was added dropwise, and the reaction mixture was heated to 60°C and reacted for 3 hours after the addition was completed. TLC showed that the raw materials basically disappeared. Additional dichloromethane (2000 mL) was added thereto, then water (100 mL) and diatomite (20 g) were sequentially added to the system, stirred for 10 minutes, filtered, and the filtrate was concentrated and purified by column chromatography (ethyl acetate/petroleum ether = 0 to 5%) to obtain **A20-3** (22.00 g, yield: 27.00%) as a yellow oily product.

¹H NMR (400 MHz, DMSO-*d*₆) δ 7.97 (s, 1H), 7.56 - 7.38 (m, 5H), 7.17 (d, J= 9.7 Hz, 1H), 4.06 (d, *J =* 16.1 Hz, 1H), 3.55 (d, *J =* 16.1 Hz, 1H), 0.92 (s, 9H).

Step 3: **A20-3** (22 g, 47.95 mmol) was dissolved in tetrahydrofuran (220 mL), and the system was replaced with nitrogen three times, cooled to 0°C, and **A20-4** (20.90 g, 143.86 mmol) was slowly added dropwise, and then the reaction mixture was stirred at 0°C for 2 hours after the addition was completed. TLC showed that the reaction was completed. Ammonium chloride (50 mL) was added to quench the reaction, and the mixture was extracted with dichloromethane (500 mL × 3). The organic phases were combined and concentrated to dryness, and purified by column chromatography (ethyl acetate/petroleum ether = 0 to 20%) to obtain **A20-5** (17.00 g, yield: 67.24%) as a yellow oil.

LC-MS: m/z 499.7 [M+H]⁺.

Step 4: **A20-5** (17 g, 33.94 mmol) was dissolved in dichloromethane (300 mL), and *m-*chloroperoxybenzoic acid (17.51 g, 101.83 mmol) was added thereto, and the reaction mixture was stirred and reacted at 25°C for 16 hours. Additional 300 mL of dichloromethane was added thereto, and the reaction mixture was washed with saturated sodium bicarbonate (500 mL × 2) and saturated brine (500 mL), respectively. The organic phase was separated and concentrated to obtain **A20-6** (14.30 g, crude) as a yellow oil, which was used directly in the next step.

LC-MS: m/z 553.8 [M+Na]⁺.

Step 5: **A20-6** (14.3 g, 26.84 mmol) and potassium carbonate (11.13 g, 80.51 mmol) were added to *N,N-*dimethylformamide (200 mL), and the system was replaced with nitrogen three times, and then the reaction mixture was heated to 100°C, stirred and reacted for 12 hours. TLC showed that the reaction was completed. The reaction mixture was cooled to room temperature, filtered, and the filtrate was concentrated directly to dryness to obtain **A20-7** (13.10 g, crude) as a yellow solid product.

Step 6: **A20-7** (13.1 g, 24.58 mmol) was dissolved in dichloromethane (200 mL), cooled to 0°C, and trifluoromethanesulfonic acid (11.07 g, 73.75 mmol) was added dropwise, and then the system was stirred and reacted at 0°C for 2 hours after the addition was completed. TLC showed that the reaction was completed. The pH was adjusted to 7 to 8 by slowly adding 1 M sodium hydroxide solution. The organic phase was separated, washed with saturated brine (100 mL), dried over anhydrous sodium sulfate, filtered, and the filtrate was concentrated to dryness to obtain **A20-8** (10.00 g, crude) as a yellow solid product.

LC-MS: m/z 411.7 [M+H]⁺.

Step 7: **A20-8** (10 g, 24.23 mmol), Boc anhydride (6.35 g, 29.08 mmol) and triethylamine (7.36 g, 72.70 mmol) were sequentially added to 1,4-dioxane (200 mL), and the reaction mixture was stirred and reacted at 25°C for 1 hour. The reaction mixture was concentrated to dryness, and ethyl acetate (200 mL) was added thereto, washed with water (100 mL) and saturated brine (100 mL) respectively, dried over anhydrous sodium sulfate, and then purified by column chromatography (ethyl acetate/petroleum ether = 0 to 20%) to obtain **A20-9** (4.00 g, yield: 30.58%) as a yellow solid product.

¹H NMR (400 MHz, DMSO-*d*₆) δ 7.48 - 7.26 (m, 5H), 7.19 (d, *J* = 9.5 Hz, 1H), 4.55 (s, 1H), 4.33 (d, *J =* 15.6 Hz, 1H), 4.01 - 3.61 (m, 2H), 3.11 (s, 1H), 2.61 (s, 1H), 1.83 (s, 1H), 1.59 - 1.05 (m, 10H).

LC-MS: m/z 411.7 [M+H-100]⁺.

Step 8: Referring to the synthesis method of compound **A2,** intermediate **A20-9** was used instead of intermediate **A2-9,** and one-step reaction was carried out to obtain intermediate **A20.**

LC-MS: m/z 460 [M+H-100]⁺.

### Synthesis of intermediate A23: tert-Butyl (S)-2-(S)-4-(S)-6-cyano-2-fluoro-3,4-dihydroxyphenyl)-5-chloro-6-fluoro-2-phenyl-2,3-dihydrobenzofuran-2-yl)pyrrolidine-1-carboxylate

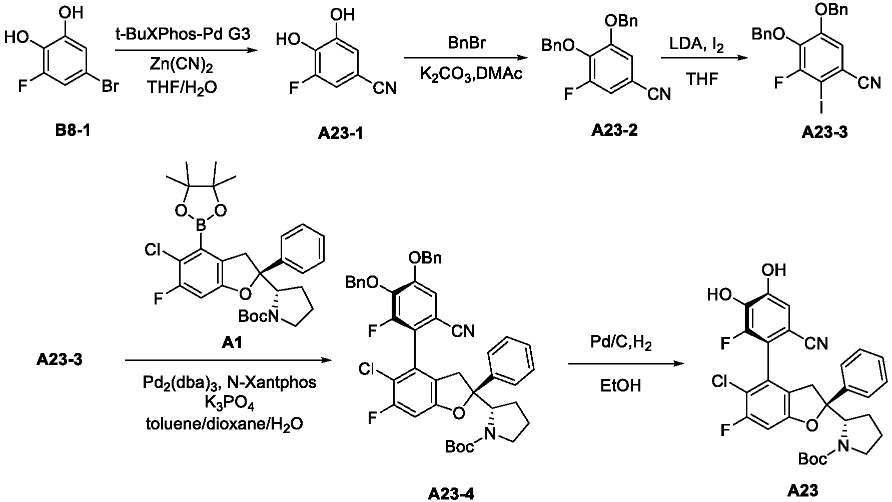

Step 1: Zinc cyanide (0.08 g, 77.295 mmol) and t-BuXPhos-Pd-G3 (1.54 g, 1.932 mmol) were added to a solution of **B8-1** (8 g, 38.647 mmol) in water (100 mL) and tetrahydrofuran (20 mL) under argon atmosphere. The reaction mixture was stirred at 70°C for 12 hours under nitrogen atmosphere. Insoluble substances were filtered out with diatomite, and the filter residue was thoroughly rinsed with ethyl acetate (200 mL). Dilute hydrochloric acid (100 mL) was added thereto and stirred thoroughly to separate the organic phase. The organic phase was washed with saturated brine (2 × 100 mL), dried over anhydrous sodium sulfate, and filtered. The filtrate was concentrated under reduced pressure, stirred thoroughly for 3 hours with 100 mL of a mixed solvent (ethyl acetate/petroleum ether = 1/3), and filtered to obtain **A23-1** as a white solid (5.4 g, yield: 91.26%).

LCMS: m/z 305 [2M-H]⁻.

¹H NMR (400 MHz, DMSO-*d*₆) δ 10.33 (s, 2H), 7.24 (d, *J* = 10.3 Hz, 1H), 6.96 (s, 1H).

Step 2: Potassium carbonate (10.52 g, 76.154 mmol ) and benzyl bromide (12.73 g, 74.424 mmol) were added to a DMAc (70 mL) solution of **A23-1** (5.3 g, 34.616 mmol). The reaction mixture was stirred at 50°C for 12 hours. The reaction mixture was filtered, diluted with ethyl acetate (200 mL), washed first with water (5 × 100 mL), then with saturated brine (2 × 100 mL), dried over anhydrous sodium sulfate, filtered, and the filtrate was concentrated under reduced pressure and purified by silica gel chromatography (ethyl acetate/petroleum ether/dichloromethane = 1/10/100) to obtain **A23-2** as a white solid (10 g, yield: 87%).

¹H NMR (400 MHz, CDCl₃) δ 7.28 (d, *J* = 11.9 Hz, 2H), 7.26 - 7.21 (m, 4H), 7.21 - 7.16 (m, 3H), 7.13 (s, 1H), 6.91 (dd, *J =* 9.6, 1.7 Hz, 1H), 6.86 (s, 1H), 5.07 (s, 2H), 4.98 (s, 2H).

Step 3: LDA (2.0 mol/L in THF/hexane, 3.75 mL, 7.499 mmol) was added dropwise to a tetrahydrofuran (20 mL) solution of **A23-2** (1000 mg, 3 mmol) at -70°C under argon atmosphere. After the dropwise addition was completed, the mixture was stirred at -70°C for 30 minutes. A tetrahydrofuran solution (5 mL) of iodine (914 mg, 3.6 mmol) was added dropwise. The reaction mixture was reacted at -70°C for 1 hour, slowly poured into ice-cold saturated ammonium chloride solution (30 mL), and extracted by adding ethyl acetate (3 × 50 mL). The organic phases were combined, washed with saturated brine (2 × 10 mL), dried over anhydrous sodium sulfate, filtered. The filtrate was concentrated under reduced pressure and purified by silica gel chromatography (ethyl acetate/petroleum ether/dichloromethane = 1/10/100) to obtain **A23-3** (1000 mg, yield: 72.6 %).

¹H NMR (400 MHz, CDCl₃) δ 7.41 - 7.31 (m, 10H), 7.04 (d, *J =* 1.6 Hz, 1H), 5.19 (s, 2H), 5.11 (s, 2H).

Step 4: **A23-3** (1.3 g, 2.829 mmol), intermediate **A1** (2308 mg, 3.395 mmol), potassium phosphate (3.6 g, 16.975 mmol), toluene (14 mL), water (5.6 mL), 1,4-dioxane (14 mL), Pd₂(dba)₃ (259 mg, 0.283 mmol) and *N*-XantPhos (312 mg, 0.566 mmol) were sequentially added to a reaction flask. The reaction mixture was stirred at 100°C for 18 hours under nitrogen atmosphere. The reaction mixture was added with water and extracted with ethyl acetate. The organic phase was dried over anhydrous sodium sulfate, filtered, concentrated, and the residue was purified by silica gel chromatography (petroleum ether/ethyl acetate = 5:1) to obtain a mixture as light yellow solid **A23-4** (600 mg, yield: 28.3%).

LC-MS: m/z 693 [M-55+H]⁺.

Step 5: **A23-4** (900 mg, 1.201 mmol), 10% wet palladium on carbon (320 mg, 0.3 mmol) and ethanol (20 mL) were sequentially added to a reaction flask, and the reaction mixture was stirred at 30°C for 18 hours under hydrogen atmosphere. Insoluble substances were filtered out with diatomite, and the filter residue was thoroughly rinsed with ethanol (100 mL) and concentrated to obtain **A23** as a white solid (660 mg, yield: 97%).

LC-MS: m/z 567 [M-H]⁻.

### Synthesis of intermediate A24: tert-Butyl (S)-2-((S)-5-chloro-6-fluoro-2-(phenyl-d₅)-4-(4,4,5,5-tetramethyl-1,3,2-dioxaborolan-2-yl)-2,3-dihydrobenzofuran-2-yl)pyrrolidine-1-carboxylate

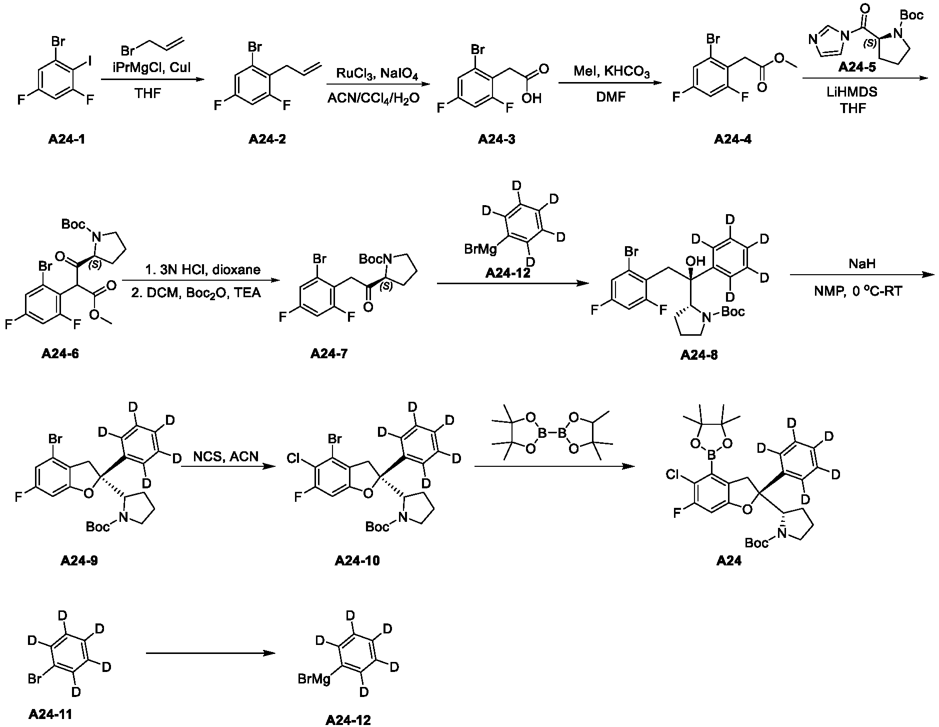

Step 1: **A24-1** (10 g, 31.359 mmol) and tetrahydrofuran (100 mL) were added to a dry three-neck flask and cooled to -40°C under nitrogen atmosphere. Isopropylmagnesium chloride (20.383 mL, 40.766 mmol, 2 mmol/L) was slowly added dropwise to the reaction mixture. The reaction mixture was warmed to 0°C and stirred for 1 hour. CuI (1.49 g, 7.840 mmol) was added to the reaction mixture and stirred for 10 minutes to obtain a gray solution, then a tetrahydrofuran solution of 3-bromoprop-1-ene (4.094 mL, 47.038 mmol) was slowly added dropwise (with obviously exothermic, controlling the internal temperature to be less than 10°C). The reaction mixture was warmed to room temperature (17°C) and stirred overnight. TLC showed that the reaction was completed. The reaction mixture was quenched with saturated NH₄Cl aqueous solution and ammonia water, and extracted with dichloromethane. The combined organic phases were washed with saturated brine, dried over anhydrous sodium sulfate, filtered, and concentrated under reduced pressure. The concentrated residue was subjected to silica gel column chromatography (PE = 1) to obtain **A24-2** as a colorless oil (7 g, yield: 95.76%).

Step 2: **A24-2** (7.8 g, 33.468 mmol), carbon tetrachloride (150 mL), water (300 mL) and sodium periodate (35.79 g, 167.339 mmol) were sequentially added to a dry three-neck flask under nitrogen atmosphere. Then an acetonitrile (150 mL) solution of RuCl₃ (0.88 g, 3.347 mmol) was added dropwise to the reaction mixture, and the reaction mixture was reacted at room temperature (13°C) for 18 hours until TLC showed that the reaction was completed. 10% sulfuric acid aqueous solution (100 mL) was added to the reaction mixture, and extracted with ethyl acetate. The combined organic phases were washed with saturated brine, dried over anhydrous sodium sulfate, filtered, and concentrated under reduced pressure. The concentrated residue was subjected to silica gel column chromatography (30% ethyl acetate/petroleum ether) to obtain **A24-3** (6.50 g, yield: 77.37%).

Step 3: **A24-3** (6.64 g, 26.451 mmol), DMF (65 mL), iodomethane (2.141 mL, 34.386 mmol) and K₂CO₃ (7.31 g, 52.902 mmol) were sequentially added to a dry three-neck flask under nitrogen atmosphere. The reaction mixture was stirred at room temperature (13°C) for 18 hours. TLC showed that the reaction was completed. The reaction mixture was quenched with water and extracted with ethyl acetate. The combined organic phases were washed with saturated brine, dried over anhydrous sodium sulfate, filtered, and concentrated under reduced pressure to obtain **A24-4** as a colorless oil (6.9 g, yield: 98.42%).

Step 4: A three-neck flask was cooled to -65°C under nitrogen atmosphere. **A24-4** (1 g, 3.77 mmol), **A24-5** (1.1 g, 4.15 mmol), THF (10 mL) and LiHMDS (7.5 mL, 7.5 mmol) were sequentially added thereto and stirred for 30 minutes, and the reaction mixture was warmed to 13°C and stirred for 3 hours. TLC showed that the reaction was completed. The reaction mixture was quenched with saturated ammonium chloride and the pH was adjusted to about 4 with 1 mmol/L hydrochloric acid. The mixture was extracted with ethyl acetate. The combined organic phases were washed with saturated brine, dried over anhydrous sodium sulfate, filtered, and concentrated under reduced pressure. The concentrated residue was subjected to silica gel column chromatography (6% ethyl acetate/petroleum ether) to obtain **A24-6** as a colorless oil (1.7 g, yield: 97.5%).

Step 5: **A24-6** (1.7 g, 3.68 mmol), dioxane (9 mL) and 3 mmol/L hydrochloric acid (9 mL) were sequentially added to a dry three-neck flask under nitrogen atmosphere. The reaction mixture was heated to 95°C and stirred for 6 hours. TLC showed that the reaction was completed. The pH of the reaction mixture was adjusted to about 9 with saturated sodium carbonate aqueous solution. Sodium carbonate (0.78 g, 7.35 mmol) and di-*tert*-butyl dicarbonate (1.61 g, 7.35 mmol) were added to the solution and stirred at room temperature (13°C) for 3 hours. TLC showed that the reaction was completed. The reaction mixture was quenched with water and extracted with ethyl acetate. The combined organic phases were washed with saturated brine, dried over anhydrous sodium sulfate, filtered, and concentrated under reduced pressure. The concentrated residue was subjected to silica gel column chromatography (5% ethyl acetate/petroleum ether) to obtain **A24-7** as a white solid (1.35 g, yield: 90.8%).

Step 6: **A24-7** (8 g, 19.79 mmol) was dissolved in dichloromethane (20 mL) and *n-*heptane (20 mL), then **A24-12** (20.65 g, 110.82 mmol, see step 10 for the synthesis method) was added thereto at 0°C, and the reaction mixture was reacted at 20°C for 2 hours. LC-MS detected that the reaction was completed. The reaction mixture was poured into saturated ammonium chloride (200 mL), and the pH was adjusted to 6 with 6N hydrochloric acid. The mixture was extracted with ethyl acetate (100 mL × 3), dried, concentrated under reduced pressure, and subjected to column chromatography (ethyl acetate/petroleum ether = 0 to 10%) to obtain target product **A24-8** (1.50 g, yield: 12.44%) as a colorless oil.

LC-MS: m/z 509.20 [M+23]⁺.

Step 7: Compound **A24-8** (1.5 g, 3.08 mmol) was dissolved in NMP (20 mL), and NaH (492.42 mg, 12.31 mmol, purity of 60%) was added thereto at 25°C and stirred for 2 hours. LC-MS showed that the reaction was completed. The reaction mixture was quenched by pouring into ice water (50 mL), extracted with ethyl acetate (30 mL × 3). The organic phase was washed with saturated brine (30 mL × 3), dried over anhydrous sodium sulfate, filtered, concentrated under reduced pressure, and subjected to column chromatography (ethyl acetate/petroleum ether = 0 to 10%) to obtain target product **A24-9** (1.25 g, yield: 78.21%) as a colorless oil.

LC-MS: m/z 411.20 [M-56+1]⁺.

Step 8: **A24-9** (1.25 g, 2.67 mmol) was dissolved in acetonitrile (20 mL), then NCS (535.68 mg, 4.01 mmol) was added thereto, and then the reaction mixture was reacted at 60°C for 4 hours. LC-MS showed that the reaction was completed. The reaction mixture was concentrated under reduced pressure and subjected to column chromatography (ethyl acetate/petroleum ether = 0 to 5%) to obtain target product **A24-10** (1.20 g, yield: 80.47%) as a colorless oil.

LC-MS: m/z 445.20 [M-56+1]⁺.

Step 9: Compound **A24-10** (1.25 g, 2.49 mmol), bis(pinacolato)diboron (948.81 mg, 3.74 mmol), Pd(dppf)Cl₂ (90.38 mg, 124.54 µmol) and KOAc (488.91 mg, 4.98 mmol) were sequentially added to toluene (20 mL), and the reaction mixture was reacted at 100°C for 16 hours. LC-MS showed that the reaction was completed. The reaction mixture was directly concentrated under reduced pressure and subjected to column chromatography (ethyl acetate/petroleum ether = 0 to 10%) to obtain target product **A24** (1.0 g, yield: 58.51%) as a yellow oil.

LC-MS: m/z 493.30 [M-56+1]⁺.

Step 10: **A24-11** (18 g, 111.08 mmol) was dissolved in tetrahydrofuran (150 mL), and polished magnesium strips (4.05 g, 166.63 mmol) and iodine (281.94 mg, 1.11 mmol) were added thereto, and then the reaction mixture was reacted at 70°C for 2 hours under N₂ atmosphere. **A24-12** (20.65 g, 110.82 mmol) was obtained as a light yellow liquid, which was directly used in the next reaction step.

The following intermediates were synthesized from the corresponding intermediates or raw materials, using the above methods or improved methods and the methods reported in the literature (WO2021186324).

| **Intermediate number** | **Structural formula** | **Name** | **Analytical data** |
|---|---|---|---|
| **A5** | 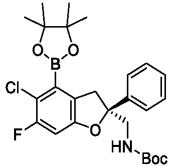 | *tert*-Butyl (*S*)-(5-chloro-6-fluoro-2-phenyl-4-(4,4,5,5-tetramethyl-1,3,2-dioxaborolan-2-yl)-2,3-dihydrobenzofuran-2-yl)methyl)carbamate | LCMS: m/z 504.20 [M+H]⁺. |
| **A6** | 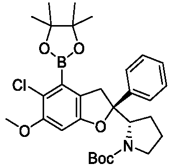 | *tert*-Butyl (*S*)-2-((*S*)-5-chloro-6-methoxy-2-phenyl-4-(4,4,5,5-tetramethyl-1,3,2-dioxaborolan-2-yl)-2,3-dihydrobenzofuran-2-yl)pyrrolidine-1-carboxylate | LCMS: m/z 556.26 [M+H]⁺. |
| **A15** | 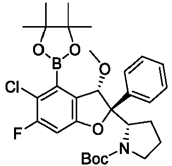 | *tert*-Butyl (*S*)-2-((2*S*,3*S*)-5-chloro-6-fluoro-3-methoxy-2-phenyl-4-(4,4,5,5-tetramethyl-1,3,2-dioxaborolan-2-yl)-2,3-dihydrobenzofuran-2-yl)pyrrolidine-1-carboxylate | LCMS: m/z 574.2 [M+H]⁺. |
| **A16** | 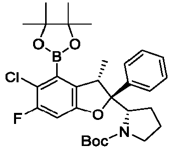 | *tert*-Butyl (*S*)-2-((2*S*,3*S*)-5-chloro-6-fluoro-3-methyl-2-phenyl-4-(4,4,5,5-tetramethyl-1,3,2-dioxaborolan-2-yl)-2,3-dihydrobenzofuran-2-yl)pyrrolidine-1-carboxylate | LCMS: m/z 558.2 [M+H]⁺. |
| **A17** | 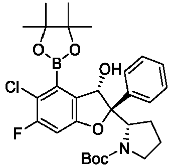 | *tert*-Butyl (*S*)-2-((2*S*,3*S*)-5-chloro-6-fluoro-3-hydroxy-2-phenyl-4-(4,4,5,5-tetramethyl-1,3,2-dioxaborolan-2-yl)-2,3-dihydrobenzofuran-2-yl)pyrrolidine-1-carboxylate | LCMS: m/z 560.2 [M+H]⁺. |
| **A25** | 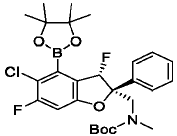 | *tert*-Butyl (((2*S*,3*S*)-5-chloro-3,6-difluoro-2-phenyl-4-(4,4,5,5-tetramethyl-1,3,2-dioxaborolan-2-yl)-2,3-dihydrobenzofuran-2-yl)methyl)(methyl)carbamate | LCMS: m/z 536 [M+H]⁺. |
| **A27** | 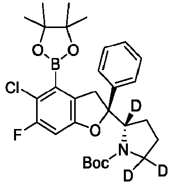 | *tert*-Butyl (*S*)-2-((*S*)-5-chloro-6-fluoro-2-phenyl-4-(4,4,5,5-tetramethyl-1,3,2-dioxaborolan-2-yl)-2,3-dihydrobenzofuran-2-yl)pyrrolidine-1-carboxylate-2,5,5-*d*₃ | LCMS: m/z 547 [M+H]⁺. |

### Intermediate A26: tert-Butyl (S)-2-((S)-5-chloro-6-fluoro-2-phenyl-2,3-dihydrobenzofuran-2-yl)pyrrolidine-1-carboxylate-4-trifluoro-λ⁴-borate, potassium salt:

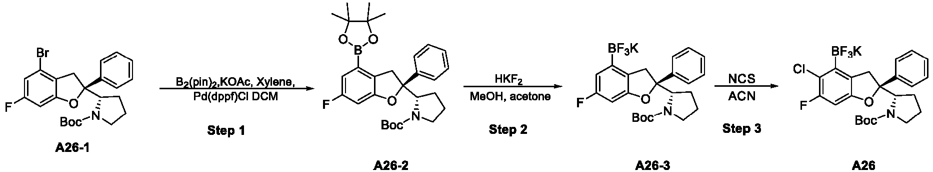

Step 1: Toluene (10 to 20 kg) was added to a reaction kettle under nitrogen atmosphere, and stirring was started. **A26-1** (1.74 kg, see WO2021186324A for synthesis steps), potassium acetate (0.227 to 1.13 kg) and bis(pinacolato)diboron (0.6 to 1.8 kg) were added thereto, and the reaction mixture was bubbled with nitrogen for 0.1 to 2 hours with stirring. 1,1'-Bis(diphenylphosphino)ferrocene-palladium(II)dichloride dichloromethane complex (9.2 g to 0.64 kg) was added thereto, and the reaction mixture was bubbled with nitrogen for 0.1 to 2 hours with stirring. The reaction mixture was heated to 75 to 105°C under nitrogen atmosphere, stirred at this temperature for 5 to 20 hours. HPLC detected that the reaction was completed, and then the reaction mixture was cooled to 25 to 50°C, filtered with 300 to 400 mesh silica gel (0.35 to 0.90 kg) on a Buchner funnel, soaked in *n*-heptane (0.87 kg), compacted and filtered. n-Heptane (3.1 to 6.2 kg) was added to the reaction kettle to rinse the filter cake. The filtrates were combined and concentrated at 25 to 50°C under reduced pressure until no fraction was distilled off, thus obtaining crude **A26-2** (1.65 kg, yield: 86%), which was directly used in the next production.

Step 2: Acetone (5.50 to 9.16 kg) and **A26-2** (2.3 kg) were added to a reaction kettle, stirred until dissolved, and methanol (5.28 to 8.80 kg) was added thereto, and then prepared potassium hydrogen fluoride aqueous solution (10.56 kg, 3 M) was added thereto. The reaction mixture was stirred at 0 to 35°C for 6 to 16 hours. HPLC detected that the reaction was completed, and then tap water (12 to 20 kg) was added to the reaction kettle and stirred for 30 minutes. The temperature was controlled at 25 to 50°C and the reaction mixture was concentrated under reduced pressure. After concentrating until there was no obvious fraction, distillation under reduced pressure was carried out for another 2 to 3 hours. The temperature was reduced to 5 to 15°C. The residue was stirred for 1 hour and filtered. The filtrate was put into a barrel, and the filter cake was put into a bag. Tap water (12 to 20 kg) was added to the reaction kettle, and the filter cake was added to the reaction kettle. The temperature was controlled at 0 to 30°C and the mixture was stirred for 0.5 to 1 hour. The mixture was filtered. The filtrate was put into a barrel, and the filter cake was put into a bag. *n*-Heptane (2 to 6 kg) was added to the reaction kettle and the filter cake was added to the reaction kettle. The temperature was controlled at 0 to 30°C and the mixture was stirred for 1 to 2 hours. The mixture was filtered. The filtrate was put into a barrel, and the filter cake was put into a bag. The filter cake was transferred to a vacuum oven with the oven temperature set at 25 to 50°C and vacuum dried for 10 to 30 hours. The crude was slurried with *n-*heptane and dichloromethane (20:1 to 5:1, v/v) at room temperature for 0.5 to 2 hours, filtered, rinsed and dried to obtain **A26-3** as a white solid (1.64 kg, yield: 89%, purity > 95%).

Step 3: **A26-3** (1.75 kg) and acetonitrile (15 to 20 L) were sequentially added to a three-neck flask, and stirring was started at room temperature. *p*-Toluenesulfonic acid hydrate (0.34 to 0.68 kg) was added to the reaction mixture with continued stirring, and NCS (429 to 716 g, added in three times with an interval of 0.5 to 1 hour), and then the reaction was continued with stirring at room temperature for 3 to 20 hours. The process control results by HPLC met the predetermined quality standards, and the reaction mixture was subjected to suction filtration. The filter cake was rinsed with acetonitrile (2.2 L). Potassium bicarbonate (715 to 1430 g) and di-*tert*-butyl dicarbonate (780 to 1559 g) were added to the filtrate, and stirred at room temperature. The process control results by HPLC met the predetermined quality standards. Methyl *tert*-butyl ether (3 to 5 V) and 10% potassium bicarbonate aqueous solution (10 V) were added to the reaction mixture, and the reaction mixture was stirred for another 0.5 to 1 hour, left to stand, and then the phases were separated. The reaction mixture was extracted twice, and the organic phases were combined twice and concentrated to dryness under reduced pressure at 10 to 50. The organic phase that was concentrated to dryness was dissolved by adding methyl *tert*-butyl ether (5 to 10 V) and transferred to a reaction kettle. 10% potassium bicarbonate aqueous solution (10 V) was added to the reaction kettle, stirred for 0.5 to 1 hour, and the phases were separated (repeated 4 times). The organic phase was concentrated to dryness under reduced pressure at 10 to 50°C. Methyl *tert*-butyl ether (2 to 5 V) was added to dissolve the organic phase that was concentrated to dryness, and concentrated to dryness under reduced pressure at 10 to 50°C (repeat 2 times). The organic phase that was concentrated to dryness was transferred to a reaction kettle, and methyl *tert*-butyl ether (3 to 5 V) was added thereto, then n-heptane (3 to 5 V) was added slowly dropwise, and stirring was continued for 1 to 5 hours after the addition. The reaction mixture was subjected to suction filtration and the filter cake was rinsed with *n*-heptane (0.5 to 2 V). The filter cake was dried in a vacuum drying oven at 20 to 40°C to obtain **A26** as an off-white solid (1.175 kg, yield: 62.5%, purity > 90%).

### Synthesis of intermediate A28 (mixture): tert-Butyl (S)-2-((S)-5-chloro-4-((S)-8-cyano-6-fluoro-4-oxo-3,4-dihydrobenzo[5,6][1,4]dioxo [2,3-d]pyridin-7-yl)-6-fluoro-2-phenyl-2,3-dihydrobenzofuran-2-yl)pyrrolidine-1-carboxylate and tert-butyl (S)-2-((S)-5-chloro-4-((S)-8-cyano-6-fluoro-1-oxo-1,2-dihydrobenzo[5,6][1,4]dioxo2,3-d]pyridin-7-yl)-6-fluoro-2-phenyl-2,3-dihydrobenzofuran-2-yl)pyrrolidine-1-carboxylate

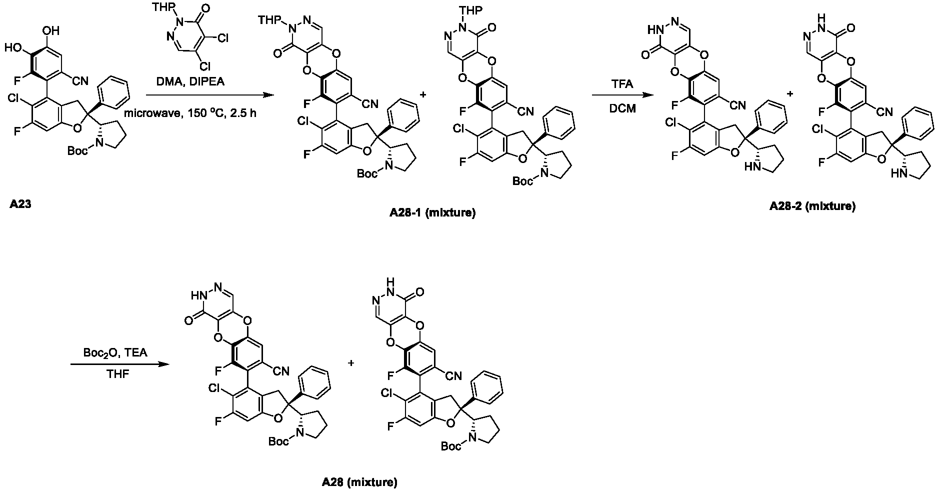

Step 1: Referring to step 1 of example 5, 4,5-dichloro-2-(tetrahydro-pyran-2-yl)-2*H-*pyrazin-3-one was used in the reaction instead of 2,3-dichloropyridazine, and compound **A28-1** (mixture) was obtained after purification.

LC-MS: m/z 745 [M+H]⁺.

Step 2: **A28-1** (120 mg, 0.16 mmol, mixture) was dissolved in DCM (2 mL), and TFA (1 mL) was added thereto. The reaction mixture was reacted at room temperature for 3 hours, and the TLC plate showed that the reaction was completed. The reaction mixture was concentrated under reduced pressure to obtain **A28-2** as a white solid (90 mg, mixture).

LC-MS: m/z 561 [M+H]⁺.

Step 3: **A28-2** (90 mg, mixture) was dissolved in THF (2 mL), and then Boc₂O (52 mg, 0.24 mmol) and TEA (32 mg, 0.32 mmol) were added thereto. The reaction mixture was reacted at room temperature for 1 hour, and the TLC plate showed that the reaction was completed. The reaction mixture was concentrated under reduced pressure. The crude was purified by column chromatography (petroleum ether: ethyl acetate = 2:1) to obtain **A28** as a white solid (90 mg, mixture).

LC-MS: m/z 661 [M+H]⁺.

### Synthesis of intermediate B1: 8-Fluoro-7-iodo-2,3-dihydrobenzo[b][1,4]dioxine-6-carbonitrile

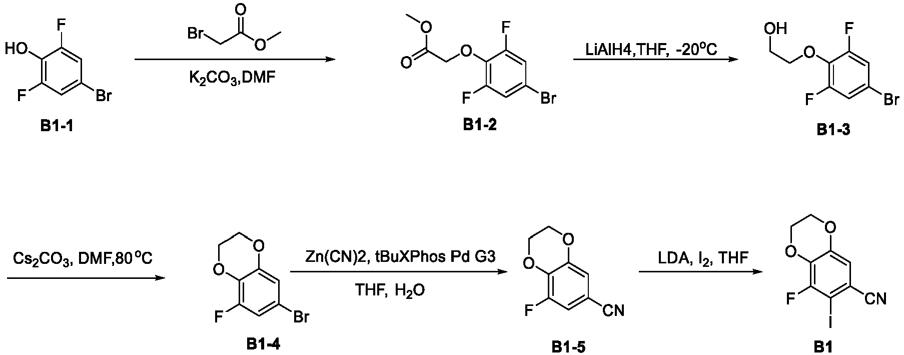

Step 1: **B1-1** (125.00 g, 598.115 mmol), methyl bromoacetate (62.511 mL, 657.926 mmol), K₂CO₃ (124.00 g, 897.172 mmol) and DMF (1000 mL) were sequentially added to a single-neck flask. The reaction mixture was stirred at 80°C for 8 hours under nitrogen atmosphere. The mixture was diluted with water and extracted three times with a mixed solution of petroleum ether/ethyl acetate (1/1). The organic layer was dried over Na₂SO₄ and concentrated under reduced pressure. The residue was purified by silica gel chromatography (petroleum ether/ethyl acetate = 20/1) to obtain **B1-2** (168.1 g, yield: 100%) as a colorless oil.

¹H NMR (400 MHz, CD₃OD) δ 7.11 - 7.02 (m, 2H), 4.71 (s, 2H), 3.83 (s, 3H).

Step 2: **B1-2** (164 g, 583.526 mmol) and THF (1500 mL) were added to a single-neck flask. The mixture was cooled to -20°C under nitrogen atmosphere, and LiAlH₄ (33.22 g, 875.289 mmol) was slowly added thereto, and then the reaction mixture was stirred at -20°C for 1 hour. TLC showed that the reaction was completed. H₂O (33 mL), 15% NaOH solution (33 mL) and H₂O (99 mL) were added to the reaction mixture, filtered, and the filtrate was diluted with water and extracted three times with ethyl acetate (500 mL * 3). The organic layer was dried over Na₂SO₄ and concentrated under reduced pressure to obtain **B1-3** (129 g, yield: 87%) as a yellow solid.

¹H NMR (400 MHz, CD₃OD) δ 6.99 - 6.98 (m, 2H), 4.25 - 4.21 (m, 2H), 3.90 - 3.83 (m, 2H).

Step 3: **B1-3** (60 g, 237.117 mmol), Cs₂CO₃ (115.89 mg, 355.675 mmol) and DMF (500 mL) were sequentially added to a single-neck flask. The reaction mixture was stirred at 80°C for 8 hours under nitrogen atmosphere. TLC showed that the reaction was completed. The mixture was diluted with water and extracted three times with ethyl acetate (300 mL * 3). The organic layer was dried over Na₂SO₄ and concentrated under reduced pressure. The residue was purified by silica gel chromatography (petroleum ether/ethyl acetate = 5/1) to obtain **B1-4** (40 g, yield: 72.39%) as a white solid.

Step 4: **B1-4** (40000 mg, 171.644 mmol), t-BuXPhos-Pd-G3 (6826.12 mg, 8.582 mmol), Zn(CN)₂ (14110.54 mg, 120.151 mmol), THF (300 mL) and H₂O (100 mL) were sequentially added to a single-neck flask, and the reaction mixture was stirred at 40°C under nitrogen atmosphere overnight. TLC detected that the reaction was completed. The mixture was filtered, and the filtrate was concentrated under reduced pressure. The residue was purified by silica gel column chromatography (petroleum ether/ethyl acetate = 10/1) to obtain **B1-5** (26500 mg, yield: 86.18%).

¹H NMR (400 MHz, CD₃OD) δ 7.01 - 6.98 (m, 2H), 4.40 - 4.38 (m, 2H), 4.34 - 4.32 (m, 2H).

Step 5: **B1-5** (21500 mg, 120.011 mmol) was dissolved in THF (1000 mL), and n-butyllithium solution (62.406 mL, 156.015 mmol) was slowly added dropwise at -78°C, and then the reaction mixture was stirred at -78°C for 1 hour after the dropwise addition was completed. Iodine (45690.05 mg, 180.017 mmol) was added thereto and stirred at -78°C for 1 hour after the addition was completed. The reaction mixture was quenched with water and poured into saturated sodium sulfite aqueous solution. The mixture was extracted with ethyl acetate (300 mL * 3), and the organic phase was washed with saturated brine, dried over anhydrous sodium sulfate, filtered, and the volatiles were removed under reduced pressure. The crude was purified by silica gel column chromatography (petroleum ether/ethyl acetate = 3/1) to obtain B1 as a white solid (16300 mg, yield: 52.63%).

### Synthesis of intermediate B2: 6-Fluoro-7-iodo-2-methyl-2,4-dihydrochromeno[3,4-c]pyrazole-8-carbonitrile

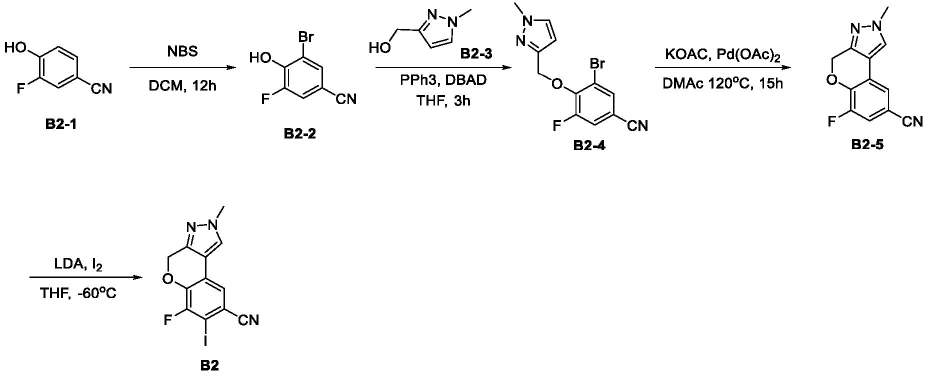

Step 1: Dichloromethane (20 mL), **B2-1** (2 g, 14.6 mmol, 1.0 eq) and *N-*bromosuccinimide (NBS) (3.12 g, 17.504 mmol, 1.2 eq) were sequentially added to a dry single-neck flask. The reaction mixture was stirred at 25°C for 12 hours. After the reaction was completed, the reaction mixture was diluted with water (100 mL) and extracted with ethyl acetate (35 mL * 3). The combined organic phases were washed with saturated brine (100 mL), dried over anhydrous sodium sulfate, filtered, and concentrated under reduced pressure. The resulting residue was purified by silica gel chromatography (0 to 15% ethyl acetate/petroleum ether) to obtain **B2-**2 as a yellow solid (3 g, yield: 95.2%).

¹HNMR (400 MHz, CDCl₃) δ 6.19 (s, 1H), 7.39 (dd, *J =* 1.6, 9.6 Hz, 1H), 7.63 (d, *J =* 1.6 Hz, 1H).

Step 2: Extra dry tetrahydrofuran (10 mL), **B2-2** (1 g, 4.629 mmol, 1.0 eq), **B2-3** (0.62 g, 5.555 mmol, 1.2 eq) were sequentially added to a dry three-neck flask, and then triphenylphosphine (1.2 g, 4.6 mmol, 1 eq) and di-*tert*-butyl azodicarboxylate (DBAD) (1.05 g, 4.6 mmol, 1 eq) were added thereto under nitrogen atmosphere. The reaction mixture was stirred at 25°C for 3 hours. After the reaction was complete, the reaction mixture was diluted with water (100 mL) and extracted with ethyl acetate (40 mL * 2). The combined organic phases were washed with saturated brine (100 mL), dried over anhydrous sodium sulfate, filtered, and concentrated under reduced pressure. The resulting residue was purified by silica gel chromatography (0 to 15% ethyl acetate/petroleum ether) to obtain **B2-4** as a white solid (950 mg, yield: 66%).

LC-MS: m/z 309.99 [M+H]⁺.

Step 3: Extra dry dimethylacetamide (DMAc) (16 mL), **B2-4** (500 mg, 1.6 mmol, 1.0 eq), palladium acetate (36.2 mg, 0.161 mmol, 0.1 eq) and potassium acetate (190 mg, 1.935 mmol, 1.2 eq) were sequentially added to a dry single-neck flask, and the reaction mixture was stirred at 120°C for 15 hours under nitrogen atmosphere. After the reaction was complete, the reaction mixture was diluted with water (30 mL) and extracted with ethyl acetate (20 mL * 2). The combined organic phases were washed with saturated brine (100 mL), dried over anhydrous sodium sulfate, filtered, and concentrated under reduced pressure. The resulting solid was slurried with petroleum ether and ethyl acetate (10/1) to obtain **B2-5** as a brown solid (220 mg, yield: 59%).

LC-MS: m/z 230 [M+H]⁺.

Step 4: **B2-5** (1.25 g, 5.43 mmol) and iodine (1 capsule, about 40 mg) were dissolved in dry tetrahydrofuran (2.0 L). After three replacements with argon, the mixture was cooled to -65°C with a dry ice acetone bath. LDA was added dropwise until the color of the iodine faded (became colorless), then lithium diisopropylamide (LDA) (5.4 mL, 10.8 mmol) was added dropwise. After the addition was completed, the reaction mixture was reacted at -65°C for 1 hour. Iodine (1.45 g, 5.71 mmol) dissolved in dry tetrahydrofuran (20 mL) was slowly added dropwise (over about 2 hours) to the reaction mixture. After the addition was completed, the reaction mixture was reacted at -65°C for 30 minutes, and the reaction mixture was poured into ice water, concentrated to remove most of the tetrahydrofuran, extracted with dichloromethane/methanol (10:1, 3 * 500 mL). The organic phases were combined, washed with saturated brine, dried, filtered, dried, concentrated, and the residue was purified by column chromatography (dichloromethane: methanol = 1:0 to 500:1) to obtain **B2** as a light yellow solid (a total of 0.6 g of product was obtained, yield: 31.0%).

LCMS: m/z: 355.9 [M+H]⁺.

### Synthesis of intermediate B3: 8-Fluoro-7-iodoquinoline-6-carbonitrile

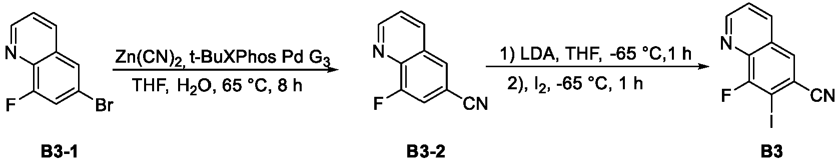

Step 1: Zn(CN)₂ (405.20 mg, 3.45 mmol) was added to a solution of **B3-1** (1.00 g, 4.42 mmol) in tetrahydrofuran (45 mL) and water (15 mL). After vacuuming, the mixture was replaced with argon three times. Methanesulfonato(2-di-t-butylphosphino-2',4',6'-tri-*i*-propyl-1,1'-biphenyl)(2'-amino-1,1'-biphenyl-2-yl)palladium(II) (t-BuXphos-Pd-G3) (175.70 mg, 0.22 mmol) was added thereto. After vacuuming, the mixture was replaced with argon three times. The reaction mixture was reacted at 65°C for 8 hours. The reaction mixture was slowly poured into ice water (20 mL), extracted by adding ethyl acetate (3 * 30 mL), filtered to remove the insoluble substances and the filter cake was rinsed with ethyl acetate (50 mL). The organic phases were combined, washed with saturated brine (2 * 30 mL), dried over anhydrous sodium sulfate, filtered, and the filtrate was concentrated under reduced pressure and purified by silica gel chromatography (ethyl acetate/petroleum ether = 1/30 to 1/5) to obtain **B3-2** (705 mg, yield: 92.6%).

¹H NMR (400 MHz, CDCl₃): δ ppm 9.13 (dd, *J =* 4.0, 1.5 Hz, 1H), 8.29 - 8.27 (m, 1H), 8.06 (s, 1H), 7.66 - 7.62 (M, 1H), 7.61 (dd, *J =* 9.6, 1.6 Hz, 1H).

¹⁹F NMR (376 MHz, CDCl₃): δ ppm -120.74.

Step 2: LDA (2.0 mol/L in THF/hexane, 2.46 mL, 4.92 mmol) was added dropwise to a tetrahydrofuran (10 mL) solution of **B3-2** (705.0 mg, 4.09 mmol) at -65°C under argon atmosphere. After the dropwise addition was completed, the mixture was stirred at -65°C for 1 hour. A tetrahydrofuran (10 mL) solution of iodine (1.04 g, 4.09 mmol) was added dropwise. The reaction mixture was reacted at -65°C for 1 hour, slowly poured into ice water (20 mL), and extracted by adding ethyl acetate (3 * 30 mL). The organic phases were combined, washed with saturated brine (2 * 30 mL), dried over anhydrous sodium sulfate, and filtered. The filtrate was concentrated under reduced pressure and purified by silica gel chromatography (ethyl acetate/petroleum ether = 1/50 to 1/4) to obtain **B3** (940 mg, yield: 77.0%).

LCMS: m/z 298.9 [M+H]⁺

¹H NMR (400 MHz, CDCl₃): δ ppm 9.13 (dd, *J* = 4.0, 1.2 Hz, 1H), 8.27 (d, *J* = 8.4 Hz, 1H), 8.06 (d, *J =* 1.2 Hz, 1H), 7.65 (dd, *J =* 8.4, 4.4 Hz, 1H).

¹⁹F NMR (376 MHz, CDCl₃): δ ppm -95.55.

### Synthesis of intermediate B4: 5-Fluoro-6-iodo-3H-spiro[benzo[b][1,4]dioxine-2,1'-cyclopropane]-7-carbonitrile

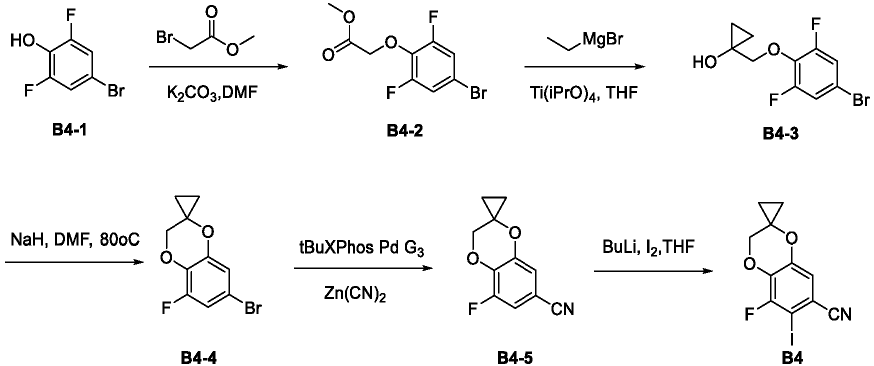

Step 1: A suspension of **B4-1** (9000 mg, 43064 mmol), methyl 2-bromoacetate (4.5 mL, 47371 mmol) and K₂CO₃ (8927.87 mg, 64596 mmol) in DMF (70 mL) was stirred at room temperature for 1.5 hours. The reaction mixture was diluted with water and extracted with a mixed solution of ethyl acetate/petroleum ether (1/1). The organic layer was dried over Na₂SO₄ and concentrated under reduced pressure, and the residue was purified by silica gel chromatography (ethyl acetate/petroleum ether = 1/20) to obtain intermediate **B4-2** (12500 mg, yield: 103.28%) as a colorless oil.

¹H NMR (400 MHz, CDCl₃) δ 7.16 - 7.03 (m, 2H), 4.75 (s, 2H), 3.80 (s, 3H).

Step 2: Intermediate **B4-2** (4000 mg, 14232 mmol) was added to THF (60 mL), and tetraisopropyl titanate (4.3 mL, 14232 mmol) was added thereto at 0°C. Ethylmagnesium bromide (3.4 M, 11.3 mL) was slowly added thereto at 0°C, and the reaction mixture was stirred at room temperature for 1 hour. 1 M hydrochloric acid was added to the reaction mixture at 0°C, and the mixture was extracted with ethyl acetate. The organic layer was washed with brine, dried over anhydrous sodium sulfate, filtered and the solvent was removed. The resulting crude was purified by silica gel column chromatography (ethyl acetate/petroleum ether = 1/4) to obtain intermediate **B4-3** (3400 mg, yield: 85.60%) as a yellow oil.

¹H NMR (400 MHz, CDCl₃) δ 7.14 - 7.08 (m, 2H), 4.14 (s, 2H), 0.96 - 0.87 (m, 2H), 0.64 - 0.56 (m, 2H).

Step 3: A suspension of intermediate **B4-3** (1000 mg, 3583 mmol) and NaH (214.99 mg, 5375 mmol) in DMF (30 mL) was stirred at 80°C for 4 hours under nitrogen atmosphere. The reaction mixture was poured into ice water and extracted with ethyl acetate. The organic phase was washed with saturated brine, dried, concentrated, and purified by silica gel column chromatography (ethyl acetate/petroleum ether= 1/10) to obtain intermediate **B4-4** (550 mg, yield: 59.25%).

Step 4: Intermediate **B4-4** (440 mg, 1.698 mmol), t-BuXPhos-Pd-G3 (67.54 mg, 0.085 mmol) and Zn(CN)₂ (179.51 mg, 1.529 mmol) were dissolved in THF (20 mL) and H₂O (6 mL), and the reaction mixture was stirred at 40°C under nitrogen atmosphere overnight. The mixture was filtered, and the filtrate was concentrated under reduced pressure. The residue was purified by silica gel column chromatography (ethyl acetate/petroleum ether = 1/10) to obtain intermediate **B4-5** (210 mg, yield: 60.26%).

Step 5: Intermediate **B4-5** (220 mg, 1.072 mmol) was dissolved in THF (10 mL), cooled to -78°C, then *n*-BuLi (0.558 mL, 1.394 mmol) was added dropwise. After the addition was completed, the reaction mixture was stirred at -78°C for 20 minutes, and iodine (408.19 mg, 1.608 mmol) was added to the reaction mixture, and the mixture was warmed to room temperature. Water was added to quench the reaction, and the reaction mixture was extracted with ethyl acetate, dried over Na₂SO₄, and concentrated. The crude was purified by silica gel column chromatography (ethyl acetate/petroleum ether = 1/10) to obtain intermediate **B4** (200 mg, 0604 mmol, 56.34%) as a yellow solid.

### Synthesis of intermediate B5: 7-Fluoro-8-iodo-5H-chromeno[3,4-b]pyrazine-9-carbonitrile

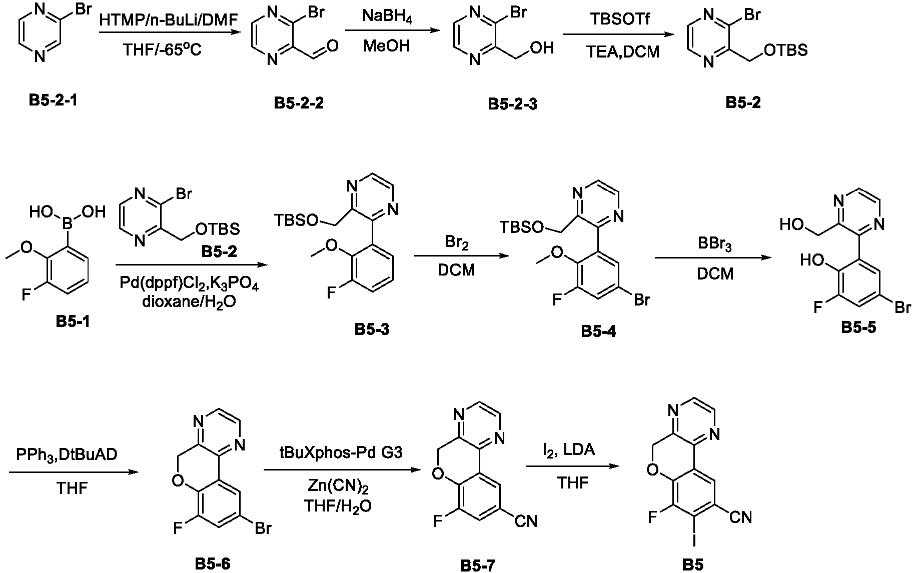

Step 1: 2,2,6,6-Tetramethylpiperidine (10.276 mL, 60.381 mmol) was dissolved in THF (192 mL), and the mixture was cooled to -65°C under argon atmosphere in a dry ice acetone bath. Butyl lithium (26.165 mL, 65.413 mmol) was added dropwise, and then the temperature of the mixture was maintained at 0°C for half an hour and then lowered to -65°C again. A mixture **of B5-2-1** (4.553 mL, 50.318 mmol) and THF (192 mL) was added dropwise, and the temperature was maintained for 30 minutes. A mixture of DMF (9.729 mL, 125.794 mmol) and THF (192 mL) was added dropwise, and the resulting mixture was stirred for another 2 hours. The reaction was then completed to produce **B5-2-2.** The untreated reaction mixture was directly used in the next step.

Step 2: MeOH (120 mL) was added dropwise at -65°C, and the mixture was stirred for 10 minutes. NaBH₄ (3.678 mL, 100.635 mmol) was slowly added thereto, and the temperature of the mixture was slowly raised to 0°C and maintained for 1 hour, then the reaction was completed. The reaction mixture was poured into 500 mL of NH₄Cl ice-aqueous solution and extracted three times with ethyl acetate. The organic phase was washed with saturated brine. The organic phases were combined, dried over anhydrous sodium sulfate, filtered, and concentrated to obtain **B5-2-3** (7 g, yield: 73.61%).

LCMS: m/z: 191 [M+H]⁺.

Step 3: Triethylamine (1.798 mL, 12.936 mmol) and tert-butyldimethylsilyl trifluoromethanesulfonate (2.181 mL, 9.486 mmol) were added to a DCM (30 mL) solution of**B5-2-3** (1630 mg, 8.624 mmol). The reaction mixture was reacted at room temperature for 1 hour under argon atmosphere. The reaction mixture was concentrated under reduced pressure and purified by silica gel chromatography (ethyl acetate/petroleum ether = 1/40 to 1/10) to obtain **B5-2** (2550 mg, yield: 97.5%).

¹H NMR (400 MHz, CDCl₃) δ 8.50 (d, *J =* 2.5 Hz, 1H), 8.26 (d, *J =* 2.4 Hz, 1H), 4.91 (s, 2H), 0.92 (s, 9H), 0.13 (s, 6H).

Step 4: Intermediate **B5-1** (950 mg, 5.59 mmol), intermediate **B5-2** (1525.72 mg, 5.031 mmol), dioxane (60 mL), water (12 mL), Pd(dppf)Cl₂ (204.51 mg, 0.279 mmol) and potassium phosphate (2373.13 mg, 11.18 mmol) were sequentially added to a dry single-neck flask. The reaction mixture was stirred at 90°C for 12 hours under nitrogen atmosphere. After the reaction was completed, the reaction mixture was diluted with water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 1/10 to 1/1) to obtain **B5-3** (1620 mg, yield: 83.16%).

¹H NMR (400 MHz, CDCl₃) δ 8.58 - 8.55 (m, 2H), 7.22 - 7.07 (m, 3H), 4.74 (s, 2H), 3.73 (d, *J =* 2.0 Hz, 3H), 0.77 (d, *J =* 2.9 Hz, 9H), -0.08 (s, 6H).

Step 5: Liquid bromine (0.287 mL, 5.596 mmol) was added to a dichloromethane (13 mL) solution of **B5-3** (1300 mg, 3.73 mmol), and the reaction mixture was stirred at 0°C for 6 hours under nitrogen atmosphere. The reaction mixture was slowly poured into ice water (20 mL) and extracted by adding dichloromethane (3 * 20 mL). The organic phases were combined, washed with saturated sodium thiosulfate solution (30 mL), washed with saturated brine (30 mL), dried over anhydrous sodium sulfate, and filtered. The filtrate was concentrated under reduced pressure and purified by silica gel chromatography (ethyl acetate/petroleum ether = 1/10 to 1/1) to obtain **B5-4** (600 mg, yield: 37.63%).

¹H NMR (400 MHz, CDCl₃) δ 8.57 (d, *J* = 0.7 Hz, 2H), 7.37 - 7.34 (m, 1H), 7.32 - 7.30 (m, 1H), 4.74 (s, 2H), 3.73 (d, *J=* 2.1 Hz, 3H), 0.80 (s, 9H), -0.05 (s, 6H).

Step 6: Boron tribromide (1.106 mL, 11.7 mmol) was added to a dichloromethane (12 mL) solution of **B5-4** (500 mg, 1.17 mmol) at 0°C, and the reaction mixture was stirred at room temperature for 12 hours under nitrogen atmosphere. The reaction mixture was slowly poured into ice water (20 mL) and extracted by adding dichloromethane (3 * 20 mL). The organic phases were combined, washed with saturated brine (30 mL), dried over anhydrous sodium sulfate, and filtered. The filtrate was concentrated under reduced pressure, and the residue was purified by silica gel chromatography (ethyl acetate/petroleum ether = 1/10 to 1/1) to obtain **B5-5** (340 mg, yield: 97.17%).

LCMS: m/z: 301 [M+H]⁺.

Step 7: Triphenylphosphine (434 mg, 1.655 mmol) and di-*tert*-butyl azodicarboxylate (381 mg, 1.655 mmol) were added to a tetrahydrofuran (30 mL) solution of **B5-5** (450 mg, 1.505 mmol). After vacuuming, the mixture was replaced with argon three times. The reaction mixture was reacted at room temperature for 18 hours, slowly poured into ice water (10 mL), and extracted with ethyl acetate (3 * 20 mL). The organic phases were combined, washed with saturated brine (2 * 20 mL), dried over anhydrous sodium sulfate, filtered, and the filtrate was concentrated under reduced pressure and purified by silica gel chromatography (ethyl acetate/petroleum ether = 1/10 to 2/5) to obtain **B5-6** (420 mg, yield: 99.2%).

¹H NMR (400 MHz, CDCl₃) δ 8.53 (d, *J =* 2.5 Hz, 1H), 8.45 (d, *J* = 2.6 Hz, 1H), 8.10 - 8.06 (m, 1H), 7.32 (dd, *J =* 9.7, 2.3 Hz, 1H), 5.43 (s, 2H).

LCMS: m/z: 281 [M+H]⁺.

Step 8: Zinc cyanide (334.25 mg, 2.846 mmol) and t-BuXPhos-Pd-G3 (67.83 mg, 0.085 mmol) were added to a solution of **B5-6** (400 mg, 1.423 mmol) in water (20 mL) and tetrahydrofuran (4 mL) under argon atmosphere. The reaction mixture was stirred at 70°C for 12 hours under nitrogen atmosphere. The reaction mixture was diluted with ethyl acetate (20 mL), washed with saturated brine (2 * 20 mL), dried over anhydrous sodium sulfate, and filtered. The filtrate was concentrated under reduced pressure and purified by silica gel chromatography (ethyl acetate/petroleum ether = 1/3) to obtain **B5-7** (256 mg, yield: 79.18%).

¹H NMR (400 MHz, CDCl₃) δ 8.57 (d, *J* = 2.5 Hz, 1H), 8.50 (d, *J =* 2.5 Hz, 1H), 8.29 (t, *J =* 1.6 Hz, 1H), 7.43 (dd, *J =* 9.7, 1.9 Hz, 1H), 5.55 (s, 2H).

Step 9: A tetrahydrofuran (2 mL) solution of iodine (268 mg, 1.056 mmol) was added dropwise to a tetrahydrofuran (8 mL) solution of **B5-7** (160 mg, 0.704 mmol) at -65°C under argon atmosphere. After the dropwise addition was completed, the mixture was stirred at -65°C for 10 minutes. LDA (2.0 mol/L in THF/hexane, 0.704 mL, 1.408 mmol) was added dropwise. The reaction mixture was reacted at -65°C for 2 hours, slowly poured into ice-cold saturated ammonium chloride solution (20 mL), and extracted by adding ethyl acetate (3 * 30 mL). The organic phases were combined, washed with saturated brine (2 * 30 mL), dried over anhydrous sodium sulfate, and filtered. The filtrate was concentrated under reduced pressure and purified by silica gel chromatography (ethyl acetate/petroleum ether = 1/3) to obtain **B5** (50 mg, yield: 20.11%).

¹H NMR (400 MHz, CDCl₃) δ 8.57 (d, *J* = 2.5 Hz, 1H), 8.51 (d, *J* = 2.5 Hz, 1H), 8.26 (d, *J =* 1.6 Hz, 1H), 5.56 (s, 2H).

### Synthesis of intermediate B6: 6-Fluoro-7-iodo-2-((2-(trimethylsilyl)ethoxy)methyl)-2,4-dihydrochromeno[3,4-d][1,2,3]triazole-8-carbonitrile

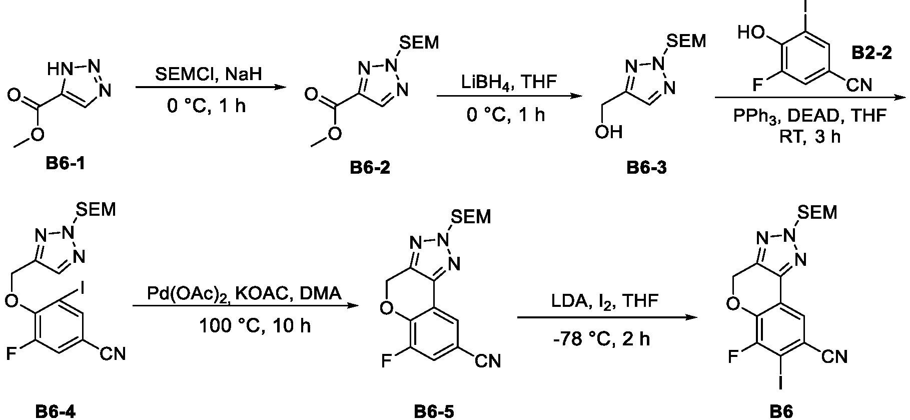

Step 1: Sodium hydride (60% dispersed in oil, 1.57 g, 39.34 mmol) was added to a tetrahydrofuran (250 mL) solution of **B6-1** (2.5 g, 19.67 mmol) at 0°C under argon atmosphere. The mixture was stirred and reacted at 0°C for 10 minutes. 2-(Trimethylsilyl)ethoxymethyl chloride (SEMCl) (3.44 g, 20.65 mmol) was added dropwise. The reaction mixture was stirred and reacted at 0°C for 1 hour, poured into ice water (100 mL), and extracted with ethyl acetate (3 * 200 mL). The organic phases were combined, washed with saturated brine (2 * 100 mL), dried over anhydrous sodium sulfate, filtered, and the filtrate was concentrated to dryness under reduced pressure. The residue was purified by silica gel chromatography (petroleum ether/ethyl acetate = 50/1 to 10/1) to obtain **B6-2** as a colorless oil (1.1 g, yield: 21.7%).

¹H NMR (400 MHz, CDCl₃): δ ppm 8.16 (s, 1H), 5.76 (s, 2H), 4.00 (s, 3H), 3.78 - 3.64 (m, 2H), 1.02 - 0.89 (m, 2H), 0.06 (s, 9H).

Step 2: A tetrahydrofuran solution of LiBH₄ (2 M, 4.27 mL, 8.54 mmol) was added dropwise to a tetrahydrofuran (20 mL) solution of **B6-2** (1.1 g, 4.27 mmol) at 0°C under argon atmosphere. The reaction mixture was stirred and reacted at 0°C for 1 hour. The reaction mixture was poured into ice water (15 mL). The reaction mixture was extracted with ethyl acetate (3 * 20 mL), and the organic phases were combined, washed with saturated brine (2 * 10 mL), dried over anhydrous sodium sulfate, and filtered. The filtrate was concentrated to dryness under reduced pressure and purified by silica gel chromatography (ethyl acetate/petroleum ether = 1/30 to 1/1) to obtain **B6-3** as a colorless liquid (870 mg, yield: 88.8%).

¹H NMR (400 MHz, CDCl₃): δ ppm 7.68 (s, 1H), 5.66 (s, 2H), 4.83 (s, 2H), 3.66 (dd, *J =* 11.1, 5.5 Hz, 2H), 0.94 (dd, *J =* 11.0, 5.6 Hz, 2H), 0.07 (s, 9H).

Step 3: Diethyl azodicarboxylate (951.8 mg, 5.57 mmol) was added dropwise to a tetrahydrofuran (260 mL) solution of **B6-3** (850 mg, 3.71 mmol), **B2-2** (975.8 mg, 3.71 mmol) and triphenylphosphine (1.24 g, 5.57 mmol) at 0°C under argon atmosphere. The reaction mixture was warmed to room temperature and reacted for 3 hours, slowly poured into ice water (40 mL), and extracted with ethyl acetate (3 * 60 mL). The phases were separated. The organic phases were combined, washed with saturated brine (2 * 40 mL), dried over anhydrous sodium sulfate, and filtered. The filtrate was concentrated under reduced pressure and purified by silica gel chromatography (ethyl acetate/petroleum ether = 1/40 to 1/1) to obtain **B6-4** as a light white powder (1.6 g, yield: 91.0%).
LCMS: m/z 475.0 [M+H]⁺
¹H NMR (400 MHz, DMSO-*d*₆): δ ppm 7.90 - 7.84 (m, 2H), 7.43 (dd, *J =* 10.6, 1.9 Hz, 1H), 5.67 (s, 2H), 5.42 (d, *J =* 1.1 Hz, 2H), 3.70 - 3.59 (m, 2H), 1.00 - 0.89 (m, 2H), 0.05 (s, 9H).

Step 4: Palladium acetate (75.8 mg, 0.34 mmol) and potassium acetate (662.5 mg, 6.74 mmol) were added to a dry *N,N*-dimethylacetamide (DMA) (40 mL) solution of **B6-4** (1.6 g, 3.37 mmol) under argon atmosphere. After vacuuming, the mixture was replaced with argon three times. The reaction mixture was reacted at 100°C for 10 hours, slowly poured into ice water (20 mL), and extracted by adding ethyl acetate (3 * 60 mL). The organic phases were combined, washed with saturated brine (2 * 30 mL), dried over anhydrous sodium sulfate, filtered, and the filtrate was concentrated under reduced pressure and purified by silica gel chromatography (ethyl acetate/petroleum ether = 1/20 to 1/1) to obtain **B6-5** as a light white powder (790 mg, yield: 67.6%).

¹H NMR (400 MHz, CDCl₃): δ ppm 7.85 (s, 1H), 7.40 (d, *J =* 9.7 Hz, 1H), 5.87 (s, 2H), 5.71 (s, 2H), 3.71 - 3.63 (m, 2H), 1.03 - 0.93 (m, 2H), 0.00 (s, 9H).

Step 5: A solution of LDA (2.0 M, 2.85 mL, 5.70 mmol) in tetrahydrofuran and n-hexane was added dropwise to a dry tetrahydrofuran (20 mL) solution of **B6-5** (790 mg, 2.28 mmol) at - 78°C under argon atmosphere. After vacuuming, the mixture was replaced with argon three times. The mixture was reacted at -78°C for 1 hour. A tetrahydrofuran (10 mL) solution of iodine (264.18 mg, 4.56 mmol) was added dropwise. After vacuuming, the reaction mixture was replaced with argon three times. The reaction mixture was reacted at this temperature for another 4 hours, slowly poured into ice water (20 mL), and extracted by adding ethyl acetate (3 * 40 mL). The organic phases were combined, washed with saturated brine (2 * 30 mL), dried over anhydrous sodium sulfate, filtered, and the filtrate was concentrated under reduced pressure and purified by silica gel chromatography (ethyl acetate/petroleum ether = 1/20 to 1/1) to obtain **B6** as a light yellow powder (325 mg, yield: 30.2%).

¹H NMR (400 MHz, DMSO-*d*₆): δ ppm 7.98 (d, *J =* 1.1 Hz, 1H), 5.79 (d, *J =* 13.7 Hz, 4H), 3.74 - 3.65 (m, 2H), 0.98 - 0.85 (m, 2H), 0.07 (s, 9H).

### Synthesis of intermediate B7: 8-Fluoro-7-iodo-2-methyl-2H-benzo[5,6][1,4]dioxacyclo[2,3-c]pyrazole-6-carbonitrile

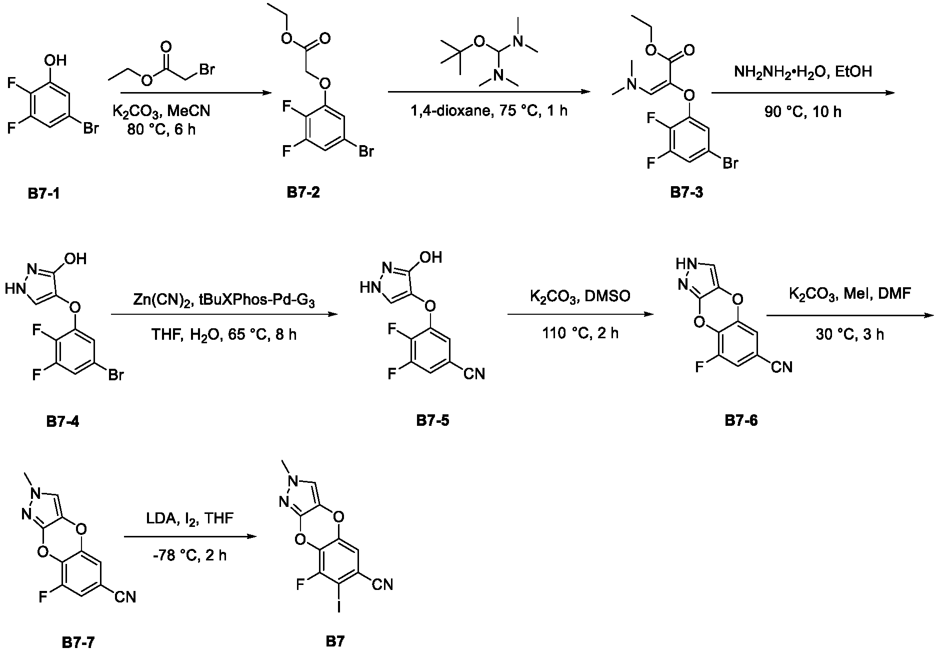

Step 1: K₂CO₃ (29.5 g, 210.6 mmol) was added to an acetone (200 mL) solution of**B7-1** (22.0 g, 105.3 mmol) under argon atmosphere. Ethyl bromoacetate (17.6 g, 105.3 mmol) was added dropwise. The reaction mixture was stirred and reacted at 80°C for 6 hours. The reaction mixture was poured into ice water (100 mL). The reaction mixture was extracted with ethyl acetate (3 * 400 mL). The organic phases were combined, washed with saturated brine (2 * 100 mL), dried over anhydrous sodium sulfate, filtered, and the filtrate was concentrated to dryness under reduced pressure. The residue was purified by silica gel chromatography (petroleum ether/ethyl acetate = 50/1 to 10/1) to obtain **B7-2** as a white solid (21.5 g, yield: 69.2%).

¹H NMR (400 MHz, MeOD-*d₄*): δ ppm 7.15 - 7.11 (m, 1H), 7.08 - 7.05 (m, 1H), 4.83 9s, 2H), 4.25 (q, *J =* 7.2 Hz, 2H), 1.28 (t, *J =* 7.2 Hz, 3H).

Step 2: tert-Butoxy bis(dimethylamino)methane (7.1 g, 40.7 mmol) was added to a 1,4-dioxane (80 mL) solution of **B7-2** (8.0 g, 27.1 mmol) under argon atmosphere. The reaction mixture was stirred and reacted at 75°C for 1 hour. The filtrate of the reaction mixture was concentrated to dryness under reduced pressure to obtain crude **B7-3** (6.0 g, yield: 63.2%). The crude was directly used in the next step.
LCMS: m/z 350.0 [M+H]⁺

Step 3: Hydrazine hydrate (98%, 8.4 mL) was added to an ethanol (20 mL) solution of **B7-3** (6.0 g, 17.14 mmol) under argon atmosphere. The reaction mixture was reacted at 90°C for 10 hours, slowly poured into ice water (20 mL), and extracted by adding ethyl acetate (3 * 40 mL). The organic phases were combined, washed with saturated brine (2 * 20 mL), dried over anhydrous sodium sulfate, and filtered. The filtrate was concentrated under reduced pressure and purified by silica gel chromatography (methanol/dichloromethane = 1/40 to 1/10) to obtain **B7-4** as a white powder (3.7 g, yield: 74.2%).
LCMS: m/z 290.9 [M+H]⁺

Step 4: Zinc cyanide (723.7 mg, 6.16 mmol) was added to a solution of **B7-4** (2.3 g, 7.90 mmol) in tetrahydrofuran (50 mL) and water (15 mL). After vacuuming, the mixture was replaced with argon three times. t-BuXPhos-Pd-G3(313.8 mg, 0.40 mmol) was added thereto. The reaction mixture was replaced with argon three times, reacted at 65°C for 8 hours, and slowly poured into ice water (50 mL). The reaction mixture was extracted by adding ethyl acetate (3 * 100 mL), and filtered to remove the solid. The organic phases were combined, washed with saturated brine (2 * 50 mL), dried over anhydrous sodium sulfate, and filtered. The filtrate was concentrated under reduced pressure and purified by silica gel chromatography (methanol/dichloromethane = 1/40 to 1/10) to obtain **B7-5** as a light white powder (1.36 g, yield: 72.6%).
LCMS: m/z 236.2 [M-H]⁻

Step 5: K₂CO₃ (2.3 g, 16.86 mmol) was added to a DMSO (20 mL) solution of **B7-5** (2.0 g, 8.43 mmol) under argon atmosphere. After vacuuming, the mixture was replaced with argon three times. The reaction mixture was stirred and reacted at 110°C for 2 hours, slowly poured into ice water (20 mL), and extracted by adding ethyl acetate (3 * 40 mL). The organic phases were combined, washed with saturated brine (2 * 30 mL), dried over anhydrous sodium sulfate, filtered, and the filtrate was concentrated under reduced pressure and purified by silica gel chromatography (ethyl acetate/petroleum ether = 1/20 to 1/1) to obtain **B7-6** as a light yellow powder (980 mg, yield: 53.5%).
LCMS: m/z 216.2 [M-H]⁻

Step 6: K₂CO₃ (935.4 mg, 9.02 mmol) was added to a DMF (20 mL) solution of **B7-6** (980 mg, 4.51 mmol) under argon atmosphere. Then iodomethane (1.9 g, 13.53 mmol) was added dropwise using a syringe. After vacuuming, the reaction mixture was replaced with argon three times, warmed to 30°C and reacted for 3 hours, slowly poured into ice water (20 mL), and extracted by adding ethyl acetate (3 * 40 mL). The organic phases were combined, washed with saturated brine (2 * 30 mL), dried over anhydrous sodium sulfate, and filtered. The filtrate was concentrated under reduced pressure and purified by silica gel chromatography (ethyl acetate/petroleum ether = 1/20 to 1/1) to obtain **B7-7** as a white powder (515 mg, yield: 49.4%).
LCMS: m/z 232.1 [M+H]⁺
¹H NMR (400 MHz, CDCl₃): δ ppm 7.09 (dd, *J =* 9.2, 1.9 Hz, 1H), 6.98 (t, *J =* 1.9 Hz, 1H), 6.95 (s, 1H), 3.72 (s, 3H).
¹⁹F NMR (376 Hz, CDCl₃): δ ppm -128.97.

Step 7: A solution of LDA (2.0 M, 1.79 mL, 3.58 mmol) in tetrahydrofuran and n-hexane was added dropwise to a dry tetrahydrofuran (20 mL) solution of **B7-7** (690 mg, 2.98 mmol) at - 78°C under argon atmosphere. After vacuuming, the reaction mixture was replaced with argon three times and reacted at -78°C for 1 hour. A tetrahydrofuran solution (20 mL) of iodine (757.5 mg, 2.98 mmol) was slowly added dropwise. The reaction mixture was replaced with argon three times, reacted at this temperature for another 4 hours, slowly poured into ice water (20 mL), and extracted by adding ethyl acetate (3 * 30 mL). The organic phases were combined, washed with saturated brine (2 * 20 mL), dried over anhydrous sodium sulfate, and filtered. The filtrate was concentrated under reduced pressure, and the crude was purified by Prep-HPLC to obtain intermediate **B7** as a white powder (95 mg, yield: 8.9%).

¹H NMR (400 MHz, CDCl₃): δ ppm 7.64 (s, 1H), 7.60 (d, *J* = 1.7 Hz, 1H), 3.65 (s, 3H).

¹⁹F NMR (376 Hz, CDCl₃): δ ppm -108.85.

### Synthesis of intermediate B8: 9-Fluoro-8-iodobenzo[5,6][1,4]dioxino[2,3-b]pyrazine-7-carbonitrile

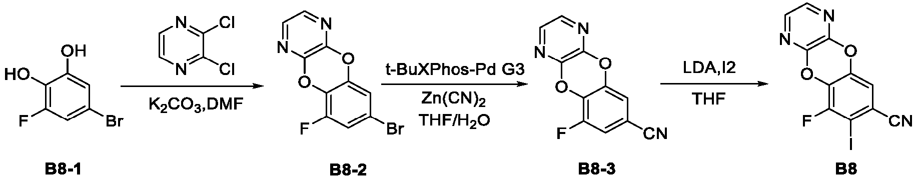

Step 1: 2,3-Dichloropyrazine (950 mg, 6.377 mmol) and potassium carbonate (2644 mg, 19.131 mmol) were added to a DMF (10 mL) solution of **B8-1** (950 mg, 4.589 mmol), and the mixture was heated to 100°C under microwave irradiation and maintained for 0.5 hours, and then the reaction was completed. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 1/10 to 5/1) to obtain **B8-2** (1000 mg, yield: 55.4%).

¹H NMR (400 MHz, CDCl₃) δ 7.88 (s, 2H), 7.05 (dd, *J* = 9.1, 2.2 Hz, 1H), 7.00 (t, *J* = 2.1 Hz, 1H).

Step 2: Zinc cyanide (788.3 mg, 6.712 mmol) and t-BuXPhos-Pd-G3 (160 mg, 0.201 mmol) were added to a solution of **B8-2** (950 mg, 3.356 mmol) in water (20 mL) and tetrahydrofuran (4 mL) under argon atmosphere. The reaction mixture was stirred at 70°C for 12 hours under nitrogen atmosphere. The reaction mixture was diluted with ethyl acetate (20 mL), washed with saturated brine (2 * 20 mL), dried over anhydrous sodium sulfate, and filtered. The filtrate was concentrated under reduced pressure and purified by silica gel chromatography (ethyl acetate/petroleum ether = 1/3) to obtain **B8-3** (730 mg, yield: 94.91%).

¹H NMR (400 MHz, CDCl₃) δ 7.95 (dd, *J =* 5.9, 2.7 Hz, 2H), 7.21 (dd, *J =* 9.0, 1.8 Hz, 1H), 7.15 (t, *J =* 1.8 Hz, 1H).

Step 3: LDA (2.0 mol/L in THF/hexane, 0.655 mL, 1.309 mmol) was added dropwise to a tetrahydrofuran (2 mL) solution of **B8-3** (100 mg, 0.436 mmol) at -65°C under argon atmosphere. After the dropwise addition was completed, the mixture was stirred at -65°C for 30 minutes. A tetrahydrofuran solution (2 mL) of iodine (166.1 mg, 0.655 mmol) was added dropwise. The reaction mixture was reacted at -65°C for 2 hours, slowly poured into ice-cold saturated ammonium chloride solution (10 mL), and extracted by adding ethyl acetate (3 * 10 mL). The organic phases were combined, washed with saturated brine (2 * 10 mL), dried over anhydrous sodium sulfate, and filtered. The filtrate was concentrated under reduced pressure and purified by silica gel chromatography (ethyl acetate/petroleum ether = 1/3) to obtain **B8** (100 mg, yield: 64.54%).

¹H NMR (400 MHz, CDCl3) δ 7.89 (dd, *J* = 7.1, 2.7 Hz, 2H), 7.11 (d, *J =* 1.9 Hz, 1H).

### Synthesis of intermediate B9: tert-Butyl 5-cyano-7-fluoro-6-iodo-2H-spiro[benzofuran-3,4'-piperidine]-1'-carboxylate

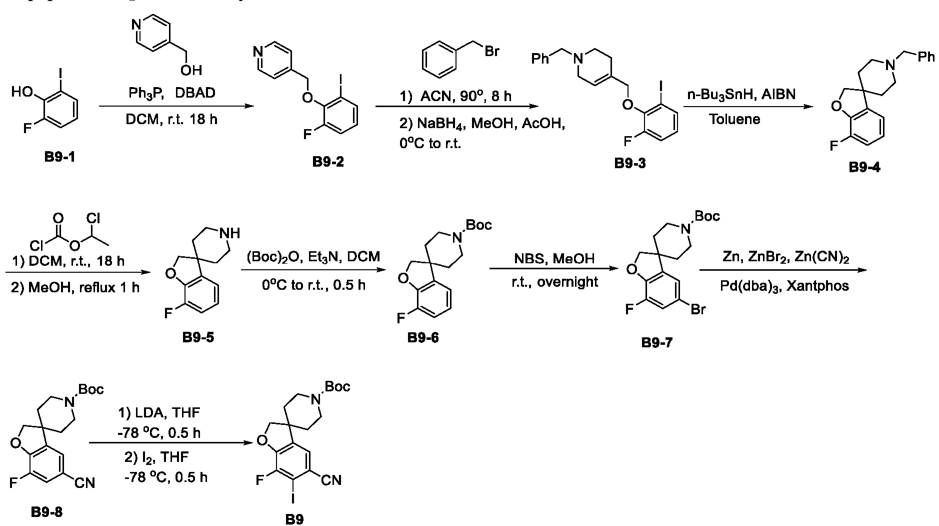

Step 1: 4-Pyridylmethanol (2.40 g, 22.00 mmol) and triphenylphosphine (5.07 g, 22.00 mmol) were added to a dichloromethane (100 mL) solution of **B9-1** (4.77 g, 20.04 mmol). A dichloromethane (50 mL) solution of di-*tert*-butyl azodicarboxylate (11.94 g, 22.00 mmol) was slowly added dropwise under nitrogen atmosphere. The reaction mixture was reacted overnight at room temperature. TLC detected that the reaction was completed, and then the reaction mixture was added with water (100 mL) and extracted with dichloromethane (2 * 100 mL). The organic phases were combined, washed with saturated brine (2 * 30 mL), and dried over anhydrous sodium sulfate. The filtrate was concentrated to dryness under reduced pressure and purified by silica gel column chromatography (ethyl acetate/petroleum ether = 1/100 to 1/5) to obtain **B9-2** as a gray oil (5.89 g, yield: 89.34%).

Step 2: Benzyl bromide (3.91 g, 22.85 mmol) was added to an acetonitrile (50 mL) solution of **B9-2** (5.79 g, 17.58 mmol). The reaction mixture was refluxed at 90°C for 8 hours. The reaction mixture was cooled to room temperature, cooled to 0°C with an ice bath, and a methanol (50 mL) solution of sodium borohydride (781 mg, 21.10 mmol) was added dropwise. After returning to room temperature, glacial acetic acid (1 mL) was added to catalyze, and the reaction mixture was reacted at room temperature for 1 hour. The reaction mixture was diluted with water (100 mL), and then extracted with ethyl acetate (3 * 100 mL). The organic phases were combined, washed with saturated brine (100 mL), dried over anhydrous sodium sulfate, and filtered. The filtrate was concentrated to dryness under reduced pressure and purified by silica gel column chromatography (ethyl acetate/petroleum ether = 1/100 to 1/5) to obtain **B9-3** as a light yellow oil (3.75 g, yield: 50%).

LCMS: m/z 423.9 [M+H]⁺.

Step 3: Tributyltin hydride (16.43 g, 22.1 mmol) was added to a toluene (300 mL) solution of **B9-3** (3.75 g, 8.84 mmol). The mixture was refluxed at 130°C for 4.5 hours. Azobisisobutyronitrile (290 mg, 1.77 mmol) was added thereto. The reaction mixture was refluxed at 130°C for 2 hours, and no raw material was detected by thin-layer chromatography. The reaction mixture was added with saturated potassium fluoride solution (150 mL) and extracted with ethyl acetate (2 * 100 mL). The organic phases were combined, washed with saturated brine (100 mL), dried over anhydrous sodium sulfate, and filtered. The filtrate was concentrated to dryness under reduced pressure to obtain **B9-4** as a colorless oil (2.50 g, yield: 95%).

LCMS: m/z 298 [M+H]⁺.

Step 4: Chloroethyl 1-chloroformate (2.59 g, 18.13 mmol) was added to a dichloromethane (30 mL) solution of **B9-4** (2.50 g, 8.40 mmol). The reaction mixture was stirred overnight at room temperature, and no reactant was detected by LCMS. The reaction mixture was added with methanol (30 mL), refluxed for 1.5 hours, concentrated to dryness under reduced pressure, and purified by silica gel column chromatography (ethyl acetate/petroleum ether = 1/100 to 1/10) to obtain **B9-5** as a white solid (1.72 g, yield: 99%).

LCMS: m/z: 208 [M+H] ⁺.

Step 5: Triethylamine (2.45 mL, 17.6 mmol) was added to a dichloromethane (50 mL) solution of **B9-5** (1.72 g, 8.30 mmol). After cooling to 0°C, di-*tert*-butyl dicarbonate (3.84 g, 17.6 mmol) was added thereto. The reaction mixture was warmed to room temperature and stirred for 0.5 hours. The reaction mixture was added with water (100 mL) and extracted with dichloromethane (2 * 30 mL). The organic phases were combined, washed with saturated brine (100 mL), dried over anhydrous sodium sulfate, and filtered. The filtrate was concentrated to dryness under reduced pressure and purified by silica gel column chromatography (ethyl acetate/petroleum ether = 1/100 to 1/10) to obtain **B9-6** as a colorless oil (2.09 g, yield: 77%).

LCMS: m/z: 308 [M+H]⁺.

Step 6: N-Bromosuccinimide (0.70 g, 3.90 mmol) was added to a methanol (50 mL) solution of **B9-6** (1.00 g, 3.25 mmol). The reaction mixture was stirred overnight at room temperature to obtain a bright yellow solution. The reaction mixture was added with saturated sodium thiosulfate solution (25 mL) and extracted with ethyl acetate (3 * 50 mL). The organic phases were combined, washed with saturated brine (50 mL), dried over anhydrous sodium sulfate, and filtered. The filtrate was concentrated to dryness under reduced pressure and purified by silica gel column chromatography (ethyl acetate/petroleum ether = 1/100 to 1/10) to obtain **B9-7** as a white solid (0.96 g, yield: 76%).

LCMS: m/z: 386 [M+H]⁺.

Step 7: Zinc powder (15.9 mg, 0.245 mmol), zinc bromide (55 mg, 0.245 mmol), zinc cyanide (205.5 mg, 1.75 mmol), tris(dibenzylideneacetone)dipalladium (56 mg, 0.06 mmol) and 4,5-bis(diphenylphosphino)-9,9-dimethylxanthene (77.5 mg, 0.134 mmol) were added to a dimethylacetamide (8 mL) solution of **B9-7** (0.95 g, 2.45 mmol). The reaction mixture was replaced with nitrogen three times and then stirred at 100°C for 16 hours. The reaction mixture was added with water (20 mL) and extracted with ethyl acetate (3 * 20 mL). The organic phases were combined, washed with saturated brine (30 mL), dried over anhydrous sodium sulfate, and filtered. The filtrate was concentrated to dryness under reduced pressure and purified by silica gel column chromatography (ethyl acetate/petroleum ether = 1/100 to 1/10) to obtain **B9-8** as a white solid (0.79 g, yield: 95%).

LCMS: m/z: 333 [M+H]⁺.

¹H NMR (400 MHz, Chloroform-d) δ 7.29 - 7.26 (m, 1H), 7.19 (d, J = 1.5 Hz, 1H), 4.61 (s, 2H), 4.19 - 4.04 (m, 2H), 2.86 (t, J = 13.0 Hz, 2H), 1.90 - 1.72 (m, 4H), 1.48 (s, 9H).

Step 8: After cooling a tetrahydrofuran (20 mL) solution of **B9-8** (0.70 g, 2.10 mmol) to -78°C, lithium diisopropylamide (1.60 mL, 3.16 mmol) was added thereto, and stirred for 30 minutes to obtain a light yellow solution. The temperature was maintained at -78°C, and a tetrahydrofuran (10 mL) solution of iodine (3.84 g, 17.6 mmol) was added dropwise, then the mixture first faded and then gradually turned red, stirred for 30 minutes. The reaction mixture was warmed to room temperature, added with saturated sodium thiosulfate solution (20 mL), and extracted with ethyl acetate (3 * 30 mL). The organic phases were combined, washed with saturated brine (50 mL), dried over anhydrous sodium sulfate, and filtered. The filtrate was concentrated to dryness under reduced pressure and purified by silica gel column chromatography (ethyl acetate/petroleum ether = 1/100 to 1/20) to obtain white intermediate **B9** (0.876 g, yield: 91%).

¹H NMR (400 MHz, Chloroform-d) δ 7.19 (d, J = 0.9 Hz, 1H), 4.62 (s, 2H), 4.12 (d, J = 7.1 Hz, 2H), 2.86 (t, J = 12.6 Hz, 2H), 1.79 (dt, J = 21.2, 9.0 Hz, 4H), 1.48 (s, 9H).

### Synthesis of intermediate B53: Synthesis of 6-fluoro-7-iodo-3-methyl-3,4-dihydrobenzofuro[3,4-c]pyrazole-8-carbonitrile

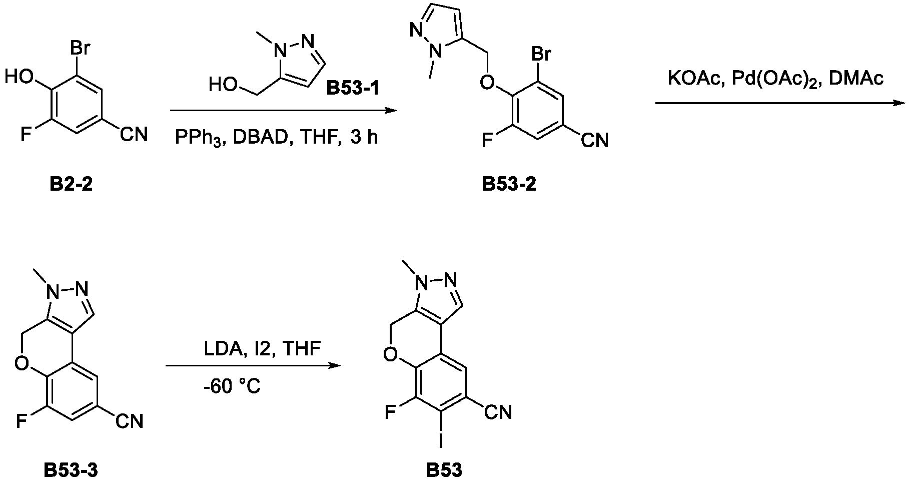

Step 1: Extra dry tetrahydrofuran (30 mL), **B2-2** (2.5 g, 11.57 mmol) and **B53-1** (1.56 g, 13.89 mmol) were sequentially added to a dry three-neck flask. Under nitrogen atmosphere, triphenylphosphine (2.97 g, 11.34 mmol) and DBAD (3.38 g, 11.34 mmol) were added thereto. The reaction mixture was stirred at 25°C for 3 hours. After the reaction was completed, the reaction mixture was diluted by adding water (100 mL) and extracted with ethyl acetate (40 mL * 2). The combined organic phases were washed with saturated brine (100 mL), dried over anhydrous sodium sulfate, filtered, and concentrated under reduced pressure. The resulting residue was purified by silica gel chromatography (0 to 15% ethyl acetate/petroleum ether) to obtain crude **B53-2** (2.0 g, yield: 55.7%) as a white solid.

Step 2: Extra dry DMAc (16 mL), **B53-2** (2.0 g, 6.45 mmol), palladium acetate (2.0 g, 6.45 mmol) and potassium acetate (2.0 g, 6.45 mmol) were sequentially added to a dry single-neck flask, and the reaction mixture was stirred at 120°C for 15 hours under nitrogen atmosphere. After the reaction was completed, the reaction mixture was diluted by adding water (30 mL) and extracted with ethyl acetate (60 mL * 2). The combined organic phases were washed with saturated brine (100 mL), dried over anhydrous sodium sulfate, filtered, and concentrated under reduced pressure. The resulting solid was slurried with petroleum ether and ethyl acetate (10/1) to obtain **B53-3** as a brown solid (400 mg, yield: 27.1%).

¹H NMR (400 MHz, CDCl₃): δ ppm 7.94 (s, 1H), 7.86 (s, 1H), 7.67 (dd, *J =* 10.8, 1.6 Hz, 1H), 5.69 (s, 2H), 3.79 (s, 3H).

LC-MS: m/z 230.0 [M +H]⁺.

Step 3: **B53-3** (230 mg, 1.00 mmol) and iodine (1 capsule, about 40 mg) were dissolved in dry tetrahydrofuran (120 mL). After three replacements with argon, the mixture was cooled to -65°C with a dry ice acetone bath. LDA was added dropwise until the color of the iodine faded (became colorless), then LDA (1 M in THF, 2.0 mL, 2.00 mmol) was added dropwise. After the addition was completed, the reaction mixture was reacted at -65°C for 30 minutes, poured into ice water, concentrated to remove most of the tetrahydrofuran, and extracted with dichloromethane: methanol (10:1, 3 * 500 mL). The organic phases were combined, washed with saturated brine, dried, filtered, dried, concentrated, and the residue was subjected to column chromatography (dichloromethane: methanol = 1:0 to 500: 1) to obtain intermediate **B53** as a light yellow solid (120 mg, yield: 33.7%).

¹H NMR (400 MHz, CDCl₃): δ ppm 7.71 (s, 1H), 7.41 (d, *J* = 1.2 Hz, 1H), 5.55 (s, 2H), 3.85 (s, 3H).

LC-MS: m/z 356.1 [M+H]⁺.

### Synthesis of intermediate B54: 6-Fluoro-7-iodo-2-((2-(trimethylsilyl)ethoxy)methyl)-2,4-dihydrochromeno[3,4-c]pyrazole-8-carbonitrile

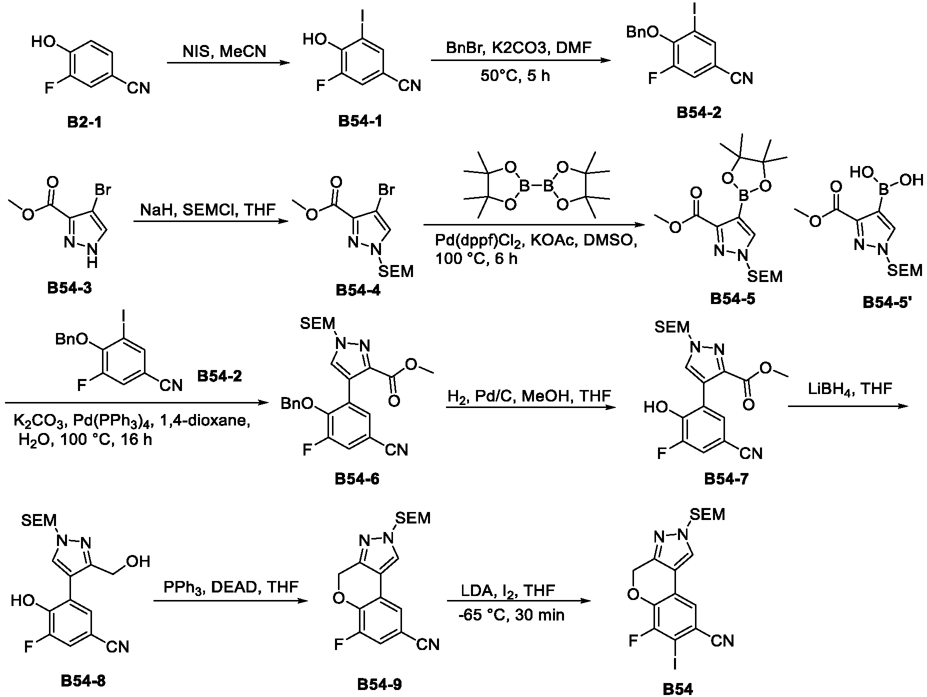

Step 1: NIS (90.2 g, 400.9 mmol) was added to an acetonitrile (600 mL) solution of B2-1 (50.0 g, 364.7 mmol) under an ice water bath. The reaction mixture was stirred at room temperature overnight. The reaction mixture was concentrated under reduced pressure to remove nearly half of the solvent, poured into saturated sodium sulfite aqueous solution (1200 mL), and extracted with ethyl acetate (3 * 800 mL). The extracts were combined, washed with saturated brine, dried over anhydrous sodium sulfate, filtered, concentrated and purified by silica gel chromatography (petroleum ether: ethyl acetate = 25:1) to obtain **B54-1** as a light yellow solid (85.5 g, yield: 89.2%).

¹H NMR (400 MHz, CDCl₃): δ ppm 7.81 (t, *J =* 1.6 Hz, 1H), 7.41 (dd, *J =* 9.2, 1.6 Hz, 1H), 6.17 (br, 1H).

Step 2: Benzyl bromide (14.3 g, 83.65 mmol) was added dropwise to a DMF (200 mL) solution of **B54-1** (20.0 g, 76.04 mmol) and potassium carbonate (21.0 g, 152.09 mmol). The reaction mixture was heated to 50°C, stirred and reacted for 5 hours, and then cooled to room temperature. The reaction mixture was diluted by adding ethyl acetate (500 mL), washed with saturated brine (3 * 2.0 L), dried over anhydrous sodium sulfate, and filtered. The filtrate was concentrated to dryness under reduced pressure and purified by silica gel chromatography (petroleum ether: ethyl acetate = 30: 1) to obtain **B54-2** as a white solid (25.0 g, yield: 93.1%).

¹H NMR (400 MHz, DMSO-*d*₆): δ ppm 8.18 (t, *J =* 1.6 Hz, 1H), 8.01 (dd, *J =* 11.6, 2.0 Hz, 1H), 7.53 - 7.51 (m, 2H), 7.44 - 7.35 (m, 3H), 5.27 (d, *J =* 1.2 Hz, 2H).

Step 3: Sodium hydride (60% dispersed in oil, 2.93 g, 73.38 mmol) was added to a dry tetrahydrofuran (120 mL) solution of **B54-3** (10.03 g, 49.65 mmol) in batches at 0°C under argon atmosphere. After the addition was completed, the mixture was stirred for another 30 minutes. 2-(Trimethylsilyl)ethoxymethyl chloride (9.80 g, 58.78 mmol) was added dropwise. The reaction mixture was stirred and reacted at room temperature for 2 hours. The reaction was quenched by adding ice water. The reaction mixture was extracted with ethyl acetate. The organic phases were combined, washed with saturated brine, dried over anhydrous sodium sulfate, and filtered. The filtrate was concentrated under reduced pressure and purified by silica gel chromatography (petroleum ether: ethyl acetate = 10:1) to obtain **B54-4** as a colorless oil (10.28 g, yield: 61.7%).
¹H NMR (400 MHz, CDCl₃): δ ppm 7.69 (s, 1H), 5.45 (s, 2H), 3.93 (s, 3H), 3.57 - 3.53 (m, 2H), 0.91 - 0.87 (m, 2H), -0.04 (s, 9H).
LCMS: m/z 337.0 [M+H]⁺

Step 4: Pd(dppf)Cl₂ (1.5 g, 1.98 mmol) was added to a DMSO (150 mL) solution of**B54-4** (13.3 g, 39.67 mmol), bis(pinacolato)diboron (50.4 g, 198.47 mmol) and potassium acetate (7.8 g, 79.47 mmol) under argon atmosphere. The reaction mixture was stirred and reacted at 100°C for 6 hours. The reaction mixture was poured into water, filtered and the filter cake was purified by silica gel chromatography (0 to 10% gradient of ethyl acetate: petroleum ether) to obtain a light yellow oil (10.1 g, **B54-5/B54-5'** = 1/2).

Step 5: Tetrakis(triphenylphosphine)palladium (3.5 g, 3.03 mmol) was added to a suspension of a mixture of **B54-5** and **B54-5'** (9.1 g), **B54-2** (10.7 g, 30.3 mmol) and potassium carbonate (8.4 g, 60.6 mmol) in 1,4-dioxane (250 mL) and water (30 mL). After vacuuming, the reaction mixture was replaced with argon three times, heated to 100°C and reacted for 16 hours. The reaction mixture was concentrated, added with ethyl acetate (500 mL) and water (500 mL). The phases were separated, and the aqueous phase was extracted with ethyl acetate (300 mL). The organic phases were combined, concentrated, and the residue was purified by silica gel chromatography (ethyl acetate/petroleum ether = 1/3) to obtain **B54-6** as a light yellow solid (7.0 g, two-step yield: 36.6%).
¹H NMR (400 MHz, CDCl₃): δ ppm 7.53 (s, 1H), 7.45 - 7.41 (m, 2H), 7.29 - 7.24 (m, 3H), 7.12 - 7.09 (m, 2H), 5.49 (s, 2H), 5.09 (m, 2H), 3.80 (s, 3H), 3.62 (t, *J =* 8.0 Hz, 2H), 0.94 (t, *J =* 8.4 Hz, 2H), 0.00 (s, 9H).
LCMS: m/z 482.6 [M+H]⁺

Step 6: Palladium on carbon (10%, 1.0 g) was added to a solution of **B54-6** (2.0 g, 4.15 mmol) in methanol (100 mL) and tetrahydrofuran (15 mL). The reaction mixture was reacted overnight at 60°C under 3 MP hydrogen pressure. The reaction mixture was filtered and concentrated. The residue was purified by silica gel chromatography to obtain **B54-7** as a white solid (700 mg, yield: 43.1%).
¹H NMR (400 MHz, DMSO-*d*₆): δ ppm 10.98 (brs, 1H), 8.20 (s, 1H), 7.80 (dd, *J =* 10.8, 1.6 Hz, 1H), 7.55 (s, 1H), 5.52 (s, 2H), 3.72 (s, 3H), 3.64 (t, *J =* 8.0 Hz, 2H), 0.89 (t, *J =* 8.0 Hz, 2H), 0.00 (s, 9H).
LCMS: m/z 392.2 [M+H]⁺

Step 7: A lithium borohydride suspension (2.0 mol/L in THF, 4.2 mL, 8.4 mmol) was added dropwise to an anhydrous tetrahydrofuran (30 mL) solution of **B54-7** (680 mg, 1.74 mmol) at 0°C under argon atmosphere. After the addition was completed, the reaction mixture was slowly warmed to room temperature and reacted for 16 hours. The reaction mixture was added with cold water (200 mL), concentrated to remove most of the tetrahydrofuran, and extracted with dichloromethane (2 * 150 mL). The organic phases were combined, washed with saturated brine, dried over anhydrous sodium sulfate, filtered and concentrated. The residue was purified by silica gel chromatography to obtain **B54-8** as an off-white solid (550 mg, yield: 87.1%).
¹H NMR (400 MHz, DMSO-*d*₆): δ ppm 8.19 (s, 1H), 7.92 (s, 1H), 7.72 (dd, *J =* 10.4, 1.6 Hz, 1H), 5.44 (s, 2H), 4.50 (s, 2H), 3.61 (t, *J =* 7.6 Hz, 2H), 0.88 (t, *J =* 8.0 Hz, 2H), 0.00 (s, 9H).
LCMS: m/z 364.5 [M+H]⁺

Step 8: Diisopropyl azodicarboxylate (400 mg, 1.98 mmol) was added dropwise to a tetrahydrofuran (100 mL) solution of **B54-8** (480 mg, 1.32 mmol) and triphenylphosphine (520 mg, 1.98 mmol) at 0°C under argon atmosphere. After the addition was completed, the reaction mixture was warmed to room temperature and reacted for 2 hours. The reaction mixture was slowly poured into ice water (100 mL), concentrated to remove most of the tetrahydrofuran, and extracted with dichloromethane/methanol (10/1, 3 * 80 mL). The organic phases were combined, washed with saturated brine (100 mL), dried over anhydrous sodium sulfate, concentrated, and the residue was purified by silica gel chromatography to obtain **B54-9** as a white solid (400 mg, yield: 87.7%).

¹H NMR (400 MHz, DMSO-*d*₆): δ ppm 8.40 (s, 1H), 7.90 (d, *J =* 1.2 Hz, 1H), 7.71 (dd, *J =* 10.8, 1.6 Hz, 1H), 5.48 (s, 2H), 5.45 (s, 2H), 3.57 (t, *J =* 8.0 Hz, 2H), 0.88 (t, *J =* 8.0 Hz, 2H), -0.04 (s, 9H).

Step 9: **B54-9** (380 mg, 1.10 mmol) and iodine (1 capsule, about 40 mg) were dissolved in dry tetrahydrofuran (60 mL) at -78°C under argon atmosphere. After three replacements with argon, the mixture was cooled to -65°C with a dry ice acetone bath, and LDA (2.0 mol/L in THF/hexane) was added dropwise until the color of the iodine faded (became colorless), then LDA (2.0 mol/L in THF/hexane, 1.1 mL, 2.20 mmol) was added dropwise. After the addition was completed, the mixture was reacted at -65°C for 1 hour. A dry tetrahydrofuran solution (20 mL) of iodine (293 mg, 1.16 mmol) was slowly added dropwise (over about 30 min). After the addition was completed, the reaction mixture was reacted at -65°C for 30 minutes. The reaction mixture was poured into ice water, concentrated to remove most of the tetrahydrofuran, and extracted with dichloromethane: methanol (10:1, 3 * 500 mL). The organic phases were combined, washed with saturated brine, dried over anhydrous sodium sulfate, filtered, concentrated, and the residue was purified by column chromatography (dichloromethane: methanol = 1:0 to 500: 1) to obtain **B54** as an off-white solid (340 mg, yield: 65.6%).
¹H NMR (400 MHz, DMSO-*d*₆): δ ppm 8.44 (s, 1H), 7.99 (d, *J =* 1.2 Hz, 1H), 5.54 (s, 2H), 5.48 (s, 2H), 3.60 (t, *J =* 8.0 Hz, 2H), 0.89 (t, *J =* 8.0 Hz, 2H), 0.00 (s, 9H).
LCMS: m/z: 472.0 [M+H]⁺

### Synthesis of intermediate B55: 6-Fluoro-7-iodo-4H-benzo[b][1,2,4]triazolo[4,3-d][1,4]oxazine-8-carbonitrile

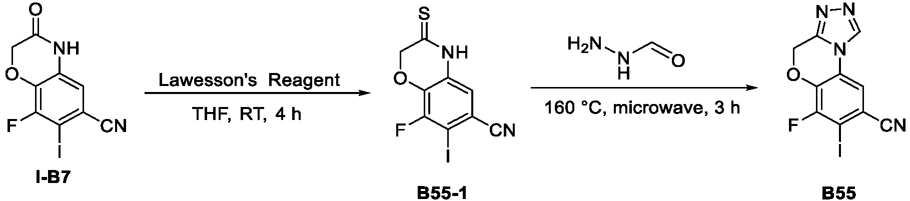

Step 1: A tetrahydrofuran (20 mL) solution of Lawesson's reagent (316.75 mg, 282.90 µmol) was slowly added dropwise to a tetrahydrofuran (20 mL) solution of **I-B7** (300 mg, 0.94 mmol) under argon atmosphere. After vacuuming, the reaction mixture was replaced with argon three times. The reaction mixture was stirred and reacted at room temperature for 4 hours, slowly poured into ice water (10 mL) and extracted by adding ethyl acetate (3 * 20 mL). The organic phases were combined, washed with saturated brine (2 * 20 mL), dried over anhydrous sodium sulfate, filtered, and the filtrate was concentrated under reduced pressure and purified by silica gel chromatography (ethyl acetate/petroleum ether = 1/20 to 1/1) to obtain **B55-1** as a light yellow powder (211 mg, yield: 67.1%).
¹H NMR (400 MHz, DMSO-*d*₆): δ ppm 13.12 (s, 1H), 7.28 (d, *J =* 1.6 Hz, 1H), 5.08 (s, 2H).
LCMS: m/z: 334.9 [M+H]⁺

Step 2: A mixture of intermediate **B55-1** (300 mg, 0.90 mmol) and formylhydrazine (64.7 mg, 1.08 mmol) was reacted under microwave irradiation at 160°C for 3 hours. After the reaction was completed, the reaction mixture was slowly poured into ice water (10 mL) and extracted by adding ethyl acetate (3 * 20 mL). The organic phases were combined, washed with saturated brine (2 * 20 mL), dried over anhydrous sodium sulfate, and filtered. The filtrate was concentrated under reduced pressure and purified by silica gel chromatography (methanol/dichloromethane = 1/50 to 1/15) to obtain **B55** as a white powder (200 mg, yield: 65.1%).

LCMS: m/z: 342.9 [M+H]⁺.

### Synthesis of intermediate B56: 6-Fluoro-7-iodo-1-methyl-4H-benzo[b] [1,2,4]triazolo[4,3 -d] [1,4]oxazine-8-carbonitrile

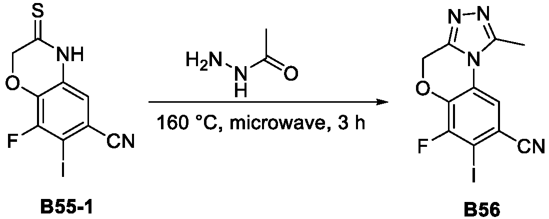

Step 1: A mixture of intermediate **B55-1** (400 mg, 1.20 mmol) and acethydrazide (106.43 mg, 1.44 mmol) was reacted under microwave irradiation at 160°C for 3 hours. After the reaction was completed, the reaction mixture was slowly poured into ice water (10 mL) and extracted by adding ethyl acetate (3 * 20 mL). The organic phases were combined, washed with saturated brine (2 * 20 mL), dried over anhydrous sodium sulfate, and filtered. The filtrate was concentrated under reduced pressure and purified by silica gel chromatography (methanol/dichloromethane = 1/50 to 1/15) to obtain white intermediate **B56** (198 mg, yield: 46.4%).

LCMS: m/z: 356.9 [M+H]⁺.

### Synthesis of intermediate B70: 9-Fluoro-8-iodo-5-((tetrahydro-2H-pyran-2-yl)oxy)-5H-chromeno[2,3-b]pyridine-7-carbonitrile

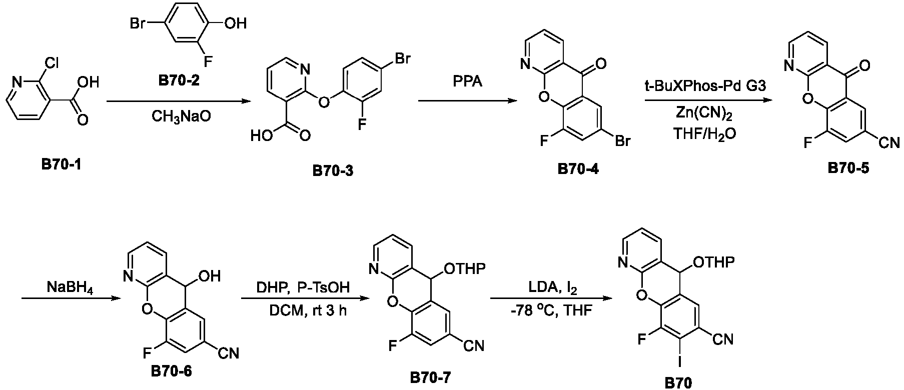

Step 1: **B70-2** (3031 mg, 15.87 mmol) was added to a sodium methoxide solution (3 mL, 30 wt% in methanol) of **B70-1** (500 mg, 3.17 mmol). The reaction mixture was reacted under a low vacuum oil pump at 50°C for 2 hours, cooled to room temperature and then heated to 168°C and reacted for 3 hours in an open environment. After cooling to room temperature, the reaction mixture was dissolved with dilute hydrochloric acid (10 mL), extracted by adding ethyl acetate (30 mL * 3), washed with saturated brine (10 mL), dried over anhydrous sodium sulfate, and filtered. The filtrate was concentrated to dryness under reduced pressure and purified by silica gel column chromatography (ethyl acetate/petroleum ether = 1/100 to 1/1) to obtain **B70-3** as a light yellow solid (950 mg, yield: 96%).

LCMS: m/z 314 [M+H]⁺.

Step 2: **B70-3** (950 mg, 3.044 mmol) was added to polyphosphoric acid (10 mL), heated to 160°C and reacted for 6 hours. The reaction mixture was slowly poured into ice water (50 mL). The pH was adjusted to 10 with 10 N sodium hydroxide solution. The mixture was extracted with ethyl acetate (3 × 50 mL), washed with saturated brine (100 mL), dried over anhydrous sodium sulfate, concentrated, and purified by silica gel column chromatography (ethyl acetate/petroleum ether = 1/40 to 1/2) to obtain **B70-4** as a light yellow solid (800 mg, yield: 89.4 %).

LCMS: m/z 294 [M+H]⁺.

Step 3: Zn(CN)₂ (639 mg, 5.44 mmol) was added to a solution of **B70-4** (800 mg, 2.72 mmol) in tetrahydrofuran (4 mL) and water (20 mL). After vacuuming, the mixture was replaced with argon three times. t-BuXPhos-Pd-G3 (794.4 mg, 0.163 mmol) was added thereto. After vacuuming, the reaction mixture was replaced with argon three times. The reaction mixture was reacted at 45°C for 15 hours. The reaction mixture was slowly poured into ice water (50 mL), extracted by adding ethyl acetate (3 × 50 mL), filtered with diatomite to remove the insoluble substances and the filter residue was rinsed with ethyl acetate (50 mL). The organic phases were combined, washed with saturated brine (2 × 100 mL), dried over anhydrous sodium sulfate, concentrated and purified by silica gel column chromatography (ethyl acetate/petroleum ether = 1/40 to 1/2) to obtain **B70-5** as a light yellow solid (500 mg, yield: 76.5%).

¹H NMR (400 MHz, CDCl₃) δ 8.84 (dd, *J =* 4.5, 1.9 Hz, 1H), 8.74 (dd, *J* = 7.8, 2.0 Hz, 1H), 8.43 (s, 1H), 7.80 (dd, *J =* 9.2, 1.9 Hz, 1H), 7.57 (dd, *J =* 7.7, 4.6 Hz, 1H).

Step 4: THF (5 mL), MeOH (10 mL), **B70-5** (300 mg, 1.25 mmol) and NaBH₄ (142 mg, 3.75 mmol) were sequentially added to a dry 50 mL single-neck flask. The reaction mixture was stirred at 20°C for 1 hour. TLC showed that the reaction was completed. The reaction mixture was quenched by pouring into saturated NH₄Cl aqueous solution at 0°C and extracted with ethyl acetate, then the combined organic phases were washed with saturated brine, dried over anhydrous sodium sulfate, filtered, and concentrated under reduced pressure to obtain **B70-6** as a light yellow solid (300 mg, crude), which was used directly in the next step.

LCMS: m/z 243 [M+H]⁺.

Step 5: DCM (20 mL), **B70-6** (290 mg, 1.20 mmol) and *p*-toluenesulfonic acid (21 mg, 0.125 mmol) were sequentially added to a dry 50 mL single-neck flask. The reaction mixture was stirred at 20°C for 3 hours. TLC showed that the reaction was completed. The reaction mixture was poured into saturated sodium bicarbonate aqueous solution and extracted with ethyl acetate. The combined organic phases were washed with saturated brine, dried over anhydrous sodium sulfate, filtered, concentrated under reduced pressure and purified by silica gel column chromatography (ethyl acetate/petroleum ether = 1/10 to 1/2) to obtain **B70-7** as a colorless oil (215 mg, yield: 56%).

LCMS: m/z 326.9 [M+H]⁺.

Step 6: A solution of LDA (2.0 M, 0.975 mL, 1.95 mmol) in tetrahydrofuran and n-hexane was added dropwise to a dry tetrahydrofuran (5 mL) solution of **B70-7** (215 mg, 0.65 mmol) at - 78°C under argon atmosphere. After vacuuming, the mixture was replaced with argon three times. The mixture was reacted at -78°C for 1 hour. A tetrahydrofuran solution (2 mL) of iodine (214.5 mg, 0.845 mmol) was slowly added dropwise. The reaction mixture was replaced with argon three times, reacted at this temperature for another 0.5 hours, slowly poured into ice water (20 mL), and extracted by adding ethyl acetate (3 * 30 mL). The organic phases were combined, washed with saturated brine (2 * 20 mL), dried over anhydrous sodium sulfate, and filtered. The filtrate was concentrated under reduced pressure, and the crude was purified by Prep-HPLC to obtain intermediate **B70** as a white powder (66 mg, yield: 22.5%).

LCMS: m/z 453 [M+H]⁺.

### Synthesis of intermediate I-B1: 8-Fluoro-7-iodo-6-nitrilo-imidazo[1,2-a]pyridin

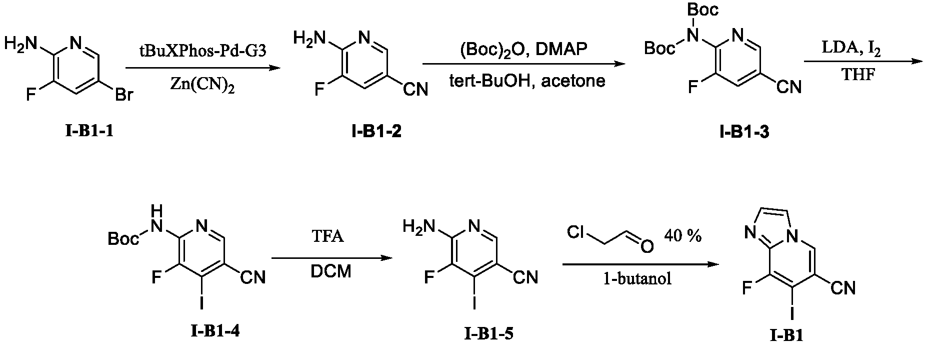

Step 1: **I-B1-1** (74.50 g, 390.05 mmol) was dissolved in tetrahydrofuran (260 mL) and water (1000 mL), then Zn(CN)₂(30.22 g, 257.45 mmol) was added thereto. After vacuuming, the mixture was replaced with argon three times. t-BuXPhos-Pd-G3 (6.80 g, 8.56 mmol) was added thereto. After vacuuming, the reaction mixture was replaced with argon three times and reacted at 40°C for 15 hours. The reaction mixture was slowly poured into ice water (1.0 L), extracted by adding ethyl acetate (3 × 400 mL), filtered with diatomite to remove the insoluble substances and the filter residue was rinsed with tetrahydrofuran (300 mL). The organic phases were combined, washed with saturated brine (2 × 500 mL), dried over anhydrous sodium sulfate, concentrated and purified by silica gel column chromatography (ethyl acetate/petroleum ether = 1/20 to 1/1) to obtain **I-B1-2** as a light yellow powder (28.53 g, yield: 53.3%).

¹H NMR(400 MHz, DMSO-*d*₆): δ ppm 8.21 (d, *J =* 1.2 Hz, 1H), 7.85 (dd, *J =* 11.2, 1.6 Hz, 1H), 7.36 (s, 2H).

¹⁹F NMR (376 MHz, DMSO-*d*₆): δ ppm -137.53.

LCMS: m/z 138.2 [M+H]⁺.

Step 2: **I-B1-2** (28.53 g, 208.10 mmol) was dissolved in acetone (290 mL) and *tert-*butanol (145 mL), and DMAP (82.84 g, 677.9 mmol) was added thereto. (Boc)₂O (200.39 g, 919.26 mmol) was slowly added thereto, and the reaction mixture was reacted at 60°C for 12 hours. The reaction mixture was concentrated to nearly dryness, loaded onto the column using the wet method, and purified by silica gel column chromatography (ethyl acetate/petroleum ether = 1/100 to 1/15) to obtain **I-B1-3** as a white solid (58.32 g, yield: 83.1%).

¹H NMR (400 MHz, CDCl₃): δ ppm 8.60 (d, *J* = 2.0 Hz, 1H), 7.75 (dd, *J* = 8.4, 2.0 Hz, 1H), 1.44 (s, 18H).

¹⁹F NMR (376 MHz, CDCl₃): δ ppm -121.23.

LCMS: m/z 360.0 [M+Na]⁺.

Step 3: **I-B1-3** (58.32 g, 172.85 mmol) was dissolved in tetrahydrofuran (630 mL), and iodine (87.72 g, 345.64 mmol) was added thereto, and then the mixture was cooled to -65°C under nitrogen atmosphere. LDA (2.0 M in THF, 440 mL, 880.00 mmol) was added dropwise. After the dropwise addition was completed, the reaction mixture was stirred for 10 minutes, then warmed to room temperature and reacted for 16 hours. The reaction mixture was slowly poured into ice water (1.0 L) and extracted by adding ethyl acetate (3 × 400 mL). The organic phase was washed with saturated brine (2 × 500 mL), dried, concentrated, and purified by silica gel column chromatography (ethyl acetate/petroleum ether = 0/1 to 1/10), and then slurried with dichloromethane/petroleum ether (1/50) to obtain **I-B1-4** as a light yellow powder (23.27 g, yield: 37.1%).

¹H NMR (400 MHz, CDCl₃) δ ppm: 8.35 (s, 1H), 7.21 (s, 1H), 1.55 (s, 9H).

¹⁹F NMR (400 MHz, CDCl₃) δ ppm: -110.85.

LCMS: m/z 362.1 [M-H]⁻.

Step 4: TFA (130 mL) was added dropwise to a dichloromethane (360 mL) solution of**I-B1-4** (32.06 g, 88.29 mmol) at 0°C. The reaction mixture was reacted at room temperature for 2 hours, and concentrated to remove most of the solvent and TFA. The reaction mixture was diluted with ethyl acetate (60 mL) and poured into saturated sodium carbonate aqueous solution (500 mL). The aqueous phase was extracted with ethyl acetate (3 × 200 mL), and the organic phase was washed with saturated brine (100 mL), dried, and concentrated to obtain **I-B1-5** as an off-white solid (19.08 g, yield: 82.2%).

¹H NMR (400 MHz, DMSO-*d*₆) δ ppm 8.10 (s, 1H), 7.53 (s, 2H).

¹⁹F NMR (400 MHz, DMSO-*d*₆) δ ppm: -118.25.

LCMS: m/z: 264.0 [M+H]⁺.

Step 5: 40 wt.% chloroacetaldehyde aqueous solution (290 mL) was added to a solution of **I-B1-5** (14.29 g, 54.35 mmol) dissolved in n-butanol (290 mL). The reaction mixture was heated to 100°C and reacted for 2 hours. The reaction mixture was cooled to room temperature to precipitate a large number of crystals, poured into ice water (800 mL), filtered, and the filter cake was washed with water to obtain crude 1. The filtrate was extracted with ethyl acetate (3 × 200 mL). The organic phase was washed with saturated brine (2 × 100 mL), dried, and concentrated to obtain an oily crude product, which was crystallized by adding *n*-hexane (300 mL) and filtered to obtain crude 2. Crude 1 and crude 2 were combined and slurried with *n*-hexane/ethyl acetate (300 mL/20 mL) to obtain **I-B1** as a yellow powder (12.32 g, yield: 79.0%).

¹H NMR (400 MHz, DMSO-*d*₆) δ ppm: 9.28 (s, 1H), 8.18 (dd, *J* = 3.2, 1.6 Hz, 1H), 7.76 (d, *J =* 1.2 Hz, 1H).

¹⁹F NMR (376 MHz, DMSO-*d*₆) δ ppm: -107.29.

LCMS: m/z: 288.0 [M+H]⁺.

### Synthesis of intermediate I-B2: 5-Bromo-4-fluoro-1-tosyl-6-cyano-indole

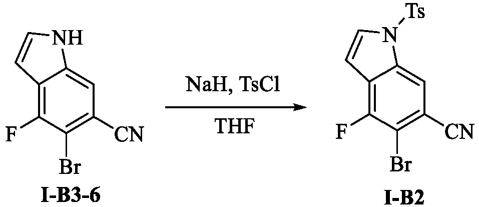

Step 1: **I-B3-6** (30 mg, 0.125 mmol) and THF (1 mL) were sequentially added to a 25 mL round-bottom flask under an ice bath. Then NaH (60%) (7.5 mg, 0.188 mmol) was added thereto, and after stirring for 1 hour, TsCl (28.6 mg, 0.15 mmol) was added thereto. The reaction mixture was stirred at room temperature for 12 hours. TLC showed that the reaction was completed, then the reaction mixture was diluted by adding water (10 mL) and extracted with ethyl acetate (10 mL * 3). The combined organic phases were washed with saturated sodium bicarbonate aqueous solution (30 mL) and saturated brine (30 mL), dried over anhydrous sodium sulfate, filtered, and concentrated under reduced pressure. The resulting residue was purified by silica gel chromatography (0 to 30% ethyl acetate/petroleum ether) to obtain **I-B2** as a white solid (38 mg, yield: 77.2%).

¹H NMR (400 MHz, CDCl₃) δ 8.16 (s, 1H), 7.79 - 7.74 (m, 3H), 7.33 (d, *J* = 8.4 Hz, 2H), 6.81 (d, *J* = 3.6 Hz, 1H), 2.40 (s, 3H).

¹⁹F-NMR (376 MHz, CDCl₃): -108.89.

LC-MS: m/z 393.1 [M-H]⁻.

### Synthesis of intermediate I-B3: 5-Bromo-4-fluoro-1-methyl-1H-indole-6-carbonitrile

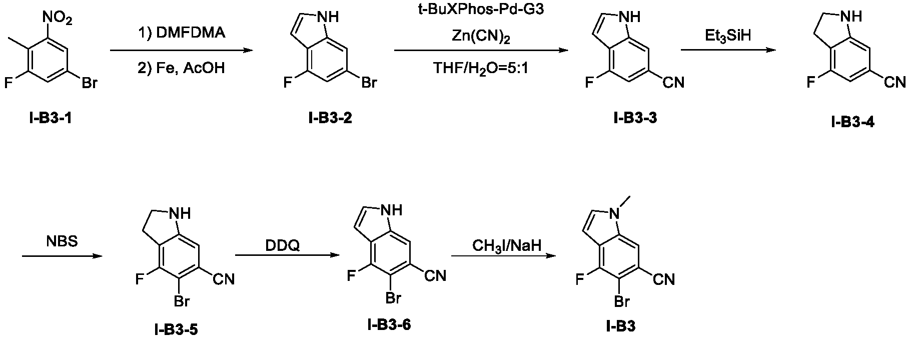

Step 1: **I-B3-1** (6.5 g, 27.8 mmol), DMF-DMA (13 mL, 61.1 mmol), triethylamine (4.25 mL, 61.1 mmol) and DMF (25 mL) were sequentially added to a 500 mL round-bottom flask. Then the reaction mixture was heated to 110°C and stirred for 3 hours. TLC showed that the reaction was completed, then the reaction mixture was diluted by adding water (50 mL) and extracted with ethyl acetate (50 mL * 3). The combined organic phases were washed with saturated sodium bicarbonate aqueous solution (150 mL), saturated sodium thiosulfate aqueous solution (150 mL) and saturated brine (150 mL), dried over anhydrous sodium sulfate, filtered, and concentrated under reduced pressure. The resulting residue was dissolved in acetic acid/toluene (100 mL/150 mL), and reduced iron powder (31 g, 555 mmol) was added thereto. Then the reaction mixture was heated to 100°C and stirred for 1 hour. The reaction mixture was cooled, filtered, poured into ice water, and extracted with ethyl acetate (150 mL * 3). The combined organic phases were washed with saturated brine (300 mL), dried over anhydrous sodium sulfate, filtered, and concentrated under reduced pressure. The resulting residue was purified by silica gel chromatography (0 to 40% ethyl acetate/petroleum ether) to obtain **I-B3-2** as a yellow solid (3.7 g, yield: 60%).

¹H NMR (400 MHz, CDCl₃) δ 8.27 (brs, 1H), 7.34 (t, *J =* 1.2 Hz, 1H), 7.16 - 7.14 (m, 1H), 6.96 (dd, *J =* 9.6, 1.2 Hz, 1H), 6.61 - 6.59 (m, 1H).

¹⁹F NMR (376 MHz, CDCl₃) δ -119.68.

Step 2: Under nitrogen atmosphere, intermediate **I-B3-2** (3.7 g, 17.3 mmol), zinc cyanide (1.34 g, 11.42 mmol) and t-BuXPhos-Pd-G3 (686 mg, 0.86 mmol) were sequentially added to a dry three-neck flask, and then THF/H₂O (10 mL/50 mL) was added thereto. The reaction mixture was stirred at 40°C for 12 hours. After the reaction was completed, the reaction mixture was concentrated under reduced pressure, and the resulting residue was purified by silica gel chromatography (0 to 40% ethyl acetate/petroleum ether) to obtain **I-B3-3** as a yellow solid (2.85 g, yield: 93%).

¹H NMR (400 MHz, CDCl₃) δ 8.76 (brs, 1H), 7.59 (s, 1H), 7.41(t, *J =* 1.6 Hz, 1H), 7.05 (dd, *J =* 9.6, 0.8 Hz, 1H), 6.73(t, *J =* 2.0 Hz, 1H).

¹⁹F NMR (376 MHz, CDCl₃) δ -118.93.

LC-MS: m/z 159.0 [M-H]⁻.

Step 3: Under nitrogen atmosphere, intermediate **I-B3-3** (0.68 g, 4.25 mmol), triethylsilane (1 mL, 26.6 mmol) and tetrahydrofuran (6 mL) were sequentially added to a dry round-bottom flask. The reaction mixture was stirred at 60°C for 5 hours. TLC showed that the reaction was completed, then the reaction mixture was subjected to rotary evaporation to dryness, diluted by adding water (10 mL) and extracted with ethyl acetate (10 mL * 3). The combined organic phases were washed with saturated sodium bicarbonate aqueous solution (30 mL) and saturated brine (30 mL), dried over anhydrous sodium sulfate, filtered, and concentrated under reduced pressure. The resulting residue was purified by silica gel chromatography (0 to 50% ethyl acetate/petroleum ether) to obtain **I-B3-4** as a yellow solid (566 mg, yield: 82.2%).

¹H NMR (400 MHz, CDCl₃) δ 6.68 (dd, *J =* 8.0, 0.8 Hz, 1H), 6.58(s, 1H), 4.09 (brs, 1H), 3.69 (t, *J =* 8.8 Hz, 2H), 3.12 (t, *J =* 8.8 Hz, 2H).

¹⁹F NMR (376 MHz, CDCl₃) δ -116.52.

LC-MS: m/z 163.1 [M+H]⁺.

Step 4: Intermediate **I-B3-4** (562 mg, 3.47 mmol) and acetonitrile (10 mL) were sequentially added to a dry round-bottom flask under cooling in an ice bath, and then an acetonitrile (10 mL) solution of NBS (617 mg, 3.47 mmol) was added dropwise. The reaction mixture was stirred at 0°C for 0.5 hours. TLC showed that the reaction was completed, then the reaction mixture was diluted by adding water (30 mL) and extracted with ethyl acetate (30 mL * 3). The combined organic phases were washed with saturated sodium bicarbonate aqueous solution (60 mL) and saturated brine (60 mL), dried over anhydrous sodium sulfate, filtered, and concentrated under reduced pressure. The resulting residue was purified by silica gel chromatography (0 to 30% ethyl acetate/petroleum ether) to obtain **I-B3-5** as a white solid (423 mg, yield: 59.6%).

¹H NMR (400 MHz, CDCl₃): 6.62 (s, 1H), 4.08 (brs, 1H), 3.72 (t, *J=* 8.8 Hz, 2H), 3.19 - 3.14 (m, 2H).

¹⁹F-NMR (376 MHz, CDCl₃): -108.97.

Step 5: Under nitrogen atmosphere, intermediate **I-B3-5** (200 mg, 0.83 mmol), DDQ (377 mg, 1.66 mmol) and xylene (10 mL) were sequentially added to a dry three-neck flask, and the reaction mixture was stirred at 120°C for 12 hours. After the reaction was completed, the reaction mixture was concentrated under reduced pressure, and the resulting residue was purified by silica gel chromatography (0 to 50% ethyl acetate/petroleum ether) to obtain **I-B3-6** as a yellow-white solid (170 g, yield: 86%).

¹H NMR (400 MHz, CDCl₃): 8.68 (brs, 1H), 7.62 (s, 1H), 7.42 (t, *J =* 2.8 Hz, 1H), 6.74 - 6.72 (m, 1H).

¹⁹F-NMR (376 MHz, CDCl₃): -110.50.

Step 6: **I-B3-6** (50 mg, 0.20 mmol) and DMF (1 mL) were sequentially added to a 25 mL round-bottom flask under an ice bath. Then NaH (60%) (12 mg, 0.24 mmol) was added thereto, and after stirring for 5 minutes, iodomethane (42.6 mg, 0.3 mmol) was added thereto. The reaction mixture was stirred at room temperature for 2 hours. TLC showed that the reaction was completed, then the reaction mixture was diluted by adding water (10 mL) and extracted with ethyl acetate (10 mL * 3). The combined organic phases were washed with saturated sodium bicarbonate aqueous solution (30 mL) and saturated brine (30 mL), dried over anhydrous sodium sulfate, filtered, and concentrated under reduced pressure. The resulting residue was purified by silica gel chromatography (0 to 30% ethyl acetate/petroleum ether) to obtain **I-B3** as a white solid (37 mg, yield: 73%).

¹H NMR (400 MHz, CDCl₃): 7.52 (s, 1H), 7.25 (d, *J =* 2.8 Hz, 1H), 6.64 (dd, *J =* 3.2, 0.4 Hz, 1H), 3.85 (s, 3H).

¹⁹F-NMR (376 MHz, CDCl₃): -110.85.

LC-MS: m/z 253.0 [M+H]⁺.

### Synthesis of intermediate I-B4: tert-Butyl 5-bromo-6-cyano-4-fluoroindoline-1-carboxylate

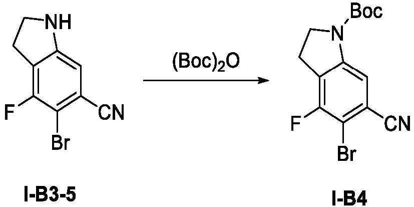

Step 1: Intermediate **I-B3-5** (50 mg, 0.2 mmol) and dichloromethane (4 mL) were sequentially added to a dry round-bottom flask under cooling in an ice bath, and then (BOC)₂O (66 mg, 0.3 mmol) and DMAP (24.4 mg, 0.2 mmol) were added thereto. The reaction mixture was stirred at room temperature for 2 hours. TLC showed that the reaction was completed, and the reaction mixture was concentrated. The resulting residue was purified by silica gel chromatography (0 to 20% ethyl acetate/petroleum ether) to obtain **I-B4** as a white solid (50 mg, yield: 73%).

¹H NMR (400 MHz, CDCl₃): 7.97 (brs, 1H), 4.08 (t, *J* = 8.8 Hz, 2H), 3.20 (t, *J* = 8.4 Hz, 2H), 1.56 (s, 9H).

¹⁹F-NMR (376 MHz, CDCl₃): -108.61.

### Synthesis of intermediate I-B5: 8-Fluoro-7-iodo-2-methylimidazo[1,2-a]pyridine-6-carbonitrile

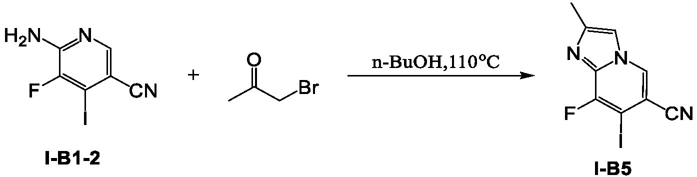

Step 1: *n*-Butanol (2.5 mL), **I-B1-1** (120 mg, 0.456 mmol) and bromoacetone (249.85 mg, 1.824 mmol) were sequentially added to a dry single-neck flask, and the resulting reaction mixture was heated to 110°C and stirred for 16 hours. TLC showed that the reaction was completed. The reaction mixture was subjected to rotary evaporation to dryness, slurried with ethyl acetate/petroleum ether (3:1), and filtered to obtain **I-B5** as a yellow solid (41 mg, yield: 29%).

¹H NMR (400 MHz, DMSO-*d*₆): δ ppm 9.29 (s, 1H), 7.93 (d, *J* = 2.8 Hz, 1H), 2.40 (s, 3H).

LCMS: m/z=302 (M+H)⁺.

### Synthesis of intermediate I-B6: 7-Fluoro-6-iodo-5-cyano-2,3-dihydrobenzofuran

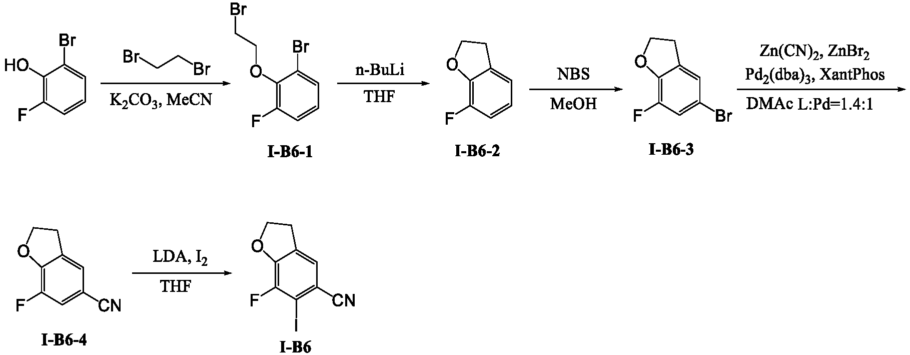

Step 1: 2-Bromo-6-fluorophenol (7.60 g, 40 mmol), 1,2-dibromoethane (14.9 g, 80 mmol), potassium carbonate (11 g, 80 mmol) and acetonitrile (400 mL) were sequentially added to a dry single-neck flask, and the reaction mixture was stirred at 65°C for 16 hours. TLC showed that the reaction was completed, and the reaction mixture was filtered and concentrated. The resulting residue was purified by silica gel chromatography (petroleum ether) to obtain **I-B6-1** as a colorless oily liquid (9.10 g, yield: 77%).
¹H NMR (400 MHz, CDCl₃) δ ppm: 7.34 - 7.32 (m, 1H), 7.10 - 7.05 (m, 1H), 6.97 - 6.92 (m, 1H), 4.38 (t, *J* =7.2 Hz, 2H), 3.66 (t, *J =* 6.8 Hz, 2H)
¹⁹F NMR (376 MHz, CDCl₃) δ -126.41.

Step 2: **I-B6-1** (9.11 g, 30.8 mmol) and tetrahydrofuran (240 mL) were sequentially added to a dry three-neck flask, and *n*-butyllithium (15.4 mL, 38.5 mmol, 2.5 M) was added dropwise at -70°C under nitrogen atmosphere for 15 minutes. The resulting colorless solution was stirred at - 70°C for 90 minutes. TLC showed that the reaction was completed. The reaction mixture was quenched by pouring into water and brine. The aqueous layer was extracted with methyl tert-butyl ether. The combined organic phases were washed with saturated brine, dried over anhydrous sodium sulfate, filtered, and concentrated under reduced pressure. The resulting residue was purified by silica gel chromatography (petroleum ether) to obtain **I-B6-2** as a colorless oily liquid (3.4 g, yield: 80%).

¹H NMR (400 MHz, CDCl₃) δ ppm: 6.97 - 6.95 (m, 1H), 6.92 - 6.87 (m, 1H), 6.79 - 6.74 (m, 1H), 4.66 (t, *J =* 8.8 Hz, 2H), 3.26 (t, *J =* 8.8 Hz, 2H).

¹⁹F NMR (376 MHz, CDCl₃) δ -139.36.

Step 3: Compound **I-B6-2** (3.4 g, 24.6 mmol), methanol (100 mL) and NBS (4.9 g, 27.8 mmol) were sequentially added to a dry single-neck flask at 20°C. The reaction mixture was stirred at 20°C for 16 hours. TLC showed that the reaction was completed. The reaction mixture was quenched with saturated sodium thiosulfate aqueous solution and concentrated to dryness under reduced pressure. The resulting residue was diluted with petroleum ether/ethyl acetate (1:1), and the organic layer was washed twice with water, and the combined aqueous layers were extracted once with petroleum ether/ethyl acetate (1:1). The combined organic phases were washed with saturated brine, dried over anhydrous sodium sulfate, filtered, and concentrated under reduced pressure. The resulting residue was purified by silica gel chromatography (8% ethyl acetate/petroleum ether) to obtain **I-B6-3** as a yellow-green oily liquid (4.57 g, yield: 86%).

¹H NMR (400 MHz, CDCl₃) δ ppm: 7.09 - 7.05 (m, 2H), 4.67 (t, *J* = 8.8 Hz, 2H), 3.25 (t, *J =* 8.8 Hz, 2H). ¹⁹F NMR (376 MHz, CDCl₃) δ -136.10.

Step 4: Compound **I-B6-3** (4.57 g, 21.2 mmol), zinc cyanide (1.72 g, 14.8 mmol), zinc powder (137 mg, 2.1 mmol), Pd₂(dba)₃ (484 mg, 0.53 mmol), XantPhos (672 mg, 1.16 mmol), zinc bromide (470 mg, 2.1 mmol) and dry *N,N*-dimethylacetamide (37 mL) were sequentially added to a dry 50 mL single-neck flask, and the reaction flask was purged with nitrogen. The reaction mixture was stirred at 100°C for 16 hours under nitrogen atmosphere. TLC showed that the reaction was completed. The reaction mixture was filtered, and the filter cake was washed with ethyl acetate. The organic layer was washed twice with water and the combined aqueous layers were extracted once with ethyl acetate. The combined organic phases were washed with saturated brine, dried over anhydrous sodium sulfate, filtered, and concentrated under reduced pressure. The resulting residue was purified by silica gel chromatography (40 to 80% dichloromethane/petroleum ether) to obtain **I-B6-4** as a light yellow solid (3.2 g, yield: 95%).

¹H NMR (400 MHz, CDCl₃) δ ppm: 7.29 - 7.23 (m, 2H), 4.78 (t, *J* = 8.8 Hz, 2H), 3.32 (t, *J* = 8.8 Hz, 2H). ¹⁹F NMR (376 MHz, CDCl₃) δ -136.04.

Step 5: **I-B6-4** (3.55 g, 21.8 mmol) and tetrahydrofuran (100 mL) were sequentially added to a dry three-neck flask, and LDA (16.3 mL, 32.7 mmol, 2.0 M) was added dropwise at -70°C under nitrogen atmosphere. The yellow suspension was stirred at -70°C for 30 minutes. A tetrahydrofuran (50 mL) solution of iodine (8.3 g, 32.7 mmol) was then added to the suspension, and the reaction mixture was stirred at -70°C for another 30 minutes. TLC showed that there was a small amount of raw material remaining. The reaction was quenched with water and returned to room temperature, and 10% sodium thiosulfate aqueous solution was added thereto. The aqueous layer was extracted with ethyl acetate. The combined organic phases were washed with saturated brine, dried over anhydrous sodium sulfate, filtered, and concentrated under reduced pressure. The resulting residue was slurried with petroleum ether/ethyl acetate (10:1) to obtain **I-B6** as a yellow solid (6.4 g, crude). A small amount of analytically pure sample was obtained by preparative TLC.

¹H NMR (400 MHz, CDCl₃) δ ppm: 7.28 (d, *J =* 0.8 Hz, 1H), 4.80 (t, *J =* 8.8 Hz, 2H), 3.33 (t, *J =* 8.8 Hz, 2H).

¹⁹F NMR (376 MHz, CDCl₃) δ -113.04.

### Synthesis of intermediate I-B7: Synthesis of 8-fluoro-7-iodo-3-oxo-3,4-dihydro-2H-benzo[b][1,4]oxazine-6-carbonitrile

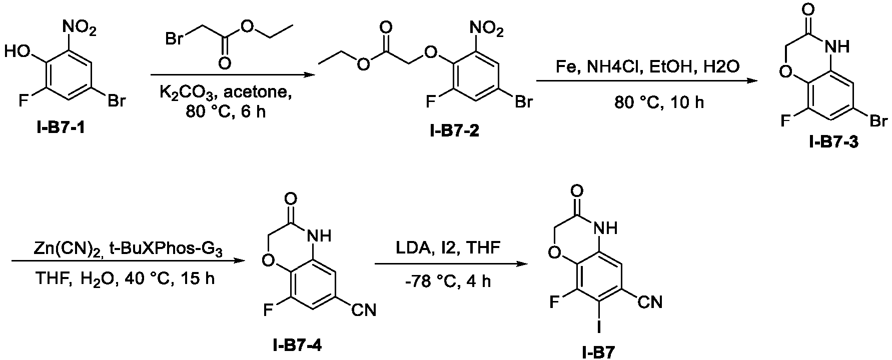

Step 1: Potassium carbonate (11.71 g, 84.75 mmol) was added to an acetone (200 mL) solution of 4-bromo-2-fluoro-6-nitrophenol (10 g, 42.37 mmol). After vacuuming, the mixture was replaced with argon three times, and ethyl bromoacetate (11.94 g, 63.56 mmol) was slowly added dropwise. After vacuuming, the reaction mixture was replaced with argon three times. The reaction mixture was reacted at 80°C for 6 hours. The reaction mixture was cooled to room temperature, added with ethyl acetate (300 mL), washed with saturated brine (2 × 100 mL), dried over anhydrous sodium sulfate, concentrated, and purified by silica gel chromatography (ethyl acetate/petroleum ether = 1/100 to 1/5) to obtain **I-B7-2** as a colorless liquid (7.28 g, yield: 50.1%).

Step 2: Ammonium chloride (5.68 g, 106.14 mmol) and deionized water (15 mL) were added to an absolute ethanol (50 mL) solution of ethyl 2-(4-bromo-2-fluoro-6-nitrophenoxy)acetate **(I-B7-2,** 7.28 g, 21.23 mmol). Iron powder (5.93 g, 106.14 mmol) was added thereto during stirring. After vacuuming, the mixture was replaced with argon three times. The reaction mixture was reacted at 80°C for 10 hours, cooled to room temperature, filtered, and the filter cake was rinsed with ethanol (300 mL). The filtrate was concentrated under reduced pressure and purified by silica gel chromatography (ethyl acetate/petroleum ether = 1/40) to obtain **I-B7-3** as an off-white powder (4.55 g, yield: 92.4%).

¹H NMR (400 MHz, DMSO-*d*₆): δ ppm 11.01 (s, 1H), 7.22 (dd, J = 10.4, 2.4 Hz, 1H), 6.87 (t, J = 2.0 Hz, 1H), 4.69 (s, 2H).

Step 3: Zinc cyanide (1.61 g, 13.73 mmol) was added to a solution of 6-bromo-8-fluoro-2*H*-benzo[*b*][1,4]oxazin-3(4*H*)-one **(I-B7-3,** 4.55 g, 19.61 mmol) in tetrahydrofuran (50 mL) and water (15 mL). After vacuuming, the mixture was replaced with argon three times. t-BuXPhos-Pd-G3 (682.50 mg, 0.98 mmol) was added thereto. After vacuuming, the reaction mixture was replaced with argon three times. The reaction mixture was reacted at 40°C for 15 hours. The reaction mixture was slowly poured into ice water (50 mL), filtered, and extracted by adding ethyl acetate (3 × 100 mL). The organic phases were combined, washed with saturated brine (2 × 100 mL), dried over anhydrous sodium sulfate, concentrated, and purified by silica gel chromatography (ethyl acetate/petroleum ether = 1/40 to 1/5) to obtain **I-B7-4** as a light yellow powder (2.91 g, yield: 83.3%).

¹H NMR (400 MHz, DMSO-*d*₆): δ ppm 11.19 (s, 1H), 7.57 (dd, J = 10.4, 2.0 Hz, 1H), 7.06 (t, J = 1.2 Hz, 1H), 4.80 (s, 2H).

¹⁹F NMR(376 Hz, DMSO-*d*₆)ppm: δ -133.71.

Step 4: LDA (1 M in THF, 1.56 mL, 1.56 mmol) was added dropwise to a tetrahydrofuran (10 mL) solution of 8-fluoro-3,4-dihydro-2*H*-benzo[*b*][1,4]oxazine-6-carbonitrile **(I-B7-4,** 100 mg, 520.43 µmmol) at -78°C under argon atmosphere. The mixture was reacted at -78°C for 1 hour. A tetrahydrofuran solution (10 mL) of iodine (264.18 mg, 1.04 mmol) was slowly added dropwise. The reaction mixture was reacted at -78°C for another 4 hours. The reaction mixture was slowly poured into ice water (10 mL) and extracted by adding ethyl acetate (3 × 20 mL). The organic phases were combined, washed with saturated brine (2 × 20 mL), dried over anhydrous sodium sulfate, concentrated, and purified by silica gel chromatography (ethyl acetate/petroleum ether = 1/20 to 1/1) to obtain **I-B7** as a light yellow powder (137 mg, yield: 82.7%).

¹H NMR (400 MHz, DMSO-*d*₆): δ ppm 11.27 (s, 1H), 7.14 (d, *J=* 1.2 Hz, 1H), 4.87 (s, 2H).

¹⁹F NMR(376 Hz, DMSO-*d*₆): δ ppm -111.86.

LCMS: m/z: 317 [M-H]⁻.

### Synthesis of intermediate I-B8: tert-Butyl 7-bromo-6-cyano-8-fluoro-2,3-dihydro-4H-benzo[b][1,4]oxazine-carboxylate

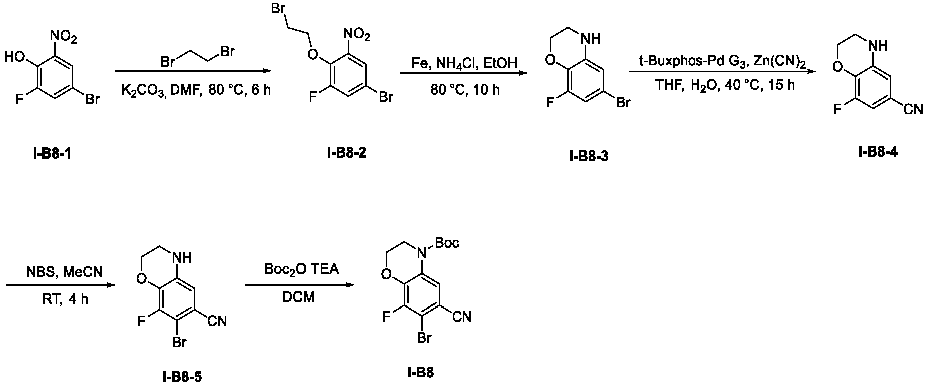

Step 1: K₂CO₃ (11.71 g, 84.75 mmol) was added to a DMF (200 mL) solution of **I-B8-1** (10.0 g, 42.37 mmol). 1,2-Dibromoethane (11.94 g, 63.56 mmol) was slowly added dropwise under argon atmosphere. The reaction mixture was reacted at 80°C for 6 hours. The reaction mixture was cooled to room temperature, diluted with ethyl acetate (300 mL), washed with saturated brine (2 × 500 mL), dried over anhydrous sodium sulfate, concentrated, and purified by silica gel column chromatography (ethyl acetate/petroleum ether = 1/100 to 1/5) to obtain **I-B8-2** as a colorless liquid (7.28 g, yield: 50.1%).

¹H NMR (400 MHz, CDCl₃): δ ppm 7.77 (t, *J* = 2.0 Hz, 1H), 7.55 (dd, *J =* 10.0, 2.4 Hz, 1H), 4.51 - 4.47 (m, 2H), 3.66 (t, *J =* 6.4 Hz, 2H).

Step 2: A deionized water (15 mL) solution of ammonium chloride (5.68 g, 106.14 mmol) was added to an ethanol (50 mL) solution of **I-B8-2** (7.28 g, 21.23 mmol). Iron powder (5.93 g, 106.14 mmol) was added thereto during stirring. After vacuuming, the reaction mixture was replaced with argon three times. The reaction mixture was reacted at 80°C for 10 hours. The reaction mixture was cooled to room temperature, filtered, and the filter cake was rinsed with ethanol (300 mL). The filtrate was concentrated, and ethyl acetate (100 mL) was added to dissolve the residue, washed with saturated brine (2 × 100 mL), dried over anhydrous sodium sulfate, and filtered. The filtrate was concentrated to dryness under reduced pressure and purified by silica gel column chromatography (ethyl acetate/petroleum ether = 1/40) to obtain **I-B8-3** as an off-white solid (4.55 g, yield: 92.4%).

¹H NMR (400 MHz, CDCl₃): δ ppm 6.63 (dd, *J =* 10.0, 2.0 Hz, 1H), 6.50 (t, *J =* 2.0 Hz, 1H), 4.26 (t, *J =* 4.4 Hz, 1H), 3.95 (br, 1H), 3.45 - 3.42 (m, 2H).

LCMS: m/z 234.0 [M+H]⁺.

Step 3: Zn(CN)₂ (1.61 g, 13.73 mmol) was added to a solution of **I-B8-3** (4.55 g, 19.61 mmol) in tetrahydrofuran (50 mL) and water (15 mL). After vacuuming, the mixture was replaced with argon three times. t-BuXPhos-Pd-G3 (682.50 mg, 0.98 mmol) was added thereto. After vacuuming, the reaction mixture was replaced with argon three times. The reaction mixture was reacted at 40°C for 15 hours, slowly poured into ice water (50 mL), extracted by adding ethyl acetate (3 × 50 mL), filtered with diatomite to remove the insoluble substances, and the filter residue was rinsed with ethyl acetate (50 mL). The organic phases were combined, washed with saturated brine (2 × 100 mL), dried over anhydrous sodium sulfate, concentrated and purified by silica gel column chromatography (ethyl acetate/petroleum ether = 1/40 to 1/5) to obtain **I-B8-4** as a light yellow solid (2.91 g, yield: 83.3%).

¹H NMR (400 MHz, CDCl₃): δ ppm 6.78 (dd, *J =* 10.0, 2.0 Hz, 1H), 6.65 (t, *J =* 1.6 Hz, 1H), 4.36 - 4.34 (m, 2H), 4.15 (br, 1H), 3.49 - 3.47 (m, 2H).

LCMS: m/z 179.0 [M+H]⁻.

Step 4: NBS (928.97 mg, 16.33 mmol) was added to an acetonitrile (100 mL) solution of **I-B8-4** (2.91 g, 16.33 mmol) under an ice water bath. The reaction mixture was stirred and reacted at room temperature for 4 hours. The reaction mixture was poured into ice-cold saturated sodium sulfite aqueous solution (50 mL) and filtered. The filter cake was washed with water and purified by silica gel chromatography (ethyl acetate/petroleum ether = 1/20 to 1/5) to obtain **I-B8-5** as a white solid (2.20 g, yield: 52.40%).

¹H NMR (400 MHz, CDCl₃): δ ppm 6.71 (d, *J* = 1.6 Hz, 1H), 4.37 - 4.34 (m, 2H), 4.19 (br, 1H), 3.49 - 3.47 (m, 2H).

Step 5: **I-B8-5** (200 mg, 0.781 mmol), Boc₂O (255 mg, 1.17 mmol), triethylamine (217 mL, 1.4 mmol) and dichloromethane (5 mL) were sequentially added to a reaction flask, and the reaction mixture was stirred at room temperature for 16 hours. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 5:1) to obtain **I-B8** as a colorless oil (220 mg, yield: 79.1%).

¹H NMR (400 MHz, CDCl₃): δ ppm 8.12 (s, 1H), 4.39 (t, *J* = 4.4 Hz, 2H), 3.92 (t, *J =* 4.4 Hz, 2H), 1.56 (s, 9H).

LC-MS: m/z 358.9 [M+H]⁺.

### Synthesis of intermediate I-B9: 7-Bromo-8-fluoro-4-methyl-3,4-dihydro-2H-benzo[b][1,4]oxazine-6-carbonitrile

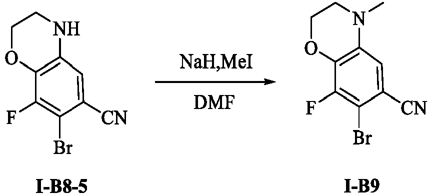

Step 1: **I-B8-5** (0.1 g, 0.389 mmol) was dissolved in DMF (0.5 mL), and sodium hydride (35 mg, 0.584 mmol) was added thereto. The mixture was cooled to 0°C and stirred for 30 minutes under nitrogen atmosphere. Iodomethane (83 mg, 0.584 mmol) was added dropwise. After the dropwise addition was completed, the reaction mixture was warmed to room temperature and reacted for 1 hour. The reaction mixture was slowly poured into ice-cold saturated ammonium chloride solution (10 mL), and extracted by adding ethyl acetate (3 × 10 mL). The organic phase was washed with saturated brine (10 mL), dried, concentrated, and purified by silica gel chromatography (0 to 50% ethyl acetate/petroleum ether) to obtain **I-B9** as a light yellow solid (80 mg, yield: 76%).

¹H NMR (400 MHz, CDCl₃): δ ppm 6.69 (d, *J =* 1.6 Hz, 2H), 4.41 - 4.39 (m, 2H), 3.36 - 3.34 (m, 2H), 2.93 (s, 3H).

LC-MS: m/z = 271.2 [M+H]⁺.

### Synthesis of intermediate I-B10: 8-Fluoro-7-iodo-4-methyl-3-oxo-3,4-dihydro-2H-benzo[b][1,4]oxazine-6-carbonitrile

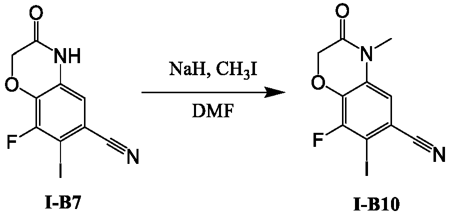

Step 1: DMF (1 mL) and **I-B7** (100 mg, 0.31 mmol) were sequentially added to a dry two-neck flask under nitrogen atmosphere, cooled to 0°C, added with NaH (19 mg, 0.47 mmol, 60% dispersion in mineral oil), and stirred for 10 minutes. Then CH₃I (67 mg, 0.47 mmol) was added thereto, and the reaction mixture was warmed to room temperature and stirred for 1 hour. TLC showed that the reaction was completed. The reaction mixture was poured into water and extracted with ethyl acetate. The combined organic phases were washed with saturated brine, dried over anhydrous sodium sulfate, filtered, and concentrated under reduced pressure. The resulting residue was purified by silica gel chromatography to obtain **I-B10** as a yellow solid (70 mg, yield: 67.1%).

¹H NMR (400 MHz, DMSO-*d*₆): δ ppm 7.63 (s, 1H), 4.90 (s, 2H), 3.29 (s, 3H).

### Synthesis of intermediate I-B11: 7-Bromo-8-fluoro-4-propionyl-3,4-dihydro-2H-benzo[b][1,4]oxazine-6-carbonitrile

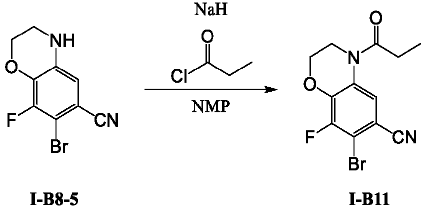

Step 1: DMF (3 mL) and **I-B8-5** (150 mg, 0.58 mmol) were sequentially added to a dry two-neck flask under nitrogen atmosphere, cooled to 0°C, added with NaH (30 mg, 0.76 mmol, 60% dispersion in mineral oil), and stirred for 10 minutes. Then propionyl chloride (81 mg, 0.87 mmol) was added thereto, and the reaction mixture was warmed to room temperature and stirred for 1 hour. TLC showed that the reaction was completed. The reaction mixture was poured into water and extracted with ethyl acetate. The combined organic phases were washed with saturated brine, dried over anhydrous sodium sulfate, filtered, and concentrated under reduced pressure. The resulting residue was purified by silica gel chromatography to obtain **I-B11** as a yellow solid (160 mg, yield: 87.5%).

¹H NMR (400 MHz, CDCl₃) δ 8.06 (brs, 1H), 4.45 (t, *J* = 4.8 Hz, 2H), 3.96 (t, *J* = 4.4 Hz, 2H), 2.57 (q, *J =* 7.32 Hz, 2H), 1.23 (t, *J =* 7.2 Hz, 3H).

LCMS: m/z= 313.0 (M+H)⁺.

### Synthesis of intermediate I-B12: 4-Acetyl-7-bromo-8-fluoro-3,4-dihydro-2H-benzo[b][1,4]oxazine-6-carbonitrile

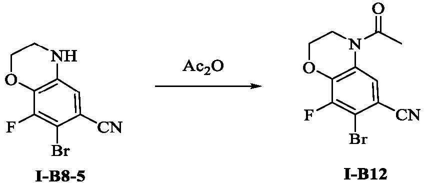

Step 1: **I-B8-5** (1.1 g, 4.279 mmol) was dissolved in acetic anhydride (5 mL), heated to 100°C and reacted for 3 hours. The reaction mixture was slowly poured into ice-cold saturated sodium bicarbonate solution (100 mL) until the pH was equal to 8, and extracted by adding ethyl acetate (3 × 100 mL). The organic phase was washed with saturated brine (100 mL), dried, concentrated, and purified by silica gel chromatography (0 to 50% ethyl acetate/petroleum ether) to obtain **I-B12** as a light yellow solid (1.06 g, yield: 82.81%).

¹H NMR (400 MHz, CDCl₃) δ 8.03 (brs, 1H), 4.46 (t, *J =* 4.8 Hz, 2H), 3.96 (t, *J* = 4.4 Hz, 2H), 2.34 (s, 3H).

LC-MS: m/z =297.0 [M+H]⁺.

### Synthesis of intermediate I-B13: 7-Fluoro-6-iodo-1-((2-(trimethylsilyl)ethoxy)methyl)-1H-indazole-5-carbonitrile

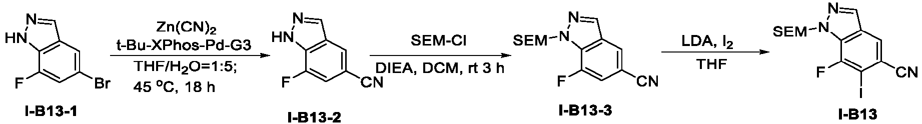

Step 1: Zn(CN)₂ (352 mg, 3.0 mmol) was added to a solution of **I-B13-1** (483 mg, 2.24 mmol) in tetrahydrofuran (1.0 mL) and water (5.0 mL). After vacuuming, the mixture was replaced with argon three times. *t*-BuXPhos-Pd-G3 (119 mg, 0.15 mmol) was added thereto. After vacuuming, the reaction mixture was replaced with argon three times. The reaction mixture was reacted at 45°C for 18 hours. The reaction mixture was slowly poured into ice water (10 mL), extracted by adding ethyl acetate (3 × 10 mL), filtered with diatomite to remove the insoluble substances and the filter residue was rinsed with ethyl acetate (20 mL). The organic phases were combined, washed with saturated brine (2 × 50 mL), dried over anhydrous sodium sulfate, concentrated and purified by silica gel column chromatography (ethyl acetate/petroleum ether = 1/40 to 1/2) to obtain **I-B13-2** as a light yellow solid (170 mg, yield: 47%).

¹H NMR (400 MHz, DMSO-*d*₆) δ 14.21 (brs, 1H), 8.41 (d, *J*=3.2 Hz, 1H), 8.30 (d, *J*=0.8 Hz, 1H), 7.72 (d, *J=*10.8 Hz, 1H).

LCMS: m/z 162.0 [M+H]⁺.

Step 2: SEM-Cl (320 mg, 1.92 mmol) and DIEA (0.53 mL, 2.88 mmol) were added to a dichloromethane (5.0 mL) solution of **I-B13-2** (154 mg, 0.96 mmol), and the reaction mixture was reacted at room temperature 2 hours. The reaction mixture was poured into water (10 mL) and extracted by adding ethyl acetate (3 × 10 mL). The organic phases were combined, washed with saturated brine (2 × 50 mL), dried over anhydrous sodium sulfate, concentrated and purified by silica gel column chromatography (ethyl acetate/petroleum ether = 1/40 to 1/2) to obtain **I-B13-3** as a light yellow solid (145 mg, yield: 52%).

¹H NMR (400 MHz, CDCl₃) δ 8.16 (d, J = 2.0 Hz, 1H), 7.94 (d, J = 1.2 Hz, 1H), 7.34 (dd, J = 10.8, 1.2 Hz, 1H), 5.84 (s, 2H), 3.60 (t, J=8.4 Hz, 2H), 0.89 (t, J=8.0 Hz, 2H), 0.00 (s, 9H).

¹⁹F NMR (376 MHz, CDCl₃): δ ppm -128.48.

Step 3: **I-B13-3** (243 mg, 0.83 mmol) was dissolved in tetrahydrofuran (15 mL), and iodine (423 mg, 1.67 mmol) was added thereto, and then the mixture was cooled to -65°C under nitrogen atmosphere. LDA (2.0 M in THF, 2.08 mL, 4.15 mmol) was added dropwise. After the dropwise addition was completed, the reaction mixture was stirred for 30 minutes, then warmed to room temperature and reacted for 1 hour. The reaction mixture was slowly poured into ice water (30 mL) and extracted by adding ethyl acetate (3 × 50 mL). The organic phase was washed with saturated brine (2 × 50 mL), dried, concentrated, and purified by silica gel column chromatography (ethyl acetate/petroleum ether = 0/1 to 1/10) to obtain **I-B13** as a colorless oil (20 mg, yield: 5.1%).

LCMS: m/z 416.1 [M-H]⁻.

### Synthesis of intermediate I-B21: 5-Bromo-4-fluoro-1-methyl-1H-indazole-6-carbonitrile

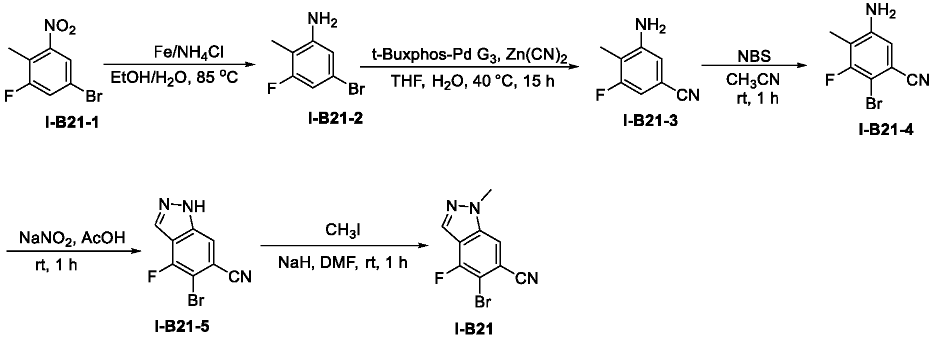

Step 1: Ammonium chloride (6.11 g, 114.3 mmol) and reduced iron powder (6.4 g, 114.3 mmol) were added to a solution of **I-B21-1** (5.35 g, 22.9 mmol) in ethanol (60 mL) and water (30 mL). The reaction mixture was reacted at 80°C for 2 hours. The reaction mixture was cooled to room temperature, filtered, and the filter cake was rinsed with ethanol (300 mL). The filtrate was concentrated, added with ethyl acetate (100 mL) to dissolve, washed with saturated brine (2 × 100 mL), dried over anhydrous sodium sulfate, and filtered. The filtrate was concentrated to dryness under reduced pressure and purified by silica gel column chromatography (ethyl acetate/petroleum ether = 1/100 to 1/3) to obtain **I-B21-2** as a colorless oil (4.65 g, yield: 99%).

LCMS: m/z 204 [M+H]⁺.

Step 2: Zn(CN)₂ (2.69 g, 22.8 mmol) was added to a solution of **I-B21-2** (4.65 g, 22.8 mmol) in tetrahydrofuran (10 mL) and water (50 mL). After vacuuming, the mixture was replaced with argon three times. *t*-BuXPhos-Pd-G3 (909 mg, 1.15 mmol) was added thereto. After vacuuming, the reaction mixture was replaced with argon three times. The reaction mixture was reacted at 45°C for 15 hours. The reaction mixture was slowly poured into ice water (50 mL), extracted by adding ethyl acetate (3 × 50 mL), filtered with diatomite to remove the insoluble substances and the filter residue was rinsed with ethyl acetate (50 mL). The organic phases were combined, washed with saturated brine (2 × 100 mL), dried over anhydrous sodium sulfate, concentrated and purified by silica gel column chromatography (ethyl acetate/petroleum ether = 1/40 to 1/2) to obtain **I-B21-3** as a light yellow solid (3.03 g, yield: 87.6%).

¹H NMR (400 MHz, DMSO-*d₆*) δ 6.82 - 6.78 (m, 2H), 5.74 (s, 2H), 2.01 (d, J = 1.6 Hz, 3H).

Step 3: NBS (3.18 mg, 17.9 mmol) was added to an acetonitrile (60 mL) solution of **I-B21-3** (2.70 g, 17.9 mmol) under an ice water bath. The reaction mixture was stirred and reacted at room temperature for 1 hour. The reaction mixture was poured into ice-cold saturated sodium sulfite aqueous solution (50 mL) and filtered. The filter cake was washed with water and purified by silica gel chromatography (ethyl acetate/petroleum ether = 1/20 to 1/5) to obtain **I-B21-4** as a white solid (3.5 g, yield: 95%).

LCMS: m/z 228.9 [M+H]⁺.

Step 4: **I-B21-4** (3.5 g, 15.3 mmol), acetic acid (50 mL) and sodium nitrite (2.11 g, 30.6 mmol) were sequentially added to a reaction flask, and the reaction mixture was stirred at room temperature for 1 hour. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (ethyl acetate/petroleum ether = 1/40 to 1/2) to obtain **I-B21-5** as a colorless oil (3.2 g, yield: 87%).

LC-MS: m/z 239.3 [M+H]⁺.

Step 5: **I-B21-5** (500 mg, 2.08 mmol) and DMF (10 mL) were sequentially added to a 25 mL round-bottom flask under an ice bath. Then NaH (60%) (166 mg, 4.16 mmol) was added thereto, and after stirring for 5 minutes, iodomethane (590 mg, 4.16 mmol) was added thereto. The reaction mixture was stirred at room temperature for 1 hour. TLC showed that the reaction was completed, then the reaction mixture was diluted by adding water (10 mL) and extracted with ethyl acetate (15 mL * 3). The combined organic phases were washed with saturated sodium bicarbonate aqueous solution (30 mL) and saturated brine (30 mL), dried over anhydrous sodium sulfate, filtered, and concentrated under reduced pressure. The resulting residue was purified by silica gel chromatography (0 to 30% ethyl acetate/petroleum ether) to obtain **I-B21** as a white solid (270 mg, yield: 51%).

¹H NMR (400 MHz, DMSO-*d*₆): δ ppm 8.48 (s, 1H), 8.38 (s, 1H), 4.14 (s, 3H).

LC-MS: m/z 255.8 [M+H]⁺.

### Synthesis of intermediate I-B22: 9-Fluoro-8-iodo-2,3-dihydro-[1,4]oxazino[2,3,4-hi]indazole-7-carbonitrile

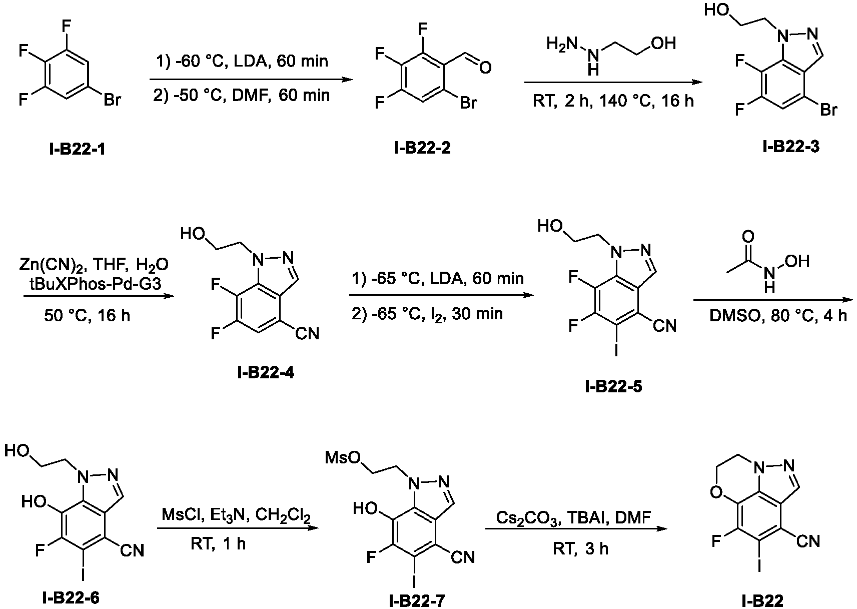

Step 1: LDA (2.0 M in THF/hexane, 52.1 mL, 104.2 mmol) was added dropwise to a THF (200 mL) solution of **I-B22-1** (20.0 g, 94.8 mmol) at -65°C under argon atmosphere. The mixture was reacted at -65°C for 1 hour. DMF (20 mL) was added dropwise quickly. The reaction mixture was reacted at -65°C for another 1 hour. The reaction mixture was poured into ice-cold saturated ammonium chloride solution (500 mL) and extracted with dichloromethane (3 × 500 mL). The organic phases were combined, dried over anhydrous sodium sulfate, and filtered. The filtrate was concentrated to dryness under reduced pressure, and the crude was purified by silica gel chromatography (petroleum ether: ethyl acetate = 100:1) to obtain **I-B22-2** as a yellow oil (18.6 g, yield: 82.1%).

¹H NMR (400 MHz, CDCl₃): δ ppm 10.24 (s, 1H), 7.41 - 7.36 (m, 1H).

Step 2: An ethanol (180 mL) solution of **I-B22-2** (18.0 g, 75.3 mmol) and 2-hydrazinoethanol (11.7 g, 154.2 mmol) was placed in a sealed jar. The reaction mixture was reacted at room temperature for 2 hours, then heated to 140°C and reacted for 16 hours. The reaction mixture was concentrated, and the residue was purified by column chromatography (dichloromethane: petroleum ether: ethyl acetate = 2:4:1) to obtain **I-B22-3** as a white solid (14.7 g, yield: 70.4%).

¹H NMR (400 MHz, CDCl₃): δ ppm 7.99 (d, J = 2.0 Hz, 1H), 7.21(dd, J = 9.6, 5.6 Hz, 1H), 4.64 (t, J = 4.8 Hz, 2H), 4.12 (q, J = 5.2 Hz, 2H), 2.55 (t, J = 6.0 Hz, 1H).

¹⁹F NMR (376 MHz, CDCl₃): δ ppm -139.98, -161.46.

Step 3: Methanesulfonato(2-di-t-butylphosphino-2',4',6'-tri-*i*-propyl-1,1'-biphenyl)(2'-amino-1,1'-biphenyl-2-yl)palladium(II) (2.26 g, 2.84 mmol) was added to a solution of **I-B22-3** (15.8 g, 57.0 mmol) and zinc cyanide (4.7 g, 40.0 mmol) in THF (1.05 L) and water (700 mL). The air was replaced with argon three times. The reaction mixture was heated to 50°C, reacted for 16 hours, and filtered. The filtrate was concentrated under reduced pressure to remove the solvent THF. The reaction mixture was diluted with water (500 mL), then extracted with dichloromethane (3 × 500 mL). The organic phases were combined, washed with saturated brine, dried over anhydrous sodium sulfate, filtered, and the filtrate was concentrated to dryness under reduced pressure. The crude was purified by column chromatography (petroleum ether: ethyl acetate = 3:1) to obtain **I-B22-4** as an off-white solid (12 g, yield: 94.3%).

¹H NMR (400 MHz, DMSO-*d*₆): δ ppm 8.44 (d, J = 2.4 Hz, 1H), 8.11 (dd, J = 10.4, 6.0 Hz, 1H), 4.92 (t, J = 5.6 Hz, 1H), 4.57 (t, J = 5.6 Hz, 2H), 3.81 (q, J = 5.2 Hz, 2H).

¹⁹F NMR (376 MHz, DMSO-*d*₆): δ ppm -142.12, -148.35.

Step 4: LDA (2.0 mol/L in THF/hexane, 60.5 mL, 60.5 mmol) was added dropwise to a THF (250 mL) solution of **I-B22-4** (9.0 g, 40.3 mmol) at -65°C under argon atmosphere. The mixture was reacted at -65°C for 60 minutes. A THF (30 mL) solution of iodine (15.4 g, 60.5 mmol) was added dropwise. The reaction mixture was reacted at -65°C for 30 minutes. The reaction mixture was poured into ice-cold saturated ammonium chloride aqueous solution (500 mL) and extracted with ethyl acetate (3 × 300 mL). The organic phases were combined, washed with saturated brine (3 × 500 mL), dried over anhydrous sodium sulfate, and filtered. The filtrate was concentrated to dryness under reduced pressure and purified by silica gel chromatography (petroleum ether: ethyl acetate = 2:1) to obtain **I-B22-5** as a yellow oil (7.6 g, yield: 54.0%).

¹H NMR (400 MHz, DMSO-*d*₆): δ ppm 8.38 (d, J = 2.0 Hz, 1H), 4.55 (t, J = 5.2 Hz, 2H), 3.79 (t, J = 5.2 Hz, 2H).

¹⁹F NMR (376 MHz, DMSO-*d*₆): δ ppm -120.26, -145.05.

Step 5: Acetohydroxamic acid (4.3 g, 56.7 mmol) was added to a DMSO (70 mL) solution of **I-B22-5** (6.6 g, 18.9 mmol) and potassium carbonate (13.1 g, 94.5 mmol). The reaction mixture was heated to 80°C, reacted for 4 hours, and poured into ice water (200 mL). The pH was adjusted to 6 with aq. 2M HCl. The mixture was filtered, and the filter cake was purified by silica gel chromatography (dichloromethane: methanol = 10:1) to obtain **I-B22-6** as a yellow oil (5.6 g, yield: 85.3%).

¹H NMR (400 MHz, DMSO-*d*₆): δ ppm 8.08 (s, 1H), 4.66 (t, J = 5.6 Hz, 2H), 3.79 (t, J = 5.6 Hz, 2H).

¹⁹F NMR (376 MHz, DMSO-*d*₆): δ ppm -120.23.

Step 6: A dichloromethane (5 mL) solution of methanesulfonyl chloride (180.4 mg, 1.57 mmol) was slowly added dropwise to a dichloromethane (20 mL) solution of **I-B22-8** (366 mg, 1.05 mol) and triethylamine (213.1 mg, 2.10 mol) at 0°C under argon atmosphere. The reaction mixture was reacted at room temperature for 1 hour. The reaction mixture was diluted by adding dichloromethane (20 mL), washed with saturated brine (2 × 20 mL), dried over anhydrous sodium sulfate, and filtered. The filtrate was concentrated nearly to dryness under reduced pressure to obtain crude **I-B22-9,** which was directly used in the next step.

¹H NMR (400 MHz, DMSO-*d*₆): δ ppm 8.24 (s, 1H), 4.85 (t, J = 4.8 Hz, 2H), 3.61 (t, J = 5.2 Hz, 2H), 3.32 (s, 3H).

¹⁹F NMR (376 MHz, DMSO-*d*₆): δ ppm -121.16.

Step 7: Cesium carbonate (687 mg, 2.10 mmol) and tetrabutylammonium iodide (9 mg, 0.1 mmol) were added to a DMF (10 mL) solution of crude **I-B22-9** from the previous step. After vacuuming, the reaction mixture was replaced with argon three times and reacted at room temperature for 3 hours. The reaction mixture was slowly poured into ice water (10 mL) and extracted by adding ethyl acetate (3 × 20 mL). The organic phases were combined, washed with saturated brine (2 × 20 mL), dried over anhydrous sodium sulfate, and filtered. The filtrate was concentrated under reduced pressure and purified by silica gel chromatography (ethyl acetate/petroleum ether = 1/50 to 1/3) to obtain **I-B22** 9-fluoro-8-iodo-2,3-dihydro-[1,4]oxazino[2,3,4-*hi*]indazole-7-carbonitrile (198 mg, two-step yield: 57.1%).

¹H NMR (400 MHz, DMSO-*d*₆): δ ppm 8.24 (s, 1H), 4.85 (t, J = 4.4 Hz, 2H), 4.61 (t, J = 5.2 Hz, 2H).

¹⁹F NMR (376 MHz, DMSO-*d*₆): δ ppm -121.16.

### Synthesis of intermediate I-B24: 1-((tert-Butyldiphenylsilyl)oxy)-7-fluoro-6-iodo-2,3-dihydro-1H-indene-5-carbonitrile

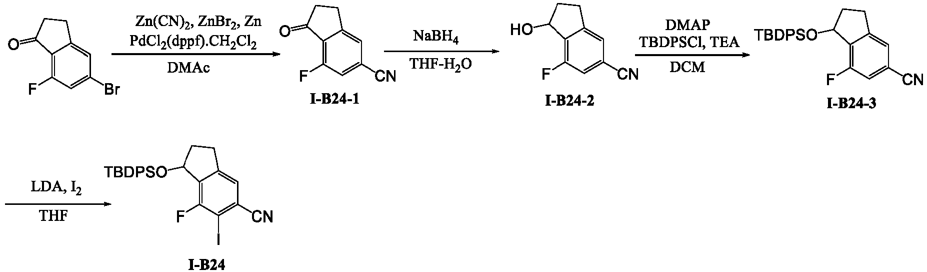

Step 1: 5-Bromo-7-fluoro-2,3-dihydro-1*H*-inden-1-one (1.37 g, 6 mmol), 1,1'-bis(diphenylphosphino)ferrocene-palladium(II)dichloride dichloromethane complex (245 mg, 0.30 mmol), zinc cyanide (493.2 mg, 4.2 mmol), zinc bromide (135.1 mg, 0.60 mmol), zinc powder (19.6 mg, 0.30 mmol) and *N*,*N*-dimethylacetamide (15 mL) were sequentially added to a dry single-neck flask. The reaction system was purged with nitrogen for half a minute, and the reaction mixture was stirred at 100°C for 17 hours. TLC showed that the reaction was completed. Ethyl acetate was added thereto, and the reaction mixture was filtered with diatomite. The filter cake was washed with ethyl acetate. The filtrate was added with water and extracted with ethyl acetate. The organic phase was washed with brine, dried over sodium sulfate and concentrated. The residue was purified by silica gel chromatography (25% ethyl acetate/petroleum ether) to obtain **I-B24-1** as a brown solid (987 mg, yield: 94%).

¹H NMR (400 MHz, CDCl₃) δ7.58 (s,1H), 7.28 (d, J = 8.4 Hz, 1H), 3.23 (t, J = 6.0 Hz, 2H), 2.80 (t, J = 6.0 Hz, 2H).

Step 2: **I-B24-1** (800 mg, 4.57 mmol), tetrahydrofuran (12 mL) and water (4 mL) were sequentially added to a dry three-neck flask, and sodium borohydride (172 mg, 4.57 mmol) was added thereto, and the reaction mixture was stirred at room temperature for 16 hours. TLC showed that the reaction was completed, then water and dilute hydrochloric acid were added thereto. The aqueous layer was extracted with ethyl acetate. The organic phase was washed with brine, dried over sodium sulfate and concentrated to obtain crude **B24-2** as a brownish-yellow solid (782 mg, yield: 96%).

Step 3: Compound **I-B24-2** (870 mg, 4.91 mmol) was dissolved in dichloromethane (20 mL) in a dry single-neck flask at 20°C. Triethylamine (993 mg, 9.82 mmol) and 4-dimethylaminopyridine (60 mg, 0.49 mmol) were added thereto. Finally, *tert-*butylchlorodiphenylsilane (2.02 g, 7.37 mmol) was added thereto. The reaction mixture was stirred at 20°C for 16 hours. TLC showed that the reaction was completed. The reaction mixture was concentrated to dryness under reduced pressure. The resulting residue was purified by silica gel chromatography (3% ethyl acetate/petroleum ether) to obtain **I-B24-3** as a yellow oily liquid (675 mg, yield: 33%).

¹H NMR (400 MHz, CDCl₃) δ7.75-7.65 (m, 4H), 7.50-7.35 (m, 6H), 7.30 (s, 1H),7.13 (d, J = 8.4 Hz, 1H), 5.45 (t, J = 5.2 Hz, 1H), 3.17-3.07 (m, 1H), 2.76-2.65 (m, 1H), 2.05-1.99 (m, 2H), 1.05 (s, 9H).

Step 4: **I-B24-3** (575 mg, 1.38 mmol) and tetrahydrofuran (5 mL) were sequentially added to a dry three-neck flask, and LDA (1.38 mL, 2.77 mmol, 2.0 M) was added dropwise at -70°C under nitrogen atmosphere. The resulting yellow solution was stirred at -70°C for 90 minutes. Iodine (702 mg, 2.77 mmol) was then added to the solution, and the reaction mixture was stirred at -70°C for another 30 minutes. TLC showed that there was a very small amount of raw material remaining. The reaction was quenched with water and returned to room temperature, and 10% sodium thiosulfate aqueous solution was added thereto. The aqueous layer was extracted with ethyl acetate. The combined organic phases were washed with saturated brine, dried over anhydrous sodium sulfate, filtered, and concentrated under reduced pressure. The resulting residue was purified by silica gel chromatography (5% ethyl acetate/petroleum ether) to obtain **I-B24** as a colorless oily liquid (648 mg, yield: 86%).

### Synthesis of intermediate I-B26: 7-Fluoro-6-iodo-2-(((tetrahydro-2H-pyran-2-yl)oxy)methyl)-2,3-dihydrobenzofuran-5-carbonitrile

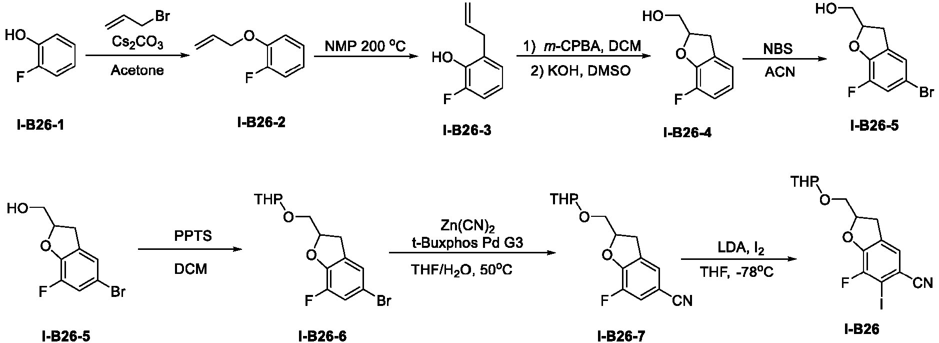

Step 1: **I-B26-1** (10 g, 89.206 mmol), allyl bromide (12.95 g, 107 mmol) and Cs₂CO₃ (43.6 g, 133.8 mmol) were added to acetone (100 mL), and the reaction mixture was stirred at room temperature for 12 hours. The reaction mixture was diluted with water and extracted with ethyl acetate. The organic layer was dried over Na₂SO₄ and concentrated under reduced pressure, and the residue was purified by silica gel chromatography (ethyl acetate/petroleum ether = 1/20) to obtain intermediate **I-B26-2** (13 g, yield: 95.77%) as a colorless oil.

¹H NMR (400 MHz, Chloroform-d) δ 7.14 - 6.85 (m, 4H), 6.06 (ddt, J = 17.3, 10.6, 5.4 Hz, 1H), 5.42 (dq, J = 17.4, 1.7 Hz, 1H), 5.30 (dq, J = 10.5, 1.4 Hz, 1H), 4.60 (dt, J = 5.4, 1.5 Hz, 2H).

Step 2: Intermediate **I-B26-2** (8 g, 52.56 mmol) was dissolved in NMP (80 mL), heated to 200°C and stirred for 3 hours, cooled to room temperature and diluted by adding ethyl acetate (300 mL). The organic layer was washed with brine, dried over anhydrous sodium sulfate, filtered and the solvent was removed. The resulting intermediate **I-B26-3** (5 g, yield: 62.5%) was a yellow oil.

Step 3: Intermediate **I-B26-3** (5 g, 32.85 mmol) was dissolved in dichloromethane (50 mL). *m*-CPBA (7.37 g, 42.715 mmol) was added thereto at 0°C, and the reaction mixture was stirred at room temperature for 4 hours. The reaction mixture was diluted with ethyl acetate, washed with saturated sodium bicarbonate solution, and washed with saturated sodium sulfite aqueous solution. The organic phase was washed with saturated brine, dried over anhydrous sodium sulfate, and concentrated to obtain a crude. The crude was dissolved in DMSO (50 mL), and an aqueous solution (15 mL) of KOH (2.4 g, 42.715 mmol) was added thereto at 0°C. The reaction mixture was reacted at room temperature for 4 hours. The reaction mixture was quenched by adding ice water and diluted with ethyl acetate. The organic phase was hydrolyzed, washed with saturated brine, dried over anhydrous sodium sulfate, concentrated under reduced pressure, and purified by silica gel column chromatography (ethyl acetate/petroleum ether = 1/10) to obtain intermediate **I-B26-4** (5 g, yield: 90.49%).

Step 4: Intermediate **I-B26-4** (5 g, 29.73 mmol) was dissolved in acetonitrile (50 mL), and NBS (5.82 g, 32.71 mmol) was slowly added thereto at room temperature. The reaction mixture was stirred at room temperature for 2 hours. The mixture was filtered, and the filtrate was concentrated under reduced pressure. The residue was purified by silica gel column chromatography (ethyl acetate/petroleum ether = 1/10) to obtain intermediate **I-B26-5** (6 g, yield: 81.68%).

Step 5: Intermediate **I-B26-5** (2 g, 8.095 mmol), DHP (1.36 g, 16.19 mmol) and PPTS (0.2 g, 0.81 mmol) were dissolved in DCM (20 mL), and the reaction mixture was stirred at room temperature overnight. The mixture was filtered, and the filtrate was concentrated under reduced pressure. The residue was purified by silica gel column chromatography (ethyl acetate/petroleum ether = 1/20) to obtain intermediate **I-B26-6** (2.7 g, yield: 98%).

Step 6: Intermediate **I-B26-6** (2.7 g, 8.153 mmol), t-BuXPhos-Pd-G3 (0.32 g, 0.408 mmol) and Zn(CN)₂ (0.96 g, 8.153 mmol) were dissolved in THF (30 mL) and H₂O (10 mL), and the reaction mixture was stirred at 40°C under nitrogen atmosphere overnight. The mixture was filtered, and the filtrate was concentrated under reduced pressure. The residue was purified by silica gel column chromatography (ethyl acetate/petroleum ether = 1/10) to obtain intermediate **I-B26-7** (2 g, yield: 88.47%).

Step 7: A tetrahydrofuran (20 mL) solution of iodine (2.47 g, 9.437 mmol) was added dropwise to a tetrahydrofuran (8 mL) solution of **I-B26-7** (1.8 g, 6.491 mmol) at -65°C under argon atmosphere. After the dropwise addition was completed, the mixture was stirred at -65°C for 10 minutes. LDA (2.0 mol/L in THF/hexane, 6.5 mL, 12.982 mmol) was added dropwise. The reaction mixture was reacted at -65°C for 2 hours. The reaction mixture was slowly poured into ice-cold saturated ammonium chloride solution (20 mL). The reaction mixture was extracted by adding ethyl acetate (3 * 30 mL), and the organic phases were combined, washed with saturated brine (2 * 30 mL), dried over anhydrous sodium sulfate, and filtered. The filtrate was concentrated under reduced pressure and purified by silica gel chromatography (ethyl acetate/petroleum ether = 1/5) to obtain **I-B26** (1.9 g, yield: 72.60%).

### Synthesis of intermediate I-B27: 8-Fluoro-7-iodo-2-(((tetrahydro-2H-pyran-2-yl)oxy)methyl)-2,3-dihydrobenzo[b][1,4] dioxine-6-carbonitrile

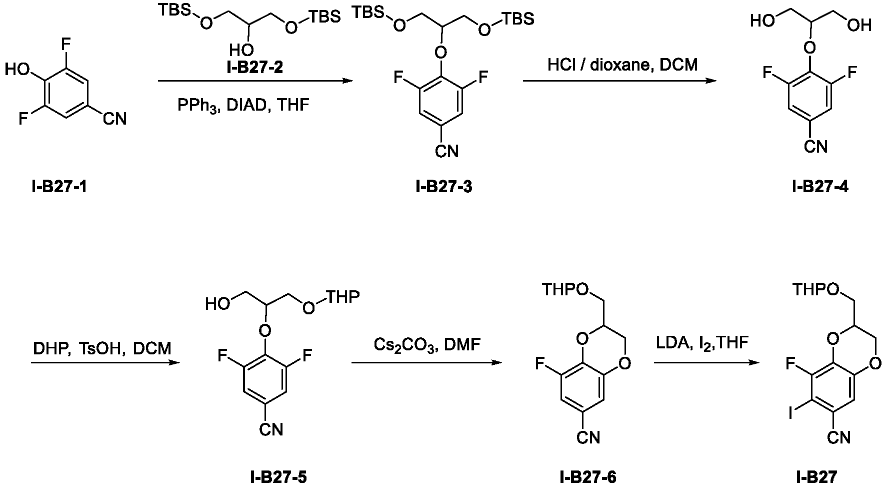

Step 1: Extra dry tetrahydrofuran (50 mL), **I-B27-1** (0.5 g, 3.33 mmol) and **I-B27-2** (1.24 g, 3.87 mmol) were sequentially added to a dry three-neck flask. Under nitrogen atmosphere, triphenylphosphine (1.27 g, 4.84 mmol) and DIAD (0.978 g, 4.84 mmol) were added thereto. The reaction mixture was stirred at 25°C for 3 hours. After the reaction was completed, the reaction mixture was diluted by adding water (100 mL) and extracted with ethyl acetate (40 mL * 2). The combined organic phases were washed with saturated brine (100 mL), dried over anhydrous sodium sulfate, filtered, and concentrated under reduced pressure. The resulting residue was purified by silica gel chromatography (0 to 15% ethyl acetate/petroleum ether) to obtain **I-B27-3** (1.476 g, yield: 100%).

Step 2: DCM (20 mL), **I-B27-3** (1.476 g, 3.33 mmol) and 1,4-dioxane hydrochloride solution (20 mL, 4.0 M) were sequentially added to a dry three-neck flask, and the reaction mixture was stirred at 25°C for 2 hours. After the reaction was completed, the reaction mixture was concentrated under reduced pressure, and the resulting residue was purified by silica gel chromatography to obtain **I-B27-4** (0.58 g, yield: 77.2%).

¹H NMR (400 MHz, CD₃OD) δ 7.31 - 7.24 (m, 2H), 4.43-4.40 (m, 1H), 3.97 - 3.96 (m, 4H).

Step 3: DCM (5 mL), **I-B27-4** (100 mg, 0.436 mmol), DHP (37 mg, 0.436 mmol) and TsOH (7.5 mg, 0.0436 mmol) were sequentially added to a dry three-neck flask, and the reaction mixture was stirred at 25°C overnight. After the reaction was completed, the reaction mixture was concentrated under reduced pressure, and the resulting residue was purified by silica gel chromatography to obtain **I-B27-5** (40 mg, yield: 29.3%).

¹H NMR (400 MHz, DMSO-*d*₆) δ 7.88-7.80 (m, 2H), 5.01-4.97 (m, 1H), 4.55-4.50 (m, 2H), 3.63-3.60 (m, 4H), 3.40-3.33 (m, 1H), 1.51-1.39 (m, 6H).

Step 4: DMF (5 mL), **I-B27-5** (100 mg, 0.319 mmol) and Cs₂CO₃ (156 mg, 0.479 mmol) were sequentially added to a dry three-neck flask, and the reaction mixture was stirred under microwave irradiation at 80°C for 0.5 hours. After the reaction was completed, the reaction mixture was diluted by adding water (20 mL) and extracted with ethyl acetate (10 mL * 2). The combined organic phases were washed with saturated brine (30 mL), dried over anhydrous sodium sulfate, filtered, and concentrated under reduced pressure. The resulting residue was purified by silica gel chromatography to obtain **I-B27-6** (40 mg, yield: 42.7%).

Step 5: After cooling a tetrahydrofuran (20 mL) solution of **I-B27-6** (0.20 g, 682 mmol) to -78°C, lithium diisopropylamide (0.68 mL, 1.36 mmol) was added thereto, and the mixture was stirred for 30 minutes to obtain a light yellow solution. The temperature was maintained at -78°C, and a tetrahydrofuran (2 mL) solution of iodine (0.260 g, 1.02 mmol) was added dropwise, then the reaction mixture first faded and then gradually turned red, stirred for 30 minutes. The reaction mixture was warmed to room temperature, added with saturated sodium sulfite solution (20 mL), and extracted with ethyl acetate (3 * 30 mL). The organic phases were combined, washed with saturated brine (50 mL), dried over anhydrous sodium sulfate, and filtered. The filtrate was concentrated to dryness under reduced pressure and purified by silica gel column chromatography to obtain intermediate **I-B27** (0.10 g, yield: 35%).

The following intermediates were synthesized from the corresponding intermediates or raw materials, using the above methods or improved methods and the methods reported in the literature.

| **Intermediate number** | **Structural formula** | **Name** | **Analytical data** |
|---|---|---|---|
| **B10** | 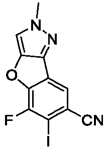 | 5-Fluoro-6-iodo-2-methyl-2*H-*benzofuro[3,2-*c*]pyrazole-7-carbonitrile | LCMS: m/z 342.1 [M+H]⁺. |
| **B12** | 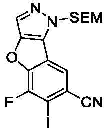 | 5-Fluoro-6-iodo-1-((2-(trimethylsilyl)ethoxy)methyl)-1*H*-benzofuro[3,2-*c*]pyrazole-7-carbonitrile | LCMS: m/z 458 [M+H]⁺. |
| **B39** | 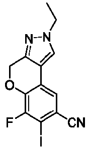 | 2-Ethyl-6-fluoro-7-iodo-2,4-dihydrochromeno[4,3 - *c*]pyrazole-8-carbonitrile | LCMS: m/z 369.9 [M+H]⁺. |
| **B71** | 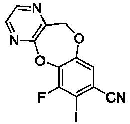 | 6-Fluoro-7-iodo-11*H-*benzo[2,3][1,4]dioxa [5,6-*b*]pyrazine-8-carbonitrile | LCMS: m/z 370 [M+H]⁺. |
| **I-B16** | 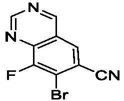 | 7-Bromo-8-fluoroquinazoline-6-carbonitrile | LCMS: m/z 252 [M+H]⁺. |
| **I-B17** | 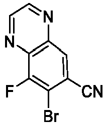 | 7-Bromo-8-fluoroquinoxaline-6-carbonitrile | LCMS: m/z 252 [M+H]⁺. |
| **I-B23** | 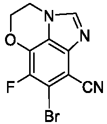 | 7-Bromo-6-fluoro-3,4-dihydro-5-oxa-1,2a-diazaacenaphthylene-8-carbonitrile | LCMS: m/z 282 [M+H]⁺. |
| **I-B25** | 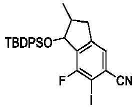 | 1*-*((*tert-*Butyldiphenylsilyl)oxy)-7-fluoro-6-iodo-2-methyl-2, 3 - dihydro-1*H*-indene-5-carbonitrile | LCMS: m/z 556 [M+H]⁺. |

### Synthesis of intermediate C1: 4,5-Dichloro-2-cyclopropylpyridazin-3(2H)-one

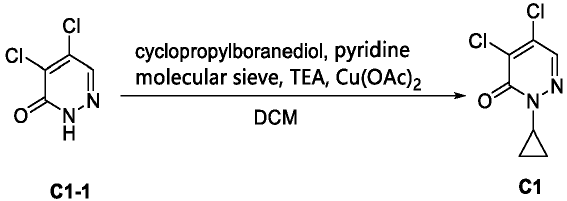

Step 1: Compound **C1-1** (100 mg, 0.606 mmol), cyclopropylboronic acid (78 mg, 0.909 mmol), pyridine (96 mg, 1.212 mmol), copper acetate (220 mg, 1.212 mmol), triethylamine (123 mg, 1.212 mmol), molecular sieve (210 mg, 1.212 mmol) and DCM (10 mL) were sequentially added to a single-neck flask. The reaction mixture was stirred at 40°C for 18 hours. The insoluble substances were filtered off with diatomite and the filtrate residue was rinsed with DCM (100 mL). The filtrate was concentrated to dryness under reduced pressure and purified by silica gel column chromatography (ethyl acetate/petroleum ether = 1/10 to 1/1) to obtain **C1** as a white solid (60 mg, yield: 48.3%).

LCMS: m/z 205 [M+H]⁺.

¹H NMR (400 MHz, CDCl₃) δ 7.72 (s, 1H), 4.12 (ddd, J = 14.4, 7.5, 4.1 Hz, 1H), 1.16 - 1.10 (m, 2H), 1.08 - 1.01 (m, 2H).

### Synthesis of intermediate C2: (5,6-Dichloropyrazin-2-yl)methanol

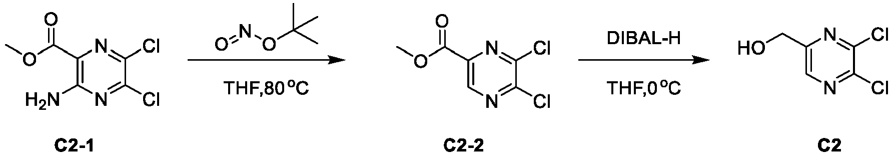

Step 1: **C2-1** (2 g, 9 mmol), THF (15 mL) and *tert*-butyl nitrite (8.6 mL, 72 mmol) were sequentially added to a single-neck flask. The reaction mixture was stirred at 80°C for 18 hours, cooled and concentrated under reduced pressure. The residue was purified by silica gel chromatography (petroleum ether/ethyl acetate = 10/1) to obtain **C2-2** (0.96 g, yield: 100%) as a colorless oil.

¹H NMR (400 MHz, CDCl₃) δ 9.00 (s, 1H), 4.05 (s, 3H).

Step 2: **C2-2** (950 mg, 4.59 mmol) and THF (15 mL) were added to a single-neck flask. The reaction mixture was cooled to 0°C, slowly added with DIBAL-H (9.2 mL, 9.18 mmol), and stirred at 0°C for 1 hour. TLC showed that the reaction was completed. Saturated potassium sodium tartrate aqueous solution was added to the reaction mixture and stirred for half an hour. The filtrate was diluted with water and extracted with ethyl acetate. The organic layer was dried over Na₂SO₄ and concentrated under reduced pressure. The residue was purified by silica gel chromatography (petroleum ether/ethyl acetate = 3/1) to obtain **C2** (620 mg, yield: 75.4%) as a yellow oil.

LC-MS: m/z 179 [M+H]⁺.

### Synthesis of intermediate C3: 5,6-Dichloro-2-cyclopropyl-3H,2H-1,2-diazin-3-one

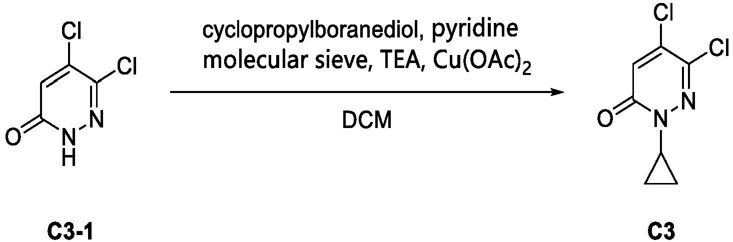

Step 1: Compound **C3-1** (200 mg, 1.21 mmol), cyclopropylboronic acid (156 mg, 1.82 mmol), pyridine (192 mg, 2.42 mmol), copper acetate (440 mg, 2.42 mmol), triethylamine (246 mg, 2.42 mmol), molecular sieve (210 mg) and DCM (15 mL) were sequentially added to a single-neck flask. The reaction mixture was stirred at 40°C for 18 hours. The insoluble substances were filtered off with diatomite and the filtrate residue was rinsed with DCM (100 mL). The filtrate was concentrated to dryness under reduced pressure and purified by silica gel column chromatography (ethyl acetate/petroleum ether, 0 to 25%) to obtain **C3** as a yellow solid (105 mg, yield: 42.2%).

¹H NMR (400 MHz, CDCl₃) δ 7.08 (s, 1H), 4.03 (ddd, J = 7.6, 4.4, 3.3 Hz, 1H), 1.17 - 1.01 (m, 4H).

### Synthesis of intermediate C4: 4,5-Dichloro-2-(methyl-d₃)pyridazin-3(2H)-one

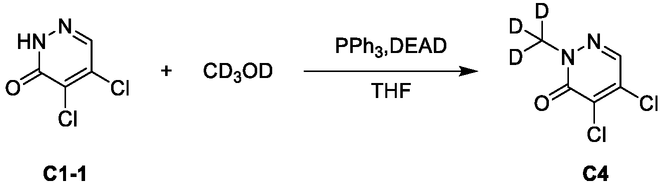

Step 1: THF (2 mL), compound **C1-1** (100 mg, 0.6 mmol), CD₃OD (24 mg, 0.67 mmol), PPh₃ (191 mg, 0.73 mmol) and DEAD (127 mg, 0.73 mmol) were sequentially added to a single-neck flask, and the reaction mixture was stirred at room temperature for 1 hour. TLC showed the formation of new dots, and the reaction mixture was subjected to rotary evaporation to dryness and column chromatography (ethyl acetate/petroleum ether, 0 to 20%) to obtain **C4** as a white solid (95 mg, 0.52 mmol, yield: 86.1%).
LCMS: m/z 182[M+H] ⁺
¹H NMR (400 MHz, CDCl₃) δ 7.77 (s, 1H).

### Synthesis of intermediate C5: 4,5-Dichloro-2-(oxetan-3-yl)pyridazin-3(2H)-one

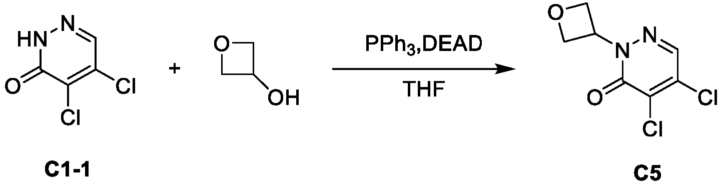

Step 1: THF (5 mL), compound **C1-1** (300 mg, 1.82 mmol), oxetan-3-ol (270 mg, 3.64 mmol), PPh₃ (954 mg, 3.64) and DEAD (127 mg, 0.73 mmol) were sequentially added to a single-neck flask, and the reaction mixture was stirred at 35°C for 1 hour. LCMS showed the formation of the target product, and the reaction mixture was subjected to rotary evaporation to dryness and column chromatography (ethyl acetate/petroleum ether, 0 to 35%) to obtain **C5** as a white solid (110 mg, 0.5 mmol, yield: 27.3%).
LCMS: m/z 221[M+H] ⁺
¹H NMR (400 MHz, CDCl₃) δ 7.94 (s, 1H), 5.97 - 5.83 (m, 1H), 5.04 - 4.93 (m, 4H).

### Example 1: Synthesis of compounds 1 and 1'

### Compound 1: 7-((2S,4S)-5-Chloro-6-fluoro-2-phenyl-2-((S)-pyrrolidin-2-yl)-2,3-dihydrobenzofuran-4-yl)-8-fluoro-2,3-dihydrobenzo[b][1,4] dioxine-6-carboxamide

### Compound 1': 7-((2S,4R)-5-Chloro-6-fluoro-2-phenyl-2-((S)-pyrrolidin-2-yl)-2,3-dihydrobenzofuran-4-yl)-8-fluoro-2,3-dihydrobenzo[b][1,4]dioxine-6-carboxamide

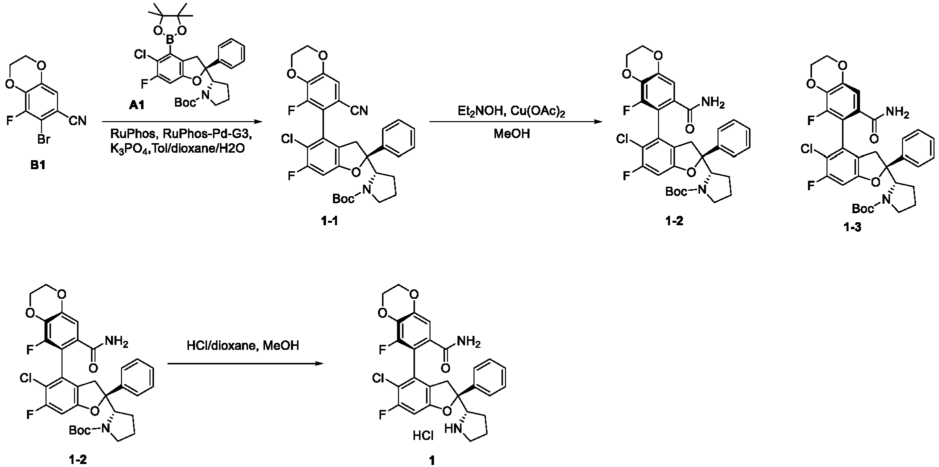

Step 1: Intermediate **A1** (3.57 g, 6.556 mmol), intermediate **B1** (2 g, 6.556 mmol), potassium phosphate (5.57 g, 26.225 mmol), toluene (30 mL), water (15 mL), 1,4-dioxane (30 mL), Pd₂(dba)₃ (0.90 g, 0.983 mmol) and *N*-XantPhos 97% (CAS: 261733-18-0, 0.72 g, 1.311 mmol) were sequentially added to a reaction flask. The reaction mixture was stirred at 110°C for 18 hours under nitrogen atmosphere. The reaction mixture was added with water and extracted with ethyl acetate. The organic phase was dried over anhydrous sodium sulfate, filtered, concentrated, and the residue was purified by silica gel chromatography (petroleum ether/ethyl acetate = 5:1) to obtain a mixture as yellow solid **1-1** (2.03 g, yield: 52.03%). LC-MS: m/z 595.1 [M+H]⁺.

Step 2: Compound **1-1** (2000 mg, 3.361 mmol), methanol (30 mL), copper acetate (1825.09 mg, 10.083 mmol) and diethylhydroxylamine (10.367 mL, 100.834 mmol) were sequentially added to a single-neck flask, and the reaction mixture was stirred at 25°C for 16 hours. A red solid precipitated. After the reaction was completed, the reaction mixture was concentrated, and the resulting residue was added with ammonia water and extracted with ethyl acetate. The organic phase was dried over anhydrous sodium sulfate, filtered, concentrated, and purified by silica gel chromatography (petroleum ether/ethyl acetate = 2:1) to obtain **1-2** (660 mg, yield: 36.03%) and **1-3** (100 mg, yield: 5.5%). LC-MS: m/z 613.2 [M+H]⁺.

Step 3: Compound **1-2** (470 mg, 0.767 mmol) was dissolved in MeOH (10 mL), and HCl/1,4-dioxane (4 M, 10 mL) was added thereto. The mixture was stirred at 20°C for 3 hours. The mixture was concentrated to obtain the hydrochloride of compound **1** (385 mg, yield: 91.41%) as a yellow solid.

¹H NMR (400 MHz, Methanol-*d₄*) δ 7.58 - 7.56 (m, 2H), 7.51 - 7.47 (m, 2H), 7.43 - 7.39 (m, 1H), 7.15 - 7.14 (m, 1H), 6.98 (d, *J* =1.2 Hz, 1H), 4.30 (s, 4H), 4.23 - 4.21 (m, 1H), 3.61 - 3.56 (m, 1H), 3.23 - 3.13 (m, 3H), 2.25 - 2.19 (m, 1H), 2.06 - 1.89 (m, 3H).

LC-MS: m/z 513.1 [M+H]⁺.

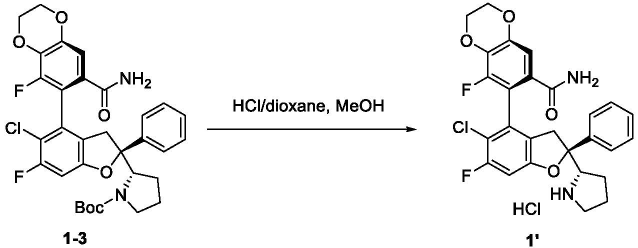

Step 4: Compound **1-3** (100 mg, 0.163 mmol) was dissolved in MeOH (5 mL), and HCl/1,4-dioxane (4 M, 5 mL) was added thereto. The mixture was stirred at 20°C for 3 hours. The mixture was concentrated to obtain the hydrochloride of compound **1'** (80 mg, yield: 89.27%) as a white solid.

¹H NMR (400 MHz, Methanol-*d₄*) δ 7.45 - 7.43 (m, 2H), 7.39 - 7.37 (m, 2H), 7.35 - 7.31 (m, 1H), 7.09 (s, 1H), 6.95 (d, *J* =1.2 Hz, 1H), 4.36 - 4.33 (m, 4H), 4.25 - 4.21 (m, 1H), 3.35 - 3.47 (m, 1H), 3.31 - 3.29 (m, 1H), 3.21 - 3.19 (m, 1H), 3.18 - 3.15 (m, 1H), 2.27 - 2.19(m, 1H), 2.05 - 2.00 (m, 2H), 1.87 - 1.81 (m, 1H).

LC-MS: m/z 513.1 [M+H]⁺.

### Example 2: Synthesis of compounds 3 and 3'

### Compound 3: (S)-7-((S)-5-Chloro-6-fluoro-2-phenyl-2-((S)-pyrrolidin-2-yl)-2,3-dihydrobenzofuran-4-yl)-6-fluoro-2-methyl-2,4-dihydrochromeno[3,4-c]pyrazole-8-carboxamide

### Compound 3': (R)-7-((S)-5-Chloro-6-fluoro-2-phenyl-2-((S)-pyrrolidin-2-yl)-2,3-dihydrobenzofuran-4-yl)-6-fluoro-2-methyl-2,4-dihydrochromeno[3,4-c]pyrazole-8-carboxamide

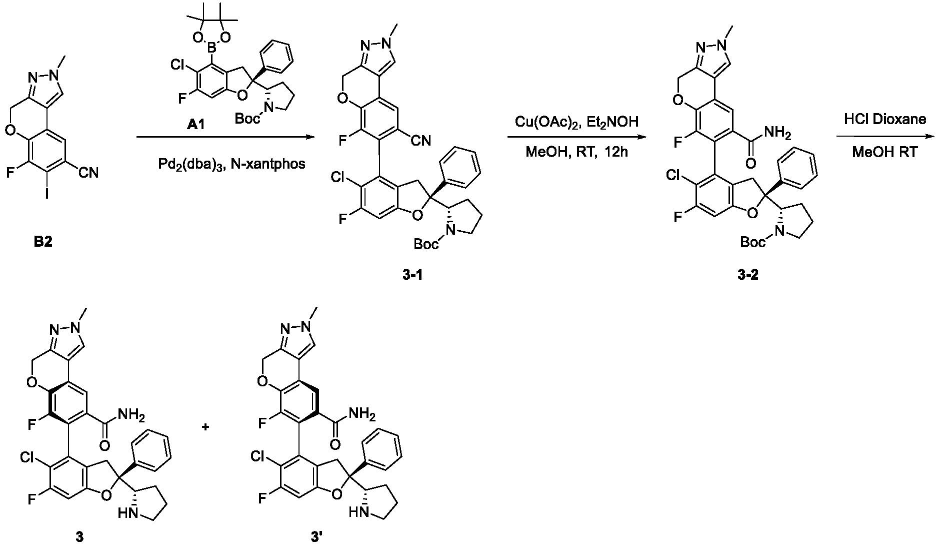

Step 1: Intermediate **B2** (90 mg, 0.25 mmol), intermediate **A1** (138 mg, 0.25 mmol), potassium phosphate (323 mg, 1.52 mmol), toluene (1 mL), 1,4-dioxane (1 mL), water (0.4 mL), Pd₂(dba)₃ (23 mg, 0.025 mmol) and *N-*XantPhos (28 mg, 0.05 mmol) were sequentially added to a reaction flask. The reaction mixture was stirred at 100°C for 12 hours under nitrogen atmosphere. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 3:1) to obtain intermediate **3-1** (90 mg, yield: 33.61%).

LC-MS: m/z 545 [M-100]⁺.

Step 2: Intermediate **3-1** (60 mg, 0.14 mmol), methanol (5 mL), copper acetate (76 mg, 0.419 mmol) and diethylhydroxylamine (313 mg, 4.185 mmol) were sequentially added to a reaction flask, and the reaction mixture was stirred at room temperature for 12 hours. After the reaction was completed, the reaction mixture was filtered and concentrated. The resulting residue was purified by thin-layer chromatography (petroleum ether/ethyl acetate = 1:1) to obtain **3-2** as a white solid (70 mg, yield: 76.1%).

LC-MS: m/z 663 [M+H]⁺.

Step 3: Intermediate **3-2** (70 mg, 0.105 mmol), methanol (1 mL) and 1,4-dioxane hydrochloride solution (1 mL, 4.0 M) were sequentially added to a reaction flask, and the reaction mixture was stirred at 25°C for 2 hours. After the reaction was completed, the reaction mixture was concentrated under reduced pressure. The residue was purified by preparative HPLC and lyophilized to obtain compound **3** as a yellow solid (18.81 mg, hydrochloride) and compound **3'** as a yellow solid (13.68 mg, hydrochloride).

Compound **3:** ¹H NMR (400 MHz, Methanol-*d₄*) δ 7.92 (s, 1H), 7.50 (d, J = 1.4 Hz, 1H), 7.44 (dt, J = 6.8, 1.3 Hz, 2H), 7.36 (t, J = 7.6 Hz, 2H), 7.31 - 7.25 (m, 1H), 6.89 (d, J = 9.1 Hz, 1H), 5.31 (s, 2H), 4.16-4.11 (m, 1H), 3.86 (s, 3H), 3.53-3.48 (m, 3H), 3.1-3.06 (m, 1H), 2.17-2.13 (m, 1H), 2.01-1.91 (m, 2H), 1.82 - 1.68 (m, 1H).

LC-MS: m/z 563 [M+H]⁺.

Compound **3':** ¹H NMR (400 MHz, Methanol-*d₄*) δ 8.03 (s, 1H), 7.61 (d*, J =* 1.4 Hz, 1H), 7.60 - 7.53 (m, 2H), 7.47 (t, *J =* 7.6 Hz, 2H), 7.43 - 7.37 (m, 1H), 7.00 (d, *J =* 9.2 Hz, 1H), 5.42 (d, *J =* 2.2 Hz, 2H), 4.24 (dd, *J =* 9.8, 7.0 Hz, 1H), 3.97 (s, 3H), 3.65 - 3.47 (m, 3H), 3.44 - 3.33 (m, 1H), 2.38 - 2.16 (m, 1H), 2.16 - 1.98 (m, 2H), 1.87 (dt, *J =* 21.2, 8.7 Hz, 1H).

LC-MS: m/z 563 [M+H]⁺.

### Example 2' Synthesis of compound 3-1: Using A26 as raw material

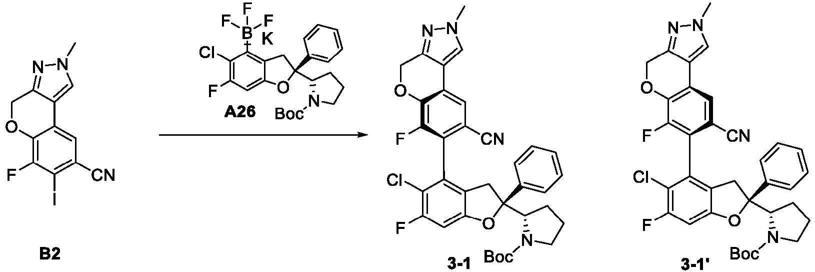

**B2** (104 g), **A26** (174.69 g, 1.15 eq), Na₂CO₃ (76.84 g, 2.5 eq), CPME (1787 mL, 20 V) and H₂O (894 mL, 10 V) were sequentially added to a three-neck flask. A CPME solution of [Pd(C₃H₃)Cl]₂ (2.12 g, 0.02 eq) and (t-Bu)PhCPhos (9.39 g, 0.08 eq) was added thereto under nitrogen atmosphere. The reaction mixture was heated to 85°C and stirred for 18 hours under nitrogen atmosphere, with a controlled conversion rate of over 95%. The reaction mixture was cooled and filtered. The organic phase was washed with water, dried and concentrated under reduced pressure to obtain 190 g of a light yellow solid product (**3-1/3-1'** = 54/46, purity: 90%, yield: 99%).

### Example 3: Synthesis of compound 9:

### (S)-7-((S)-5-Chloro-6-fluoro-2-phenyl-2-((S)-5-azaspiro[2.4]heptan-6-yl)-2,3-dihydrobenzofuran-4-yl)-6-fluoro-2-methyl-2,4-dihydrochromeno[3,4-c]pyrazole-8-carboxamide hydrochloride

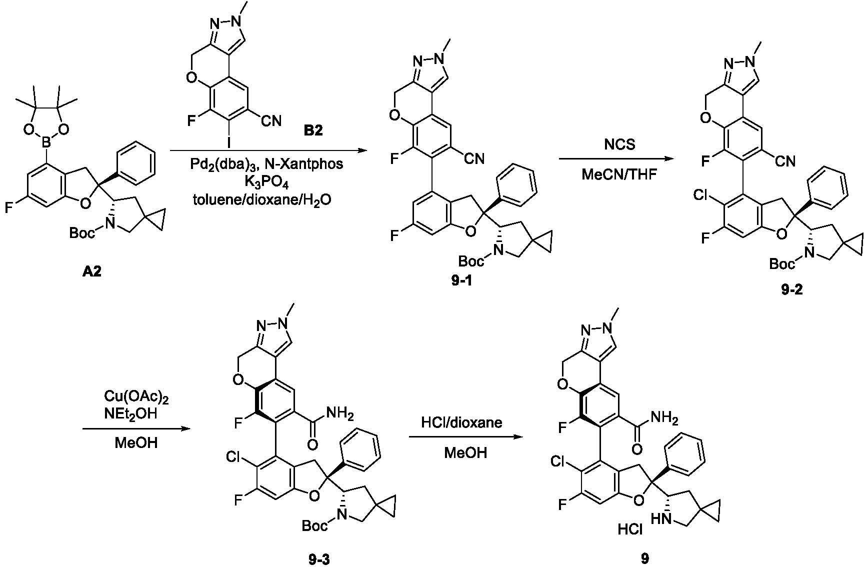

Step 1: Referring to the synthesis method of compound **1,** intermediate **A2** was used instead of intermediate **A1,** and intermediate **B2** was used instead of intermediate **B1,** and then one-step reaction was carried out to obtain intermediate **9-1.**

LC-MS: m/z 537 [M+H-100]⁺.

Step 2: Intermediate **9-1** (55 mg, 0.086 mmol), MeCN (1.6 mL), THF (0.4 mL) and NCS (13.26 mg, 0.099 mmol) were sequentially added to a pressure-resistant sealed tube, and the reaction mixture was stirred at 47°C overnight. The reaction mixture was diluted by adding water and extracted three times with ethyl acetate. The combined organic phases were washed with saturated brine, dried over anhydrous sodium sulfate, filtered, and concentrated under reduced pressure. The resulting residue was purified by silica gel chromatography (ethyl acetate/petroleum ether = 3/1) to obtain **9-2** as a yellow solid (57.5 mg, yield: 99%).

LC-MS: m/z 615 [M-55]⁺.

Step 3: Referring to the synthesis method of compound **1,** intermediate **9-2** was used instead of intermediate **1-1,** and one-step reaction was carried out to obtain intermediate **9-3.**

LC-MS: m/z 589 [M+H-100]⁺.

Step 4: Referring to the synthesis method of compound **1,** intermediate **9-3** was used instead of intermediate **1-2,** and one-step reaction was carried out to obtain compound **9.**

¹H NMR (400 MHz, Methanol-*d*₄) δ 8.03 (s, 1H), 7.63 (d, *J =* 1.3 Hz, 1H), 7.61 - 7.55 (m, 2H), 7.48 (t, *J =* 7.6 Hz, 2H), 7.41 (t, *J =* 7.3 Hz, 1H), 7.01 (d, *J =* 9.1 Hz, 1H), 5.36 (s, 2H), 4.46 (t, *J =* 8.6 Hz, 1H), 3.96 (s, 3H), 3.56 (d, *J =* 16.4 Hz, 1H), 3.24 - 3.06 (m, 3H), 2.05 (d, *J =* 8.5 Hz, 2H), 0.82 - 0.64 (m, 4H).

LC-MS: m/z 589 [M+H]⁺.

### Example 4: Synthesis of compound 10:

### (S)-8-((S)-5-Chloro-6-fluoro-2-phenyl-2-((S)-pyrrolidin-2-yl)-2,3-dihydrobenzofuran-4-yl)-9-fluorobenzo[5,6][1,4]dioxino[2,3-b]pyrazine-7-carboxamide

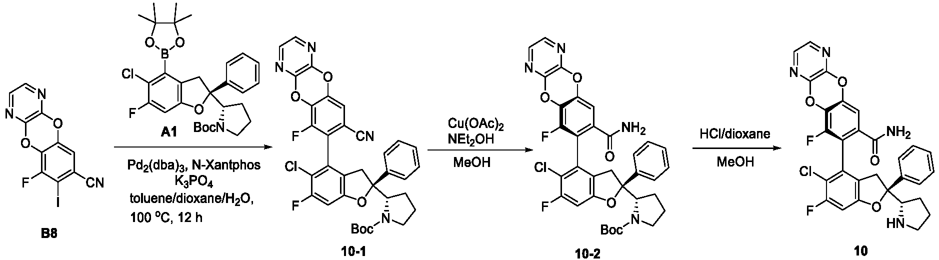

Step 1: Intermediate **B8** (390 mg, 0.717 mmol), intermediate **A1** (300 mg, 0.591 mmol), potassium phosphate (761.08 mg, 3.585 mmol), toluene (3 mL), dioxane (3 mL), water (1.2 mL), Pd₂(dba)₃ (54.72 mg, 0.060 mmol) and *N-*XantPhos (65.92 mg, 0.120 mmol) were sequentially added to a reaction flask. The reaction mixture was stirred at 100°C for 12 hours under nitrogen atmosphere. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 3:1) to obtain intermediate **10-1** (160 mg, yield: 41.51%).

LC-MS: m/z 645 [M+H]⁺.

Step 2: Intermediate **10-1** (160 mg, 0.248 mmol), methanol (5 mL), copper acetate (148.56 mg, 0.744 mmol) and diethylhydroxylamine (0.765 mL, 7.441 mmol) were sequentially added to a reaction flask, and the reaction mixture was stirred at 30°C for 1.5 hours. After the reaction was completed, the reaction mixture was filtered and concentrated. The resulting residue was purified by column chromatography (petroleum ether/ethyl acetate = 2:1) to obtain **10-2** as a white solid (60 mg, yield: 36.3%).

Step 3: Intermediate **10-2** (60 mg, 0.090 mmol), dichloromethane (2 mL) and 1,4-dioxane hydrochloride (2 mL, 4.0 M) were sequentially added to a reaction flask, and the reaction mixture was stirred at 35°C for 2 hours. After the reaction was completed, the reaction mixture was concentrated under reduced pressure. The residue was lyophilized to obtain the hydrochloride of compound **10** (26 mg, yield: 48%).

¹H NMR (400 MHz, Methanol-*d*₄) δ 7.89 (dd, *J =* 13.7, 2.8 Hz, 2H), 7.59 - 7.54 (m, 2H), 7.48 (t, *J =* 7.6 Hz, 2H), 7.42 (d, *J =* 7.3 Hz, 1H), 7.28 (d, *J =* 1.7 Hz, 1H), 7.02 (t, *J =* 6.5 Hz, 1H), 4.29 - 4.18 (m, 1H), 3.63 (dd, *J =* 22.7, 7.1 Hz, 2H), 3.25 - 3.16 (m, 2H), 2.30 - 2.21 (m, 1H), 2.05 (dd, *J =* 15.5, 7.5 Hz, 2H), 1.98 - 1.91 (m, 1H).

LC-MS: m/z 563 [M+H]⁺.

### Example 5: Synthesis of compound 71 (or 103) and compound 103 (or 71):

### Compound 71 (or 103): (S)-8-((S)-5-Chloro-6-fluoro-2-phenyl-2-((S)-pyrrolidin-2-yl)-2,3-dihydrobenzofuran-4-yl)-9-fluorobenzo[5,6][1,4]dioxo [2,3-c]pyridazine-7-carboxamide

### Compound 103 (or 71): (S)-7-((S)-5-Chloro-6-fluoro-2-phenyl-2-((S)-pyrrolidin-2-yl)-2,3-dihydrobenzofuran-4-yl)-6-fluorobenzo[5,6][1,4]dioxo [2,3-c]pyridazine-8-carboxamide

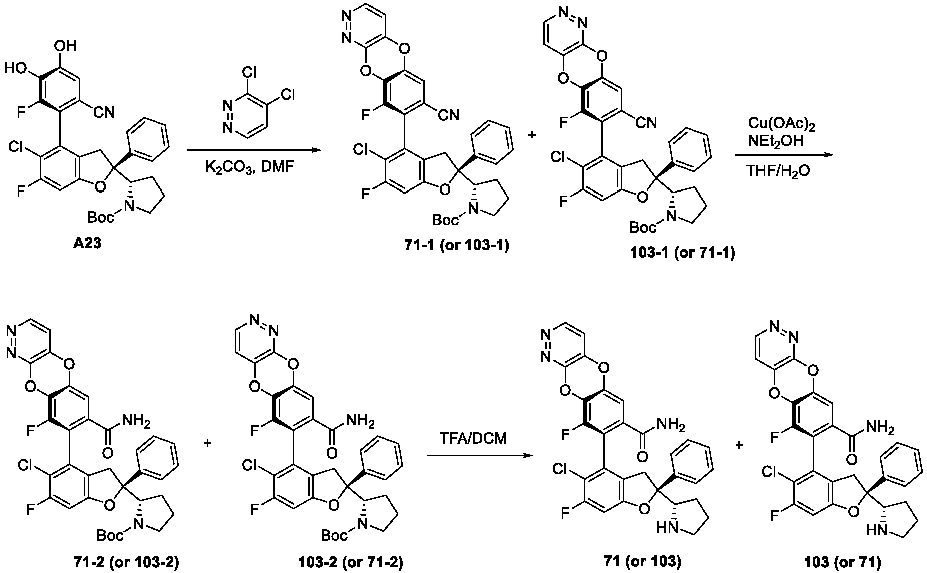

Step 1: Compound **A23** (60 mg, 0.105 mmol), 3,4-dichloropyridazine (22 mg, 0.148 mmol), DMF (1 mL) and potassium carbonate (44 mg, 0.316 mmol) were sequentially added to an 8 mL microwaveable tube, and the reaction mixture was stirred at 130°C for half an hour under microwave irradiation in a sealed tube. TLC showed that the reaction was completed. The reaction mixture was diluted by adding water and extracted three times with ethyl acetate. The combined organic phases were washed with saturated brine, dried over anhydrous sodium sulfate, filtered, and concentrated under reduced pressure. The resulting residue was purified by silica gel chromatography (PE/EA = 1:1) to obtain a mixture of **71-1 (or 103-1)** and **103-1 (or 71-1)** as a light yellow foamy solid (30 mg, yield: 44.1%).

LC-MS: m/z 589 [M-56+H]⁺.

Step 2: The mixture of **71-1 (or 103-1)** and **103-1 (or 71-1)** (30 mg, 0.047 mmol), THF (2 mL), water (0.2 mL), copper acetate (28 mg, 0.14 mmol) and diethylhydroxylamine (124.4 mg, 1.395 mmol) were sequentially added to a single-neck flask, and the reaction mixture was stirred at 30°C for 12 hours. A red solid precipitated. After the reaction was completed, the reaction mixture was filtered and concentrated. The resulting residue was purified by preparative TLC (PE/EA = 1:10) to obtain **71-2 (or 103-2)** as a light yellow solid (10 mg, yield: 32.43%) and **103-2 (or 71-2)** as a light yellow solid (5 mg, yield: 16.22%), respectively.

**71-2 (or 103-2):** LC-MS: m/z 563 [M-100+H]⁺.

**103-2 (or 71-1):** LC-MS: m/z 563 [M-100+H]⁺.

Step 3: **71-2 (or 103-2)** (7 mg, 0.011 mmol), DCM (1 mL) and TFA (0.1 mL) were sequentially added to a reaction flask, and the reaction mixture was stirred at 30°C for 1 hour. After the reaction was completed, the reaction mixture was concentrated under reduced pressure. The residue was purified by preparative HPLC and lyophilized to obtain compound **71 (or 103)** as a white solid (2 mg, hydrochloride).

**103-2 (or 71-2)** (5 mg, 0.007 mmol), DCM (1 mL) and TFA (0.1 mL) were sequentially added to a reaction flask, and the reaction mixture was stirred at 30°C for 1 hour. After the reaction was completed, the reaction mixture was concentrated under reduced pressure. The residue was purified by preparative HPLC and lyophilized to obtain compound **103 (or 71)** as a white solid (1.34 mg, hydrochloride).

Compound **71 (or 103):** ¹H NMR (400 MHz, DMSO-d₆) δ 9.31 - 9.17 (m, 1H), 8.89 - 8.80 (m, 1H), 8.39 - 8.28 (m, 1H), 8.04 - 7.94 (m, 1H), 7.58 - 7.29 (m, 6H), 7.21 - 7.13 (m, 1H), 4.38 - 4.26 (m, 1H), 3.18 - 3.06 (m, 2H), 2.25 - 2.13 (m, 1H), 2.04 - 1.67 (m, 4H), 1.54 - 1.39 (m, 1H).

LC-MS: m/z 563 [M+H]⁺.

Compound **103 (or 71):** LC-MS: m/z 563 [M+H]⁺.

### Example 6: Synthesis of compound 67:

### (S)-8-((S)-5-Chloro-6-fluoro-2-phenyl-2-((S)-pyrrolidin-2-yl)-2,3-dihydrobenzofuran-4-yl)-9-fluorobenzo[5,6][1,4]dioxo[2,3-d]pyridazine-7-carboxamide

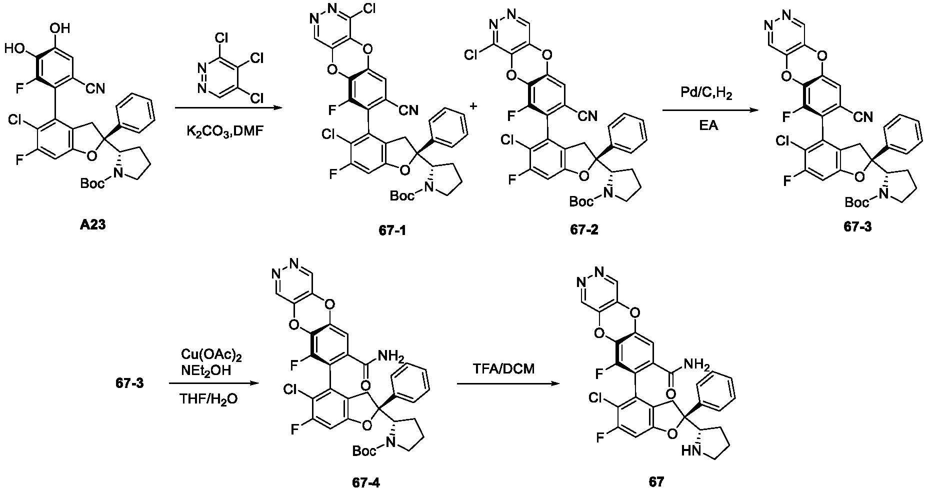

Step 1: Referring to the synthesis method of compound **71,** intermediate 3,4,5-trichloropyridazine was used instead of intermediate 3,4-dichloropyridazine, and one-step reaction was carried out to obtain intermediates **67-1** and **67-2.**

LC-MS: m/z 623 [M-56+H]⁺.

Step 2: Compound **67-1** (5 mg, 0.007 mmol), **67-2** (5 mg, 0.007 mmol), ethyl acetate (2 mL) and 10% wet palladium on carbon (3 mg, 0.03 mmol) were sequentially added to a single-neck flask, and the reaction mixture was stirred at 30°C for 18 hours under hydrogen atmosphere. Insoluble substances were filtered out with diatomite, and the filter residue was rinsed with ethyl acetate (50 mL) and concentrated to obtain **67-3** as a white solid (9 mg, yield: 95%).

LC-MS: m/z 589 [M-55+H]⁺.

Step 3: Referring to the synthesis method of compound **71,** intermediate **67-3** was used instead of intermediate **71-1,** and one-step reaction was carried out to obtain intermediate **67-4.**

LC-MS: m/z 563 [M-100+H]⁺.

Step 4: Referring to the synthesis method of compound **71,** intermediate **67-4** was used instead of intermediate **71-2,** and one-step reaction was carried out to obtain product **67.**

¹HNMR (400 MHz, DMSO-d₆) δ 9.24 (s, 1H), 9.05 (d, *J* = 6.6 Hz, 1H), 8.34 (s, 1H), 7.97 (s, 1H), 7.52 (d, *J =* 7.4 Hz, 2H), 7.45 (t, *J =* 7.5 Hz, 2H), 7.38 (t, *J =* 7.3 Hz, 1H), 7.30 (d, *J =* 1.5 Hz, 1H), 7.17 (d, *J =* 9.3 Hz, 1H), 4.30 (d, *J =* 6.6 Hz, 1H), 3.16 (s, 3H), 2.16 (s, 1H), 2.00 (d, *J* = 7.6 Hz, 1H), 1.89 (d, *J =* 28.9 Hz, 2H), 1.73 (d, *J=* 12.3 Hz, 1H).

LC-MS: m/z 563 [M+H]⁺.

### Example 7: Synthesis of compound 78:

### (S)-7-((S)-5-Chloro-6-fluoro-2-((((1R,4S)-4-methoxycyclohexyl)amino)methyl)-2-phenyl-2,3-dihydrobenzofuran-4-yl)-6-fluoro-2-methyl-2,4-dihydrochromeno[3,4-c]pyrazole-8-carboxamide

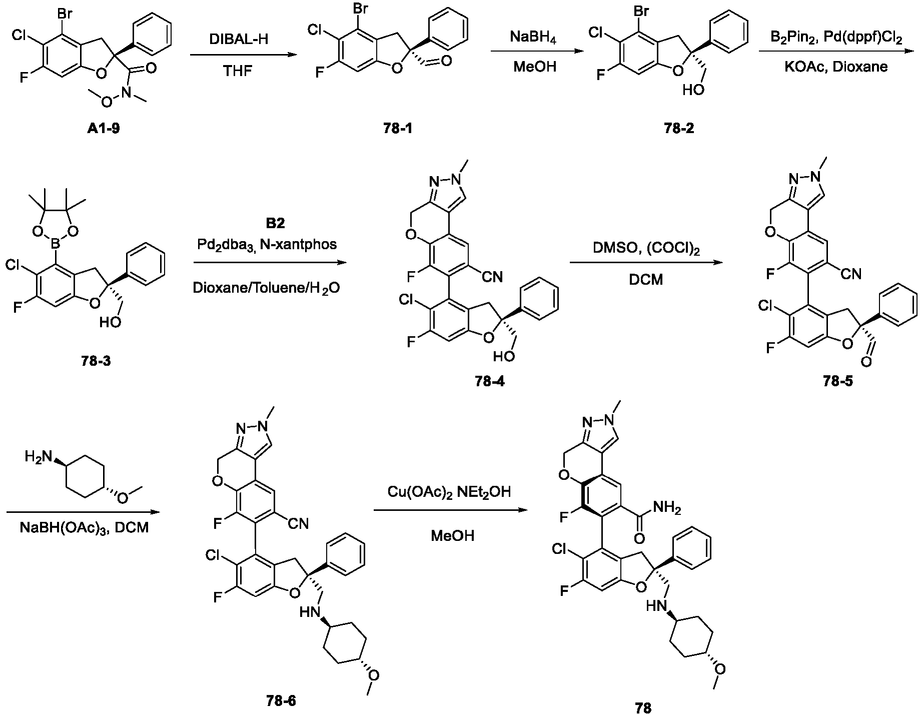

Step 1: Compound **A1-9** (4 g, 9.65 mmol) was dissolved in dry THF (40 mL), and the reaction mixture was cooled to -70°C. Dibal-H (19 mL, 19.29 mmol,! M) was slowly added dropwise, and the reaction mixture was stirred at this temperature for another 1 hour. TLC showed that the reaction was completed. The reaction mixture was slowly added with saturated sodium potassium tartrate aqueous solution (30 mL), naturally warmed to room temperature, and extracted with ethyl acetate. The combined organic phases were washed with saturated brine, dried over anhydrous sodium sulfate, filtered, and concentrated under reduced pressure to obtain yellow oily **78-1** (3.7 g, crude), which was used directly in the next step.

Step 2: Compound **78-1** (3.7 g, 10.41 mmol) was dissolved in methanol (30 mL), and sodium borohydride (0.59 g, 15.61 mmol) was slowly added under an ice bath, and then the reaction mixture was stirred at room temperature for another 2 hours. TLC showed that the reaction was completed. The reaction mixture was slowly added with ice water and extracted with ethyl acetate, then the combined organic phases were washed with saturated brine, dried over anhydrous sodium sulfate, filtered, and concentrated under reduced pressure. The resulting residue was subjected to silica gel column chromatography (0 to 30% ethyl acetate/petroleum ether) to obtain compound **78-2** (3.3 g, yield: 88.71%).

LC-MS: m/z 357 [M+H]⁺.

Step 3: Anhydrous 1,4-dioxane (35 mL), **78-2** (3.3 g, 9.23 mmol), bis(pinacolato)diboron (3.28 g, 12.92 mmol, 1.4 eq), Pd(dppf)Cl₂.CH₂Cl₂ (0.75 g, 0.92 mmol) and KOAc (2.72 g, 27.69 mmol) were sequentially added to a dry 250 mL single-neck flask, and the reaction mixture was replaced with N₂ three times and then heated to 100°C and stirred for 12 hours. TLC showed that the reaction was completed. The reaction mixture was filtered, subjected to rotary evaporation to dryness under reduced pressure, and subjected to silica gel column chromatography (0 to 30% ethyl acetate/petroleum ether) to obtain compound **78-3** (3 g, purity: 80.43%).

LC-MS: m/z 405.1 [M+H]⁺.

Step 4: Compound **B2** (600 mg, 1.95 mmol), compound **78-3** (946 mg, 2.34 mmol), potassium phosphate (1240 mg, 5.84 mmol), toluene (1 mL), dioxane (1 mL), water (0.5 mL), Pd₂(dba)₃ (178 mg, 0.195 mmol) and *N*-XantPhos (215 mg, 0.39 mmol) were sequentially added to a reaction flask. The reaction mixture was stirred at 100°C for 16 hours under nitrogen atmosphere. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (0 to 40% ethyl acetate/petroleum ether) to obtain compound **78-4** (900 mg, yield: 91%).

LC-MS: m/z 506.1 [M+H]⁺.

Step 5: At -78°C, anhydrous dichloromethane (2 mL), oxalyl chloride (376.31 mg, 2.97 mmol) and anhydrous DMSO (78.13 mg, 5.93 mmol) were sequentially added to a dry 10 mL single-neck flask, and the reaction mixture was stirred at this temperature for 1 hour. After that, a dichloromethane (2 mL) solution of **78-4** (500 mg, 0.988 mmol) and triethylamine (500 mg, 4.94 mmol) were added to the above reaction mixture. The reaction mixture was stirred for another 1 hour. The reaction mixture was quenched by adding water and extracted twice with ethyl acetate. The combined organic phases were washed with saturated brine, dried over anhydrous sodium sulfate, filtered, and concentrated under reduced pressure to obtain **78-5** (490 g, crude), which was used directly in the next step.

LC-MS: m/z 504.1[M+H]⁺.

Step 6: DCM (1 mL), **78-5** (50 mg, 0.099 mmol), *trans*-4-methoxycyclohexylamine (25 mg, 0.198 mmol) and one drop of acetic acid were sequentially added to a dry 10 mL single-neck flask. The reaction mixture was stirred at room temperature for 15 minutes, then added with NaBH(OAc)₃ (63 mg, 0.298 mmol), and stirred at room temperature overnight. The reaction solvent was subjected to rotary evaporation to dryness under reduced pressure. The reaction mixture was added with saturated NaHCO₃ aqueous solution and extracted with ethyl acetate. The combined organic phases were washed with saturated brine, dried over anhydrous sodium sulfate, filtered, and concentrated under reduced pressure. The resulting residue was purified by silica gel chromatography (0 to 80% ethyl acetate/petroleum ether) to obtain compound **78-6** (30 mg, yield: 50%).

LC-MS: m/z 617 [M+H]⁺.

Step 7: Compound **78-6** (30 mg, 0.049 mmol), methanol (5 mL), copper acetate (26.49 mg, 0.146 mmol) and diethylhydroxylamine (130 mg, 1.458 mmol) were sequentially added to a dry 10 mL single-neck flask, and the reaction mixture was stirred at room temperature for 3 hours. After the reaction was completed, the reaction mixture was filtered and concentrated. The resulting residue was purified by Prep-HPLC to obtain the formate of compound **78** (5.75 mg, yield: 18.62%).

LC-MS: m/z 635 [M+H]⁺.

### Example 8: Synthesis of compound 79:

### (S)-7-((S)-2-((((1r,4S)-4-Carbamoylcyclopropyl))methyl)-5-chloro-6-fluoro-2-phenyl-2,3-dihydrobenzofuran-4-yl)-6-fluoro-2-methyl-2,4-dihydrochromeno[3,4-c]pyrazole-8-carboxamide formate

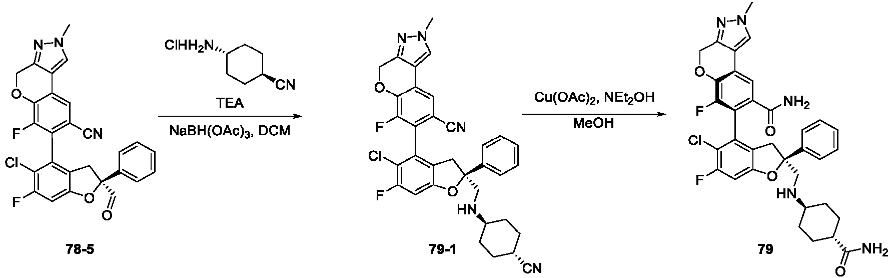

Referring to the synthesis method of compound **78**, (1r,4r)-4-aminocyclopropane-1-carbonitrile hydrochloride was used instead of *trans*-4-methoxycyclohexylamine, and a two-step reaction was carried out to obtain the formate of compound **79** (20 mg, 50%).

LC-MS: m/z 648.2 [M+H]⁺.

1H NMR (400 MHz, Methanol-*d*₄) δ 8.02 (s, 1H), 7.63 (d, J = 1.4 Hz, 1H), 7.55 - 7.32 (m, 5H), 6.94 (d, J = 9.3 Hz, 1H), 5.36 (s, 2H), 3.95 (s, 3H), 3.48 (d, J = 16.2 Hz, 1H), 3.40 (s, 1H), 3.09 (d, J = 16.0 Hz, 1H), 2.76 (s, 1H), 2.23 - 1.84 (m, 5H), 1.45 (q, J = 12.6 Hz, 2H), 1.26 (d, J = 15.7 Hz, 3H).

### Example 9: Synthesis of compound 81

### (S)-7-((S)-5-Chloro-6-fluoro-2-((((1r,4S)-4-hydroxycyclopropane)amino)methyl)-2-phenyl-2,3-dihydrobenzofuran-4-yl)-6-fluoro-2-methyl-2,4-dihydrochromeno[3,4-c]pyrazole-8-carboxamide formate

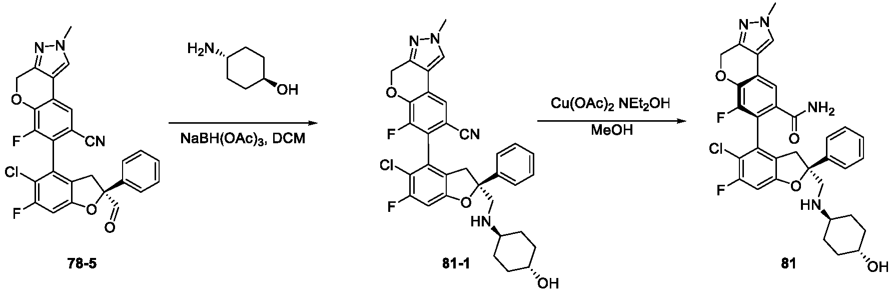

Referring to the synthesis method of compound **78**, (1*r*,4*r*)-4-aminocyclopropan-1-ol was used instead of *trans*-4-methoxycyclohexylamine, and a two-step reaction was carried out to obtain the formate of compound **81** (6 mg, formate).

LC-MS: m/z 621.1 [M+H]⁺.

¹H NMR (400 MHz, Methanol-*d*₄) δ 8.02 (s, 1H), 7.62 (d, J = 1.3 Hz, 1H), 7.48 (d, J = 7.6 Hz, 2H), 7.41 (t, J = 7.5 Hz, 2H), 7.34 (t, J = 7.1 Hz, 1H), 6.93 (d, J = 9.1 Hz, 1H), 5.36 (s, 2H), 3.95 (s, 3H), 3.47 (d, J = 15.2 Hz, 2H), 3.08 (d, J = 15.9 Hz, 1H), 2.67 (s, 1H), 1.93 (s, 4H), 1.28 - 1.16 (m, 4H).

### Example 10: Synthesis of compound 83

### (S)-7-((S)-5-Chloro-6-fluoro-2-(((3-methoxypropyl)amino)methyl)-2-phenyl-2,3-dihydrobenzofuran-4-yl)-6-fluoro-2-methyl-2,4-dihydrochromeno[3,4-c]pyrazole-8-carboxamide formate

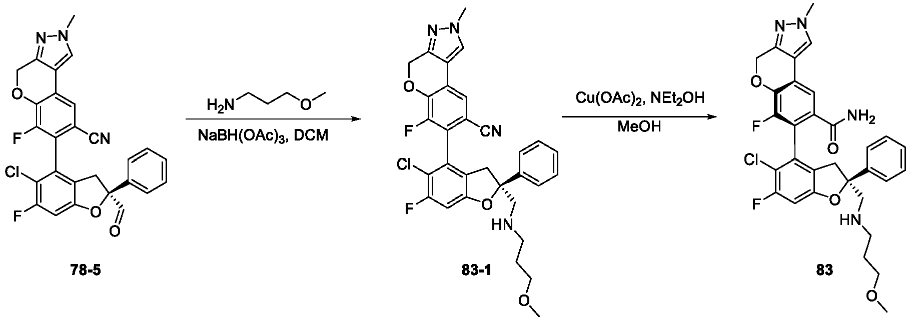

Referring to the synthesis method of compound **78**, 3-methoxypropylamine was used instead of *trans*-4-methoxycyclohexylamine, and a two-step reaction was carried out to obtain the formate of compound **83** (30 mg, yield: 50%).

LC-MS: m/z 595 [M+H]⁺.

¹H NMR (400 MHz, Methanol-*d*₄) δ 8.02 (s, 1H), 7.63 (d, J = 1.4 Hz, 1H), 7.51 - 7.41 (m, 4H), 7.36 (t, J = 7.3 Hz, 1H), 6.95 (d, J = 9.3 Hz, 1H), 5.36 (s, 2H), 3.95 (s, 4H), 3.48 (d, J = 15.6 Hz, 1H), 3.40 (t, J = 5.7 Hz, 4H), 3.19 (s, 3H), 3.11 (d, J = 16.2 Hz, 1H), 2.92 (d, J = 27.4 Hz, 2H), 1.86 - 1.71 (m, 2H).

### Example 11: Synthesis of compound 84

### (S)-7-((S)-5-Chloro-6-fluoro-2-(((((1r,3S)-3-methoxycyclobutyl)methyl)amino)methyl)-2-phenyl-2,3-dihydrobenzofuran-4-yl)-6-fluoro-2-methyl-2,4-dihydrochromeno[3,4-c]pyrazole-8-carboxamide formate

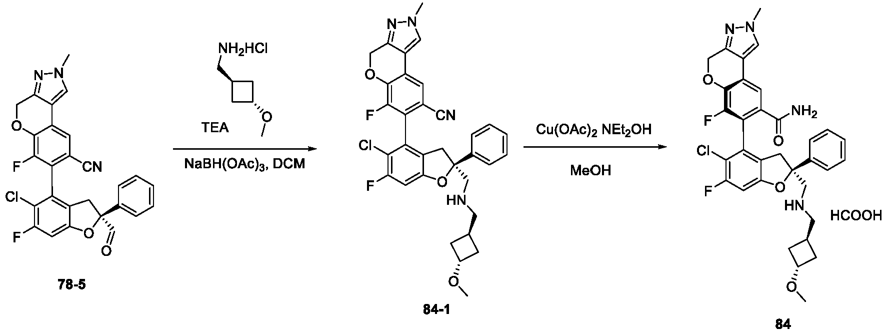

Referring to the synthesis method of compound **78**, *trans*-3-methoxycyclobutyl-1-methanamine hydrochloride was used instead of *trans*-4-methoxycyclohexylamine, and a two-step reaction was carried out to obtain the formate of compound **84** (6.58 mg, yield: 12.78%).

### I. LC-MS: m/z 621 [M+H]⁺.

### Example 12: Synthesis of compound 105 and compound 106:

### Compound 105: (S)-8-((S)-5-Chloro-6-fluoro-2-phenyl-2-((S)-pyrrolidin-2-yl)-2,3-dihydrobenzofuran-4-yl)-9-fluoro-2-methyl-1-oxo-1,2-dihydrobenzo[5,6][1,4]dioxo [2,3-d]pyridazine-7-carboxamide

### Compound 106: (S)-8-((S)-5-Chloro-6-fluoro-2-phenyl-2-((S)-pyrrolidin-2-yl)-2,3-dihydrobenzofuran-4-yl)-9-fluoro-3-methyl-4-oxo-3,4-dihydrobenzo[5,6][1,4]dioxo [2,3-d]pyridazine-7-carboxamide

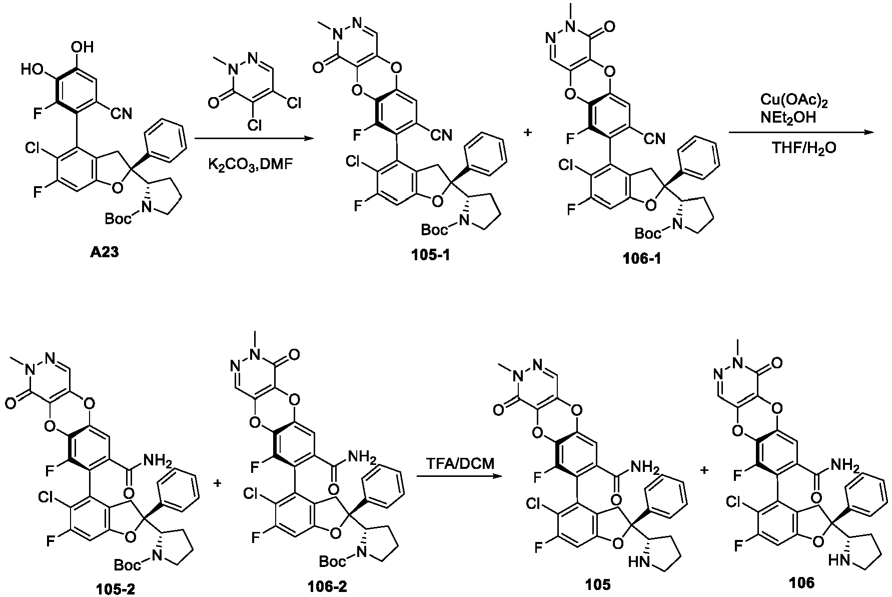

Step 1: Referring to the synthesis method of compound **71,** intermediate 4,5-dichloro-2-methylpyridazin-3-one was used instead of intermediate 3,4-dichloropyridazine, and one-step reaction was carried out to obtain intermediates **105-1** and **106-1** (mixture).

LC-MS: m/z 619 [M-56+H]⁺.

Step 2: Referring to the synthesis method of compound **71,** intermediates **105-1** and **106-**1 (mixture) were used instead of intermediate **71-1,** and one-step reaction was carried out to obtain intermediates **105-2** and **106-2.**

LC-MS: m/z 593 [M-100+H]⁺.

Step 3: Referring to the synthesis method of compound **71,** intermediates **105-2** and **106-2** were used instead of intermediate **71-2,** and one-step reaction was carried out to obtain compounds **105** and **106.**

Compound **105,** LC-MS: m/z 593 [M+H]⁺.

¹H NMR (400 MHz, DMSO-d₆) δ 9.31 (s, 1H), 8.35 (s, 1H), 7.98 (s, 1H), 7.96 (s, 1H), 7.53 (d, *J =* 7.2 Hz, 3H), 7.45 (t, *J =* 7.5 Hz, 2H), 7.39 (dd, *J =* 13.4, 6.2 Hz, 1H), 7.23 (d, *J =* 1.5 Hz, 1H), 7.16 (d, *J* = 9.3 Hz, 1H), 4.30 (s, 1H), 3.63 (s, 3H), 3.59 (d, *J =* 17.0 Hz, 1H), 3.08 (dd, J = 32.0, 17.8 Hz, 3H), 2.21 - 2.11 (m, 1H), 1.95 - 1.80 (m, 2H), 1.72 (dt, *J =* 17.4, 8.3 Hz, 1H).

Compound **106**, LC-MS: m/z 593 [M+H]⁺.

¹H NMR (400 MHz, DMSO-*d*₆) δ 9.03 (s, 1H), 8.33 (s, 1H), 7.98 (s, 1H), 7.95 (s, 1H), 7.53 (d, J = 7.6 Hz, 2H), 7.46 (t, J = 7.5 Hz, 2H), 7.40 (d, J = 8.6 Hz, 2H), 7.26 (dd, J = 8.5, 2.3 Hz, 1H), 7.16 (d, J = 9.3 Hz, 1H), 4.30 (s, 1H), 3.64 (d, J = 5.4 Hz, 3H), 3.58 (d, J = 17.1 Hz, 1H), 3.12 (d, J = 43.2 Hz, 3H), 2.17 (d, J = 6.7 Hz, 1H), 1.87 (dd, J = 17.1, 9.3 Hz, 2H), 1.73 (dd, J = 22.1, 9.3 Hz, 1H).

### Example 13: Synthesis of compound 111 and compound 112:

### Compound 111: (S)-8-((S)-5-Chloro-6-fluoro-2-phenyl-2-((S)-pyrrolidin-2-yl)-2,3-dihydrobenzofuran-4-yl)-2-cyclopropyl-9-fluoro-1-oxo-1,2-dihydrobenzo[5,6][1,4]dioxo[2,3-d]pyridazine-7-carboxamide

### Compound 112: (S)-8-((S)-5-Chloro-6-fluoro-2-phenyl-2-((S)-pyrrolidin-2-yl)-2,3-dihydrobenzofuran-4-yl)-3-cyclopropyl-9-fluoro-4-oxo-3,4-dihydrobenzo[5,6][1,4]dioxo[2,3-d]pyridazine-7-carboxamide

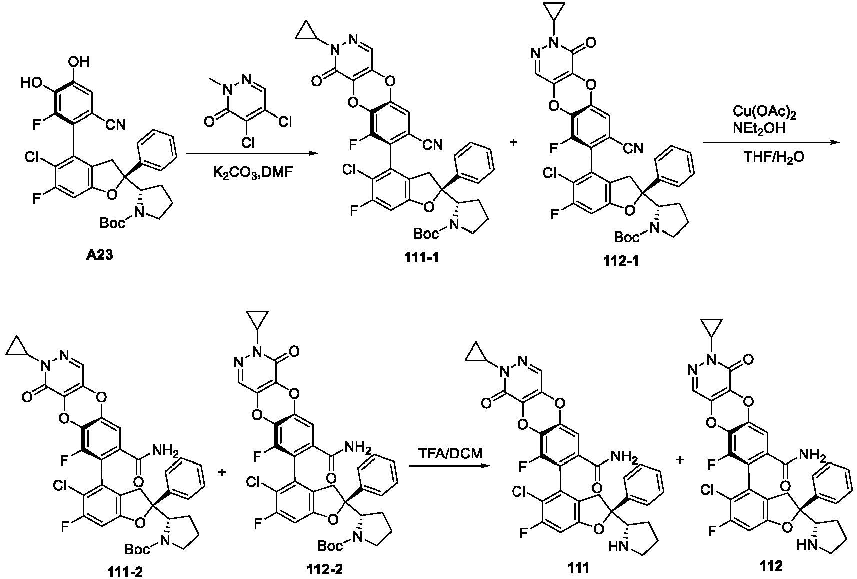

Step 1: Referring to the synthesis method of compound **71,** intermediate **C1** was used instead of intermediate 3,4-dichloropyridazine, and one-step reaction was carried out to obtain intermediates **111-1** and **112-1** (mixture).

LC-MS: m/z 645 [M-56+H]⁺.

Step 2: Referring to the synthesis method of compound **71,** intermediates **111-1** and **112-**1 (mixture) were used instead of intermediate **71-1,** and one-step reaction was carried out to obtain intermediates **111-2** and **112-2.**

LC-MS: m/z 619 [M-100+H]⁺.

Step 3: Referring to the synthesis method of compound **71,** intermediates **111-2** and **112-2** were used instead of intermediate **71-2,** and one-step reaction was carried out to obtain compounds **111** and **112.**

**Compound 111:** LC-MS: m/z 619 [M+H]⁺.

¹H NMR (400 MHz, DMSO-d₆) δ 8.99 (s, 1H), 8.33 (s, 1H), 7.96 (s, 1H), 7.95 (s, 1H), 7.53 (d, *J =* 7.3 Hz, 2H), 7.46 (t, *J =* 7.6 Hz, 2H), 7.39 (t, *J =* 9.2 Hz, 2H), 7.22 (d, *J =* 1.6 Hz, 1H), 7.17 (d, *J* = 9.3 Hz, 1H), 4.29 (s, 1H), 3.95 (dd, *J =* 11.9, 6.0 Hz, 1H), 3.58 (d, *J =* 16.9 Hz, 1H), 3.09 (t, *J =* 26.7 Hz, 3H), 2.18 (s, 1H), 1.87 (dd, *J =* 17.4, 9.6 Hz, 2H), 1.77 - 1.67 (m, 1H), 0.96 (d, *J = 7.1* Hz, 4H).

**Compound 112:** LC-MS: m/z 619 [M+H]⁺.

¹H NMR (400 MHz, DMSO-*d*₆) δ 9.00 (s, 1H), 8.32 (s, 1H), 7.95 (s, 1H), 7.94 (s, 1H), 7.55 - 7.51 (m, 2H), 7.46 (t, J = 7.5 Hz, 2H), 7.42 - 7.35 (m, 2H), 7.32 (d, J = 1.6 Hz, 1H), 7.16 (d, J = 9.3 Hz, 1H), 4.29 (s, 1H), 3.98 (dt, J = 11.5, 5.8 Hz, 1H), 3.58 (d, J = 16.3 Hz, 1H), 3.08 (t, J = 29.8 Hz, 3H), 2.17 (d, J = 7.5 Hz, 1H), 2.00 - 1.85 (m, 3H), 1.75 - 1.67 (m, 1H), 0.97 (d, J = 5.7 Hz, 4H).

### Example 14: Synthesis of compound 115:

### (S)-7-((S)-5-Chloro-6-fluoro-2-((S)-5-oxopyrrolidin-2-yl)-2-phenyl-2,3-dihydrobenzofuran-4-yl)-6-fluoro-2-methyl-2,4-dihydrochromeno[3,4-c]pyrazole-8-carboxamide

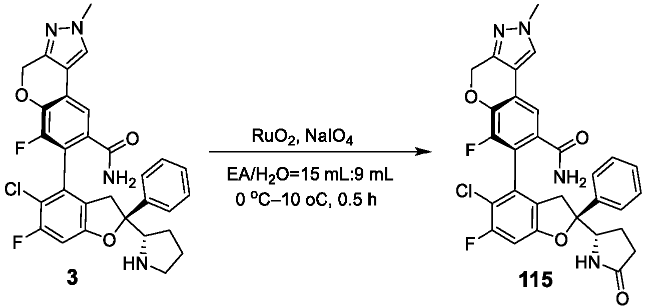

**3** (150 mg, 266.43 µmol) was added to EA/H₂O = 15 mL: 9 mL (24 mL), cooled to about 0°C, then added with solid materials RuO₂ (28.36 mg, 213.15 µmol) and NaIO₄ (227.95 mg, 1.07 mmol), and stirred for 20 minutes. TCL (EA/MeOH = 100:5) confirmed the complete reaction of raw materials, and a sample was sent for testing. After 30 minutes, EA (100 mL) and water (30 mL) were added to separate most of the aqueous phase and filtered with diatomite. The organic phase was washed with water and brine, subjected to rotary evaporation to dryness, and purified by TCL (EA/MeOH = 100:7) to obtain a crude product. Then the crude product was added with acetonitrile to dissolve and sent to preparative purification to obtain **115** as a white solid (35.00 mg, 60.66 µmol, yield: 22.77%).

¹HNMR (400 MHz, DMSO-*d*₆) δ 8.16 (s, 1H), 7.67 (s, 1H), 7.63 (s, 1H), 7.58 (d, J = 1.1 Hz, 1H), 7.37 - 7.23 (m, 5H), 7.08 (d, J = 9.5 Hz, 1H), 5.36 (s, 2H), 3.97 (dd, J = 8.5, 4.0 Hz, 1H), 3.88 (s, 3H), 3.50 - 3.45 (m, 1H), 2.96 - 2.89 (m, 1H), 2.03 - 1.94 (m, 1H), 1.91 - 1.77 (m, 2H), 1.58 - 1.44 (m, 1H).

LCMS: m/z 577.2 [M+H]⁺.

### Example 15: Synthesis of compound 118 and compound 119

### Compound 118: (S)-8-((S)-5-Chloro-6-fluoro-2-phenyl-2-((S)-pyrrolidin-2-yl)-2,3-dihydrobenzofuran-4-yl)-9-fluoro-2-(2-fluoroethyl)-1-oxo-1,2-dihydrobenzo[5,6][1,4]dioxo[2,3-d]pyridazine-7-carboxamide

### Compound 119: (S)-8-((S)-5-Chloro-6-fluoro-2-phenyl-2-((S)-pyrrolidin-2-yl)-2,3-dihydrobenzofuran-4-yl)-9-fluoro-3-(2-fluoroethyl)-4-oxo-3,4-dihydrobenzo[5,6][1,4]dioxo[2,3-d]pyridazine-7-carboxamide

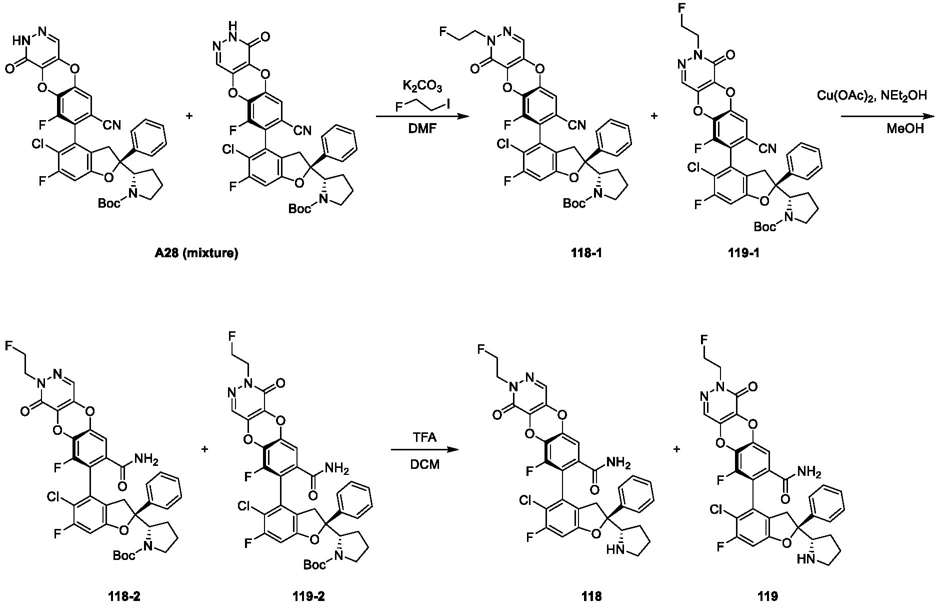

Step 1: 1-Fluoro-2-iodoethane (10 mg, 0.054 mmol) and K₂CO₃ (13 mg, 0.09 mmol) were added to a DMA (0.5 mL) solution of **A28** (30 mg, 0.045 mmol, mixture) at room temperature, and the reaction mixture was reacted at room temperature for 2 hours. TLC plate showed that the reaction was completed. The reaction mixture was diluted by adding water and extracted twice with ethyl acetate. The combined organic phases were washed with saturated brine and concentrated under reduced pressure. The resulting residue was purified by silica gel chromatography (petroleum ether/ethyl acetate = 3:1) to obtain **118-1** and **119-1** as a white solid (28 mg, mixture, yield: 87.2%).

LC-MS: m/z 707 [M+H]⁺.

Step 2: Referring to step 2 of example **5,** compounds **118-1** and **119-1** (28 mg, mixture) were used instead of **71-1** for reaction to obtain compounds **118-2** (12 mg, 41.8%) and **119-2** (6 mg, 20.9%).

LC-MS: m/z 725 [M+H]⁺.

Step 6: Referring to step 3 of example **5,** compounds **118-2** and **119-2** were used instead **of 71-2** for reaction to obtain compound **118** and compound **119,** respectively.

Compound **118,** LC-MS: m/z 625 [M+H]⁺.

¹H NMR (400 MHz, Methanol-*d₄*) δ 7.89 (s, 1H), 7.59 - 7.50 (m, 2H), 7.48 - 7.34 (m, 3H), 7.18 (d, J = 1.8 Hz, 1H), 7.02 (d, J = 9.1 Hz, 1H), 4.82 (t, J = 4.9 Hz, 1H), 4.70 (t, J = 4.9 Hz, 1H), 4.44 (ddd, J = 25.2, 5.6, 4.2 Hz, 2H), 4.25 (dd, J = 9.6, 7.2 Hz, 1H), 3.69 - 3.60 (m, 1H), 3.38 - 3.32 (m, 1H), 3.25 - 3.14 (m, 2H), 2.25 (dt, J = 12.4, 6.1 Hz, 1H), 2.03 (dt, J = 13.7, 7.1 Hz, 2H), 1.92 (dt, J = 12.8, 9.0 Hz, 1H).

Compound **119,** LC-MS: m/z 625 [M+H]⁺.

¹H NMR (400 MHz, Methanol-*d₄*) δ 7.86 (s, 1H), 7.59-7.53 (m, 2H), 7.51-7.45 (m, 2H), 7.41 (d, J = 7.3 Hz, 1H), 7.23 (d, J = 1.8 Hz, 1H), 7.03 (d, J = 9.1 Hz, 1H), 4.83 (t, J = 4.9 Hz, 1H), 4.72 (t, J = 4.9 Hz, 1H), 4.46 (dq, J = 25.3, 4.3 Hz, 2H), 4.26 (dd, J = 9.4, 7.3 Hz, 1H), 3.63 (d, J = 16.6 Hz, 1H), 3.35 (d, J = 7.0 Hz, 1H), 3.19 (d, J = 15.9 Hz, 2H), 2.30 - 2.21 (m, 1H), 2.03 (dt, J = 13.5, 5.0 Hz, 2H), 1.97 - 1.89 (m, 1H).

### Example 16: Synthesis of compound 120 and compound 121

### Compound 120: (S)-8-((S)-5-Chloro-6-fluoro-2-phenyl-2-((S)-pyrrolidin-2-yl)-2,3-dihydrobenzofuran-4-yl)-9-fluoro-2-(2,2-difluoroethyl)-1-oxo-1,2-dihydrobenzo[5,6][1,4]dioxo[2,3-d]pyridazine-7-carboxamide

### Compound 121: (S)-8-((S)-5-Chloro-6-fluoro-2-phenyl-2-((S)-pyrrolidin-2-yl)-2,3-dihydrobenzofuran-4-yl)-9-fluoro-3-(2,2-difluoroethyl)-4-oxo-3,4-dihydrobenzo[5,6][1,4]dioxo[2,3-d]pyridazine-7-carboxamide

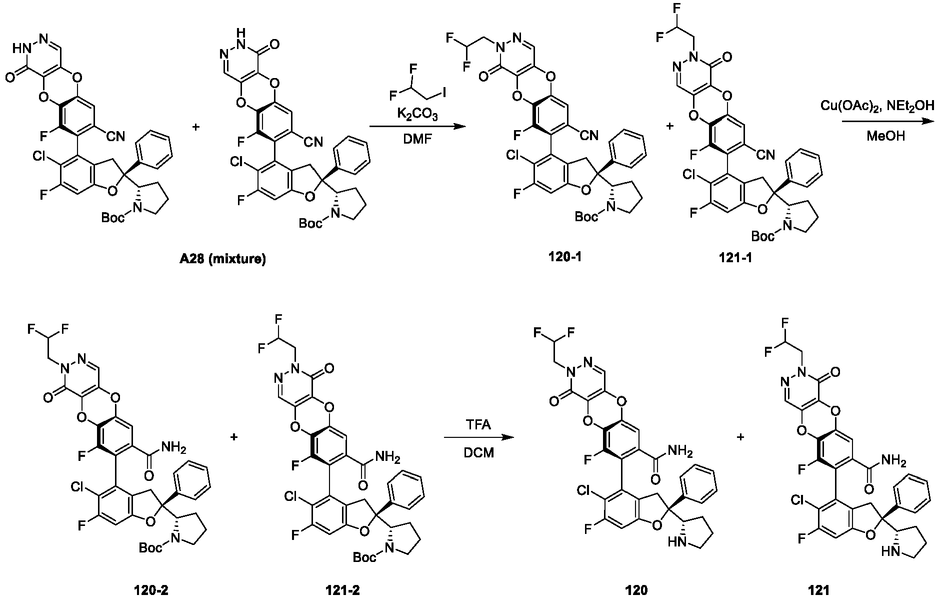

Referring to example **15,** compound 1,1-difluoro-2-iodoethane was used instead of 1-fluoro-2-iodoethane for reaction to obtain compounds **120** and **121.**

Compound **120,** LC-MS: m/z 643 [M+H]⁺.

¹H NMR (400 MHz, Methanol-*d₄*) δ 7.90 (s, 1H), 7.58 - 7.50 (m, 2H), 7.48 - 7.34 (m, 3H), 7.18 (d, J = 1.7 Hz, 1H), 7.03 (d, J = 9.1 Hz, 1H), 6.40 - 6.05 (m, 1H), 4.51 (td, J = 13.7, 4.3 Hz, 2H), 4.25 (dd, J = 9.6, 7.2 Hz, 1H), 3.64 (d, J = 16.5 Hz, 1H), 3.35 (d, J = 7.4 Hz, 1H), 3.19 (t, J = 12.3 Hz, 2H), 2.26 (q, J = 5.2 Hz, 1H), 2.03 (dt, J = 13.6, 7.3 Hz, 2H), 1.97 - 1.86 (m, 1H).

Compound **121,** LC-MS: m/z 643 [M+H]⁺.

¹H NMR (400 MHz, Methanol-*d*₄) δ 7.87 (s, 1H), 7.60 - 7.53 (m, 2H), 7.52 - 7.38 (m, 3H), 7.23 (d, J = 1.9 Hz, 1H), 7.03 (d, J = 9.1 Hz, 1H), 6.24 (tt, J = 55.6, 4.3 Hz, 1H), 4.58 - 4.47 (m, 2H), 4.26 (dd, J = 9.5, 7.3 Hz, 1H), 3.63 (d, J = 16.5 Hz, 1H), 3.35 (d, J = 7.0 Hz, 1H), 3.25 - 3.14 (m, 2H), 2.29 - 2.21 (m, 1H), 2.04 (p, J = 7.1, 6.2 Hz, 2H), 1.92 (dd, J = 12.7, 8.8 Hz, 1H).

### Example 17: Synthesis of compound 124 and compound 125:

### Compound 124: (S)-8-((S)-5-Chloro-6-fluoro-2-phenyl-2-((S)-pyrrolidin-2-yl)-2,3-dihydrobenzofuran-4-yl)-9-fluoro-N,2-dimethyl-1-oxo-1,2-dihydrobenzo[5,6][1,4]dioxo[2,3-d]pyridazine-7-carboxamide

### Compound 125: (S)-8-((S)-5-Chloro-6-fluoro-2-phenyl-2-((S)-pyrrolidin-2-yl)-2,3-dihydrobenzofuran-4-yl)-9-fluoro-N,3-dimethyl-4-oxo-3,4-dihydroxybenzo[5,6][1,4]dioxo[2,3-d]pyridazine-7-carboxamide

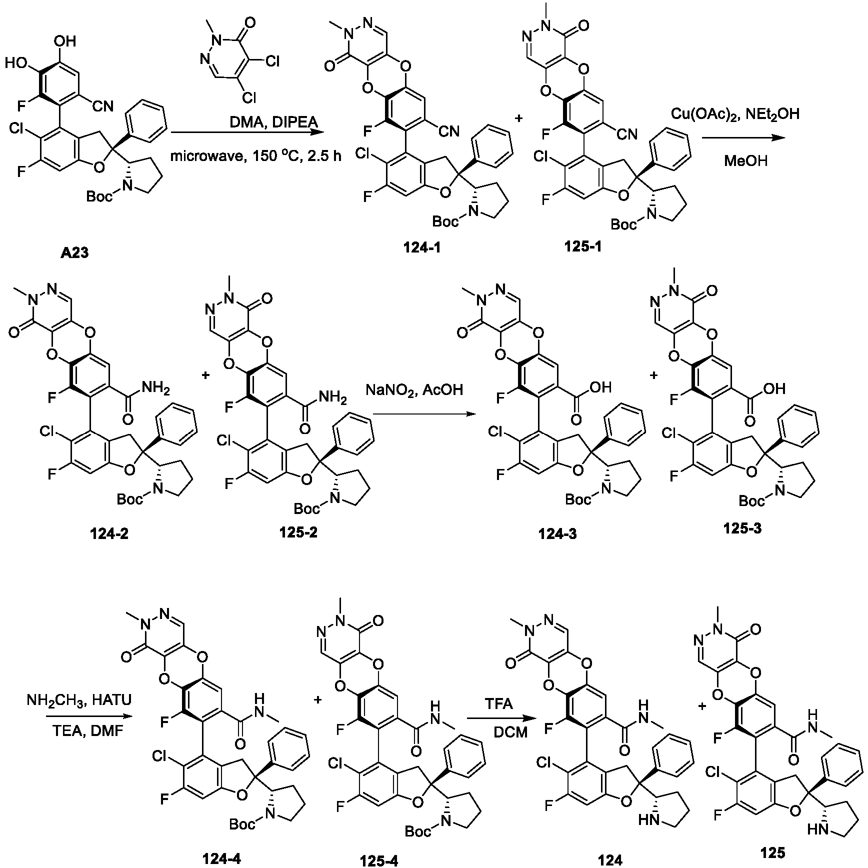

Step 1: Referring to the synthesis method of compound **71,** intermediate 4,5-dichloro-2-methylpyridazin-3-one was used instead of intermediate 3,4-dichloropyridazine, and one-step reaction was carried out to obtain intermediates **124-1** and **125-1** (mixture).

LC-MS: m/z 619 [M-56+H]⁺.

Step 2: Referring to the synthesis method of compound **71,** intermediates **124-1** and **125-1** (mixture) were used instead of intermediate **71-1,** and one-step reaction was carried out to obtain intermediates **124-2** and **125-2.**

LC-MS: m/z 593 [M-100+H]⁺.

Step 3: Referring to the synthesis method of compound **I-26,** intermediates **124-2** and 125-2 (mixture) were used instead of intermediate **I-26-2**, and one-step reaction was carried out to obtain intermediates **124-3** and **125-3.**

LC-MS: m/z 594 [M-100+H]⁺.

Step 4: Referring to the synthesis method of compound **I-26,** intermediates **124-3** and 125-3 (mixture) were used instead of intermediate **I-26-3,** and one-step reaction was carried out to obtain intermediates **124-4** and **125-4.**

LC-MS: m/z 607 [M-100+H]⁺.

Step 5: Referring to the synthesis method of compound **I-26,** intermediates **124-4** and **125-4** were used instead of intermediate **I-26-4,** and one-step reaction was carried out to obtain compounds **124** and **125.**

Compound **124:** ¹H NMR (400 MHz, DMSO-d₆) δ 9.42 (s, 1H), 8.46 (d, *J* = 4.5 Hz, 1H), 8.40 (s, 1H), 7.98 (s, 1H), 7.56 - 7.36 (m, 5H), 7.22 (s, 1H), 7.16 (d, *J =* 9.3 Hz, 1H), 4.31 (s, 1H), 3.62 (s, 3H), 3.04 (d, *J =* 17.0 Hz, 4H), 2.63 (d, *J =* 4.2 Hz, 3H), 2.09 (s, 1H), 1.96 - 1.83 (m, 2H), 1.74 (d, *J =* 8.5 Hz, 1H).

LC-MS: m/z 607 [M+H]⁺.

Compound **125:** ¹H NMR (400 MHz, DMSO-d₆) δ 9.27 (s, 1H), 8.44 (s, 1H), 8.35 (s, 1H), 7.98 (d, *J =* 4.9 Hz, 1H), 7.52 (d, *J =* 7.7 Hz, 2H), 7.45 (t, *J =* 7.5 Hz, 2H), 7.41 - 7.36 (m, 1H), 7.24 (d, *J =* 31.1 Hz, 1H), 7.19 - 7.14 (m, 1H), 4.31 (s, 1H), 3.64 (s, 3H), 3.23 - 2.97 (m, 4H), 2.63 (d, *J* = 4.6 Hz, 3H), 2.10 (s, 1H), 1.84 (s, 2H), 1.69 (s, 1H).

LC-MS: m/z 607 [M+H]⁺.

### Example 18: Synthesis of compound 126 and compound 127:

### Compound 126: (S)-7-((S)-5-Chloro-6-fluoro-2-phenyl-2-((S)-pyrrolidin-2-yl)-2,3-dihydrobenzofuran-4-yl)-6-fluoro-3-methyl-4-oxo-3,4,7,8-tetrahydrobenzo[5,6][1,4]dioxo[2,3-d]pyrimidine-8-carboxamide

### Compound 127: (S)-8-((S)-5-Chloro-6-fluoro-2-phenyl-2-((S)-pyrrolidin-2-yl)-2,3-dihydrobenzofuran-4-yl)-9-fluoro-3-methyl-4-oxo-3,4-dihydrobenzo[5,6][1,4]dioxo [2,3-d]pyrimidine-7-carboxamide

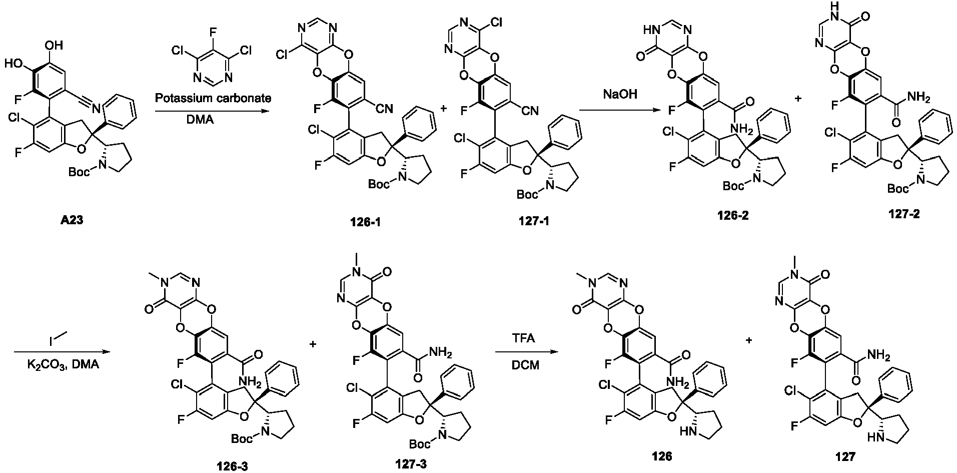

Step 1: Referring to the synthesis method of compound **71,** intermediate 4,6-dichloro-5-fluoropyrimidine was used instead of intermediate 3,4-dichloropyridazine, and one-step reaction was carried out to obtain intermediates **126-1** and **127-1** (mixture).

LC-MS: m/z 623 [M-56+H]⁺.

Step 2: Compounds **126-1** and **127-1** (mixture) (70 mg, 0.103 mmol), sodium hydroxide solution (1 mL, 2 mol/L) and dioxane (1 mL) were sequentially added to an 8 mL microwave tube, and the reaction mixture was stirred at 135°C for half an hour under microwave irradiation in a sealed tube. TLC showed that the reaction was completed. The reaction mixture was diluted by adding water and extracted three times with ethyl acetate. The combined organic phases were washed with saturated brine, dried over anhydrous sodium sulfate, filtered, and concentrated under reduced pressure. The resulting residue was purified by silica gel chromatography (PE/EA =1:1) to obtain **126-2** (15 mg, yield: 21.5%) and **127-2** (30 mg, yield: 43%) as a light yellow foamy solid.

LC-MS: m/z 579 [M-100+H]⁺.

Step 3: Referring to the synthesis method of compounds **118** and **119,** intermediates **126-2** and **127-2** were used instead of intermediates **118-3** and **119-3,** and intermediate iodomethane was used instead of 1-fluoro-2-iodoethane, and then one-step reaction was carried out to obtain intermediates **126-3** and **127-3.**

LC-MS: m/z 593 [M-100+H]⁺.

Step 4: Referring to the synthesis method of compound **71,** intermediates **126-3** and **127-3** were used instead of intermediate **71-2,** and one-step reaction was carried out to obtain compounds **126** and **127.**

Compound 126: ¹H NMR (400 MHz, DMSO-d₆) δ 9.38 (s, 1H), 8.35 (s, 1H), 8.29 (s, 1H), 7.93 (s, 1H), 7.53 (d, *J =* 7.6 Hz, 3H), 7.44 (t, *J =* 7.5 Hz, 2H), 7.37 (t, *J =* 7.0 Hz, 1H), 7.27 (s, 1H), 7.15 (d, *J =* 9.2 Hz, 1H), 4.35 - 4.21 (m, 1H), 3.41 (s, 3H), 3.10 (s, 2H), 2.94 (s, 1H), 2.78 (s, 1H), 2.16 (d, *J =* 6.0 Hz, 1H), 1.86 (dd, *J =* 17.4, 9.7 Hz, 2H), 1.71 (dd, *J =* 19.8, 7.8 Hz, 1H).

LC-MS: m/z 593 [M+H]⁺.

Compound **127:** ¹H NMR (400 MHz, DMSO-d₆) δ 9.29 (s, 1H), 8.32 (s, 1H), 8.23 (s, 1H), 7.95 (s, 1H), 7.53 (d, *J =* 7.3 Hz, 2H), 7.45 (t, *J =* 7.4 Hz, 2H), 7.42 - 7.34 (m, 2H), 7.33 (s, 1H), 7.16 (d, *J=* 9.3 Hz, 1H), 4.30 (s, 1H), 3.97 (s, 3H), 3.10 (s, 2H), 2.94 (s, 1H), 2.78 (s, 1H), 2.21 - 2.11 (m, 2H), 1.85 (s, 1H), 1.75 (s, 1H).

LC-MS: m/z 593 [M+H]⁺.

### Example 19: Synthesis of compound 131 and compound 132:

### Compound 131: (S)-8-((S)-5-Chloro-6-fluoro-2-phenyl-2-((S)-pyrrolidin-2-yl)-2,3-dihydrobenzofuran-4-yl)-9-fluoro-2-(methyl-d₃)-1-oxo-1,2-dihydrobenzo[5,6][1,4]dioxo[2,3-d]pyridazine-7-carboxamide

### Compound 132: (S)-8-((S)-5-Chloro-6-fluoro-2-phenyl-2-((S)-pyrrolidin-2-yl)-2,3-dihydrobenzofuran-4-yl)-9-fluoro-3-(methyl-d₃)-4-oxo-3,4-dihydrobenzo[5,6][1,4]dioxo[2,3-d]pyridazine-7-carboxamide

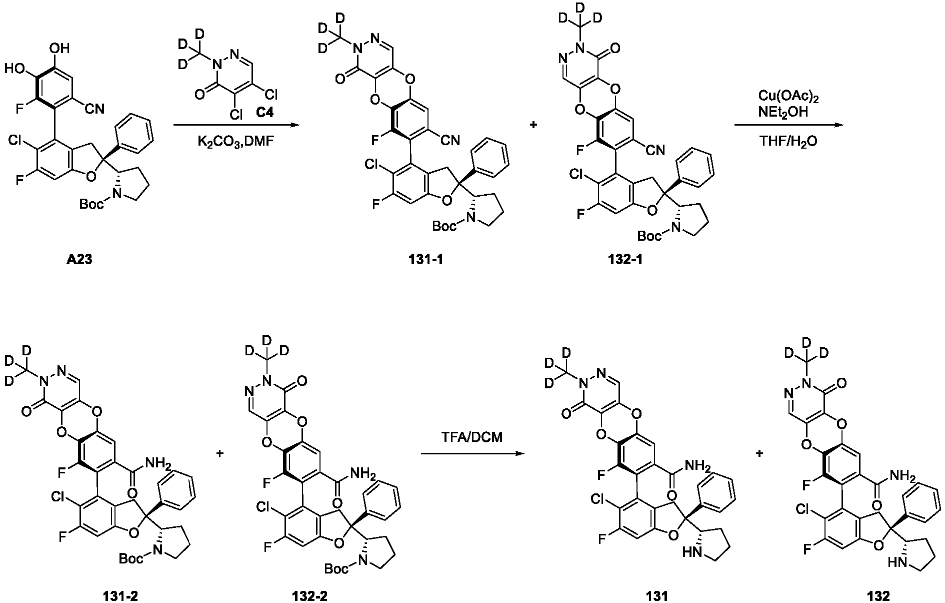

Step 1: Referring to the synthesis method of compound **71,** intermediate **C4** was used instead of intermediate 3,4-dichloropyridazine, and one-step reaction was carried out to obtain intermediates **131-1** and **132-1** (mixture).

LC-MS: m/z 645 [M-56+H]⁺.

Step 2: Referring to the synthesis method of compound **71,** intermediates **131-1** and **132-1** (mixture) were used instead of intermediate **71-1,** and one-step reaction was carried out to obtain intermediates **131-2** and **132-2.**

LC-MS: m/z 596 [M-100+H]⁺.

Step 3: Referring to the synthesis method of compound **71,** intermediates **131-2** and **132-2** were used instead of intermediate **71-2,** and one-step reaction was carried out to obtain compounds **131** and **132.**

Compound **131:** LC-MS: m/z 596 [M+H]⁺.

¹H NMR (400 MHz, Methanol-*d*₄) δ 7.83 (s, 1H), 7.58 - 7.50 (m, 2H), 7.47 - 7.41 (m, 2H), 7.38 (d, J = 7.3 Hz, 1H), 7.17 (d, J = 1.7 Hz, 1H), 7.02 (d, J = 9.1 Hz, 1H), 4.25 (dd, J = 9.6, 7.2 Hz, 1H), 3.64 (dd, J = 16.8, 1.6 Hz, 1H), 3.35 (d, J = 7.1 Hz, 1H), 3.18 (d, J = 16.7 Hz, 2H), 2.30 - 2.21 (m, 1H), 2.04 (dq, J = 13.5, 7.4, 6.1 Hz, 2H), 1.92 (dt, J = 12.9, 8.9 Hz, 1H).

Compound **132:** LC-MS: m/z 596 [M+H]⁺.

¹H NMR (400 MHz, Methanol-*d*₄) δ 7.80 (s, 1H), 7.56 (dt, J = 6.4, 1.3 Hz, 2H), 7.48 (t, J = 7.6 Hz, 2H), 7.43 - 7.37 (m, 1H), 7.23 (d, J = 1.7 Hz, 1H), 7.03 (d, J = 9.1 Hz, 1H), 4.26 (dd, J = 9.5, 7.3 Hz, 1H), 3.63 (d, J = 16.5 Hz, 1H), 3.36 - 3.32 (m, 1H), 3.25 - 3.13 (m, 2H), 2.25 (dt, J = 12.4, 6.1 Hz, 1H), 2.04 (dp, J = 13.0, 7.0 Hz, 2H), 1.96 - 1.86 (m, 1H).

### Example 20: Synthesis of compound 133 and compound 134:

### Compound 133: (S)-8-((S)-5-Chloro-6-fluoro-2-phenyl-2-((S)-pyrrolidin-2-yl)-2,3-dihydrobenzofuran-4-yl)-9-fluoro-2-(3,3,3-trifluoropropyl)-1-oxo-1,2-dihydrobenzo[5,6][1,4]dioxo[2,3-d]pyridazine-7-carboxamide

### Compound 134: (S)-8-((S)-5-Chloro-6-fluoro-2-phenyl-2-((S)-pyrrolidin-2-yl)-2,3-dihydrobenzofuran-4-yl)-9-fluoro-3-(3,3,3-trifluoropropyl)-4-oxo-3,4-dihydrobenzo[5,6][1,4]dioxo[2,3-d]pyridazine-7-carboxamide

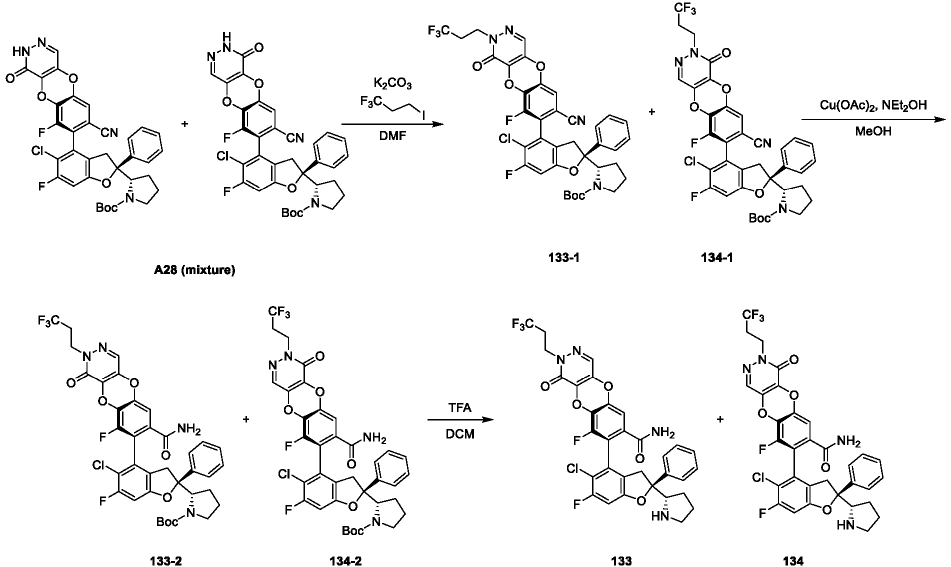

Referring to example **15,** compound 1,1,1-trifluoro-3-iodopropane was used instead of 1-fluoro-2-iodoethane for reaction to obtain compounds **133** and **134.**

Compound **133**, LC-MS: m/z 675 [M+H]⁺.

¹H NMR (400 MHz, Methanol-*d₄*) δ 7.89 (s, 1H), 7.58 - 7.32 (m, 5H), 7.18 (d, J = 1.8 Hz, 1H), 7.02 (d, J = 9.1 Hz, 1H), 4.39 (t, J = 7.0 Hz, 2H), 4.25 (dd, J = 9.6, 7.2 Hz, 1H), 3.68 - 3.58 (m, 1H), 3.38 - 3.32 (m, 1H), 3.25 - 3.13 (m, 2H), 2.73 (dtd, J = 17.7, 10.8, 7.0 Hz, 2H), 2.25 (dt, J = 12.4, 5.8 Hz, 1H), 2.02 (td, J = 13.1, 12.7, 5.6 Hz, 2H), 1.95 - 1.86 (m, 1H).

Compound **134**, LC-MS: m/z 675 [M+H]⁺.

¹H NMR (400 MHz, Methanol-*d₄*) δ 7.86 (s, 1H), 7.60 - 7.37 (m, 5H), 7.23 (d, J = 1.9 Hz, 1H), 7.04 (s, 1H), 4.41 (td, J = 7.0, 1.6 Hz, 2H), 4.25 (dd, J = 9.5, 7.3 Hz, 1H), 3.63 (d, J = 16.5 Hz, 1H), 3.35 (d, J = 7.0 Hz, 1H), 3.19 (d, J = 16.6 Hz, 2H), 2.74 (qt, J = 10.8, 6.9 Hz, 2H), 2.30 - 2.20 (m, 1H), 2.04 (dq, J = 13.7, 7.2, 6.2 Hz, 2H), 1.93 (dt, J = 12.8, 8.8 Hz, 1H).

### Example 21: Synthesis of compound 135 and compound 136:

### Compound 135: (S)-8-((S)-5-Chloro-6-fluoro-2-phenyl-2-((S)-pyrrolidin-2-yl)-2,3-dihydrobenzofuran-4-yl)-9-fluoro-2-methyl-1-oxo-1,2-dihydrobenzo[5,6][1,4]dioxo [2,3-c]pyridine-7-carboxamide

### Compound 136: (S)-7-((S)-5-Chloro-6-fluoro-2-phenyl-2-((S)-pyrrolidin-2-yl)-2,3-dihydrobenzofuran-4-yl)-6-fluoro-2-methyl-1-oxo-1,2-dihydrobenzo[5,6][1,4]dioxo[2,3-c]pridine-8-carboxamide

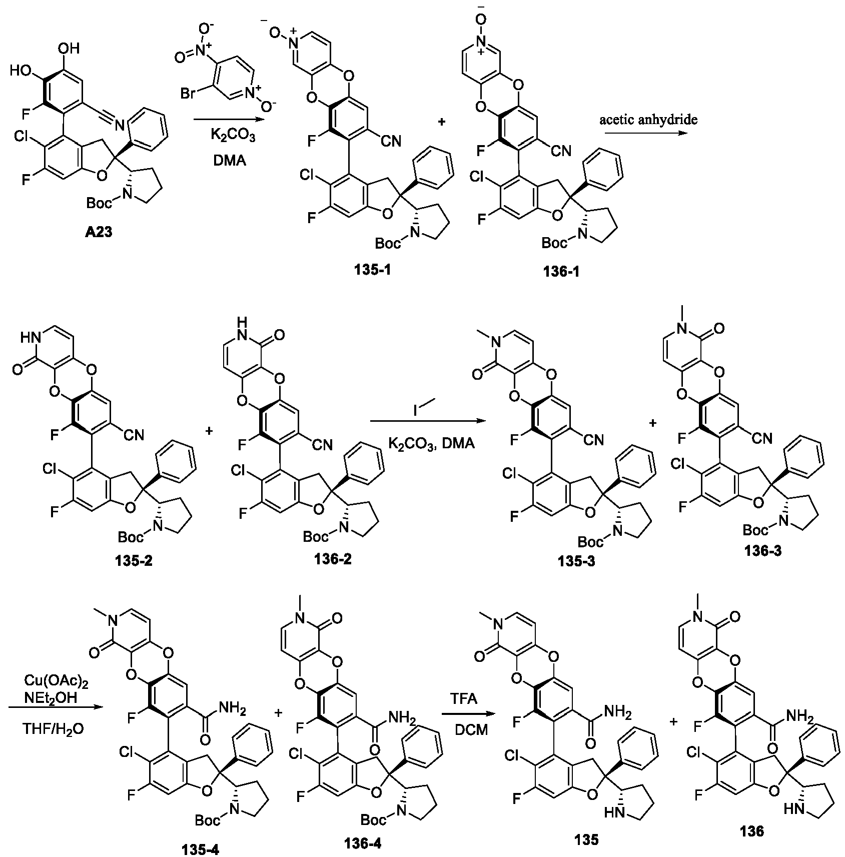

Step 1: Referring to the synthesis method of compound 71, intermediate 3-bromo-4-nitropyridine *N*-oxide was used instead of intermediate 3,4-dichloropyridazine, and one-step reaction was carried out to obtain intermediates **135-1** and **136-1** (mixture).

LC-MS: m/z 701 [M+41+H]⁺.

Step 2: Compounds **135-1** and **136-1** (mixture) (50 mg, 0.076 mmol) and acetic anhydride (1.5 mL) were sequentially added to an 8 mL microwave tube, and the reaction mixture was stirred at 135°C for 4 hours under microwave irradiation in a sealed tube. TLC showed that the reaction was completed. The reaction mixture was diluted by adding water and extracted three times with ethyl acetate. The combined organic phases were washed with saturated brine, dried over anhydrous sodium sulfate, filtered, and concentrated under reduced pressure. The resulting residue was purified by silica gel chromatography (PE/EA = 1:1) to obtain **135-2** and **136-2** (mixture, 30 mg, yield: 60%) as a light yellow foamy solid.

LC-MS: m/z 604 [M-56+H]⁺.

Step 3: Referring to the synthesis method of compounds **118** and **119,** intermediates **135-2** and **136-2** were used instead of intermediates **118-3** and **119-3,** and intermediate iodomethane was used instead of 1-fluoro-2-iodoethane, and then one-step reaction was carried out to obtain intermediates **135-3** and **136-3** (mixture).

LC-MS: m/z 574 [M-100+H]⁺.

Step 4: Referring to the synthesis method of compound **71,** intermediates **135-3** and **136-3** (mixture) were used instead of intermediate **71-1,** and one-step reaction was carried out to obtain compounds **135-4** and **136-4.**

LC-MS: m/z 592 [M-100+H]⁺.

Step 5: Referring to the synthesis method of compound **71,** intermediates **135-4** and **136-4** were used instead of intermediate **71-2,** and one-step reaction was carried out to obtain compounds **135** and **136.**

Compound **135:** ¹H NMR (400 MHz, DMSO-d₆) δ 9.36 (s, 1H), 8.34 (s, 1H), 7.92 (s, 1H), 7.55 (dd, *J =* 12.7, 7.5 Hz, 3H), 7.46 (dd, *J =* 15.3, 8.0 Hz, 3H), 7.37 (t, *J =* 7.2 Hz, 1H), 7.20 (d, *J =* 1.3 Hz, 1H), 7.15 (d, *J*= 9.3 Hz, 1H), 6.16 (d, *J =* 7.6 Hz, 1H), 4.32 - 4.25 (m, 1H), 3.58 (d, *J* = 16.8 Hz, 1H), 3.41 (s, 3H), 3.09 (dd, *J =* 24.3, 15.9 Hz, 3H), 2.21 - 2.11 (m, 1H), 1.93 - 1.79 (m, 2H), 1.78 - 1.68 (m, 1H).

LC-MS: m/z 592 [M+H]⁺.

Compound **136:** ¹H NMR (400 MHz, DMSO-*d*₆) δ 9.37 (s, 1H), 8.32 (s, 1H), 7.93 (s, 1H), 7.57 - 7.35 (m, 7H), 7.24 (s, 1H), 7.14 (d, *J =* 9.4 Hz, 1H), 6.15 (d, *J =* 7.5 Hz, 1H), 4.29 (s, 1H), 3.58 (d, *J=* 16.8 Hz, 1H), 3.43 (s, 3H), 3.06 (t, *J =* 15.6 Hz, 3H), 2.17 (d, *J =* 7.7 Hz, 1H), 2.02 - 1.81 (m, 3H).

LC-MS: m/z 592 [M+H]⁺.

### Example 22: Synthesis of compound 141:

### Compound 141: (S)-8-((S)-5-Chloro-6-fluoro-2-phenyl-2-((S)-pyrrolidin-2-yl)-2,3-dihydrobenzofuran-4-yl)-2-(2,2-difluoroethyl)-9-fluoro-N-methyl-1-oxo-1,2-dihydrobenzo[5,6][1,4]dioxo[2,3-d]pyridazine-7-carboxamide

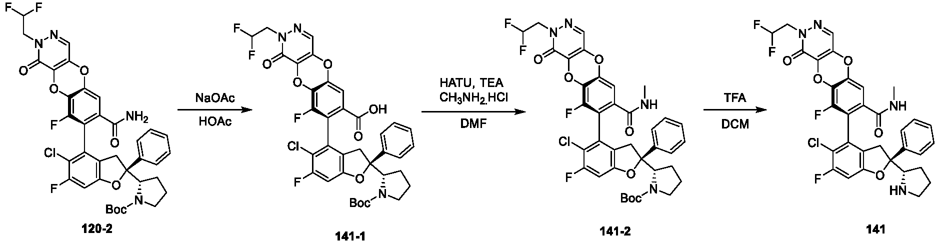

Step 1: Referring to the synthesis method of compound **I-26,** intermediate **120-1** was used instead of intermediate **I-26-2,** and one-step reaction was carried out to obtain intermediate **141-1.**

LC-MS: m/z 742 [M-H]⁻.

Step 2: Referring to the synthesis method of compound **I-26,** intermediate **141-1** was used instead of intermediate **I-26-3,** and one-step reaction was carried out to obtain intermediate **141-2.**

LC-MS: m/z 757 [M+H]⁺.

Step 3: Referring to the synthesis method of compound **I-26,** intermediate **141-2** was used instead of intermediate **I-26-4,** and one-step reaction was carried out to obtain compound **141.**

LC-MS: m/z 657 [M+H]⁺.

¹H NMR (400 MHz, Methanol-*d*₄) δ 7.90 (s, 1H), 7.62 - 7.33 (m, 5H), 7.12 (s, 1H), 7.03 (d, J = 9.0 Hz, 1H), 6.23 (t, J = 55.7 Hz, 1H), 4.51 (td, J = 13.7, 4.0 Hz, 2H), 4.27 (s, 1H), 3.73 - 3.58 (m, 1H), 3.22 (s, 2H), 2.76 (s, 3H), 2.25 (m, 1H), 2.07 (m, 2H), 1.94 (m, 1H).

### Example 23: Synthesis of compound 142:

### (R)-8-((S)-5-Chloro-6-fluoro-2-phenyl-2-((S)-pyrrolidin-2-yl)-2,3-dihydrobenzofuran-4-yl)-9-fluoro-N-methyl-1-oxo-2-(3,3,3-trifluoropropyl)-1,2-dihydrobenzo[5,6][1,4]dioxa[2,3-d]pyridazine-7-carboxamide

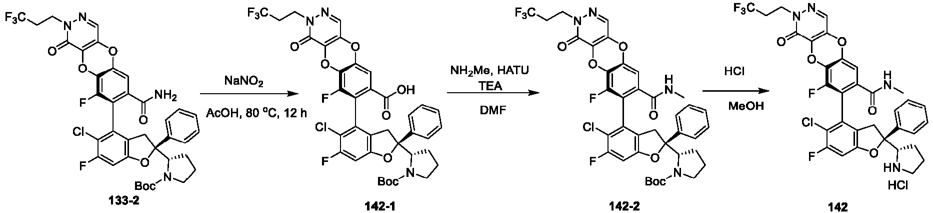

Step 1: **133-2** (35 mg, 0.045 mmol) was dissolved in acetic acid (2 mL) at room temperature, and sodium nitrite (62.31 mg, 0.903 mmol) was added thereto. The reaction mixture was stirred at 80°C for 12 hours. The mixture was filtered, and the filtrate was concentrated under reduced pressure to obtain crude product **142-1** (30 mg, yield: 85.64%).

LC-MS: m/z 776 [M+H]⁺.

Step 2: Crude product **142-1** (30 mg, 0.039 mmol), HATU (17.64 mg, 0.046 mmol), TEA (15 mg, 0.116 mmol) and methylamine hydrochloride (7.83 mg, 0.116 mmol) were dissolved in DMF (1 mL), and the reaction mixture was stirred at room temperature overnight. The mixture was filtered, and the filtrate was concentrated under reduced pressure. The residue was purified by silica gel column chromatography (ethyl acetate/petroleum ether = 1/1) to obtain **142-2** (16 mg, yield: 52.46%).

LC-MS: m/z 789 [M+H]⁺.

Step 3: **142-2** (16 mg, 0.020 mmol), methanol (1 mL) and 1,4-dioxane hydrochloride (1 mL, 4.0 M) were sequentially added to a reaction flask, and the reaction mixture was stirred at 35°C for 2 hours. After the reaction was completed, the reaction mixture was concentrated under reduced pressure. The residue was lyophilized to obtain the hydrochloride of compound **142** (13 mg, yield: 89.59%).

LC-MS: m/z 689 [M+H]⁺.

1HNMR (400 MHz, Methanol-*d*₄) δ 7.88 (s, 1H), 7.58 - 7.51 (m, 2H), 7.45 (t, J = 7.5 Hz, 2H), 7.38 (dd, J = 8.3, 6.1 Hz, 1H), 7.11 (d, J = 1.7 Hz, 1H), 7.03 (d, J = 9.2 Hz, 1H), 4.39 (t, J = 7.0 Hz, 2H), 4.27 (t, J = 8.4 Hz, 1H), 3.60 (d, J = 9.8 Hz, 1H), 3.38 - 3.32 (m, 1H), 3.22 (ddd, J = 14.9, 10.0, 5.7 Hz, 2H), 2.82 (s, 3H), 2.75 - 2.66 (m, 2H), 2.31 - 2.19 (m, 1H), 2.04 (td, J = 14.2, 13.0, 7.1 Hz, 2H), 1.99 - 1.88 (m, 1H).

### Example 24: Synthesis of compound 143:

### (S)-8-((S)-5-Chloro-6-fluoro-2-phenyl-2-((S)-pyrrolidin-2-yl)-2,3-dihydrobenzofuran-4-yl)-9-fluoro-1-oxo-2-(2,2,2-trifluoroethyl)-1,2-dihydroxybenzo[5,6][1,4]dioxo[2,3-d]pyridazine-7-carboxamide

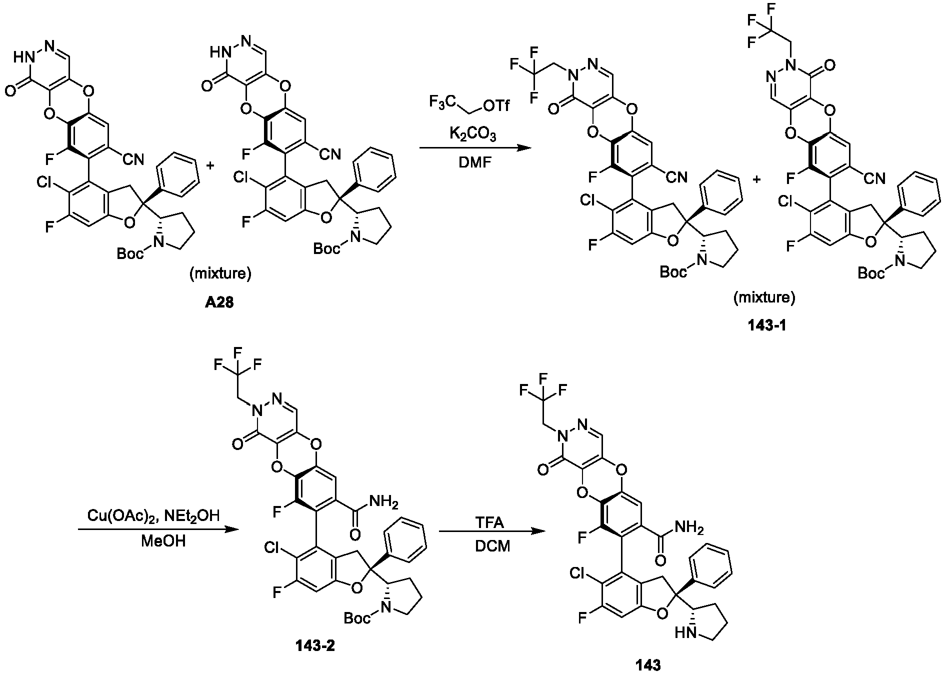

Step 1: Referring to the synthesis method of compound **118,** intermediate 2,2,2-trifluoroethyl trifluoromethanesulfonate was used instead of 1-fluoro-2-iodoethane, and one-step reaction was carried out to obtain intermediate **143-1.**

LC-MS: m/z 643 [M-100+H]⁺.

Step 2: Referring to the synthesis method of compound 71, intermediate **143-1** was used instead of intermediate **71-1,** and one-step reaction was carried out to obtain compound **143-2.**

LC-MS: m/z 661 [M-100+H]⁺.

Step 3: Referring to the synthesis method of compound **71,** intermediate **143-2** was used instead of intermediate **71-2,** and one-step reaction was carried out to obtain compound **143.**

¹H NMR (400 MHz, DMSO-d₆) δ 9.39 (s, 1H), 8.33 (s, 1H), 8.13 (s, 1H), 7.95 (s, 1H), 7.44 (ddd, *J =* 44.9, 29.6, 16.0 Hz, 7H), 7.16 (d, *J =* 9.3 Hz, 1H), 4.97 (d, *J =* 8.2 Hz, 2H), 4.31 (s, 1H), 3.59 (d, *J =* 17.1 Hz, 1H), 3.10 (s, 2H), 3.00 (d, *J =* 17.1 Hz, 1H), 2.16 (s, 1H), 1.95 - 1.81 (m, 2H), 1.71 (s, 1H).

LC-MS: m/z 661 [M+H]⁺.

### Example 25: Synthesis of compound 144:

### (S)-8-((S)-5-Chloro-6-fluoro-2-phenyl-2-((S)-pyrrolidin-2-yl)-2,3-dihydrobenzofuran-4-yl)-9-fluoro-1-oxo-2-(2,2,2-trifluoroethyl)-1,2-dihydroxybenzo[5,6][1,4]dioxo[2,3-d]pyridazine-7-carboxamide

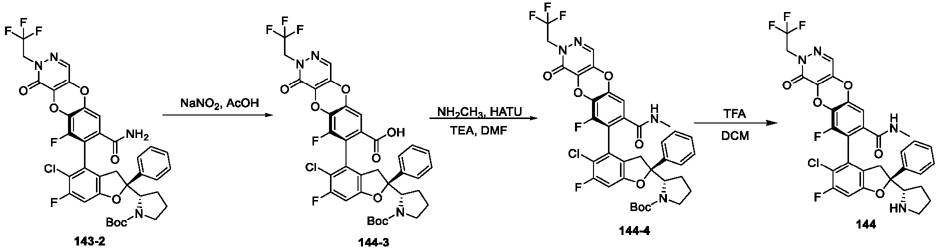

Step 1: Referring to the synthesis method of compound **124,** intermediate **143-2** was used instead of intermediate **124-2,** and one-step reaction was carried out to obtain compound **144-3.**

LC-MS: m/z 662 [M-100+H]⁺.

Step 2: Referring to the synthesis method of compound **124,** intermediate **144-3** was used instead of intermediate **124-3,** and one-step reaction was carried out to obtain compound **144-4.**

LC-MS: m/z 675 [M-100+H]⁺.

Step 3: Referring to the synthesis method of compound **124,** intermediate **144-4** was used instead of intermediate **124-4,** and one-step reaction was carried out to obtain compound **144.**

¹H NMR (400 MHz, DMSO-d₆) δ 9.38 (s, 1H), 8.44 (d, *J =* 4.5 Hz, 1H), 8.37 (s, 1H), 8.13 (s, 1H), 7.44 (ddd, *J =* 28.2, 19.5, 7.5 Hz, 5H), 7.29 (s, 1H), 7.16 (d, *J =* 9.4 Hz, 1H), 5.03 - 4.90 (m, 2H), 4.31 (s, 1H), 3.38 (s, 1H), 3.13 (d, *J=* 23.1 Hz, 2H), 3.01 (d, *J* = 16.9 Hz, 1H), 2.63 (d, *J* = 4.3 Hz, 3H), 2.10 (d, *J*= 7.1 Hz, 1H), 1.97 - 1.82 (m, 2H), 1.73 (d, *J =* 11.6 Hz, 1H).

LC-MS: m/z 675 [M+H]⁺.

### Example 26: Synthesis of compound 147:

### (S)-8-((S)-5-Chloro-6-fluoro-2-phenyl-2-((S)-pyrrolidin-2-yl)-2,3-dihydrobenzofuran-4-yl)-2-(3,3-difluorocyclobutyl)-9-fluoro-1-oxo-1,2-dihydrobenzo[5,6][1,4]dioxa[2,3-d]pyridazine-7-carboxamide

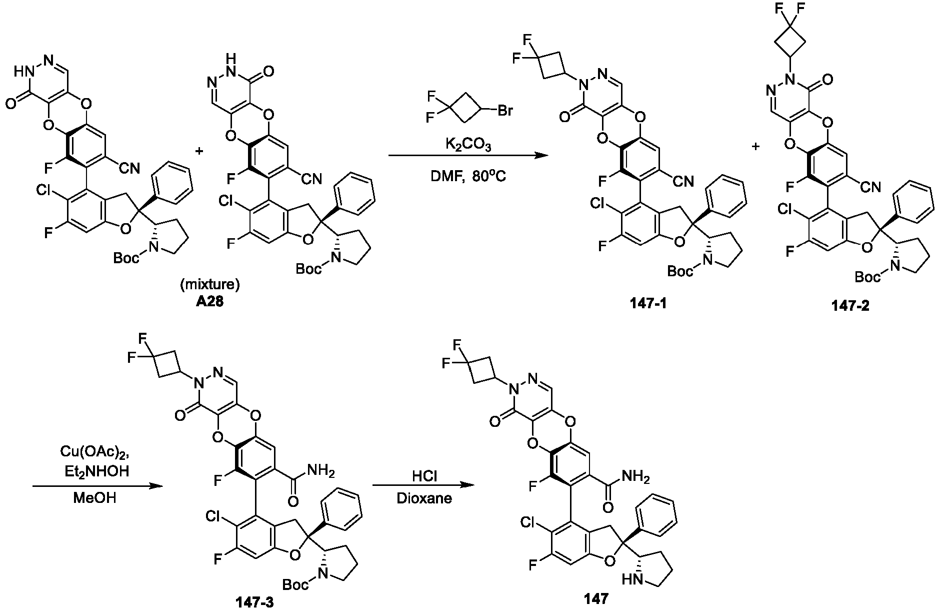

Step 1: **A28** (200 mg, 0.303 mmol) was dissolved in DMF (3 mL), then potassium carbonate (83.62 mg, 0.605 mmol) and 3-bromo-1,1-difluorocyclobutane (103.46 mg, 0.605 mmol) were added thereto at room temperature, and the reaction mixture was stirred at 80°C for 2 hours. The reaction mixture was diluted with ethyl acetate and washed with saturated sodium bicarbonate solution. The organic phase was washed with saturated brine, dried over anhydrous sodium sulfate, and concentrated to obtain a crude. The crude was purified by silica gel column chromatography (ethyl acetate/petroleum ether = 1/3) to obtain a mixture of **147-1** and **147-2** (15 mg, yield: 6.6%).

LC-MS: m/z 751 [M+H]⁺.

Step 2: The mixture of **147-1** and **147-2** (15 mg, 0.02 mmol) was dissolved in methanol (1 mL), then copper acetate (7.25 mg, 0.04 mmol) and diethylhydroxylamine (35.6 mg, 0.4 mmol) were added thereto, and stirred at room temperature for 1 hour. After the reaction was completed, the reaction mixture was filtered and concentrated. The resulting residue was purified by Prep-TLC (petroleum ether/ethyl acetate = 1:1) to obtain **147-3** (6 mg, yield: 32.55%).

LC-MS: m/z 769 [M+H]⁺.

Step 3: **147-3** (6 mg, 0.007 mmol), methanol (1 mL) and 1,4-dioxane hydrochloride (1 mL, 4.0 M) were sequentially added to a reaction flask, and the reaction mixture was stirred at 35°C for 1 hour. After the reaction was completed, the reaction mixture was concentrated under reduced pressure. The residue was lyophilized to obtain the hydrochloride of compound **147** (1.5 mg, yield: 27.36%).

LC-MS: m/z 669 [M+H]⁺.

¹H NMR (400 MHz, Methanol-*d*₄): δ 7.96 (s, 1H), 7.54 (d, J = 7.4 Hz, 2H), 7.40 (dt, J = 26.3, 7.3 Hz, 3H), 7.18 (s, 1H), 7.03 (d, J = 9.1 Hz, 1H), 3.22 - 2.96 (m, 6H), 2.38 - 2.19 (m, 2H), 1.98 (d, J = 53.7 Hz, 4H), 1.59 (s, 2H).

### Example 28: Synthesis of compound 149:

### (R)-8-((S)-5-Chloro-6-fluoro-2-phenyl-2-((S)-pyrrolidin-2-yl)-2,3-dihydrobenzofuran-4-yl)-9-fluoro-2-(2-methoxyethyl)-1-oxo-1,2-dihydrobenzo[5,6][1,4]dioxa[2,3-d]pyridazine-7-carboxamide

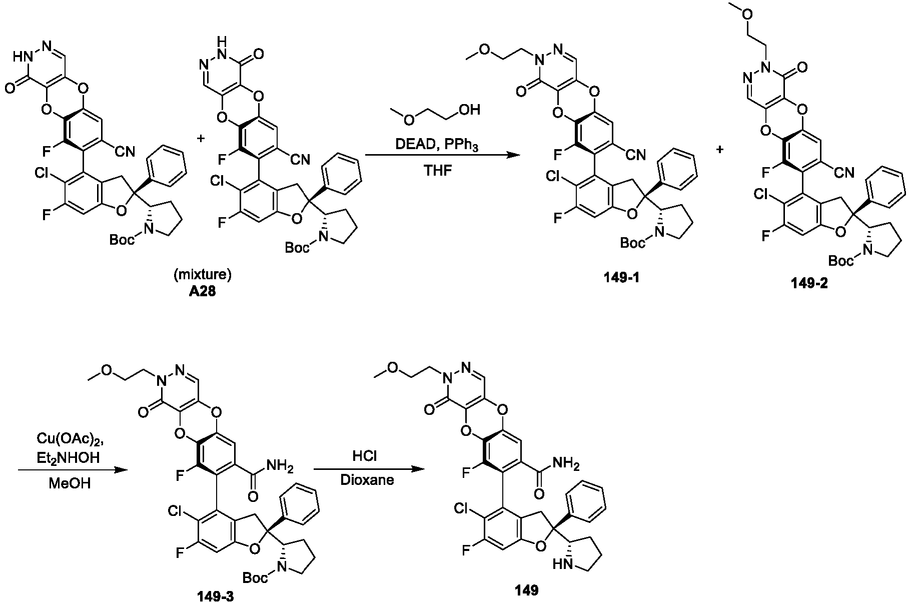

Step 1: **A28** (25 mg, 0.038 mmol), 2-methoxyethanol (5.76 mg, 0.076 mmol), DEAD (13.17 mg, 0.076 mmol) and triphenylphosphine (19.84 mg, 0.076 mmol) were added to tetrahydrofuran (1 mL), and the reaction mixture was stirred at room temperature for 12 hours. The reaction mixture was diluted with water and extracted with ethyl acetate. The organic layer was dried over Na₂SO₄ and concentrated under reduced pressure, and the residue was purified by silica gel chromatography (ethyl acetate/petroleum ether = 1/3) to obtain a mixture of **149-1** and **149-2** (25 mg, yield: 70%).

LC-MS: m/z 719 [M+H]⁺.

Step 2: The mixture of **149-1** and **149-2** (25 mg, 0.035 mmol) was dissolved in methanol (1 mL), then copper acetate (12.63 mg, 0.07 mmol) and diethylhydroxylamine (62 mg, 0.695 mmol) were added thereto, and stirred at room temperature for 1 hour. After the reaction was completed, the reaction mixture was filtered and concentrated. The resulting residue was purified by Prep-TLC (petroleum ether/ethyl acetate = 1:1) to obtain **149-3** (6 mg, yield: 15%).

LC-MS: m/z 737 [M+H]⁺.

Step 3: **149-3** (6 mg, 0.008 mmol), methanol (1 mL) and 1,4-dioxane hydrochloride (1 mL, 4.0 M) were sequentially added to a reaction flask, and the reaction mixture was stirred at 35°C for 2 hours. After the reaction was completed, the reaction mixture was concentrated under reduced pressure. The residue was lyophilized to obtain the hydrochloride of compound **149** (1.5 mg, yield: 27.36%).

LC-MS: m/z 673 [M+H]⁺.

¹H NMR (400 MHz, Methanol-*d*₄) δ 7.86 (s, 1H), 7.55 (d, J = 7.3 Hz, 2H), 7.47 (t, J = 7.5 Hz, 2H), 7.40 (t, J = 7.2 Hz, 1H), 7.18 (d, J = 1.7 Hz, 1H), 7.02 (d, J = 9.2 Hz, 1H), 4.33 (td, J = 6.2, 5.8, 2.7 Hz, 2H), 4.28 - 4.19 (m, 2H), 3.76 (t, J = 5.4 Hz, 2H), 3.59 (s, 4H), 3.24 - 3.17 (m, 2H), 2.10 - 1.87 (m, 4H).

### Example 29: Synthesis of compound I-11:

### (S)-7-((S)-5-Chloro-6-fluoro-2-phenyl-2-((S)-pyrrolidin-2-yl)-2,3-dihydrobenzofuran-4-yl)-8-fluoroimidazo[1,2-a]pyridine-6-carboxamide

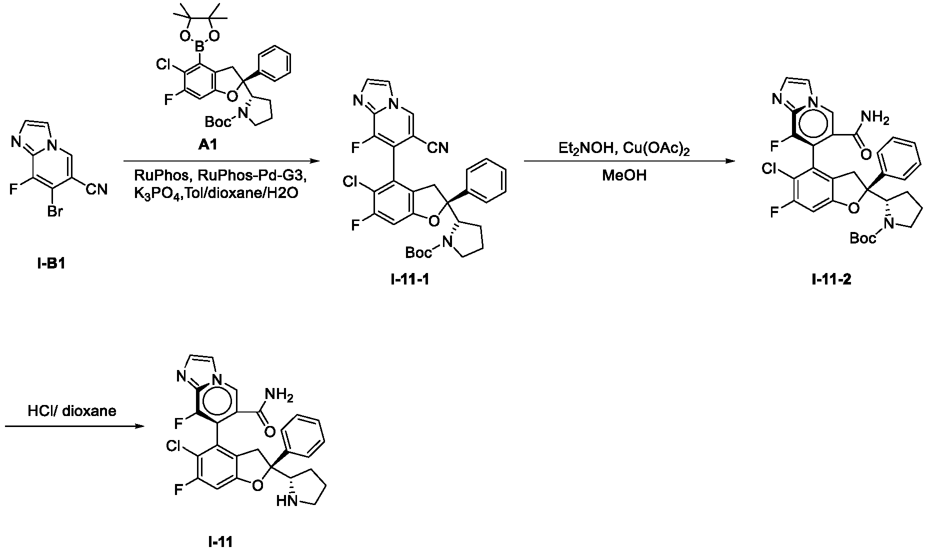

Step 1: Compound **A1** (227 mg, 0.418 mmol), compound **I-B1** (100 mg, 0.348 mmol), potassium phosphate (443.7 mg, 2.09 mmol), toluene (5 mL), water (1 mL), 1,4-dioxane (5 mL), RuPhos-Pd-G3 (23.07 mg, 0.028 mmol) and RuPhos (12.87 mg, 0.028 mmol) were sequentially added to a reaction flask. The reaction mixture was stirred at 100°C for 18 hours under nitrogen atmosphere. TLC showed that the reaction was completed. The reaction mixture was concentrated under reduced pressure, and the resulting residue was purified by silica gel chromatography (PE/EA = 3:1) to obtain **I-11-1** as a yellow solid (65 mg, yield: 32%).

Step 2: Compound **I-11-1** (50 mg, 0.274 mmol), methanol (10 mL), copper acetate (261.65 mg, 1.44 mmol) and diethylhydroxylamine (128.41 mg, 1.44 mmol) were sequentially added to a single-neck flask, and the reaction mixture was stirred at 25°C for 12 hours. A red solid precipitated. After the reaction was completed, the reaction mixture was filtered and concentrated. The resulting residue was purified by silica gel chromatography (DCM/MeOH = 20:1) to obtain **I-11-2** as a yellow solid (16 mg, yield: 31.2%).

Step 3: Intermediate **I-11-2** (15 mg, 0.105 mmol), methanol (2 mL) and 1,4-dioxane (2 mL, 4.0 M) were sequentially added to a reaction flask, and the reaction mixture was stirred at 25°C for 2 hours. After the reaction was completed, the reaction mixture was concentrated under reduced pressure. The residue was dissolved in water and lyophilized to obtain compound **I-11** as a yellow solid (11 mg, hydrochloride).

¹H NMR (400 MHz, Methanol-*d₄*) δ 8.78 (s, 1H), 8.18 (s, 1H), 8.03 (d, J = 1.9 Hz, 1H), 7.64 (d, J = 1.3 Hz, 1H), 7.42 - 7.32 (m, 3H), 7.30 - 7.23 (m, 2H), 7.02 (d, J = 9.2 Hz, 1H), 4.19 - 4.14 (m, 1H), 3.60 - 3.54 (m, 1H), 3.27 (dd, J = 11.4, 4.0 Hz, 2H), 2.95 (d, J = 7.4 Hz, 1H), 1.97 - 1.79 (m, 3H), 1.64 (s, 1H).

LC-MS: m/z 495.2 [M+H]⁺.

### Example 30: Synthesis of compound I-19:

### (S)-7-((S)-5-Chloro-6-fluoro-2-phenyl-2-((S)-pyrrolidin-2-yl)-2,3-dihydrobenzofuran-4-yl)-8-fluoro-3,4-dihydro-2H-benzo[b][1,4]oxazine-6-carboxamide

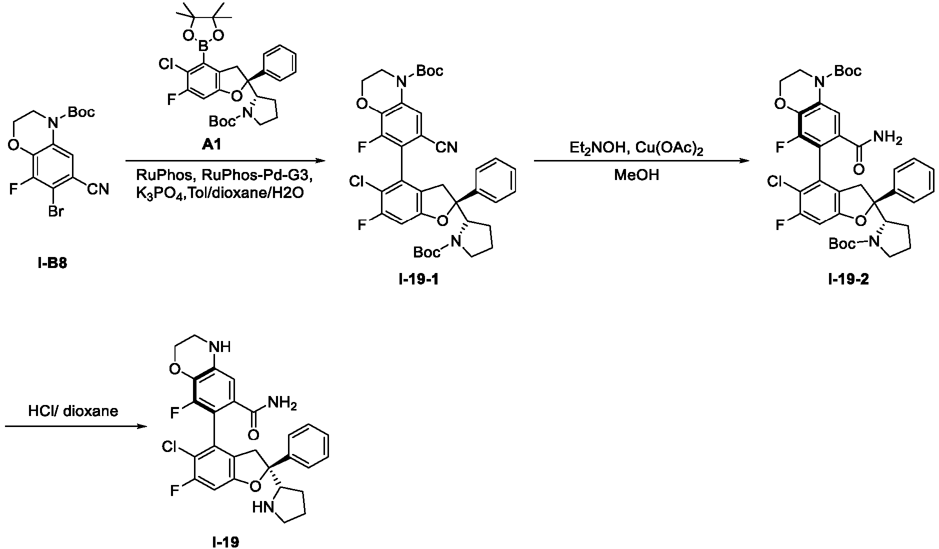

Step 1: Compound **A1** (152 mg, 0.280 mmol), compound **I-B8** (100 mg, 0.280 mmol), potassium phosphate (356.56 mg, 1.655 mmol), toluene (0.5 mL), water (0.2 mL), 1,4-dioxane (0.5 mL), Pd₂(dba)₃ (30 mg, 0.19 mmol) and Ni-Xantphos (20 mg, 0.036 mmol) were sequentially added to a reaction flask. The reaction mixture was stirred at 100°C for 18 hours under nitrogen atmosphere. TLC showed that the reaction was completed. The reaction mixture was concentrated under reduced pressure, and the resulting residue was purified by silica gel chromatography (PE/EA = 3:1) to obtain **I-19-1** as a yellow solid (100 mg, yield: 52%).

Step 2: Compound **I-19-1** (50 mg, 0.072 mmol), methanol (20 mL), copper acetate (261.6 mg, 1.44 mmol) and diethylhydroxylamine (128.4 mg, 1.44 mmol) were sequentially added to a single-neck flask, and the reaction mixture was stirred at 25°C for 12 hours. A red solid precipitated. After the reaction was completed, the reaction mixture was filtered and concentrated. The resulting residue was purified by silica gel chromatography (DCM/MeOH = 20:1) to obtain **I-19-2** as a yellow solid (22 mg, yield: 17.95%).

LC-MS: m/z 712.2 [M+H]⁺.

Step 3: Intermediate **I-19-2** (20 mg, 0.105 mmol), methanol (2 mL) and 1,4-dioxane hydrochloride (2 mL, 4.0 M) were sequentially added to a reaction flask, and the reaction mixture was stirred at 25°C for 2 hours. After the reaction was completed, the reaction mixture was concentrated under reduced pressure. The residue was purified by preparative HPLC and lyophilized to obtain compound **I-19** as a yellow solid (15 mg, hydrochloride).

¹H NMR (400 MHz, Methanol-*d₄*) δ 7.47-7.45 (m, 2H), 7.39-7.36 (m, 2H), 7.32-7.28 (m, 1H), 6.85 (d, *J=* 12Hz, 1H), 6.74 (s, 1H),4.18 - 4.16 (m, 2H),4.13-4.09 (m, 1H), 3.57 - 3.52 (m, 1H), 3.49-3.45 (m, 1H), 3.35-3.33 (m, 2H), 3.13-3.03 (m, 2H), 2.13-2.06 (m, 1H), 1.93-1.79 (m, 3H).

LC-MS: m/z 512.9 [M+H]⁺.

### Example 31: Synthesis of compound I-26:

### (S)-7-((2S)-5-Chloro-6-fluoro-2-phenyl-2-((S)-pyrrolidin-2-yl)-2,3-dihydrobenzofuran-4-yl)-8-fluoro-N-methyl-3,4-dihydro-2H-benzo[b][1,4]oxazine-6-carboxamide

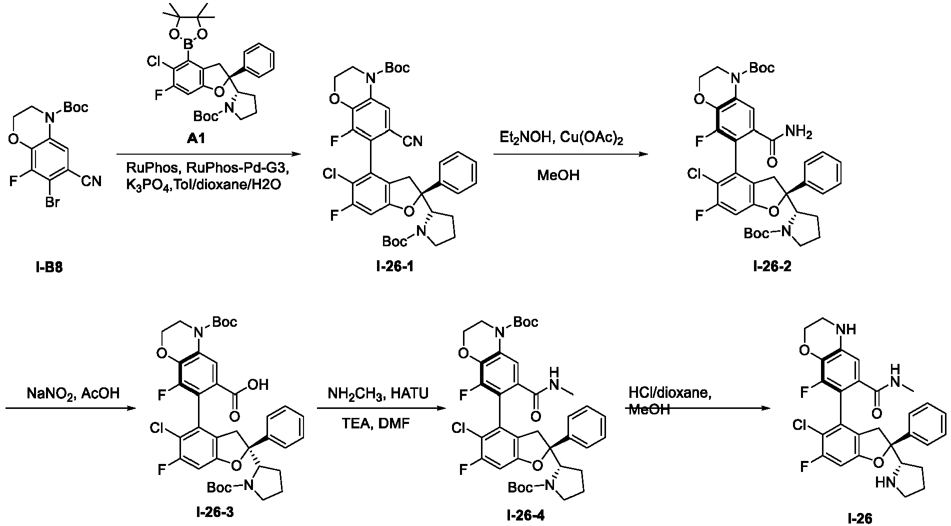

Step 1: Compound **A1** (152 mg, 0.28 mmol), compound **I-B8** (100 mg, 0.28 mmol), potassium phosphate (356 mg, 1.68 mmol), toluene (5 mL), water (1 mL), 1,4-dioxane (5 mL), RuPhos-Pd-G3 (23.07 mg, 0.028 mmol) and RuPhos (12.87 mg, 0.028 mmol) were sequentially added to a reaction flask. The reaction mixture was stirred at 100°C for 18 hours under nitrogen atmosphere. TLC showed that the reaction was completed. The reaction mixture was concentrated under reduced pressure, and the resulting residue was purified by silica gel chromatography (PE/EA = 3:1) to obtain **I-26-1** as a yellow solid (100 mg, yield: 53.35%).

Step 2: Compound **I-26-1** (190 mg, 0.274 mmol), methanol (20 mL), copper acetate (149.14 mg, 0.821 mmol) and diethylhydroxylamine (487.97 mg, 5.474 mmol) were sequentially added to a single-neck flask, and the reaction mixture was stirred at 25°C for 12 hours. A red solid precipitated. After the reaction was completed, the reaction mixture was filtered and concentrated. The resulting residue was purified by silica gel chromatography (DCM/MeOH = 20:1) to obtain **1-26-2** as a yellow solid (35 mg, yield: 17.95%).

LC-MS: m/z 712.2 [M+H]⁺.

Step 3: Compound **1-26-2** (100 mg, 0.140 mmol), acetic acid (10 mL) and sodium nitrite (96.88 mg, 1.404 mmol) were sequentially added to a pressure-resistant sealed tube, and the reaction mixture was stirred at 60°C overnight. The reaction mixture was diluted by adding water and extracted three times with ethyl acetate. The combined organic phases were washed with saturated brine, dried over anhydrous sodium sulfate, filtered, and concentrated under reduced pressure. I**-26-3** (77.3 mg, yield: 89.8%) was obtained without purification and used directly in the next reaction step.

LC-MS: m/z 713.2 [M+H]⁺.

Step 4: Compound I**-26-3** (270 mg, 0.379 mmol), methylamine hydrochloride (177.5 mg, 2.650 mmol), HATU (431.9 mg, 1.136 mmol), DMF (15 mL) and TEA (766.19 mg, 7.572 mmol) were sequentially added to a single-neck flask, and the reaction mixture was stirred at room temperature for 4 hours. The reaction mixture was diluted by adding water and extracted three times with ethyl acetate. The combined organic phases were washed with saturated brine, dried over anhydrous sodium sulfate, filtered, and concentrated under reduced pressure. The resulting residue was purified by silica gel chromatography (DCM/MeOH = 20:1) to obtain I**-26-4** as a yellow solid (160 mg, yield: 58.1%).

LC-MS: m/z 726.2 [M+H]⁺.

Step 5: Referring to the synthesis method of compound **I-19,** compound **I-26-4** was used instead of compound **I**-**19-2**, and one-step reaction was carried out to obtain compound **I-26.**

¹HNMR(400MHz, MeOD) δ 7.56 (s, 2H), 7.49 (s, 2H), 7.41 (t,J = 7.3Hz, 1H), 6.95 (d,J = 9.2Hz, 1H), 6.77 (d,J = 1.4Hz, 1H), 4.29 (s, 2H), 4.25 - 4.19 (m, 1H), 3.52 - 3.45 (m, 2H), 3.36 (dd,J = 11.3, 7.2Hz, 2H), 3.21 (dd,J = 13.2, 7.2Hz, 2H), 2.75 (s, 3H), 2.22 - 2.15 (m, 1H), 2.00 (ddd,J = 19.3, 13.1, 5.0Hz, 3H).

LC-MS: m/z 526.1 [M+H]⁺.

### Example 32: Synthesis of compound 1-54:

### (S)-8-((S)-5-Chloro-6-fluoro-2-phenyl-2-((S)-pyrrolidin-2-yl)-2,3-dihydrobenzofuran-4-yl)-9-fluoro-2,3-trihydro-5H-benzo[e][1,4]dioxa-7-carboxamide

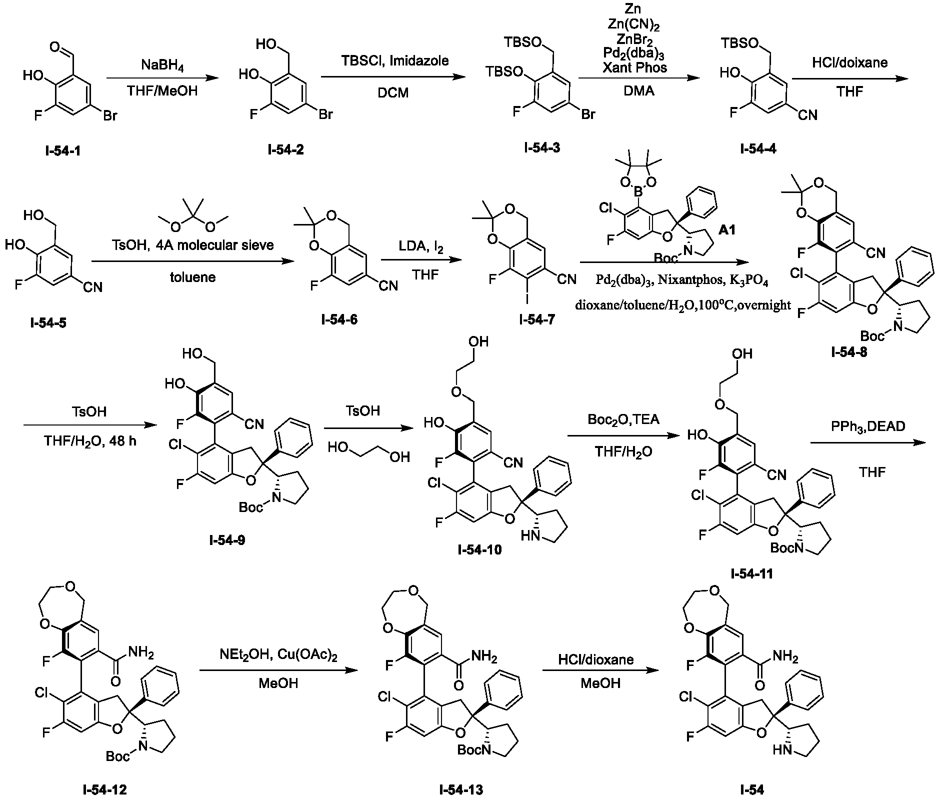

Step 1: NaBH₄ (0.83 g, 21.92 mmol) was added to a solution of **I-54-1** (4.0 g, 18.26 mmol) in THF (200 mL) and MeOH (8 mL) at 0°C. The reaction mixture was reacted at room temperature for 0.5 hours. TLC plate showed that the reaction was completed. The reaction mixture was subjected to rotary evaporation to dryness, acidified with 1 M HCl solution, and extracted twice with EA. The organic phases were combined, dried over anhydrous sodium sulfate, filtered, and the filtrate was concentrated to dryness under reduced pressure. The crude was purified by silica gel chromatography (petroleum ether: ethyl acetate = 2:1) to obtain I**-54-2** as a white solid (3.9 g, yield: 96.6%).

¹H NMR (400 MHz, CDCl₃) δ 7.20 (dd, J = 9.7, 2.3 Hz, 1H), 7.10 (ddt, J = 2.3, 1.5, 0.7 Hz, 1H), 6.67 (s, 1H), 4.82 (s, 2H).

Step 2: **I-54-2** (3.9 g, 17.6 mmol) was dissolved in DCM (50 mL), and then TBSCl (5.85 g, 38.82 mmol) and imidazole (3 g, 44.1 mmol) were added thereto. The reaction mixture was reacted at room temperature for 1.5 hours, and the TLC plate showed that the reaction was completed. The reaction mixture was concentrated, and the residue was purified by column chromatography (petroleum ether: ethyl acetate = 10:1) to obtain **I-54-3** as a white solid (7.7 g, yield: 97.1%).

¹H NMR (400 MHz, CDCl₃) δ 7.30 - 7.20 (m, 1H), 7.00 (dd, J = 9.9, 2.4 Hz, 1H), 4.61 (s, 2H), 0.92 - 0.87 (m, 9H), 0.84 (d, J = 1.7 Hz, 9H), 0.08 (d, J = 2.4 Hz, 6H), 0.03 - -0.04 (m, 6H).

Step 3: Methanesulfonato(2-di-t-butylphosphino-2',4',6'-tri-i-propyl-1,1'-biphenyl)(2'-amino-1,1'-biphenyl-2-yl)palladium(II) (0.82 g, 1.03 mmol) was added to a solution of **I-54-3** (7.7 g, 17.1 mmol) and zinc cyanide (4.0 g, 34.2 mmol) in THF (15 mL) and water (75 mL). The air was replaced with nitrogen three times. The reaction mixture was heated to 70°C, reacted for 16 hours, and filtered. The filtrate was concentrated under reduced pressure to remove the solvent THF. The reaction mixture was diluted with water (200 mL), then extracted with dichloromethane (3 × 150 mL). The organic phases were combined, washed with saturated brine, dried over anhydrous sodium sulfate, filtered, and the filtrate was concentrated to dryness under reduced pressure. The crude was purified by column chromatography (petroleum ether: ethyl acetate = 2:1) to obtain **I-54-4** as an off-white solid (4.6 g, yield: 95.4%).

Step 4: HCl/dioxane (8.2 mL) was added to a MeOH (40 mL) solution of **I-54-4** (4.6 g, 16.3 mmol) at room temperature. The mixture was reacted for 2 minutes. A THF (30 mL) solution of iodine (15.4 g, 60.5 mmol) was added dropwise. TLC plate showed that the reaction was completed. The reaction mixture was concentrated to dryness under reduced pressure and purified by silica gel chromatography (petroleum ether: ethyl acetate = 1:1) to obtain **I-54-5** as a white solid (2.7 g, yield: 98.8%).

¹H NMR (400 MHz, DMSO-*d*₆) δ 10.89 (s, 1H), 7.69 (dd, J = 10.7, 2.1 Hz, 1H), 7.51 (dd, J = 2.1, 1.1 Hz, 1H), 4.51 (s, 2H), 3.57 (s, 1H).

Step 5: 2,2-Dimethoxypropane (8 mL, 64.6 mmol), TsOH.H₂O (0.46 g, 2.4 mmol) and 4 A molecular sieve (0.5 g) were added to a toluene (30 mL) solution of **I-54-5** (2.7 g, 16.1 mmol) and a DMSO (70 mL) solution of potassium carbonate (13.1 g, 94.5 mmol). The reaction mixture was heated to 110°C, reacted for 18 hours, and filtered. The filtrate was concentrated to dryness under reduced pressure and purified by silica gel chromatography (petroleum ether: ethyl acetate = 10:1) to obtain **I-54-6** as a white solid (2.1 g, yield: 62.7%).

¹H NMR (400 MHz, CDCl₃) δ 7.30 - 7.25 (m, 1H), 7.16 - 7.10 (m, 1H), 4.87 (s, 2H), 1.61 (s, 6H).

Step 6: LDA (2.0 mol/L in THF/hexane, 5.3 mL, 10.6 mmol) was added dropwise to a THF (10 mL) solution of **I-54-6** (1 g, 4.8 mmol) at -65°C under nitrogen atmosphere. The mixture was reacted at -65°C for 30 minutes. A THF (2 mL) solution of iodine (1.47 g, 5.8 mmol) was added dropwise. The reaction mixture was reacted at -65°C for 30 minutes. The reaction mixture was poured into ice-cold saturated ammonium chloride aqueous solution (50 mL) and extracted with ethyl acetate (3 × 30 mL). The organic phases were combined, washed with saturated brine, dried over anhydrous sodium sulfate, and filtered. The filtrate was concentrated to dryness under reduced pressure and purified by silica gel chromatography (petroleum ether: ethyl acetate = 5:1) to obtain **I-54-7** as a yellow solid (800 mg, yield: 49.7%).

¹H NMR (400 MHz, CDCl₃) δ 7.16 - 7.12 (m, 1H), 4.83 (d, J = 1.0 Hz, 2H), 1.61 (s, 6H).

Step 7: Intermediate **I-54-7** (780 mg, 2.34 mmol), intermediate A1 (1.5 g, 2.34 mmol), potassium phosphate (2.99 g, 14.01 mmol), toluene (7 mL), dioxane (7 mL), water (3 mL), Pd₂(dba)₃ (190 mg, 0.21 mmol) and *N-*XantPhos (230 mg, 0.42 mmol) were sequentially added to a reaction flask. The reaction mixture was stirred at 100°C for 12 hours under nitrogen atmosphere. The reaction mixture was diluted by adding water and extracted twice with ethyl acetate. The combined organic phases were washed with saturated brine, dried over anhydrous sodium sulfate, filtered, and concentrated under reduced pressure. The resulting residue was purified by silica gel chromatography (petroleum ether/ethyl acetate = 3:1) obtain intermediate **I-54-8** (260 mg, yield: 17.8%).

LC-MS: m/z 623 [M+H]⁺.

Step 8: Intermediate **I-54-8** (260 mg, 0.417 mmol), THF (2.6 mL), water (1.2 mL) and TsOH.H₂O (198 mg, 1.04 mmol) were sequentially added to a reaction flask, and the reaction mixture was stirred at room temperature for 12 hours. After the reaction was completed, the reaction mixture was filtered and concentrated. The resulting residue was purified by column chromatography (DCM/MeOH = 10:1) to obtain **I-54-9** as a white solid (157 mg, yield: 64.5%).

LC-MS: m/z 581 [M-H]⁻.

Step 9: Intermediate **I-54-9** (70 mg, 0.12 mmol), ethylene glycol (1.5 mL) and TsOH.H₂O (46 mg, 0.24 mmol) were sequentially added to a reaction flask, and the reaction mixture was stirred at 100°C for 12 hours. After the reaction was completed, water and ethyl acetate were added for extraction and the organic phase was concentrated under reduced pressure to obtain **I-54-10** (60 mg, crude).

LC-MS: m/z 527 [M+H]⁺.

Step 10: Intermediate **I-54-10** (60 mg, crude), THF (1 mL), Boc₂O (30 mg, 0.14 mmol) and TEA (23 mg, 0.23 mmol) were sequentially added to a reaction flask, and the reaction mixture was stirred at room temperature for 0.5 hours. After the reaction was completed, the reaction mixture was filtered and concentrated. The resulting residue was purified by column chromatography (DCM/MeOH = 10:1) to obtain **I-54-11** as a white solid (65 mg, yield: 91%).

LC-MS: m/z 625 [M-H]⁻.

Step 11: Intermediate **I-54-11** (65 mg, 0.1 mmol), THF (1.5 mL), triphenylphosphine (33 mg, 0.12 mmol) and DEAD (27 mg, 0.16 mmol) were sequentially added to a reaction flask, and the reaction mixture was stirred at room temperature for 0.5 hours. After the reaction was completed, the reaction mixture was filtered and concentrated. The resulting residue was purified by column chromatography (petroleum ether/ethyl acetate = 3:1) to obtain **I-54-12** as a white solid (40 mg, yield: 63.3%).

LC-MS: m/z 609 [M+H]⁺.

Step 12: Referring to the synthesis method of compound **1,** intermediate **I-54-12** was used instead of intermediate **1-1,** and one-step reaction was carried out to obtain intermediate **I-54-13.**

LC-MS: m/z 627 [M+H]⁺.

Step 13: Referring to the synthesis method of compound **1,** intermediate **I-54-13** was used instead of intermediate **1-2,** and one-step reaction was carried out to obtain compound **I-54.**

LC-MS: m/z 527 [M+H]⁺.

¹H NMR (400 MHz, Methanol-d₄) δ 7.58 - 7.52 (m, 2H), 7.51 - 7.43 (m, 3H), 7.43 - 7.37 (m, 1H), 7.00 (d, J = 9.1 Hz, 1H), 4.75 (s, 2H), 4.26 - 4.16 (m, 3H), 4.08 - 4.01 (m, 2H), 3.60 - 3.54 (m, 1H), 3.35 (d, J = 7.3 Hz, 1H), 3.23 - 3.09 (m, 2H), 2.28 - 2.15 (m, 1H), 2.08 - 1.89 (m, 3H).

### Example 33: Synthesis of compound I-55:

### (2S)-5-Chloro-2'-(cyanomethyl)-6,7'-difluoro-2-phenyl-2-((S)-pyrrolidin-2-yl)-2,2',3,3'-tetrahydro-[4,6'-bibenzofuran]-5'-carboxamide

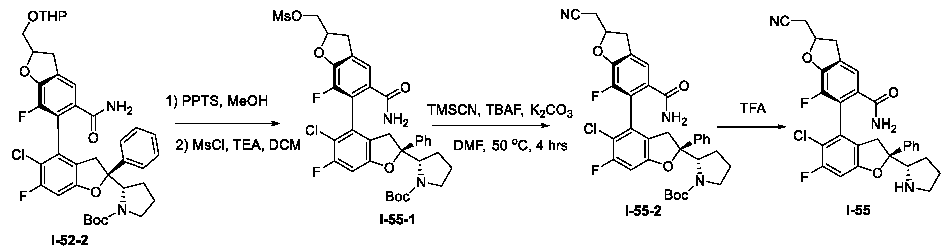

Step 1: Intermediate I**-52-2** (160 mg, 0.225 mmol) was dissolved in methanol (5 mL), and PPTS (5.65 mg, 0.022 mmol) was added thereto at room temperature. The reaction mixture was stirred at room temperature for 6 hours. The reaction mixture was concentrated under reduced pressure to obtain a crude (160 mg). The crude, MSCl (82.2 mg, 0.718 mmol) and TEA (72.62 mg, 0.718 mmol) were dissolved in DCM (4 mL), and the reaction mixture was stirred at room temperature for 3 hours. The filtrate was concentrated under reduced pressure. The residue was purified by silica gel column chromatography (ethyl acetate/petroleum ether = 1/4) to obtain intermediate I**-55-1** (120 mg, yield: 70%).

Step 2: I**-55-1** (65 mg, 92.18 µmol) was dissolved in DMF (2 mL), then K₂CO₃ (25.44 mg, 184.35 µmol), TMSCN (13.72 mg, 138.27 µmol) and TBAF (1 M, 92.18 µL) were added thereto, and the reaction mixture was reacted at 50°C for 4 hours. LC-MS showed that the reaction was completed, and the reaction mixture was prepared by Prep-HPLC (YMC-PACK ODS-A, 250 × 20 mml.D.S-5 µm, 12 nm AA12S05-2520WT, Ser.No.114ZB00083) (FA), and lyophilized to obtain target product I**-55-2** (9 mg, yield: 13.81%) as a white solid.

LCMS: m/z 658.3 [M+H]⁺.

Step 3: I**-55-2** (11.93 mg, 18.76 µmol) was dissolved in DCM (4 mL), then TFA (0.25 mL) was added thereto, and the reaction mixture was reacted at room temperature (30°C) for 2 hours. LC-MS showed the presence of the target product, and the reaction mixture was prepared by Prep-HPLC (YMC-PACK ODS-A, 250 × 20 mml.D.S-5 µm, 12 nm AA12S05-2520WT, Ser.No.114ZB00083) (FA) to obtain target product **I-55** (5.74 mg, yield: 52.06%) as a yellow solid.

¹H NMR (400 MHz, Methanol-d₄) δ 7.64 (d, J = 1.1 Hz, 1H), 7.53 (d, J = 7.4 Hz, 2H), 7.44 (t, J = 7.6 Hz, 2H), 7.36 (t, J = 7.2 Hz, 1H), 7.07 - 6.93 (m, 2H), 6.43 (dd, J = 16.0, 5.7 Hz, 1H), 4.18 (t, J = 8.3 Hz, 1H), 3.55 (d, J = 17.5 Hz, 1H), 3.46 (dd, J = 5.7, 1.7 Hz, 2H), 3.34 - 3.29 (m, 1H), 3.12 (m, 3H), 2.99 (s, 1H), 2.18 (m,1H), 2.08 - 1.85 (m, 4H).

LCMS: m/z 536.3 [M+H]⁺.

The following compounds were synthesized using the corresponding intermediate **A** and intermediate **B** or intermediates **I-B,** using the methods described above or improved methods. If the N-Boc protecting group needs to be removed from the final product, the following conditions can be used for removal: 1) 4 N HCl/1,4-dioxane/methanol (1/1) solution, stirring at room temperature or heating with stirring until the reaction is completed, and the reaction mixture is concentrated and then separated by preparative HPLC to obtain the target product; 2) trifluoroacetic acid/dichloromethane (1/10) solution, reacting at room temperature until the reaction is completed, and the reaction mixture is concentrated and then separated by preparative HPLC to obtain the target product. If the N-Ts protecting group needs to be removed from the final product, 2 N sodium hydroxide/ethanol (1/1) solution can be used with stirring at room temperature or heating with stirring until the reaction is completed, extracting with an organic solvent, and the organic phase is dried and concentrated, and then separated by preparative HPLC to obtain the target product. If the O-TBS protective group needs to be removed from the final product, 4 N HCl/1,4-dioxane/methanol (1/1) solution can be used with stirring at room temperature or heating with stirring until the reaction is completed, and the reaction mixture is concentrated and then separated by preparative HPLC to obtain the target product. If the N-SEM protecting group needs to be removed from the final product, 4 N HCl/1,4-dioxane/methanol (1/1) solution can be used with stirring at room temperature or heating with stirring until the reaction is completed, and the reaction mixture is concentrated and then separated by preparative HPLC to obtain the target product.

The following compounds are free compounds or pharmaceutically acceptable salts.

| **Compound num-ber** | **Starting Intermediate** | **Structural formula** | **Name** | **Analytical data** |
|---|---|---|---|---|
| **2** | **A1** | 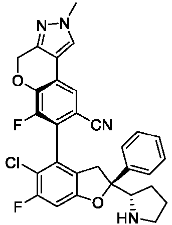 | (*S*)-7-((*S*)-5-Chloro-6-fluoro-2-phenyl-2-((*S*)-pyrrolidin-2-yl)-2,3-dihydrobenzofuran-4-yl)-6-fluoro-2-methyl-2,4-dihydrochromeno[3,4-*c*]pyrazole-8-carbonitrile | LCMS: m/z 545.2 [M+H]⁺. |
| | **B2** | | | |
| **4** | **A1** | 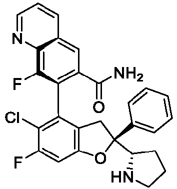 | (*S*)-7-((*S*)-5-Chloro-6-fluoro-2-phenyl-2-((*S*)-pyrrolidin-2-yl)-2,3-dihydrobenzofuran-4-yl)-8-fluoroquinoline-6-carboxamide | ¹H NMR (400 MHz, Methanol-d₄) δ 9.25 (s, 1H), 9.14 (d, *J =* 8.1 Hz, 1H), 8.45 (s, 1H), 8.14 (dd, *J* = 8.1, 4.7 Hz, 1H), 7.47 (ddd, *J* = 28.6, 23.8, 7.4 Hz, 5H), 7.13 (d, *J =* 9.1 Hz, 1H), 4.34 - 4.23 (m, 1H), 3.70 - 3.61 (m, 1H), 3.35 (s, 1H), 3.23 (t, *J =* 17.3 Hz, 2H), 2.30 (d, *J* = 7.3 Hz, 1H), 2.13 - 1.92 (m, 3H). |
| | **B3** | | | |
| | | | | LC-MS: m/z = 506 [M+H]⁺. |
| **4'** | **A1** | 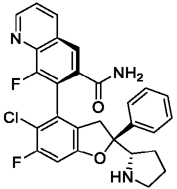 | (*R*)-7-((*S*)-5-Chloro-6-fluoro-2-phenyl-2-((*S*)-pyrrolidin-2-yl)-2,3-dihydrobenzofuran-4-yl)-8-fluoroquinoline-6-carboxamide | ¹H NMR (400 MHz, Methanol-d₄) δ 9.28 (s, 1H), 8.97 (d, *J =* 8.4 Hz, 1H), 8.32 (s, 1H), 8.06 (s, 1H), 7.60 - 7.34 (m, 5H), 7.10 (d, *J =* 9.2 Hz, 1H), 4.29 (s, 1H), 3.63 - 3.57 (m, 2H), 3.40 (d, *J =* 17.6 Hz, 1H), 3.22 (dd, *J =* 15.5, 9.5 Hz, 1H), 2.33 (s, 1H), 2.19 - 2.02 (m, 2H), 1.92 (d, *J* = 8.7 Hz, 1H). |
| | **B3** | | | |
| | | | | LC-MS: m/z = 506 [M+H]⁺. |
| **5** | **A1** | 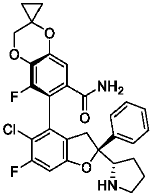 | (*S*)-6-((*S*)-5-Chloro-6-fluoro-2-phenyl-2-((*S*)-pyrrolidin-2-yl)-2,3-dihydrobenzofuran-4-yl)-5-fluoro-3H-spiro[benzo[*b*][1,4]dioxine-2,1'-cyclopropane]-7-carboxamide | LC-MS: m/z 539.2 [M+H]⁺. |
| | **B4** | | | |
| **6** | **A1** | 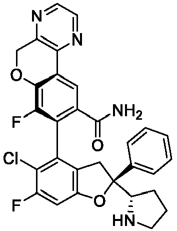 | (*S*)-8-((*S*)-5-Chloro-6-fluoro-2-phenyl-2-((S)-pyrrolidin-2-yl)-2,3-dihydrobenzofuran-4-yl)-7-fluoro-5H-chromeno [3,4-*b*]pyrazine-9-carboxamide | ¹H NMR (400 MHz, Methanol-d₄) δ 8.64 (d, *J =* 2.5 Hz, 1H), 8.54 (d, *J* = 2.6 Hz, 1H), 8.36 (d, *J* = 1.2 Hz, 1H), 7.56 (d, *J =* 7.5 Hz, 2H), 7.48 (t, *J* = 7.7 Hz, 2H), 7.39 (dd, *J =* 13.7, 6.5 Hz, 1H), 7.03 (d, *J =* 9.2 Hz, 1H), 5.53 (s, 2H), 4.31 - 4.21 (m, 1H), 3.67 - 3.56 (m, 2H), 3.21 (t, *J* = 12.9 Hz, 2H), 2.30 - 2.21 (m, 1H), 2.12 - 1.93 (m, 3H). |
| | **B5** | | | |
| | | | | LCMS: m/z 561.1 [M+H]⁺. |
| **7** | **A1** | 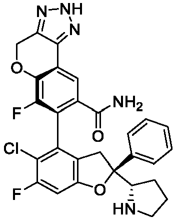 | (*S*)-7-((*S*)-5-Chloro-6-fluoro-2-phenyl-2-((*S*)-pyrrolidin-2-yl)-2,3-dihydrobenzofuran-4-yl)-6-fluoro-2,4-dihydrochromeno [3,4-*d*][1,2,3]triazole-8-carboxamide | ¹H NMR (400 MHz, Methanol-d₄) δ 7.83 (d, *J =* 1.4 Hz, 1H), 7.55 - 7.49 (m, 2H), 7.43 (t, *J* = 7.6 Hz, 2H), 7.39 - 7.30 (m, 1H), 6.93 (d, *J* = 9.2 Hz, 1H), 5.62 (s, 2H), 4.00 (t, *J* = 8.0 Hz, 1H), 3.57 (dd, *J* = 16.3, 1.7 Hz, 1H), 3.25 - 3.09 (m, 2H), 3.08-3.01 (m, 1H), 2.09-2.05 (m, 1H), 1.95-1.82 (m, 3H). |
| | **B6** | | | |
| | | | | LCMS: m/z 550.2 [M+H]⁺. |
| **8** | **A1** | 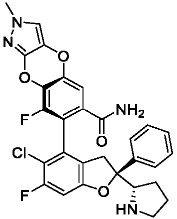 | (*S*)-7-((*S*)-5-Chloro-6-fluoro-2-phenyl-2-((S)-pyrrolidin-2-yl)-2,3-dihydrobenzofuran-4-yl)-8-fluoro-2-methyl-2H-benzo [5,6][1,4]dioxino[2,3-*c*]pyrazole-6-carboxamide | ¹H NMR (500 MHz, Methanol-d₄) δ 7.58 - 7.53 (m, 2H), 7.47 (dd, *J =* 8.5, 6.9 Hz, 2H), 7.41 (d, *J* = 1.3 Hz, 1H), 7.35 (s, 1H), 7.18 (d, *J =* 1.8 Hz, 1H), 7.01 (d, *J* = 9.1 Hz, 1H), 4.24 - 4.18 (m, 1H), 3.69 (s, 3H), 3.61 (d, *J* = 15.7 Hz, 1H), 3.34 (dd, *J* = 7.6, 3.8 Hz, 1H), 3.24 - 3.17 (m, 2H), 2.26 - 2.17 (m, 1H), 2.09 - 2.00 (m, 2H), 1.96 - 1.87 (m, 1H). |
| | **B7** | | | |
| | | | | LCMS: m/z 565.2 [M+H]⁺. |
| **11** | **A1** | 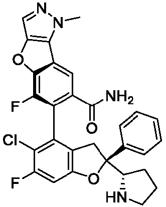 | (*S*)-6-((*S*)-5-Chloro-6-fluoro-2-phenyl-2-((*S*)-pyrrolidin-2-yl)-2,3-dihydrobenzofuran-4-yl)-5-fluoro-1-methyl-1*H-*benzofuro[3,2-*c*]pyrazole-7-carboxamide | LCMS: m/z 549 [M+H]⁺. |
| | **B10** | | | |
| **12** | **A1** | 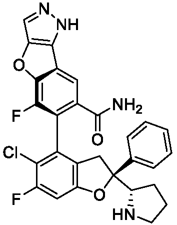 | (*S*)-6-((*S*)-5-Chloro-6-fluoro-2-phenyl-2-((*S*)-pyrrolidin-2-yl)-2,3-dihydrobenzofuran-4-yl)-5-fluoro-2*H*-benzofuro [3,2-*c*]pyrazole-7-carboxamide | LCMS: m/z 535.1 [M+H]⁺. |
| | **B12** | | | |
| **38** | **A6** | 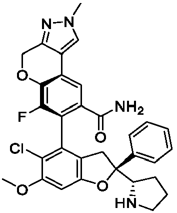 | (*S*)-7-((*S*)-5-Chloro-6-methoxy-2-phenyl-2-((*S*)-pyrrolidin-2-yl)-2,3-dihydrobenzofuran-4-yl)-6-fluoro-2-methyl-2,4-dihydrochromeno[3,4-*c*]pyrazole-8-carboxamide | LCMS: m/z 575.2 [M+H]⁺. |
| | **B2** | | | |
| **39** | **A1** | 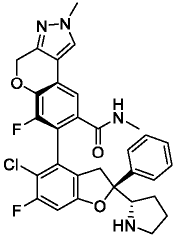 | (*S*)-7-((*S*)-5-Chloro-6-fluoro-2-phenyl-2-((*S*)-pyrrolidin-2-yl)-2,3-dihydrobenzofuran-4-yl)-6-fluoro-*N*,2-dimethyl-2,4-dihydrochromeno[3,4-*c*]pyrazole-8-carboxamide | ¹H NMR (400 MHz, Methanol-d₄) δ 8.52 (s, 1H), 8.00 (s, 1H), 7.58 - 7.51 (m, 3H), 7.45 (t, J = 7.6 Hz, 2H), 7.39 (d, J = 7.3 Hz, 1H), 6.96 (d, J = 9.3 Hz, 1H), 5.36 (s, 2H), 4.11 (dd, J = 9.0, 7.2 Hz, 1H), 3.95 (s, 3H), 3.57 (dd, J = 16.4, 1.8 Hz, 1H), 3.28 - 3.05 (m, 3H), 2.78 (s, 3H), 2.19-2.07 (m, 1H), 2.07 - 1.85 (m, 3H). |
| | **B2** | | | |
| | | | | LCMS: m/z 577 [M+H]⁺. |
| **40** | **A5** | 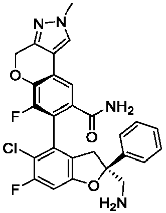 | (*S*)-7-((*S*)-2-(Aminomethyl)-5-chloro-6-fluoro-2-phenyl-2,3-dihydrobenzofuran-4-yl)-6-fluoro-2-methyl-2,4-dihydrochromeno [3,4-*c*]pyrazole-8-carboxamide | LCMS: m/z 523.1 [M+H]⁺. |
| | **B2** | | | |
| **41** | **A11** | 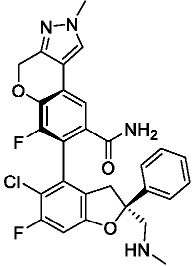 | (*S*)-7-((*S*)-5-Chloro-6-fluoro-2-((methylamino)methyl)-2-phenyl-2,3-dihydrobenzofuran-4-yl)-6-fluoro-2-methyl-2,4-dihydrochromeno [3,4-*c*]pyrazole-8-carboxamide | ¹H NMR (400 MHz, Methanol-d₄) δ 8.04 (s, 1H), 7.77 (s, 1H), 7.51-7.38 (m,5H), 7.21 (d, J = 8 Hz, 1H), 5.37 (s, 2H), 3.96 (s, 3H), 3.59-3.51 (m, 3H), 3.19-3.15 (m, 1H),2.69 (s, 3H). |
| | **B2** | | | |
| | | | | LCMS: m/z 537.1 [M+H]⁺. |
| **42** | **A1** | 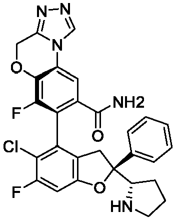 | (*S*)-7-((*S*)-5-Chloro-6-fluoro-2-phenyl-2-((*S*)-pyrrolidin-2-yl)-2,3-dihydrobenzofuran-4-yl)-6-fluoro-4*H-*benzo[*b*][1,2,4]triazolo[4,3-*d*][1,4]oxazine-8-carboxamide | ¹H NMR (400 MHz, Methanol-d₄) δ 9.70 (s, 1H), 8.11 (s, 1H), 7.73 - 7.25 (m, 5H), 7.04 (d, J = 9.1 Hz, 1H), 5.70 (s, 2H), 4.30 (d, J = 10.5 Hz, 1H), 3.72 - 3.59 (m, 2H), 3.26 - 3.11 (m, 2H), 2.28 (d, *J* = 13.8Hz, 1H), 2.04 (t, J = 11.7 Hz, 2H), 1.95 (s, 1H). |
| | **B55** | | | |
| | | | | LC-MS: m/z 550.2 [M+H]⁺. |
| **43** | **A1** | 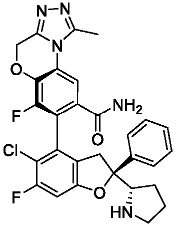 | (*S*)-7-((*S*)-5-Chloro-6-fluoro-2-phenyl-2-((*S*)-pyrrolidin-2-yl)-2,3-dihydrobenzofuran-4-yl)-6-fluoro-1-methyl-4*H-*benzo[*b*][1,2,4]triazolo[4,3-*d*][1,4]oxazine-8-carboxamide | ¹H NMR (400 MHz, Methanol-d₄) δ 7.97 (s, 1H), 7.63 - 7.37 (m, 5H), 7.07 (d, J = 9.0 Hz, 1H), 5.63 (s, 2H), 4.32 (d, J = 8.4 Hz, 1H), 3.65 (d, J = 23.0 Hz, 3H), 3.24 (d, J = 14.2 Hz, 1H), 3.13 (s, 3H), 2.27 (dd, J = 17.6, 7.8 Hz, 1H), 2.13 - 1.94 (m, 3H). |
| | **B56** | | | |
| | | | | LC-MS: m/z 564.2 [M+H]⁺. |
| **44** | **A1** | 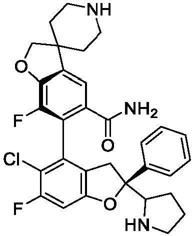 | (*S*)-6-((*S*)-5-Chloro-6-fluoro-2-phenyl-2-((*S*)-pyrrolidin-2-yl)-2,3-dihydrobenzofuran-4-yl)-7-fluoro-2*H-*spiro[benzofuran-3,4'-piperidine]-5-carboxamide | ¹H NMR (400 MHz, Methanol-d₄) δ 7.56 - 7.45 (m, 5H), 7.41 (d, *J =* 7.2 Hz, 1H), 7.00 (d, *J* = 9.1 Hz, 1H), 4.75 (s, 2H), 4.23 (t, *J =* 8.4 Hz, 1H), 3.67 (d, *J =* 5.4 Hz, 1H), 3.60 (s, 2H), 3.57 - 3.48 (m, 3H), 3.23 - 3.10 (m, 4H), 2.34 - 1.97 (m, 8H). |
| | **B9** | | | |
| | | | | LCMS: m/z 566 [M+H]⁺. |
| **46** | **A1** | 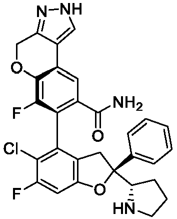 | (*S*)-7-((*S*)-5-Chloro-6-fluoro-2-phenyl-2-((*S*)-pyrrolidin-2-yl)-2,3-dihydrobenzofuran-4-yl)-6-fluoro-2,4-dihydrochromeno[3,4-*c*]pyrazole-8-carboxamide | ¹HNMR(400MHz, MeOD) δ 8.12 (s, 1H), 7.72 (d,J = 1.3Hz, 1H), 7.57 (d,J =7.3Hz, 2H), 7.48 (dd,J = 14.8, 7.0Hz, 2H), 7.41 (t,J = 7.2Hz, 1H), 7.00 (dd,J = 8.7, 3.0Hz, 2H), 6.63 (d,J = 8.4Hz, 1H), 5.44 (s, 2H), 4.26 - 4.20 (m, 1H), 3.61 (d,J = 15.9Hz, 1H), 3.20 (d,J = 14.8Hz, 3H), 2.23 (d,J = 12.3Hz, 2H), 2.02 (dd,J = 20.5, 12.9Hz, 4H). LC-MS: m/z = 549.1 [M+H]⁺. |
| | **B54** | | | |
| **47** | **A1** | 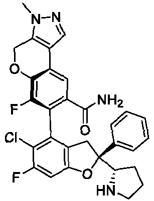 | (*S*)-7-((*S*)-5-Chloro-6-fluoro-2-phenyl-2-((*S*)-pyrrolidin-2-yl)-2,3-dihydrobenzofuran-4-yl)-6-fluoro-3-methy1-3,4-dihydrochromeno[3,4-*c*]pyrazole-8-carboxamide | LC-MS: m/z 563.2 [M+H]⁺. |
| | **B53** | | | |
| **51** | **A25** | 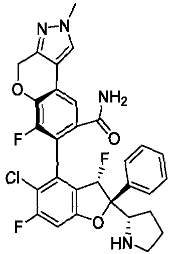 | (*S*)-7-((2*S*,3*S*)-5-Chloro-3,6-difluoro-2-phenyl-2-((*S*)-pyrrolidin-2-yl)-2,3-dihydrobenzofuran-4-yl)-6-fluoro-2-methyl-2,4-dihydrochromeno [3,4-*c*]pyrazole-8-carboxamide | LCMS: m/z 581.2 [M+H]⁺. |
| | **B2** | | | |
| **52** | **A16** | 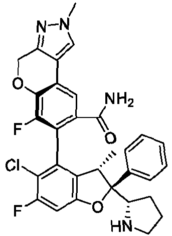 | (*S*)-7-((2*S*,3*S*)-5-Chloro-6-fluoro-3-methyl-2-phenyl-2-((*S*)-pyrrolidin-2-yl)-2,3-dihydrobenzofuran-4-yl)-6-fluoro-2-methyl-2,4-dihydrochromeno[3,4-*c*]pyrazole-8-carboxamide | LCMS: m/z 577.2 [M+H]⁺. |
| | **B2** | | | |
| **53** | **A17** | 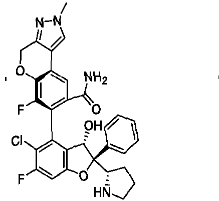 | (*S*)-7-((2*S*,3*S*)-5-Chloro-6-fluoro-3-hydroxy-2-phenyl-2-((*S*)-pyrrolidin-2-yl)-2,3-dihydrobenzofuran-4-yl)-6-fluoro-2-methyl-2,4-dihydrochromeno [3,4-*c*]pyrazole-8-carboxamide | LCMS: m/z 579.2 [M+H]⁺. |
| | **B2** | | | |
| **54** | **A15** | 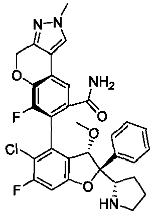 | (*S*)-7-((2*S*,3*S*)-5-Chloro-6-fluoro-3-methoxy-2-phenyl-2-((*S*)-pyrrolidin-2-yl)-2,3-dihydrobenzofuran-4-yl)-6-fluoro-2-methyl-2,4-dihydrochromeno [3,4-*c*]pyrazole-8-carboxamide | LCMS: m/z 593.2 [M+H]⁺. |
| | **B2** | | | |
| **55** | **A20** | 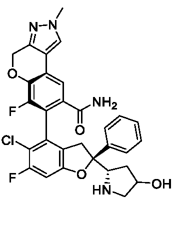 | (7*S*)-7-((2*S*)-5-Chloro-6-fluoro-2-((2*S*)-4-hydroxypyrrolidin-2-yl)-2-phenyl-2,3-dihydrobenzofuran-4-yl)-6-fluoro-2-methyl-2,4-dihydrochromeno[3,4-*c*]pyrazole-8-carboxamide | ¹H NMR (400 MHz, Methanol-d₄) δ 8.02 (s, 1H), 7.49 (s, 1H), 7.45 - 7.36 (m, 5H), 7.04 (d, J = 8.0 Hz, 1H), 5.37 (s, 2H), 4.45 (s, 1H),4.25 - 4.21 (m, 1H), 3.95 (s, 3H), 3.68 - 3.65 (m, 1H), 3.34- 3.30 (m, 2H), 3.30 - 3.27 (m, 1H), 2.07 - 2.04 (m, 1H), 1.35 - 1.26 (m, 1H). |
| | **B2** | | | |
| | | | | LCMS: m/z 579.1 [M+H]⁺. |
| **62** | **A1** | 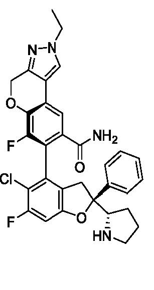 | (*S*)-7-((*S*)-5-Chloro-6-fluoro-2-phenyl-2-((*S*)-pyrrolidin-2-yl)-2,3-dihydrobenzofuran-4-yl)-2-ethyl-6-fluoro-2,4-dihydrochromeno [3,4-*c*]pyrazole-8-carboxamide | ¹H NMR (400 MHz, Methanol-d₄) δ 7.98 (s, 1H), 7.70 (d, *J =* 1.4 Hz, 1H), 7.60 - 7.54 (m, 2H), 7.47 (t, *J* = 7.6 Hz, 2H), 7.44 - 7.35 (m, 1H), 6.99 (d, *J* = 9.2 Hz, 1H), 5.59 (s, 2H), 4.21 (dq, *J =* 22.4, 7.4 Hz, 3H), 3.68 - 3.55 (m, 1H), 3.35 (d, *J* = 6.7 Hz, 1H), 3.19 (ddd, *J* = 18.6, 15.3, 4.7 Hz, 2H), 2.29 - 2.18 (m, 1H), 2.11 - 1.88 (m, 3H), 1.44 (t, *J = 7.2* Hz, 3H). |
| | **B39** | | | |
| | | | | LCMS: m/z 577 [M+H]⁺. |
| **66** | **A23** | 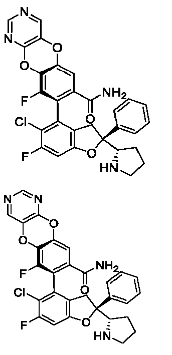 (1:1 mixture) | (*S*)-8-((*S*)-5-Chloro-6-fluoro-2-phenyl-2-((*S*)-pyrrolidin-2-yl)-2,3-dihydrobenzofuran-4-yl)-9-fluorobenzo[5,6][1,4]dioxo [2,3-*d*]pyrimidine-7-carboxamide (*S*)-7-((*S*)-5-Chloro-6-fluoro-2-phenyl-2-((*S*)-pyrrolidin-2-yl)-2,3-dihydrobenzofuran-4-yl)-6-fluorobenzo[5,6][1,4]dioxo [2,3-*d*]pyrimidine-8-carboxamide | ¹H NMR (400 MHz, DMSO-d₆) δ 9.02 (d, *J* = 17.4 Hz, 1H), 8.54 (s, 1H), 8.48 (s, 1H), 8.33 (s, 1H), 7.96 (s, 1H), 7.52 (d, *J* = 7.3 Hz, 3H), 7.46 (t, *J =* 7.5 Hz, 2H), 7.39 (d, *J =* 6.9 Hz, 1H), 7.37 (d, *J =* 1.5 Hz, 1H), 7.16 (d, *J* = 9.4 Hz, 1H), 4.30 (s, 1H), 3.21 - 3.02 (m, 4H), 2.18 (t, *J* = 7.4 Hz, 1H), 1.87 (dd, *J =* 17.3, 10.2 Hz, 2H), 1.72 (dd, *J =* 14.7, 6.4 Hz, 1H). |
| | | | | LCMS: m/z 563 [M+H]⁺. |
| **99** | **A1** | 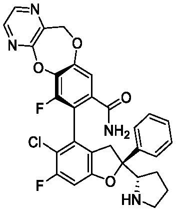 | (*S*)-7-((*S*)-5-Chloro-6-fluoro-2-phenyl-2-((*S*)-pyrrolidin-2-yl)-2,3-dihydrobenzofuran-4-yl)-6-fluoro-11*H-*benzo[2,3][1,4]dioxa [5,6-*b*]pyrazine-8-carboxamide | LCMS: m/z 577 [M+H]⁺. |
| | **B71** | | | |
| **101** | **A23** | 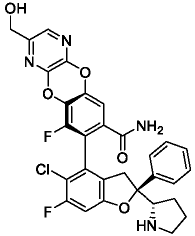 | (*S*)-8-((*S*)-5-Chloro-6-fluoro-2-phenyl-2-((*S*)-pyrrolidin-2-yl)-2,3- dihydrobenzofuran-4-yl)-9-fluoro-2-(hydroxymethyl)benzo[5,6][ 1,4]dioxo[2,3-*b*]pyrazine-7-carboxamide | ¹H NMR (400 MHz, Methanol-d₄) δ 7.97 (s, 1H), 7.62 - 7.35 (m, 5H), 7.28 (d, J = 1.8 Hz, 1H), 7.02 (d, J = 9.1 Hz, 1H), 4.57 (s, 2H), 4.22 (t, J = 8.4 Hz, 1H), 3.62 (d, J = 16.5 Hz, 1H), 3.34 (s, 1H), 3.24 - 3.15 (m, 2H), 2.30 - 2.20 (m, 1H), 2.08 - 1.99 (m, 2H), 1.92 (dd, J = 12.7, 8.5 Hz, 1H). |
| | **C2** | | | |
| | | | | LCMS: m/z 593 [M+H]⁺. |
| **102** | **A23** | 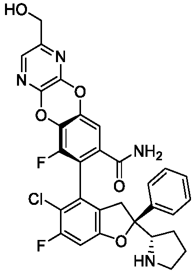 | (*S*)-8-((*S*)-5-Chloro-6-fluoro-2-phenyl-2-((*S*)-pyrrolidin-2-yl)-2,3-dihydrobenzofuran-4-yl)-9-fluoro-3-(hydroxymethyl)benzo[5,6][ 1,4]dioxo [2,3-*b*]pyrazine-7-carboxamide | ¹H NMR (400 MHz, Methanol-d₄) δ 7.93 (d, J = 0.9 Hz, 1H), 7.60 - 7.38 (m, 5H), 7.28 (d, J = 1.8 Hz, 1H), 7.03 (d, J = 9.1 Hz, 1H), 4.59 (d, J = 0.8 Hz, 2H), 4.24 (dd, J = 9.5, 7.3 Hz, 1H), 3.62 (dd, J = 16.2, 1.5 Hz, 1H), 3.35 (d, J = 7.1 Hz, 1H), 3.24 - 3.16 (m, 2H), 2.31 - 2.20 (m, 1H), 2.03 (dt, J *=* 13.5, 5.2 Hz, 2H), 1.96 - 1.88 (m, 1H). |
| | **C2** | | | |
| | | | | LCMS: m/z 593 [M+H]⁺. |
| **104** | **A1** | 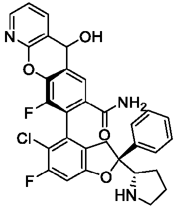 | (*S*)-8-((*S*)-5-Chloro-6-fluoro-2-phenyl-2-((*S*)-pyrrolidin-2-yl)-2,3-dihydrobenzofuran-4-yl)-9-fluoro-5-hydroxy-5*H-*benzo [2,3-*b*]pyridine-7-carboxamide | ¹H NMR (400 MHz, DMSO-d₆) δ 8.36 (d, J *=* 13.0 Hz, 2H), 8.16 (d, J = 7.4 Hz, 1H), 7.78 (d, J = 15.5 Hz, 1H), 7.57 - 7.37 (m, 5H), 7.22 - 7.16 (m, 1H), 5.94 - 5.84 (m, 1H), 3.68 - 3.65 (m, 1H), 3.30 - 3.27 (m, 4H), 2.10 - 1.90 (m, 4H). |
| | **B70** | | | |
| | | | | LCMS: m/z 576.1 [M+H]⁺. |
| **107** | **A23** | 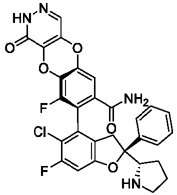 | (*S*)-8-((*S*)-5-Chloro-6-fluoro-2-phenyl-2-((*S*)-pyrrolidin-2-yl)-2,3-dihydrobenzofuran-4-yl)-9-fluoro-1-oxo-1,2-dihydrobenzo[5,6][1,4]diox o [2,3-*d*]pyridazine-7-carboxamide | ¹H NMR (400 MHz, DMSO-d₆) δ 13.31 (s, 1H), 9.14 (s, 1H), 8.34 (s, 1H), 7.95 (s, 1H), 7.93 (s, 1H), 7.53 (d, *J* = 7.2 Hz, 3H), 7.46 (t, *J* = 7.5 Hz, 2H), 7.41 - 7.35 (m, 1H), 7.22 (d, *J =* 1.6 Hz, 1H), 7.16 (d, *J* = 9.3 Hz, 1H), 4.30 (s, 1H), 3.58 (d, *J =* 16.4 Hz, 1H), 3.16 (s, 1H), 3.05 (t, *J =* 8.3 Hz, 2H), 2.18 (s, 1H), 1.91 - 1.82 (m, 2H), 1.75 - 1.70 (m, 1H). |
| | | | | LCMS: m/z 579 [M+H]⁺. |
| **108** | **A23** | 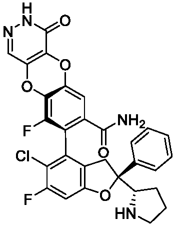 | (*S*)-8-((*S*)-5-Chloro-6-fluoro-2-phenyl-2-((*S*)-pyrrolidin-2-yl)-2,3-dihydrobenzofuran-4-yl)-9-fluoro-4-oxo-3,4-dihydrobenzo[5,6][1,4]diox o [2,3-*d*]pyridazine-7-carboxamide | ¹H NMR (400 MHz, DMSO-d₆) δ 13.31 (s, 1H), 9.13 (s, 1H), 8.32 (s, 1H), 7.95 (s, 1H), 7.91 (s, 1H), 7.53 (d, *J* = 7.1 Hz, 3H), 7.46 (t, *J* = 7.6 Hz, 2H), 7.38 (t, *J* = 7.2 Hz, 1H), 7.27 (s, 1H), 7.16 (d, *J* = 9.3 Hz, 1H), 4.30 (s, 1H), 3.58 (d, *J =* 17.5 Hz, 1H), 3.11 (d, *J* = 40.9 Hz, 3H), 2.19 (d, *J =* 7.5 Hz, 1H), 1.92 - 1.82 (m, 2H), 1.71 (s, 1H). |
| | | | | LCMS: m/z 579 [M+H]⁺. |
| **109** | **A23** | 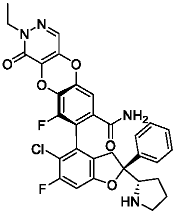 | (*S*)-8-((*S*)-5-Chloro-6-fluoro-2-phenyl-2-((*S*)-pyrrolidin-2-yl)-2,3-dihydrobenzofuran-4-yl)-2-ethyl-9-fluoro-1-oxo-1,2-dihydrobenzo[5,6][1,4]diox o [2,3-*d*]pyridazine-7-carboxamide | ¹H NMR (400 MHz, DMSO-d₆) δ 9.00 (s, 1H), 8.33 (s, 1H), 8.01 (s, 1H), 7.97 (s, 1H), 7.53 (d, *J* = 7.2 Hz, 2H), 7.46 (t, *J* = 7.6 Hz, 2H), 7.39 (t, *J* = 9.2 Hz, 2H), 7.22 (s, 1H), 7.17 (d, *J =* 9.3 Hz, 1H), 4.29 (s, 1H), 4.10 - 4.01 (m, 2H), 3.56 (s, 1H), 3.50 (s, 3H), 3.16 (s, 1H), 3.05 (d, *J =* 16.6 Hz, 2H), 2.18 (s, 1H), 1.85 (s, 2H), 1.68 (s, 1H). |
| | | | | LCMS: m/z 607 [M+H]⁺. |
| **110** | **A23** | 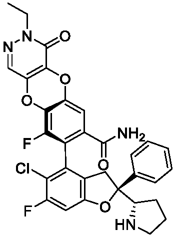 | (*S*)-8-((*S*)-5-Chloro-6-fluoro-2-phenyl-2-((*S*)-pyrrolidin-2-yl)-2,3-dihydrobenzofuran-4-yl)-3-ethyl-9-fluoro-4-oxo-3,4-dihydrobenzo[5,6][1,4]diox o [2,3-*d*]pyridazine-7-carboxamide | ¹H NMR (400 MHz, DMSO-d₆) δ 9.03 (s, 1H), 8.33 (s, 1H), 8.01 (s, 1H), 7.96 (s, 1H), 7.53 (d, *J* = 7.4 Hz, 2H), 7.46 (t, *J* = 7.6 Hz, 2H), 7.40 (d, *J* = 8.5 Hz, 2H), 7.31 (d, *J* = 1.5 Hz, 1H), 7.16 (d, *J =* 9.3 Hz, 1H), 4.30 (s, 1H), 4.07 (q, *J* = 7.2 Hz, 2H), 3.58 (d, *J* = 17.2 Hz, 1H), 3.41 (s, 3H), 3.16 (s, 1H), 3.03 (d, *J* = 16.8 Hz, 2H), 2.18 (t, *J* = 7.4 Hz, 1H), 1.87 (dd, *J =* 18.1, 9.9 Hz, 2H), 1.75 - 1.67 (m, 1H). |
| | | | | LCMS: m/z 607 [M+H]⁺. |
| **113** | **A23** | 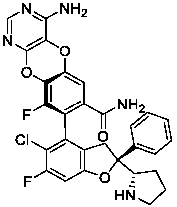 | (*S*)-4-Amino-8-((*S*)-5-chloro-6-fluoro-2-phenyl-2-((*S*)-pyrrolidin-2-yl)-2,3 - dihydrobenzofuran-4-yl)-9-fluorobenzo[5,6][1,4]dioxo [2,3-*d*]pyrimidine-7-carboxamide | ¹H NMR (400 MHz, DMSO-d₆) δ 9.01 (s, 1H), 8.33 (s, 1H), 7.94 (s, 1H), 7.87 (s, 1H), 7.49 (dt, *J =* 20.3, 7.5 Hz, 4H), 7.42 - 7.35 (m, 1H), 7.28 (d, *J* = 1.6 Hz, 1H), 7.16 (d, *J* = 9.3 Hz, 1H), 7.12 (s, 1H), 4.31 (s, 1H), 3.58 (d, *J =* 17.1 Hz, 1H), 3.20 - 2.97 (m, 3H), 2.19 (d, *J =* 6.8 Hz, 1H), 1.95 - 1.80 (m, 2H), 1.78 - 1.68 (m, 1H). |
| | | | | LCMS: m/z 578 [M+H]⁺. |
| **114** | **A23** | 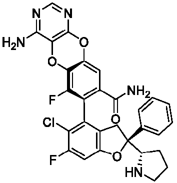 | (*S*)-4-Amino-7-((*S*)-5-chloro-6-fluoro-2-phenyl-2-((*S*)-pyrrolidin-2-yl)-2,3-dihydrobenzofuran-4-yl)-6-fluorobenzo [5,6][1,4]dioxo [2,3-*d*]pyrimidine-8-carboxamide | ¹H NMR (400 MHz, DMSO-d₆) δ 8.27 - 8.22 (m, 1H), 7.51 - 7.33 (m, 5H), 7.26 (d, *J =* 8.7 Hz, 2H), 7.23 -7.18 (m, 1H), 6.83 (s, 1H), 6.70 - 6.65 (m, 1H), 6.63 (s, 1H), 3.81 (s, 1H), 3.51 (s, 2H), 2.93 (s, 2H), 1.99 (d, *J =* 7.5 Hz, 4H). |
| | | | | LCMS: m/z 578 [M+H]⁺. |
| **116** | **A24** | 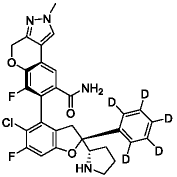 | (*S*)-7-((*S*)-5-Chloro-6-fluoro-2-(phenyl-*d*₅)-2-((*S*)-pyrrolidin-2-yl)-2,3-dihydrobenzofuran-4-yl)-6-fluoro-2-methyl-2,4-dihydrochromeno [3,4-*c*]pyrazole-8-carboxamide | ¹H NMR (400 MHz, Methanol-d₄) δ 7.99 (s, 1H), 7.60 (d, *J =* 1.4 Hz, 1H), 6.91 (d, *J* = 9.3 Hz, 1H), 5.34 (s, 2H), 3.95 (m, 1H), 3.93 (s, 3H), 3.61-3.46 (m, 1H), 3.17-3.07 (m, 2H), 3.04-2.95 (m, 1H), 2.15-1.70 (m, 4H). |
| | **B2** | | | |
| | | | | LCMS: m/z 568.30 [M+H]⁺. |
| **117** | **A24** | 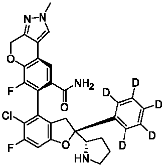 | (*S*)-7-((*S*)-5-Chloro-6-fluoro-2-(phenyl-d₅)-2-((*S*)-pyrrolidin-2-yl)-2,3-dihydrobenzofuran-4-yl)-6-fluoro-2-methyl-2,4-dihydrochromeno [3,4-*c*]pyrazole-8-carboxamide | ¹H NMR (400 MHz, Methanol-d₄) δ 7.98 (s, 1H), 7.56 (d, *J =* 1.6 Hz, 1H), 6.91 (d, *J* = 9.3 Hz, 1H), 5.38 (s, 2H), 4.00-3.94 (m, 1H), 3.93 (s, 3H), 3.44 (d, *J* = 17.5 Hz, 1H), 3.31 (d, *J* = 1.6 Hz, 1H), 3.16-2.92 (m, 2H), 2.11-1.66 (m, 4H). |
| | **B2** | | | |
| | | | | LCMS: m/z 568.40 [M+H]⁺. |
| **122 (or 123)** | **A23** | 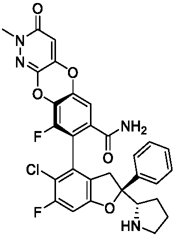 | (*S*)-8-((*S*)-5-Chloro-6-fluoro-2-phenyl-2-((*S*)-pyrrolidin-2-yl)-2,3-dihydrobenzofuran-4-yl)-9-fluoro-2-methyl-3-oxo-2,3-trihydrobenzo [5,6][1,4]dioxo[2,3-*c*]pyridazine-7-carboxamide | ¹H NMR (400 MHz, Methanol-*d*₄) δ 7.58 - 7.52 (m, 2H), 7.48 (dd, J = 8.5, 6.7 Hz, 2H), 7.43 - 7.38 (m, 1H), 7.34 (d, J = 1.8 Hz, 1H), 7.03 (d, J = 9.2 Hz, 1H), 4.25 (dd, J = 9.5, 7.3 Hz, 1H), 3.62 (s, 4H), 3.34 (t, J = 5.2 Hz, 1H), 3.23 - 3.13 (m, 2H), 2.30 - 2.22 (m, 1H), 2.03 (q, J = 7.0, 6.2 Hz, 2H), 1.93 (dt, J = 12.9, 8.8 Hz, 1H). |
| | | | | LCMS: m/z 593 [M+H]⁺. |
| **123 (or 122)** | **A23** | 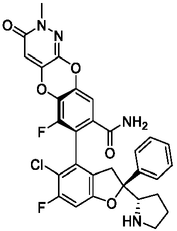 | (*S*)-7-((*S*)-5-Chloro-6-fluoro-2-phenyl-2-((*S*)-pyrrolidin-2-yl)-2,3-dihydrobenzofuran-4-yl)-6-fluoro-1-methyl-3-oxo-2,3-trihydrobenzo [5,6][1,4]dioxo [2,3-*c*]pyridazine-8-carboxamide | ¹H NMR (400 MHz, Methanol-d₄) δ 7.58 - 7.52 (m, 2H), 7.51 - 7.45 (m, 2H), 7.44 - 7.38 (m, 1H), 7.34 (d, J = 1.8 Hz, 1H), 7.03 (d, J = 9.1 Hz, 1H), 6.56 (s, 1H), 4.26 (dd, J = 9.5, 7.3 Hz, 1H), 3.60 (s, 4H), 3.36 -3.32 (m, 1H), 3.23 -3.15 (m, 2H), 2.31 - 2.17 (m, 1H), 2.03 (dd, J = 14.3, 6.9 Hz, 2H), 1.96 - 1.86 (m, 1H). |
| | | | | LCMS: m/z 593 [M+H]⁺. |
| **128** | **A23** | 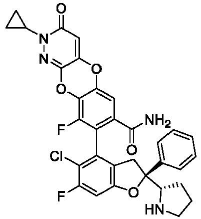 | (*S*)-8-((*S*)-5-Chloro-6-fluoro-2-phenyl-2-((*S*)-pyrrolidin-2-yl)-2,3-dihydrobenzofuran-4-yl)-2-cyclopropyl-9-fluoro-3-oxo-2,3-hydrobenzo[5,6][1,4]dioxo [2,3-*c*]pyridazine-7-carboxamide | ¹H NMR (400 MHz, Methanol-d₄) δ 7.57 - 7.38 (m, 5H), 7.33 (d, J = 1.7 Hz, 1H), 7.03 (d, J = 9.1 Hz, 1H), 6.56 (s, 1H), 4.32 - 4.19 (m, 1H), 3.88 (dt, J = 7.6, 3.5 Hz, 1H), 3.61 (d, J = 16.6 Hz, 1H), 3.35 (d, J = 7.3 Hz, 1H), 3.23 - 3.13 (m, 2H), 2.30 - 2.21 (m, 1H), 2.04 (dt, J = 13.6, 7.3 Hz, 2H), 1.92 (dd, J = 12.6, 8.8 Hz, 1H), 1.08 - 0.96 (m, 4H). |
| | | | | LCMS: m/z 619 [M+H]⁺. |
| **129** | **A23** | 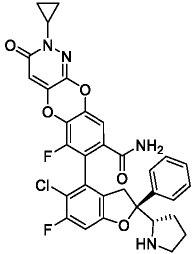 | (*S*)-8-((*S*)-5-Chloro-6-fluoro-2-phenyl-2-((*S*)-pyrrolidin-2-yl)-2,3-dihydrobenzofuran-4-yl)-2-cyclopropyl-9-fluoro-3-oxo-2,3-hydrobenzo[5,6][1,4]dioxo [2,3-*c*]pyridazine-7-carboxamide | ¹H NMR (400 MHz, Methanol-d₄) δ 7.58 - 7.37 (m, 5H), 7.34 (d, J = 1.9 Hz, 1H), 7.03 (d, J = 9.1 Hz, 1H), 6.55 (s, 1H), 4.26 (dd, J = 9.5, 7.3 Hz, 1H), 3.87 (tt, J = 7.6, 4.0 Hz, 1H), 3.61 (dd, J = 16.2, 1.5 Hz, 1H), 3.37 - 3.32 (m, 1H), 3.24 - 3.13 (m, 2H), 2.30 - 2.19 (m, 1H), 2.05 (dq, J = 14.1, 7.0, 6.5 Hz, 2H), 1.95 - 1.85 (m, 1H), 1.05 (dd, J = 4.0, 2.4 Hz, 2H), 1.00 - 0.92 (m, 2H). |
| | | | | LCMS: m/z 619 [M+H]⁺. |
| **137** | **A23** | 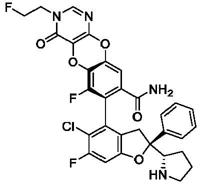 | (*S*)-7-((*S*)-5-Chloro-6-fluoro-2-phenyl-2-((*S*)-pyrrolidin-2-yl)-2,3-dihydrobenzofuran-4-yl)-6-fluoro-3-(2-fluoroethyl)-4-oxo-3,4-dihydrobenzo[5,6][1,4]diox o [2,3-*d*]pyrimidine-8-carboxamide | ¹H NMR (400 MHz, DMSO-d₆) δ 9.16 (s, 1H), 8.29 (s, 1H), 8.23 (d, *J* = 7.9 Hz, 1H), 7.95 (s, 1H), 7.52 (d, *J* = 7.2 Hz, 3H), 7.45 (t, *J* = 7.5 Hz, 2H), 7.41 - 7.36 (m, 1H), 7.33 (s, 1H), 7.16 (d, *J =* 9.3 Hz, 1H), 4.79 (d, *J* = 3.9 Hz, 1H), 4.67 (s, 2H), 4.61 (d, *J* = 3.5 Hz, 1H), 4.30 (s, 1H), 3.58 (d, *J* = 17.0 Hz, 1H), 3.15 (s, 2H), 3.03 (d, *J=* 17.3 Hz, 1H), 2.17 (s, 1H), 1.86 (dd,J= 18.7, 10.7 Hz, 2H), 1.76 - 1.69 (m, 1H). |
| | | | | LCMS: m/z 625 [M+H]⁺. |
| **138** | **A23** | 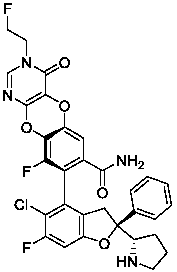 | (*S*)-8-((*S*)-5-Chloro-6-fluoro-2-phenyl-2-((*S*)-pyrrolidin-2-yl)-2,3-dihydrobenzofuran-4-yl)-9-fluoro-3-(2-fluoroethyl)-4-oxo-3,4-dihydrobenzo[5,6][1,4]diox o [2,3-*d*]pyrimidine-7-carboxamide | ¹H NMR (400 MHz, DMSO-d₆) δ 9.50 (s, 1H), 8.35 (s, 1H), 8.27 (d, *J* = 8.7 Hz, 1H), 7.94 (s, 1H), 7.53 (d, *J =* 7.7 Hz, 3H), 7.44 (t, *J =* 7.4 Hz, 2H), 7.38 (d, *J* = 7.1 Hz, 1H), 7.28 (s, 1H), 7.15 (d, *J =* 9.3 Hz, 1H), 4.71 (s, 1H), 4.60 (s, 1H), 4.29 (s, 2H), 4.22 (s, 1H), 3.59 (d, *J =* 17.0 Hz, 1H), 3.12 (s, 2H), 3.03 (d, *J =* 17.2 Hz, 1H), 2.15 (s, 1H), 1.92 - 1.77 (m, 2H), 1.74 (d, *J* = 8.0 Hz, 1H). |
| | | | | LCMS: m/z 625 [M+H]⁺. |
| **139** | **A23** | 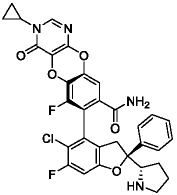 | (*S*)-7-((*S*)-5-Chloro-6-fluoro-2-phenyl-2-((*S*)-pyrrolidin-2-yl)-2,3-dihydrobenzofuran-4-yl)-3-cyclopropyl-6-fluoro-4-oxo-3,4-dihydrobenzo[5,6][1,4]diox o [2,3-*d*]pyrimidine-8-carboxamide | ¹H NMR (400 MHz, DMSO-d₆) δ 9.14 (s, 1H), 8.31 (s, 1H), 8.26 (s, 1H), 7.95 (s, 1H), 7.46 (tt, *J =* 20.7, 14.8 Hz, 6H), 7.30 (d, *J =* 17.1 Hz, 1H), 7.16 (d, *J =* 9.1 Hz, 1H), 4.37 (d, *J* = 3.3 Hz, 1H), 4.30 (s, 1H), 3.12 (d, *J =* 32.3 Hz, 4H), 2.18 (s, 1H), 1.86 (dd, *J* = 17.9, 10.4 Hz, 2H), 1.73 (d, *J =* 12.6 Hz, 1H), 0.87 - 0.78 (m, 4H). |
| | | | | LCMS: m/z 619 [M+H]⁺. |
| **140** | **A23** | 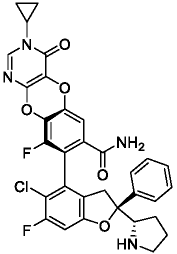 | (*S*)-8-((*S*)-5-Chloro-6-fluoro-2-phenyl-2-((*S*)-pyrrolidin-2-yl)-2,3-dihydrobenzofuran-4-yl)-3-cyclopropyl-9-fluoro-4-oxo-3,4-dihydrobenzo[5,6][1,4]diox o [2,3-*d*]pyrimidine-7-carboxamide | ¹H NMR (400 MHz, DMSO-*d*₆) δ 9.07 (s, 1H), 8.33 (s, 1H), 8.19 (d, *J* = 7.8 Hz, 1H), 7.93 (s, 1H), 7.56 - 7.34 (m, 6H), 7.27 (s, 1H), 7.16 (d, *J =* 9.3 Hz, 1H), 4.28 (s, 1H), 3.58 (d, *J =* 17.0 Hz, 1H), 3.20 (s, 2H), 3.11 - 3.06 (m, 2H), 2.18 (s, 2H), 1.84 (d, *J* = 7.4 Hz, 1H), 1.74 (s, 1H), 1.01 (d, *J =* 6.9 Hz, 2H), 0.93 (d, *J* = 3.9 Hz, 2H). |
| | | | | LCMS: m/z 619 [M+H]⁺. |
| **145** | **A23** | 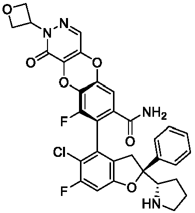 | (*S*)-8-((*S*)-5-Chloro-6-fluoro-2-phenyl-2-((*S*)-pyrrolidin-2-yl)-2,3-dihydrobenzofuran-4-)-9-fluoro-2-(oxetan-3-yl)-1-oxo-1,2-dihydrobenzo[5,6][1,4]diox o [2,3-*d*]pyridazine-7-carboxamide | ¹H NMR (400 MHz, Methanol-d₄) δ 8.01-7.93 (m, 1H), 7.57 - 7.50 (m, 2H), 7.48 - 7.33 (m, 3H), 7.20 (d, J = 1.9 Hz, 1H), 7.02 (d, J *=* 9.1 Hz, 1H), 4.99 - 4.89 (m, 2H), 4.85 - 4.79 (m, 1H), 4.26 (t, J = 8.5 Hz, 1H), 3.96 - 3.81 (m, 2H), 3.64 (d, J = 16.6 Hz, 1H), 3.35 (d, J = 7.3 Hz, 1H), 3.25 - 3.12 (m, 2H), 2.26 (d, J = 8.8 Hz, 1H), 2.05 (q, J = 7.6 Hz, |
| | | | | 2H), 1.95 - 1.84 (m, 1H). |
| | | | | LCMS: m/z 635 [M+H]⁺. |
| **146** | **A23** | 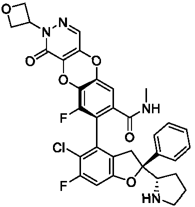 | (*S*)-8-((*S*)-5-Chloro-6-fluoro-2-phenyl-2-((*S*)-pyrrolidin-2-yl)-2,3-dihydrobenzofuran-4-yl)-9-fluoro-*N*-methyl-2-(oxetan-3-yl)-1-oxo-1,2-dihydrobenzo[5,6][1,4]diox o [2,3-*d*]pyridazine-7-carboxamide | LCMS: m/z 649 [M+H]⁺. |
| **148** | **A28** | 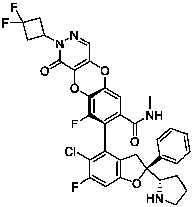 | (*S*)-8-((*S*)-5-Chloro-6-fluoro-2-phenyl-2-((*S*)-pyrrolidin-2-yl)-2,3-dihydrobenzofuran-4-yl)-2-(3,3-difluorocyclobutyl)-9-fluoro-*N*-methyl-1-oxo-1,2-dihydrobenzo[5,6][1,4]diox o [2,3-*d*]pyridazine-7-carboxamide | LCMS: m/z 683 [M+H]⁺. |
| **150** | **A28** | 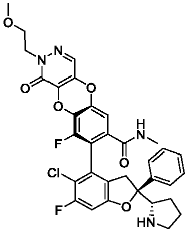 | (*S*)-8-((*S*)-5-Chloro-6-fluoro-2-phenyl-2-((*S*)-pyrrolidin-2-yl)-2,3-dihydrobenzofuran-4-yl)-9-fluoro-2-(2-methoxyethyl)-*N*-methyl-1-oxo-1,2-dihydrobenzo[5,6][1,4]diox o [2,3-*d*]pyridazine-7-carboxamide | ] LCMS: m/z 651 [M+H]⁺. |
| **151** | **A23** | 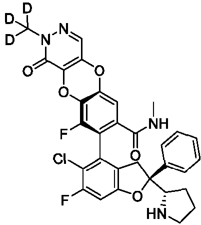 | (*S*)-8-((*S*)-5-Chloro-6-fluoro-2-phenyl-2-((*S*)-pyrrolidin-2-yl)-2,3-dihydrobenzofuran-4-yl)-9-fluoro-*N*-methyl-2-(methyl-d₃)-1-oxo-1,2-dihydrobenzo[5,6][1,4]diox o [2,3-*d*]pyridazine-7-carboxamide | LCMS: m/z 610 [M+H]⁺. |
| **152** | **A27** | 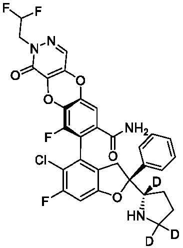 | (*S*)-8-((*S*)-5-Chloro-6-fluoro-2-phenyl-2-((*S*)-pyrrolidin-2-yl-2,5,5-*d*₃)-2,3-dihydrobenzofuran-4-yl)-2-(2,2-difluoroethyl)-9-fluoro-1-oxo-1,2-dihydrobenzo[5,6][1,4]diox o [2,3-*d*]pyridazine-7-carboxamide | LCMS: m/z 646 [M+H]⁺. |
| **153** | **A27** | 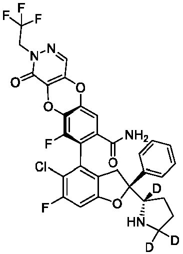 | (*S*)-8-((*S*)-5-Chloro-6-fluoro-2-phenyl-2-((*S*)-pyrrolidin-2-yl-2,5,5-*d*₃)-2,3 -dihydrobenzofuran-4-yl)-9-fluoro-1-oxo-2-(2,2,2-trifluoroethyl)-1,2-dihydroxybenzo[5,6][1,4]di oxo [2,3-*d*]pyridazine-7-carboxamide | LCMS: m/z 664 [M+H]⁺. |
| **154** | **A27** | 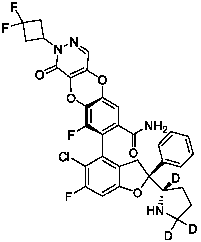 | (*S*)-8-((*S*)-5-Chloro-6-fluoro-2-phenyl-2-((*S*)-pyrrolidin-2-yl-2,5,5-*d*₃)-2,3 -dihydrobenzofuran-4-yl)-2-(3,3-difluorocyclobutyl)-9-fluoro-1-oxo-1,2-dihydrobenzo[5,6][1,4]diox o [2,3-*d*]pyridazine-7-carboxamide | LCMS: m/z 672 [M+H]⁺. |
| **155** | **A27** | 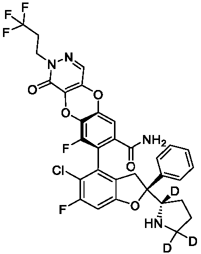 | (*S*)-8-((*S*)-5-Chloro-6-fluoro-2-phenyl-2-((*S*)-pyrrolidin-2-yl-2,5,5-*d*₃)-2,3-dihydrobenzofuran-4-yl)-9-fluoro-1-oxo-2-(3,3,3-trifluoropropyl)-1,2-dihydroxybenzo[5,6][1,4]di oxo [2,3-*d*]pyridazine-7-carboxamide | LCMS: m/z 678 [M+H]⁺. |
| **156** | **A27** | 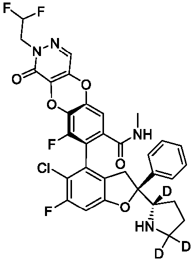 | (*S*)-8-((*S*)-5-Chloro-6-fluoro-2-phenyl-2-((*S*)-pyrrolidin-2-yl-2,5,5-*d*₃)-2,3-dihydrobenzofuran-4-yl)-2-(2,2-difluoroethyl)-9-fluoro-*N*-methyl-1-oxo-1,2-dihydrobenzo[5,6][1,4]diox o [2,3-*d*]pyridazine-7-carboxamide | LCMS: m/z 660 [M+H]⁺. |
| **157** | **A27** | 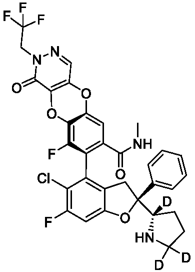 | (*S*)-8-((*S*)-5-Chloro-6-fluoro-2-phenyl-2-((*S*)-pyrrolidin-2-yl-2,5,5-d3)-2,3-dihydrobenzofuran-4-yl)-9-fluoro-*N*-methyl-1-oxo-2-(2,2,2-trifluoroethyl)-1,2-dihydroxybenzo[5,6][1,4]di oxo [2,3-*d*]pyridazine-7-carboxamide | LCMS: m/z 678 [M+H]⁺. |
| **158** | **A27** | 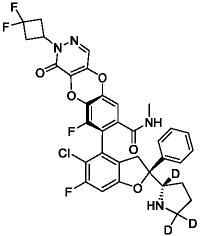 | (*S*)-8-((*S*)-5-Chloro-6-fluoro-2-phenyl-2-((*S*)-pyrrolidin-2-yl-2,5,5-*d*₃)-2,3 -dihydrobenzofuran-4-yl)-2-(3,3-difluorocyclobutyl)-9-fluoro-*N-*methyl-1-oxo-1,2-dihydrobenzo[5,6][1,4]diox o [2,3-*d*]pyridazine-7-carboxamide | LCMS: m/z 686 [M+H]⁺. |
| **159** | **A27** | 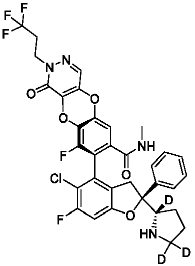 | (*S*)-8-((*S*)-5-Chloro-6-fluoro-2-phenyl-2-((*S*)-pyrrolidin-2-yl-2,5,5-d₃)-2,3-dihydrobenzofuran-4-yl)-9-fluoro-*N*-methyl-1-oxo-2-(3,3,3-trifluoropropyl)-1,2-dihydroxybenzo[5,6][1,4]di oxo [2,3-*d*]pyridazine-7-carboxamide | LCMS: m/z 692 [M+H]⁺. |
| **160** | **A28** | 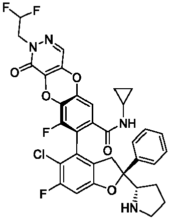 | (*S*)-8-((*S*)-5-Chloro-6-fluoro-2-phenyl-2-((*S*)-pyrrolidin-2-yl-2,5,5-d₃)-2,3-dihydrobenzofuran-4-yl)-9-fluoro-*N*-methyl-1-oxo-2-(3,3,3-trifluoropropyl)-1,2-dihydroxybenzo[5,6][1,4]di oxo [2,3-*d*]pyridazine-7-carboxamide | ¹H NMR (400 MHz, Methanol-d₄) δ 7.80 (s, 1H), 7.45 (dd, J = 7.5, 1.6 Hz, 2H), 7.36 (t, J = 7.5 Hz, 2H), 7.30 (d, J = 7.2 Hz, 1H), 7.00 - 6.92 (m, 2H), 6.13 (tt, J = 55.6, 4.2 Hz, 1H), 4.41 (td, *J =* 13.7, 4.3 Hz, 2H), 4.21 - 4.13 (m, 1H), 3.55 (d, J = 16.4 Hz, 1H), 3.31 - 3.24 (m, 1H), 3.11 (d, J = 16.4 Hz, 2H), 2.56 (tt, J = 7.5, 3.9 Hz, 1H), 2.20 (dd, J = 12.1, 5.4 Hz, 1H), 1.95 (dd, J = 14.8, 7.2 Hz, 2H), 1.89 - 1.78 (m, 1H), 0.68 - 0.57 (m, 2H), 0.46 (dd, J = 10.5, 4.4 Hz, 1H), 0.30 (dt, J = 10.7, 4.9 Hz, 1H). |
| | | | | LCMS: m/z 683 [M+H]⁺. |
| **I-1** | **A11** | 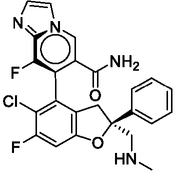 | (*S*)-7-((*S*)-(5-Chloro-6-fluoro-2-((methylamino)methyl)-2-phenyl-2,3-dihydrobenzofuran-4-yl)-8-fluoroimidazo[1,2-a]pyridine-6-carboxamide | ¹H NMR (400 MHz, Methanol-d₄) δ 8.99 (s, 1H), 8.26 (dd, J = 2.8, 1.6 Hz, 1H), 7.94 (d, J = 1.7 Hz, 1H), 7.57 - 7.39 (m, 5H), 7.12 (d, J = 9.1 Hz, 1H), 3.68 (s, 2H), 3.57 (dd, J = 16.4, 1.5 Hz, 1H), 3.30 - 3.24 (m, 1H), 2.69 (s, 3H). |
| | **I-B1** | | | |
| | | | | LCMS: m/z 469.3 [M+H]⁺. |
| **I-2** | **A11** | 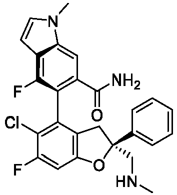 | (*S*)-5-((*S*)-5-Chloro-6-fluoro-2-((methylamino)methyl)-2-phenyl-2,3-dihydrobenzofuran-4-yl)-4-fluoro-1-methyl-1H-indole-6-carboxamide | ¹H NMR (400 MHz, DMSO-d₆) δ |
| | **B3** | | | 8.59 (s, 1H), 8.43 (s, 1H), 7.93 (s, 1H), 7.77 (s, 1H), 7.58 (d, J = 3.1 Hz, 1H), 7.52 - 7.31 (m, 6H), 7.15 (d, J = 9.4 Hz, 1H), 6.53 (d, J = 3.0 Hz, 1H), 3.90 (s, 3H), 3.66 - 3.48 (m, 6H), 3.98 - 2.94 (d, J = 16.4 Hz, 1H). |
| | | | | LC-MS: m/z 482 [M+H]⁺. |
| **I-16** | **A11** | 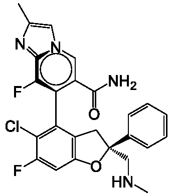 | (*S*)-7-((*S*)-5-Chloro-6-fluoro-2-((methylamino)methyl)-2-phenyl-2,3-dihydrobenzofuran-4-yl)-8-fluoro-2-methylimidazo[1,2-*a*]pyridine-6-carboxamide | ¹H NMR (400 MHz, MeOD-*d*₄) δ |
| | **I-B5** | | | 8.73 (s, 1H), 7.84 (d, J = 2.3 Hz, 1H), 7.51 - 7.35 (m, 6H), 7.01 (d, J = 9.3 Hz, 1H), 3.49 (s, 1H), 3.39 (d, J = 5.1 Hz, 1H), 3.35 (s, 1H), 3.20 (dd, J = 16.1, 1.5 Hz, 1H), 2.52 (s, 3H), 2.45 (t, J = 3.2 Hz, |
| | | | | 3H). LCMS: m/z 483.1 [M+H]⁺. |
| **I-17** | **A11** | 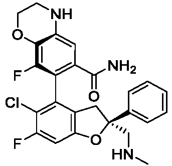 | (*S*)-7-((*S*)-5-Chloro-6-fluoro-2-((methylamino)methyl)-2-phenyl-2,3-dihydrobenzofuran-4-yl)-8-fluoro-3,4-dihydro-2*H-*benzo[*b*][1,4]oxazine-6-carboxamide | 1H NMR (400 MHz, Methanol-*d*₄) δ 7.45 - 7.40 (m, 2H), 7.39 - 7.32 (m, 2H), 7.31 - 7.26 (m, 1H), 6.88 (d, J = 9.2 Hz, 1H), 6.83 (d, J = 1.5 Hz, 1H), 4.32 - 4.21 (m, 2H), 3.60 (d, J = 13.3 Hz, 1H), 3.50 (s, 1H), 3.45-3.44 (m, 2H), 3.27 - 3.21 (m, 2H), 2.54 (s, 3H). |
| | **I-B8** | | | |
| | | | | LCMS: m/z 486 [M+H]⁺. |
| **I-23** | **A1** | 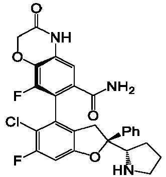 | (*S*)-7-((*S*)-5-Chloro-6-fluoro-2-phenyl-2-((*S*)-pyrrolidin-2-yl)-2,3 - dihydrobenzofuran-4-yl)-8-fluoro-3-oxo-3,4-dihydro-2*H-*benzo[*b*][1,4]oxazine-6-carboxamide | ¹H NMR (400 MHz, Methanol-*d*₄) δ 7.55 - 7.36 (m, 5H), 7.08 (d, J = 1.3 Hz, 1H), 6.96 (d, J = 9.2 Hz, 1H), 4.72 (s, 2H), 4.06 (t, J = 8.1 Hz, 1H), 3.56 (d, J = 17.3 Hz, 1H), 3.25 - 3.03 (m, 4H), 2.12 (dd, J = 12.1, 5.0 Hz, 1H), 2.00 - 1.79 (m, 3H). |
| | **I-B7** | | | |
| | | | | LCMS: m/z 526.1 [M+H]⁺. |
| **I-24** | **A1** | 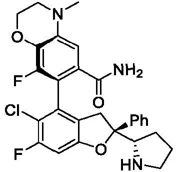 | (*S*)-7-((*S*)-5-Chloro-6-fluoro-2-phenyl-2-((*S*)-pyrrolidin-2-yl)-2,3 -dihydrobenzofuran-4-yl)-8-fluoro-4-methyl-3,4-dihydro-2*H-*benzo[*b*][1,4]oxazine-6-carboxamide | ¹H NMR (400 MHz, Methanol-*d*₄) δ 7.56 (d, *J* = 7.5 Hz, 2H), 7.46 (t, J = 7.2 Hz, 2H), 7.38 (t, J = 7.2 Hz, 1H), 6.94 (d, J = 9.2 Hz, 2H), 4.35 (t, J = 3.9 Hz, 2H), 4.23 (d, J = 8.9 Hz, 1H), 3.67 - 3.61 (m, 1H), 3.60 - 3.52 (m, 1H), 3.40 (d, J = 4.3 Hz, 2H), 3.26 - 3.09 (m, 2H), 3.03 (s, 3H), 2.20 (d, J = 11.6 Hz, 1H), 2.04 (s, 2H), 1.93 (s, 1H). |
| | **I-B9** | | | |
| | | | | LCMS: m/z 526.2 [M+H]⁺. |
| **I-25** | **A1** | 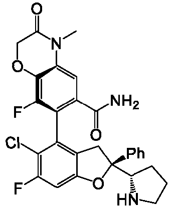 | (*S*)-7-((*S*)-5-Chloro-6-fluoro-2-phenyl-2-((*S*)-pyrrolidin-2-yl)-2,3-dihydrobenzofuran-4-yl)-8-fluoro-4-methyl-3-oxo-3,4-dihydro-2*H-*benzo[*b*][1,4]oxazine-6-carboxamide | ¹H NMR (400 MHz, Methanol-*d*₄) δ 7.55 (d, *J* = 7.4 Hz, 2H), 7.47 (t, *J* = 7.6 Hz, 2H), 7.41 (d, *J =* 7.2 Hz, 1H), 7.34 (s, 1H), 7.00 (d, *J =* 9.1 Hz, 1H), 4.77 (s, 2H), 4.29 - 4.19 (m, 1H), 3.58 (d, *J =* 14.1 Hz, 1H), 3.45 (s, 3H), 3.35 (d, *J =* 6.3 Hz, 1H), 3.24 - 3.11 (m, 2H), 2.28 - 2.19 (m, 1H), 2.03 (dt, *J* = 13.0, 6.7 Hz, 2H), 1.99 - 1.90 (m, 1H). |
| | **I-B10** | | | |
| | | | | LCMS: m/z 540.1 [M+H]⁺. |
| **I-27** | **A1** | 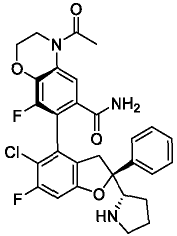 | (*S*)-4-Acetyl-7-((*S*)-5-chloro-6-fluoro-2-phenyl-2-((*S*)-pyrrolidin-2-yl)-2,3-dihydrobenzofuran-4-yl)-8-fluoro-3,4-dihydro-2*H*-benzo[*b*][1,4]oxazine-6-carboxamide | ¹H NMR (400 MHz, Methanol-*d*₄) δ 8.17 (s, 1H), 7.65 - 7.30 (m, 6H), 6.99 (d, J *=* 9.1 Hz, 1H), 4.44 (q, J = 4.9 Hz, 2H), 4.23 (dd, J = 9.5, 7.3 Hz, 1H), 4.02 (t, J = 4.5 Hz, 2H), 3.59 (dd, J = 16.5, 1.6 Hz, 1H), 3.37 - 3.32 (m, 1H), 3.24 - 3.19 (m, 1H), 3.15 (dd, J = 16.4, 1.7 Hz, 1H), 2.39 (s, 3H), 2.22 (dt, J = 12.4, 6.4 Hz, 1H), 2.09 - 1.91 (m, 3H). |
| | **I-B12** | | | |
| | | | | LCMS: m/z 554.2 [M+H]⁺. |
| **I-28** | **A1** | 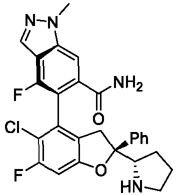 | (*S*)-5-((*S*)-5-Chloro-6-fluoro-2-phenyl-2-((*S*)-pyrrolidin-2-yl)-2,3-dihydrobenzofuran-4-yl)-4-fluoro-1-methyl-1*H-*indazole-6-carboxamide | ¹H NMR (400 MHz, Methanol-*d*₄) δ 8.02 (s, 1H), 7.73 (s, 1H), 7.47 - 7.41 (m, 2H), 7.37 (t, J = 7.6 Hz, 2H), 7.30 (t, J = 7.2 Hz, 1H), 6.91 (d, J *=* 9.2 Hz, 1H), 4.22 (d, J = 7.4 Hz, 1H), 4.14 - 4.10 (m, 1H), 4.07 (s, 3H), 3.57 (q, J = 4.9 Hz, 1H), 3.49 - 3.45 (m, 1H), 3.11 (d, J = 7.8 Hz, 1H), 2.13 (td, J = 14.6, 13.3, 6.4 Hz, 1H), 2.03 - 1.83 (m, 3H). |
| | **I-B21** | | | |
| | | | | LCMS: m/z 509.2 [M+H]⁺. |
| **I-29** | **A1** | 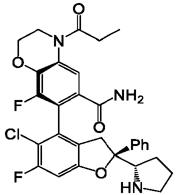 | (*S*)-7-((*S*)-5-Chloro-6-fluoro-2-phenyl-2-((*S*)-pyrrolidin-2-yl)-2,3-dihydrobenzofuran-4-yl)-8-fluoro-4-propiony1-3,4-dihydro-2*H-*benzo[*b*][1,4]oxazine-6-carboxamide | ¹H NMR (400 MHz, CDCl₃) δ 7.53 (d, J = 6.8 Hz, 2H), 7.44 - 7.27 (m, 5H), 6.87 (d, J = 8.4 Hz, 1H), 4.44 (s, 3H), 4.19 - 3.88 (m, 4H), 3.69 (d, J = 22.2 Hz, 2H), 3.33 (dd, J = 55.0, 14.4 Hz, 4H), 2.69 (s, 3H), 1.92 (s, 2H). |
| | **I-B11** | | | |
| | | | | LCMS: m/z 568.2 [M+H]⁺. |
| **I-30** | **A1** | 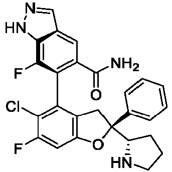 | (*S*)-6-((*S*)-5-Chloro-6-fluoro-2-phenyl-2-((*S*)-pyrrolidin-2-yl)-2,3-dihydrobenzofuran-4-yl)-7-fluoro-1*H*-indazole-5-carboxamide | ¹H NMR (400 MHz, Methanol-*d*₄) δ 8.19 (d, J = 3.3 Hz, 1H), 7.96 (s, 1H), 7.46 (d, J = 7.7 Hz, 2H), 7.37 (t, J = 7.5 Hz, 2H), 7.31 (d, J = 7.7 Hz, 1H), 6.93 (d, J = 9.2 Hz, 1H), 3.49 (d, J = 16.5 Hz, 1H), 3.25 (s, 1H), 3.12 - 3.02 (m, 3H), 2.05 -2.11 (m, 1H), 1.96-1.90 (m, 3H). |
| | **I-B13** | | | |
| | | | | LC-MS: m/z 495 [M+H]⁺. |
| **I-31** | **A1** | 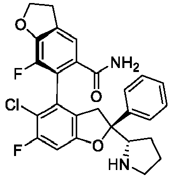 | (2*S*,4*S*)-5-Chloro-6,7'-difluoro-2-phenyl-2-((*S*)-pyrrolidin-2-yl)-2,2',3,3'-tetrahydrofuran-[4,6'-bibenzofuran]-5'-carboxamide | ¹H NMR (400 MHz, Methanol-*d*₄) δ 7.58 - 7.38 (m, 6H), 6.97 (d, *J* = 9.1 Hz, 1H), 4.80 - 4.72 (m, 2H), 4.22 (t, *J =* 8.3 Hz, 1H), 3.76 - 3.51 (m, 2H), 3.40 - 3.34 (m, 2H), 3.17 (ddd, *J* = 28.9, 15.2, 4.6 Hz, 2H), 2.21 (dd, *J* = 12.3, 6.0 Hz, 1H), 2.11 - 1.90 (m, 3H). |
| | **I-B6** | | | |
| | | | | LCMS: m/z 497.3 [M+H]⁺. |
| **I-32** | **A1** | 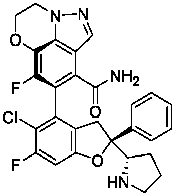 | 8-((2*S*,4*S*)-5-Chloro-6-fluoro-2-phenyl-2-((*S*)-pyrrolidin-2-yl)-2,3-dihydrobenzofuran-4-yl)-9-fluoro-2,3 -dihydro-[1,4]oxazino[2,3,4-*hi*]indazole-7-carboxamide | ¹H NMR (400 MHz, MeOD) δ 8.16 (s, 1H), 7.56 - 7.50 (m, 2H), 7.47 (t, *J* = 7.5 Hz, 2H), 7.41 (d, *J* = 7.2 Hz, 1H), 7.03 (d, *J* = 9.2 Hz, 1H), 4.76 (t, *J =* 4.8 Hz, 2H), 4.58 (dt, *J* = 8.3, 4.4 Hz, 2H), 4.24 - 4.17 (m, 1H), 3.62 (d, *J =* 14.5 Hz, 1H), 3.37 - 3.32 (m, 1H), 3.25 -3.17 (m, 1H), 3.13 (d, *J* = 16.6 Hz, 1H), 2.29 - 2.17 (m, 1H), 2.10 - 2.01 (m, 2H), 1.99 - 1.91 (m, 1H). |
| | **I-B22** | | | |
| | | | | LC-MS: m/z 537 [M+H]⁺. |
| **I-33** | **A1** | 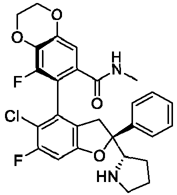 | (*S*)-7-((2*S*)-5-Chloro-6-fluoro-2-phenyl-2-((*S*)-pyrrolidin-2-yl)-2,3-dihydrobenzofuran-4-yl)-8-fluoro-*N*-methyl-2,3-dihydrobenzo[*b*][1,4]dioxi ne-6-carboxamide | LCMS: m/z 527.1 [M+H]⁺. |
| | **B1** | | | |
| **I-35** | **A1** | 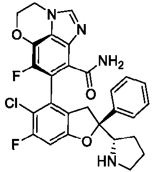 | (*S*)-7-((2*S*)-5-Chloro-6-fluoro-2-phenyl-2-((*S*)-pyrrolidin-2-yl)-2,3-dihydrobenzofuran-4-yl)-6-fluoro-3 ,4-dihydro-5-oxa-1,2a-diazaacenaphthylene-8-carboxamide | LCMS: m/z 537 [M+H]⁺. |
| | **I-B23** | | | |
| **I-43** | **A1** | 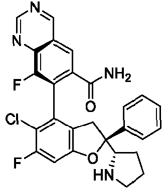 | (*S*)-7-((*S*)-5-Chloro-6-fluoro-2-phenyl-2-((*S*)-pyrrolidin-2-yl)-2,3-dihydrobenzofuran-4-yl)-8-fluoroquinazoline-6-carboxamide | LCMS: m/z 507 [M+H]⁺. |
| | **I-B16** | | | |
| **I-44** | **A1** | 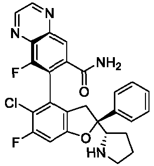 | (*S*)-7-((*S*)-5-Chloro-6-fluoro-2-phenyl-2-((*S*)-pyrrolidin-2-yl)-2,3-dihydrobenzofuran-4-yl)-8-fluoroquinoxaline-6-carboxamide | LCMS: m/z 507 [M+H]⁺. |
| | **I-B17** | | | |
| **I-52** | **A1** | 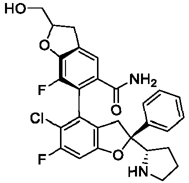 | (2*S*,6'*S*)-5-Chloro-6,7'-difluoro-2'-(hydroxymethyl)-2-phenyl-2-((*S*)-pyrrolidin-2-yl)-2,2',3,3'-tetrahydro-[4,6'-bibenzofuran]-5'-carboxamide | ¹H NMR (400 MHz, Methanol-*d*₄) δ 7.49 - 7.42 (m, 2H), 7.38 (t, *J* = 7.6 Hz, 2H), 7.35 - 7.28 (m, 2H), 6.88 (d, *J* = 9.1 Hz, 1H), 4.98 (td, *J* = 8.2, 4.0 Hz, 1H), 4.12 (td, *J* = 7.5, 7.1, 3.8 Hz, 1H), 3.76 (ddd, *J =* 12.4, 7.8, 3.5 Hz, 1H), 3.62 (ddd, *J* = 12.4, 8.9, 4.9 Hz, 1H), 3.46 (dd, *J =* 16.3, 1.7 Hz, 1H), 3.37 - 3.23 (m, 2H), 3.19 - 3.00 (m, 3H), 2.11 (dt, *J =* 12.6, 6.4 Hz, 1H), 2.01 - 1.78 (m, 3H). |
| | **I-B26** | | | |
| | | | | LC-MS: m/z 527 [M+H]⁺. |
| **I-53** | **A1** | 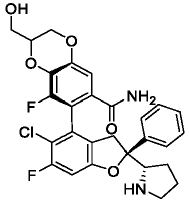 | (*S*)-7-((*S*)-5-Chloro-6-fluoro-2-phenyl-2-((*S*)-pyrrolidin-2-yl)-2,3-dihydrobenzofuran-4-yl)-8-fluoro-2-(hydroxymethyl)-2,4-dihydrobenzo[*b*][1,4]dioxi ne-6-carboxamide | ¹H NMR (400 MHz, Methanol-*d*₄) δ 7.56 - 7.54 (m, 2H), 7.47 - 7.45 (m, 2H), 7.42 - 7.38 (m, 1H), 7.15 (s, 1H), 6.96 (d, J = 12.0 Hz, 1H), 4.48 - 4.45 (m, 1H), 4.35 - 4.31 (m, 1H), 4.20 - 4.12 (m, 2H), 3.83 - 3.82 (m, 2H), 3.68 - 3.66 (m, 1H), 3.42 - 3.36 (m, 1H), 3.22 - 3.13 (m, 2H), 2.22 - 2.18 (m, 1H), 1.97 - 1.90 (m, 3H). |
| | **I-B27** | | | |
| | | | | LCMS: m/z 543.1 [M+H]⁺. |
| **I-56** | **A1** | 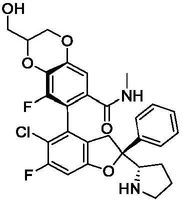 | (*S*)-7-((*S*)-5-Chloro-6-fluoro-2-phenyl-2-((*S*)-pyrrolidin-2-yl)-2,3-dihydrobenzofuran-4-yl)-8-fluoro-2-(hydroxymethyl)-*N*-methyl-2,3-dihydrobenzo[*b*][1,4]dioxin e-6-carboxamide | ¹H NMR (400 MHz, Methanol-d₄) δ 7.51 - 7.49 (m, 2H), 7.41 - 7.39 (m, 2H), 7.35 - 7.30 (m, 1H), 7.06 - 7.04 (m, 1H), 6.90 (d, J = 12.0 Hz, 1H), 4.61 - 4.45 (m, 1H), 4.29 - 4.25 (m, 1H), 4.20 - 4.12 (m, 1H), 3.88 - 3.82 (m, 3H), 3.50 - 3.46 (m, 1H), 3.11 - 3.06 (m, 2H), 2.97 - 2.95 (m, 1H), 2.74 (s, 3H), 1.97 - 1.73 (m, 4H). |
| | **I-B27** | | | |
| | | | | LCMS: m/z 557.1 [M+H]⁺. |
| **I-57 (or** I-**58)** | **A1** | 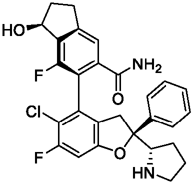 | (1*S*,6*S*)-6-((*S*)-5-Chloro-6-fluoro-2-phenyl-2-((*S*)-pyrrolidin-2-yl)-2,3-dihydrobenzofuran-4-yl)-7-fluoro-1-hydroxy-2,3-dihydro-1*H*-indene-5-carboxamide | ¹H NMR (400 MHz, Methanol-d₄) δ 8.51 (s, 1H), 7.56 - 7.36 (m, 5H), 6.97 (d, J = 12.0 Hz, 1H), 5.38 (dd, J = 4.0, 8.0 Hz, 1H), 4.13 (t, J = 8.0 Hz, 1H), 3.53 (d, J = 16.0 Hz, 1H), 3.28 - 3.05 (m, 3H), 2.95 - 2.85 (m, 1H), 2.51 - 2.37 (m, 1H), 2.23 - 1.82 (m, 5H). |
| | **I-B24** | | | |
| | | | | LCMS: m/z 511 [M+H]⁺. |
| **I-58 (or I-57)** | **A1** | 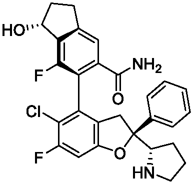 | (1*R*,6*S*)-6-((1*S*)-5-Chloro-6-fluoro-2-phenyl-2-((*S*)-pyrrolidin-2-yl)-2,3-dihydrobenzofuran-4-yl)-7-fluoro-1-hydroxy-2,3-dihydro-1*H*-indene-5-carboxamide | ¹H NMR (400 MHz, Methanol-d₄) δ 8.51 (s, 1H), 7.56 - 7.36 (m, 5H), 6.97 (d, J = 12.0 Hz, 1H), 5.41 (dd, J = 4.0, 8.0 Hz, 1H), 4.16 (t, J = 8.0 Hz, 1H), 3.56 (d, J = 16.0 Hz, 1H), 3.25 - 3.10 (m, 4H), 3.25 - 2.92 (m, 1H), 2.52 - 2.42 (m, 1H), 2.25 - 1.82 (m, 5H). |
| | **I-B24** | | | |
| | | | | LCMS: m/z 511 [M+H]⁺. |
| **I-59 (or** I-**60)** | **A1** | 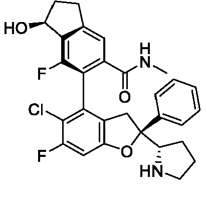 | (1*S*,6*S*)-6-((*S*)-5-Chloro-6-fluoro-2-phenyl-2-((*S*)-pyrrolidin-2-yl)-2,3-dihydrobenzofuran-4-yl)-7-fluoro-1-hydroxy-*N*-methyl-2,3-dihydro-1*H*-indene-5-carboxamide | LCMS: m/z 525 [M+H]⁺. |
| | **I-B24** | | | |
| **I-60 (or I-59)** | **A1** | 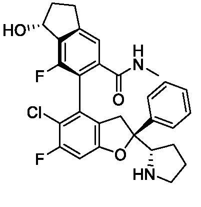 | (1*R*,6*S*)-6-((*S*)-5-Chloro-6-fluoro-2-phenyl-2-((*S*)-pyrrolidin-2-yl)-2,3-dihydrobenzofuran-4-yl)-7-fluoro-1-hydroxy-*N*-methyl-2,3-dihydro-1*H*-indene-5-carboxamide | LCMS: m/z 525 [M+H]⁺. |
| | **I-B24** | | | |
| **I-61** | **A1** | 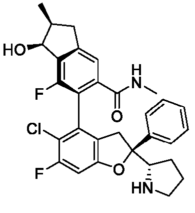 | (1*S*,2*S*,6*S*)-6-((*S*)-5-Chloro-5-fluoro-2-phenyl-2-((*S*)-pyrrolidin-2-yl)-2,3-dihydrobenzofuran-4-yl)-7-fluoro-1-hydroxy-*N*,2-dimethy1-2,3-dihydro-1*H-*indene-5-carboxamide | LCMS: m/z 539 [M+H]⁺. |
| | **I-B25** | | | |
| **I-62** | **A1** | 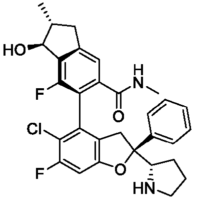 | (1*S*,2*S*,6*S*)-6-((*S*)-5-Chloro-6-fluoro-2-phenyl-2-((*S*)-pyrrolidin-2-yl)-2,3-dihydrobenzofuran-4-yl)-7-fluoro-1-hydroxy-*N*,2-dimethy1-2,3-dihydro-1*H-*indene-5-carboxamide | LCMS: m/z 539 [M+H]⁺. |
| | **I-B25** | | | |
| **I-63** | **A1** | 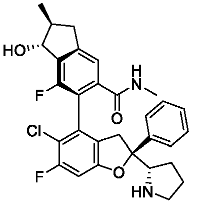 | (1*R*,2*S*,6*S*)-6-((*S*)-5-Chloro-6-fluoro-2-phenyl-2-((*S*)-pyrrolidin-2-yl)-2,3-dihydrobenzofuran-4-yl)-7-fluoro-1-hydroxy-*N*,2-dimethy1-2,3-dihydro-1*H-*indene-5-carboxamide | LCMS: m/z 539 [M+H]⁺. |
| | **I-B25** | | | |
| **I-64** | **A1** | 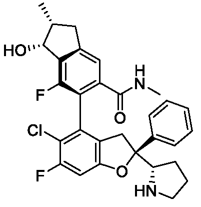 | (1*R*,2*R*,6*S*)-6-((*S*)-5-Chloro-6-fluoro-2-phenyl-2-((*S*)-pyrrolidin-2-yl)-2,3-dihydrobenzofuran-4-yl)-7-fluoro-1-hydroxy-*N*,2-dimethyl-2,2-dihydro-1*H-*indene-5-carboxamide | LCMS: m/z 539 [M+H]⁺. |
| | **I-B25** | | | |

The following compounds are free compounds or pharmaceutically acceptable salts.

| **Compound num-ber** | **Structural formula** | **Name** | **Analytical data** |
|---|---|---|---|
| **I-65** | 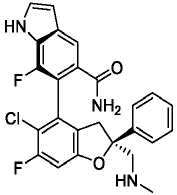 | (*S*)-6-((*S*)-5-Chloro-6-fluoro-2-((methylamino)methyl)-2-phenyl-2,3-dihydrobenzofuran-4-yl)-7-fluoro-1*H*-indole-5-carboxamide | LC-MS: m/z = 468.1 [M+H]⁺. |
| **I-66** | 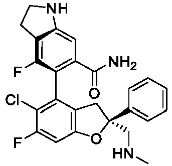 | (*S*)-5-((*S*)-5-Chloro-6-fluoro-2-((methylamino)methyl)-2-phenyl-2,3-dihydrobenzofuran-4-yl)-4-fluoroindoline-6-carboxamide | LC-MS: m/z 470.3 [M+H]⁺. |
| **I-67** | 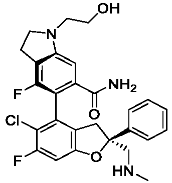 | (*S*)-5-((*S*)-5-Chloro-6-fluoro-2-((methylamino)methyl)-2-phenyl-2,3-dihydrobenzofuran-4-yl)-4-fluoro-1-(2-hydroxyethyl)indoline-6-carboxamide | LC-MS: m/z 514 [M+H]⁺. |
| **I-68** | 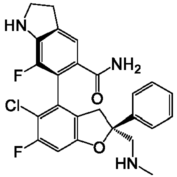 | (*S*)-6-((*S*)-5-Chloro-6-fluoro-2-((methylamino)methyl)-2-phenyl-2,3-dihydrobenzofuran-4-yl)-7-fluoroindoline-5-carboxamide | LCMS: m/z 470.2 [M+H]⁺. |
| **I-69** | 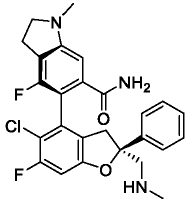 | (*S*)-5-((*S*)-5-Chloro-6-fluoro-2-((methylamino)methyl)-2-phenyl-2,3-dihydrobenzofuran-4-yl)-4-fluoro-1-methylindoline-6-carboxamide | LC-MS: m/z 484 [M+H]⁺. |
| **I-70** | 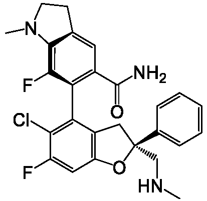 | (*S*)-6-((*S*)-5-Chloro-6-fluoro-2-((methylamino)methyl)-2-phenyl-2,3-dihydrobenzofuran-4-yl)-7-fluoro-1-methylindoline-5-carboxamide | LCMS: m/z 484.3 [M+H]⁺. |
| **I-71** | 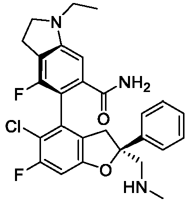 | (*S*)-5-((*S*)-5-Chloro-6-fluoro-2-((methylamino)methyl)-2-phenyl-2,3-dihydrobenzofuran-4-yl)-1-ethyl-4-fluoroindoline-6-carboxamide | LC-MS: m/z 498.25 [M+H]⁺. |
| **I-72** | 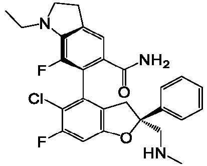 | (*S*)-6-((*S*)-5-Chloro-6-fluoro-2-((methylamino)methyl)-2-phenyl-2,3-dihydrobenzofuran-4-yl)-1-ethyl-7-fluoroindoline-5-carboxamide | LCMS: m/z 498.3 [M+H]⁺. |
| **I-73** | 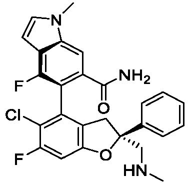 | (*S*)-5-((*S*)-5-Chloro-6-fluoro-2-((methylamino)methyl)-2-phenyl-2,3-dihydrobenzofuran-4-yl)-4-fluoro-1-methyl-1*H-*indole-6-carboxamide | LC-MS: m/z 482 [M+H]⁺. |
| **I-74** | 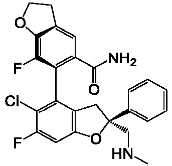 | (2*S*,6'*S*)-5-Chloro-6,7'-difluoro-2-((methylamino)methyl)-2-phenyl-2,2',3,3'-tetrahydrofuran-[4,6'-bibenzofuran]-5'-carboxamide | LC-MS: m/z 471.2 [M+H]⁺. |
| **I-75** | 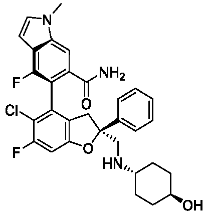 | (*S*)-5-((*S*)-5-Chloro-6-fluoro-2-((((1*R*,4*S*)-4-hydroxycyclohexyl)amino)methyl)-2-phenyl-2,3-dihydrobenzofuran-4-yl)-4-fluoroindole-6-carboxamide | LCMS: m/z 554.3 [M+H]⁺. |
| **I-76** | 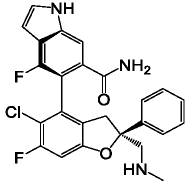 | 5-((2*S*,4*S*)-5-Chloro-6-fluoro-2-((methylamino)methyl)-2-phenyl-2,3-dihydrobenzofuran-4-yl)-4-fluoro-1*H*-indole-6-carboxamide | LC-MS: m/z 468.1 [M+H]⁺. |
| **I-77** | 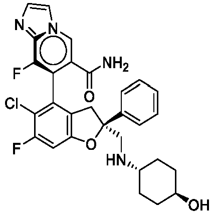 | (*S*)-7-((*S*)-5-Chloro-6-fluoro-2-((((1*r*,4*S*)-4-hydroxycyclohexyl)amino)methyl)-2-phenyl-2,3-dihydrobenzofuran-4-yl)-8-fluoroimidazo[1,2-a]pyridine-6-carboxamide | LC-MS: m/z = 553.3 [M+H]⁺. |
| **I-78** | 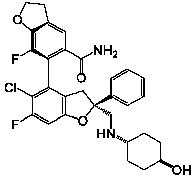 | (2*S*,6'*S*)-5-Chloro-6,7'-difluoro-2-((((1*R*,4*S*)-4-hydroxycyclohexyl)amino)methyl)-2-phenyl-2,2',3,3'-tetrahydrofuran-[4,6'-bibenzofuran]-5'-carboxamide | LC-MS: m/z 555.2 [M+H]⁺. |
| **I-79** | 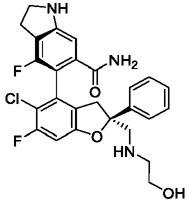 | (*S*)-5-((*S*)-Chloro-6-fluoro-2-(((2-hydroxyethyl)amino)methyl)-2-phenyl-2,3-dihydrobenzofuran-4-yl)-4-fluoroindoline-6-carboxamide | LC-MS: m/z = 500.3 [M+H]⁺. |
| **I-80** | 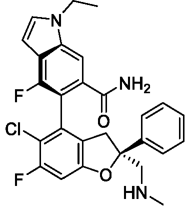 | (*S*)-5-((*S*)-5-Chloro-6-fluoro-2-((methylamino)methyl)-2-phenyl-2,3-dihydrobenzofuran-4-yl)-1-ethyl-4-fluoro-1*H-*indole-6-carboxamide | LC-MS: m/z 496.25 [M+H]⁺. |
| **I-81** | 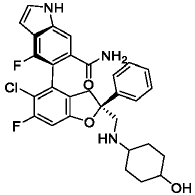 | (*S*)-5-((*S*)-5-Chloro-6-fluoro-2-(((4-hydroxycyclohexyl)amino)methyl)-2-phenyl-2,3-dihydrobenzofuran-4-yl)-4-fluoro-1H-indole-6-carboxamide | LC-MS: m/z = 552.3 [M+H]⁺. |
| **I-82** | 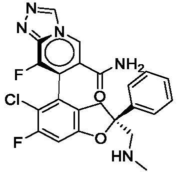 | (*S*)-7-((*S*)-5-Chloro-6-fluoro-2-((methylamino)methyl)-2-phenyl-2,3-dihydrobenzofuran-4-yl)-8-fluoro-[1,2,4]triazolo[4,3-a]pyridine-6-carboxamide | LC-MS: m/z 470.2 [M+H]⁺. |
| **I-83** | 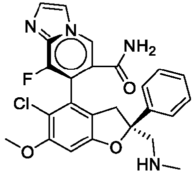 | (*S*)-7-((*S*)-5-Chloro-6-methoxy-2-((methylamino)methyl)-2-phenyl-2,3-dihydrobenzofuran-4-yl)-8-fluoroimidazo[1,2-a]pyridine-6-carboxamide | LCMS: m/z 481.1 [M+H]⁺. |
| **I-84** | 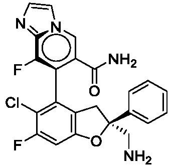 | (*S*)-7-((*S*)-(2-(Aminomethyl)-5-chloro-6-fluoro-2-phenyl-2,3-dihydrobenzofuran-4-yl)-8-fluoroimidazo[1,2-*a*]pyridine-6-carboxamide | LC-MS: m/z = 457.1 [M+H]⁺. |
| **I-85** | 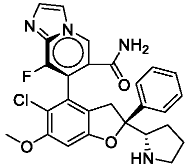 | (*S*)-7-((*S*)-5-Chloro-6-methoxy-2-phenyl-2-((*S*)-pyrrolidin-2-yl)-2,3-dihydrobenzofuran-4-yl)-8-fluoroimidazo[1,2-*a*]pyridine-6-carboxamide | LC-MS: m/z 507.1 [M+H]⁺. |
| **I-86** | 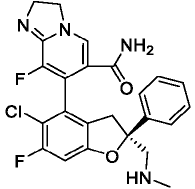 | (*S*)-7-(5-Chloro-6-fluoro-2-((methylamino)methyl)-2-phenyl-2,3-dihydrobenzofuran-4-yl)-8-fluoro-2,3-dihydroimidazo[1,2-*a*]pyridine-6-carboxamide | LC-MS: m/z 471.1 [M+H]⁺. |
| **I-87** | 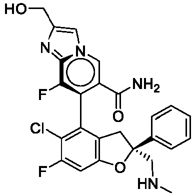 | (*S*)-7-((*S*)-(5-Chloro-6-fluoro-2-((methylamino)methyl)-2-phenyl-2,3-dihydrobenzofuran-4-yl)-8-fluoro-2-(hydroxymethyl)imidazo[1,2-*a*]pyridine-6-carboxamide | LC-MS: m/z = 499.2 [M+H]⁺. |
| **I-88** | 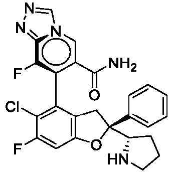 | (*S*)-7-((*S*)-5-Chloro-6-fluoro-2-phenyl-2-(pyrrolidin-2-yl)-2,3-dihydrobenzofuran-4-yl)-8-fluoro-[1,2,4]triazolo[4,3-*a*]pyridine-6-carboxamide | LC-MS: m/z 496.1 [M+H]⁺. |
| **I-89** | 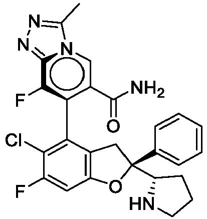 | (*S*)-7-((*S*)-5-Chloro-6-fluoro-2-phenyl-2-((*S*)-pyrrolidin-2-yl)-2,3-dihydrobenzofuran-4-yl)-8-fluoro-3-methyl-[1,2,4]triazolo[4,3-*a*]pyridine-6-carboxamide | LC-MS: m/z 510.1 [M+H]⁺. |
| **I-90** | 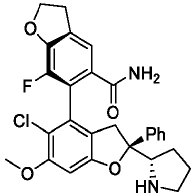 | (*S*)-6'-((*S*)-5-Chloro-7'-fluoro-6-methoxy-2-phenyl-2-((*S*)-pyrrolidin-2-yl))-2,2',3,3'-tetrahydro-[4,6'-bibenzofuran]-5'-carboxamide | LC-MS: m/z 509.2 [M+H]⁺. |
| **I-91** | 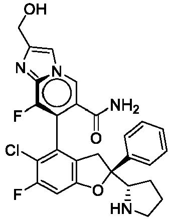 | (*S*)-7-((*S*)-5-Chloro-6-fluoro-2-phenyl-2-((*S*)-pyrrolidin-2-yl)-2,3-dihydrobenzofuran-4-yl)-8-fluoro-2-(hydroxymethyl)imidazo[1,2-*a*]pyridine-6-carboxamide | LC-MS: m/z = 525.2 [M+H]⁺. |
| **I-92** | 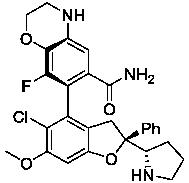 | (*S*)-7-((*S*)-5-Chloro-6-methoxy-2-phenyl-2-((*S*)-pyrrolidin-2-yl)-2,3-dihydrobenzofuran-4-yl)-8-fluoro-3,4-dihydro-2*H*-benzo[*b*][1,4]oxazine-6-carboxamide | LC-MS: m/z 524.2 [M+H]⁺. |
| **I-94** | 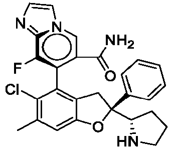 | (*S*)-7-((*S)*-5-Chloro-6-methyl-2-phenyl-2-((*S*)-pyrrolidin-2-yl)-2,3-dihydrobenzofuran-4-yl)-8-fluoroimidazo[1,2-*a*]pyridine-6-carboxamide | LC-MS: m/z 491.2 [M+H]⁺. |
| **I-95** | 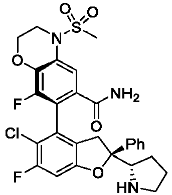 | (*S*)-7-((*S*)-5-Chloro-6-fluoro-2-phenyl-2-((*S*)-pyrrolidin-2-yl)-2,3-dihydrobenzofuran-4-yl)-8-fluoro-4-(methylsulfonyl)-3,4-dihydro-2*H-*benzo[*b*][1,4]oxazine-6-carboxamide | LC-MS: m/z = 590.2 [M+H]⁺. |
| **I-96** | 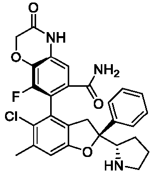 | (*S*)-7-((*S*)-5-Chloro-6-methyl-2-phenyl-2-((*S*)-tetrahydropyrrol-2-yl)-2,3-dihydrobenzofuran-4-yl)-8-fluoro-3-oxo-3,4-dihydro-2*H-*benzo[*b*][1,4]oxazine-6-carboxamide | LC-MS: m/z 522.2 [M+H]⁺. |
| **I-97** | 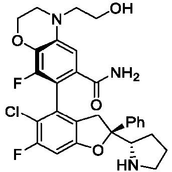 | (*S*)-7-((*S*)-5-Chloro-6-fluoro-2-phenyl-2-((*S*)-pyrrolidin-2-yl)-2,3-dihydrobenzofuran-4-yl)-8-fluoro-4-(2-hydroxyethyl)-3,4-dihydro-2*H-*benzo[*b*][1,4]oxazine-6-carboxamide | LC-MS: m/z =556.2 [M+H]⁺. |
| **I-98** | 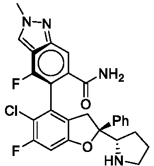 | (*S*)-5-((*S*)-5-Chloro-6-fluoro-2-phenyl-2-((*S*)-pyrrolidin-2-yl)-2,3-dihydrobenzofuran-4-yl)-4-fluoro-2-methyl-2*H*-indazole-6-carboxamide | LC-MS: m/z =509.2 [M+H]⁺. |
| **I-99** | 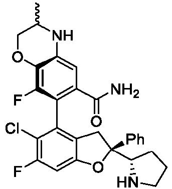 | (*S*)-7-((*S*)-5-Chloro-6-fluoro-2-phenyl-2-((*S*)-pyrrolidin-2-yl)-2,3-dihydrobenzofuran-4-yl)-8-fluoro-3-methyl-3,4-dihydro-2*H-*benzo[*b*][1,4]oxazine-6-carboxamide | LC-MS: m/z =526.1 [M+H]⁺. |
| **I-100** | 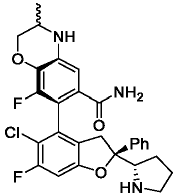 | (*S*)-7-((*S*)-5-Chloro-6-fluoro-2-phenyl-2-((*S*)-pyrrolidin-2-yl)-2,3-dihydrobenzofuran-4-yl)-8-fluoro-3-methyl-3,4-dihydro-2H-benzo[*b*][1,4]oxazine-6-carboxamide | LC-MS: m/z =526.1 [M+H]⁺. |
| **I-101** | 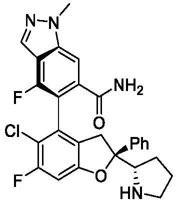 | (*S*)-5-((*S*)-5-Chloro-6-fluoro-2-phenyl-2-((*S*)-pyrrolidin-2-yl)-2,3-dihydrobenzofuran-4-yl)-4-fluoro-1-methyl-1*H*-indazole-6-carboxamide | LC-MS: m/z =509.2 [M+H]⁺. |
| **I-102** | 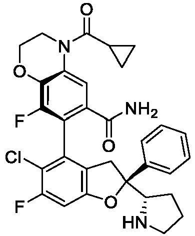 | (*S*)-7-((*S*)-5-Chloro-6-fluoro-2-phenyl-2-((*S*)-pyrrolidin-2-yl)-2,3-dihydrobenzofuran-4-yl)-4-(cyclopropanecarbonyl)-8-fluoro-3,4-dihydro-2*H*-benzo[*b*][1,4]oxazine-6-carboxamide | LC-MS: m/z 580.1 [M+H]⁺. |
| **I-103** | 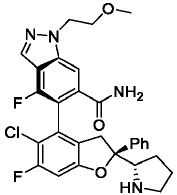 | (*S*)-5-((*S*)-5-Chloro-6-fluoro-2-phenyl-2-((*S*)-pyrrolidin-2-yl)-2,3-dihydrobenzofuran-4-yl)-4-fluoro-1-(2-methoxyethyl)-1*H*-indazole-6-carboxamide | LC-MS: m/z =553.2 [M+H]⁺. |
| **I-104** | 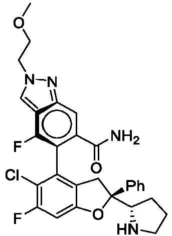 | (*S*)-5-((*S*)-5-Chloro-6-fluoro-2-phenyl-2-((*S*)-pyrrolidin-2-yl)-2,3-dihydrobenzofuran-4-yl)-4-fluoro-2-(2-methoxyethyl)-2*H*-indazole-6-carboxamide | LC-MS: m/z = 553.2 [M+H]⁺. |
| **I-105** | 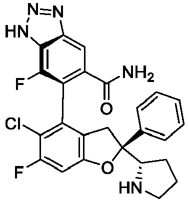 | (*S*)-6-((*S*)-5-Chloro-6-fluoro-2-phenyl-2-((*S*)-pyrrolidin-2-yl)-2,3-dihydrobenzofuran-4-yl)-7-fluoro-1*H*-benzo[*d*][1,2,3]triazole-5-carboxamide | LC-MS: m/z 496 [M+H]⁺. |
| **I-106** | 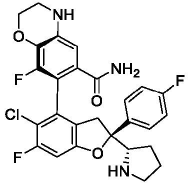 | (*S*)-7-((*S*)-5-Chloro-6-fluoro-2-(4-fluorophenyl)-2-((*S*)-pyrrolidin-2-yl)-2,3-dihydrobenzofuran-4-yl)-8-fluoro-3,4-dihydro-2*H*-benzo[*b*][1,4]oxazine-6-carboxamide | LC-MS: m/z 530.2 [M+H]⁺. |
| **I-107** | 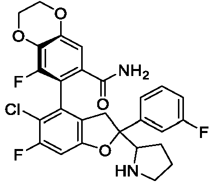 | 7-((4*S*)-5-Chloro-6-fluoro-2-(3-fluorophenyl)-2-(pyrrolidin-2-yl)-2,3-dihydrobenzofuran-4-yl)-8-fluoro-2,3-dihydrobenzo[*b*][1,4]dioxine-6-carboxamide | LCMS: m/z 531.2 [M+H]⁺. |
| **I-108** | 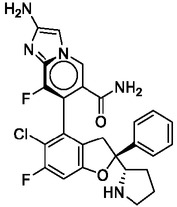 | 2-Amino-(*S*)-7-((*S*)-5-chloro-6-fluoro-2-phenyl-2-((*S*)-pyrrolidin-2-yl)-2,3-dihydrobenzofuran-4-yl)-8-fluoroimidazo[1,2-*a*]pyridine-6-carboxamide | LCMS: m/z 510.1 [M+H]⁺. |
| **I-109** | 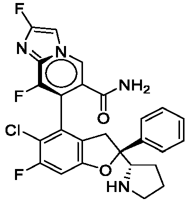 | (*S*)-7-((*S*)-5-Chloro-6-fluoro-2-phenyl-2-((*S*)-pyrrolidin-2-yl)-2,3-dihydrobenzofuran-4-yl)-2,8-difluoroimidazo[1,2-*a*]pyridine-6-carboxamide | LCMS: m/z 513.1 [M+H]⁺. |
| **I-110** | 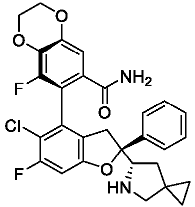 | (*S*)-7-((*S*)-5-Chloro-6-fluoro-2-phenyl-2-((*S*)-5-azaspiro[2.4]heptan-6-yl)-2,3-dihydrobenzofuran-4-yl)-8-fluoro-2,3-dihydrobenzo[*b*][1,4]dioxine-6-carboxamide | LCMS: m/z 539.2 [M+H]⁺. |
| **I-111** | 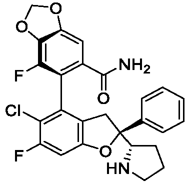 | (*S*)-6-((*S*)-5-Chloro-6-fluoro-2-phenyl-2-((*S*)-pyrrolidin-2-yl)-2,3-dihydrobenzofuran-4-yl)-7-fluorobenzo[*d*][1,3]dioxole-5-carboxamide | LCMS: m/z 499.1 [M+H]⁺. |
| **I-112** | 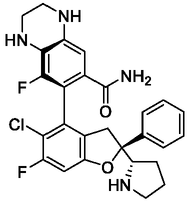 | (*S*)-7-((*S*)-5-Chloro-6-fluoro-2-phenyl-2-((*S*)-pyrrolidin-2-yl)-2,3-dihydrobenzofuran-4-yl)-8-fluoro-1,2,3,4-tetrahydroquinoline-6-carboxamide | LCMS: m/z 511.2 [M+H]⁺. |
| **I-113** | 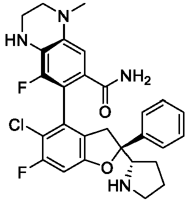 | (*S*)-7-((*S*)-5-Chloro-6-fluoro-2-phenyl-2-((*S*)-pyrrolidin-2-yl)-2,3-dihydrobenzofuran-4-yl)-8-fluoro-4-methyl-1,2,3,4-tetrahydroquinoline-6-carboxamide | LCMS: m/z 525.2 [M+H]⁺. |
| **I-114** | 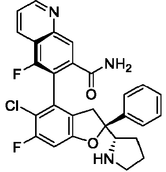 | (*S*)-6-((*S*)-5-Chloro-6-fluoro-2-phenyl-2-((*S*)-pyrrolidin-2-yl)-2,3-dihydrobenzofuran-4-yl)-5-fluoroquinoline-7-carboxamide | LCMS: m/z 506.1 [M+H]⁺. |
| **I-115** | 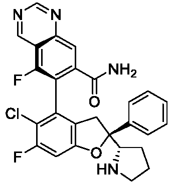 | (*S*)-6-((*S*)-5-Chloro-6-fluoro-2-phenyl-2-((*S*)-pyrrolidin-2-yl)-2,3-dihydrobenzofuran-4-yl)-5-fluoroquinazoline-7-carboxamide | LCMS: m/z 507.1 [M+H]⁺. |
| **I-117** | 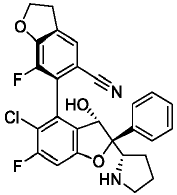 | (*S*)-6'-(2*S*,3*S*)-5-Chloro-6,7'-difluoro-3-hydroxy-2-phenyl-2-((*S*)-pyrrolidin-2-yl)-2,2',3,3'-tetrahydrofuran-[4,6'-bibenzofuran]-5'-carbonitrile | LCMS: m/z 495.1 [M+H]⁺. |
| **I-118** | 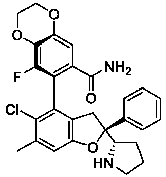 | (S)-7-((S)-5-Chloro-6-methyl-2-phenyl-2-((S)-pyrrolidin-2-yl)-2,3-dihydrobenzofuran-4-yl)-8-fluoro-2,3-dihydrobenzo[b] [1,4]dioxine-6-carboxamide | LCMS: m/z 59.2 [M+H]⁺. |
| **I-119** | 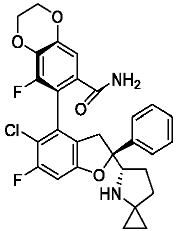 | (*S*)-7-((*S*)-5-Chloro-6-fluoro-2-phenyl-2-((*S*)-4-azaspiro[2.4]heptan-5-yl)-2,3-dihydrobenzofuran-4-yl)-8-fluoro-2,3-dihydrobenzo[*b*][1,4]dioxine-6-carboxamide | LCMS: m/z 539.2 [M+H]⁺. |
| **I-120** | 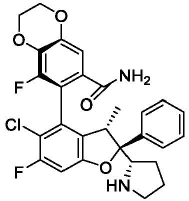 | (*S*)-7-((2*S*,3*S*)-5-Chloro-6-fluoro-3-methyl-2-phenyl-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 527.2 [M+H]⁺. |
| **I-121** | 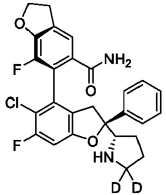 | (*S*)-6'-(2*S*)-5-Chloro-6,7'-difluoro-2-phenyl-2-((*S*)-pyrrolidin-2-yl-5,5-*d*₂)-2,2',3,3'-tetrahydrofuran-[4,6'-bibenzofuran]-5'-carboxamide | LCMS: m/z 499.2 [M+H]⁺. |
| **I-122** | 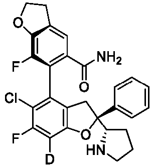 | (*S*)-6'-(2*S*)-5-Chloro-6,7'-difluoro-2-phenyl-2-((*S*)-pyrrolidin-2-yl)-2,2',3,3'-tetrahydrofuran-[4,6'-bibenzofuran]-7-*d*-5'-carboxamide | LCMS: m/z 498.1 [M+H]⁺. |
| **I-123** | 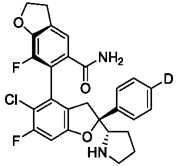 | (*S*)-6'-(2*S*)-5-Chloro-6,1'-difluoro-2-(phenyl-4-*d*)-2-((*S*)-pyrrolidin-2-yl)-2,2',3,3'-tetrahydrofuran-[4,6'-bibenzofuran]-5'-carboxamide | LCMS: m/z 498.1 [M+H]⁺. |
| **I-124** | 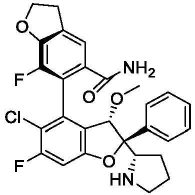 | (*S*)-6'-(2*S*,3*S*)-5-Chloro-6,1'-difluoro-3-methoxy-2-phenyl-2-((S)-pyrrolidin-2-yl)-2,2',3,3'-tetrahydrofuran-[4,6'-bibenzofuran]-5'-carboxamide | LCMS: m/z 527.2 [M+H]⁺. |
| **I-125** | 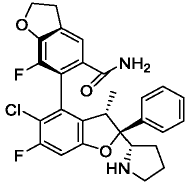 | (S)-6'-(2S,3S)-5-Chloro-6,7'-difluoro-3-methyl-2-phenyl-2-((*S*)-pyrrolidin-2-yl)-2,2',3,3'-tetrahydrofuran-[4,6'-bibenzofuran]-5'-carboxamide | LCMS: m/z 511.2 [M+H]⁺. |
| **I-126** | 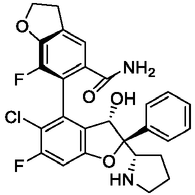 | (*S*)-6'-(2*S*,3*S*)-5-Chloro-6,7'-difluoro-3-hydroxy-2-phenyl-2-((S)-pyrrolidin-2-yl)-2,2',3,3'-tetrahydrofuran-[4,6'-bibenzofuran]-5'-carboxamide | LCMS: m/z 513.1 [M+H]⁺. |
| **I-127** | 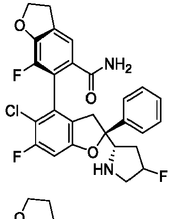 | (*S*)-6'-(2*S*)-5-Chloro-6,7'-difluoro-2-((2*S*)-4-fluoropyrrolidin-2-yl)-2-phenyl-2,2',3,3'-tetrahydrofuran-[4,6'-bibenzofuran]-5'-carboxamide | LCMS: m/z 515.1 [M+H]⁺. |
| **I-128** | 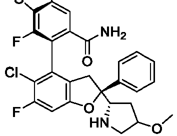 | (*S*)-6'-(2*S*)-5-Chloro-6,7'-difluoro-2-((2*S*)-4-methoxypyrrolidin-2-yl)-2-phenyl-2,2',3,3'-tetrahydrofuran-[4,6'-bibenzofuran]-5'-carboxamide | LCMS: m/z 527.2 [M+H]⁺. |
| **I-129** | 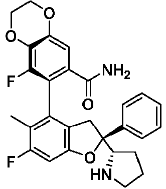 | (*S*)-8-Fluoro-7-((*S*)-6-fluoro-5-methyl-2-phenyl-2-((*S*)-pyrrolidin-2-yl)-2,3-dihydrobenzofuran-4-yl)-2,3-dihydrobenzo[*b*][1,4]dioxopyrimidine-6-carboxamide | LCMS: m/z 493.2 [M+H]⁺. |
| **I-130** | 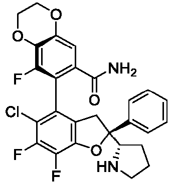 | (*S*)-7-((*S*)-5-Chloro-6,7-difluoro-2-phenyl-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 493.2 [M+H]⁺. |
| **I-131** | 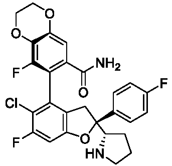 | (S)-7-((S)-5-Chloro-6-fluoro-2-(4-fluorophenyl)-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 531.1 [M+H]⁺. |
| **I-132** | 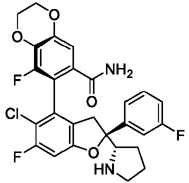 | (*S*)-7-((*S*)-5-Chloro-6-fluoro-2-(3-fluorophenyl)-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 531.1 [M+H]⁺. |
| **I-133** | 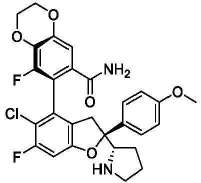 | (S)-7-((S)-5-Chloro-6-fluoro-2-(4-methoxyphenyl)-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 543.1 [M+H]⁺. |
| **I-134** | 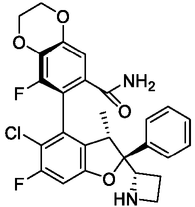 | (*S*)-7-((2*S*,3*S*)-2-((*S*)-Azetidin-2-yl)-5-chloro-6-fluoro-3-methyl-2-phenyl-2,3-dihydrobenzofuran-4-yl)-8-fluoro-2,3-dihydrobenzo[*b*][1,4]dioxopyrimidine-6-carboxamide | LCMS: m/z 513.1 [M+H]⁺. |
| **I-135** | 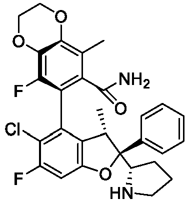 | (*S*)-7-((2*S*,3*S*)-5-Chloro-6-fluoro-3-methyl-2-phenyl-2-((*S*)-pyrrolidin-2-yl)-2,3-dihydrobenzofuran-4-yl)-8-fluoro-5-methyl-2,3-dihydrobenzo[*b*][1,4]dioxopyrimidine-6-carboxamide | LCMS: m/z 541.2 [M+H]⁺. |
| **I-136** | 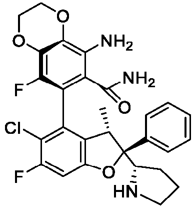 | (*S*)-5-Amino-7-((2*S*,3*S*)-5-chloro-6-fluoro-3-methyl-2-phenyl-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 542.2 [M+H]⁺. |
| **I-137** | 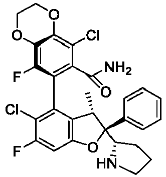 | (*S*)-5-Chloro-7-((2*S*,3*S*)-5-chloro-6-fluoro-3-methyl-2-phenyl-2-((*S*)-pyrrolidin-2-yl)-2,3-dihydrobenzofuran-4-yl)-8-fluoro-2,3-dihydrobenzo[*b*][1,4]dioxine-6-carboxamide | LCMS: m/z 561.1 [M+H]⁺. |

### VI. Biological assay

### Test example 1: YAP-TEAD protein interaction HTRF experiment

In this example, the YAP-TEAD protein interaction HTRF experiment was used to detect the activity of each compound in blocking the YAP-TEAD protein interaction.

The experiment was carried out according to the following operation steps:
Step 1: His-TEAD1 (209-426) protein was added to a 384-well detection white plate to a final concentration of 10 nM, and buffer was used as the background signal well. The compounds to be tested were subjected to 3-fold dilution and a total of 10 concentration gradients were set up, and each diluted solution was mixed well. A certain volume of the compound to be tested was added to each well separately, 2 replicates were set for each concentration, and the final concentration of DMSO was controlled at 1%. Incubation was carried out at room temperature for 20 minutes. The amino acid sequence of TAED1 (209-426) used in this step is shown in SEQ ID NO: 1:
   SEQ ID NO: 1: 
RSIGTTKLRL VEFSAFLEQQ RDPDSYNKHL FVHIGHANHS YSDPLLESVD
IRQIYDKFPE
KKGGLKELFG KGPQNAFFLV KFWADLNCNI QDDAGAFYGV TSQYESSENM
TVTCSTKVCS
FGKQVVEKVE TEYARFENGR FVYRINRSPM CEYMINFIHK LKHLPEKYMM
NSVLENFTIL
LVVTNRDTQE TLLCMACVFE VSNSEHGAQH HIYRLVKD
Step 2: The YAP peptide (60-100) part of the Bio-YAP peptide was added to each well, achieving a final concentration of 50 nM. After mixing evenly, the mixture was incubated at room temperature for 10 minutes. The amino acid sequence of the YAP peptide (60-100) part of the Bio-YAP peptide used in this step is shown in SEQ ID NO: 2:
   SEQ ID NO: 2: 
DSETDLEALF NA
VMNPKTAN VPQTVPMRLR KLPDSFFKPP E
.
Step 3: Anti-His-Eu cryptate gold and streptavidin-XL665 were diluted according to the instructions and mixed in equal proportions. A certain volume of the detection reagent was added to the well, and the mixture was incubated at room temperature for 1 hour.
Step 4: The signal was detected using an Envision (Perkin Elmer) microplate reader at an excitation wavelength of 320 nm and emission wavelengths of 665 nm and 620 nm.
Step 5: The signal was calculated based on the ratio of Em665 / Em620 * 10000.

The inhibition percentage (%) was calculated using the following formula: Inhibition percentage (%) = (1 - (compound (cpd)signal - backgroundsignal) / (DMSOsignal - backgroundsignal)) * 100

The half-maximal inhibitory concentration (IC₅₀) values were calculated using GraphPad Prism software.

The activity of each compound in blocking the YAP-TEAD protein interaction (expressed by grades A, B, C and D of IC₅₀ values, where the letter A represents an IC₅₀ of less than 0.05 µM; the letter B represents an IC₅₀ between 0.05 µM and 0.5 µM; the letter C represents an IC₅₀ between 0.5 µM and 5 µM; the letter D represents an IC₅₀ greater than 5 µM) is as shown in Table 1.

**Table 1: Activity of each compound in blocking YAP-TEAD protein interaction**

| Compound number | Activity | Compound number | Activity | Compound number | Activity |
|---|---|---|---|---|---|
| Compound 1 | A | Compound 110 | A | Compound 149 | A |
| Compound 1' | B | Compound 111 | A | Compound 150 | A |
| Compound 2 | A | Compound 112 | A | Compound 151 | A |
| Compound 3 | A | Compound 113 | B | Compound 152 | A |
| Compound 3' | B | Compound 114 | A | Compound 153 | A |
| Compound 4 | A | Compound 115 | A | Compound 154 | A |
| Compound 5 | A | Compound 116 | A | Compound 155 | A |
| Compound 6 | A | Compound 117 | D | Compound 156 | A |
| Compound 7 | A | Compound 118 | A | Compound 157 | A |
| Compound 8 | A | Compound 119 | A | Compound 158 | A |
| Compound 9 | A | Compound 120 | A | Compound 159 | A |
| Compound 10 | A | Compound 121 | A | Compound 160 | A |
| Compound 39 | A | Compound 122 | A | Compound I-1 | A |
| Compound 41 | A | Compound 123 | A | Compound I-2 | A |
| Compound 42 | A | Compound 124 | A | Compound I-11 | A |
| Compound 43 | B | Compound 125 | A | Compound I-16 | A |
| Compound 44 | B | Compound 126 | A | Compound I-17 | A |
| Compound 46 | A | Compound 127 | A | Compound I-19 | A |
| Compound 55 | C | Compound 128 | A | Compound I-23 | A |
| Compound 62 | A | Compound 129 | A | Compound I-24 | A |
| Compound 66 | A | Compound 131 | A | Compound I-25 | A |
| Compound 67 | A | Compound 132 | A | Compound I-26 | A |
| Compound 71 | A | Compound 133 | A | Compound I-27 | A |
| Compound 78 | A | Compound 134 | A | Compound I-28 | A |
| Compound 79 | A | Compound 135 | A | Compound I-29 | A |
| Compound 81 | A | Compound 136 | A | Compound I-30 | A |
| Compound 83 | A | Compound 137 | A | Compound I-31 | A |
| Compound 84 | A | Compound 138 | A | Compound I-32 | A |
| Compound 101 | A | Compound 139 | A | Compound I-52 | A |
| Compound 102 | A | Compound 140 | A | Compound I-53 | A |
| Compound 103 | A | Compound 141 | A | Compound I-54 | A |
| Compound 104 | A | Compound 142 | A | Compound I-55 | A |
| Compound 105 | A | Compound 143 | A | Compound I-56 | A |
| Compound 106 | A | Compound 144 | A | Compound I-57 | A |
| Compound 107 | A | Compound 145 | A | Compound I-58 | B |
| Compound 108 | A | Compound 147 | A | Compound I-59 | A |
| Compound 109 | A | Compound 148 | A | Compound I-60 | B |

### Test example 2: YAP/TEAD reporter gene inhibition experiment

In this example, the inhibition of the compound on the target was detected by the signal of the reporter gene in the stable cell line SF268-YAP-Luc containing the YAP/TEAD reporter gene.

A sequence containing 6 tandem YAP/TEAD binding sites and a basal transcription promoter was constructed on the vector pGL4.76 of Promega Company. The constructed reporter gene vector was transfected into SF268 cells (NCI DCTD tumor/cell line repository) and screened with 0.5 µg/mL hygromycin to obtain SF268-YAP-Luc stable strain.

SF268-YAP-Luc cells were seeded into a 96-well plate at a density of 3000 cells per well with a volume of 100 µL per well (cell culture medium composition: RPMI1640 (Gibco-A10491-01) + 10% FBS (Gibco-10099-141C) + 1% penicillin-streptomycin (5,000 U/mL, Gibco-15070-063)), and the cells were incubated overnight in a 37°C, 5% carbon dioxide incubator.

On the next day, the compounds to be tested were 3-fold diluted, and a total of 8 concentration gradients were set up; a certain volume of DMSO or the compounds to be tested was added to each well, respectively, and 2 replicates were set up for each concentration, and the final concentration of DMSO was controlled to be no higher than 0.5%. The cells were incubated for 24 hours in a 37°C, 5% carbon dioxide incubator. Two identical parallel plates were set up for cell seeding and compound treatment.

Renila-Glo luciferase assay system kit (Promega, E2720) was used to detect the reporter gene signals of the control group and treatment group. 70 µL of Renila-Glo was added to each well, mixed well, and incubated at room temperature for 10 minutes. The signal was read using En Vision^{®} Multilabel Plate Reader (Perkin Elmer).

Cell viability was detected in the control group and treatment group using CellTiter-Glo Luminescent Cell Viability Assay kit (Promega, G7570). 50 µL of CellTiter-Glo was added to each well, mixed well, and incubated at room temperature for 10 minutes. The signal was read using En Vision^{®} Multilabel Plate Reader (Perkin Elmer).

The signal of Renila luciferase after correcting for cell viability was analyzed, and the inhibition rate of the compound on the reporter gene was calculated. The inhibition percentage (%) is calculated by the following formula: Inhibition percentage (%) = (1-(compound-treated well Renilasignal / DMSO-treated well Renilasignal) / (compound-treated well CTGsignal / DMSO-treated well CTGsignal)) * 100

Using Graphpad Prism software, the IC₅₀ values for the compounds' inhibitory activity against Renila luciferase signaling were calculated.

The activity of each compound in inhibiting the expression of YAP/TEAD reporter gene (expressed by grades A, B, C and D of IC₅₀ values, where the letter A represents an IC₅₀ of less than 0.05 µM; the letter B represents an IC₅₀ between 0.05 µM and 0.5 µM; the letter C represents an IC₅₀ between 0.5 µM and 5 µM; the letter D represents an IC₅₀ greater than 5 µM) is as shown in Table 2.

**Table 2: Activity of each compound in inhibiting the expression of YAP/TEAD reporter gene**

| Compound number | Activity | Compound number | Activity |
|---|---|---|---|
| Compound 1 | A | Compound I-23 | B |
| Compound 3 | A | Compound I-24 | A |
| Compound 8 | A | Compound I-25 | B |
| Compound I-1 | A | Compound I-27 | A |
| Compound I-19 | A | | |

### Test example 3: Cell proliferation inhibition experiment

In this example, the intracellular ATP was quantitatively determined by the CellTiter-Glo luminescence cell viability detection kit to detect the number of living cells in the culture.

Step 1: MSTO-211H (ATCC, CRL-2081^{™}) or NCI-H2052 (ATCC, CRL-5915^{™}) cells were inoculated in a 96-well plate at a density of 1500 cells per well with a volume of 100 µL per well, and the cells were incubated overnight in a 37°C, 5% carbon dioxide incubator. The culture medium used was RPMI1640 medium (GIBCO-A10491-01) + 10% FBS (GIBCO-10099141C) + 1% penicillin/streptomycin (Pen/Strep) (GIBCO-15070-063).

Step 2: The cells were treated with the compounds to be tested. The compounds to be tested were 3-fold diluted, and a total of 9 concentration gradients were set up; a certain volume of DMSO or the compounds to be tested was added to each well, respectively, and 2 replicates were set up for each concentration, and the final concentration of DMSO was controlled to be no higher than 0.5%. The cells were incubated for 96 hours in a 37°C, 5% carbon dioxide incubator.

Step 3: Cell viability was detected in the control group and treatment group using CellTiter-Glo Luminescent Cell Viability Assay kit (Promega, G7570). 50 µL of CellTiter-Glo was added to each well, mixed well, and incubated at room temperature for 10 minutes. The signal was read using EnVision^{®} Multilabel Plate Reader (Perkin Elmer).

The inhibition percentage (%) was calculated using the following formula: Inhibition percentage (%) = (1 - compound-treated well CTGsignal / DMSO-treated well CTGsignal) * 100

Using Graphpad Prism software, the IC₅₀ values of the compounds inhibiting the CTG signal of cell proliferation activity was calculated.

The inhibitory activity of each compound on the proliferation of MSTO-211H cells (expressed by grades A, B, C and D of IC₅₀ values, where the letter A represents an IC₅₀ of less than 0.2 µM; the letter B represents an IC₅₀ between 0.2 µM and 0.5 µM; the letter C represents an IC₅₀ between 0.5 µM and 5 µM; the letter D represents an IC₅₀ greater than 5 µM) is as shown in Table 3.

**Table 3: Inhibitory activity of each compound on the proliferation of MSTO-211H cells**

| Compound number | Activity | Compound number | Activity | Compound number | Activity |
|---|---|---|---|---|---|
| Compound 1 | A | Compound 109 | A | Compound 144 | A |
| Compound 2 | B | Compound 110 | B | Compound 147 | A |
| Compound 3 | A | Compound 111 | A | Compound 148 | A |
| Compound 4 | A | Compound 112 | B | Compound 149 | A |
| Compound 5 | A | Compound 114 | C | Compound 150 | A |
| Compound 6 | A | Compound 115 | C | Compound 151 | A |
| Compound 7 | B | Compound 116 | A | Compound 152 | A |
| Compound 8 | A | Compound 118 | A | Compound 153 | A |
| Compound 9 | A | Compound 119 | B | Compound 154 | A |
| Compound 10 | A | Compound 120 | A | Compound 155 | A |
| Compound 39 | A | Compound 121 | B | Compound 156 | A |
| Compound 41 | A | Compound 122 | A | Compound 157 | A |
| Compound 42 | C | Compound 123 | B | Compound 158 | A |
| Compound 44 | D | Compound 124 | A | Compound 159 | A |
| Compound 46 | A | Compound 125 | A | Compound 160 | A |
| Compound 62 | A | Compound 126 | A | Compound I-1 | A |
| Compound 66 | B | Compound 127 | B | Compound I-14 | A |
| Compound 67 | B | Compound 128 | A | Compound I-16 | A |
| Compound 71 | B | Compound 129 | B | Compound I-17 | A |
| Compound 78 | A | Compound 131 | A | Compound I-18 | B |
| Compound 79 | B | Compound 132 | A | Compound I-21 | A |
| Compound 81 | A | Compound 133 | A | Compound I-23 | A |
| Compound 83 | A | Compound 134 | C | Compound I-24 | A |
| Compound 84 | B | Compound 135 | A | Compound I-25 | B |
| Compound 101 | A | Compound 136 | A | Compound I-27 | B |
| Compound 102 | A | Compound 137 | A | Compound I-28 | A |
| Compound 103 | C | Compound 138 | A | Compound I-29 | B |
| Compound 104 | B | Compound 139 | A | Compound I-30 | B |
| Compound 105 | A | Compound 140 | A | | |
| Compound 106 | B | Compound 141 | A | | |
| Compound 107 | A | Compound 142 | A | | |
| Compound 108 | C | Compound 143 | A | | |

### Test example 4: Pharmacokinetic experiment of compounds

Pharmacokinetic assays were performed on the compounds of the present disclosure. Specifically, pharmacokinetic parameters of the compounds of the present disclosure were determined using the following methods.

Healthy male adult mice were used in this study and each group of animals was administrated 5 to 100 mg/kg by single gavage. Fasting was maintained from 10 hours before administration to 4 hours after administration. After administration, blood was collected at different time points, and the plasma content of the compound was determined (using LC-MS/MS). Plasma concentration-time relationships were analyzed using professional software (WinNonlin) to calculate the pharmacokinetic parameters of the compounds. The compounds of the present disclosure have excellent pharmacokinetic properties.

All documents mentioned in the present disclosure are incorporated herein by reference in their entirety as if each document is individually cited and incorporated herein. The above content describes the preferred embodiments of the present disclosure in detail, but the present disclosure is not limited to the specific details in the above embodiments. Within the scope of the technical concept of the present disclosure, various simple modifications can be made to the technical solution of the present disclosure, and these simple modifications all belong to the protection scope of the present disclosure.

In addition, it should be noted that each specific technical feature described in the above detailed description of the preferred embodiment can be combined in any suitable manner as long as there is no contradiction. In order to avoid unnecessary repetition, various possible combinations are not further described in the present disclosure. In addition, various embodiments of the present disclosure can also be arbitrarily combined, as long as they do not violate the idea of the present disclosure, which should also be regarded as the contents disclosed in the present disclosure.

## Claims

1. A compound of the following formula I or a pharmaceutically acceptable salt, enantiomer, diastereomer, tautomer, solvate, isotopic substitute, polymorph, prodrug or metabolite thereof: 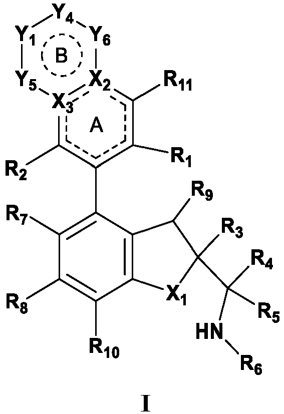 in the formula:
X₁ is selected from: O and NRₐ;
X₂ is selected from: C and N;
X₃ is selected from: C and N;
Y₅ and Y₆ are independently selected from: a bond, O, S, N, -S(O)-, -S(O)₂-, C, CH(R₁₃), C(R₁₃)₂, C(R₁₃), -OCH₂-, -N(R₁₂)CH₂-, -CH₂CH₂- and N(R₁₂), and Y₅ and Y₆ are not simultaneously a bond;
Y₁ and Y₄ are independently selected from: a bond, O, N, C, S, -S(O)-, -C(O)-, -S(O)₂-, CH, CH₂, NH, C(R₁₃), CH(R₁₃), C(R₁₃)₂ and N(R₁₂), provided that Y₁ and Y₄, Y₁ and Y₅, Y₄ and Y₆ are not simultaneously a bond, O, S, -S(O)- or -S(O)₂- and satisfy the valence bond theory;
alternatively, when Y₁, Y₄ and Y₆ are arbitrarily selected from C(R₁₃)₂, two R₁₃ on the same carbon atom, together with the C atom to which they are attached, can form a substituted or unsubstituted 3- to 8-membered cyclohydrocarbyl or substituted or unsubstituted 4- to 9-membered heterocyclyl ring, thereby forming a spiro ring structure with ring B;
alternatively, the ring atom in Y₁ or Y₆ and the ring atom in Y₄, together with the substituent attached to the ring atom, form a substituted or unsubstituted 3- to 8-membered cyclohydrocarbyl, substituted or unsubstituted 4- to 9-membered heterocyclyl, substituted or unsubstituted 6- to 10-membered aryl or substituted or unsubstituted 5- to 10-membered heteroaryl ring, thereby forming a fused ring structure with ring B;
alternatively, X₂, together with the ring atom in Y₆ and the ring atom attached to R₁₁ and the substituent attached to the ring atom, forms a substituted or unsubstituted 5- or 6-membered cyclohydrocarbyl, substituted or unsubstituted 5- or 6-membered heterocyclyl, substituted or unsubstituted phenyl or substituted or unsubstituted 5- or 6-membered heteroaryl ring, thereby forming a three-fused ring structure with rings A and B;
ring B is a 5-, 6- or 7-membered heterocyclyl ring, a benzene ring or a 5-, 6- or 7-membered heteroaryl ring; ring B can be optionally substituted by one or more R₁₃ and/or R₁₂ that do not participate in ring formation, and can be further optionally substituted by 1, 2 or 3 substituents selected from hydroxyl, amino, halogen, substituted or unsubstituted alkyl, -NR'R" and substituted or unsubstituted alkoxy;
R₁ is selected from: cyano and -C(O)NR'R";
R₂ is selected from: H, halogen, substituted or unsubstituted alkyl and substituted or unsubstituted alkoxy;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₇ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl;
R₁₁ is selected from: H, hydroxyl, amino, halogen, substituted or unsubstituted alkyl, -NR'R" and substituted or unsubstituted alkoxy;
each R₁₂ that does not participate in ring formation is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 6- to 14-membered aryl-S(O)₂-, substituted or unsubstituted 4- to 9-membered heterocyclyl, NR'R"-C(O)-, substituted or unsubstituted 3- to 8-membered cyclohydrocarbyl-C(O)- and substituted or unsubstituted C1-C4 alkyl;
each R₁₃ that does not participate in ring formation is independently selected from: H, halogen, substituted or unsubstituted C1-C4 acyl, hydroxyl, cyano, -NR'R", substituted or unsubstituted C1-C4 alkoxy, substituted or unsubstituted C1-6 alkyl, substituted or unsubstituted C1-C4 alkylS(O)₂-, substituted or unsubstituted C6-14 aryl-S(O)₂-, substituted or unsubstituted 3- to 6-membered cyclohydrocarbyl-C(O)- and NR'R"-C(O)-, or two R₁₃ on the same carbon atom form an oxo (=O) or thio (=S) group;
after R₁₃ or R₁₂ participates in ring formation, its ring atom can be optionally substituted by 1 to 3 heteroatoms selected from O, N and S;
R' and R" are each independently selected from H, C1-C4 alkyl, C3-C6 cycloalkyl, C1-C4 alkyl-S(O)₂- and C1-C4 haloalkyl;
Rₐ is selected from H and C1-C4 alkyl;
wherein the dashed line indicates the presence or absence of a double bond.

2. The compound or the pharmaceutically acceptable salt, enantiomer, diastereomer, tautomer, solvate, isotopic substitute, polymorph, prodrug or metabolite thereof according to claim 1, wherein ring A is a benzene ring or a 6-membered heteroaryl ring.

3. The compound or the pharmaceutically acceptable salt, enantiomer, diastereomer, tautomer, solvate, isotopic substitute, polymorph, prodrug or metabolite thereof according to claim 1 or 2, wherein ring A is a benzene ring or a pyridine ring.

4. The compound or the pharmaceutically acceptable salt, enantiomer, diastereomer, tautomer, solvate, isotopic substitute, polymorph, prodrug or metabolite thereof according to any one of claims 1 to 3, wherein the compound of formula I has a structure shown in the following formula Ia:

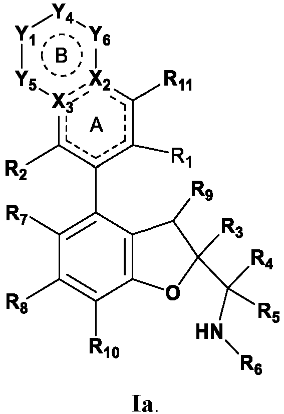

5. The compound or the pharmaceutically acceptable salt, enantiomer, diastereomer, tautomer, solvate, isotopic substitute, polymorph, prodrug or metabolite thereof according to any one of claims 1 to 4, wherein the compound of formula I has a structure shown in the following formula Ib: 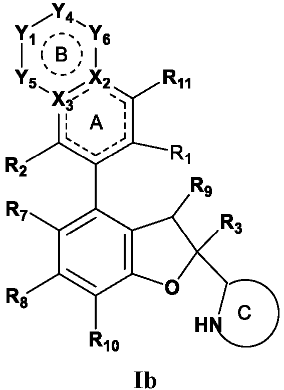 in the formula,
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

6. The compound or the pharmaceutically acceptable salt, enantiomer, diastereomer, tautomer, solvate, isotopic substitute, polymorph, prodrug or metabolite thereof according to any one of claims 1 to 5, wherein when R₁₂, which does not participate in ring formation, is present, each R₁₂ is independently selected from: H, substituted or unsubstituted C1-C4 acyl, substituted or unsubstituted C1-C4 alkoxy, substituted or unsubstituted C1-C4 alkyl-S(O)₂-, substituted or unsubstituted 4- to 9-membered heterocyclyl, NR'R"-C(O)-, substituted or unsubstituted 3- to 8-membered cyclohydrocarbyl-C(O)- and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 8-membered cyclohydrocarbyl-C(O)- and C1-C4 alkyl are optionally substituted by 1, 2 or 3 substituents selected from F, hydroxyl and cyano.

7. The compound or the pharmaceutically acceptable salt, enantiomer, diastereomer, tautomer, solvate, isotopic substitute, polymorph, prodrug or metabolite thereof according to any one of claims 1 to 6, wherein when R₁₂, which does not participate in ring formation, is present, each R₁₂ is independently selected from: H, acetyl, propionyl, C1-C4 alkoxy, C1-C4 alkyl-S(O)₂-, 4- to 6-membered heterocyclyl, 3- to 5-membered cyclohydrocarbyl-C(O)- and substituted or unsubstituted C1-C4 alkyl; preferably, the C1-C4 alkyl is optionally substituted by 1, 2 or 3 substituents selected from F, hydroxyl and cyano.

8. The compound or the pharmaceutically acceptable salt, enantiomer, diastereomer, tautomer, solvate, isotopic substitute, polymorph, prodrug or metabolite thereof according to any one of claims 1 to 7, wherein when R₁₃, which does not participate in ring formation, is present, each R₁₃ is independently selected from: H, halogen, substituted or unsubstituted C1-C4 acyl, hydroxyl, cyano, -NR'R", substituted or unsubstituted C1-C4 alkoxy, substituted or unsubstituted C1-C6 alkyl, substituted or unsubstituted C1-C4 alkyl-S(O)₂-, substituted or unsubstituted 3- to 6-membered cyclohydrocarbyl-C(O)- and NR'R"-C(O)-, or two R₁₃ on the same carbon atom form an oxo (=O) or thio (=S) group; wherein R' and R" are each independently selected from H and C1-C4 alkyl; preferably, the C1-C4 acyl, C1-C4 alkoxy, C1-C6 alkyl, C1-C4 alkyl-S(O)₂- and 3-to 6-membered cyclohydrocarbyl-C(O)- are optionally substituted by 1, 2 or 3 substituents selected from F, hydroxyl and cyano.

9. The compound or the pharmaceutically acceptable salt, enantiomer, diastereomer, tautomer, solvate, isotopic substitute, polymorph, prodrug or metabolite thereof according to any one of claims 1 to 8, wherein when R₁₃, which does not participate in ring formation, is present, each R₁₃ is independently selected from: H, fluorine, chlorine, C1-C4 acyl, hydroxyl, cyano, C1-C4 alkoxy, C1-C6 alkyl, C1-C4 alkyl-S(O)₂- and 3- to 4-membered cyclohydrocarbyl-C(O)-.

10. The compound or the pharmaceutically acceptable salt, enantiomer, diastereomer, tautomer, solvate, isotopic substitute, polymorph, prodrug or metabolite thereof according to any one of claims 1 to 9, wherein the compound of formula I has a structure shown in the following formula IIa: 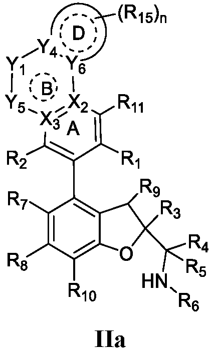 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

11. The compound or the pharmaceutically acceptable salt, enantiomer, diastereomer, tautomer, solvate, isotopic substitute, polymorph, prodrug or metabolite thereof according to any one of claims 1 to 9, wherein the compound of formula I has a structure shown in the following formula lib: 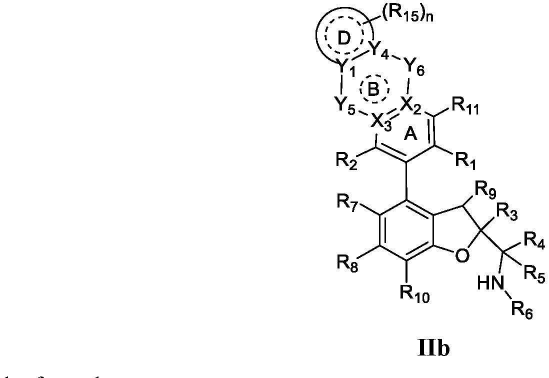 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

12. The compound or the pharmaceutically acceptable salt, enantiomer, diastereomer, tautomer, solvate, isotopic substitute, polymorph, prodrug or metabolite thereof according to claim 10 or 11, wherein ring D is a 3- to 6-membered cyclohydrocarbyl, 4- to 6-membered heterocyclyl, 6- to 10-membered aryl or 5- to 8-membered heteroaryl ring, wherein the 4- to 6-membered heterocyclyl and 5- to 8-membered heteroaryl contain 1, 2 or 3 heteroatoms selected from O, N and S; R₁₅ is selected from: H, halogen, substituted or unsubstituted C1-C4 acyl, hydroxyl, substituted or unsubstituted C1-C4 alkoxy, cyano, -NR'R", substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; R' and R" are each independently selected from H and C1-C4 alkyl; n is 0, 1 or 2.

13. The compound or the pharmaceutically acceptable salt, enantiomer, diastereomer, tautomer, solvate, isotopic substitute, polymorph, prodrug or metabolite thereof according to any one of claims 10 to 12, wherein R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl; n is 0 or 1.

14. The compound or the pharmaceutically acceptable salt, enantiomer, diastereomer, tautomer, solvate, isotopic substitute, polymorph, prodrug or metabolite thereof according to any one of claims 1 to 9, wherein the compound of formula I has a structure shown in the following formula IIc: 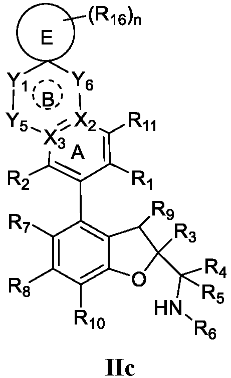 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

15. The compound or the pharmaceutically acceptable salt, enantiomer, diastereomer, tautomer, solvate, isotopic substitute, polymorph, prodrug or metabolite thereof according to any one of claims 1 to 9, wherein the compound of formula I has a structure shown in the following formula IId: 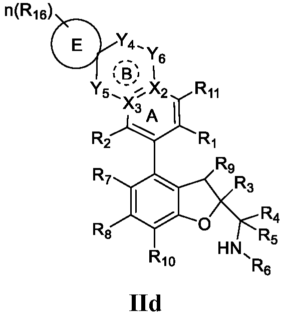 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

16. The compound or the pharmaceutically acceptable salt, enantiomer, diastereomer, tautomer, solvate, isotopic substitute, polymorph, prodrug or metabolite thereof according to claim 14 or 15, wherein ring E is a 3- to 6-membered cyclohydrocarbyl or 4- to 6-membered heterocyclyl ring, wherein the 4- to 6-membered heterocyclyl contains 1, 2 or 3 heteroatoms selected from O, N and S; R₁₆ is selected from H, halogen, C1-C4 alkyl, hydroxyl and acetyl; n is 0 or 1.

17. The compound or the pharmaceutically acceptable salt, enantiomer, diastereomer, tautomer, solvate, isotopic substitute, polymorph, prodrug or metabolite thereof according to any one of claims 14 to 16, wherein ring E is a cyclopropyl, cyclobutyl, cyclopentyl, cyclohexyl, oxetanyl, oxolanyl, tetrahydropyranyl, azetidinyl, azacyclopentyl or piperidinyl ring; R₁₆ is selected from H, fluorine, methyl, ethyl, hydroxyl and acetyl; n is 0 or 1.

18. The compound or the pharmaceutically acceptable salt, enantiomer, diastereomer, tautomer, solvate, isotopic substitute, polymorph, prodrug or metabolite thereof according to any one of claims 1 to 9, wherein the compound of formula I has a structure shown in the following formula IIe: 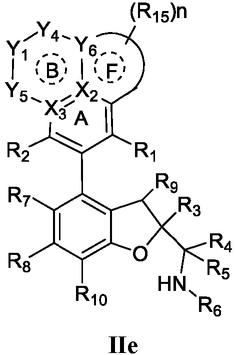 in the formula,
X₂ is C;
Y₆ is selected from: C, CH and N;
ring F is a 5- or 6-membered cyclohydrocarbyl, 5- or 6-membered heterocyclyl, phenyl or 5- or 6-membered heteroaryl ring, wherein the 5- or 6-membered heterocyclyl or 5- or 6-membered heteroaryl contains 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; and
n is 0, 1, 2 or 3.

19. The compound or the pharmaceutically acceptable salt, enantiomer, diastereomer, tautomer, solvate, isotopic substitute, polymorph, prodrug or metabolite thereof according to claim 18, wherein ring F is a phenyl or 5- or 6-membered heteroaryl ring, wherein the 5- or 6-membered heteroaryl contains 1, 2 or 3 heteroatoms selected from O, N and S; and/or
R₁₅ is selected from: H, halogen, substituted or unsubstituted C1-C4 acyl, hydroxyl, substituted or unsubstituted C1-C4 alkoxy, cyano, -NR'R", substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano; wherein R' and R" are each independently selected from H and C1-C4 alkyl, and n is 0, 1 or 2.

20. The compound or the pharmaceutically acceptable salt, enantiomer, diastereomer, tautomer, solvate, isotopic substitute, polymorph, prodrug or metabolite thereof according to claim 18 or 19, wherein ring F is a 5-membered heteroaryl ring, wherein the 5-membered heteroaryl contains 1 or 2 heteroatoms selected from O and N; and/or
R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl; n is 0 or 1.

21. The compound or the pharmaceutically acceptable salt, enantiomer, diastereomer, tautomer, solvate, isotopic substitute, polymorph, prodrug or metabolite thereof according to any one of claims 18 to 20, wherein ring F is a pyrrolyl, furyl, pyrazolyl, imidazolyl, oxazolyl or isoxazolyl ring.

22. The compound or the pharmaceutically acceptable salt, enantiomer, diastereomer, tautomer, solvate, isotopic substitute, polymorph, prodrug or metabolite thereof according to claim 10, wherein the compound of formula IIa has a structure shown in the following formula IIIa: 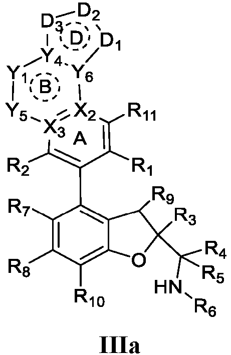 in the formula,
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRₑ, N-NRₑ, CR_{f}-NRₑ, 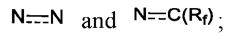
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group; and
each p is independently 1, 2, 3 or 4, preferably 1 or 2.

23. The compound or the pharmaceutically acceptable salt, enantiomer, diastereomer, tautomer, solvate, isotopic substitute, polymorph, prodrug or metabolite thereof according to claim 11, wherein the compound of formula IIb has a structure shown in the following formula IIIb: 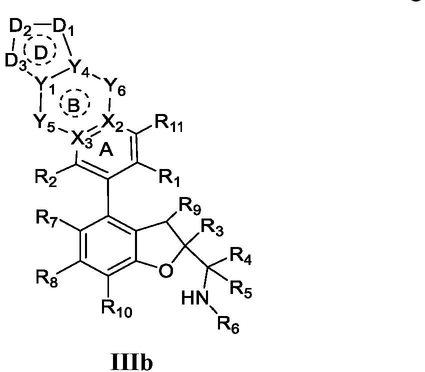 in the formula,
D₁ is selected from: O, S, S(O), S(O)₂, N, NR_{c}, CR_{f} and CR_{f}R_{g};
D₂ is selected from: O, S, S(O), S(O)₂, N, NR_{c}, (CR_{f})ₚ, (CR_{f}R_{g})ₚ, C(O)-NR_{c}, N-NRₑ, CR_{f}-NRₑ, 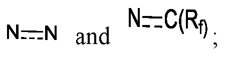
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group; and
each p is independently 1, 2, 3 or 4, preferably 1 or 2.

24. The compound or the pharmaceutically acceptable salt, enantiomer, diastereomer, tautomer, solvate, isotopic substitute, polymorph, prodrug or metabolite thereof according to claim 22, wherein the compound of formula IIIa has a structure shown in the following formula IVa: 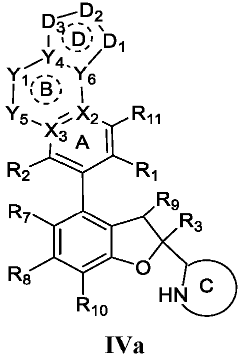 in the formula,
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

25. The compound or the pharmaceutically acceptable salt, enantiomer, diastereomer, tautomer, solvate, isotopic substitute, polymorph, prodrug or metabolite thereof according to claim 23, wherein the compound of formula IIIb has a structure shown in the following formula IVb: 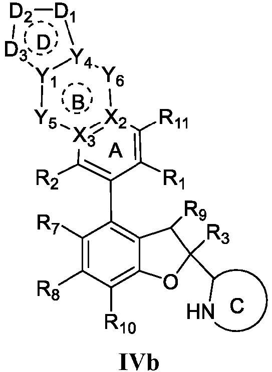 in the formula,
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

26. The compound or the pharmaceutically acceptable salt, enantiomer, diastereomer, tautomer, solvate, isotopic substitute, polymorph, prodrug or metabolite thereof according to claim 18, wherein the compound of formula IIe has a structure shown in the following formula IVc: 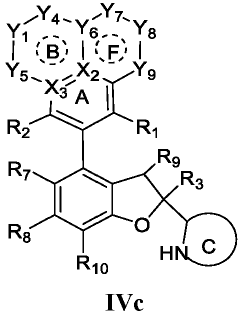 in the formula,
Y₇, Y₈ and Y₉ are independently selected from: a bond, O, S, S(O), S(O)₂, N, NRₑ, CR_{f} and CR_{f}R_{g}, provided that at most one of Y₇, Y₈ and Y₉ is a bond, Y₇ and Y₈, Y₈ and Y₉ are not simultaneously O, S, -S(O)- or -S(O)₂- and satisfy the valence bond theory,
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

27. The compound or the pharmaceutically acceptable salt, enantiomer, diastereomer, tautomer, solvate, isotopic substitute, polymorph, prodrug or metabolite thereof according to any one of claims 1 to 26, wherein R₁ is -C(O)NR'R".

28. The compound or the pharmaceutically acceptable salt, enantiomer, diastereomer, tautomer, solvate, isotopic substitute, polymorph, prodrug or metabolite thereof according to any one of claims 1 to 27, wherein R₁ is -C(O)NHR'; the R' is selected from H, C1-C4 alkyl and C3-C6 cycloalkyl.

29. The compound or the pharmaceutically acceptable salt, enantiomer, diastereomer, tautomer, solvate, isotopic substitute, polymorph, prodrug or metabolite thereof according to any one of claims 1 to 28, wherein R₁ is -C(O)NH₂.

30. The compound or the pharmaceutically acceptable salt, enantiomer, diastereomer, tautomer, solvate, isotopic substitute, polymorph, prodrug or metabolite thereof according to any one of claims 1 to 28, wherein R₁ is -C(O)NHCH₃ and

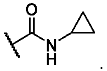

31. The compound or the pharmaceutically acceptable salt, enantiomer, diastereomer, tautomer, solvate, isotopic substitute, polymorph, prodrug or metabolite thereof according to any one of claims 1 to 30, wherein R₂ is selected from H, halogen, C1-C4 alkyl and C1-C4 alkoxy.

32. The compound or the pharmaceutically acceptable salt, enantiomer, diastereomer, tautomer, solvate, isotopic substitute, polymorph, prodrug or metabolite thereof according to any one of claims 1 to 31, wherein R₂ is selected from H, halogen and methoxy.

33. The compound or the pharmaceutically acceptable salt, enantiomer, diastereomer, tautomer, solvate, isotopic substitute, polymorph, prodrug or metabolite thereof according to any one of claims 1 to 32, wherein R₂ is halogen.

34. The compound or the pharmaceutically acceptable salt, enantiomer, diastereomer, tautomer, solvate, isotopic substitute, polymorph, prodrug or metabolite thereof according to any one of claims 1 to 33, wherein R₂ is selected from fluorine and chlorine.

35. The compound or the pharmaceutically acceptable salt, enantiomer, diastereomer, tautomer, solvate, isotopic substitute, polymorph, prodrug or metabolite thereof according to any one of claims 1 to 34, wherein R₂ is fluorine.

36. The compound or the pharmaceutically acceptable salt, enantiomer, diastereomer, tautomer, solvate, isotopic substitute, polymorph, prodrug or metabolite thereof according to any one of claims 1 to 35, wherein R₃ is selected from substituted or unsubstituted aryl and substituted or unsubstituted heteroaryl.

37. The compound or the pharmaceutically acceptable salt, enantiomer, diastereomer, tautomer, solvate, isotopic substitute, polymorph, prodrug or metabolite thereof according to any one of claims 1 to 36, wherein R₃ is selected from substituted or unsubstituted phenyl and substituted or unsubstituted pyridyl.

38. The compound or the pharmaceutically acceptable salt, enantiomer, diastereomer, tautomer, solvate, isotopic substitute, polymorph, prodrug or metabolite thereof according to any one of claims 1 to 37, wherein R₃ is phenyl, fluorophenyl or pyridyl.

39. The compound or the pharmaceutically acceptable salt, enantiomer, diastereomer, tautomer, solvate, isotopic substitute, polymorph, prodrug or metabolite thereof according to any one of claims 1 to 38, wherein R₄ and R₅ are each independently selected from: H and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S.

40. The compound or the pharmaceutically acceptable salt, enantiomer, diastereomer, tautomer, solvate, isotopic substitute, polymorph, prodrug or metabolite thereof according to any one of claims 1 to 39, wherein R₄ and R₅ are each independently selected from: H and substituted or unsubstituted C1-C4 alkyl.

41. The compound or the pharmaceutically acceptable salt, enantiomer, diastereomer, tautomer, solvate, isotopic substitute, polymorph, prodrug or metabolite thereof according to any one of claims 1 to 40, wherein R₄ and R₅ are each independently selected from: Hand C1-C3 alkyl.

42. The compound or the pharmaceutically acceptable salt, enantiomer, diastereomer, tautomer, solvate, isotopic substitute, polymorph, prodrug or metabolite thereof according to any one of claims 1 to 41, wherein R₄ and R₅ are each independently selected from: H and methyl.

43. The compound or the pharmaceutically acceptable salt, enantiomer, diastereomer, tautomer, solvate, isotopic substitute, polymorph, prodrug or metabolite thereof according to any one of claims 1 to 42, wherein R₄ and R₅ are both H.

44. The compound or the pharmaceutically acceptable salt, enantiomer, diastereomer, tautomer, solvate, isotopic substitute, polymorph, prodrug or metabolite thereof according to any one of claims 1 to 43, wherein R₇ and R₈ are each independently selected from: H, halogen, cyano, substituted or unsubstituted C1-C4 alkyl, substituted or unsubstituted C1-C4 haloalkyl, substituted or unsubstituted C1-C4 alkoxy, substituted or unsubstituted C2-C4 alkenyl and substituted or unsubstituted C2-C4 alkynyl.

45. The compound or the pharmaceutically acceptable salt, enantiomer, diastereomer, tautomer, solvate, isotopic substitute, polymorph, prodrug or metabolite thereof according to any one of claims 1 to 44, wherein R₇ and R₈ are each independently selected from: H, halogen, cyano, methyl and methoxy.

46. The compound or the pharmaceutically acceptable salt, enantiomer, diastereomer, tautomer, solvate, isotopic substitute, polymorph, prodrug or metabolite thereof according to any one of claims 1 to 45, wherein R₇ and R₈ are each independently selected from: fluorine and chlorine.

47. The compound or the pharmaceutically acceptable salt, enantiomer, diastereomer, tautomer, solvate, isotopic substitute, polymorph, prodrug or metabolite thereof according to any one of claims 1 to 46, wherein R₇ is chlorine, R₈ is fluorine.

48. The compound or the pharmaceutically acceptable salt, enantiomer, diastereomer, tautomer, solvate, isotopic substitute, polymorph, prodrug or metabolite thereof according to any one of claims 1 to 47, wherein R₉ is selected from H, C1-C4 alkyl, C1-C4 alkoxy, hydroxyl and halogen.

49. The compound or the pharmaceutically acceptable salt, enantiomer, diastereomer, tautomer, solvate, isotopic substitute, polymorph, prodrug or metabolite thereof according to any one of claims 1 to 48, wherein R₉ is selected from H, methyl, methoxy, hydroxyl and F.

50. The compound or the pharmaceutically acceptable salt, enantiomer, diastereomer, tautomer, solvate, isotopic substitute, polymorph, prodrug or metabolite thereof according to any one of claims 1 to 49, wherein R₉ is H.

51. The compound or the pharmaceutically acceptable salt, enantiomer, diastereomer, tautomer, solvate, isotopic substitute, polymorph, prodrug or metabolite thereof according to any one of claims 1 to 50, wherein R₁₀ is selected from: H, halogen and C1-C4 alkyl.

52. The compound or the pharmaceutically acceptable salt, enantiomer, diastereomer, tautomer, solvate, isotopic substitute, polymorph, prodrug or metabolite thereof according to any one of claims 1 to 51, wherein R₁₀ is selected from: H, F and methyl.

53. The compound or the pharmaceutically acceptable salt, enantiomer, diastereomer, tautomer, solvate, isotopic substitute, polymorph, prodrug or metabolite thereof according to any one of claims 1 to 52, wherein R₁₀ is H.

54. The compound or the pharmaceutically acceptable salt, enantiomer, diastereomer, tautomer, solvate, isotopic substitute, polymorph, prodrug or metabolite thereof according to any one of claims 1 to 53, wherein R₁₁ is selected from: H, hydroxyl, amino, halogen, C1-C4 alkyl, - NR'R" and C1-C4 alkoxy, wherein R' and R" are independently selected from C1-C4 alkyl.

55. The compound or the pharmaceutically acceptable salt, enantiomer, diastereomer, tautomer, solvate, isotopic substitute, polymorph, prodrug or metabolite thereof according to any one of claims 1 to 54, wherein R₁₁ is selected from: H, hydroxyl, amino, fluorine, chlorine, methyl, methoxy and -NH(CH₃).

56. The compound or the pharmaceutically acceptable salt, enantiomer, diastereomer, tautomer, solvate, isotopic substitute, polymorph, prodrug or metabolite thereof according to any one of claims 1 to 55, wherein R₁₁ is H.

57. The compound or the pharmaceutically acceptable salt, enantiomer, diastereomer, tautomer, solvate, isotopic substitute, polymorph, prodrug or metabolite thereof according to any one of claims 1 to 56, wherein X₂ is C.

58. The compound or the pharmaceutically acceptable salt, enantiomer, diastereomer, tautomer, solvate, isotopic substitute, polymorph, prodrug or metabolite thereof according to any one of claims 1 to 56, wherein X₂ is N.

59. The compound or the pharmaceutically acceptable salt, enantiomer, diastereomer, tautomer, solvate, isotopic substitute, polymorph, prodrug or metabolite thereof according to any one of claims 1 to 58, wherein X₃ is C.

60. The compound or the pharmaceutically acceptable salt, enantiomer, diastereomer, tautomer, solvate, isotopic substitute, polymorph, prodrug or metabolite thereof according to any one of claims 1 to 58, wherein X₃ is N.

61. The compound or the pharmaceutically acceptable salt, enantiomer, diastereomer, tautomer, solvate, isotopic substitute, polymorph, prodrug or metabolite thereof according to any one of claims 1 to 60, wherein Y₅ is selected from: a bond, O, S, -S(O)-, N, CH₂, CH, -C(O)-, - CH(OH)-, NH, -N(CH₃)-, -OCH₂-, -NHCH₂-, -N(CH₃)CH₂- and -CH₂CH₂-.

62. The compound or the pharmaceutically acceptable salt, enantiomer, diastereomer, tautomer, solvate, isotopic substitute, polymorph, prodrug or metabolite thereof according to any one of claims 1 to 61, wherein Y₅ is O.

63. The compound or the pharmaceutically acceptable salt, enantiomer, diastereomer, tautomer, solvate, isotopic substitute, polymorph, prodrug or metabolite thereof according to any one of claims 1 to 61, wherein Y₅ is CH or CH₂.

64. The compound or the pharmaceutically acceptable salt, enantiomer, diastereomer, tautomer, solvate, isotopic substitute, polymorph, prodrug or metabolite thereof according to any one of claims 1 to 61, wherein Y₅ is NH or N.

65. The compound or the pharmaceutically acceptable salt, enantiomer, diastereomer, tautomer, solvate, isotopic substitute, polymorph, prodrug or metabolite thereof according to any one of claims 1 to 64, wherein Y₆ is selected from: a bond, O, S, -S(O)-, -S(O)₂-, N, CH, -C(O)-, CH₂, NH, -N(CH₃)-, -N(C₂H₄OH)-, -N(C(O)CH₃)-, -N(C(O)C₂H₅)-, -OCH₂-, -NHCH₂-, - N(CH₃)CH₂- and -CH₂CH₂-.

66. The compound or the pharmaceutically acceptable salt, enantiomer, diastereomer, tautomer, solvate, isotopic substitute, polymorph, prodrug or metabolite thereof according to any one of claims 1 to 65, wherein Y₁ and Y₄ are independently selected from: a bond, O, N, NH, - N(CH₃)-, CH, CH₂ and -CH(CH₃)-, and Y₁ and Y₄ are not simultaneously a bond or O.

67. The compound or the pharmaceutically acceptable salt, enantiomer, diastereomer, tautomer, solvate, isotopic substitute, polymorph, prodrug or metabolite thereof according to any one of claims 1 to 66, wherein R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl.

68. The compound or the pharmaceutically acceptable salt, enantiomer, diastereomer, tautomer, solvate, isotopic substitute, polymorph, prodrug or metabolite thereof according to any one of claims 1 to 67, wherein R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, -NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl.

69. The compound or the pharmaceutically acceptable salt, enantiomer, diastereomer, tautomer, solvate, isotopic substitute, polymorph, prodrug or metabolite thereof according to any one of claims 1 to 68, wherein R₆ is -L-W-Z, wherein L is a bond or substituted or unsubstituted C1-C4 alkyl; W is a bond, substituted or unsubstituted C1-C4 alkyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl; Z is hydrogen, halogen, C1-C4 alkyl, C1-C4 alkoxy, hydroxyl, -S(O)₂-C1-C4 alkyl, -S(O)₂-NR'R", -S(O)₂-NH₂, cyano, -C(O)-C1-C4 alkyl, -O-3- to 8-membered cyclohydrocarbyl or -NR'-C(O)-C1-C4 alkyl, wherein R' and R" are each independently H or C1-C4 alkyl.

70. The compound or the pharmaceutically acceptable salt, enantiomer, diastereomer, tautomer, solvate, isotopic substitute, polymorph, prodrug or metabolite thereof according to claim 69, wherein the -L-W-Z is selected from: 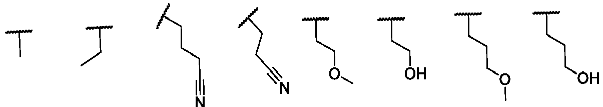 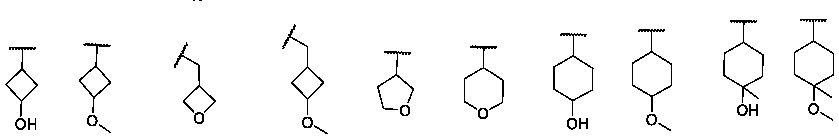 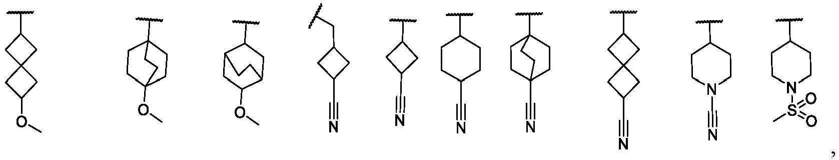 wherein the wavy line indicates the bond connection position.

71. The compound or the pharmaceutically acceptable salt, enantiomer, diastereomer, tautomer, solvate, isotopic substitute, polymorph, prodrug or metabolite thereof according to any one of claims 1 to 70, wherein R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl or 6- to 12-membered spiro heterocyclyl are optionally substituted by 1, 2 or 3 substituents selected from H, deuterium, hydroxyl, cyano, C1-C4 alkyl, halogen and C1-C4 alkoxy.

72. The compound or the pharmaceutically acceptable salt, enantiomer, diastereomer, tautomer, solvate, isotopic substitute, polymorph, prodrug or metabolite thereof according to any one of claims 1 to 71, wherein R₄ or R₅ and R₆, together with the atom to which they are attached, form the following group optionally substituted by 1, 2 or 3 substituents selected from H, deuterium, hydroxyl, methyl, F and methoxy: 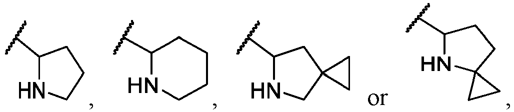 wherein the wavy line indicates the bond connection position.

73. The compound or the pharmaceutically acceptable salt, enantiomer, diastereomer, tautomer, solvate, isotopic substitute, polymorph, prodrug or metabolite thereof according to any one of claims 1 to 72, wherein R₄ or R₅ and R₆, together with the atom to which they are attached, form 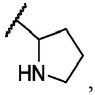 which is optionally substituted by 1, 2 or 3 substituents selected from H, deuterium, hydroxyl, methyl, F and methoxy.

74. The compound or the pharmaceutically acceptable salt, enantiomer, diastereomer, tautomer, solvate, isotopic substitute, polymorph, prodrug or metabolite thereof according to any one of claims 5 and 24 to 26, wherein ring C is a 4- to 9-membered monocyclic or fused heterocyclyl ring, which is optionally substituted by 1, 2 or 3 substituents selected from H, deuterium, hydroxyl, cyano, C1-C4 alkyl, halogen and C1-C4 alkoxy.

75. The compound or the pharmaceutically acceptable salt, enantiomer, diastereomer, tautomer, solvate, isotopic substitute, polymorph, prodrug or metabolite thereof according to any one of claims 5, 24 to 26 and 74, wherein ring C is the following group optionally substituted by 1, 2 or 3 substituents selected from H, deuterium, hydroxyl, methyl, F and methoxy: 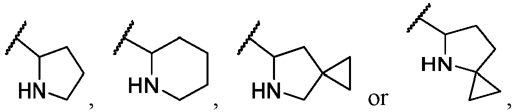 wherein the wavy line indicates the bond connection position.

76. The compound or the pharmaceutically acceptable salt, enantiomer, diastereomer, tautomer, solvate, isotopic substitute, polymorph, prodrug or metabolite thereof according to any one of claims 5, 24 to 26 and 74 to 75, wherein ring C is 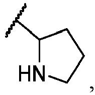 which is optionally substituted by 1, 2 or 3 substituents selected from H, deuterium, hydroxyl, methyl, F and methoxy.

77. The compound or the pharmaceutically acceptable salt, enantiomer, diastereomer, tautomer, solvate, isotopic substitute, polymorph, prodrug or metabolite thereof according to any one of claims 1 to 76, wherein the ring A and ring B, together with their substituents, form any one of the following groups: 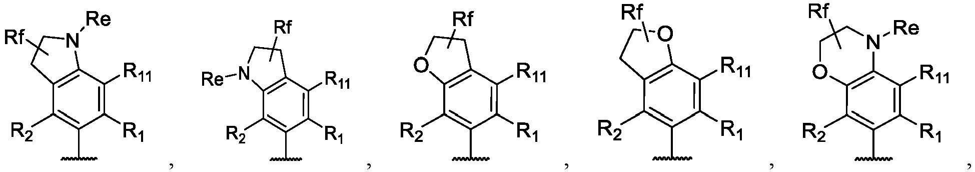 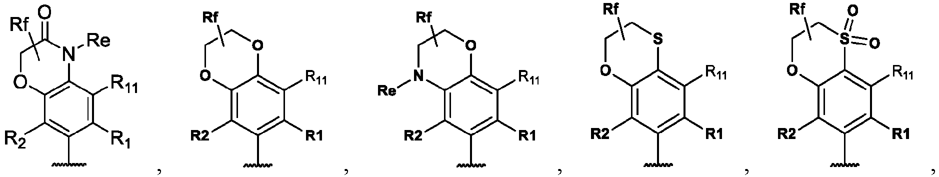 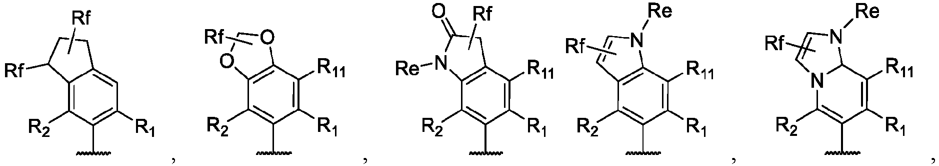 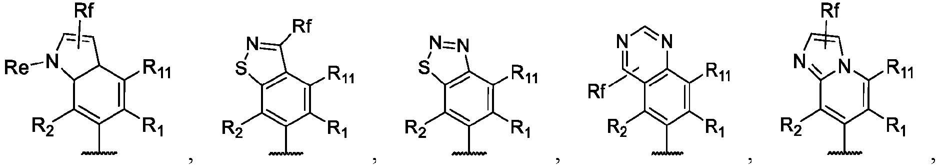 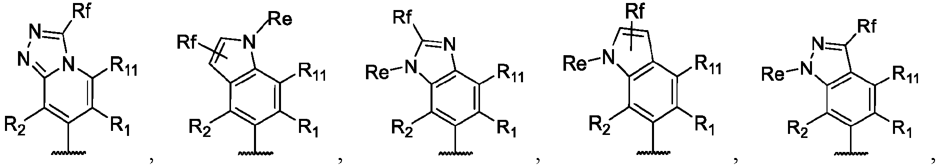 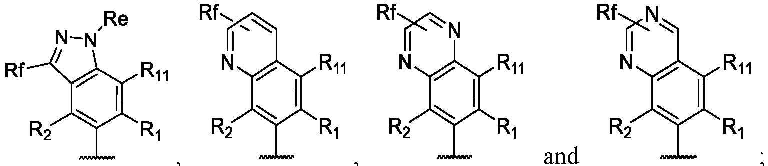 wherein R₁ is cyano or -C(O)NR'R"; R₂ is H or halogen; R₁₁ is H, hydroxyl, -NR'R", halogen or C1-C4 alkyl; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3- to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H and C1-C4 alkyl; the wavy line indicates the bond connection position.

78. The compound or the pharmaceutically acceptable salt, enantiomer, diastereomer, tautomer, solvate, isotopic substitute, polymorph, prodrug or metabolite thereof according to any one of claims 1 to 77, wherein R₁ is cyano, -C(O)NH₂ or -C(O)NHCH₃; R₂ is H or F; R₁₁ is H; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂(CH₃)OH, acetyl, propionyl and cyclopropionyl; R_{f} is selected from H, F, Cl, hydroxyl, cyano, -NH₂, -CH₂OH, -(CH₂)₂OH, -(CH₂)₂OCH3, methoxy, methyl, ethyl, cyclopropyl and cyclobutyl.

79. The compound or the pharmaceutically acceptable salt, enantiomer, diastereomer, tautomer, solvate, isotopic substitute, polymorph, prodrug or metabolite thereof according to any one of claims 1 to 78, wherein R₁ is -C(O)NH₂ or -C(O)NHCH₃; R₂ is F; R₁₁ is H; Rₑ is selected from H, methyl, difluoromethyl, trifluoromethyl, ethyl, fluoroethyl, difluoroethyl, trifluoroethyl, isopropyl, -CH₂CH₂OH, -CH₂CH₂(CH₃)OH, trifluoropropyl, oxocyclobutyl, 1,1-difluorocyclobutyl, cyclopropyl and acetyl; R_{f} is selected from H, F, Cl, hydroxyl, cyano, -NH₂, - CH₂OH, -(CH₂)₂OH, -(CH₂)₂OCH₃, methoxy and methyl.

80. The compound or the pharmaceutically acceptable salt, enantiomer, diastereomer, tautomer, solvate, isotopic substitute, polymorph, prodrug or metabolite thereof according to any one of claims 1 to 76, wherein the ring A and ring B, together with their substituents, form any one of the following groups: 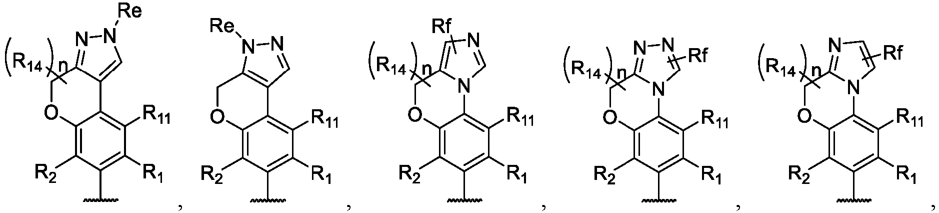 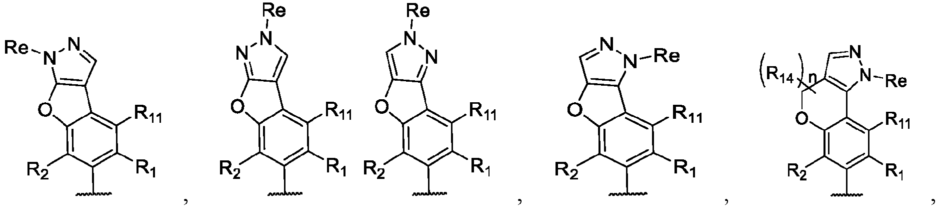 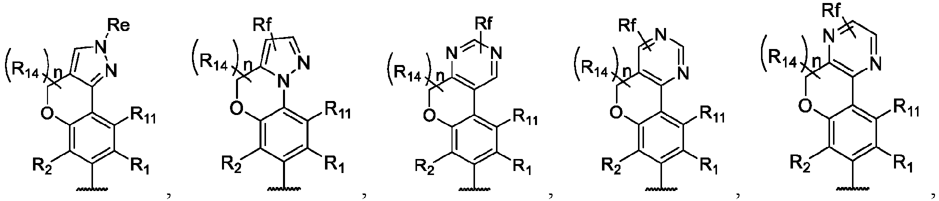 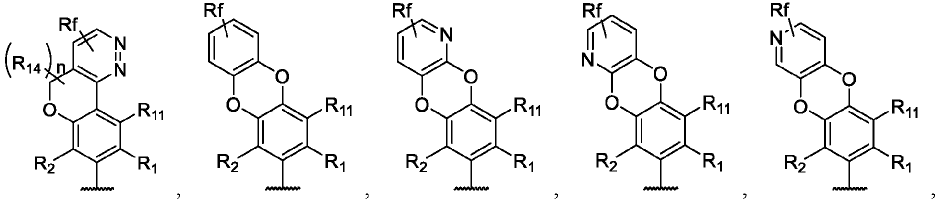 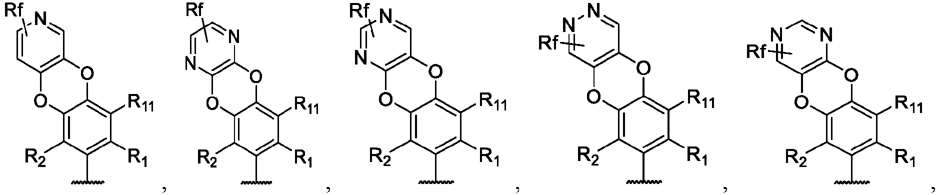 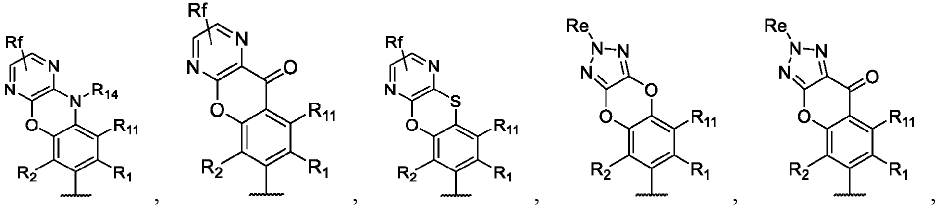 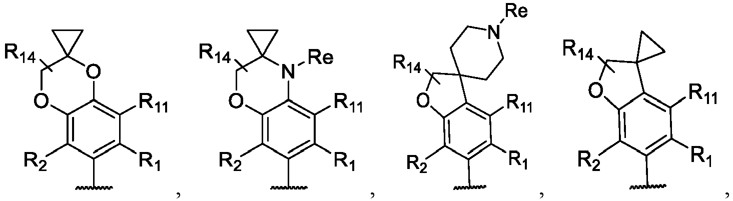 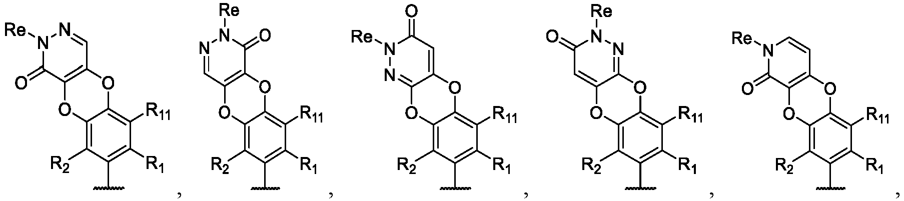 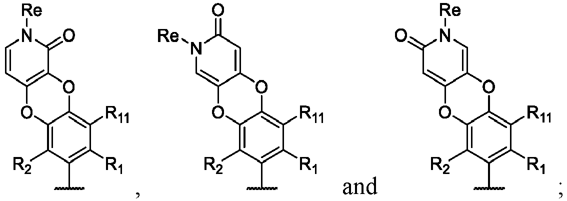 wherein R₁ is cyano or -C(O)NR'R"; R₂ is H or halogen; R₁₁ is H, hydroxyl, -NR'R", halogen or C1-C4 alkyl; R₁₄ is H, halogen or C1-C4 alkyl; n is 0, 1 or 2; R₁₆ is selected from H, hydroxyl, halogen and C1-C4 alkyl; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3- to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H, C1-C4 alkyl and C3-C6 cycloalkyl; the wavy line indicates the bond connection position.

81. The compound or the pharmaceutically acceptable salt, enantiomer, diastereomer, tautomer, solvate, isotopic substitute, polymorph, prodrug or metabolite thereof according to any one of claims 1 to 76 and 80, wherein R₁ is cyano, -C(O)NH₂ or -C(O)NHCH₃; R₂ is H or F; R₁₁ is H; R₁₄ is H or methyl; n is 0, 1 or 2; R₁₆ is H, hydroxyl or methyl; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, - CH₂CH₂(CH₃)OH, acetyl, propionyl and cyclopropionyl; R_{f} is selected from H, F, Cl, hydroxyl, cyano, -NH₂, -CH₂OH, -(CH₂)₂OH, -(CH₂)₂OCH₃, methoxy, methyl, ethyl, cyclopropyl and cyclobutyl.

82. The compound or the pharmaceutically acceptable salt, enantiomer, diastereomer, tautomer, solvate, isotopic substitute, polymorph, prodrug or metabolite thereof according to any one of claims 1 to 76 and 80 to 81, wherein R₁ is -C(O)NH₂ or -C(O)NHCH₃; R₂ is F; R₁₁ is H; R₁₄ is H; R₁₆ is H; R_{c} is selected from H, methyl, difluoromethyl, trifluoromethyl, ethyl, fluoroethyl, difluoroethyl, trifluoroethyl, isopropyl, trifluoropropyl, oxocyclobutyl, 1,1-difluorocyclobutyl, cyclopropyl, -CH₂CH₂OH, -CH₂CH₂(CH₃)OH and acetyl; R_{f} is selected from H, F, Cl, hydroxyl, cyano, -NH₂, methoxy, -CH₂OH, -(CH₂)₂OH, -(CH₂)₂OCH₃ and methyl.

83. The compound or the pharmaceutically acceptable salt, enantiomer, diastereomer, tautomer, solvate, isotopic substitute, polymorph, prodrug or metabolite thereof according to any one of claims 1 to 76, wherein the ring A and ring B, together with their substituents, form any one of the following groups: 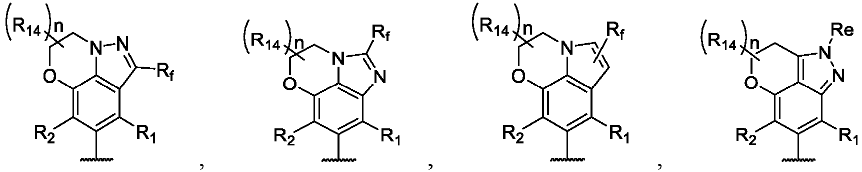 and 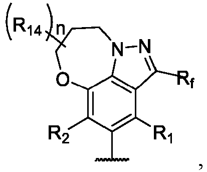 wherein R₁ is cyano or -C(O)NR'R"; R₂ is H or halogen; R₁₄ is H, halogen or C1-C4 alkyl; n is 0, 1 or 2; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3-to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H and C1-C4 alkyl; the wavy line indicates the bond connection position.

84. The compound or the pharmaceutically acceptable salt, enantiomer, diastereomer, tautomer, solvate, isotopic substitute, polymorph, prodrug or metabolite thereof according to any one of claims 1 to 76 and 83, wherein R₁ is cyano, -C(O)NH₂ or -C(O)NHCH₃; R₂ is H or F; R₁₄ is H or methyl; n is 0, 1 or 2; R_{c} is selected from H, methyl, difluoromethyl, trifluoromethyl, ethyl, fluoroethyl, difluoroethyl, trifluoroethyl, isopropyl, ethyl, cyclopropyl, cyclobutyl, -CH₂CH₂OH, -CH₂CH₂(CH₃)OH, acetyl, propionyl and cyclopropionyl; R_{f} is selected from H, F, Cl, hydroxyl, cyano, -NH₂, methoxy, -CH₂OH, -(CH₂)₂OH, -(CH₂)₂OCH₃, methyl, ethyl, cyclopropyl and cyclobutyl.

85. The compound or the pharmaceutically acceptable salt, enantiomer, diastereomer, tautomer, solvate, isotopic substitute, polymorph, prodrug or metabolite thereof according to any one of claims 1 to 84, wherein
X₂ is selected from: C and N;
X₃ is selected from: C and N;
Y₅ and Y₆ are independently selected from: O, S, N, -S(O)-, -S(O)₂-, CH(R₁₃), C(R₁₃)₂, C(R₁₃), -OCH₂-, -N(R₁₂)CH₂-, -CH₂CH₂- and N(R₁₂); and Y₅ and Y₆ are not simultaneously a bond;
Y₁ is selected from: a bond, O, N, CH, CH₂, NH, CH(R₁₃), C(R₁₃)₂ and N(R₁₂);
Y₄ is selected from: a bond, O, N, CH, CH₂, NH, CH(R₁₃), C(R₁₃)₂ and N(R₁₂);
ring B can be optionally substituted by one or more R₁₃ and/or R₁₂ that do not participate in ring formation, and can be further optionally substituted by 1, 2 or 3 substituents selected from hydroxyl, amino, halogen, substituted or unsubstituted C1-C4 alkyl, -NR'R" and substituted or unsubstituted C1-C4 alkoxy;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
each R₁₂ that does not participate in ring formation is independently selected from H, substituted or unsubstituted C1-C4 acyl, substituted or unsubstituted C1-C4 alkoxy, substituted or unsubstituted C1-C4 alkyl-S(O)₂-, substituted or unsubstituted 4- to 9-membered heterocyclyl, NR'R"-C(O)-, 3- to 8-membered cyclohydrocarbyl-C(O)- and substituted or unsubstituted C1-C4 alkyl; the C1-C4 acyl, C1-C4 alkoxy, C1-C4 alkyl-S(O)₂-, 4- to 9-membered heterocyclyl and C1-C4 alkyl are optionally substituted by 1, 2 or 3 substituents selected from F, hydroxyl and cyano;
each R₁₃ that does not participate in ring formation is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted C1-C4 alkylS(O)₂-, substituted or unsubstituted 3- to 6-membered cyclohydrocarbyl-C(O)- and NR'R"-C(O)-; the C1-C4 acyl, C1-C4 alkoxy, C1-C4 alkyl, C1-C4 alkyl-S(O)₂- and 3- to 6-membered cyclohydrocarbyl-C(O)- are optionally substituted by 1, 2 or 3 substituents selected from F, hydroxyl and cyano, or two R₁₃ on the same carbon atom form an oxo (=O) or thio (=S) group;
R' and R" are each independently selected from H, C1-C4 alkyl and C3-C6 cycloalkyl;
wherein the dashed line indicates the presence or absence of a double bond.

86. The compound or the pharmaceutically acceptable salt, enantiomer, diastereomer, tautomer, solvate, isotopic substitute, polymorph, prodrug or metabolite thereof according to any one of claims 1 to 85, wherein
X₂ is selected from: C and N;
X₃ is selected from: C and N;
Y₅ is selected from: O, S, -S(O)-, -S(O)₂-, N, CH₂, CH, -C(O)-, NH and -N(CH₃)-;
Y₆ is selected from: O, S, -S(O)-, -S(O)₂-, N, CH₂, CH, -C(O)-, NH and -N(CH₃)-;
Y₁ is selected from: a bond, O, NH, -N(CH₃)-, CH₂ and -CH(CH₃)-;
Y₄ is selected from: a bond, O, NH, -N(CH₃)-, CH₂ and -CH(CH₃)-;
H on ring B can be further optionally substituted by 1, 2 or 3 substituents selected from hydroxyl, amino, halogen, substituted or unsubstituted C1-C4 alkyl, -NR'R" and substituted or unsubstituted C1-C4 alkoxy;
R₁ is selected from: cyano, -C(O)NH₂, -C(O)NHCH₃ and 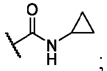
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 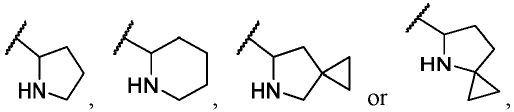 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl; R₁₁ is selected from: H, hydroxyl, amino, fluorine, chlorine, methyl, methoxy and -NH(CH₃).

87. The compound or the pharmaceutically acceptable salt, enantiomer, diastereomer, tautomer, solvate, isotopic substitute, polymorph, prodrug or metabolite thereof according to claim 10, wherein
X₂ is selected from: C and N;
X₃ is selected from: C and N;
Y₁ is selected from: a bond, O, NH, -N(CH₃)-, CH₂ and -CH(CH₃)-;
Y₅ is selected from: O, S, N, CH₂, CH, NH, -N(CH₃)-, -OCH₂-, -NHCH₂-, -N(CH₃)CH₂- and -CH₂CH₂-;
Y₄ is selected from: C, CH or N;
Y₆ is selected from: C, CH or N;
R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl;
n is 0 or 1;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H, C1-C4 alkyl and C3-C6 cycloalkyl;
wherein the dashed line indicates the presence or absence of a double bond.

88. The compound or the pharmaceutically acceptable salt, enantiomer, diastereomer, tautomer, solvate, isotopic substitute, polymorph, prodrug or metabolite thereof according to claim 87, wherein
R₁ is selected from: cyano, -C(O)NH₂, -C(O)NHCH₃ and 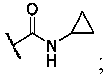
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 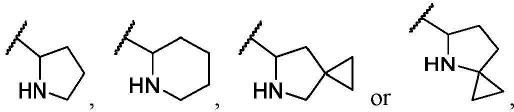 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

89. The compound or the pharmaceutically acceptable salt, enantiomer, diastereomer, tautomer, solvate, isotopic substitute, polymorph, prodrug or metabolite thereof according to claim 11, wherein
X₂ and X₃ are C;
Y₁ is selected from: C, CH and N;
Y₄ is selected from: C, CH and N;
Y₅ is selected from: a bond, O, S, -S(O)-, -S(O)₂-, -C(O)-, N, NH, -N(CH₃)-, CH₂, -CH(CH₃)-, -OCH₂-, -NHCH₂-, -N(CH₃)CH₂- and -CH₂CH₂-;
Y₆ is selected from: a bond, O, S, -S(O)-, -S(O)₂-, -C(O)-, N, CH₂, CH, NH, -N(CH₃)-, - OCH₂-, -NHCH₂-, -N(CH₃)CH₂- and -CH₂CH₂-; and Y₅ and Y₆ are not simultaneously a bond;
R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl;
n is 0 or 1;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H, C1-C4 alkyl and C3-C6 cycloalkyl;
wherein the dashed line indicates the presence or absence of a double bond.

90. The compound or the pharmaceutically acceptable salt, enantiomer, diastereomer, tautomer, solvate, isotopic substitute, polymorph, prodrug or metabolite thereof according to claim 89, wherein
R₁ is selected from: cyano, -C(O)NH₂, -C(O)NHCH₃ and 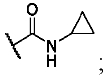
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 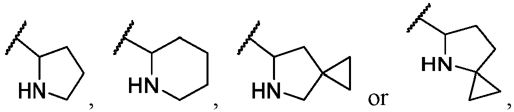 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

91. The compound or the pharmaceutically acceptable salt, enantiomer, diastereomer, tautomer, solvate, isotopic substitute, polymorph, prodrug or metabolite thereof according to claim 14, wherein
X₂ and X₃ are C;
Y₁ is selected from: O or CH₂;
Y₅ is selected from: a bond, O, NH, -N(CH₃)-, CH₂ and -CH(CH₃)-;
Y₆ is selected from: a bond, O, S, N, CH₂, CH, NH and -N(CH₃)-;
R₁₆ is selected from halogen, C1-C4 alkyl, hydroxyl, C1-C4 acyl and C1-C4 alkoxy;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H, C1-C4 alkyl and C3-C6 cycloalkyl;
wherein the dashed line indicates the presence or absence of a double bond.

92. The compound or the pharmaceutically acceptable salt, enantiomer, diastereomer, tautomer, solvate, isotopic substitute, polymorph, prodrug or metabolite thereof according to claim 91, wherein
R₁ is selected from: cyano, -C(O)NH₂, -C(O)NHCH₃ and 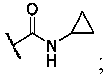
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 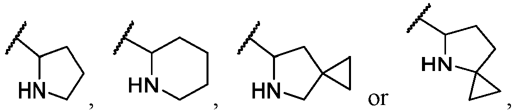 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

93. The compound or the pharmaceutically acceptable salt, enantiomer, diastereomer, tautomer, solvate, isotopic substitute, polymorph, prodrug or metabolite thereof according to claim 15, wherein
X₂ and X₃ are C;
Y₄ is selected from: O or CH₂;
Y₅ is selected from: a bond, O, NH, -N(CH₃)-, CH₂ and -CH(CH₃)-;
Y₆ is selected from: a bond, O, S, N, CH₂, CH, NH and -N(CH₃)-;
R₁₆ is selected from halogen, C1-C4 alkyl, hydroxyl, C1-C4 acyl and C1-C4 alkoxy;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H, C1-C4 alkyl and C3-C6 cycloalkyl;
wherein the dashed line indicates the presence or absence of a double bond.

94. The compound or the pharmaceutically acceptable salt, enantiomer, diastereomer, tautomer, solvate, isotopic substitute, polymorph, prodrug or metabolite thereof according to claim 93, wherein
R₁ is selected from: cyano, -C(O)NH₂, -C(O)NHCH₃ and 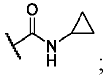
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 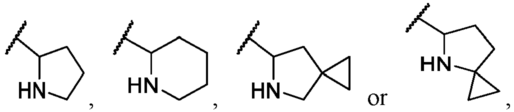 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

95. The compound or the pharmaceutically acceptable salt, enantiomer, diastereomer, tautomer, solvate, isotopic substitute, polymorph, prodrug or metabolite thereof according to claim 18, wherein
Y₁, Y₄ and Y₅ are independently selected from: O, CH, CH₂, -CH(CH₃)-, N, NH and -N(CH₃)-;
ring F is a phenyl or 5- or 6-membered heteroaryl ring, wherein the 5- or 6-membered heteroaryl contains 1, 2 or 3 heteroatoms selected from O, N and S; R₁₅ is selected from: H, fluorine, chlorine, acetyl, hydroxyl, C1-C4 alkoxy, cyano, -NR'R", NR'R"-C(O)-, 3- to 8-membered cyclohydrocarbyl-C(O)-, C1-C4 haloalkyl and C1-C4 alkyl; R' and R" are each independently selected from H and C1-C4 alkyl; n is 0 or 1;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H, C1-C4 alkyl and C3-C6 cycloalkyl;
wherein the dashed line indicates the presence or absence of a double bond.

96. The compound or the pharmaceutically acceptable salt, enantiomer, diastereomer, tautomer, solvate, isotopic substitute, polymorph, prodrug or metabolite thereof according to claim 95, wherein
R₁ is selected from: cyano, -C(O)NH₂, -C(O)NHCH₃ and 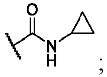
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-C1-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-C1-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 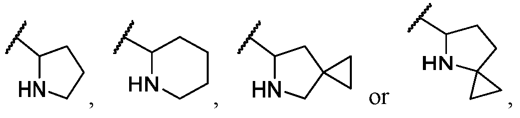 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

97. The compound or the pharmaceutically acceptable salt, enantiomer, diastereomer, tautomer, solvate, isotopic substitute, polymorph, prodrug or metabolite thereof according to claim 22, wherein
X₂ is selected from: C and N;
X₃ is selected from: C and N;
Y₁ is selected from: a bond, O, NH, -N(CH₃)-, CH₂ and -CH(CH₃)-;
Y₅ is selected from: O, S, N, CH₂, CH, NH, -N(CH₃)-, -OCH₂-, -NHCH₂-, -N(CH₃)CH₂- and -CH₂CH₂-;
Y₄ is selected from: C, CH or N;
Y₆ is selected from: C, CH or N;
D₁ is selected from: O, S, N, NR_{c}, CR_{f} and CR_{f}R_{g};
D₂ is selected from: O, S, N, NR_{c}, N-NR_{c}, C(O)-NRₑ, CR_{f}-NR_{c}, (CR_{f})ₚ and (CR_{f}R_{g})ₚ;
D₃ is selected from: O, S, N, NR_{c}, (CR_{f})ₚ and (CR_{f}R_{g})ₚ;
R₁ is selected from: cyano and -C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H, C1-C4 alkyl and C3-C6 cycloalkyl;
wherein the dashed line indicates the presence or absence of a double bond.

98. The compound or the pharmaceutically acceptable salt, enantiomer, diastereomer, tautomer, solvate, isotopic substitute, polymorph, prodrug or metabolite thereof according to claim 97, wherein
R₁ is selected from: cyano, -C(O)NH₂, -C(O)NHCH₃ and 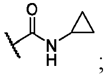
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-C1-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-Cl-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-Cl-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 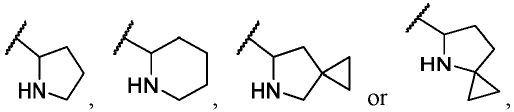 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

99. The compound or the pharmaceutically acceptable salt, enantiomer, diastereomer, tautomer, solvate, isotopic substitute, polymorph, prodrug or metabolite thereof according to claim 23, wherein
X₂ and X₃ are C;
Y₁ is selected from: C, CH and N;
Y₄ is selected from: C, CH and N;
Y₅ is selected from: a bond, O, S, -S(O)-, -S(O)₂-, -C(O)-, N, NH, -N(CH₃)-, CH, CH₂, - CH(CH₃)-, -OCH₂-, -NHCH₂-, -N(CH₃)CH₂- and -CH₂CH₂-;
Y₆ is selected from: a bond, O, S, -S(O)-, -S(O)₂-, -C(O)-, N, CH₂, CH, -CH(CH₃)-, NH, - N(CH₃)-, -OCH₂-, -NHCH₂-, -N(CH₃)CH₂- and -CH₂CH₂-; and Y₅ and Y₆ are not simultaneously a bond;
D₁ is selected from: O, S, N, NRₑ, CR_{f} and CR_{f}R_{g};
D₂ is selected from: O, S, N, NRₑ, C(O)-NRₑ, N-NRₑ, CR_{f}-NRₑ, (CR_{f})ₚ and (CR_{f}R_{g})ₚ;
D₃ is selected from: O, S, N, NRₑ, (CR_{f})ₚ and (CR_{f}R_{g})ₚ; R₁ is selected from: cyano and - C(O)NR'R";
R₂ is selected from: H, halogen, substituted or unsubstituted C1-C4 alkyl and substituted or unsubstituted C1-C4 alkoxy;
R₃ is selected from: substituted or unsubstituted aryl;
R₄ and R₅ are each independently selected from: H, halogen and substituted or unsubstituted C1-C4 alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted C1-C6 alkyl, substituted or unsubstituted C1-C6 alkoxy, substituted or unsubstituted C2-C6 alkenyl, substituted or unsubstituted C2-C6 alkynyl, substituted or unsubstituted 3- to 8-membered cyclohydrocarbyl, substituted or unsubstituted 6- to 14-membered aryl, substituted or unsubstituted 5- to 10-membered heteroaryl, substituted or unsubstituted 4- to 9-membered monocyclic or fused heterocyclyl, substituted or unsubstituted 6- to 12-membered bridged cyclyl, substituted or unsubstituted 6- to 12-membered bridged heterocyclyl, substituted or unsubstituted 6- to 12-membered spiro cyclyl and substituted or unsubstituted 6- to 12-membered spiro heterocyclyl;
alternatively, R₄ or R₅ and R₆, together with the atom to which they are attached, form a 4- to 9-membered monocyclic or fused heterocyclyl, 6- to 12-membered bridged heterocyclyl or 6- to 12-membered spiro heterocyclyl ring, and the 4- to 9-membered monocyclic or fused heterocyclyl, 6- to 12-membered bridged heterocyclyl and 6- to 12-membered spiro heterocyclyl are optionally substituted by 1, 2 or 3 substituents selected from H, deuterium, hydroxyl, cyano, C1-C4 alkyl, halogen and C1-C4 alkoxy;
R₇ and R₈ are each independently selected from: H, halogen, cyano, substituted or unsubstituted C1-C4 alkyl, substituted or unsubstituted C1-C4 haloalkyl and substituted or unsubstituted C1-C4 alkoxy;
R₉ is selected from: H, C1-C4 alkyl, C1-C4 alkoxy, hydroxyl and halogen;
R₁₀ is selected from: H, halogen and C1-C4 alkyl;
R₁₁ is selected from: H, hydroxyl, amino, halogen, C1-C4 alkyl, -NR'R" and C1-C4 alkoxy;
R' and R" are each independently selected from H and C1-C4 alkyl;
wherein the dashed line indicates the presence or absence of a double bond.

100. The compound or the pharmaceutically acceptable salt, enantiomer, diastereomer, tautomer, solvate, isotopic substitute, polymorph, prodrug or metabolite thereof according to claim 99, wherein
R₁ is selected from: cyano, -C(O)NH₂, -C(O)NHCH₃ and 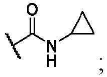
R₂ is selected from: H, fluorine and methoxy;
R₃ is phenyl;
R₄ and R₅ are each independently selected from: H and C1-C4 alkyl;
R₆ is H, substituted or unsubstituted C1-C6 alkyl, substituted or unsubstituted C1-C6 alkoxy, substituted or unsubstituted C2-C6 alkenyl or substituted or unsubstituted C2-C6 alkynyl, and their substituents are 1, 2 or 3 substituents selected from: hydroxyl, halogen, C1-C4 alkoxy optionally substituted by halogen, cyano, -S(O)₂-Cl-C4 alkyl optionally substituted by halogen, C1-C4 alkyl optionally substituted by halogen, -S(O)₂-NR'R", -S(O)₂-NH₂, -C(O)-C1-C4 alkyl, -NR'-C(O)-C1-C4 alkyl and 6- to 14-membered aryl, 5- to 10-membered heteroaryl, 3- to 8-membered cyclohydrocarbyl and 4- to 9-membered heterocyclyl, wherein the 6- to 14-membered aryl, 5- to 10-membered heteroaryl, 3- to 8-membered cyclohydrocarbyl and 4- to 9-membered heterocyclyl are optionally substituted by 1, 2 or 3 substituents selected from C1-C4 alkyl, C1-C4 haloalkyl, C1-C4 alkoxy, C1-C4 haloalkoxy, halogen, cyano, C1-C4 alkyl-S(O)₂-, hydroxyl, carboxyl, - NR'R", 6- to 14-membered aryl, 5- to 10-membered heteroaryl, 3- to 8-membered cyclohydrocarbyl and 4- to 9-membered heterocyclyl; wherein R' and R" are each independently selected from H and C1-C4 alkyl; or
R₆ is substituted or unsubstituted 3- to 8-membered cyclohydrocarbyl or substituted or unsubstituted 4- to 9-membered monocyclic or fused heterocyclyl, and their substituents are 1, 2 or 3 substituents selected from: halogen, hydroxyl, cyano, -NR'R", -S(O)₂-NR'R", -S(O)₂-NH₂, -NR'-C(O)-C1-C4 alkyl, -S(O)₂-Cl-C4 alkyl, C1-C4 alkyl, C1-C4 alkoxy, C1-C4 haloalkoxy, - C(O)-C1-C4 alkyl and C1-C4 alkoxycarbonyl, and the C1-C4 alkyl and C1-C4 alkoxy are optionally substituted by 1, 2 or 3 groups selected from halogen, hydroxyl and -NR'R", wherein the R' and R" are each independently selected from H and C1-C4 alkyl; or
R₆ is substituted or unsubstituted 6- to 12-membered bridged cyclyl, substituted or unsubstituted 6- to 12-membered bridged heterocyclyl, substituted or unsubstituted 6- to 12-membered spiro cyclyl or substituted or unsubstituted 6- to 12-membered spiro heterocyclyl, and their substituents are 1, 2 or 3 substituents selected from: C1-C4 alkyl, C1-C4 haloalkyl, C1-C4 alkoxy, C1-C4 haloalkoxy, cyano, -S(O)₂-Cl-C4 alkyl, hydroxyl, halogen, NR'R", -S(O)₂-NR'R", -S(O)₂-NH₂ and -NR'-C(O)-C1-C4 alkyl, wherein the R' and R" are each independently selected from H and C1-C4 alkyl;
alternatively, R₄ or R₅ and R₆, together with the atom to which they are attached, form the following group optionally substituted by 1, 2 or 3 substituents selected from H, deuterium, hydroxyl, methyl, F and methoxy: 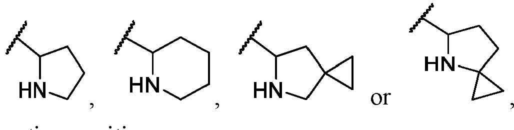 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl;
R₁₁ is selected from: H, hydroxyl, amino, fluorine, chlorine, methyl, methoxy and -NH(CH₃).

101. The compound or the pharmaceutically acceptable salt, enantiomer, diastereomer, tautomer, solvate, isotopic substitute, polymorph, prodrug or metabolite thereof according to any one of claims 1 to 100, wherein
ring A and ring B, together with their ring substituents, form any one of the following groups: 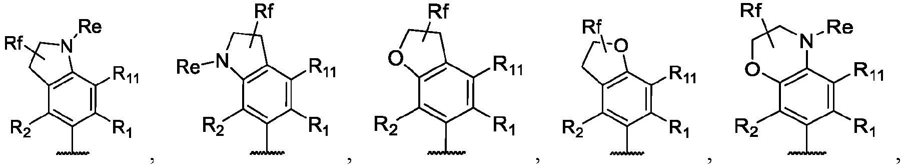 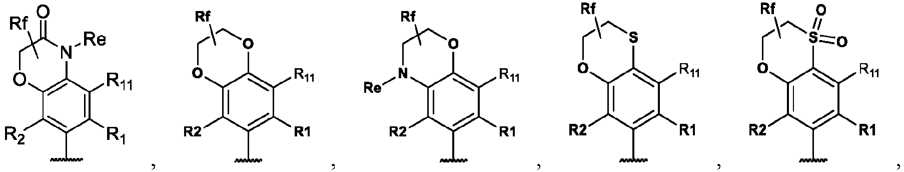 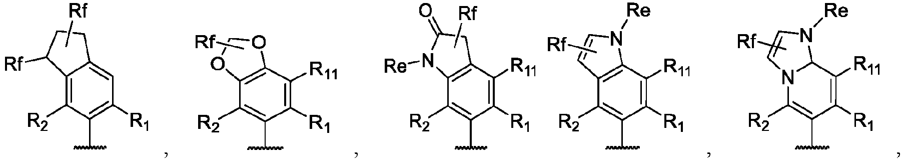 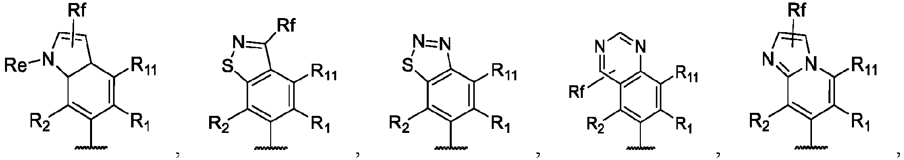 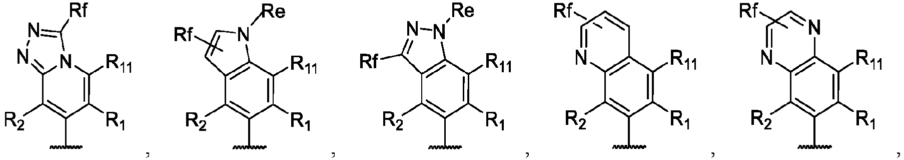 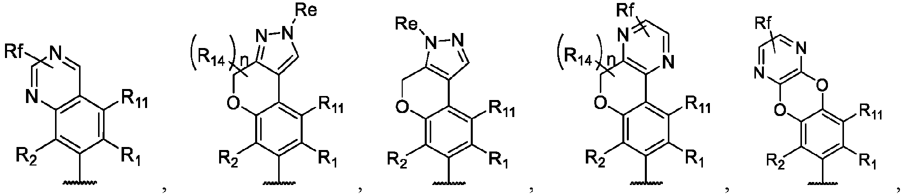 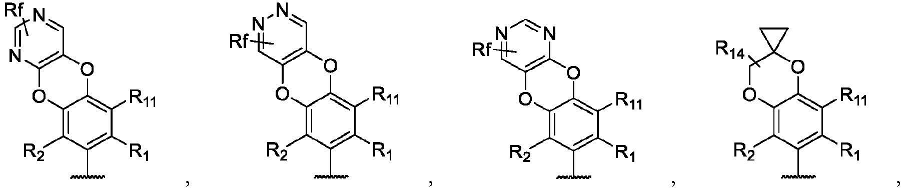 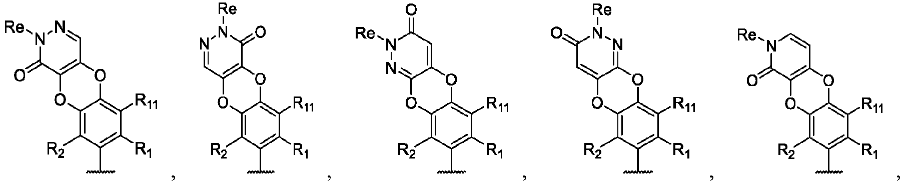 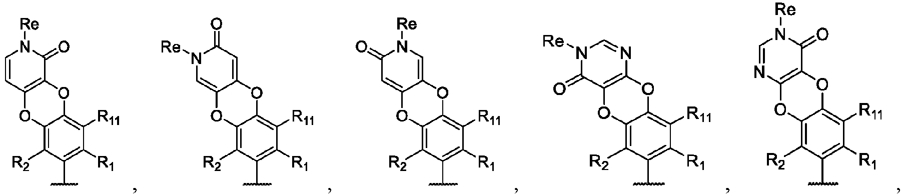 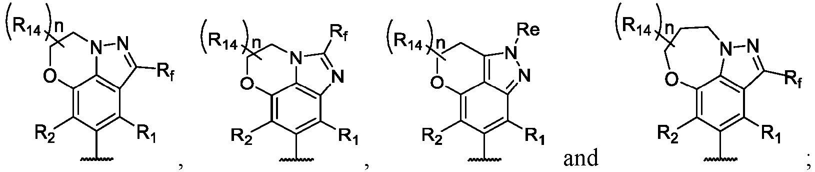
wherein R₁ is cyano or -C(O)NR'R"; R₂ is H or halogen; R₁₁ is H, hydroxyl, -NR'R", halogen or C1-C4 alkyl; R₁₄ is H, halogen or C1-C4 alkyl; n is 0, 1 or 2; R₁₆ is selected from H, hydroxyl, halogen and C1-C4 alkyl; Rₑ is selected from H, substituted or unsubstituted C1-C4 alkyl, substituted or unsubstituted C1-C4 alkoxy, substituted or unsubstituted 3- to 6-membered cyclohydrocarbyl, substituted or unsubstituted 3- to 6-membered cyclohydrocarbyl-C(O)- and substituted or unsubstituted C1-C4 acyl; R_{f} is selected from H, halogen, hydroxyl, cyano, -NR'R", substituted or unsubstituted C1-C4 alkoxy, substituted or unsubstituted C1-C4 alkyl and substituted or unsubstituted 3- to 6-membered cyclohydrocarbyl; preferably, the C1-C4 alkyl, C1-C4 alkoxy, 3- to 6-membered cyclohydrocarbyl, 3- to 6-membered cyclohydrocarbyl-C(O)- and C1-C4 acyl are optionally substituted by 1, 2 or 3 substituents selected from F, hydroxyl and cyano; wherein R' and R" are each independently selected from H, C1-C4 alkyl and C3-C6 cycloalkyl; the wavy line indicates the bond connection position;
X₁ is O;
R₃ is selected from unsubstituted phenyl or phenyl substituted by 1, 2 or 3 substituents selected from hydroxyl, halogen, C1-C4 alkyl, C1-C4 haloalkyl and C1-C4 alkoxy;
R₄ is selected from H and C1-C4 alkyl;
R₅ is selected from H and C1-C4 alkyl;
R₆ is selected from: H; C1-C4 alkyl, which is optionally substituted by 1, 2 or 3 substituents selected from hydroxyl, halogen, C1-C4 alkoxy, cyano and -S(O)₂-Cl-C4 alkyl; 3- to 8-membered cyclohydrocarbyl, which is optionally substituted by 1, 2 or 3 substituents selected from halogen, hydroxyl, cyano, -S(O)₂-Cl-C4 alkyl, C1-C4 alkyl and C1-C4 alkoxy; 4- to 9-membered monocyclic or fused heterocyclyl, which is optionally substituted by 1, 2 or 3 substituents selected from halogen, hydroxyl, cyano, -S(O)₂-Cl-C4 alkyl, C1-C4 alkyl and C1-C4 alkoxy; and 6- to 12-membered bridged cyclyl, 6- to 12-membered spiro cyclyl, 6- to 12-membered bridged heterocyclyl or 6- to 12-membered spiro heterocyclyl, each of which is optionally substituted by 1, 2 or 3 substituents selected from C1-C4 alkyl, C1-C4 alkoxy and cyano;
alternatively, R₄ or R₅ and R₆, together with the atom to which they are attached, form the following group optionally substituted by 1, 2 or 3 substituents selected from H, deuterium, hydroxyl, methyl, F and methoxy: 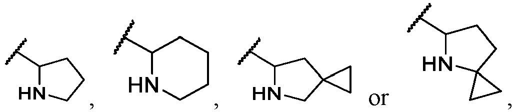 wherein the wavy line indicates the bond connection position;
R₇ and R₈ are each independently selected from: H, halogen, cyano, methyl and methoxy;
R₉ is selected from: H, methyl, methoxy, hydroxyl and F;
R₁₀ is selected from: H, F and methyl.

102. The compound or the pharmaceutically acceptable salt, enantiomer, diastereomer, tautomer, solvate, isotopic substitute, polymorph, prodrug or metabolite thereof according to any one of claims 1 to 101, wherein
ring A and ring B, together with their ring substituents, form any one of the following groups: 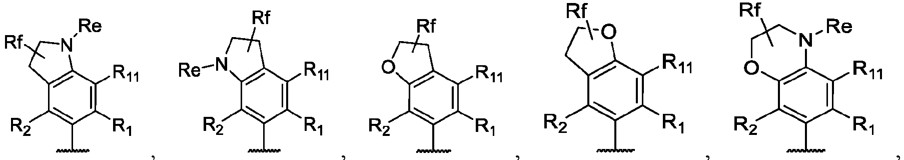 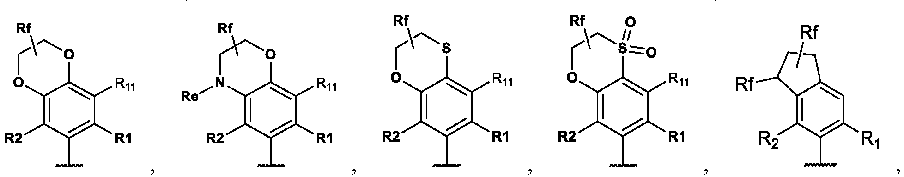 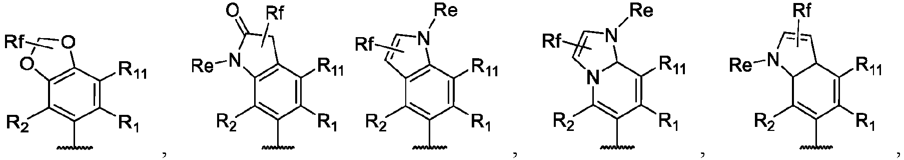 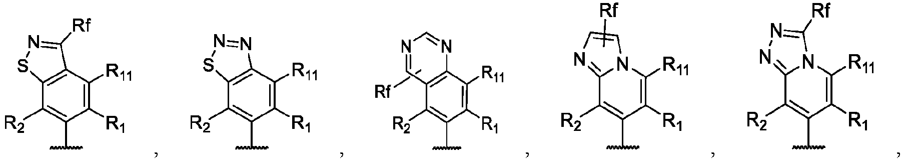 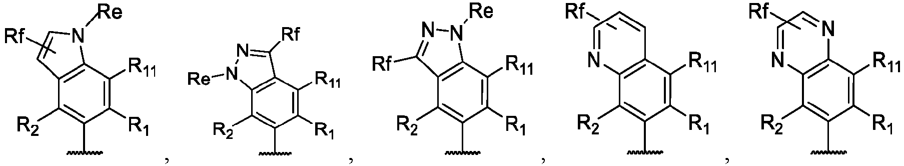
and 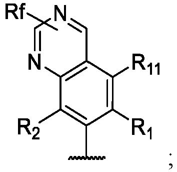
wherein R₁ is -C(O)NR'R"; R₂ is F; R₁₁ is H, F or methyl; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, - CH₂CH₂(CH₃)OH, -CH₂CH₂OCH₃ and acetyl; R_{f} is selected from H, F, Cl, hydroxyl, cyano, - NR'R", methoxy, ethoxy, -CH₂OH, -(CH₂)₂OH, -(CH₂)₂OCH₃, C1-C6 alkyl and 3- to 6-membered cyclohydrocarbyl, wherein R' and R" are each independently selected from H and methyl; the wavy line indicates the bond connection position.

103. The compound or the pharmaceutically acceptable salt, enantiomer, diastereomer, tautomer, solvate, isotopic substitute, polymorph, prodrug or metabolite thereof according to any one of claims 1 to 102, wherein
ring A and ring B, together with their ring substituents, form any one of the following groups: 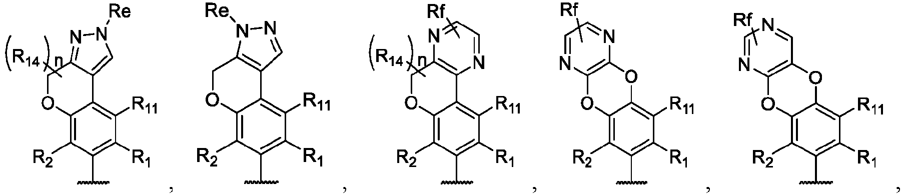 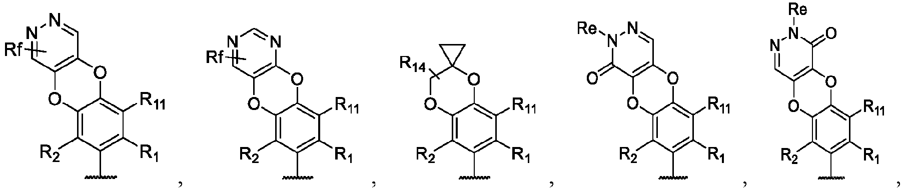 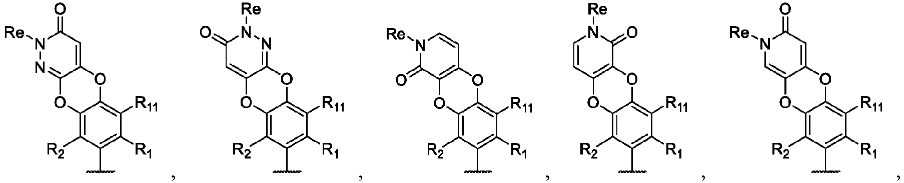 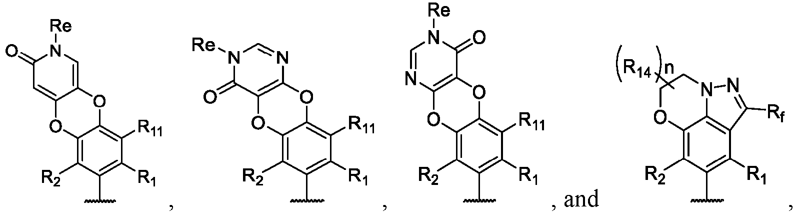
wherein R₁ is -C(O)NR'R"; R₂ is F; R₁₁ is H, F or methyl; R₁₄ is H, F or methyl; n is 0, 1 or 2; R₁₆ is selected from H, hydroxyl and methyl; Rₑ is selected from H, methyl, difluoromethyl, trifluoromethyl, ethyl, fluoroethyl, difluoroethyl, trifluoroethyl, isopropyl, cyclopropyl, cyclobutyl, trifluoropropyl, oxocyclobutyl, 1,1-difluorocyclobutyl, -CH₂CH₂OH, -CH₂CH₂(CH₃)OH, - CH₂CH₂OCH₃ and acetyl; R_{f} is selected from H, F, Cl, hydroxyl, cyano, -NR'R", methoxy, ethoxy, -CH₂OH, -(CH₂)₂OH, -(CH₂)₂OCH₃, C1-C6 alkyl and 3- to 6-membered cyclohydrocarbyl, wherein R' and R" are each independently selected from H, methyl and cyclopropyl; the wavy line indicates the bond connection position.

104. The compound or the pharmaceutically acceptable salt, enantiomer, diastereomer, tautomer, solvate, isotopic substitute, polymorph, prodrug or metabolite thereof according to any one of claims 1 to 103, wherein the compound is selected from the following compounds:

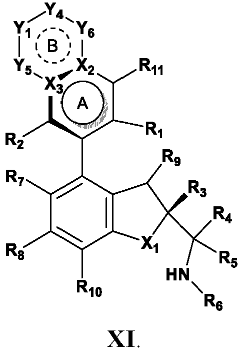

105. The compound or the pharmaceutically acceptable salt, enantiomer, diastereomer, tautomer, solvate, isotopic substitute, polymorph, prodrug or metabolite thereof according to any one of claims 1 to 104, wherein the compound is selected from the following compounds: 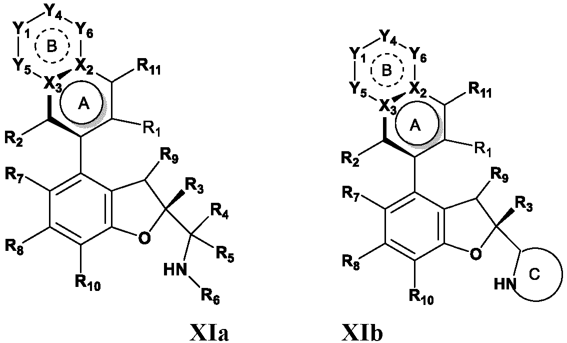 wherein ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

106. The compound or the pharmaceutically acceptable salt, enantiomer, diastereomer, tautomer, solvate, isotopic substitute, polymorph, prodrug or metabolite thereof according to any one of claims 1 to 105, wherein the compound is selected from the following compounds: 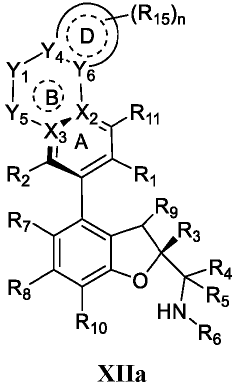 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

107. The compound or the pharmaceutically acceptable salt, enantiomer, diastereomer, tautomer, solvate, isotopic substitute, polymorph, prodrug or metabolite thereof according to any one of claims 1 to 106, wherein the compound is selected from the following compounds: 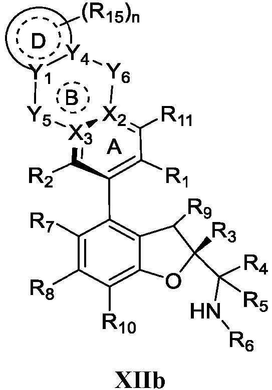 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2, 3 or 4.

108. The compound or the pharmaceutically acceptable salt, enantiomer, diastereomer, tautomer, solvate, isotopic substitute, polymorph, prodrug or metabolite thereof according to any one of claims 1 to 107, wherein the compound is selected from the following compounds: 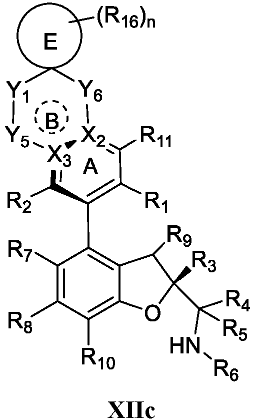 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

109. The compound or the pharmaceutically acceptable salt, enantiomer, diastereomer, tautomer, solvate, isotopic substitute, polymorph, prodrug or metabolite thereof according to any one of claims 1 to 108, wherein the compound is selected from the following compounds: 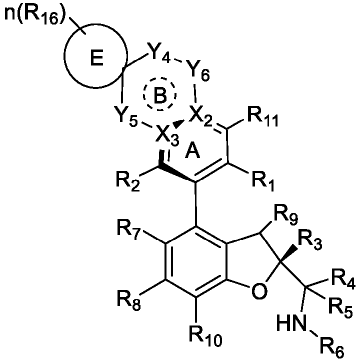 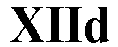 in the formula,
ring E is a 3- to 8-membered cyclohydrocarbyl or 4- to 9-membered heterocyclyl ring, wherein the 4- to 9-membered heterocyclyl contains 1, 2 or 3 heteroatoms selected from O, N and S;
R₁₆ is selected from H, halogen, substituted or unsubstituted C1-C4 alkyl, hydroxyl, substituted or unsubstituted C1-C4 acyl and substituted or unsubstituted C1-C4 alkoxy; preferably, the C1-C4 alkyl, C1-C4 acyl and C1-C4 alkoxy are optionally substituted by 1, 2 or 3 substituents selected from F, hydroxyl and cyano;
n is 0, 1, 2, 3 or 4.

110. The compound or the pharmaceutically acceptable salt, enantiomer, diastereomer, tautomer, solvate, isotopic substitute, polymorph, prodrug or metabolite thereof according to any one of claims 1 to 109, wherein the compound is selected from the following compounds: 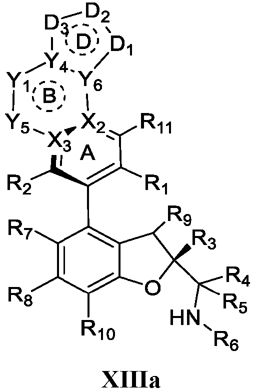 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRₑ, N-NRₑ, CR_{f}-NRₑ, 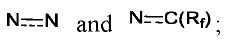
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each R_{c} at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2.

111. The compound or the pharmaceutically acceptable salt, enantiomer, diastereomer, tautomer, solvate, isotopic substitute, polymorph, prodrug or metabolite thereof according to any one of claims 1 to 110, wherein the compound is selected from the following compounds: 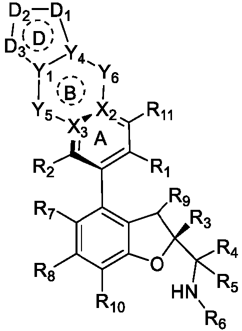 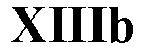 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRₑ, N-NRₑ, CR_{f}-NRₑ, N-̅ -̅ -̅N and N-̅ -̅C(R_{f);}
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2.

112. The compound or the pharmaceutically acceptable salt, enantiomer, diastereomer, tautomer, solvate, isotopic substitute, polymorph, prodrug or metabolite thereof according to any one of claims 1 to 111, wherein the compound is selected from the following compounds: 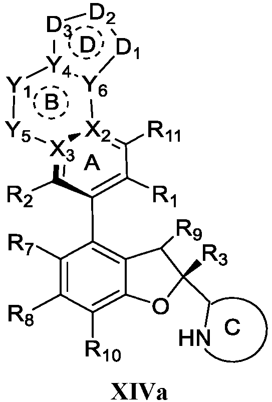 in the formula,
Y₄ is selected from: C, CH and N;
Y₆ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NRₑ, CR_{f} and CR_{f}R_{g};
D₂ is selected from: O, S, S(O), S(O)₂, N, NRₑ, (CR_{f})ₚ, (CR_{f}R_{g})ₚ, C(O)-NRₑ, N-NRₑ, CR_{f}-NRₑ, N-̅ -̅ -̅N and N-̅ -̅C(R_{f});
D₃ is selected from: O, S, S(O), S(O)₂, N, NRₑ, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

113. The compound or the pharmaceutically acceptable salt, enantiomer, diastereomer, tautomer, solvate, isotopic substitute, polymorph, prodrug or metabolite thereof according to any one of claims 1 to 112, wherein the compound is selected from the following compounds: 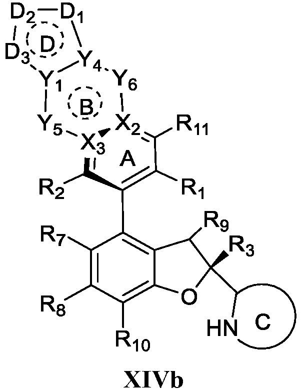 in the formula,
Y₁ is selected from: C, CH and N;
Y₄ is selected from: C, CH and N;
ring D is a 3- to 8-membered cyclohydrocarbyl, 4- to 9-membered heterocyclyl, 6- to 10-membered aryl or 5- to 10-membered heteroaryl ring, wherein the 4- to 9-membered heterocyclyl and 5- to 10-membered heteroaryl contain 1, 2 or 3 heteroatoms selected from O, N and S;
D₁ is selected from: O, S, S(O), S(O)₂, N, NR_{c}, CR_{f} and CR_{f}R_{g};
D₂ is selected from: O, S, S(O), S(O)₂, N, NR_{c}, (CR_{f})ₚ, (CR_{f}R_{g})ₚ, C(O)-NRₑ, N-NR_{c}, CR_{f}-NR_{c}, 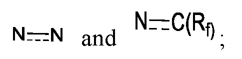
D₃ is selected from: O, S, S(O), S(O)₂, N, NR_{c}, (CR_{f})ₚ and (CR_{f}R_{g})ₚ;
provided that D₁ and D₂, D₁ and D₃ are not simultaneously O, S, -S(O)- or -S(O)₂- and satisfy the valence bond theory;
each R_{c} at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
each p is independently 1, 2, 3 or 4, preferably 1 or 2;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

114. The compound or the pharmaceutically acceptable salt, enantiomer, diastereomer, tautomer, solvate, isotopic substitute, polymorph, prodrug or metabolite thereof according to any one of claims 1 to 113, wherein the compound is selected from the following compounds: 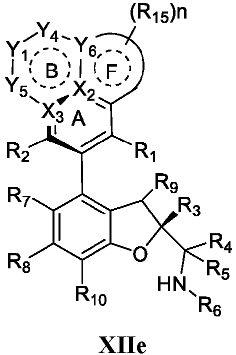 in the formula,
X₂ is C;
Y₆ is selected from: C, CH and N;
ring F is a 5- or 6-membered cyclohydrocarbyl, 5- or 6-membered heterocyclyl, phenyl or 5- or 6-membered heteroaryl ring, wherein the 5- or 6-membered heterocyclyl or 5- or 6-membered heteroaryl contains 1, 2 or 3 heteroatoms selected from O, N and S;
R₁₅ is selected from: H, halogen, oxo (=O), thio (=S), substituted or unsubstituted C1-C4 acyl, hydroxyl, substituted or unsubstituted C1-C4 alkoxy, cyano, -NR'R", substituted or unsubstituted C1-C4 alkyl-S(O)₂-, substituted or unsubstituted 6- to 10-membered aryl-S(O)₂-, substituted or unsubstituted 4- to 9-membered heterocyclyl, NR'R"-C(O)-, substituted or unsubstituted 3- to 8-membered cyclohydrocarbyl, substituted or unsubstituted 3- to 8-membered cyclohydrocarbyl-C(O)-, substituted or unsubstituted C1-C4 haloalkyl and substituted or unsubstituted C1-C4 alkyl; preferably, the C1-C4 acyl, C1-C4 alkoxy, C1-C4 alkyl-S(O)₂-, 6- to 10-membered aryl-S(O)₂-, 4- to 9-membered heterocyclyl, 3- to 8-membered cyclohydrocarbyl, 3- to 8-membered cyclohydrocarbyl-C(O)-, C1-C4 haloalkyl and C1-C4 alkyl are optionally substituted by 1, 2 or 3 substituents selected from F, hydroxyl and cyano;
n is 0, 1, 2 or 3.

115. The compound or the pharmaceutically acceptable salt, enantiomer, diastereomer, tautomer, solvate, isotopic substitute, polymorph, prodrug or metabolite thereof according to any one of claims 1 to 114, wherein the compound is selected from the following compounds: 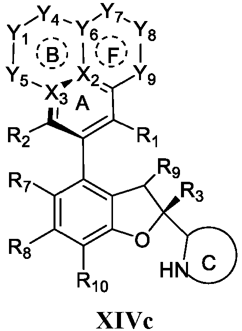 in the formula,
X₂ is C;
Y₆ is selected from: C, CH and N;
ring F is a 5- or 6-membered cyclohydrocarbyl, 5- or 6-membered heterocyclyl, phenyl or 5- or 6-membered heteroaryl ring, wherein the 5- or 6-membered heterocyclyl or 5- or 6-membered heteroaryl contains 1, 2 or 3 heteroatoms selected from O, N and S;
Y₇, Y₈ and Y₉ are independently selected from: a bond, O, S, S(O), S(O)₂, N, NRₑ, CR_{f} and CR_{f}R_{g}, provided that at most one of Y₇, Y₈ and Y₉ is a bond, Y₇ and Y₈, Y₈ and Y₉ are not simultaneously O, S, -S(O)- or -S(O)₂- and satisfy the valence bond theory,
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl, wherein R' and R" are each independently selected from H and C1-C4 alkyl; preferably, the C1-C4 acyl, C1-C4 alkoxy, C1-C4 alkyl-S(O)₂-, 4- to 9-membered heterocyclyl, 3- to 6-membered cyclohydrocarbyl, 3- to 6-membered cyclohydrocarbyl-C(O)-, C1-C4 haloalkyl and C1-C4 alkyl are optionally substituted by 1, 2 or 3 substituents selected from F, hydroxyl and cyano;
each R_{f} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
each R_{g} at each occurrence is independently selected from: H, halogen, substituted or unsubstituted C1-C4 acyl, hydroxyl, cyano, -NR'R", substituted or unsubstituted C1-C4 alkoxy, substituted or unsubstituted C1-C4 alkyl, substituted or unsubstituted 3- to 6-membered cyclohydrocarbyl, substituted or unsubstituted C1-C4 alkyl-S(O)₂-, substituted or unsubstituted 3-to 6-membered cyclohydrocarbyl-C(O)- and NR'R"-C(O)-, wherein R' and R" are each independently selected from H and C1-C4 alkyl; preferably, the C1-C4 acyl, C1-C4 alkoxy, C1-C4 alkyl, 3- to 6-membered cyclohydrocarbyl, C1-C4 alkyl-S(O)₂- and 3- to 6-membered cyclohydrocarbyl-C(O)- are optionally substituted by 1, 2 or 3 substituents selected from F, hydroxyl and cyano;
alternatively, R_{f} and R_{g} on the same carbon atom form an oxo (=O) or thio (=S) group;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring.

116. The compound or the pharmaceutically acceptable salt, enantiomer, diastereomer, tautomer, solvate, isotopic substitute, polymorph, prodrug or metabolite thereof according to any one of claims 1 to 115, wherein the compound has a structure shown in the following formula XVa: 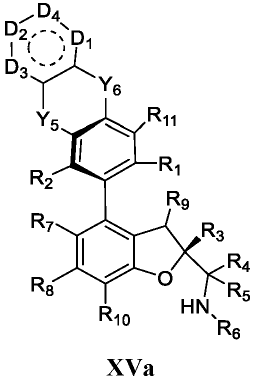 in the formula,
D₁ is selected from: O, S, S(O), S(O)₂, N, NRₑ, CR_{f}R_{g}, CR_{f} and C(=O);
D₂ is selected from: O, S, S(O), S(O)₂, N, NRₑ, CR_{f}R_{g}, CR_{f} and C(=O);
D₃ is selected from: O, S, S(O), S(O)₂, N, NRₑ, CR_{f}R_{g}, CR_{f} and C(=O);
D₄ is selected from: O, S, S(O), S(O)₂, N, NRₑ, CR_{f}R_{g}, CR_{f} and C(=O); and wherein the ring in which D₁, D₂, D₃ and D₄ are located satisfies valence bond theory and contains at most two C(=O) and three N atoms;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl; each R_{f} at each occurrence is independently selected from: H, halogen, hydroxyl, cyano, oxo, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₁ is selected from: cyano and -C(O)NR'R";
R₂ is selected from: H, halogen, substituted or unsubstituted alkyl and substituted or unsubstituted alkoxy;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted C1-C4 alkyl;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 6-membered heterocyclyl ring; the 4- to 6-membered heterocyclyl contains 1 or 2 heteroatoms selected from N and O;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
each p is independently 1, 2, 3 or 4.

117. The compound or the pharmaceutically acceptable salt, enantiomer, diastereomer, tautomer, solvate, isotopic substitute, polymorph, prodrug or metabolite thereof according to any one of claims 1 to 116, wherein the compound has a structure shown in the following formula XVb: 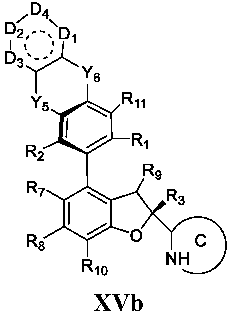 in the formula,
D₁ is selected from: O, S, S(O), S(O)₂, N, NR_{c}, CR_{f}R_{g}, CR_{f} and C(=O);
D₂ is selected from: O, S, S(O), S(O)₂, N, NRₑ, CR_{f}R_{g}, CR_{f} and C(=O);
D₃ is selected from: O, S, S(O), S(O)₂, N, NRₑ, CR_{f}R_{g}, CR_{f} and C(=O);
D₄ is selected from: O, S, S(O), S(O)₂, N, NRₑ, CR_{f}R_{g}, CR_{f} and C(=O); and wherein the ring in which D₁, D₂, D₃ and D₄ are located satisfies valence bond theory and contains at most two C(=O) and three N atoms;
each Rₑ at each occurrence is independently selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl; each R_{f} at each occurrence is independently selected from: H, halogen, hydroxyl, cyano, oxo, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₂ is selected from: H, halogen, substituted or unsubstituted alkyl and substituted or unsubstituted alkoxy;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
ring C is a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring, and the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O; ring C is preferably a substituted or unsubstituted 4- to 8-membered nitrogen-containing monocyclic heterocyclyl, substituted or unsubstituted 6- to 12-membered nitrogen-containing spiro heterocyclyl or substituted or unsubstituted 6- to 12-membered nitrogen-containing fused heterocyclyl ring;
each p is 0, 1, 2, 3 or 4.

118. The compound or the pharmaceutically acceptable salt, enantiomer, diastereomer, tautomer, solvate, isotopic substitute, polymorph, prodrug or metabolite thereof according to any one of claims 1 to 117, wherein the compound is selected from the following compounds: 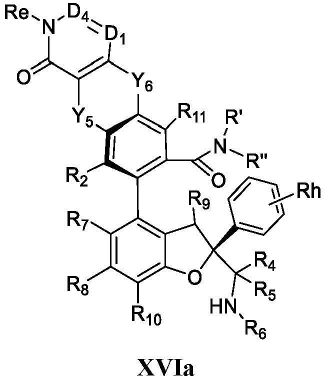 in the formula,
D₁ is selected from: N and CR_{f};
D₄ is selected from: N and CR_{f}, and D₁ and D₄ are not simultaneously N;
Rₑ is selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl;
each R_{f} at each occurrence is independently selected from: H, halogen, hydroxyl, cyano, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₂ is selected from: H, halogen, substituted or unsubstituted alkyl and substituted or unsubstituted alkoxy;
R₄ and R₅ are each independently selected from: H, halogen and substituted or unsubstituted C1-C4 alkyl;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 6-membered heterocyclyl ring; the 4- to 6-membered heterocyclyl contains 1 or 2 heteroatoms selected from N and O;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
Rₕ is selected from: H, halogen, substituted or unsubstituted C1-C4 alkyl and substituted or unsubstituted C1-C4 alkoxy.

119. The compound or the pharmaceutically acceptable salt, enantiomer, diastereomer, tautomer, solvate, isotopic substitute, polymorph, prodrug or metabolite thereof according to any one of claims 1 to 117, wherein the compound has a structure shown in the following formula XVIb: 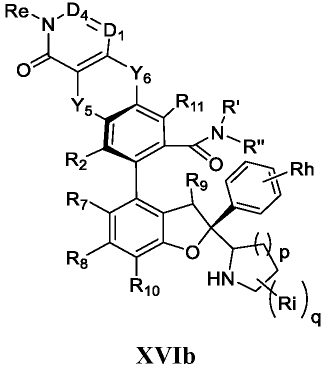 in the formula,
D₁ is selected from: N and CR_{f};
D₄ is selected from: N and CR_{f}, and D₁ and D₄ are not simultaneously N;
Rₑ is selected from: H, substituted or unsubstituted C1-C4 acyl, substituted or unsubstituted C1-C4 alkoxy, cyano, substituted or unsubstituted C1-C4 alkyl-S(O)₂-, substituted or unsubstituted 4- to 9-membered heterocyclyl, NR'R"-C(O)-, substituted or unsubstituted 3- to 6-membered cyclohydrocarbyl, substituted or unsubstituted 3- to 6-membered cyclohydrocarbyl-C(O)-, substituted or unsubstituted C1-C4 haloalkyl and substituted or unsubstituted C1-C4 alkyl; each R_{f} at each occurrence is independently selected from: H, halogen, hydroxyl, cyano, substituted or unsubstituted C1-C4 alkoxy, substituted or unsubstituted C1-C4 alkyl; preferably, the C1-C4 alkoxy and C1-C4 alkyl are optionally substituted by 1, 2 or 3 substituents selected from F, hydroxyl and cyano;
Y₅ and Y₆ are independently selected from: a bond, O, S, N, -S(O)-, -S(O)₂-, C, CH(R₁₃), C(R₁₃)₂ and N(R₁₂), and Y₅ and Y₆ are not simultaneously a bond;
R₂ is selected from: H, halogen, substituted or unsubstituted alkyl and substituted or unsubstituted alkoxy;
R₇ is selected from: H, halogen, cyano, nitro, substituted or unsubstituted C1-C4 alkyl and substituted or unsubstituted C1-C4 alkoxy;
R₈ is selected from: H, halogen, cyano, nitro, substituted or unsubstituted C1-C4 alkyl and substituted or unsubstituted C1-C4 alkoxy;
R₉ is selected from: H, substituted or unsubstituted C1-C4 alkyl, substituted or unsubstituted C1-C4 alkoxy, hydroxyl and halogen;
R₁₀ is selected from: H, halogen and substituted or unsubstituted C1-C4 alkyl;
R₁₁ is selected from: H, hydroxyl, amino, halogen, substituted or unsubstituted C1-C4 alkyl, -NR'R" and substituted or unsubstituted C1-C4 alkoxy;
R' and R" are each independently selected from H, C1-C4 alkyl, C3-C6 cycloalkyl, C1-C4 alkyl-S(O)₂- and C1-C4 haloalkyl;
Rₕ is selected from: H, halogen, substituted or unsubstituted C1-C4 alkyl and substituted or unsubstituted C1-C4 alkoxy;
Rᵢ is selected from: H, halogen, substituted or unsubstituted C1-C4 alkyl and substituted or unsubstituted C1-C4 alkoxy;
p is 1, 2 or 3;
q is 0, 1, 2, 3 or 4.

120. The compound or the pharmaceutically acceptable salt, enantiomer, diastereomer, tautomer, solvate, isotopic substitute, polymorph, prodrug or metabolite thereof according to claim 1, wherein the compound is selected from the following compounds:

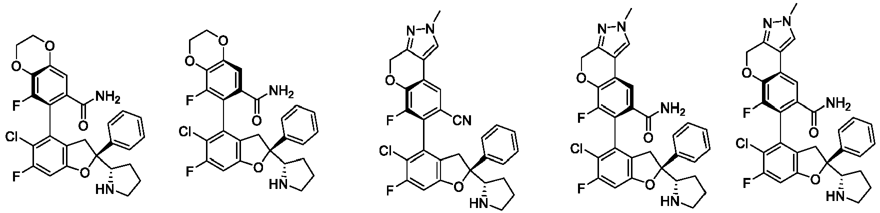

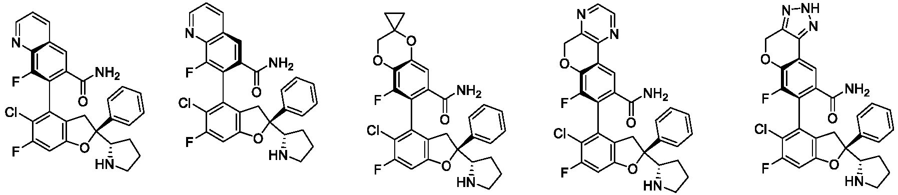

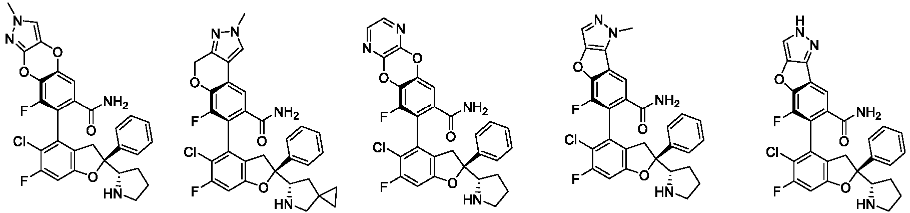

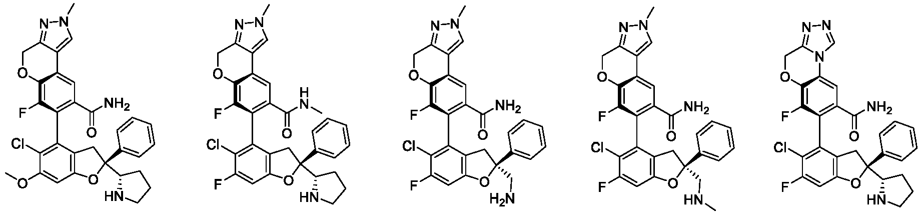

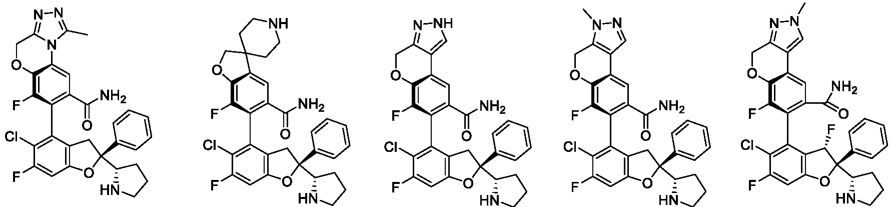

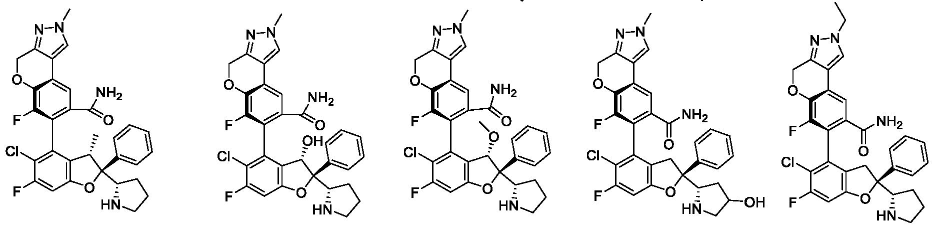

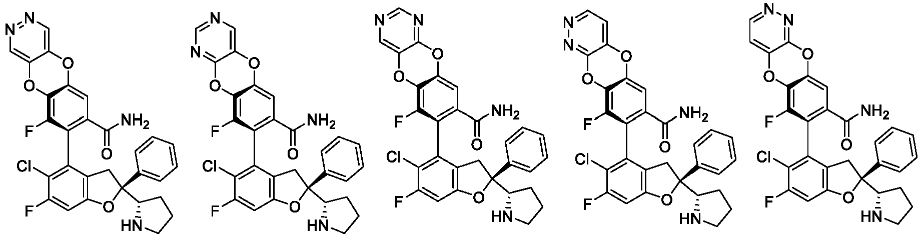

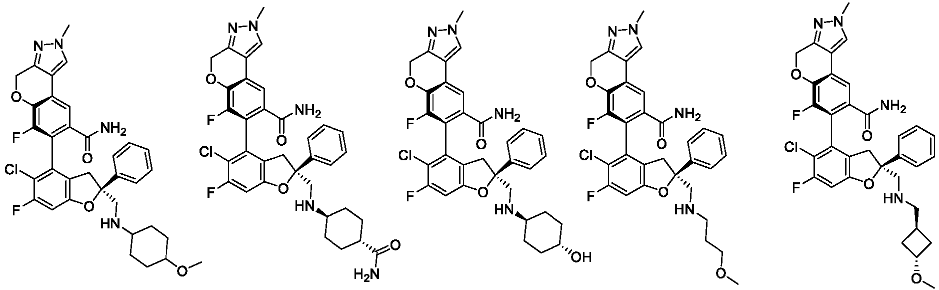

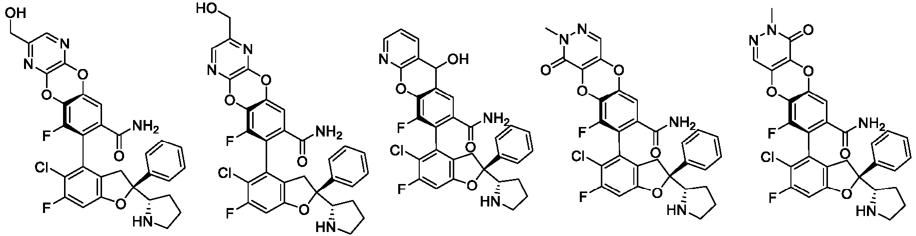

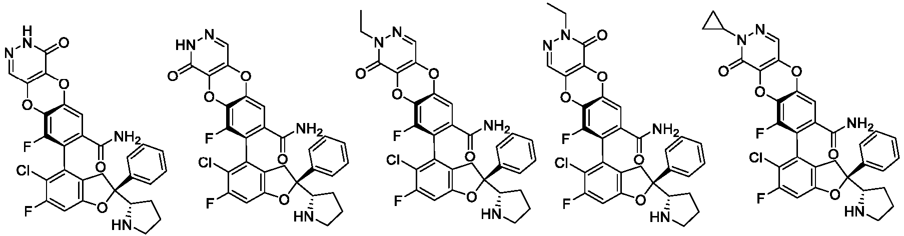

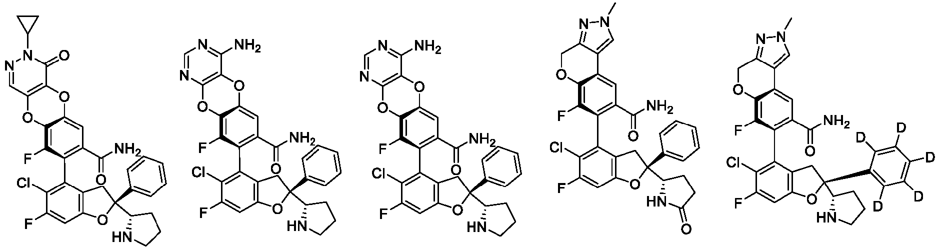

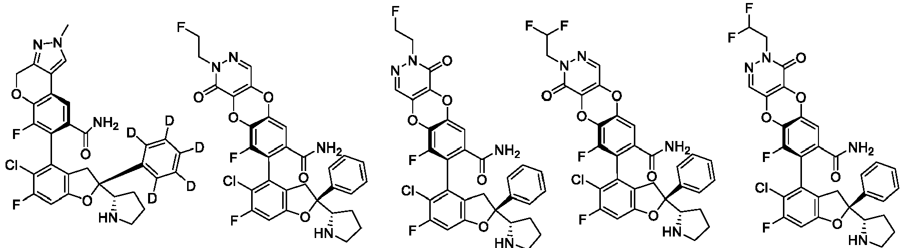

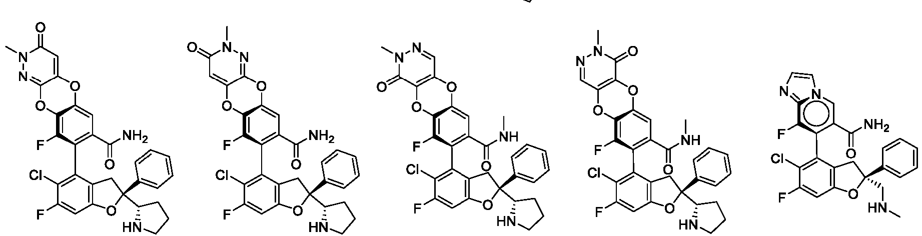

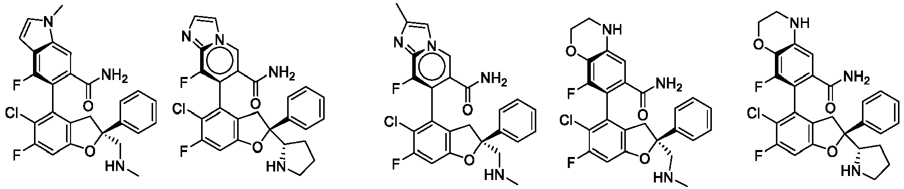

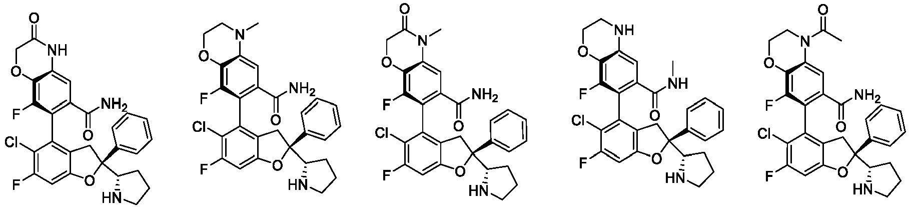

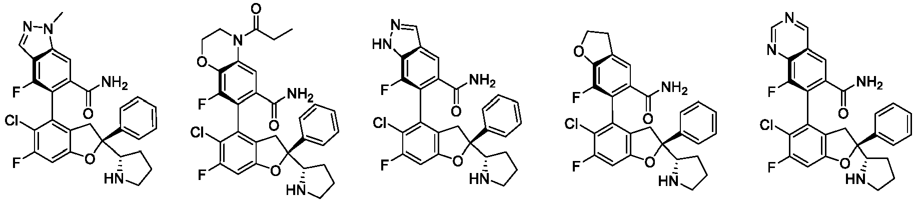

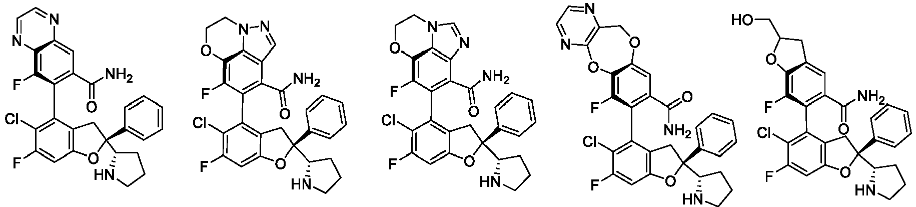

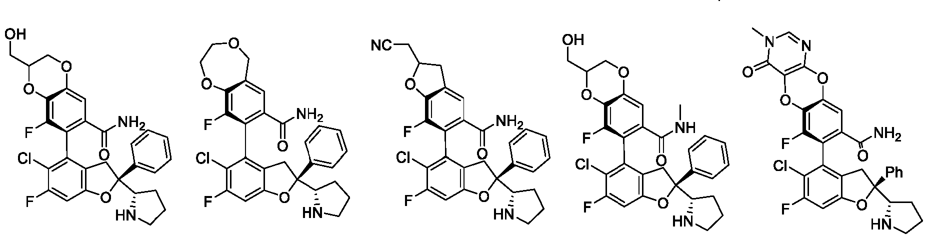

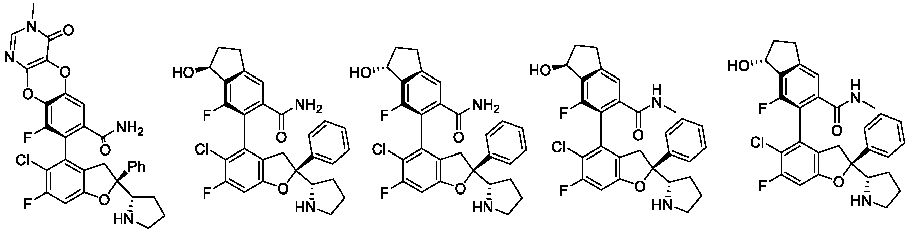

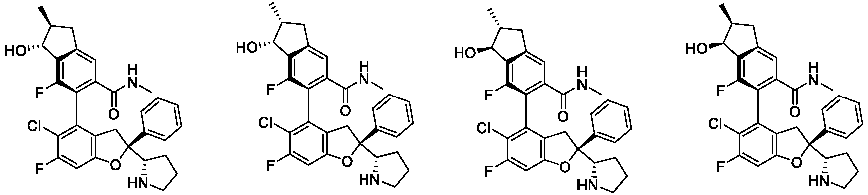

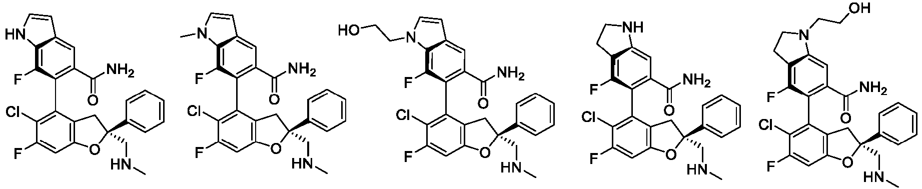

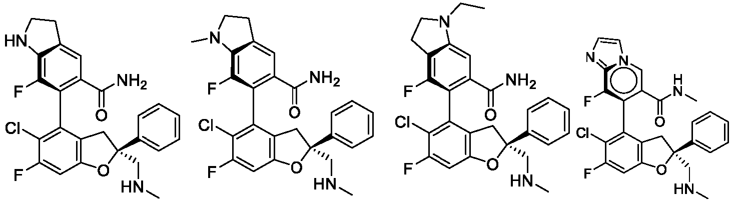

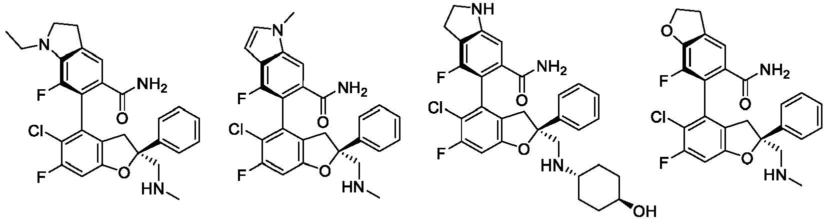

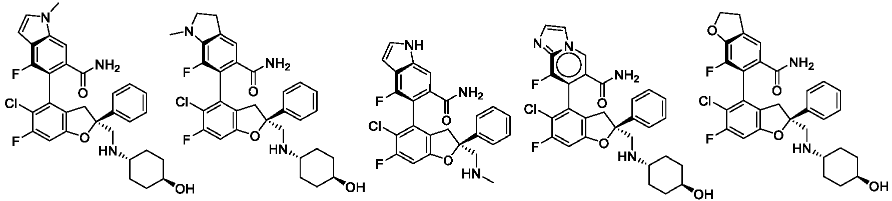

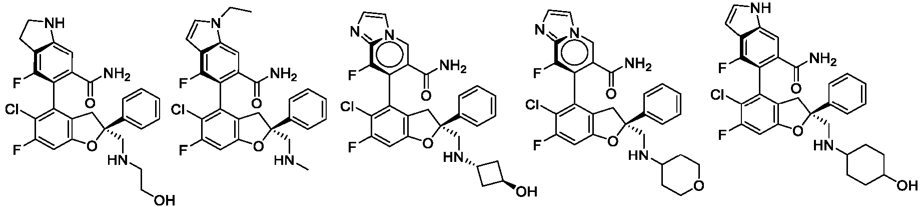

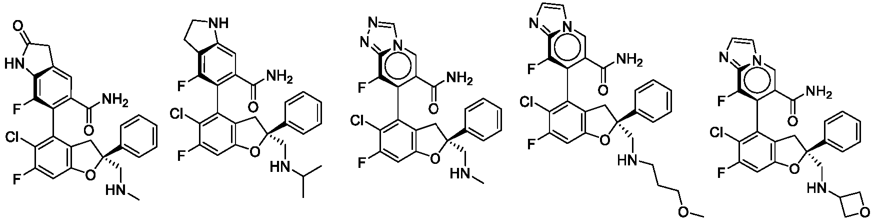

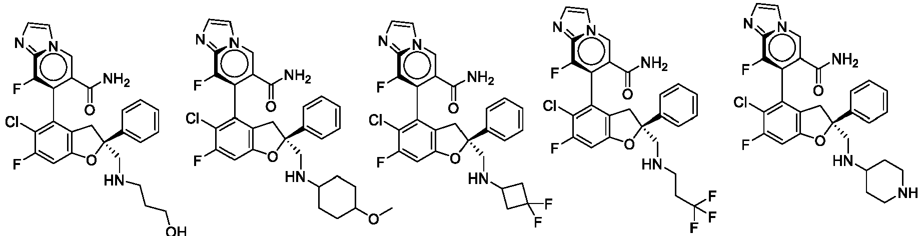

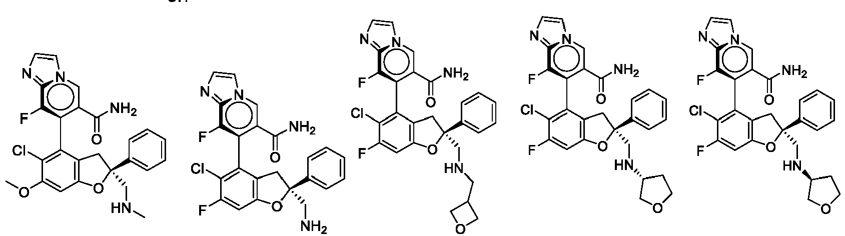

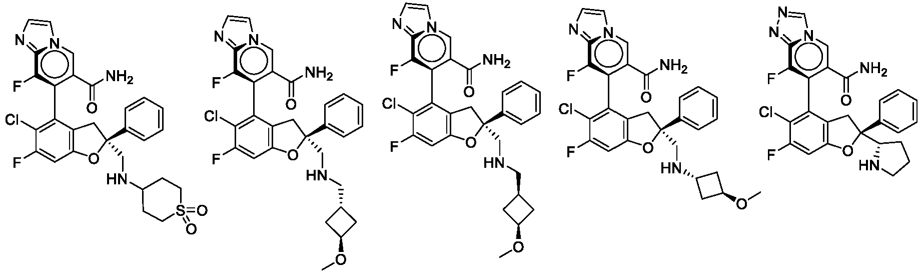

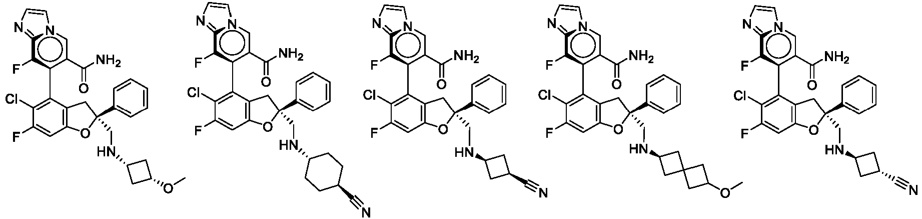

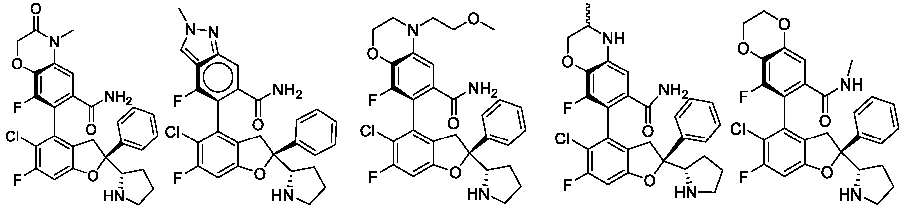

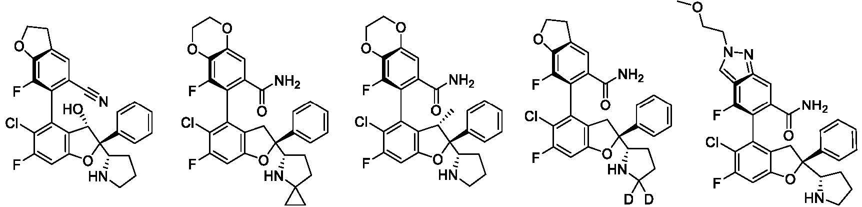

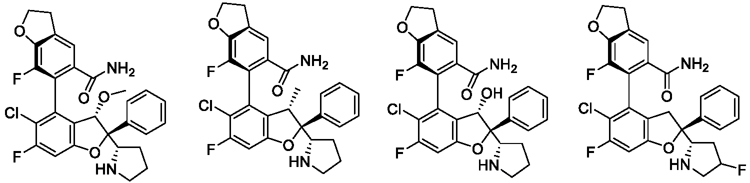

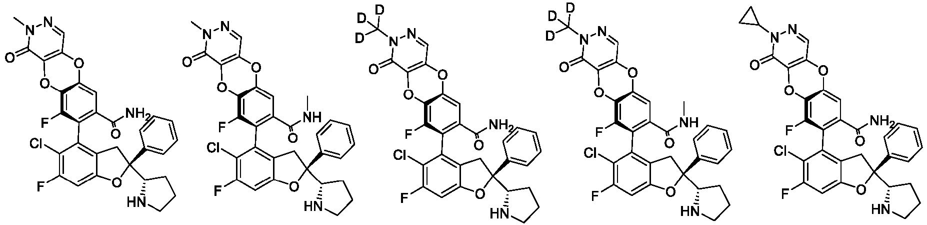

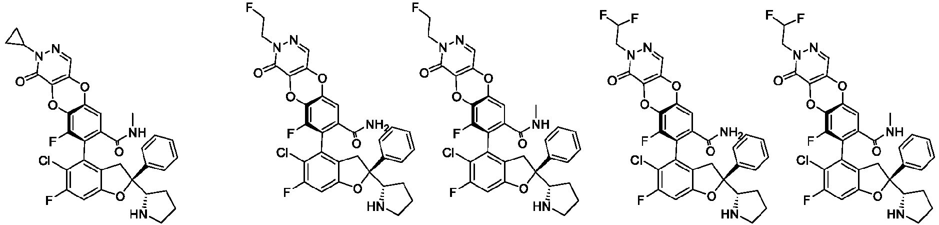

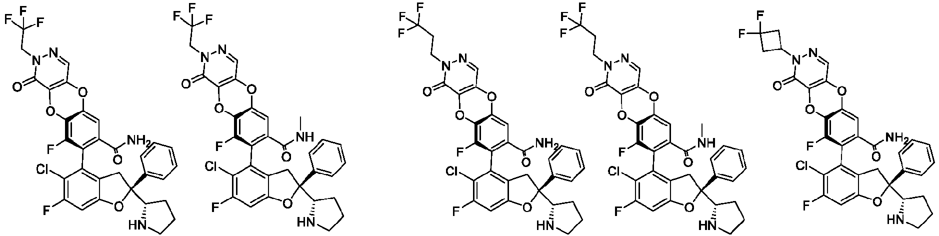

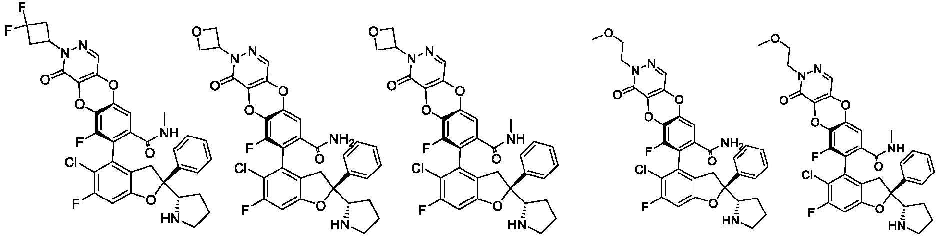

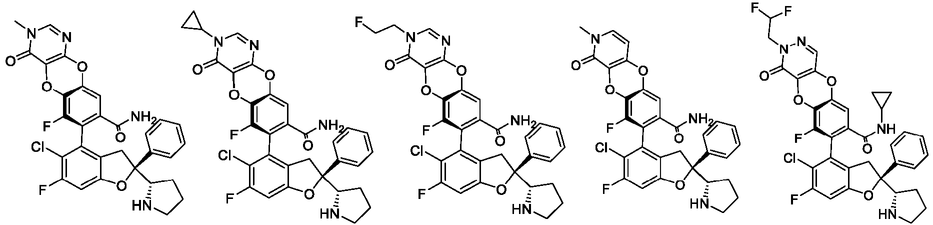

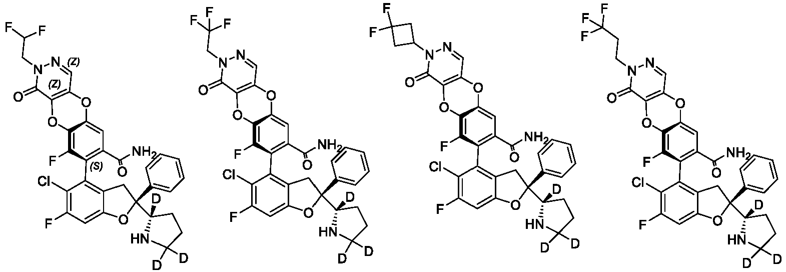

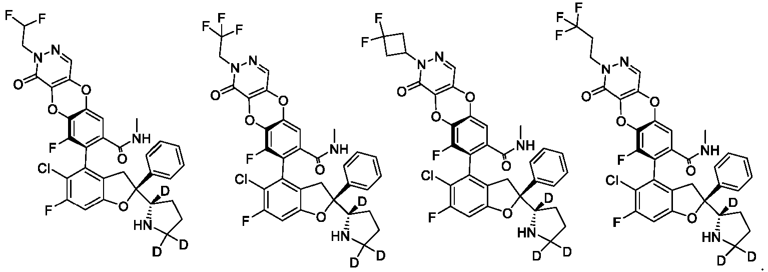

121. A pharmaceutical composition, wherein the pharmaceutical composition comprises the compound or the pharmaceutically acceptable salt, enantiomer, diastereomer, tautomer, solvate, isotopic substitute, polymorph, prodrug or metabolite thereof according to any one of claims 1 to 120 and a pharmaceutically acceptable carrier or excipient.

122. The pharmaceutical composition according to claim 121, wherein the pharmaceutical composition further comprises other known anticancer agents, including but not limited to chemotherapeutic agents, such as mitotic inhibitors, such as taxane compounds (e.g., paclitaxel or docetaxel), vinca alkaloids (e.g., vincristine, vinblastine, vinorelbine or vinflunine), other anticancer agents such as metal platinum complexes (e.g., cisplatin, carboplatin or oxaliplatin), antimetabolites (e.g., pyrimidine antagonists, such as 5-fluorouracil, 5-fluoro-2-4(1*H*,3*H*)-pyrimidinedione (5FU), gemcitabine), hormonal antitumor drugs (e.g., flutamide), and tumor immunotherapy drugs (such as anti-PD1 antibody).

123. A use of the compound or the pharmaceutically acceptable salt, enantiomer, diastereomer, tautomer, solvate, isotopic substitute, polymorph, prodrug or metabolite thereof according to any one of claims 1 to 120, or the pharmaceutical composition according to claim 117 or 118 in the manufacture of a medicament for treating or preventing diseases mediated by interaction between YAP/TAZ and TEAD; preferably, the diseases mediated by the interaction between YAP/TAZ and TEAD are tumors, liver fibrosis and renal fibrosis; preferably, the tumors comprise benign tumors and malignant tumors, especially cancers or sarcomas, such as solid tumors or hematological tumors; more preferably, the diseases mediated by the interaction between YAP/TAZ and TEAD are: mesothelioma (e.g., pleural mesothelioma (such as malignant pleural mesothelioma), peritoneal mesothelioma, pericardial mesothelioma or tunica vaginalis mesothelioma), cervical squamous cell carcinoma, endometrial carcinoma, bladder urothelial carcinoma, skin squamous cell carcinoma, poroma (e.g., benign poroma), porocarcinoma, epithelioid hemangioendothelioma, breast cancer (e.g., triple-negative breast cancer), lung cancer (e.g., non-small cell lung cancer), ovarian cancer, colorectal cancer, melanoma, pancreatic cancer (e.g., pancreatic ductal adenocarcinoma), prostate cancer, gastric cancer, esophageal cancer (e.g., esophageal squamous cell carcinoma or esophageal adenocarcinoma), liver cancer (e.g., hepatocellular carcinoma or hepatoblastoma), cholangiocarcinoma, neurilemmoma, renal cancer, sarcoma (e.g., rhabdomyosarcoma, embryonal rhabdomyosarcoma, osteosarcoma, undifferentiated pleomorphic sarcoma, Kaposi's sarcoma and soft tissue sarcoma (such as rare soft tissue sarcoma)), bone cancer, brain cancer (e.g., ependymoma (e.g., supratentorial ependymoma, such as pediatric supratentorial ependymoma), neuroblastoma, medulloblastoma, glioma or meningioma) or head and neck cancer (e.g., head and neck squamous cell carcinoma).

124. A compound of formula Ya, 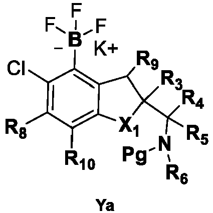 in the formula,
X₁ is selected from: O and NRₐ;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl.

125. A preparation method for a compound of formula Ya, comprising the following steps: 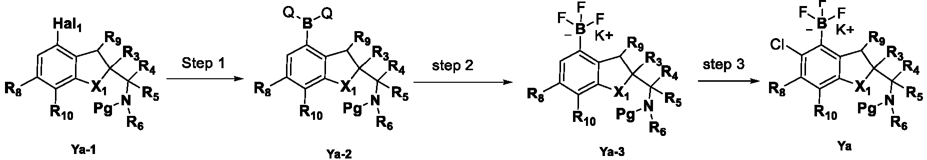
(i) coupling a borate ester under alkaline conditions with palladium catalysis, converting halogen Hal₁ in formula Ya-1 into Q-B-Q to obtain Ya-2;
(ii) converting Ya-2 into the potassium trifluoroborate salt of Ya-3 in an organic solvent at room temperature;
(iii) using a chlorinating reagent to convert Ya-3 into Ya under acid catalysis in an organic solvent;
in the formula,
X₁ is selected from: O and NRₐ; Rₐ is selected from H and C1-C4 alkyl;
R₃ is selected from: substituted or unsubstituted aryl, substituted or unsubstituted heteroaryl and substituted or unsubstituted heterocyclyl;
R₄ and R₅ are each independently selected from: H, halogen and substituted or unsubstituted alkyl; alternatively, R₄ and R₅, together with the carbon atom to which they are attached, form a 3- to 6-membered substituted or unsubstituted ring, and the ring optionally contains 1, 2 or 3 heteroatoms selected from O, N and S;
R₆ is selected from: H, substituted or unsubstituted alkyl, substituted or unsubstituted alkoxy, substituted or unsubstituted alkenyl, substituted or unsubstituted alkynyl, substituted or unsubstituted cyclohydrocarbyl, substituted or unsubstituted aryl, substituted or unsubstituted heteroaryl and substituted or unsubstituted heterocyclyl;
alternatively, R₄ or R₅ and R₆, together with the atom to which they are attached, form a substituted or unsubstituted 4- to 12-membered monocyclic or fused heterocyclyl, substituted or unsubstituted 6- to 12-membered bridged heterocyclyl or substituted or unsubstituted 6- to 12-membered spiro heterocyclyl ring; the 4- to 12-membered monocyclic or fused heterocyclyl, 6- to 12-membered bridged heterocyclyl and 6- to 12-membered spiro heterocyclyl each optionally further contain 1 or 2 heteroatoms selected from N and O;
R₈ is selected from: H, halogen, cyano, nitro, substituted or unsubstituted alkyl, substituted or unsubstituted haloalkyl, substituted or unsubstituted alkoxy, substituted or unsubstituted alkenyl and substituted or unsubstituted alkynyl;
R₉ is selected from: H, substituted or unsubstituted alkyl, substituted or unsubstituted alkoxy, hydroxyl and halogen;
R₁₀ is selected from: H, halogen and substituted or unsubstituted alkyl;
Hal₁ is halogen;
Q-B-Q is boric acid or borate ester;
Pg is a nitrogen protecting group.

126. A compound of formula Za, 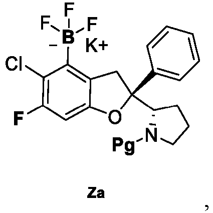 in the formula, Pg is a nitrogen protecting group, preferably Boc.

127. A preparation method for a compound of formula Za, comprising the following steps: 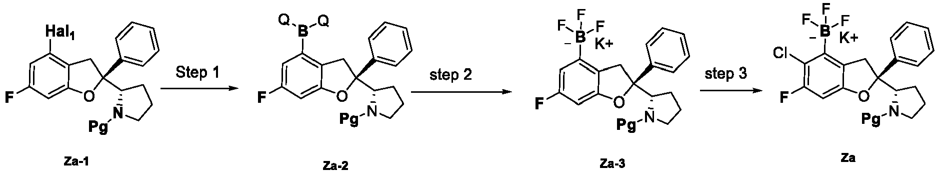
(i) coupling a borate ester under alkaline conditions with palladium catalysis, converting halogen Hal₁ in formula Za-1 into Q-B-Q to obtain Za-2;
(ii) adding potassium hydrogen fluoride aqueous solution to convert Q-B-Q into potassium trifluoroborate salt in an organic solvent at room temperature to obtain Za-3;
(iii) using a chlorinating reagent to convert Za-3 into Za under acid catalysis in an organic solvent;
in the formula, Hal₁ is halogen; Q-B-Q is boric acid or borate ester; Pg is a nitrogen protecting group.
